# EUROPEAN PATENT APPLICATION

(11) **EP 4 534 535 A1**
(43) Date of publication of application: **09.04.2025**
(21) Application number: 23815310.0
(22) Date of filing: 02.06.2023
(51) Int. Cl.: C07D 401/14, C07D 413/14, C07D 417/14, C07D 471/04, C07D 471/10, C07D 487/04, C07D 487/08, C07D 487/10, A61K 31/4704, A61K 31/506, A61P 35/00

(54) **COMPOUND FOR INHIBITING OR DEGRADING BCL6 AND USE THEREOF IN PHARMACEUTICS**

(30) Priority: 02.06.2022 CN 202210619706; 01.07.2022 CN 202210765237; 08.07.2022 CN 202210793644; 02.09.2022 CN 202211074021; 11.11.2022 CN 202211391155; 17.03.2023 CN 202310259014
(71) Applicant: Xizang Haisco Pharmaceutical Co., Ltd., Lhoka, Tibet 856099 (CN)
(72) Inventor: ZHANG, Chen, Lhoka, Tibet 856099 (CN); LIAO, Yuting, Lhoka, Tibet 856099 (CN); LU, Yonghua, Lhoka, Tibet 856099 (CN); LI, Yupeng, Lhoka, Tibet 856099 (CN); ZHAO, Junbin, Lhoka, Tibet 856099 (CN); TANG, Pingming, Lhoka, Tibet 856099 (CN); YU, Yan, Lhoka, Tibet 856099 (CN); LI, Yao, Lhoka, Tibet 856099 (CN); YAN, Pangke, Lhoka, Tibet 856099 (CN)
(74) Representative: IP TRUST SERVICES
(86) International application number: PCT/CN2023/097939
(87) International publication number: WO 2023/232133

(57) **Abstract**

The present invention relates to a compound represented by general formula (I) or a stereoisomer, a deuterated form, a solvate, a prodrug, a metabolite, a pharmaceutically acceptable salt or a eutectic thereof, and an intermediate and a pharmaceutical composition thereof, and use thereof in Bcl6-related diseases such as cancer. B-L-K (I).

## Description

### Technical Field

The present invention relates to a compound represented by general formula (I) or a stereoisomer, a deuterated form, a solvate, a prodrug, a metabolite, a pharmaceutically acceptable salt or a eutectic thereof, and an intermediate and a pharmaceutical composition thereof, and use thereof in Bcl6-related diseases such as cancer.

### Background Art

Bcl6 is a transcriptional repressor that regulates the development and function of germinal centre B cells. High expression of Bcl6 protein caused by various influencing factors such as exon mutations, regulatory pathway mutations, somatic Bcl6 translocation, and promoter mutations can lead to rapid proliferation of germinal centre B cells, thereby promoting the production of B-cell lymphomas. Moreover, Bcl6 can inhibit cell cycle checkpoint- and differentiation-related genes and DNA damage responses, and preclinical studies have shown that loss of Bcl6 in lymphoma cells can cause the arrest of tumour progression. Therefore, Bcl6 is a suitable target with potential for the treatment of multiple lymphomas.

PROTAC (proteolysis targeting chimera) molecules are a class of bifunctional compounds that can simultaneously bind targeting proteins and E3 ubiquitin ligases. Such compounds can be recognized by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years.

Therefore, it is necessary to develop novel PROTAC drugs that target Bcl6 protein and E3 ubiquitin ligases for use in the treatment of Bcl6-related tumour diseases.

### Summary of the Invention

An objective of the present invention is to provide a compound with a novel structure, good efficacy, high bioavailability and higher safety that can inhibit and degrade Bcl6, for use in the treatment of Bcl6-related diseases such as cancer.

The present invention provides a compound or a stereoisomer, a tautomer, a deuterated form, a solvate, a prodrug, a metabolite, a pharmaceutically acceptable salt or a eutectic thereof, wherein the compound is selected from a compound represented by general formula (I),

B-L-K (I);

in certain embodiments, the compound represented by general formula (I) is selected from a compound represented by general formula (II), 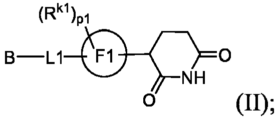
in certain embodiments, the compound represented by general formula (I) is selected from a compound represented by general formula (III), 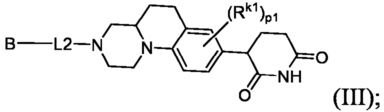
in certain embodiments, L is selected from a bond or -C₁₋₅₀ hydrocarbyl-, wherein the hydrocarbyl has 0 to 20 methylene units optionally further replaced by -Ak- or -Cy-;
in certain embodiments, L is selected from a bond or -C₁₋₂₀ hydrocarbyl-, wherein the hydrocarbyl has 0 to 20 methylene units optionally further replaced by -Ak- or -Cy-;
in certain embodiments, L is selected from a bond or -C₁₋₁₀ hydrocarbyl-, wherein the hydrocarbyl has 0 to 10 (such as 0, 1, 2, 3, 4, 5, 6, 7, 8, 9 or 10) methylene units optionally further replaced by -Ak- or -Cy-;
in certain embodiments, each -Ak- is independently selected from Ak1, Ak2, Ak3, Ak4, Ak5, Ak6, Ak7, Ak8 or Ak9;
in certain embodiments, each -Ak- is independently selected from -(CH₂)_{q}-, - (CH₂)_{q}-O-, -O-(CH₂)_{q}-, -(CH₂)_{q}-NR^{L}-, -NR^{L}-(CH₂)_{q}-, -(CH₂)_{q}-NR^{L}C(=O)-, - NR^{L}(CH₂)_{q}C(=O)-, -(CH₂)_{q}-C(=O)NR^{L}-, -C(=O)-, -C(=O)-(CH₂)_{q}-NR^{L}-, -(C≡C)_{q}-, -CH=CH-, -Si(R^{L})₂-, -Si(OH)(R^{L})-, -Si(OH)₂-, -P(=O)(OR^{L})-, -P(=O)(R^{L})-, -S-, - S(=O)-, -S(=O)₂- or a bond, wherein the -CH₂- is optionally substituted with 1 to 2 substituents selected from halogen, OH, CN, NH₂, C₁₋₆ alkyl, C₁₋₆ alkoxy, halogen-substituted C₁₋₆ alkyl, hydroxyl-substituted C₁₋₆ alkyl or cyano-substituted C₁₋₆ alkyl;
in certain embodiments, Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, -(CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from halogen, OH, CN, NH₂, C₁₋₄ alkyl, C₁₋₄ alkoxy, halogen-substituted C₁₋₄ alkyl, hydroxyl-substituted C₁₋₄ alkyl or cyano-substituted C₁₋₄ alkyl;
in certain embodiments, Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, -(CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from F, Cl, Br, I, OH, CN, NH₂, CF₃, hydroxymethyl, C₁₋₄ alkyl or C₁₋₄ alkoxy;
in certain embodiments, Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, -(CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from F, Cl, Br, I, OH, CN, NH₂, CF₃, hydroxymethyl, methyl, ethyl, methoxy or ethoxy;
in certain embodiments, Ak1, Ak2, Ak3, Ak4, Ak5, Ak6, Ak7, Ak8 and Ak9 are each independently selected from a bond, -O-, -OCH₂-, -CH₂O-, -OCH₂CH₂-, - CH₂CH₂O-, -C≡C-, -C(CH₃)₂-, -CF₂-, -CH₂-, -CH₂CH₂-, -CH₂CH₂CH₂-, -N(CH₃)-, -NH-, -CH₂N(CH₃)-, -CH₂NH-, -NHCH₂-, -CH₂CH₂N(CH₃)-,
in certain embodiments, each R^{L} is independently selected from H or C₁₋₆ alkyl;
in certain embodiments, each R^{L} is independently selected from H or C₁₋₄ alkyl;
in certain embodiments, R^{L} is selected from H, methyl or ethyl;
in certain embodiments, each q is independently selected from 0, 1, 2, 3, 4, 5 or 6;
in certain embodiments, each q is independently selected from 0, 1, 2, 3 or 4;
in certain embodiments, each q is independently selected from 0, 1, 2 or 3;
in certain embodiments, each -Cy- is independently selected from Cy1, Cy2, Cy3, Cy4 or Cy5;
in certain embodiments, each -Cy- is independently selected from a bond, a 4-to 8-membered mono-heterocyclic ring, a 4- to 10-membered fused-heterocyclic ring, a 5- to 12-membered spiro-heterocyclic ring, a 7- to 10-membered bridged-heterocyclic ring, C₃₋₇ monocycloalkyl, C₄₋₁₀ fused cycloalkyl, C₅₋₁₂ spirocycloalkyl, C₇₋₁₀ bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally substituted with 1 to 2 substituents selected from halogen, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally substituted with 1 or 2 =O when the heteroatom is selected from S;
in certain embodiments, each Cy1, Cy2, Cy3, Cy4 or Cy5 is independently selected from a bond, a 4- to 7-membered mono-heterocyclic ring, a 4- to 10-membered fused-heterocyclic ring, a 5- to 12-membered spiro-heterocyclic ring, a 7- to 10-membered bridged-heterocyclic ring, C₃₋₇ monocycloalkyl, C₄₋₁₀ fused cycloalkyl, C₅₋₁₂-membered spirocycloalkyl, C₇₋₁₀-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally substituted with 1 to 2 substituents selected from F, Cl, Br, I, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally substituted with 1 or 2 =O when the heteroatom is selected from S;
in certain embodiments, each Cy1, Cy2, Cy3, Cy4 or Cy5 is independently selected from a bond, a 4- to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, C₃₋₇ monocycloalkyl, C₄₋₁₀ fused cycloalkyl, C₅₋₁₂ spirocycloalkyl, C₇₋₁₀ bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally substituted with 1 to 2 substituents selected from F, Cl, Br, I, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the mono-heterocyclic ring, fused-heterocyclic ring, bridged-heterocyclic ring, spiro-heterocyclic ring or heteroaryl contains 1 to 4 heteroatoms selected from O, S or N, and is optionally substituted with 1 or 2 =O when the heteroatom is selected from S;
in certain embodiments, each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, pyrrolidyl, azacyclohexenyl, piperidyl, morpholinyl, piperazinyl, 1,4-diazepanyl, phenyl, pyridyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-pyrrolidyl, cyclopropyl-fused-piperidyl, cyclobutyl-fused-azetidinyl, cyclobutyl-fused-pyrrolidyl, cyclobutyl-fused-piperidyl, cyclopentyl-fused-azetidinyl, cyclopentyl-fused-pyrrolidyl, cyclopentyl-fused-piperidyl, cyclohexyl-fused-azetidinyl, cyclohexyl-fused-pyrrolidyl, cyclohexyl-fused-piperidyl, azetidinyl-fused-azetidinyl, azetidinyl-fused-pyrrolidyl, azetidinyl-fused-piperidyl, pyrrolidyl-fused-azetidinyl, pyrrolidyl-fused-pyrrolidyl, pyrrolidyl-fused-piperidyl, piperidyl-fused-azetidinyl, piperidyl-fused-pyrrolidyl, piperidyl-fused-piperidyl, cyclopropyl-spiro-azetidinyl, cyclopropyl-spiro-pyrrolidyl, cyclopropyl-spiro-piperidyl, cyclopropyl-spiro-piperazinyl, cyclobutyl-spiro-azetidinyl, cyclobutyl-spiro-pyrrolidyl, cyclobutyl-spiro-piperidyl, cyclopentyl-spiro-azetidinyl, cyclopentyl-spiro-pyrrolidyl, cyclopentyl-spiro-piperidyl, cyclohexyl-spiro-azetidinyl, cyclohexyl-spiro-pyrrolidyl, cyclohexyl-spiro-piperidyl, azetidinyl-spiro-azetidinyl, azetidinyl-spiro-pyrrolidyl, azetidinyl-spiro-piperidyl, pyrrolidyl-spiro-azetidinyl, pyrrolidyl-spiro-pyrrolidyl, pyrrolidyl-spiro-piperidyl, piperidyl-spiro-azetidinyl, piperidyl-spiro-pyrrolidyl, piperidyl-spiro-piperidyl, cyclopropyl-spiro-piperazinyl, 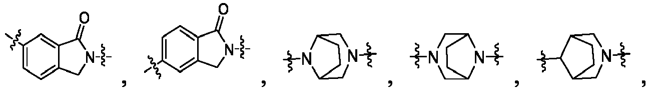 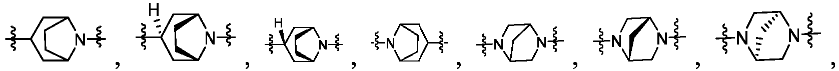 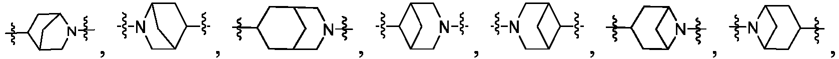 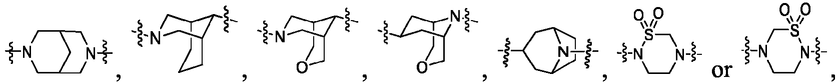 which, when substituted, is optionally substituted with 1 to 4 substituents selected from F, Cl, Br, I, OH, NH₂, COOH, CN, =O, C₁₋₄ alkyl, halogen-substituted C₁₋₄ alkyl, hydroxyl-substituted C₁₋₄ alkyl or C₁₋₄ alkoxy;
in certain embodiments, each Cy1, Cy2, Cy3, Cy4 or Cy5 is independently selected from a bond or one of the following substituted or unsubstituted groups: 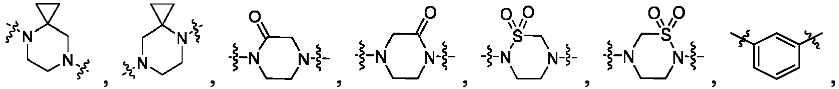 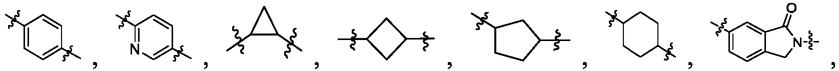 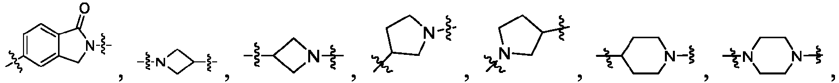 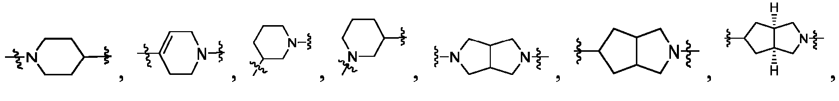 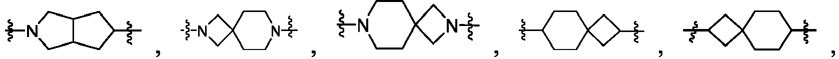 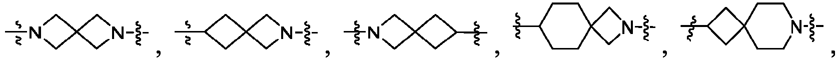 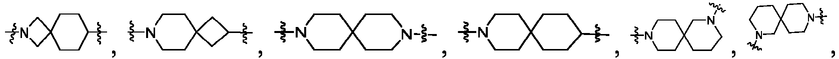 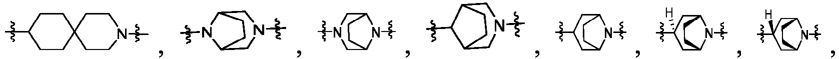 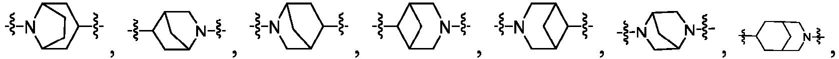 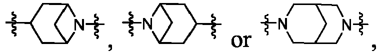 which, when substituted, is substituted with 1 to 4 substituents selected from F, CF₃, OH, methyl, =O, hydroxymethyl, COOH, CN or NH₂;
in certain embodiments, each Cy1, Cy2 or Cy3 is independently selected from a bond or one of the following substituted or unsubstituted groups: 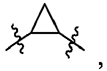 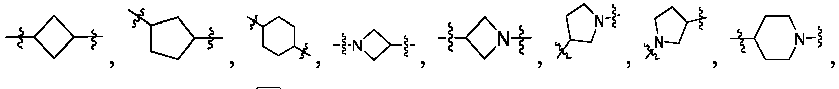 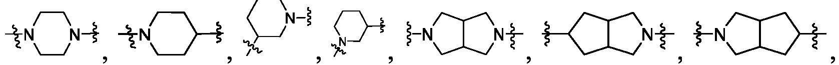 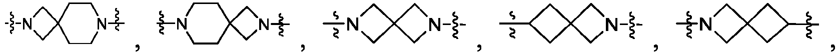 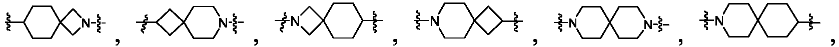 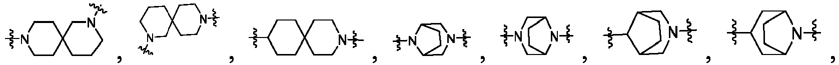 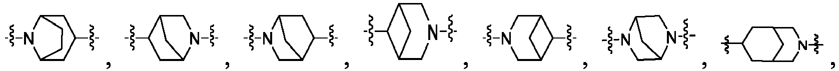 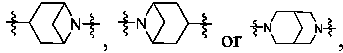 which, when substituted, is substituted with 1 to 4 substituents selected from F, CF₃, OH, methyl, =O, hydroxymethyl, COOH, CN or NH₂;
in certain embodiments, each Cy1 or Cy2 is independently selected from one of the following substituted or unsubstituted groups: 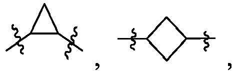 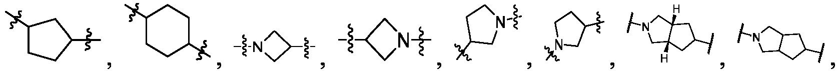 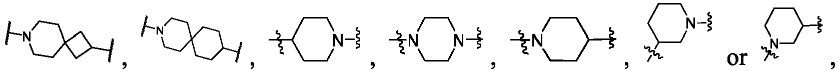 which, when substituted, is substituted with 1 to 4 substituents selected from F, CF₃, OH, methyl, =O, hydroxymethyl, COOH, CN or NH₂;
in certain embodiments, L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, - Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, -Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, - Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, -Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, -Ak 1-Ak2-Ak3-Ak4-Cy 1-Cy2-Cy3-Ak5-Cy4-, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9;
in certain embodiments, L is selected from a bond, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cy1-, -Cy1-Ak1-, -Cy1-Ak1-Ak2-, -Cy1-Ak1-Ak2-Ak3-, -Cy1-Ak1-Ak2-Ak3-Ak4-, -Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy1-Ak1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, -Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, -Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, - Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, -Ak1-Cy2-Ak2-Ak3-Ak4-Ak5-, -Ak1-Cy2-Ak2-Ak3-Ak4- or - Ak1-Cy2-Ak2-Ak3-;
in certain embodiments, L is selected from -Cy1-Ak1-, -Cy1-Ak1-Ak2-, - Cy1-Cy2-, -Cy1-Cy2-Ak2-, -Cy1-Ak1-Cy2-, -Cy1-Ak1-Cy2-Ak2-, -Cy1-Cy2-Cy3-, -Cy1-Ak1-Cy2-Cy3-, -Cy1-Cy2-Ak2-Cy3- or -Ak1-Cy2-Cy3-;
in certain embodiments, L is selected from a bond or a group shown in Table L-1, wherein the left side of the group is linked to B;
in certain embodiments, L is selected from a bond or a group shown in Table L-2, wherein the left side of the group is linked to B; 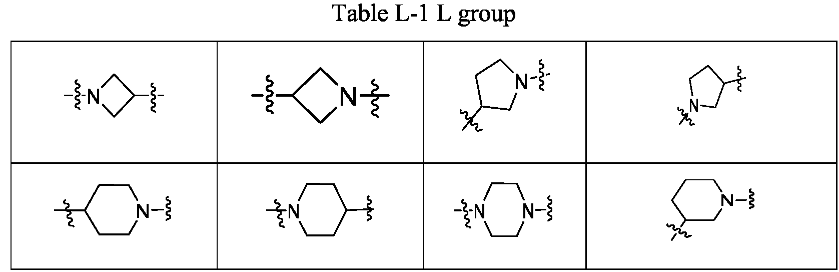 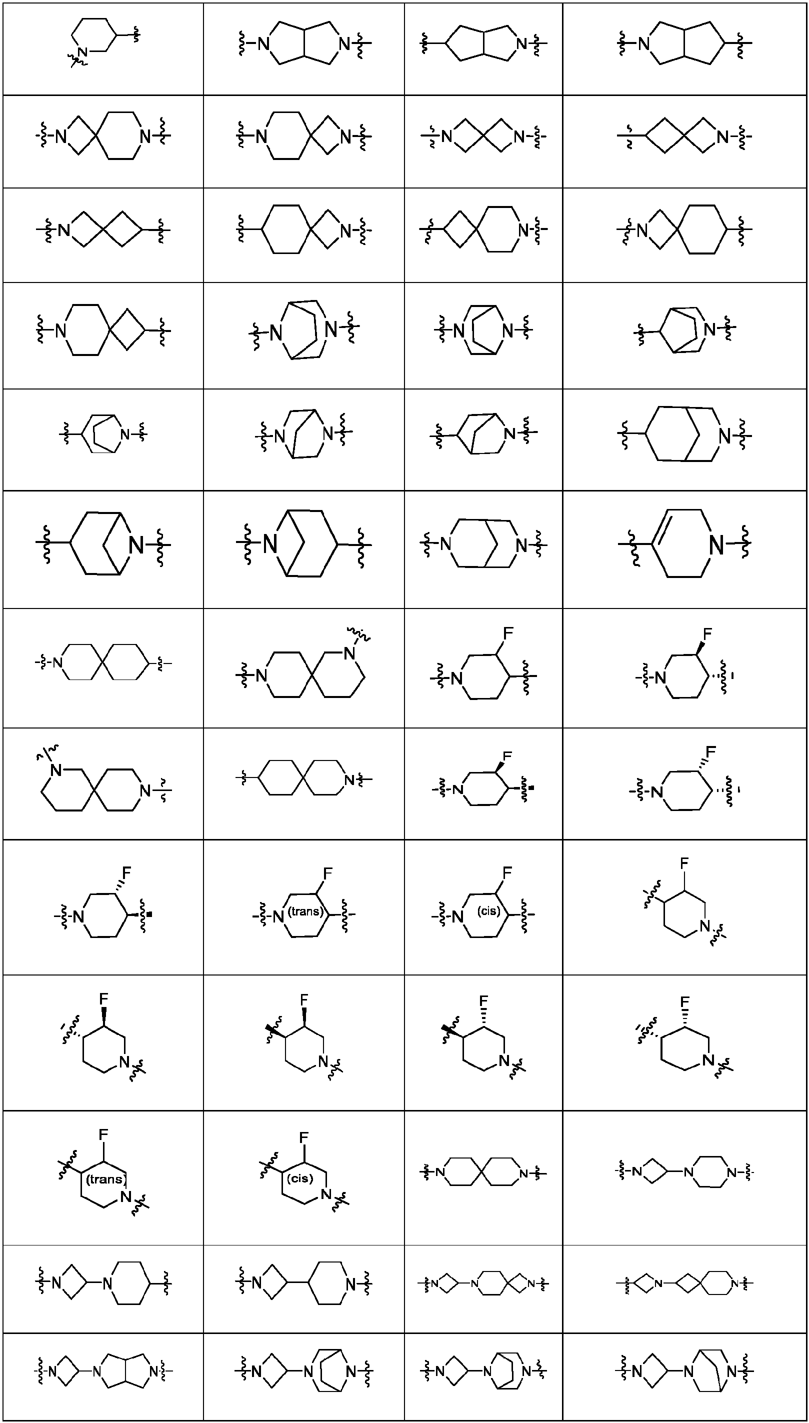 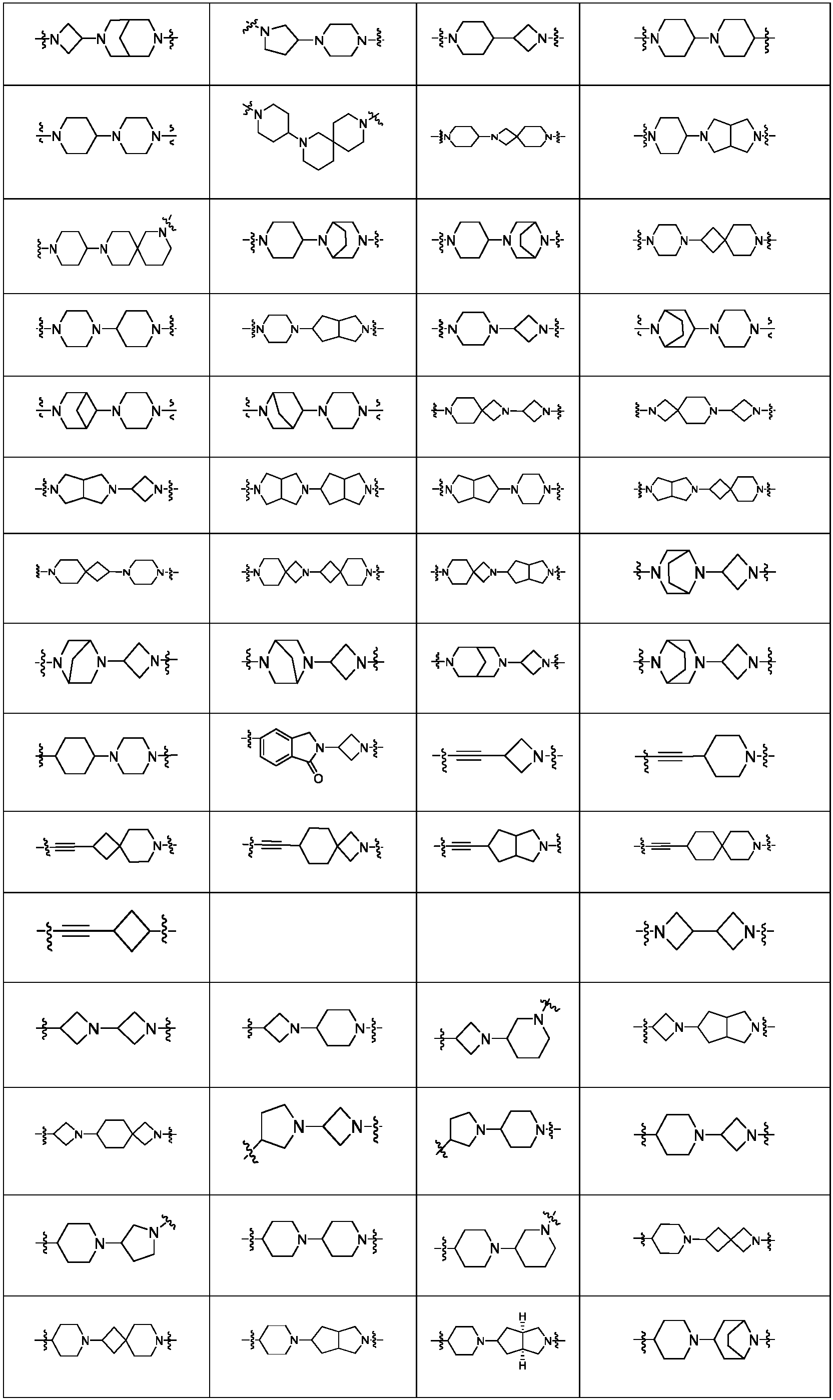 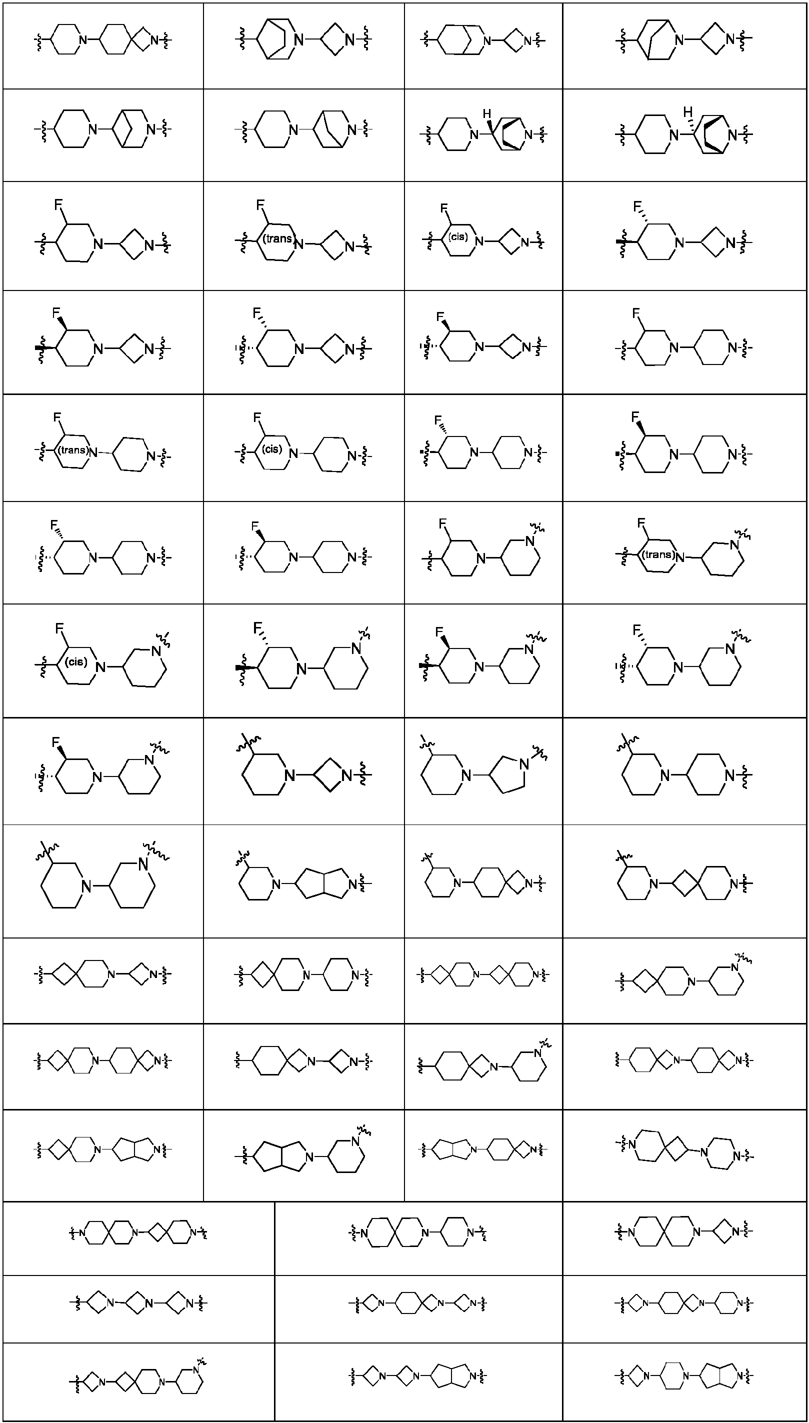 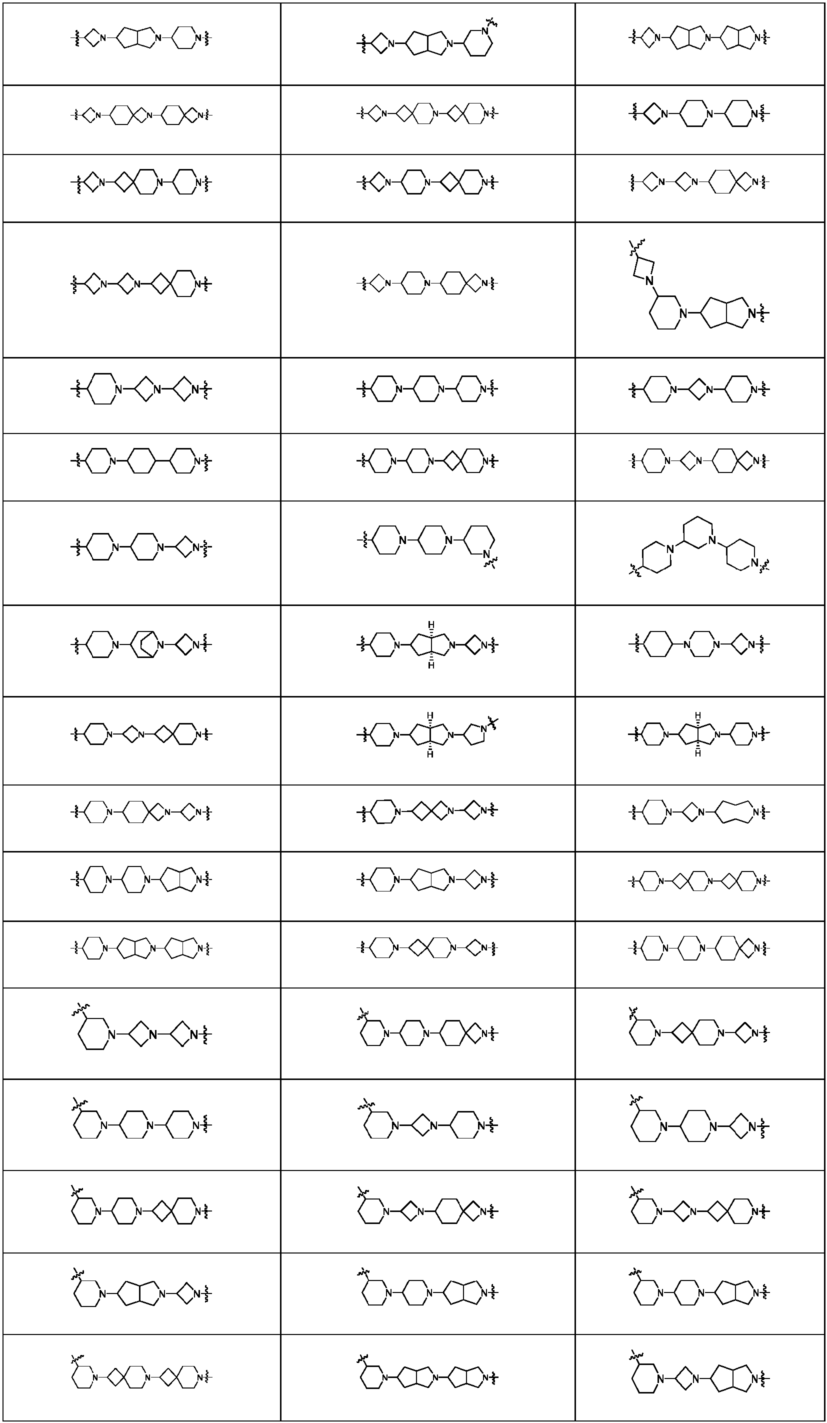 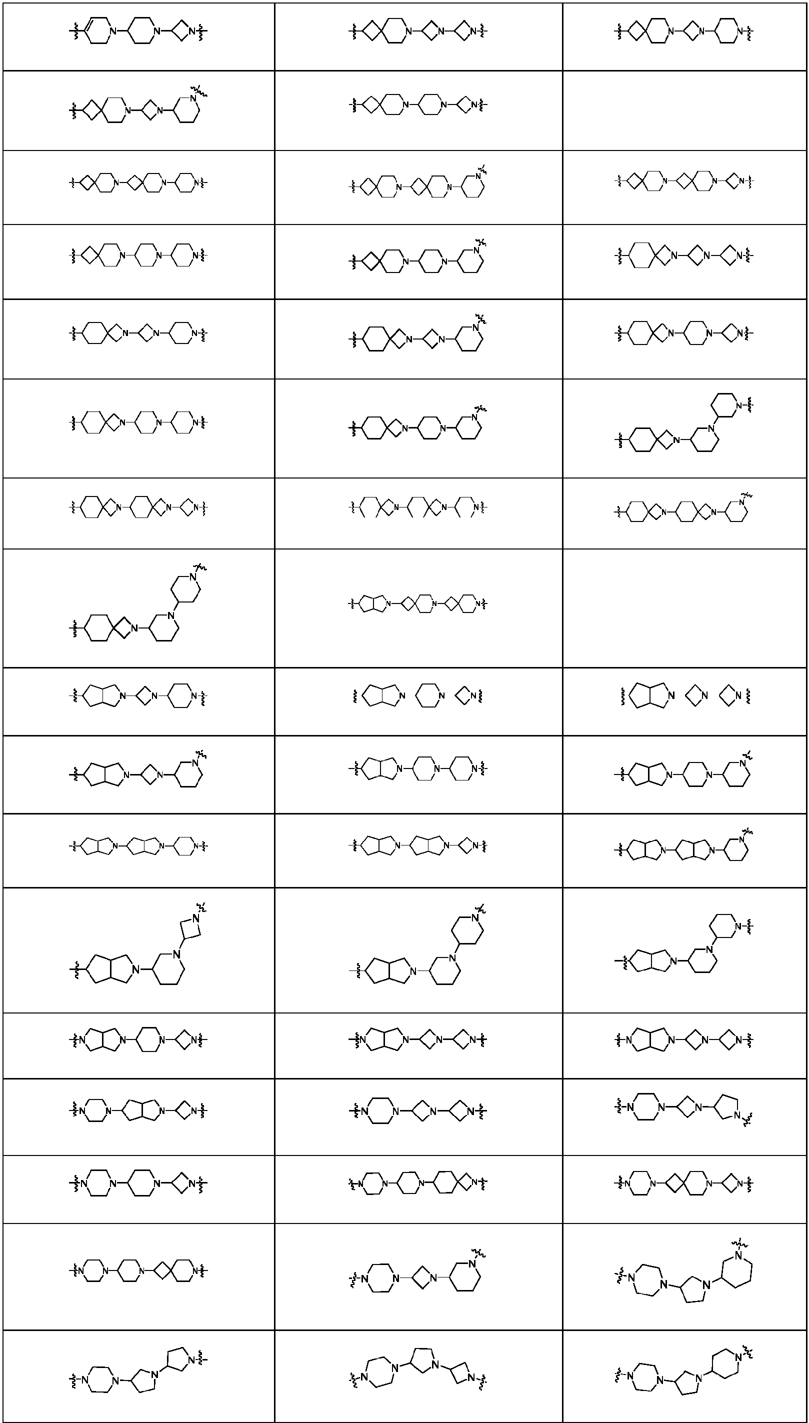 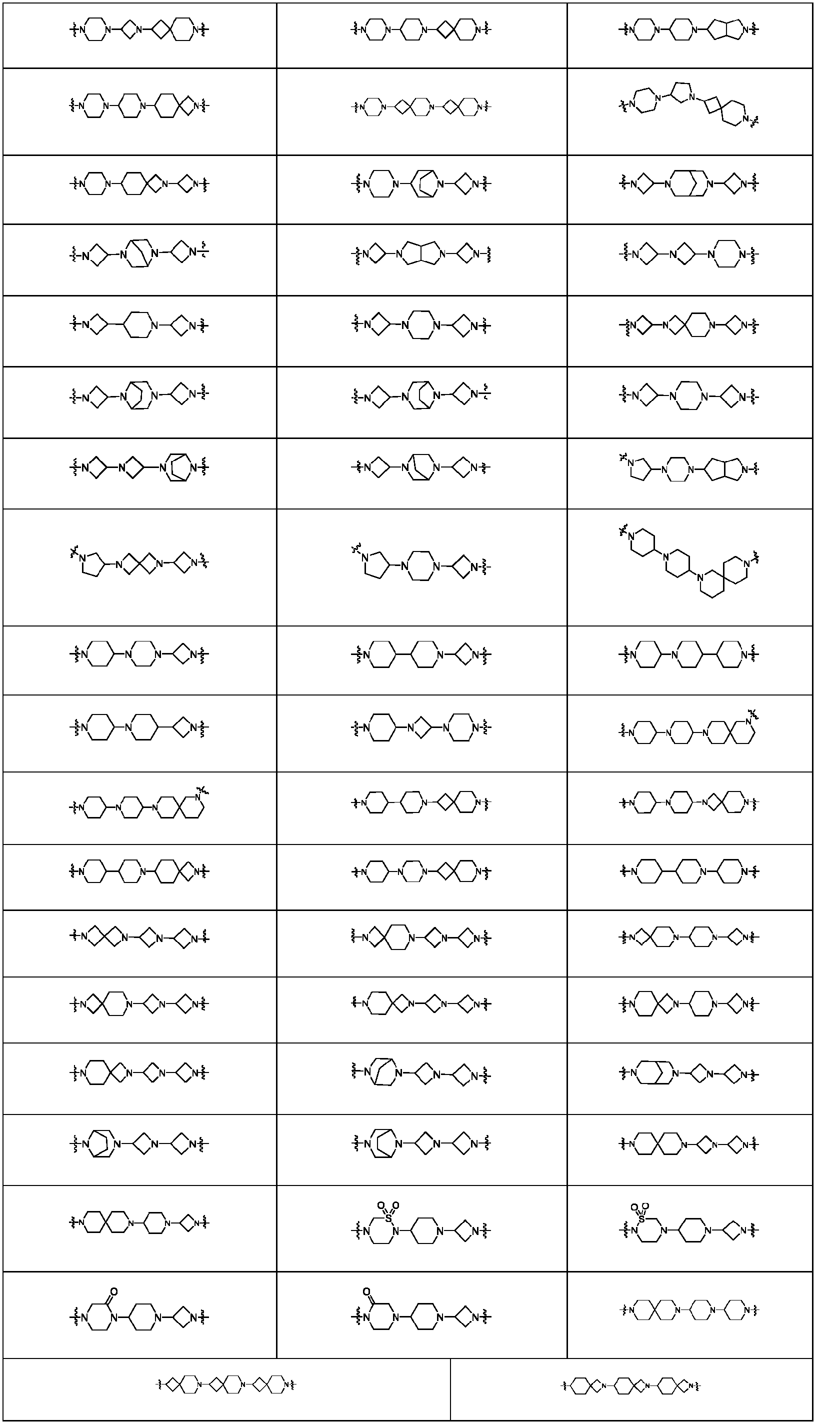 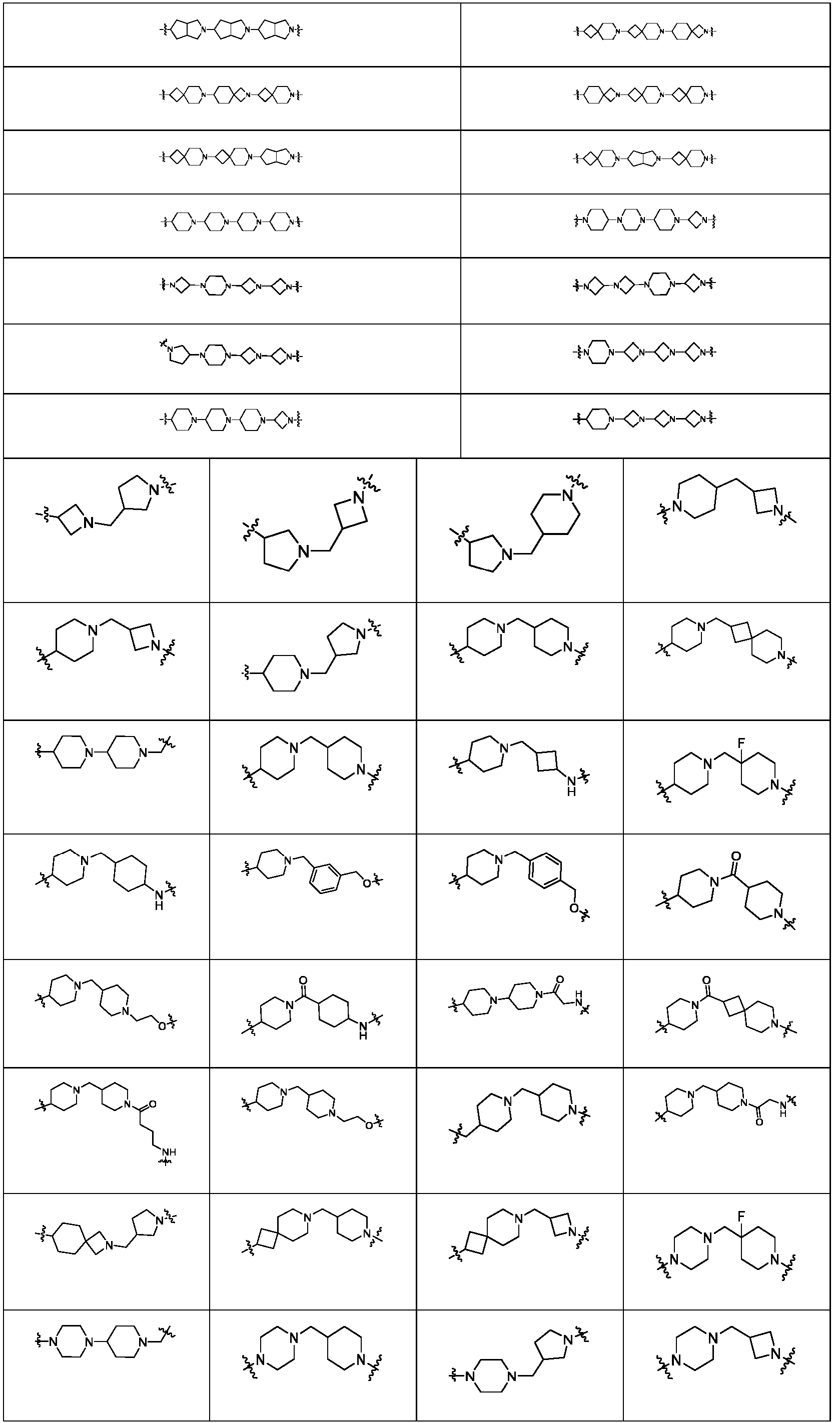 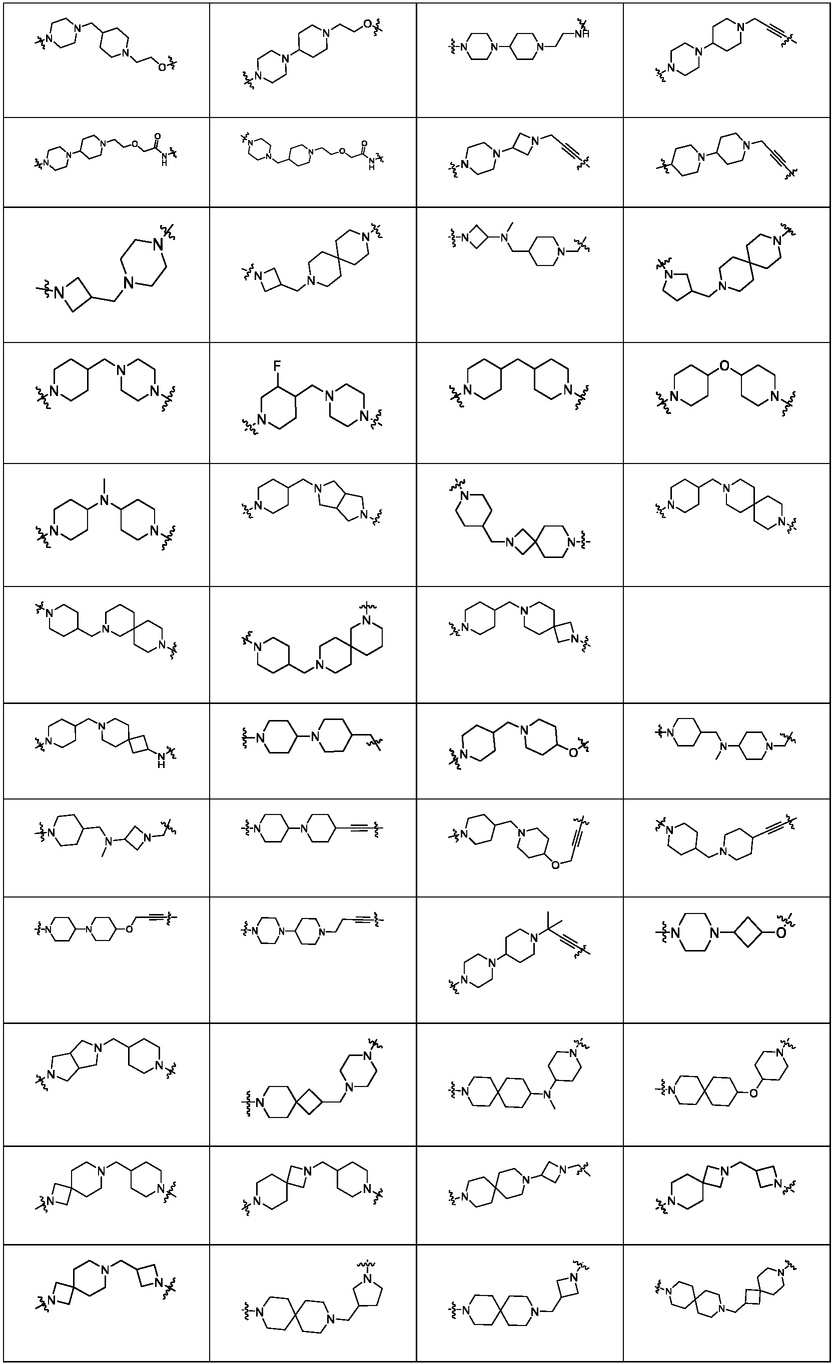 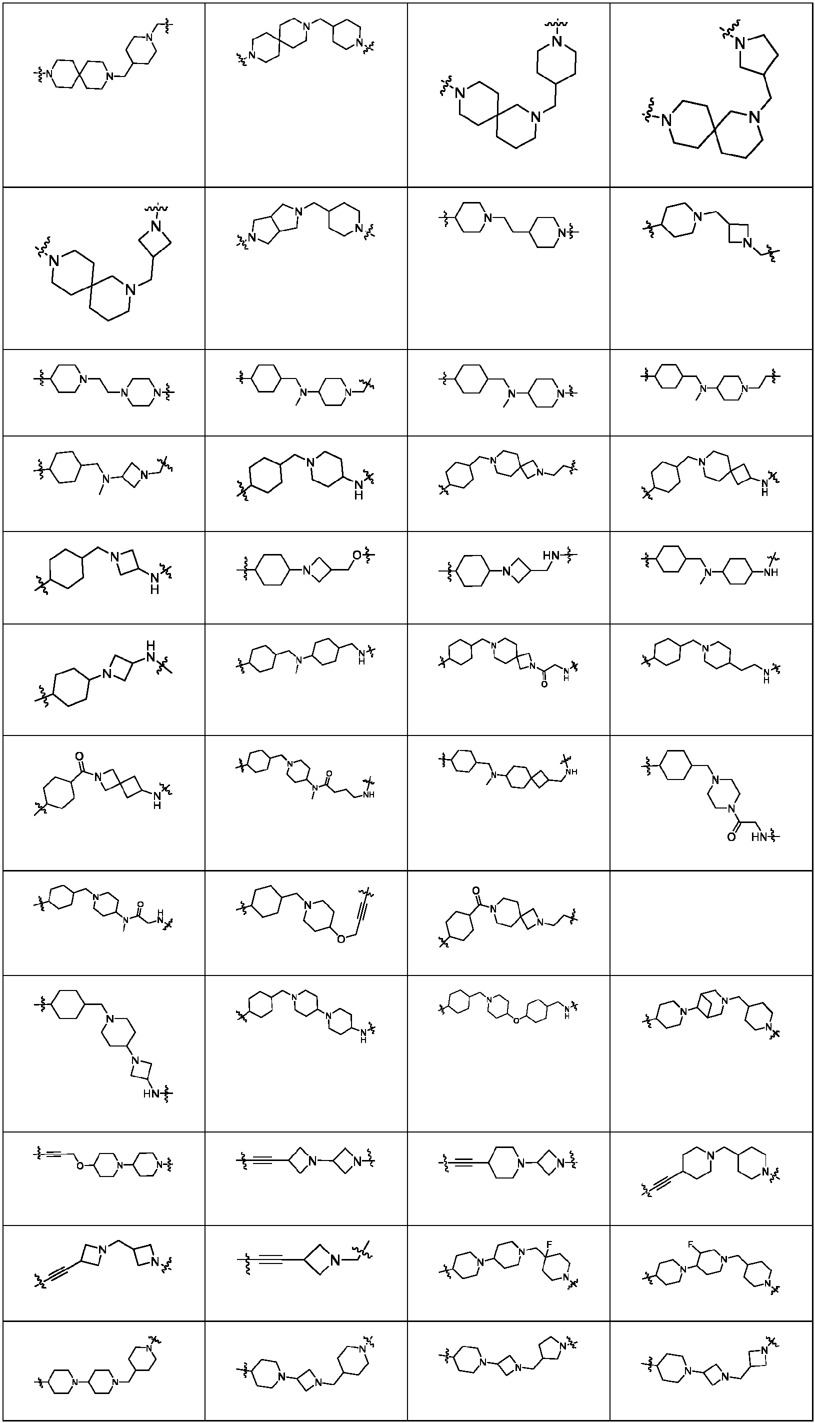 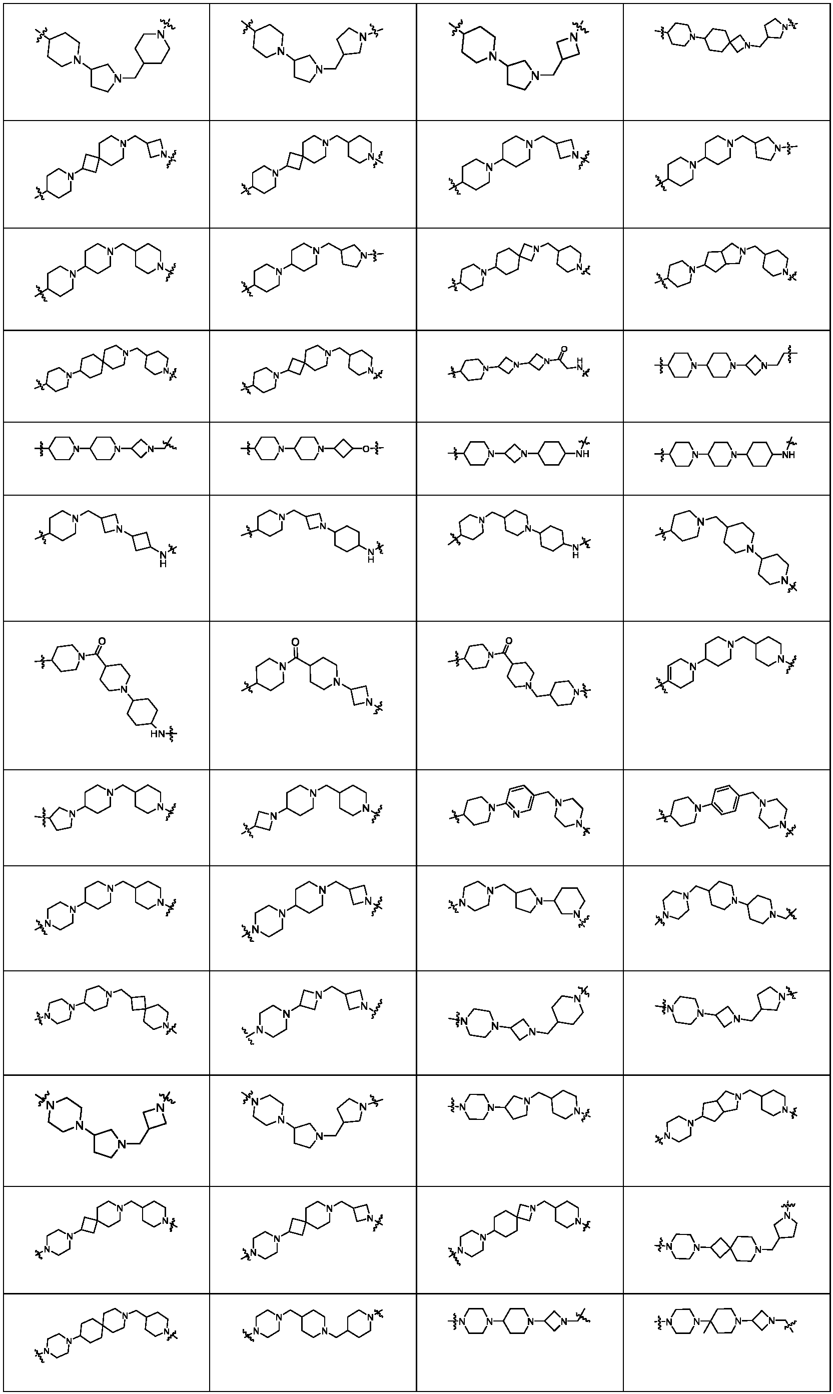 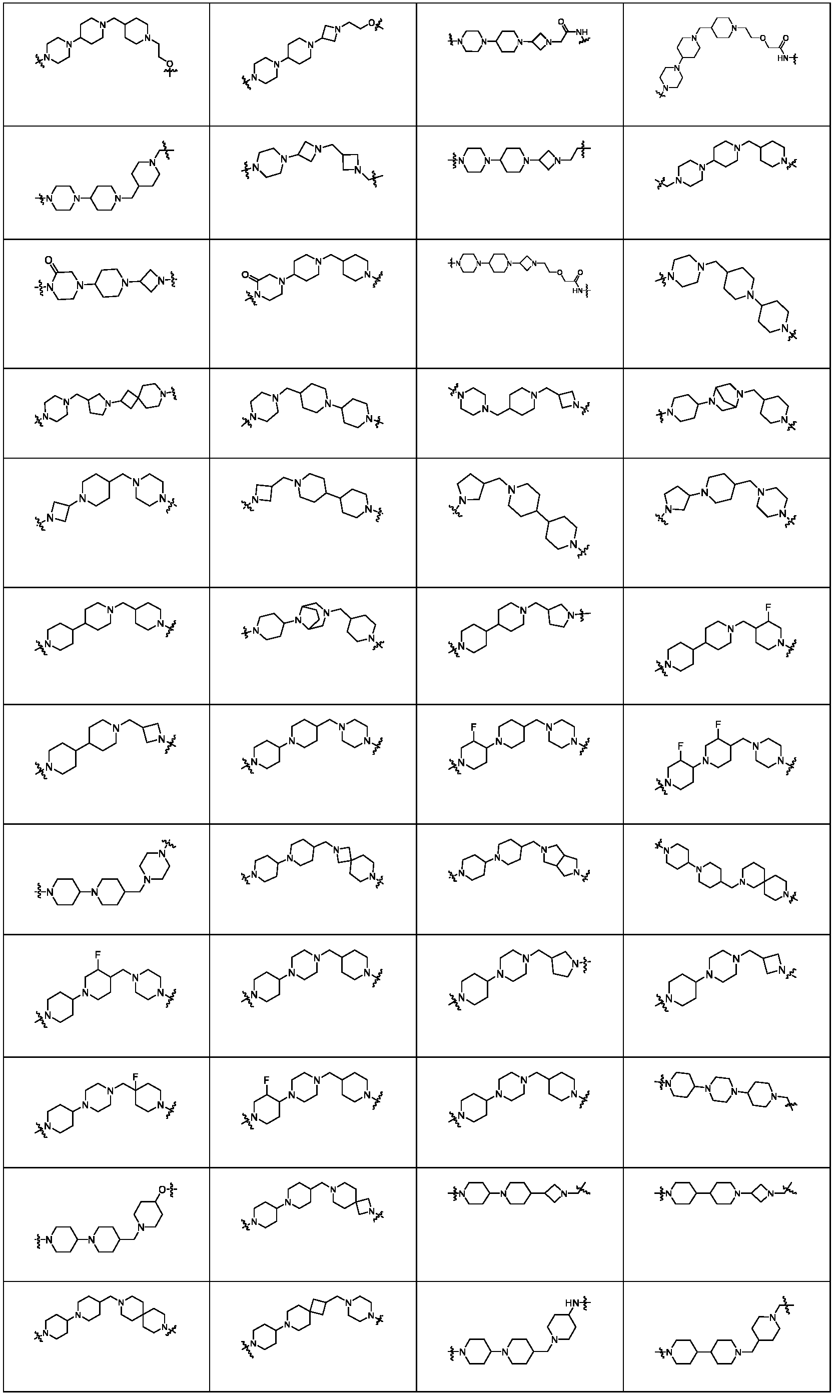 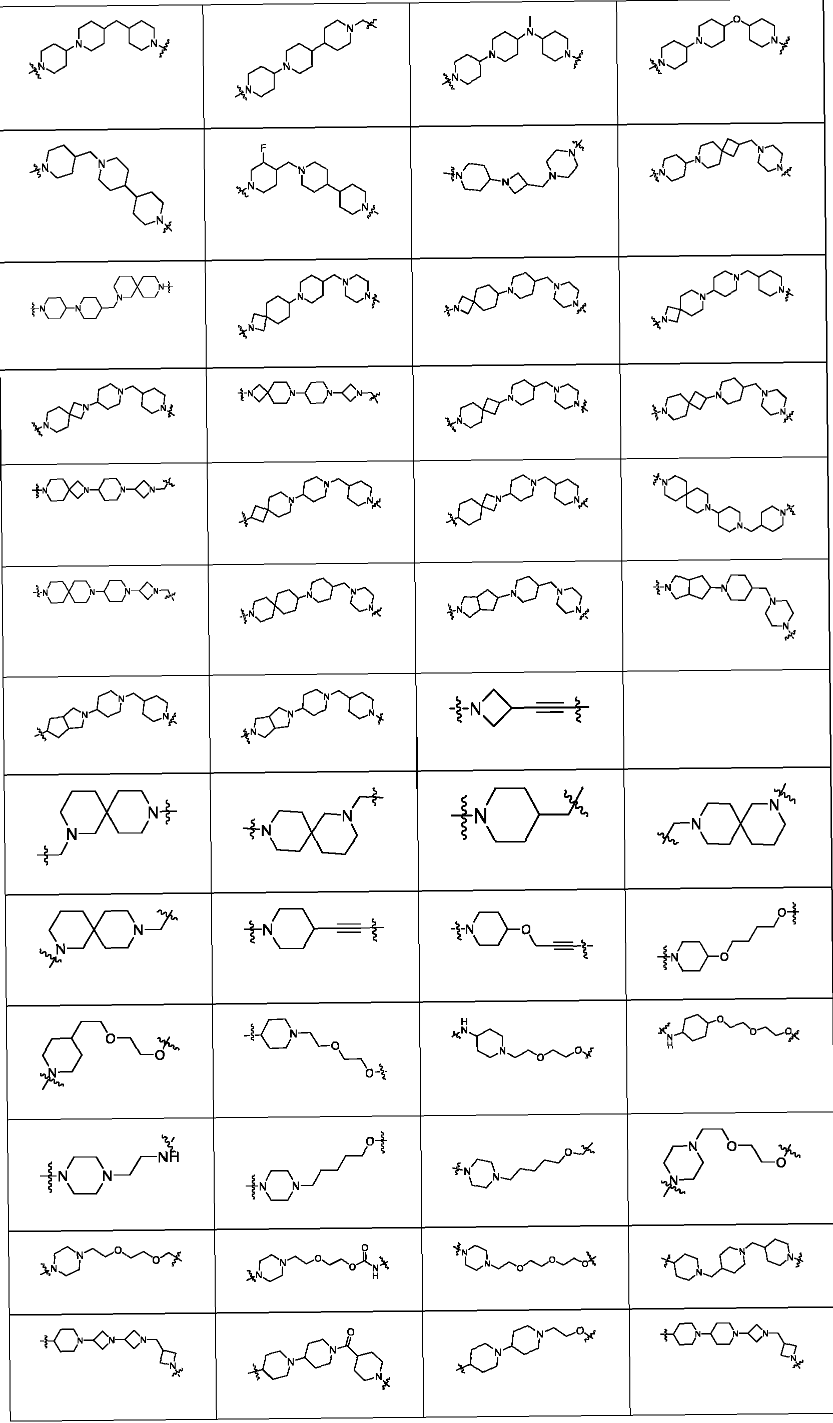 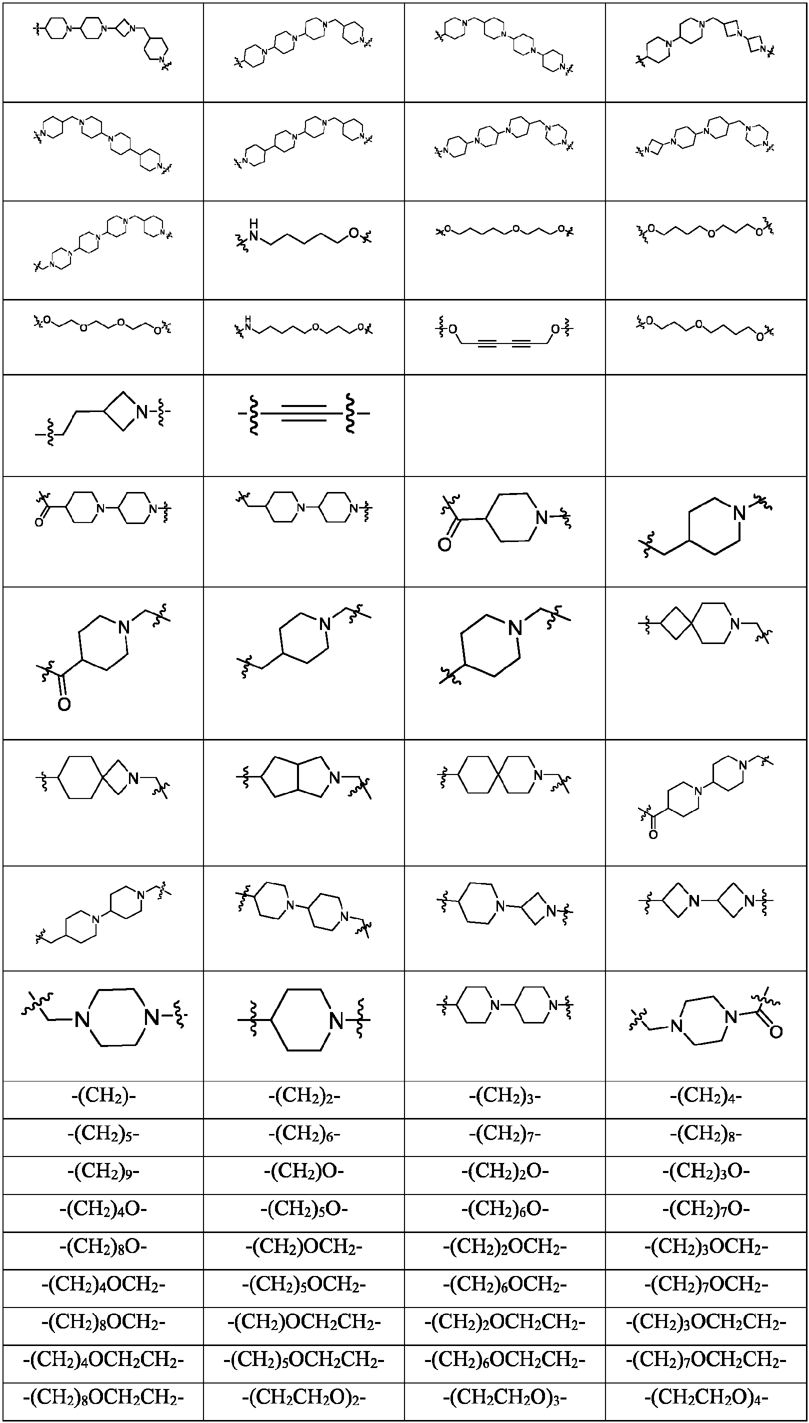 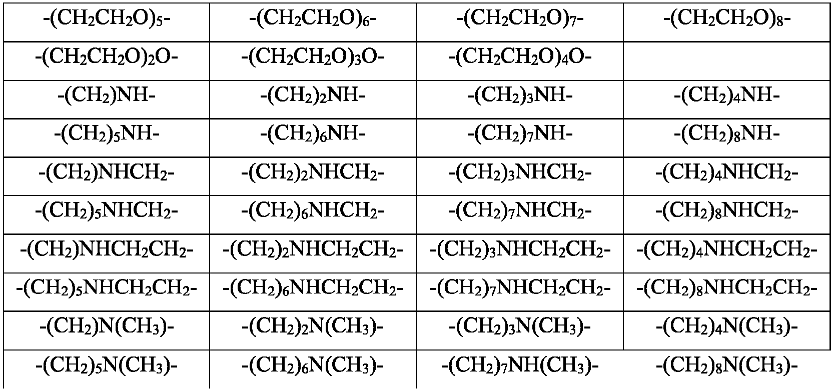 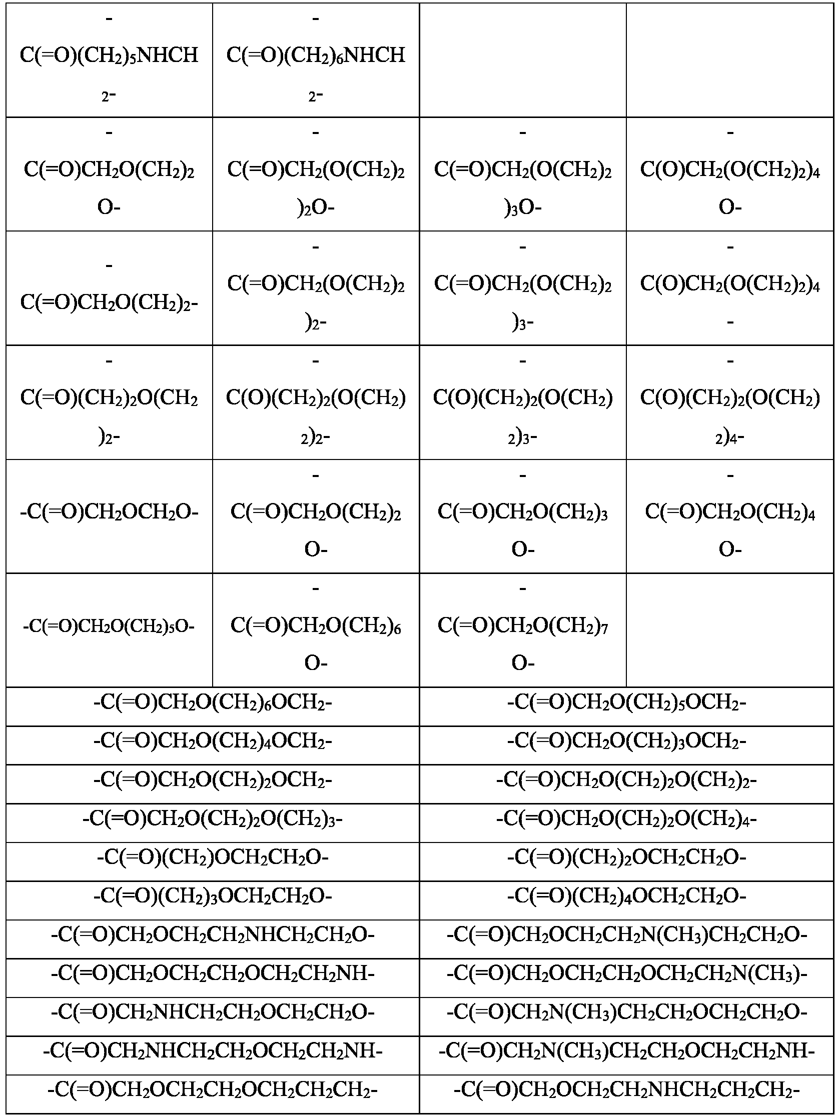 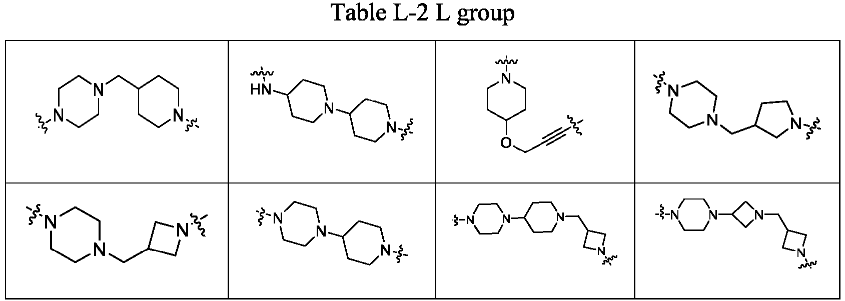 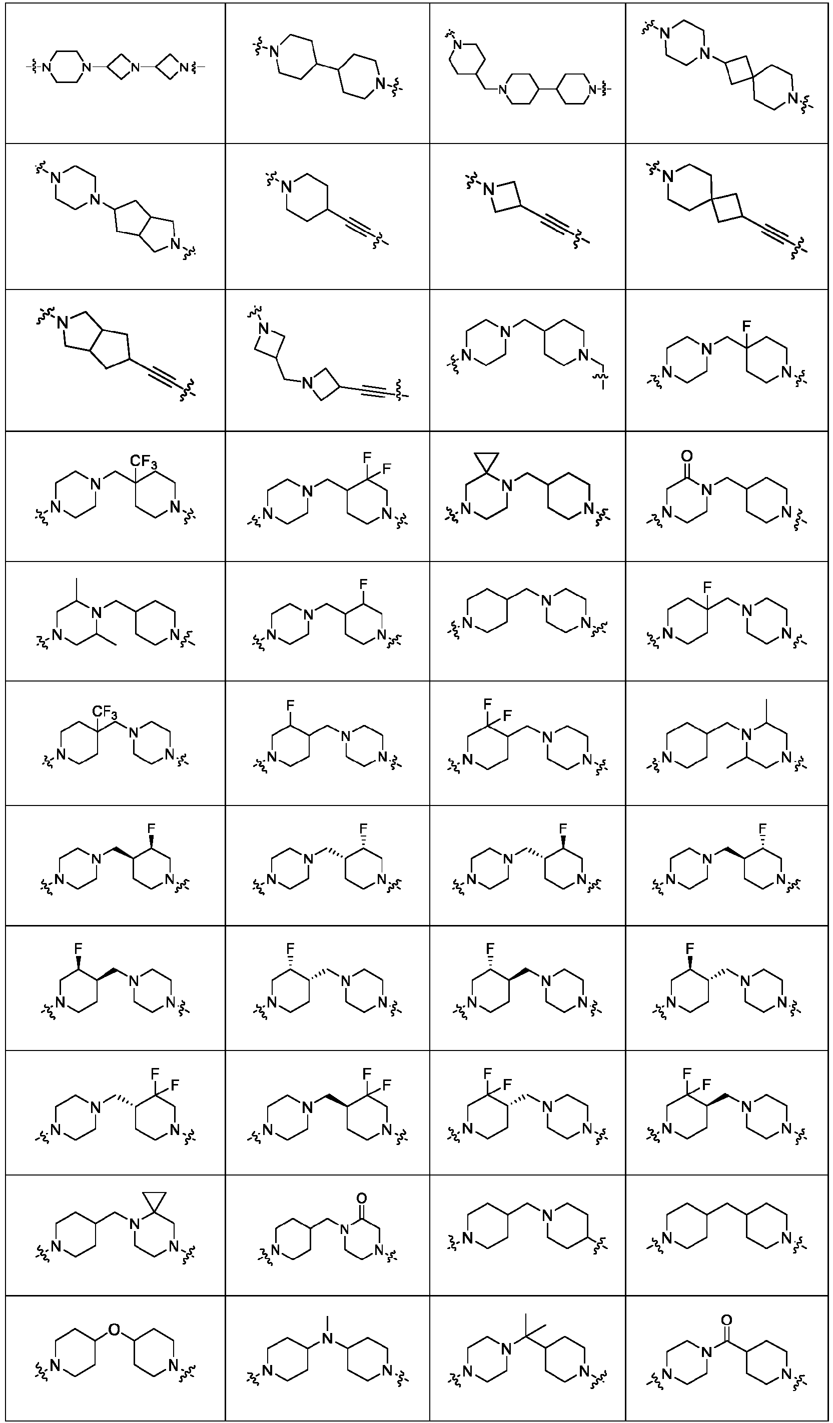 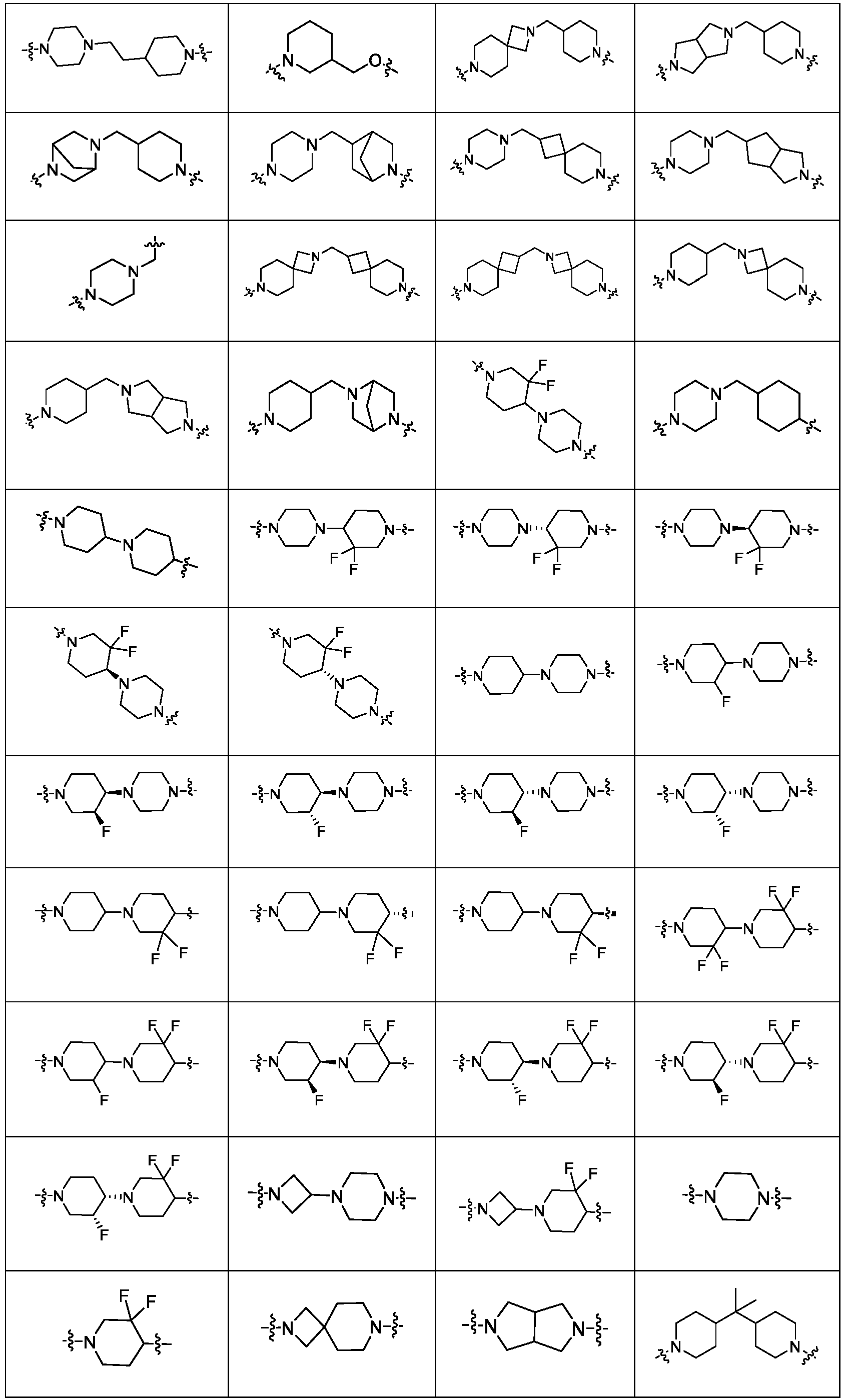 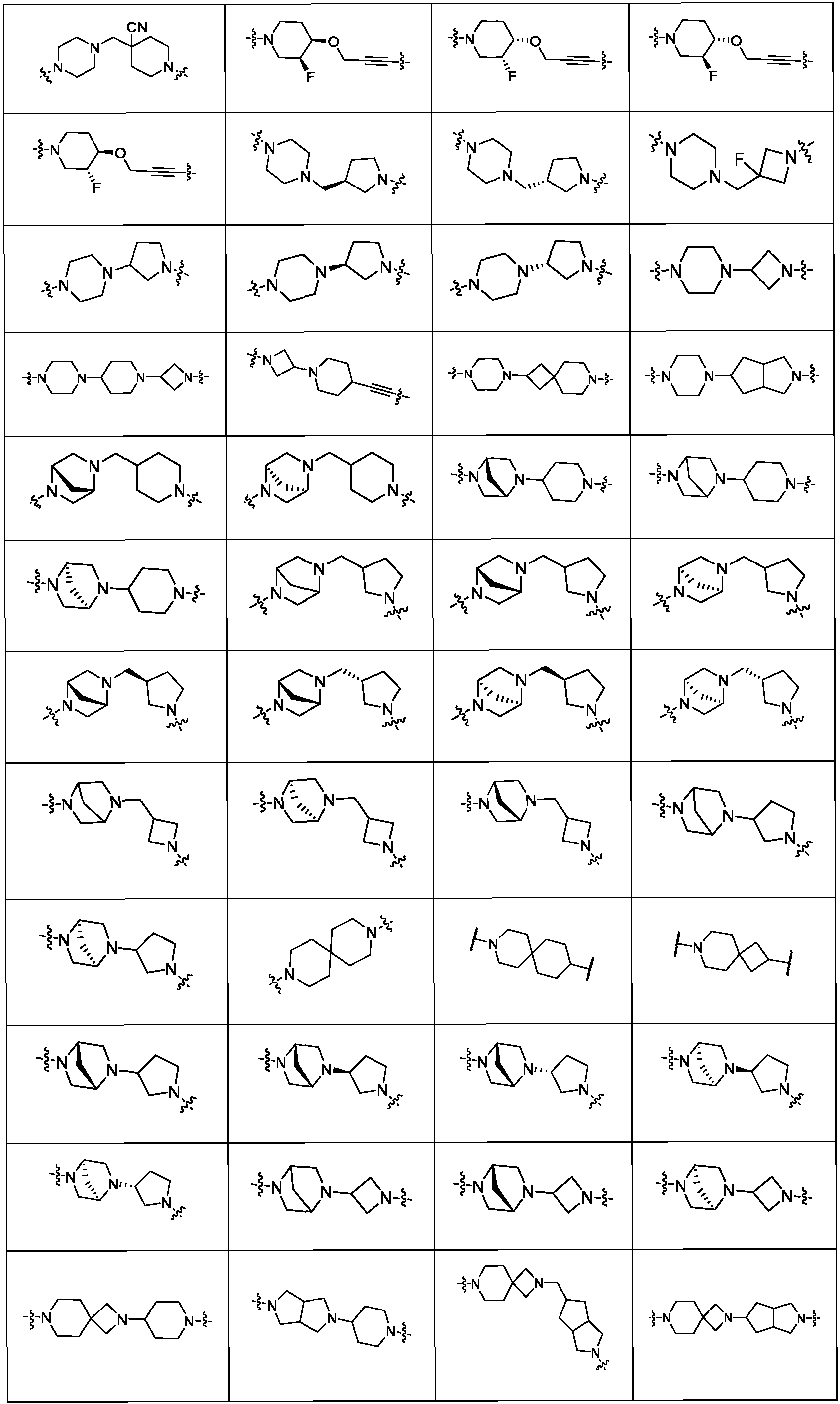 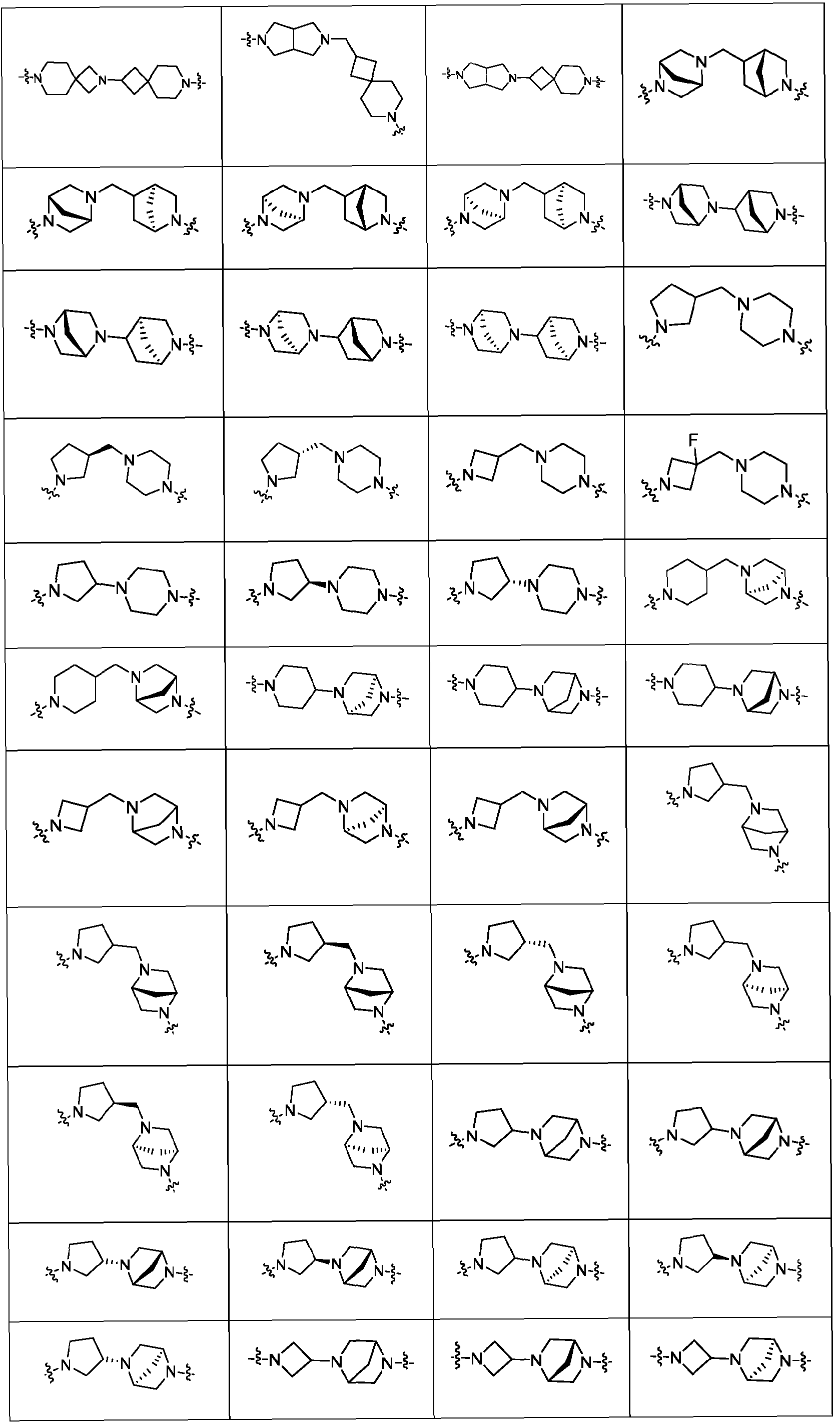 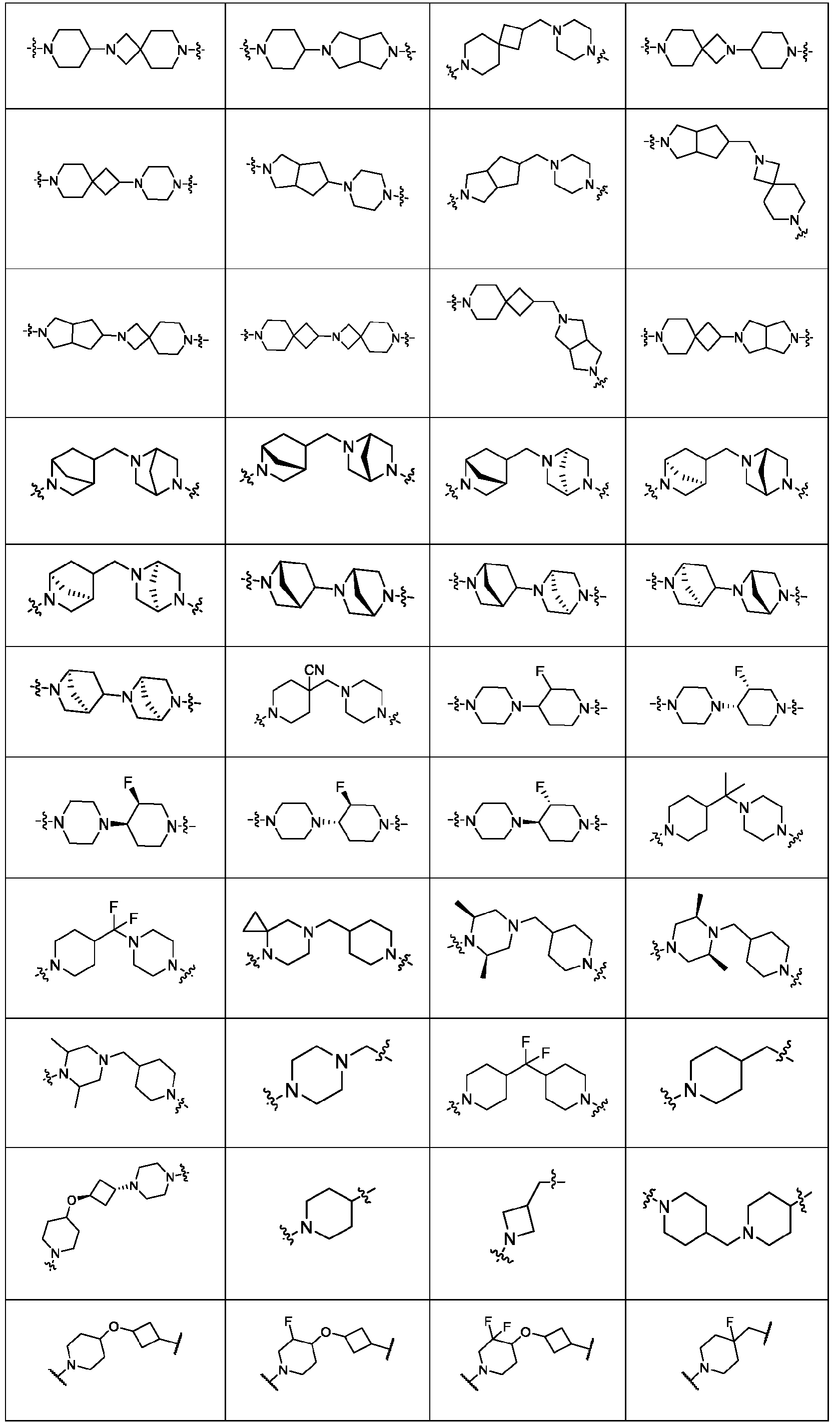 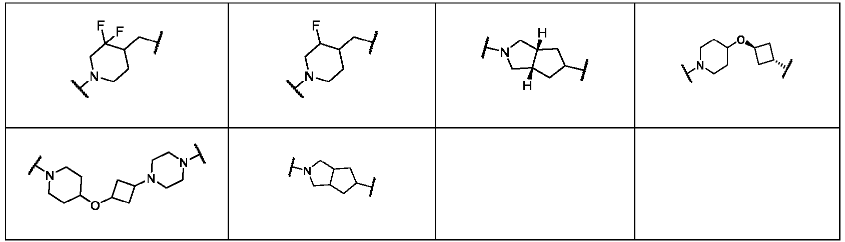 ;
in certain embodiments, L is selected from L1 or L2;
in certain embodiments, L1 is selected from -Cy1-, -Cy1- Cy2-, -Cy1-Ak1-, - Cy1-Ak1-Cy2- or -Cy1-Ak1-Cy2-Cy3-;
in certain embodiments, L2 is selected from -Cy1-, -Cy1-Ak1- or -Cy1-Ak1-Cy2-;
in certain embodiments, L1 is selected from 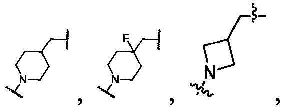 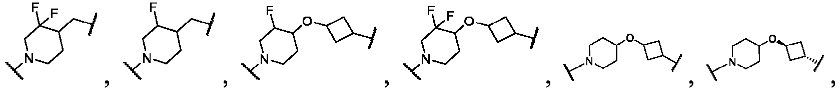 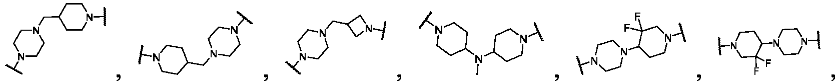 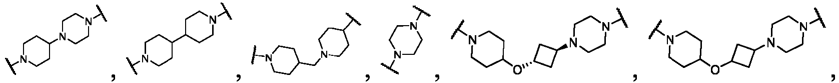 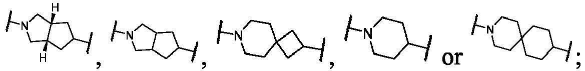
in certain embodiments, L2 is selected from 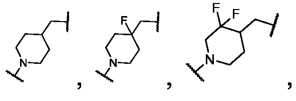 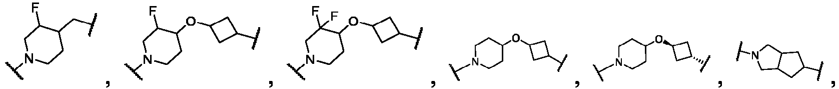 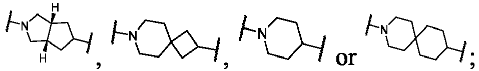
in certain embodiments, B is selected from 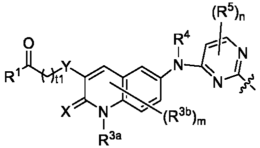 or 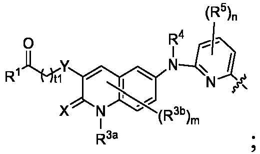
in certain embodiments, B is selected from 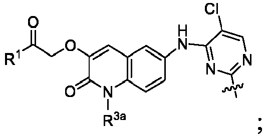
in certain embodiments, X is selected from O, S or CH₂;
in certain embodiments, X is selected from O or S;
in certain embodiments, Y is selected from NR^{Y} or O;
in certain embodiments, Y is selected from NH or O;
in certain embodiments, R^{Y} is selected from H, C₁₋₆ alkyl, C₃₋₁₀ carbocycle or 3- to 10-membered heterocycle, wherein the alkyl, carbocycle or heterocycle is optionally substituted with 1 to 3 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₃₋₆ carbocycle or 3- to 8-membered heterocycle, and the heterocycle contains 1 to 3 heteroatoms selected from O, S or N;
in certain embodiments, R^{Y} is selected from H, C₁₋₄ alkyl, C₃₋₆ carbocycle or 3- to 6-membered heterocycle, wherein the alkyl, carbocycle or heterocycle is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 8-membered heterocycle, and the heterocycle contains 1 to 3 heteroatoms selected from O, S or N;
in certain embodiments, R^{Y} is selected from H, methyl, ethyl, propyl, isopropyl or cyclopropyl, wherein the methyl, ethyl, propyl, isopropyl, cyclopropyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
in certain embodiments, R^{Y} is selected from H;
in certain embodiments, R¹ is selected from H, OH, -(CH₂)ₜ₂NR^{1a}R^{1b}, C₁₋₆ alkyl or C₁₋₆ alkoxy, wherein the alkyl or alkoxy is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl or C₁₋₆ alkoxy;
in certain embodiments, R¹ is selected from H, OH, -(CH₂)ₜ₂NR^{1a}R^{1d}, C₁₋₄ alkyl or C₁₋₄ alkoxy, wherein the alkyl or alkoxy is optionally substituted with 1 to 4 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl or C₁₋₄ alkoxy;
in certain embodiments, R¹ is selected from H, OH, NR^{1a}R^{1b}, methyl, ethyl, propyl, isopropyl or methoxy, wherein the methyl, ethyl, propyl, isopropyl or methoxy is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl or C₁₋₄ alkoxy;
in certain embodiments, R¹ is selected from H, OH, NR^{1a}R^{1b}, methyl, ethyl, propyl, isopropyl or methoxy, wherein the methyl, ethyl, propyl, isopropyl or methoxy is optionally substituted with 1 to 3 substituents selected from F, Cl, Br, I, OH, cyano, NH₂, methyl, ethyl or CF₃;
in certain embodiments, R¹ is selected from H, OH, NH₂, NH(CH₃), N(CH₃)₂, NH(CH₂CH₃), N(CH₂CH₃)₂, methyl, ethyl, propyl or isopropyl; in certain embodiments, R^{1a} and R^{1b} are each independently selected from H, OH, NH₂, C₁₋₄ alkyl, C₂₋₆ alkynyl or C₁₋₄ alkoxy, wherein the alkyl, alkynyl or alkoxy is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy or C₂₋₆ alkynyl;
in certain embodiments, R^{1a} and R^{1b} together with the atom to which they are attached form a 3- to 10-membered ring;
in certain embodiments, R^{1a} and R^{1b} are each independently selected from H, OH, NH₂, methyl, ethyl, propyl, isopropyl, methoxy, propargyl or propynyl, wherein the methyl, ethyl, propyl, isopropyl, methoxy, propargyl or propynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy or C₂₋₄ alkynyl;
in certain embodiments, R^{1a} and R^{1b} are each independently selected from H, OH, NH₂, methyl, ethyl, propyl, isopropyl, methoxy, propargyl or propynyl, wherein the methyl, ethyl, propyl, isopropyl, methoxy, propargyl or propynyl is optionally substituted with 1 to 3 substituents selected from F, Cl, Br, I, OH, cyano, NH₂, methyl, ethyl, methoxy, ethynyl or CF₃;
in certain embodiments, each R^{3a} or R⁴ is independently selected from H, OH, NH₂, C₁₋₆ alkyl, C₂₋₆ alkynyl, C₁₋₆ alkoxy, C₃₋₁₀ carbocycle or 3- to 10-membered heterocycle, wherein the alkyl, alkoxy, alkynyl, carbocycle or heterocycle is optionally substituted with 1 to 4 substituents selected from halogen, OH, cyano, NH(CH₃), N(CH₃)₂, NH(CH₂CH₃), N(CH₂CH₃)₂, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₂₋₆ alkynyl, C₃₋₆ cycloalkyloxy, C₃₋₆ carbocycle or 3- to 8-membered heterocycle, and the heterocycle contains 1 to 3 heteroatoms selected from O, S or N;
in certain embodiments, each R^{3a} or R⁴ is independently selected from H, OH, NH₂, methyl, ethyl, propyl, propargyl, propynyl, isopropyl, cyclopropyl, cyclobutyl, cyclopentyl, cyclohexyl, phenyl, azetidinyl, pyrrolidyl or piperidyl, wherein the methyl, ethyl, propyl, isopropyl, cyclopropyl, cyclobutyl, cyclopentyl, cyclohexyl, phenyl, azetidinyl, pyrrolidyl, piperidyl, propargyl or propynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ cycloalkyloxy, C₂₋₄ alkynyl, C₃₋₆ carbocycle or 3- to 6-membered heterocycle, and the heterocycle contains 1 to 3 heteroatoms selected from O, S or N;
in certain embodiments, R^{3a} is selected from H, OH, NH₂, methyl, ethyl, propyl, isopropyl, cyclopropyl, cyclobutyl, cyclopentyl, cyclohexyl, phenyl, azetidinyl, pyrrolidyl, piperidyl, propargyl or propynyl, wherein the methyl, ethyl, propyl, isopropyl, cyclopropyl, cyclobutyl, cyclopentyl, cyclohexyl, phenyl, azetidinyl, pyrrolidyl, piperidyl, propargyl or propynyl is optionally substituted with 1 to 3 substituents selected from F, Cl, Br, I, OH, cyano, NH₂, methyl, ethyl, cyclopropyl, cyclobutyl, cyclopentyl, cyclohexyl, ethynyl, methoxy or cyclopropyloxy; in certain embodiments, each R⁴ is independently selected from H, methyl, ethyl or cyclopropyl;
in certain embodiments, each R^{3b} or R⁵ is independently selected from H, halogen, cyano, NH₂, NO₂, OH, C₁₋₆ alkyl, C₂₋₄ alkenyl, C₂₋₄ alkynyl, C₁₋₆ alkoxy, C₃₋₁₀ carbocycle or 3- to 10-membered heterocycle, wherein the alkyl, alkoxy, carbocycle or heterocycle is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₃₋₆ carbocycle or 3- to 8-membered heterocycle, and the heterocycle contains 1 to 3 heteroatoms selected from O, S or N;
in certain embodiments, R^{3b} or R⁵ is each independently selected from H, halogen, cyano, NH₂, NO₂, OH, C₁₋₄ alkyl, C₂₋₄ alkenyl, C₂₋₄ alkynyl, C₁₋₄ alkoxy, C₃₋₆ carbocycle or 3- to 6-membered heterocycle, wherein the alkyl, alkoxy, carbocycle or heterocycle is optionally substituted with 1 to 4 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
in certain embodiments, each R^{3b} or R⁵ is independently selected from H, F, Cl, Br, I, cyano, NH₂, OH, methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl, piperidyl or ethynyl, wherein the methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl, piperidyl or ethynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
in certain embodiments, each R^{3b} or R⁵ is independently selected from H, F, Cl, Br, I, cyano, NH₂, OH, methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl or piperidyl, wherein the methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl or piperidyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
in certain embodiments, each R^{3b} is independently selected from H, F, Cl, Br, I, OH, CN, methoxy, methyl, ethyl or cyclopropyl;
in certain embodiments, each R^{3b} is independently selected from H, F, Cl, Br, I, OH, methyl, ethyl or cyclopropyl;
in certain embodiments, each R⁵ is independently selected from H, F, Cl, Br, I, cyano, NH₂, OH, methyl, ethyl, propyl, isopropyl, methoxy, ethoxy, propoxy, isopropoxy, cyclopropyl or ethynyl, wherein the methyl, ethyl, propyl, isopropyl, methoxy, ethoxy, propoxy, isopropoxy, cyclopropyl or ethynyl is optionally substituted with 1 to 3 substituents selected from F, Cl, Br, I, OH, cyano, NH₂, methyl, ethyl or cyclopropyl;
in certain embodiments, each R⁵ is independently selected from H, F, Cl, Br, I, cyano, NH₂, OH, methyl, ethyl, propyl, isopropyl, methoxy, ethoxy, propoxy, isopropoxy or cyclopropyl, wherein the methyl, ethyl, propyl, isopropyl, methoxy, ethoxy, propoxy, isopropoxy or cyclopropyl is optionally substituted with 1 to 3 substituents selected from F, Cl, Br, I, OH, cyano, NH₂, methyl, ethyl or cyclopropyl;
in certain embodiments, t1 is selected from 1, 2, 3, 4 or 5; in certain embodiments, t1 is selected from 1, 2 or 3; in certain embodiments, t1 is selected from 1;
in certain embodiments, t2 is selected from 0, 1, 2, 3, 4 or 5; in certain embodiments, t2 is selected from 0, 1, 2 or 3; in certain embodiments, t2 is selected from 0;
in certain embodiments, m is selected from 0, 1, 2, 3 or 4; in certain embodiments, m is selected from 0, 1, 2 or 3;
in certain embodiments, m is selected from 0 or 1;
in certain embodiments, n is selected from 0, 1 or 2; in certain embodiments, n is selected from 0 or 1;
in certain embodiments, B is selected from one of the structural fragments shown in Table B-1; 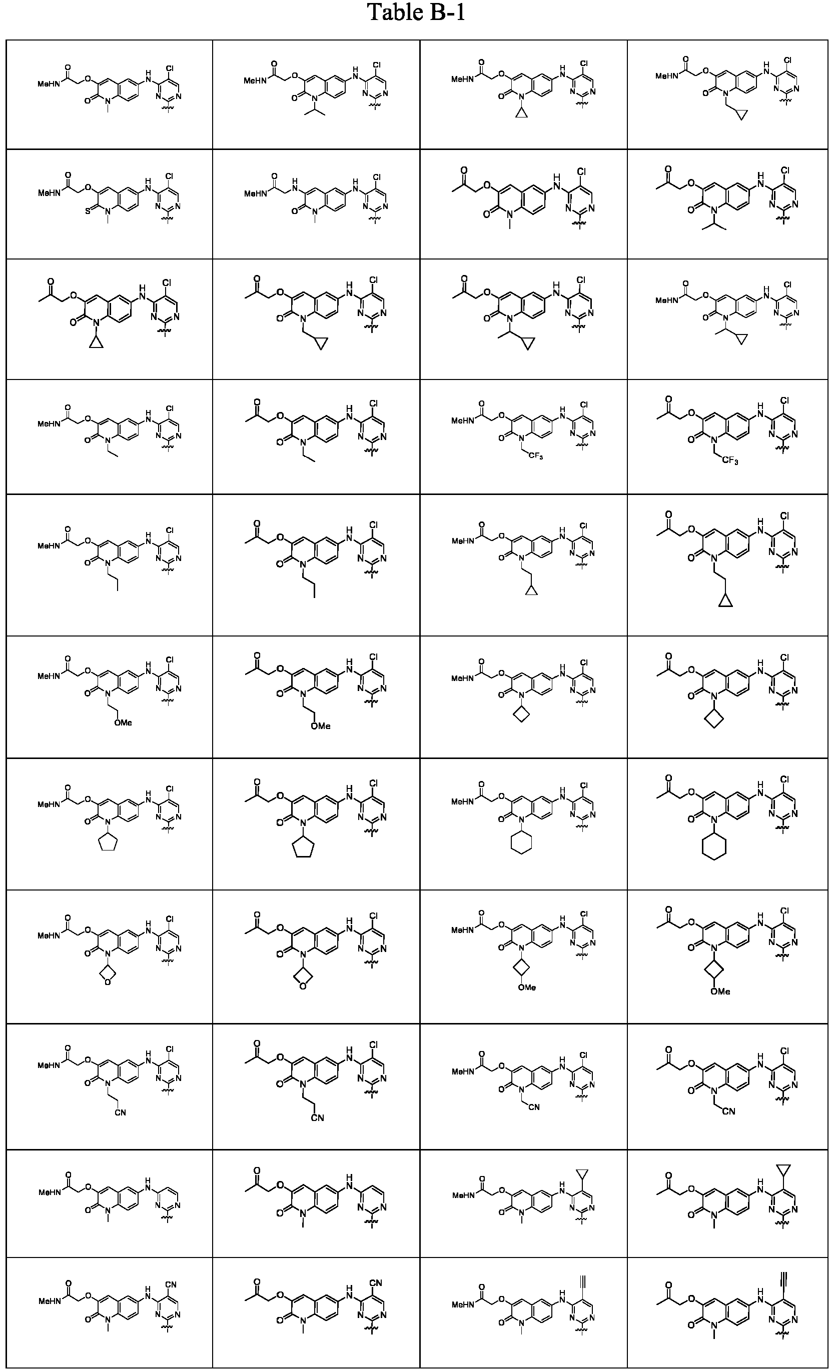 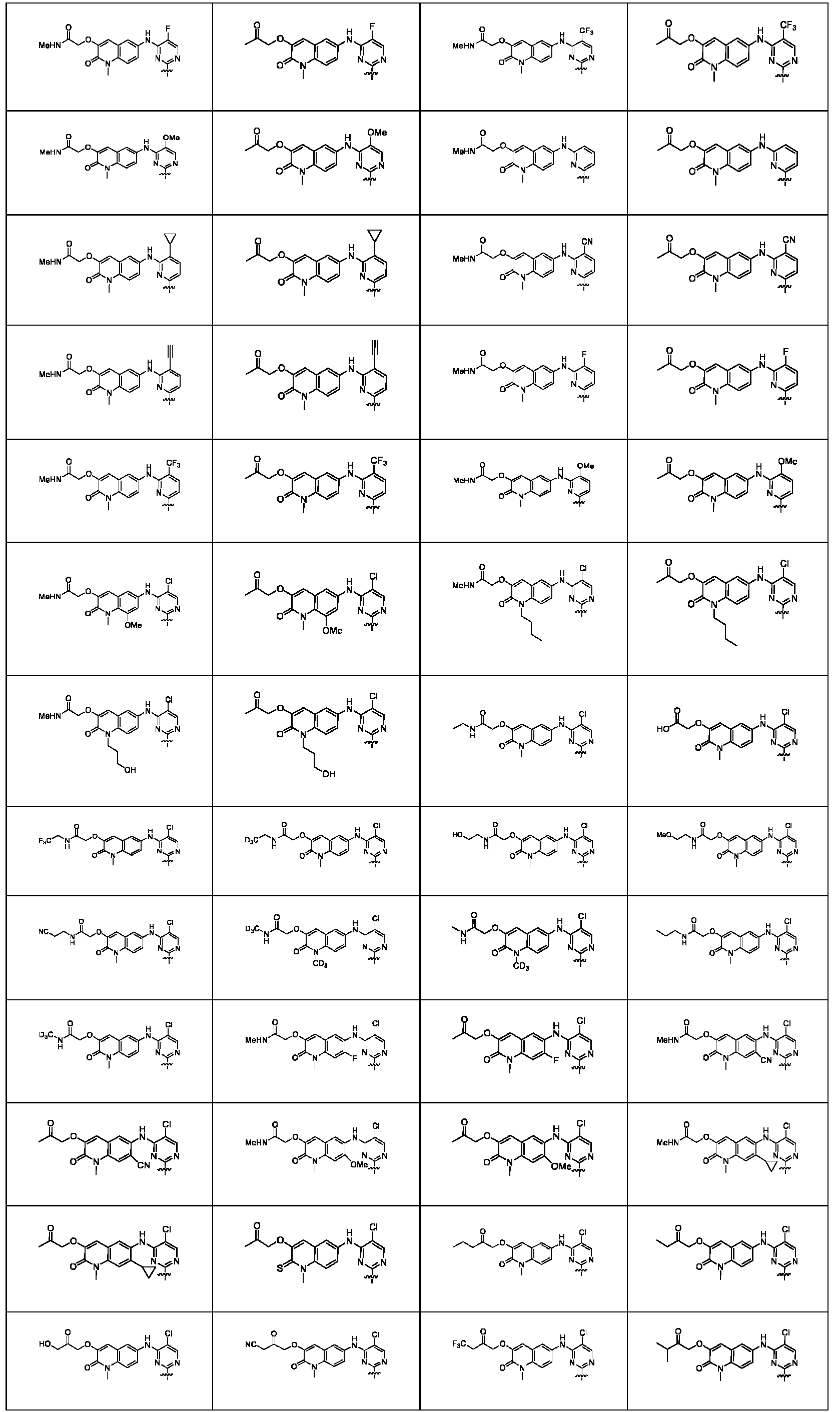 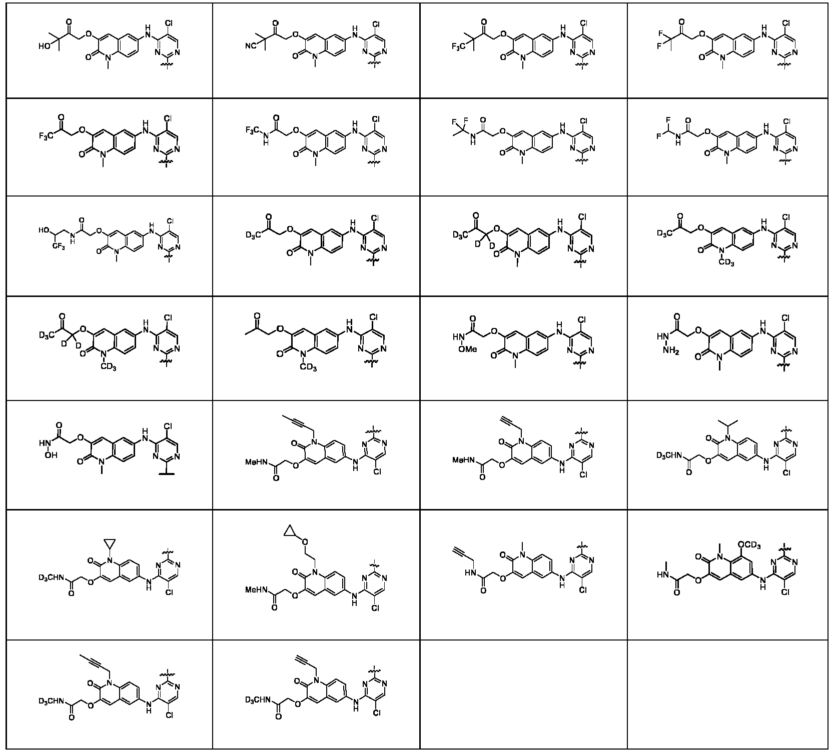
in some embodiments, B is selected from 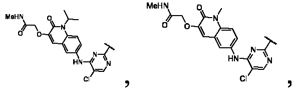 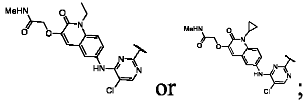
in certain embodiments, K is selected from 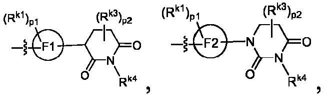 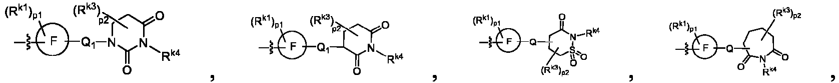 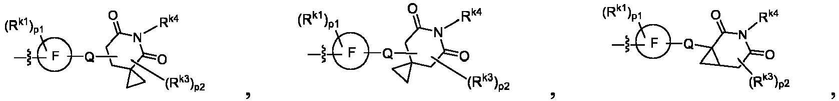 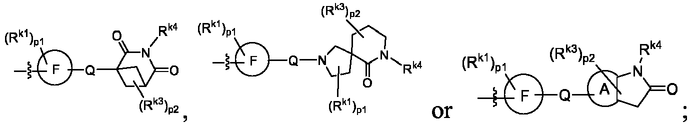
in certain embodiments, each F1 is independently selected from C₃₋₇ monocyclic carbocycle, C₄₋₁₄ fused ring carbocycle, C₅₋₁₂ spiro ring carbocycle, C₅₋₁₀ bridged ring carbocycle, C₆₋₁₄ aryl, a 6- to 7-membered non-aromatic mono-heterocyclic ring, a 5- to 14-membered bridged-heterocyclic ring, 5- to 6-membered heteroaryl, a 11- to 20-membered fused-heterocyclic ring, phthalazin-1(2*H*)-onyl, benzo[d][1,2,3]triazin-4(3H)-onyl, benzothienyl, 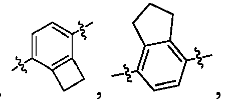 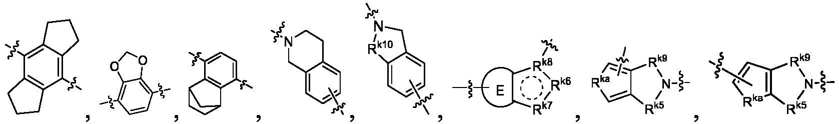 or 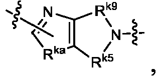 wherein the mono-heterocyclic ring, fused-heterocyclic ring, spiro-heterocyclic ring, bridged-heterocyclic ring or heteroaromatic ring contains 1 to 4 heteroatoms selected from O, S or N, provided that when F1 is selected from 6-membered heteroaryl, the right connection site on the ring is a carbon atom;
in certain embodiments, 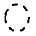 represents a ring selected from an aromatic ring or a non-aromatic ring;
in certain embodiments, each F1 is independently selected from cyclobutyl, cyclopentyl, cyclohexyl, piperidyl, piperazinyl, phenyl, naphthyl, pyridyl, pyrazinyl, pyridazinyl, pyridazinonyl, pyrimidyl, phthalazin-1(2*H*)-onyl, benzo[d][1,2,3]triazin-4(*3*H)-onyl, thienyl, benzothienyl, triazolyl, 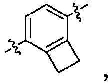 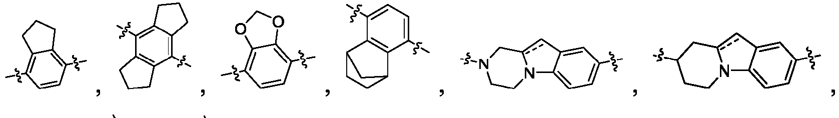 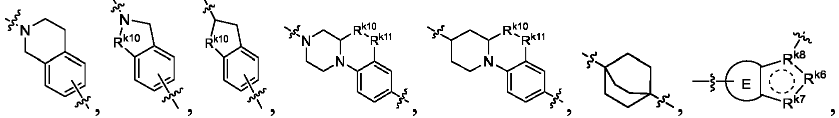 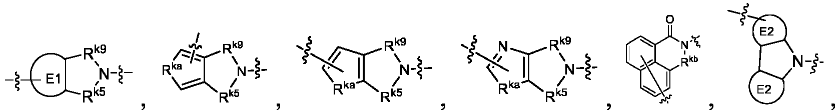 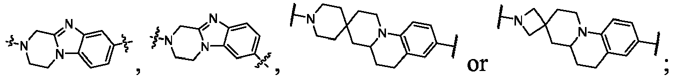
-̅ -̅ -̅ -̅ -̅ is selected from a single bond or a double bond;
in certain embodiments, each F1 is independently selected from cyclohexyl, piperidyl, piperazinyl, phenyl, naphthyl, pyridyl, pyrimidyl, pyrazinyl, pyridazinyl, pyridazinonyl, phthalazin-1(2*H*)-onyl, benzo[d][1,2,3]triazin-4(*3*H)-onyl, thienyl, triazolyl, 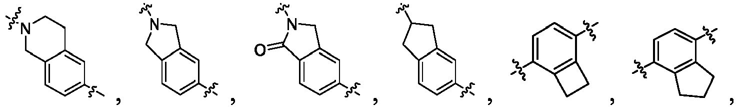 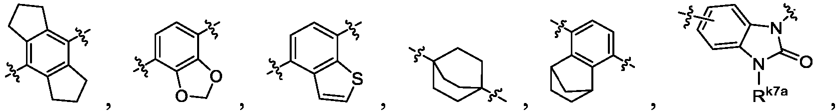 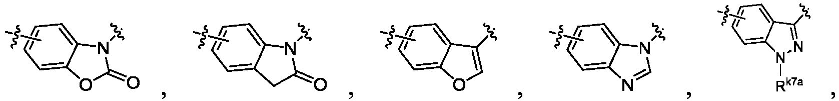 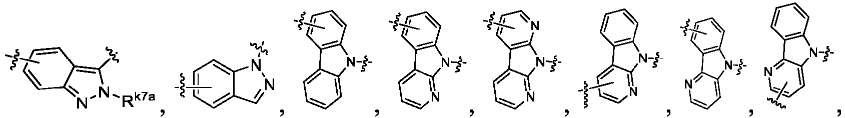 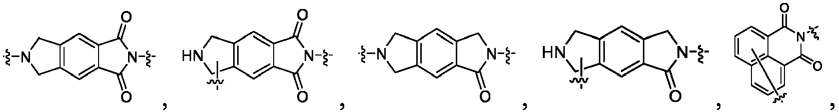 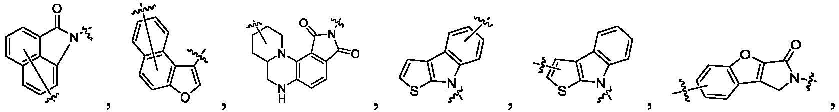 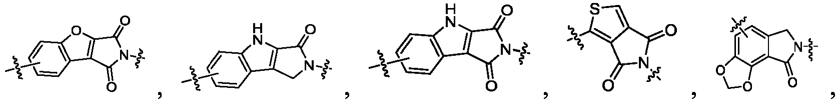 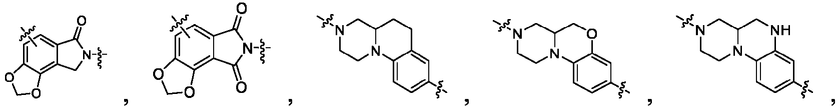 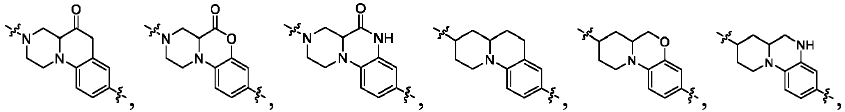 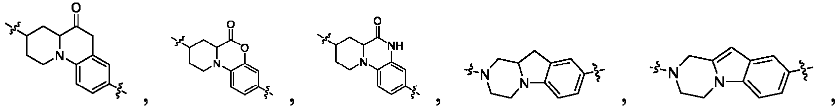 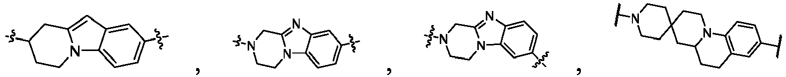 or 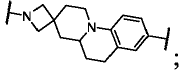
in certain embodiments, F1 is selected from phenyl, 5- to 6-membered heteroaryl or 13- to 14-membered fused heterotricyclic ring;
in certain embodiments, F1 is selected from 13- to 14-membered fused heterotricyclic ring;
in certain embodiments, F1 is selected from phenyl, pyrrolyl, pyrazolyl, triazolyl, pyridyl, pyridazinyl, pyrazinyl, thienyl, thiazolyl, triazinyl, 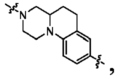 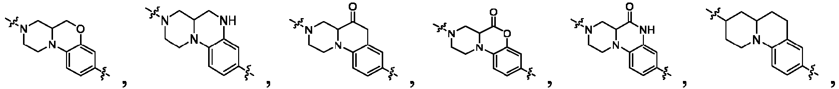 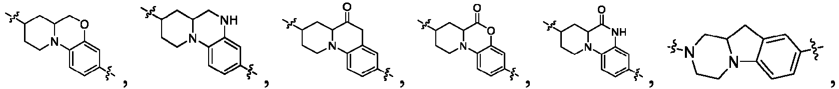 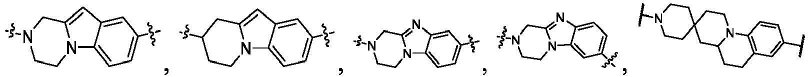 or 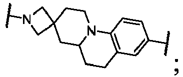
in certain embodiments, F1 is selected from 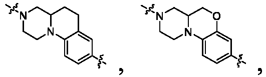 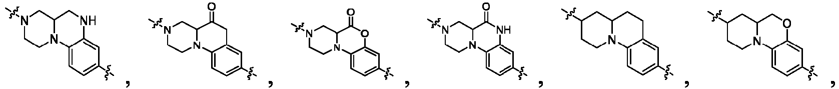 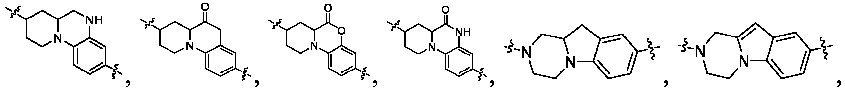 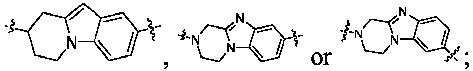
in certain embodiments, F1 is selected from phenyl;
in certain embodiments, F1 is selected from 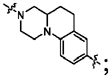
in certain embodiments, each F2 is independently selected from C₃₋₇ monocyclic non-aromatic carbocycle, C₄₋₁₀ fused ring non-aromatic carbocycle, C₅₋₁₂ spiro ring carbocycle, C₅₋₁₀ bridged ring carbocycle, C₁₂₋₁₄ aryl, a 4- to 7-membered non-aromatic mono-heterocyclic ring, a 5- to 14-membered spiro-heterocyclic ring, a 5- to 14-membered bridged-heterocyclic ring, a 11- to 20-membered fused-heterocyclic ring or 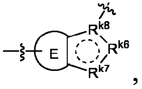 wherein the mono-heterocyclic ring, fused-heterocyclic ring, spiro-heterocyclic ring, bridged-heterocyclic ring or heteroaromatic ring contains 1 to 4 heteroatoms selected from O, S or N; in certain embodiments, each F2 is independently selected from 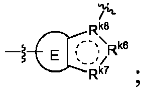 in certain embodiments, each F2 is independently selected from 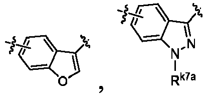 or 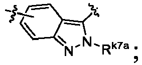
in certain embodiments, each E1 is independently selected from an 8- to 15-membered fused-heterocyclic ring; in certain embodiments, each E1 is independently selected from 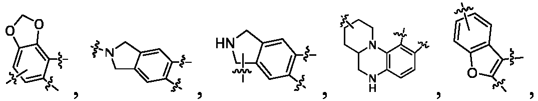 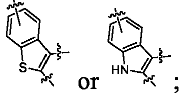
in certain embodiments, each E2 is independently selected from a benzene ring or a 5- to 6-membered heteroaromatic ring;
in certain embodiments, each E2 is independently selected from a benzene ring, a pyridine ring, a pyrimidine ring, a thiazole ring, a thiophene ring or a furan ring;
in certain embodiments, each F is independently selected from C₃₋₇ monocyclic carbocycle, C₄₋₂₀ fused ring carbocycle, C₅₋₂₀ spiro ring carbocycle, C₅₋₂₀ bridged ring carbocycle, a 4- to 7-membered mono-heterocyclic ring, a 4- to 20-membered fused-heterocyclic ring, a 5- to 20-membered spiro-heterocyclic ring or a 5- to 20-membered bridged-heterocyclic ring, wherein the mono-heterocyclic ring, fused-heterocyclic ring, spiro-heterocyclic ring, bridged-heterocyclic ring or heteroaromatic ring contains 1 to 4 heteroatoms selected from O, S or N;
in certain embodiments, each F is independently selected from cyclobutyl, cyclopentyl, cyclohexyl, azetidinyl, pyrrolidyl, piperidyl, piperazinyl, pyridyl, pyrimidyl, pyridazinyl, pyrazinyl, triazinyl, pyrrolyl, imidazolyl, triazolyl, oxazolyl, furyl, thienyl, thiazolyl, 2-pyridonyl, pyrazolyl, phenyl, naphthyl, anthryl, phenanthryl, benzothiazolyl, indolyl, 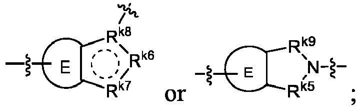
in certain embodiments, each F is independently selected from cyclobutyl, cyclopentyl, cyclohexyl, azetidinyl, pyrrolidyl, piperidyl, piperazinyl, pyridyl, pyrimidyl, pyridazinyl, pyrazinyl, triazinyl, pyrrolyl, imidazolyl, triazolyl, oxazolyl, furyl, thienyl, thiazolyl, 2-pyridonyl, pyrazolyl, phenyl, naphthyl, anthryl, phenanthryl, benzothiazolyl, indolyl, 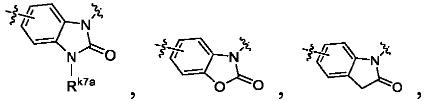 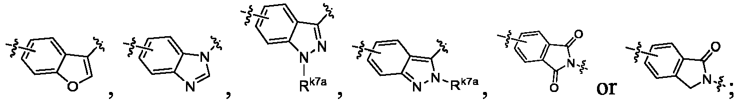
in certain embodiments, each E is independently selected from C₅₋₁₂ carbocycle, 5- to 12-membered heterocycle or a 5- to 12-membered heteroaromatic ring, wherein the heterocycle or heteroaromatic ring contains 1 to 4 heteroatoms selected from O, S or N;
in certain embodiments, each E is independently selected from C₃₋₁₀ carbocycle, a benzene ring, 4- to 12-membered heterocycle or a 5- to 12-membered heteroaromatic ring, wherein the heterocycle or heteroaromatic ring contains 1 to 4 (such as 1, 2, 3 or 4) heteroatoms selected from O, S or N;
in certain embodiments, each E is independently selected from a benzene ring, a naphthalene ring or a 5- to 6-membered heteroaromatic ring, wherein the heterocycle or heteroaromatic ring contains 1 to 3 (such as 1, 2 or 3) heteroatoms selected from O, S or N;
in certain embodiments, each E is independently selected from a benzene ring, a naphthalene ring, a pyridine ring, a pyrimidine ring, a pyrazine ring, a pyrimidine ring, a pyrrole ring, a pyrazole ring, an imidazole ring, a thiazole ring, a furan ring, a thiophene ring, an oxazole ring, an indoline ring, an isoindoline ring, a 1,2,3,4-tetrahydroquinoline ring or a 1,2,3,4-tetrahydroisoquinoline ring;
in certain embodiments, each E is independently selected from a benzene ring, a naphthalene ring, a pyridine ring, a pyrimidine ring, a pyrazine ring, a pyrimidine ring, a pyrrole ring, a pyrazole ring, an imidazole ring, a thiazole ring, a furan ring, a thiophene ring or an oxazole ring;
in certain embodiments, each E is independently selected from a benzene ring, a naphthalene ring, a pyridine ring, a pyrimidine ring, a pyrazine ring or a pyrimidine ring;
in certain embodiments, each E is independently selected from a benzene ring, a naphthalene ring or a pyridine ring;
in certain embodiments, A is selected from C₃₋₁₀ carbocycle, a C₆₋₁₀ aromatic ring, 3- to 10-membered heterocycle or a 5- to 10-membered heteroaromatic ring, wherein the heterocycle or heteroaromatic ring contains 1 to 4 (such as 1, 2, 3 or 4) heteroatoms selected from O, S or N;
in certain embodiments, A is selected from C₃₋₈ carbocycle, a benzene ring, a naphthalene ring, 4- to 7-membered heterocycle or a 5- to 6-membered heteroaromatic ring, wherein the heterocycle or heteroaromatic ring contains 1 to 4 (such as 1, 2, 3 or 4) heteroatoms selected from O, S or N;
in certain embodiments, A is selected from a benzene ring, a naphthalene ring, a pyridine ring, a pyrimidine ring, a pyrazine ring, a pyrimidine ring, a pyrrole ring, a pyrazole ring, an imidazole ring, a thiazole ring, a furan ring, a thiophene ring or an oxazole ring;
in certain embodiments, A is selected from a benzene ring, a naphthalene ring or a pyridine ring;
in certain embodiments, each Q is independently selected from a bond, -O-, - S-, -CH₂-, -NR^{q}-, -CO-, -NR^{q}CO-, -CONR^{q}- or 3- to 12-membered heterocycle, wherein the heterocycle is optionally substituted with 1 to 4 (such as 1, 2, 3 or 4) substituents selected from F, Cl, Br, I, OH, =O, NH₂, CN, COOH, CONH₂, C₁₋₄ alkyl or C₁₋₄ alkoxy, and the heterocycle contains 1 to 4 (such as 1, 2, 3 or 4) heteroatoms selected from O, S or N;
in certain embodiments, each Q is independently selected from a bond, -O-, - S-, -CH₂-, -NR^{q}-, -CO-, -NR^{q}CO-, -CONR^{q}- or 4- to 7-membered heterocycle, wherein the heterocycle is optionally substituted with 1 to 4 (such as 1, 2, 3 or 4) substituents selected from F, Cl, Br, I, OH, =O, NH₂, CN, COOH, CONH₂, C₁₋₄ alkyl or C₁₋₄ alkoxy, and the heterocycle contains 1 to 4 (such as 1, 2, 3 or 4) heteroatoms selected from O, S or N;
in certain embodiments, each Q is independently selected from a bond, CH₂, NH, N(CH₃), O, S, C(=O), NHC(=O), C(=O)NH, N(CH₃)C(=O), C(=O)N(CH₃), 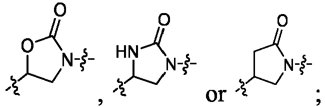
in certain embodiments, Q1 is selected from -O-, -S-, -CH₂-, NH, N(CH₃) or - CO-;
in certain embodiments, R^{q} is selected from H or C₁₋₆ alkyl; in certain embodiments, R^{q} is selected from H or C₁₋₄ alkyl;
in certain embodiments, R^{q} is selected from H, methyl or ethyl;
in certain embodiments, each R^{k1} is independently selected from H, D, F, Cl, Br, I, OH, =O, NH₂, CN, COOH, CONH₂, C₁₋₆ alkyl, C₁₋₆ alkoxy, C₃₋₆ cycloalkyl or R^{k7a}, wherein the alkyl, alkoxy or cycloalkyl is optionally substituted with 1 to 4 (such as 1, 2, 3 or 4) substituents selected from D, F, Cl, Br, I, OH, =O, NH₂, CN, COOH, CONH₂, C₁₋₄ alkyl, C₁₋₄ alkoxy or C₃₋₆ cycloalkyl;
in certain embodiments, R^{k1} is selected from R^{k7a};
in certain embodiments, each R^{k3} is independently selected from H, D, F, Cl, Br, I, OH, =O, NH₂, CN, COOH, CONH₂, C₁₋₆ alkyl, C₁₋₆ alkoxy, C₃₋₈ cycloalkyl or 3- to 8-membered heterocyclyl, wherein the alkyl, alkoxy, cycloalkyl or heterocyclyl is optionally substituted with 1 to 4 (such as 1, 2, 3 or 4) substituents selected from D, F, Cl, Br, I, OH, =O, NH₂, CN, COOH, CONH₂, C₁₋₄ alkyl or C₁₋₄ alkoxy, and the heterocyclyl contains 1 to 4 (such as 1, 2, 3 or 4) heteroatoms selected from O, S or N;
in certain embodiments, R^{k1} and R^{k3} are each independently selected from H, D, F, Cl, Br, I, OH, =O, NH₂, CF₃, CN, COOH, CONH₂, C₁₋₄ alkyl, C₁₋₄ alkoxy or R^{k7a}, wherein the alkyl or alkoxy is optionally substituted with 1 to 4 substituents selected from D, F, Cl, Br, I, OH or NH₂;
R^{k1} and R^{k3} are each independently selected from H, D, F, Cl, Br, I, OH, =O, NH₂, CF₃, CN, COOH, CONH₂, methyl, ethyl, isopropyl, methoxy, ethoxy, isopropoxy, ethynyl, cyclopropyl or azetidinyl, wherein the methyl, ethyl, isopropyl, methoxy, ethoxy, isopropoxy, ethynyl, cyclopropyl or azetidinyl is optionally substituted with 1 to 4 substituents selected from D, F, Cl, Br, I, OH, NH₂, CN, cyclopropyl or methoxy;
in certain embodiments, R^{k1} and R^{k3} are each independently selected from H, D, F, Cl, Br, I, OH, =O, NH₂, CF₃, CN, COOH, CONH₂, C₁₋₄ alkyl or C₁₋₄ alkoxy, wherein the alkyl or alkoxy is optionally substituted with 1 to 4 (such as 1, 2, 3 or 4) substituents selected from D, F, Cl, Br, I, OH or NH₂;
in certain embodiments, R^{k1} and R^{k3} are each independently selected from H, D, F, Cl, Br, I, OH, =O, NH₂, CF₃, CN, COOH, CONH₂, methyl, ethyl, isopropyl, methoxy, ethoxy or isopropoxy, wherein the methyl, ethyl, isopropyl, methoxy, ethoxy or isopropoxy is optionally substituted with 1 to 4 (such as 1, 2, 3 or 4) substituents selected from D, F, Cl, Br, I, OH or NH₂;
in certain embodiments, each R^{k1} is independently selected from H, D, F, Cl, Br, I, OH, =O, NH₂, CF₃, CN, COOH, CONH₂, methyl, ethyl, methoxy or ethoxy;
in certain embodiments, each R^{k1} is independently selected from H, D, F, Cl or Br;
in certain embodiments, two R^{k3} together with the carbon atom or ring backbone to which they are directly attached form C₃₋₈ carbocycle or 3- to 8-membered heterocycle, or two R^{k1} together with the carbon atom or ring backbone to which they are directly attached form C₃₋₈ carbocycle or 3- to 8-membered heterocycle, wherein the carbocycle or heterocycle is optionally substituted with 1 to 4 (such as 1, 2, 3 or 4) substituents selected from F, Cl, Br, I, OH, =O, NH₂, CN, COOH, CONH₂, C₁₋₄ alkyl or C₁₋₄ alkoxy, and the heterocycle contains 1 to 4 (such as 1, 2, 3 or 4) heteroatoms selected from O, S or N;
in certain embodiments, two R^{k3} together with the carbon atom or ring backbone to which they are directly attached form C₃₋₇ carbocycle or 3- to 7-membered heterocycle, or two R^{k1} together with the carbon atom or ring backbone to which they are directly attached form C₃₋₇ carbocycle or 3- to 7-membered heterocycle, wherein the carbocycle or heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
in certain embodiments, two R^{k3} together with the carbon atom or ring backbone to which they are directly attached form C₃₋₆ carbocycle or 3- to 7-membered heterocycle, or two R^{k1} together with the carbon atom or ring backbone to which they are directly attached form C₃₋₆ carbocycle or 3- to 7-membered heterocycle, wherein the carbocycle or heterocycle is optionally substituted with 1 to 4 (such as 1, 2, 3 or 4) substituents selected from F, Cl, Br, I, OH, =O, NH₂, CN, COOH, CONH₂, C₁₋₄ alkyl or C₁₋₄ alkoxy, and the heterocycle contains 1 to 4 (such as 1, 2, 3 or 4) heteroatoms selected from O, S or N;
in certain embodiments, each R^{k4} is independently selected from H, C₁₋₆ alkyl, C₃₋₈ cycloalkyl or 3- to 8-membered heterocyclyl, wherein the alkyl, cycloalkyl or heterocyclyl is optionally substituted with 1 to 4 (such as 1, 2, 3 or 4) substituents selected from F, Cl, Br, I, OH, =O, NH₂, CN, COOH, CONH₂, C₁₋₄ alkyl or C₁₋₄ alkoxy, and the heterocyclyl contains 1 to 4 (such as 1, 2, 3 or 4) heteroatoms selected from O, S or N;
in certain embodiments, each R^{k4} is independently selected from H or C₁₋₄ alkyl;
in certain embodiments, each R^{k4} is independently selected from H, methyl, ethyl or propyl;
in certain embodiments, each R^{k5} is independently selected from 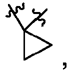 C(CH₃)₂, CO, CH₂, SO₂, 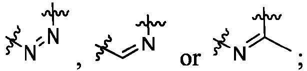 in certain embodiments, each R^{k5} is independently selected from CO, CH₂, SO₂ or 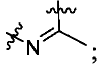
in certain embodiments, each R^{k6} is independently selected from CO, CH, SO, SO₂, CH₂, N or NR^{k7a};
in certain embodiments, each R^{k7} is independently selected from 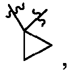 C(CH₃)₂, CO, CH, N, CH₂, O, S or NR^{k7a};
in certain embodiments, each R^{k7} is independently selected from 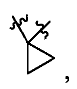 C(CH₃)₂, CO, CH, N, CH₂, O, S, N(CH₃), N(CH₂CH₃), N(cyclopropyl) or NH;
in certain embodiments, each R^{k7} is independently selected from CO, CH, N, CH₂, O, S, N(CH₃) or NH;
in certain embodiments, each R^{k7} is independently selected from CH₂, O, N(CH₃) or NH;
in certain embodiments, R^{k7a} is selected from H, C₁₋₄ alkyl, C₂₋₆ alkenyl, C₂₋₆ alkynyl, C₃₋₆ cycloalkyl or 3- to 6-membered heterocycloalkyl, wherein the alkyl, cycloalkyl or heterocycloalkyl is optionally substituted with 1 to 4 substituents selected from F, Cl, Br, I, OH, NH₂, CN, CF₃, C₁₋₆ alkyl, C₁₋₆ alkoxy, C₂₋₆ alkenyl, C₂₋₆ alkynyl or C₃₋₆ cycloalkyl;
in certain embodiments, R^{k7a} is selected from H, C₁₋₄ alkyl, C₂₋₄ alkenyl, C₂₋₄ alkynyl, C₃₋₆ cycloalkyl or 3- to 6-membered heterocycloalkyl, wherein the alkyl, cycloalkyl or heterocycloalkyl is optionally substituted with 1 to 4 substituents selected from F, Cl, Br, I, OH, NH₂, CN, CF₃, C₁₋₄ alkyl, C₁₋₄ alkoxy, C₂₋₄ alkenyl, C₂₋₄ alkynyl or C₃₋₆ cycloalkyl;
in certain embodiments, R^{k7a} is selected from H, C₁₋₄ alkyl or C₃₋₆ cycloalkyl, wherein the alkyl or cycloalkyl is optionally substituted with 1 to 4 substituents selected from F, Cl, Br, I, OH, CN, C₁₋₄ alkyl, C₁₋₄ alkoxy or C₃₋₆ cycloalkyl;
in certain embodiments, R^{k7a} is selected from H, methyl, ethyl, propyl, isopropyl, ethenyl, propenyl, allyl, ethynyl, propynyl, propargyl, cyclopropyl, cyclobutyl, cyclopentyl, cyclohexyl, azetidinyl, pyrrolidyl, piperidyl, oxacyclobutyl, tetrahydrofuryl or tetrahydropyranyl, wherein the methyl, ethyl, propyl, isopropyl, cyclopropyl, cyclobutyl, cyclopentyl, cyclohexyl, azetidinyl, pyrrolidyl, piperidyl, oxacyclobutyl, tetrahydrofuryl or tetrahydropyranyl is optionally substituted with 1 to 4 substituents selected from F, Cl, Br, I, OH, CN, CF₃, C₁₋₄ alkyl, C₁₋₄ alkoxy, ethenyl, propenyl, allyl, ethynyl, propynyl, propargyl or C₃₋₆ cycloalkyl;
in certain embodiments, R^{k7a} is selected from H, methyl, ethyl, isopropyl, cyclopropyl, oxacyclobutyl, tetrahydropyranyl, -CH₂CF₃, -CH(CH₃)CF₃, - CH(CH₃)-cyclopropyl, -CH₂-cyclopropyl, -CH₂-ethenyl, -CH₂-ethynyl or - CH₂CH₂-methoxy;
in certain embodiments, R^{k7a} is selected from H, CF₃, methyl, ethyl, cyclopropyl or -CH₂-cyclopropyl;
in certain embodiments, R^{k7a} is selected from H, CH₃, CH₂CH₃ or cyclopropyl;
in certain embodiments, each R^{k8} is independently selected from C, N or CH;
in certain embodiments, each R^{k9} is independently selected from a bond, 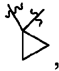 C(CH₃)₂, CO, CH₂, CH₂CH₂ or SO₂;
in certain embodiments, each R^{k9} is independently selected from CO, SO₂ or CH₂;
in certain embodiments, R^{ka} is selected from O, S or NH;
in certain embodiments, R^{kb} is selected from a bond or CO;
in certain embodiments, R^{k10} is selected from CH₂ or CO;
in certain embodiments, R^{k11} is selected from NH, O or CH₂;
in certain embodiments, each p1 or p2 is independently selected from 0, 1, 2, 3, 4 or 5;
in certain embodiments, each p1 or p2 is independently selected from 0, 1, 2 or 3;
in certain embodiments, p1 is selected from 0, 1 or 2;
in certain embodiments, K is selected from one of the structural fragments shown in Table K-1;
in certain embodiments, K is selected from one of the structural fragments shown in Table K-2;
in certain embodiments, the compound is substituted with 1-30 D (such as 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 D);
in certain embodiments, the compound is substituted with 1-20 D (such as 1, 2, 3, 4, 5, 6, 7, 8, 9, 10, 11, 12, 13, 14, 15, 16, 17, 18, 19 or 20 D);
in certain embodiments, the compound is substituted with 1-15 D (such as 1, 2, 3, 4, 5, 6, 7, 8, 9, 10, 11, 12, 13, 14 or 15 D);

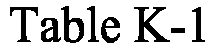

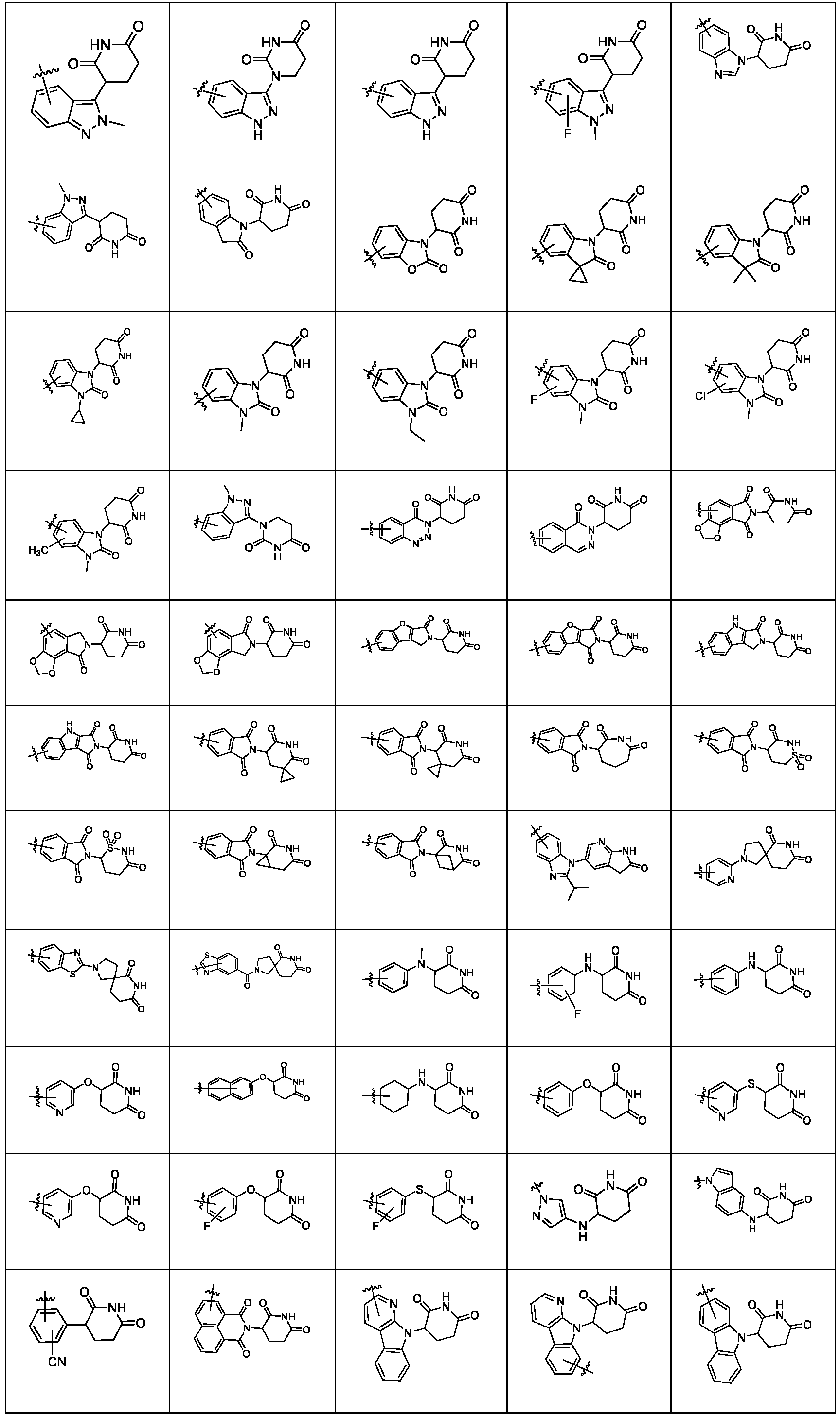

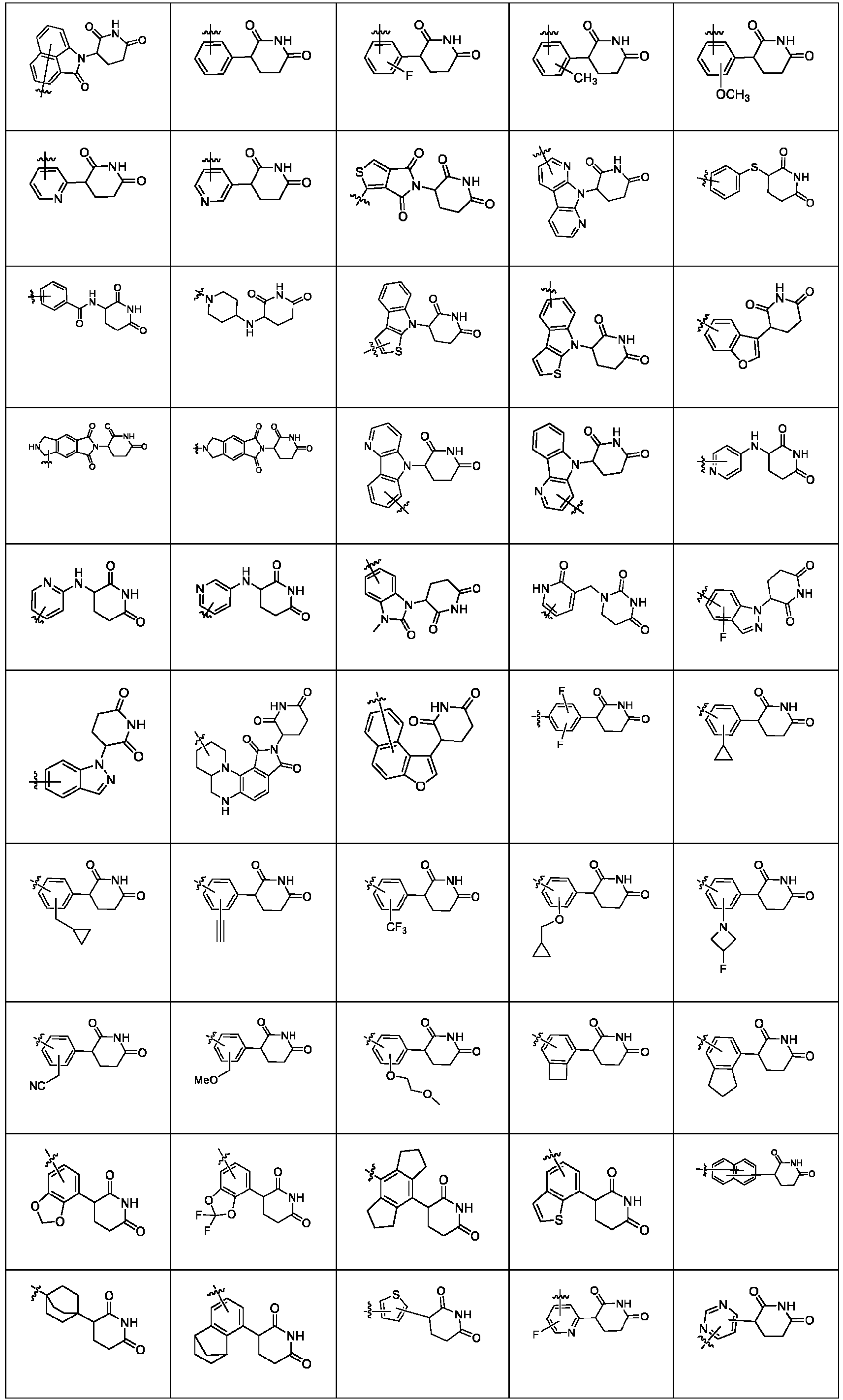

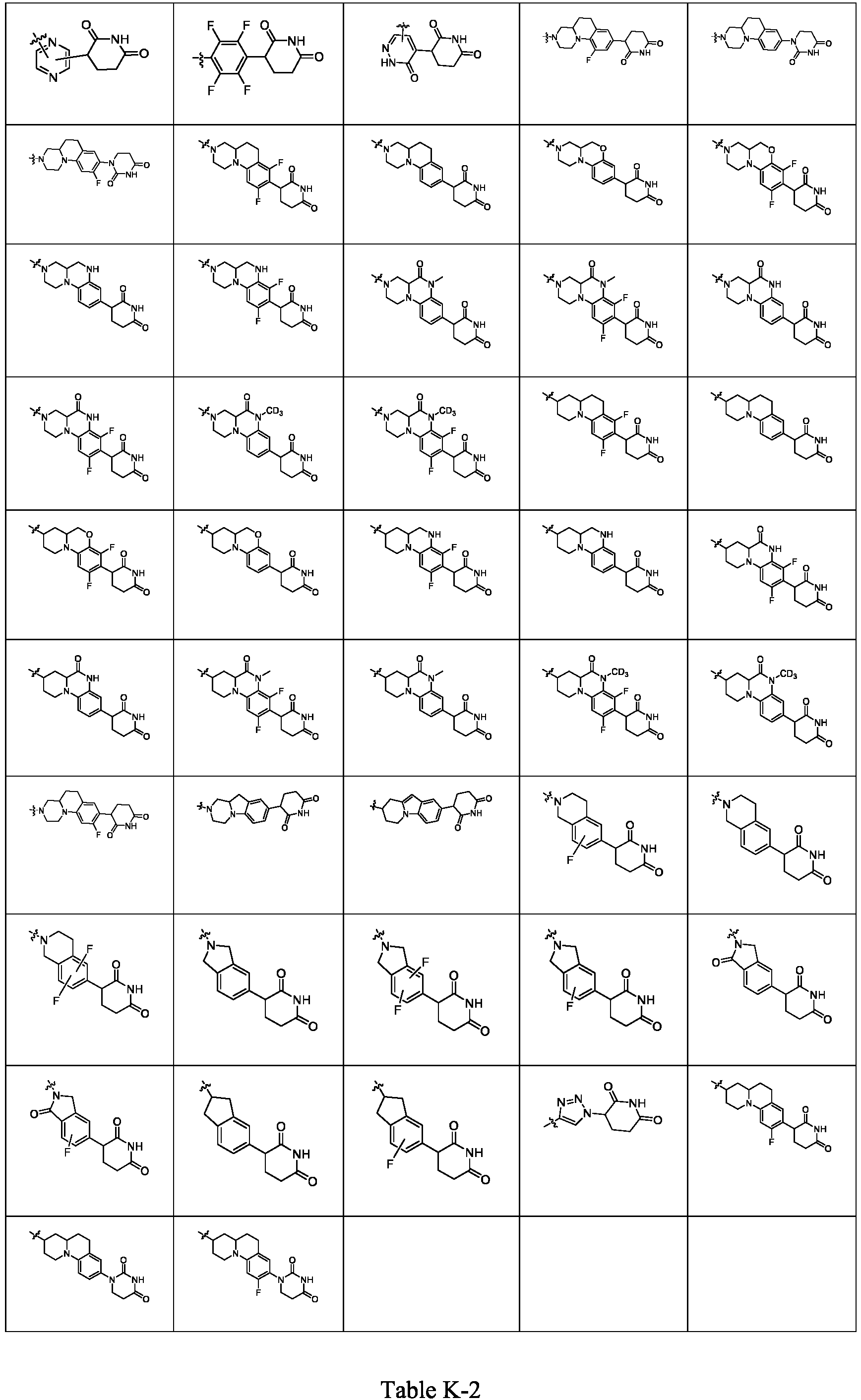

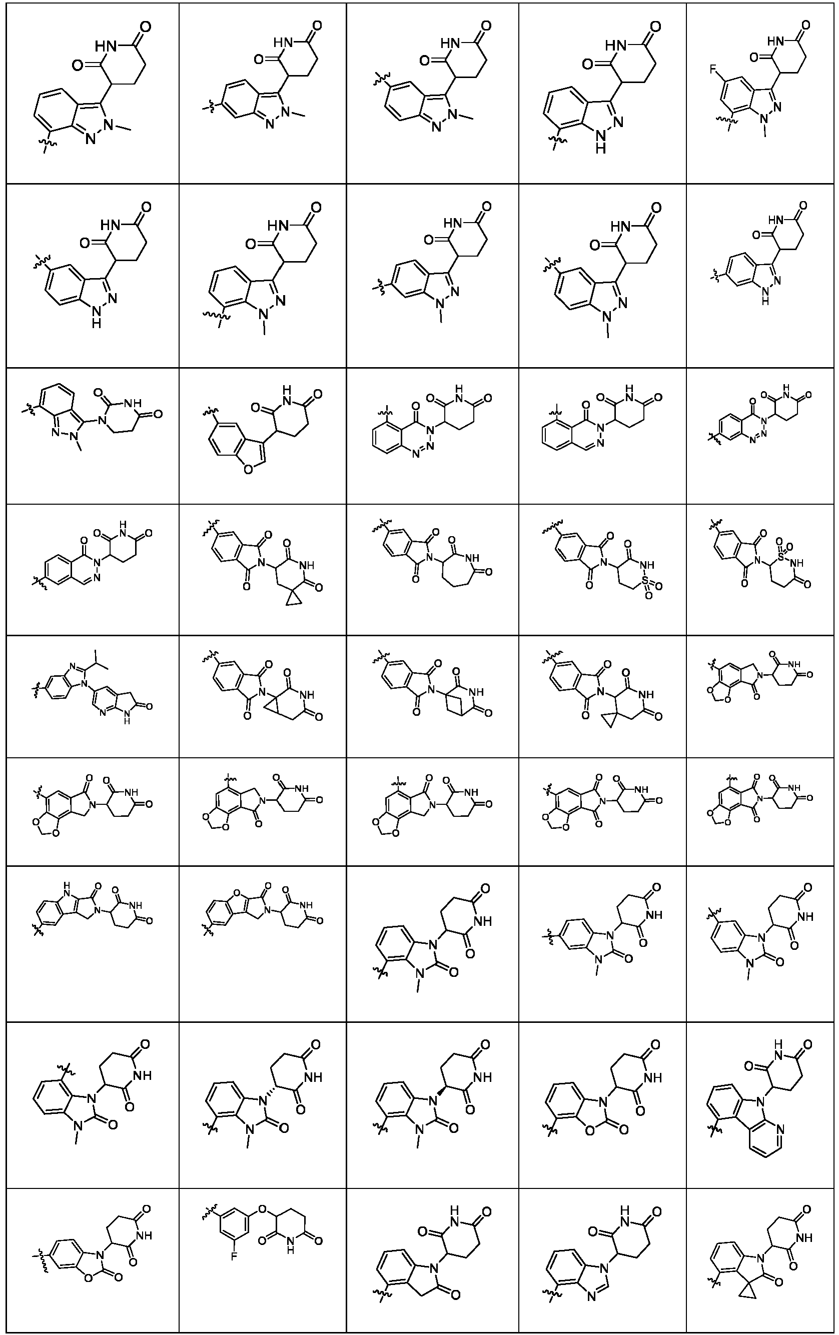

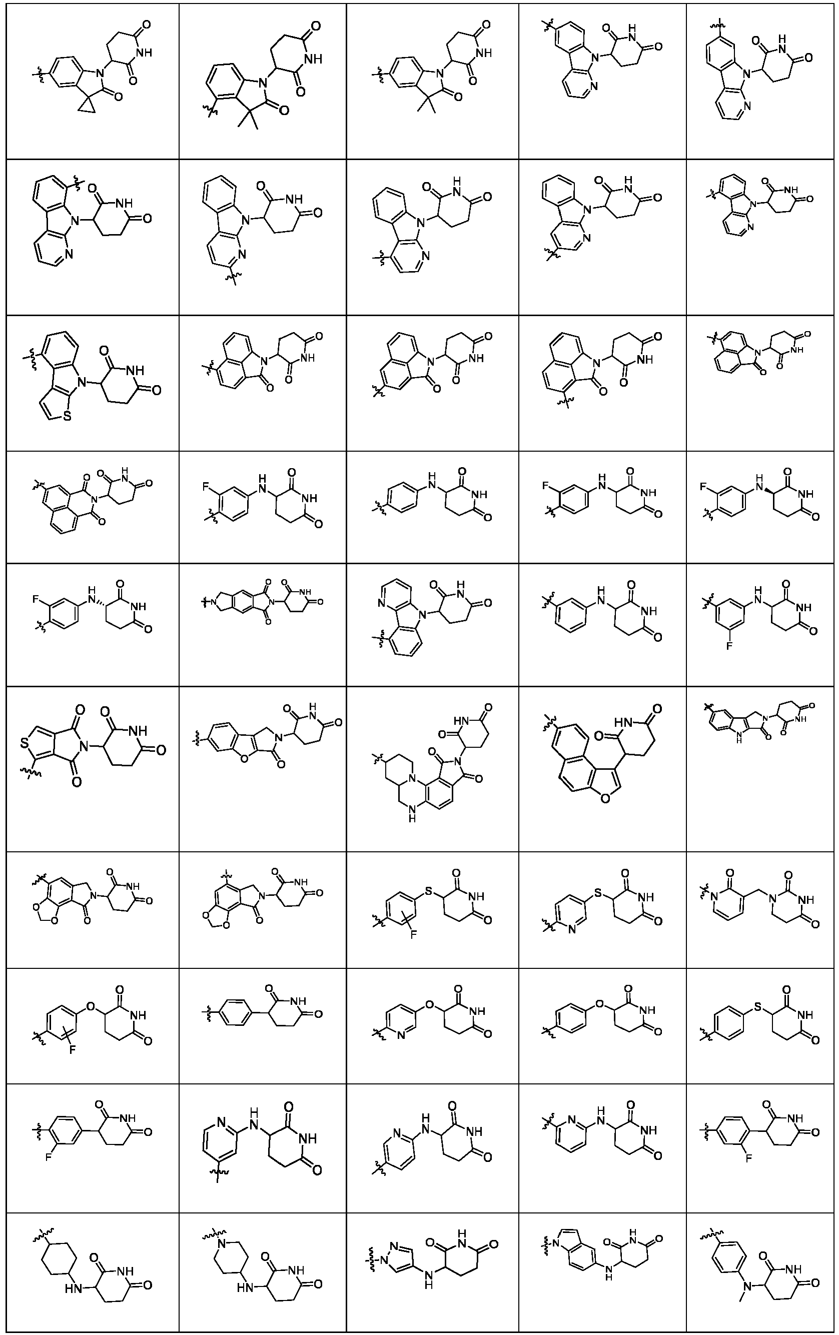

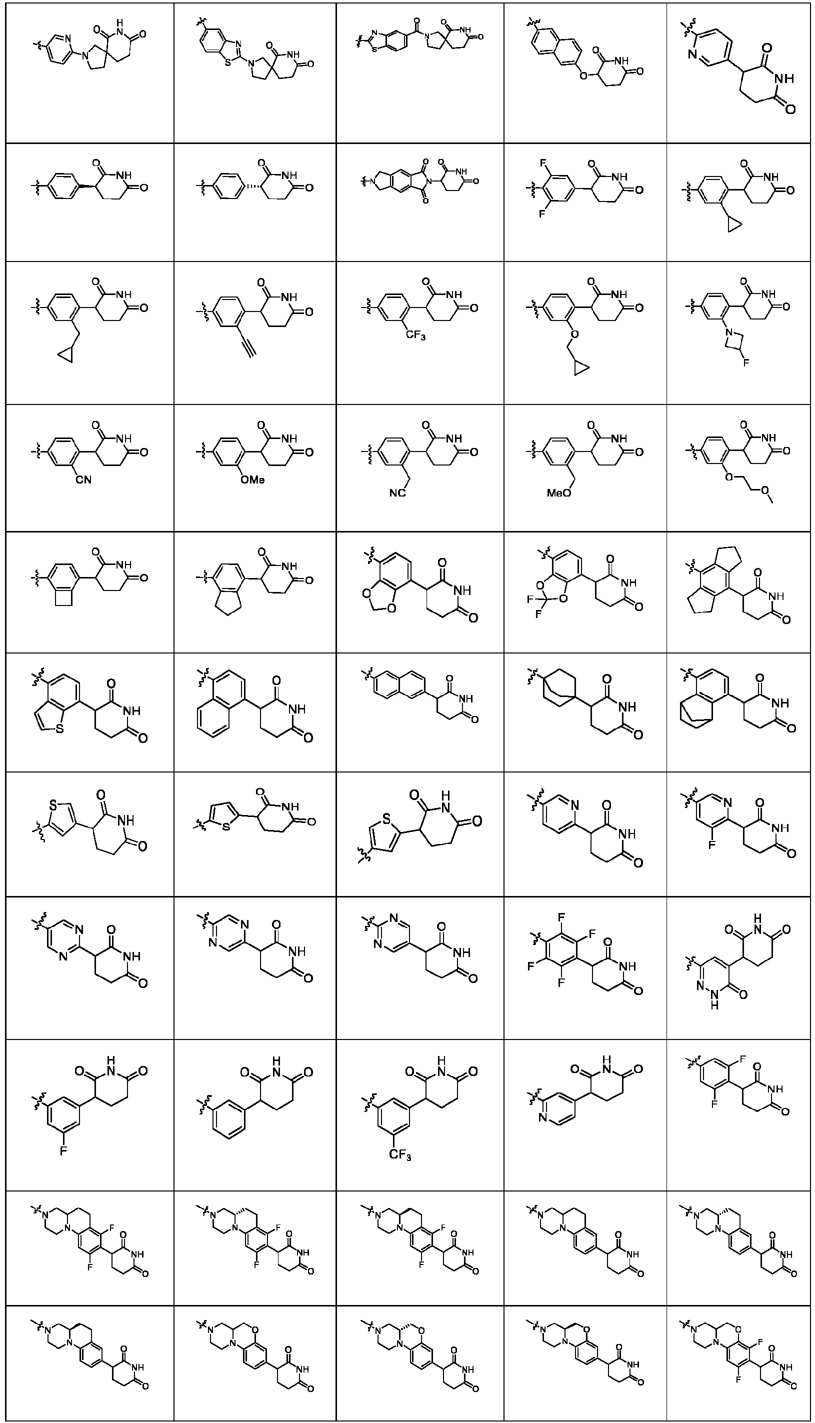

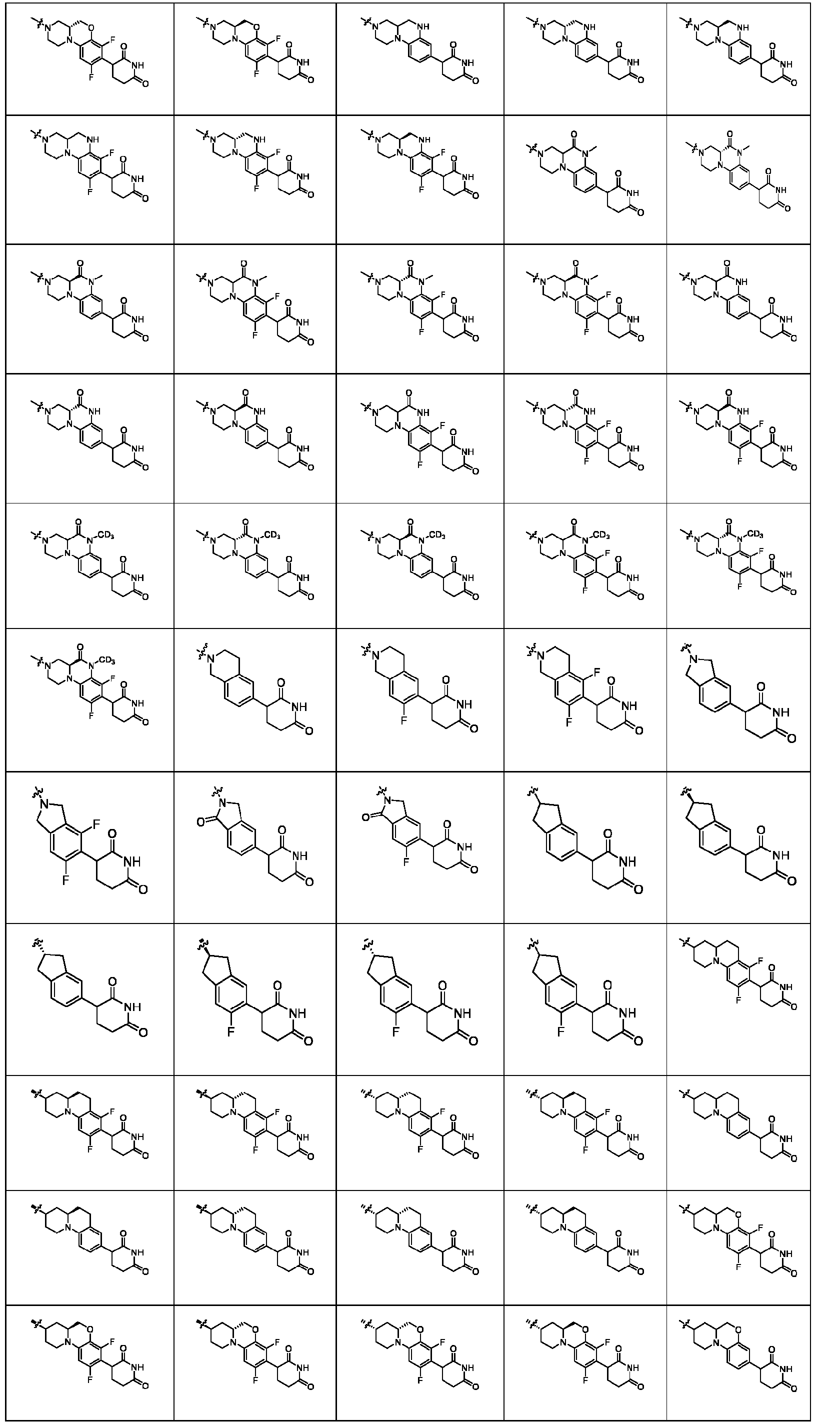

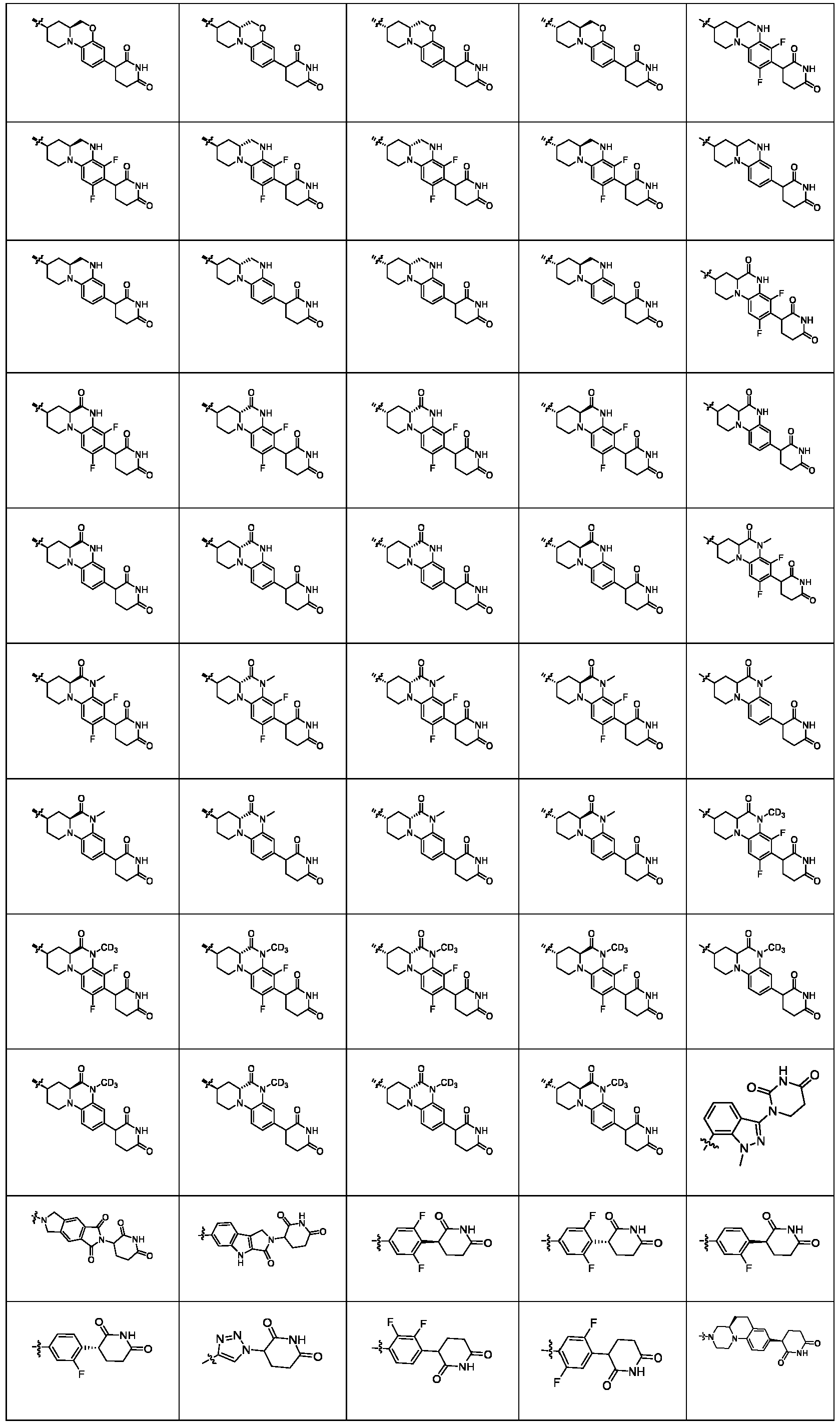

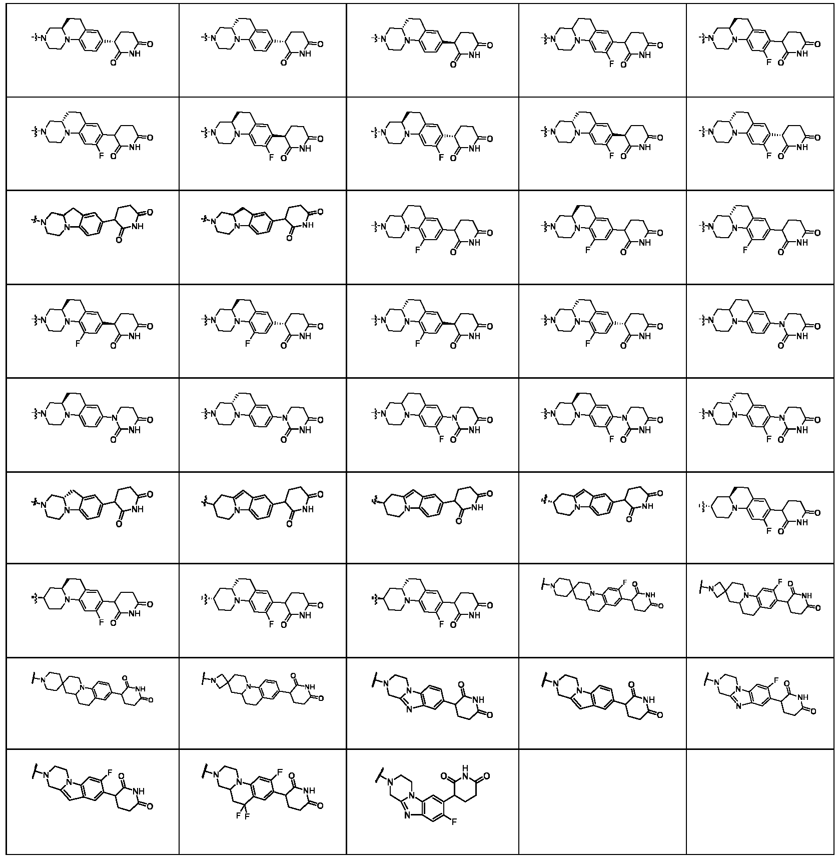

A first embodiment of the present invention is a compound represented by the above general formula (I) or a stereoisomer, a tautomer, a deuterated form, a solvate, a prodrug, a metabolite, a pharmaceutically acceptable salt or a eutectic thereof,
L is selected from a bond or -C₁₋₅₀ hydrocarbyl-, wherein the hydrocarbyl has 1 to 20 methylene units optionally replaced by -Ak- or -Cy-;
each -Ak- is independently selected from -(CH₂)_{q}-, -(CH₂)_{q}-O-, -O-(CH₂)_{q}-, - (CH₂)_{q}-NR^{L}-, -NR^{L}-(CH₂)_{q}-, -(CH₂)_{q}-NR^{L}C(=O)-, -NR^{L}(CH₂)_{q}C(=O)-, -(CH₂)_{q}-C(=O)NR^{L}-, -C(=O)-, -C(=O)-(CH₂)_{q}-NR^{L}-, -(C≡C)_{q}-, -CH=CH-, -Si(R^{L})₂-, - Si(OH)(R^{L})-, -Si(OH)₂-, -P(=O)(OR^{L})-, -P(=O)(R^{L})-, -S-, -S(=O)-, -S(=O)₂- or a bond, wherein the -CH₂- is optionally substituted with 1 to 2 substituents selected from halogen, OH, CN, NH₂, C₁₋₆ alkyl, C₁₋₆ alkoxy, halogen-substituted C₁₋₆ alkyl, hydroxyl-substituted C₁₋₆ alkyl or cyano-substituted C₁₋₆ alkyl;
each q is independently selected from 0, 1, 2, 3, 4, 5 or 6;
each R^{L} is independently selected from H, C₁₋₆ alkyl, 3- to 7-membered heterocyclyl, 3- to 7-membered cycloalkyl, phenyl or 5- to 6-membered heteroaryl, wherein the heterocyclyl or heteroaryl contains 1 to 4 heteroatoms selected from O, S or N;
each -Cy- is independently selected from a bond, a 4- to 8-membered mono-heterocyclic ring, a 4- to 10-membered fused-heterocyclic ring, a 5- to 12-membered spiro-heterocyclic ring, a 7- to 10-membered bridged-heterocyclic ring, C₃₋₇ monocycloalkyl, C₄₋₁₀ fused cycloalkyl, C₅₋₁₂ spirocycloalkyl, C₇₋₁₀ bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally substituted with 1 to 2 substituents selected from halogen, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally substituted with 1 or 2 =O when the heteroatom is selected from S;
B is selected from 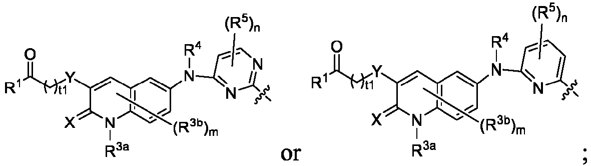
X is selected from O, S or CH₂;
Y is selected from NR^{Y} or O;
R^{Y} is selected from H, C₁₋₆ alkyl, C₃₋₁₀ carbocycle or 3- to 10-membered heterocycle, wherein the alkyl, carbocycle or heterocycle is optionally substituted with 1 to 3 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₃₋₆ carbocycle or 3- to 8-membered heterocycle, and the heterocycle contains 1 to 3 heteroatoms selected from O, S or N;
R¹ is selected from H, OH, -(CH₂)ₜ₂NR^{1a}R^{1b}, C₁₋₆ alkyl or C₁₋₆ alkoxy, wherein the alkyl or alkoxy is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl or C₁₋₆ alkoxy;
R^{1a} and R^{1b} are each independently selected from H, OH, NH₂, C₁₋₄ alkyl, C₂₋₆ alkynyl or C₁₋₄ alkoxy, wherein the alkyl, alkynyl or alkoxy is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy or C₂₋₆ alkynyl;
or R^{1a} and R^{1b} together with the atom to which they are attached form a 3- to 10-membered ring;
each R^{3a} or R⁴ is independently selected from H, OH, NH₂, C₁₋₆ alkyl, C₂₋₆ alkynyl, C₁₋₆ alkoxy, C₃₋₁₀ carbocycle or 3- to 10-membered heterocycle, wherein the alkyl, alkoxy, alkynyl, carbocycle or heterocycle is optionally substituted with 1 to 4 substituents selected from halogen, OH, cyano, NH(CH₃), N(CH₃)₂, NH(CH₂CH₃), N(CH₂CH₃)₂, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₂₋₆ alkynyl, C₃₋₆ cycloalkyloxy, C₃₋₆ carbocycle or 3- to 8-membered heterocycle, and the heterocycle contains 1 to 3 heteroatoms selected from O, S or N;
each R^{3b} or R⁵ is independently selected from H, halogen, cyano, NH₂, NO₂, OH, C₁₋₆ alkyl, C₂₋₄ alkenyl, C₂₋₄ alkynyl, C₁₋₆ alkoxy, C₃₋₁₀ carbocycle or 3- to 10-membered heterocycle, wherein the alkyl, alkoxy, carbocycle or heterocycle is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₃₋₆ carbocycle or 3- to 8-membered heterocycle, and the heterocycle contains 1 to 3 heteroatoms selected from O, S or N;
t1 is selected from 1, 2, 3, 4 or 5;
t2 is selected from 0, 1, 2, 3, 4 or 5;
m is selected from 0, 1, 2, 3 or 4;
n is selected from 0, 1 or 2;
K is selected from 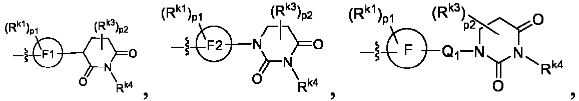 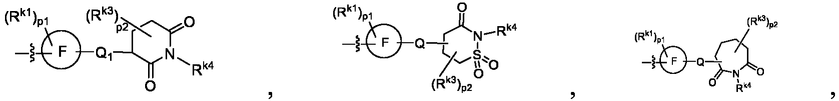 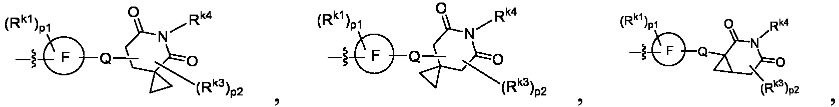 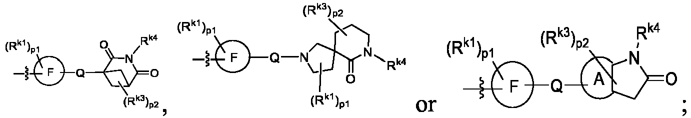
each F1 is independently selected from C₃₋₇ monocyclic carbocycle, C₄₋₁₄ fused ring carbocycle, C₅₋₁₂ spiro ring carbocycle, C₅₋₁₀ bridged ring carbocycle, C₆₋₁₄ aryl, a 6- to 7-membered non-aromatic mono-heterocyclic ring, a 5- to 14-membered bridged-heterocyclic ring, 5- to 6-membered heteroaryl, a 11- to 20-membered fused-heterocyclic ring, phthalazin-1(2*H*)-onyl, benzo[d][1,2,3]triazin-4(3H)-onyl, benzothienyl, 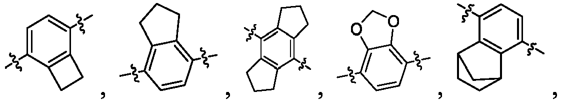 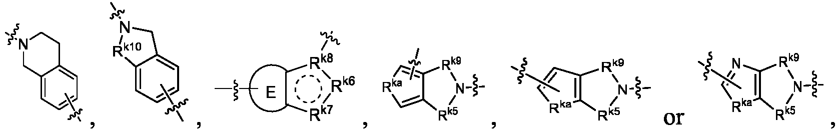 wherein the mono-heterocyclic ring, fused-heterocyclic ring, spiro-heterocyclic ring, bridged-heterocyclic ring or heteroaromatic ring contains 1 to 4 heteroatoms selected from O, S or N, provided that when F1 is selected from 6-membered heteroaryl, the right connection site on the ring is a carbon atom;
each E is independently selected from C₅₋₁₂ carbocycle, 5- to 12-membered heterocycle or a 5- to 12-membered heteroaromatic ring, wherein the heterocycle or heteroaromatic ring contains 1 to 4 heteroatoms selected from O, S or N;
each F2 is independently selected from C₃₋₇ monocyclic non-aromatic carbocycle, C₄₋₁₀ fused ring non-aromatic carbocycle, C₅₋₁₂ spiro ring carbocycle, C₅₋₁₀ bridged ring carbocycle, C₁₂₋₁₄ aryl, a 4- to 7-membered non-aromatic mono-heterocyclic ring, a 5- to 14-membered spiro-heterocyclic ring, a 5- to 14-membered bridged-heterocyclic ring, a 11- to 20-membered fused-heterocyclic ring or 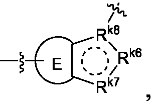 wherein the mono-heterocyclic ring, fused-heterocyclic ring, spiro-heterocyclic ring, bridged-heterocyclic ring or heteroaromatic ring contains 1 to 4 heteroatoms selected from O, S or N; 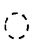
represents a ring selected from an aromatic ring or a non-aromatic ring;
each F is independently selected from C₃₋₇ monocyclic carbocycle, C₄₋₂₀ fused ring carbocycle, C₅₋₂₀ spiro ring carbocycle, C₅₋₂₀ bridged ring carbocycle, a 4- to 7-membered mono-heterocyclic ring, a 4- to 20-membered fused-heterocyclic ring, a 5- to 20-membered spiro-heterocyclic ring or a 5- to 20-membered bridged-heterocyclic ring, wherein the mono-heterocyclic ring, fused-heterocyclic ring, spiro-heterocyclic ring, bridged-heterocyclic ring or heteroaromatic ring contains 1 to 4 heteroatoms selected from O, S or N;
A is selected from C₃₋₈ carbocycle, a benzene ring, 4- to 7-membered heterocycle or a 5- to 6-membered heteroaromatic ring, wherein the heterocycle or heteroaromatic ring contains 1 to 4 heteroatoms selected from O, S or N;
each Q1 is independently selected from -O-, -S-, -CH₂-, -NR^{q}- or -CO-;
each Q is independently selected from a bond, -O-, -S-, -CH₂-, -NR^{q}-, -CO-, - NR^{q}CO-, -CONR^{q}- or 4- to 7-membered heterocycle, wherein the heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
R^{q} is selected from H or C₁₋₄ alkyl;
each R^{k1} is independently selected from H, D, F, Cl, Br, I, OH, =O, NH₂, CN, COOH, CONH₂, C₁₋₆ alkyl, C₁₋₆ alkoxy, C₃₋₆ cycloalkyl or R^{k7a} wherein the alkyl, alkoxy or cycloalkyl is optionally substituted with 1 to 4 substituents selected from D, F, Cl, Br, I, OH, =O, NH₂, CN, COOH, CONH₂, C₁₋₄ alkyl, C₁₋₄ alkoxy or C₃₋₆ cycloalkyl;
each R^{k3} is independently selected from H, D, F, Cl, Br, I, OH, =O, NH₂, CN, COOH, CONH₂, C₁₋₆ alkyl, C₁₋₆ alkoxy, C₃₋₈ cycloalkyl or 3- to 8-membered heterocyclyl, wherein the alkyl, alkoxy, cycloalkyl or heterocyclyl is optionally substituted with 1 to 4 substituents selected from D, F, Cl, Br, I, OH, =O, NH₂, CN, COOH, CONH₂, C₁₋₄ alkyl or C₁₋₄ alkoxy, and the heterocyclyl contains 1 to 4 heteroatoms selected from O, S or N;
or two R^{k3} together with the carbon atom or ring backbone to which they are directly attached form C₃₋₈ carbocycle or 3- to 8-membered heterocycle, or two R^{k1} together with the carbon atom or ring backbone to which they are directly attached form C₃₋₈ carbocycle or 3- to 8-membered heterocycle, wherein the carbocycle or heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
each R^{k4} is independently selected from H or C₁₋₄ alkyl;
each R^{k5} is independently selected from 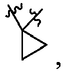 C(CH₃)₂, CO, CH₂, SO₂, 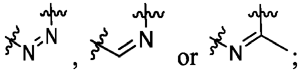
each R^{k6} is independently selected from CO, CH, SO, SO₂, CH₂, N or NR^{k7a}.
each R^{k7} is independently selected from 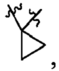 C(CH₃)₂, CO, CH, N, CH₂, O, S or NR^{k7a};
R^{k7a} is selected from H, C₁₋₄ alkyl, C₂₋₄ alkenyl, C₂₋₄ alkynyl, C₃₋₆ cycloalkyl or 3- to 6-membered heterocycloalkyl, wherein the alkyl, cycloalkyl or heterocycloalkyl is optionally substituted with 1 to 4 substituents selected from F, Cl, Br, I, OH, NH₂, CN, CF₃, C₁₋₄ alkyl, C₁₋₄ alkoxy, C₂₋₄ alkenyl, C₂₋₄ alkynyl or C₃₋₆ cycloalkyl;
each R^{k8} is independently selected from C, N or CH;
each R^{k9} is independently selected from a bond, 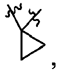 C(CH₃)₂, CO, CH₂, CH₂CH₂ or SO₂;
R^{ka} is selected from O, S or NH;
R^{k10} is selected from CH₂ or CO;
each p1 or p2 is independently selected from 0, 1, 2, 3, 4 or 5;
the compound is optionally substituted with 1-30 D.

A second embodiment of the present invention is a compound represented by the above general formula (I) or a stereoisomer, a tautomer, a deuterated form, a solvate, a prodrug, a metabolite, a pharmaceutically acceptable salt or a eutectic thereof,
L is selected from -Cy1-Ak1-Cy2-Ak2-Cy3-Ak3-Cy4-Ak4-Cy5-Ak5-, -Cy1-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cy1-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, -Ak1-Ak2-Ak3-Ak4-Cy1-Cy2-Cy3-Ak5-Cy4-, -Ak1-, -Ak1-Ak2-, -Ak1-Ak2-Ak3-, -Ak1-Ak2-Ak3-Ak4-, - Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9;
Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, - (CH₂)_{q}-NR^{L}C(=O)-, -(CH2)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from halogen, OH, CN, NH₂, C₁₋₄ alkyl, C₁₋₄ alkoxy, halogen-substituted C₁₋₄ alkyl, hydroxyl-substituted C₁₋₄ alkyl or cyano-substituted C₁₋₄ alkyl;
each Cy1, Cy2, Cy3, Cy4 or Cy5 is independently selected from a bond, a 4-to 7-membered mono-heterocyclic ring, a 4- to 10-membered fused-heterocyclic ring, a 5- to 12-membered spiro-heterocyclic ring, a 7- to 10-membered bridged-heterocyclic ring, C₃₋₇ monocycloalkyl, C₄₋₁₀ fused cycloalkyl, C₅₋₁₂-membered spirocycloalkyl, C₇.₁₀-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally substituted with 1 to 2 substituents selected from F, Cl, Br, I, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally substituted with 1 or 2 =O when the heteroatom is selected from S;
each q is independently selected from 0, 1, 2, 3 or 4;
each R^{L} is independently selected from H or C₁₋₆ alkyl;
the remaining definitions are the same as those in the first embodiment of the present invention.

A third embodiment of the present invention is a compound represented by the above general formula (I) or a stereoisomer, a tautomer, a deuterated form, a solvate, a prodrug, a metabolite, a pharmaceutically acceptable salt or a eutectic thereof,
Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from F, Cl, Br, I, OH, CN, NH₂, CF₃, hydroxymethyl, C₁₋₄ alkyl or C₁₋₄ alkoxy;
each R^{L} is independently selected from H or C₁₋₄ alkyl;
each Cy1, Cy2, Cy3, Cy4 or Cy5 is independently selected from a bond, a 4-to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, C₃₋₇ monocycloalkyl, C₄₋₁₀ fused cycloalkyl, C₅₋₁₂ spirocycloalkyl, C₇₋₁₀ bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally substituted with 1 to 2 substituents selected from F, Cl, Br, I, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the mono-heterocyclic ring, fused-heterocyclic ring, bridged-heterocyclic ring, spiro-heterocyclic ring or heteroaryl contains 1 to 4 heteroatoms selected from O, S or N, and is optionally substituted with 1 or 2 =O when the heteroatom is selected from S;
R^{Y} is selected from H, methyl, ethyl, propyl, isopropyl or cyclopropyl, wherein the methyl, ethyl, propyl, isopropyl, cyclopropyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
R¹ is selected from H, OH, NR^{1a}R^{1b}, methyl, ethyl, propyl, isopropyl or methoxy, wherein the methyl, ethyl, propyl, isopropyl or methoxy is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl or C₁₋₄ alkoxy;
R^{1a} and R^{1b} are each independently selected from H, OH, NH₂, methyl, ethyl, propyl, isopropyl, methoxy, propargyl or propynyl, wherein the methyl, ethyl, propyl, isopropyl, methoxy, propargyl or propynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy or C₂₋₄ alkynyl;
each R^{3a} or R⁴ is independently selected from H, OH, NH₂, methyl, ethyl, propyl, propargyl, propynyl, isopropyl, cyclopropyl, cyclobutyl, cyclopentyl, cyclohexyl, phenyl, azetidinyl, pyrrolidyl or piperidyl, wherein the methyl, ethyl, propyl, isopropyl, cyclopropyl, cyclobutyl, cyclopentyl, cyclohexyl, phenyl, azetidinyl, pyrrolidyl, piperidyl, propargyl or propynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₂₋₄ alkynyl, C₃₋₆ cycloalkyloxy, C₃₋₆ carbocycle or 3- to 6-membered heterocycle, and the heterocycle contains 1 to 3 heteroatoms selected from O, S or N;
each R^{3b} or R⁵ is independently selected from H, F, Cl, Br, I, cyano, NH₂, OH, methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl, piperidyl or ethynyl, wherein the methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl, piperidyl or ethynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
each F1 is independently selected from cyclobutyl, cyclopentyl, cyclohexyl, piperidyl, piperazinyl, phenyl, naphthyl, pyridyl, pyrazinyl, pyridazinyl, pyridazinonyl, pyrimidyl, phthalazin-1(2*H*)-onyl, benzo[d][1,2,3]triazin-4(*3*H)-onyl, thienyl, benzothienyl, triazolyl, 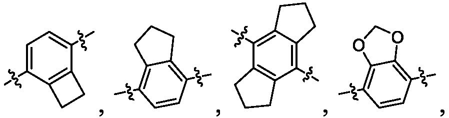 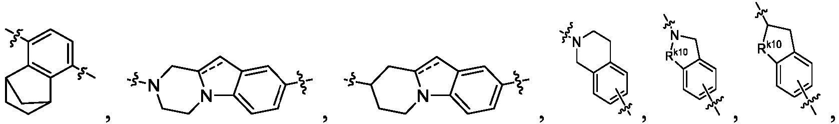 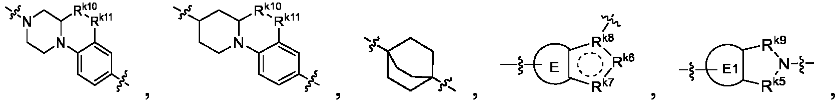 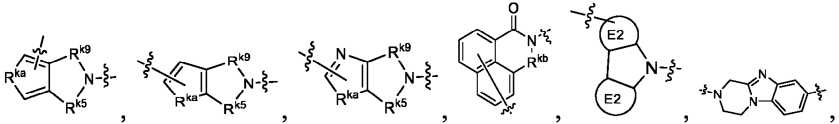 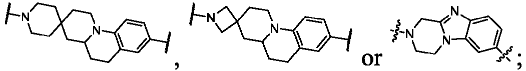
-̅ -̅ -̅ -̅ -̅ is selected from a single bond or a double bond;
each F2 is independently selected from 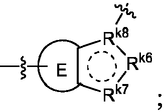
each F is independently selected from cyclobutyl, cyclopentyl, cyclohexyl, azetidinyl, pyrrolidyl, piperidyl, piperazinyl, pyridyl, pyrimidyl, pyridazinyl, pyrazinyl, triazinyl, pyrrolyl, imidazolyl, triazolyl, oxazolyl, furyl, thienyl, thiazolyl, 2-pyridonyl, pyrazolyl, phenyl, naphthyl, anthryl, phenanthryl, benzothiazolyl, indolyl, 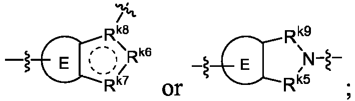
each E or A is independently selected from a benzene ring, a naphthalene ring or a pyridine ring;
each E1 is independently selected from 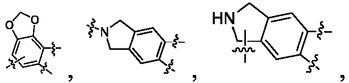 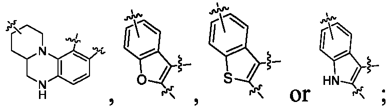
each E2 is independently selected from a benzene ring, a pyridine ring, a pyrimidine ring, a thiazole ring, a thiophene ring or a furan ring;
R^{q} is selected from H, methyl or ethyl;
R^{k1} and R^{k3} are each independently selected from H, D, F, Cl, Br, I, OH, =O, NH₂, CF₃, CN, COOH, CONH₂, C₁₋₄ alkyl, C₁₋₄ alkoxy or R^{k7a}, wherein the alkyl or alkoxy is optionally substituted with 1 to 4 substituents selected from D, F, Cl, Br, I, OH or NH₂;
or two R^{k3} together with the carbon atom or ring backbone to which they are directly attached form C₃₋₇ carbocycle or 3- to 7-membered heterocycle, or two R^{k1} together with the carbon atom or ring backbone to which they are directly attached form C₃₋₇ carbocycle or 3- to 7-membered heterocycle, wherein the carbocycle or heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
each R^{k4} is independently selected from H, methyl, ethyl or propyl;
each R^{k5} is independently selected from CO, CH₂, SO₂ or 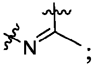
R^{k7a} is selected from H, C₁₋₄ alkyl, C₂₋₄ alkenyl, C₂₋₄ alkynyl, C₃₋₆ cycloalkyl or 3- to 6-membered heterocyclyl, wherein the alkyl, alkenyl, alkynyl, heterocyclyl or cycloalkyl is optionally substituted with 1 to 4 substituents selected from F, Cl, Br, I, OH, CN, CF₃, C₁₋₄ alkyl, C₁₋₄ alkoxy, C₂₋₄ alkenyl, C₂₋₄ alkynyl or C₃₋₆ cycloalkyl; each R^{k9} is independently selected from CO, SO₂ or CH₂;
R^{kb} is selected from a bond or CO;
R^{k10} is selected from CH₂ or CO;
R^{k11} is selected from NH, O or CH₂;
the remaining definitions are the same as those in the first or second embodiment of the present invention.

A fourth embodiment of the present invention is a compound represented by the above general formula (I) or a stereoisomer, a tautomer, a deuterated form, a solvate, a prodrug, a metabolite, a pharmaceutically acceptable salt or a eutectic thereof,
Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from F, Cl, Br, I, OH, CN, NH₂, CF₃, hydroxymethyl, methyl, ethyl, methoxy or ethoxy;
R^{L} is selected from H, methyl or ethyl;
each q is independently selected from 0, 1, 2 or 3;
each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, pyrrolidyl, azacyclohexenyl, piperidyl, morpholinyl, piperazinyl, 1,4-diazepanyl, phenyl, pyridyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-pyrrolidyl, cyclopropyl-fused-piperidyl, cyclobutyl-fused-azetidinyl, cyclobutyl-fused-pyrrolidyl, cyclobutyl-fused-piperidyl, cyclopentyl-fused-azetidinyl, cyclopentyl-fused-pyrrolidyl, cyclopentyl-fused-piperidyl, cyclohexyl-fused-azetidinyl, cyclohexyl-fused-pyrrolidyl, cyclohexyl-fused-piperidyl, azetidinyl-fused-azetidinyl, azetidinyl-fused-pyrrolidyl, azetidinyl-fused-piperidyl, pyrrolidyl-fused-azetidinyl, pyrrolidyl-fused-pyrrolidyl, pyrrolidyl-fused-piperidyl, piperidyl-fused-azetidinyl, piperidyl-fused-pyrrolidyl, piperidyl-fused-piperidyl, cyclopropyl-spiro-azetidinyl, cyclopropyl-spiro-pyrrolidyl, cyclopropyl-spiro-piperidyl, cyclopropyl-spiro-piperazinyl, cyclobutyl-spiro-azetidinyl, cyclobutyl-spiro-pyrrolidyl, cyclobutyl-spiro-piperidyl, cyclopentyl-spiro-azetidinyl, cyclopentyl-spiro-pyrrolidyl, cyclopentyl-spiro-piperidyl, cyclohexyl-spiro-azetidinyl, cyclohexyl-spiro-pyrrolidyl, cyclohexyl-spiro-piperidyl, azetidinyl-spiro-azetidinyl, azetidinyl-spiro-pyrrolidyl, azetidinyl-spiro-piperidyl, pyrrolidyl-spiro-azetidinyl, pyrrolidyl-spiro-pyrrolidyl, pyrrolidyl-spiro-piperidyl, piperidyl-spiro-azetidinyl, piperidyl-spiro-pyrrolidyl, piperidyl-spiro-piperidyl, cyclopropyl-spiro-piperazinyl, 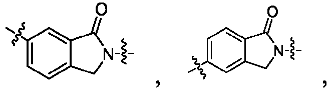 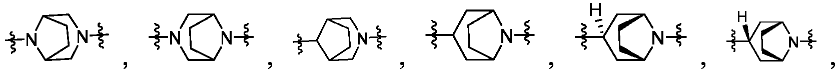 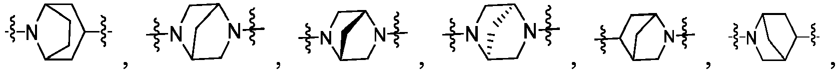 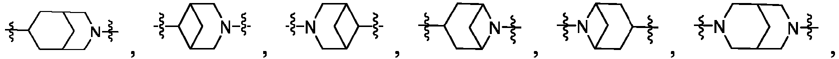 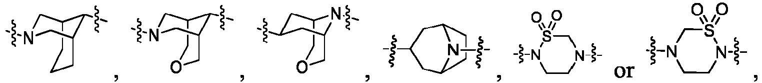 which, when substituted, is optionally substituted with 1 to 4 substituents selected from F, Cl, Br, I, OH, NH₂, COOH, CN, =O, C₁₋₄ alkyl, halogen-substituted C₁₋₄ alkyl, hydroxyl-substituted C₁₋₄ alkyl or C₁₋₄ alkoxy;
X is selected from O or S;
Y is selected from NH or O;
R¹ is selected from H, OH, NR^{1a}R^{1b}, methyl, ethyl, propyl, isopropyl or methoxy, wherein the methyl, ethyl, propyl, isopropyl or methoxy is optionally substituted with 1 to 3 substituents selected from F, Cl, Br, I, OH, cyano, NH₂, methyl, ethyl or CF₃;
R^{1a} and R^{1b} are each independently selected from H, OH, NH₂, methyl, ethyl, propyl, isopropyl, methoxy, propargyl or propynyl, wherein the methyl, ethyl, propyl, isopropyl, methoxy, propargyl or propynyl is optionally substituted with 1 to 3 substituents selected from F, Cl, Br, I, OH, cyano, NH₂, methyl, ethyl, methoxy, ethynyl or CF₃;
R^{3a} is selected from H, OH, NH₂, methyl, ethyl, propyl, isopropyl, cyclopropyl, cyclobutyl, cyclopentyl, cyclohexyl, phenyl, azetidinyl, pyrrolidyl, piperidyl, propargyl or propynyl, wherein the methyl, ethyl, propyl, isopropyl, cyclopropyl, cyclobutyl, cyclopentyl, cyclohexyl, phenyl, azetidinyl, pyrrolidyl, piperidyl, propargyl or propynyl is optionally substituted with 1 to 3 substituents selected from F, Cl, Br, I, OH, cyano, NH₂, methyl, ethyl, cyclopropyl, cyclobutyl, cyclopentyl, cyclohexyl, ethynyl, cyclopropyloxy or methoxy;
each R⁴ is independently selected from H, methyl, ethyl or cyclopropyl;
each R^{3b} is independently selected from H, F, Cl, Br, I, OH, CN, methoxy, methyl, ethyl or cyclopropyl;
each R⁵ is independently selected from H, F, Cl, Br, I, cyano, NH₂, OH, methyl, ethyl, propyl, isopropyl, methoxy, ethoxy, propoxy, isopropoxy, cyclopropyl or ethynyl, wherein the methyl, ethyl, propyl, isopropyl, methoxy, ethoxy, propoxy, isopropoxy, cyclopropyl or ethynyl is optionally substituted with 1 to 3 substituents selected from F, Cl, Br, I, OH, cyano, NH₂, methyl, ethyl or cyclopropyl;
each F1 is independently selected from cyclohexyl, piperidyl, piperazinyl, phenyl, naphthyl, pyrimidyl, pyridyl, pyrazinyl, pyridazinyl, pyridazinonyl, phthalazin-1(2*H*)-onyl, benzo[d][1,2,3]triazin-4(*3*H)-onyl, thienyl, triazolyl, 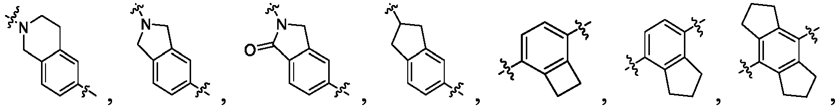 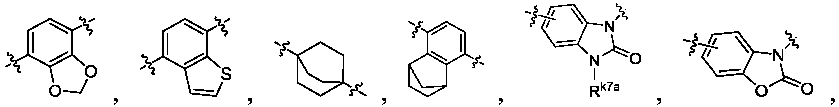 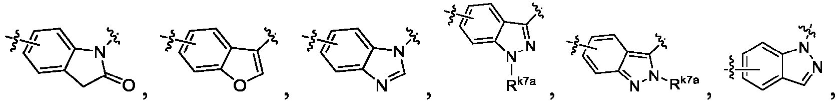 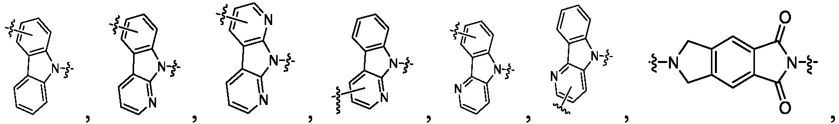 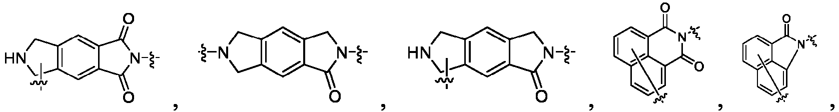 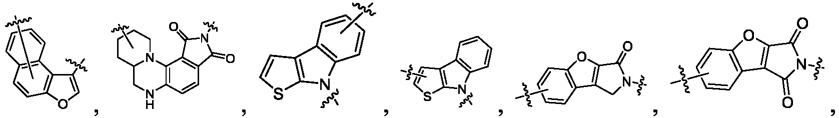 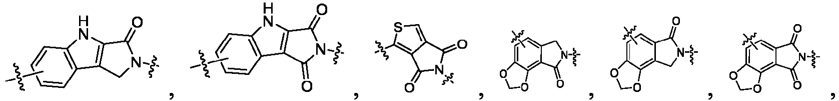 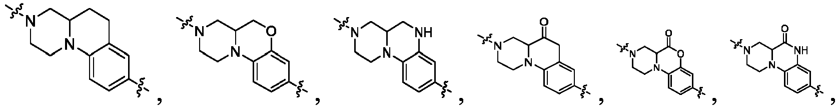 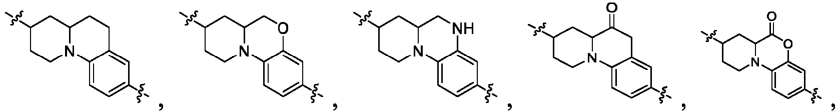 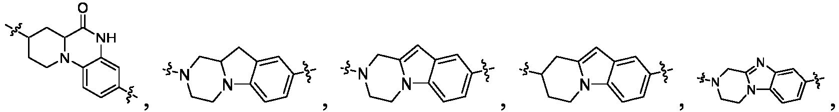 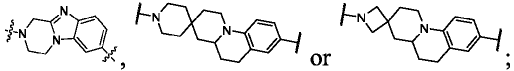
each F2 is independently selected from 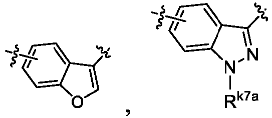 or 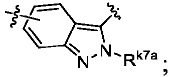
each F is independently selected from cyclobutyl, cyclopentyl, cyclohexyl, azetidinyl, pyrrolidyl, piperidyl, piperazinyl, pyridyl, pyrimidyl, pyridazinyl, pyrazinyl, triazinyl, pyrrolyl, imidazolyl, triazolyl, oxazolyl, furyl, thienyl, thiazolyl, 2-pyridone, pyrazolyl, phenyl, naphthyl, anthryl, phenanthryl, benzothiazolyl, indolyl, 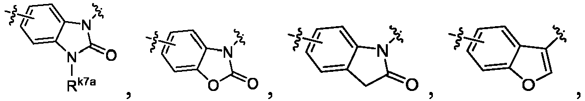 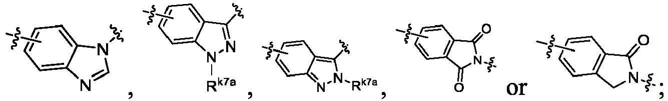
Q1 is selected from -O-, -S-, -CH₂-, NH, N(CH₃) or -CO-;
Q is selected from a bond, C(=O), CH₂, NH, N(CH₃), O, S, NHC(=O), C(=O)NH, N(CH₃)C(=O), C(=O)N(CH₃), 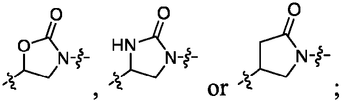
R^{k7a} is selected from H, methyl, ethyl, propyl, isopropyl, ethenyl, propenyl, allyl, ethynyl, propynyl, propargyl, cyclopropyl, cyclobutyl, cyclopentyl, cyclohexyl, azetidinyl, pyrrolidyl, piperidyl, oxacyclobutyl, tetrahydrofuryl or tetrahydropyranyl, wherein the methyl, ethyl, propyl, isopropyl, cyclopropyl, cyclobutyl, cyclopentyl, cyclohexyl, azetidinyl, pyrrolidyl, piperidyl, oxacyclobutyl, tetrahydrofuryl or tetrahydropyranyl is optionally substituted with 1 to 4 substituents selected from F, Cl, Br, I, OH, CN, CF₃, C₁₋₄ alkyl, C₁₋₄ alkoxy, ethenyl, propenyl, allyl, ethynyl, propynyl, propargyl or C₃₋₆ cycloalkyl;
R^{k1} and R^{k3} are each independently selected from H, D, F, Cl, Br, I, OH, =O, NH₂, CF₃, CN, COOH, CONH₂, methyl, ethyl, isopropyl, methoxy, ethoxy, isopropoxy, ethynyl, cyclopropyl or azetidinyl, wherein the methyl, ethyl, isopropyl, methoxy, ethoxy, isopropoxy, ethynyl, cyclopropyl or azetidinyl is optionally substituted with 1 to 4 substituents selected from D, F, Cl, Br, I, OH, NH₂, CN, cyclopropyl or methoxy; or two R^{k3} together with the carbon atom or ring backbone to which they are directly attached form C₃₋₆ carbocycle or 3- to 7-membered heterocycle, or two R^{k1} together with the carbon atom or ring backbone to which they are directly attached form C₃₋₆ carbocycle or 3- to 7-membered heterocycle, wherein the carbocycle or heterocycle is optionally substituted with 1 to 4 substituents selected from D, F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
each p1 or p2 is independently selected from 0, 1, 2 or 3;
the remaining definitions are the same as those of the first, second, or third embodiment of the present invention.

A fifth embodiment of the present invention is a compound represented by the above general formula (I) or a stereoisomer, a tautomer, a deuterated form, a solvate, a prodrug, a metabolite, a pharmaceutically acceptable salt or a eutectic thereof,
Ak1, Ak2, Ak3, Ak4, Ak5, Ak6, Ak7, Ak8 and Ak9 are each independently selected from a bond, -O-, -OCH₂-, -CH₂O-, -OCH₂CH₂-, -CH₂CH₂O-, -C=C-, - C(CH₃)₂-, -CF₂-, -CH₂-, -CH₂CH₂-, -CH₂CH₂CH₂-, -N(CH₃)-, -NH-, -CH₂N(CH₃)-, -CH₂NH-, -NHCH₂-, -CH₂CH₂N(CH₃)-, -CH₂CH₂NH-, -NHCH₂CH₂-, -C(=O)-, - C(=O)CH₂NH-, -CH₂C(=O)NH-, -C(=O)NH- or -NHC(=O)-;
each Cy1, Cy2, Cy3, Cy4 or Cy5 is independently selected from a bond or one of the following substituted or unsubstituted groups: 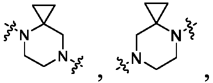 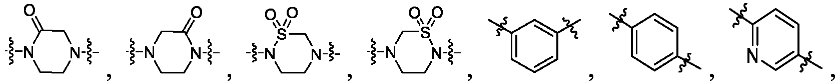 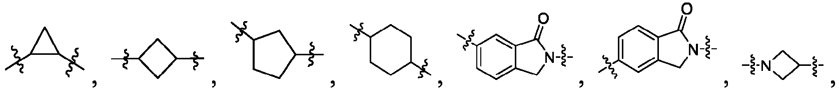 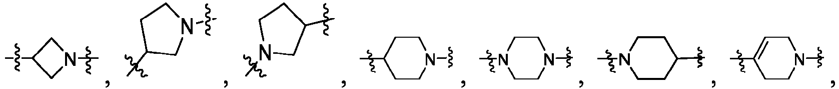 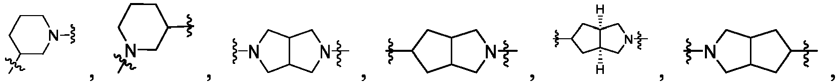 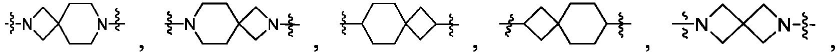 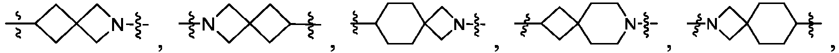 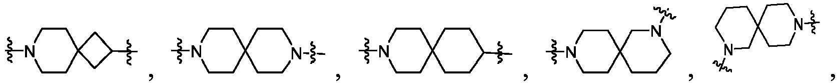 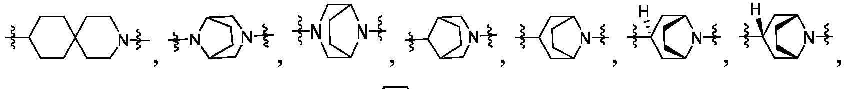 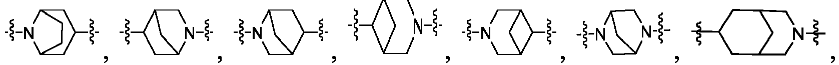 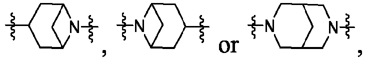 which, when substituted, is substituted with 1 to 4 substituents selected from F, CF₃, OH, methyl, =O, hydroxymethyl, COOH, CN or NH₂;
B is selected from one of the structural fragments shown in Table B-1;
K is selected from one of the structural fragments shown in Table K-1 or Table K-2;
the remaining definitions are the same as those of the first, second, third or fourth embodiment of the present invention.

A sixth embodiment of the present invention is a compound represented by the above general formula (I) or a stereoisomer, a tautomer, a deuterated form, a solvate, a prodrug, a metabolite, a pharmaceutically acceptable salt or a eutectic thereof,
L is selected from a bond, -Ak1-, -Ak1-Ak2-, -Ak1-Ak2-Ak3-, -Ak1-Ak2-Ak3-Ak4-, -Ak1-Ak2-Ak3-Ak4-Ak5-, -Ak1-Ak2-Ak3-Ak4-Ak5-Ak6-, -Cy1-, - Cy1-Ak1-, -Cy1-Ak1-Ak2-, -Cy1-Ak1-Ak2-Ak3-, -Cy1-Ak1-Ak2-Ak3-Ak4-, - Cy1-Cy2-, -Cy1-Ak1-Cy2-, -Cy1-Cy2-Ak2-, -Cy1-Ak1-Cy2-Ak2-, -Cy1-Ak1-Cy2-Ak2-Ak3-, -Cy1-Ak1-Cy2-Ak2-Ak3-Ak4-, -Cy1-Cy2-Ak2-Ak3-, -Cy1-Cy2-Ak2-Ak3-Ak4-, -Cy 1-Ak 1-Cy2-Ak2-Ak3-Ak4-, -Cy1-Ak1-Ak2-Cy3-, -Cy1-Ak1-Ak2-Cy3-Ak3-, -Cy1-Cy2-Cy3-, -Cy1-Ak1-Cy2-Cy3-, -Cy1-Cy2-Ak2-Cy3-, - Cy1-Cy2-Cy3-Ak3-, -Cy1-Ak1-Cy2-Cy3-Ak3-, -Cy1-Cy2-Ak2-Cy3-Ak3-, -Cy1-Ak1-Cy2-Ak2-Cy3-, -Cy1-Ak1-Cy2-Ak2-Cy3-Ak3-, -Cy1-Cy2-Cy3-Ak3-Ak4-, - Cy1-Cy2-Cy3-Ak3-Cy4-, -Cy1-Cy2-Cy3-Cy4-, -Cy1-Ak1-Cy2-Cy3-Cy4-, -Cy1-Cy2-Ak2-Cy3-Cy4-, -Cy1-Cy2-Cy3-Ak3-Cy4-, -Cy1-Cy2-Cy3-Cy4-Ak4-, -Cy1-Ak1-Cy2-Ak2-Cy3-Ak3-Cy4-, -Cy1-Ak1-Cy2-Ak2-Cy3-Cy4-, -Ak1-Cy2-, -Ak1-Cy2-Cy3-, -Ak1-Ak2-Cy3-, -Ak1-Ak2-Cy3-Cy4-, -Ak1-Cy2-Ak2-Cy3-, -Ak1-Cy2-Cy3-Ak3-Cy4-, -Ak1-Cy2-Cy3-Cy4-Ak4-Cy5-, -Ak1-Cy2-Ak2-, -Cy1-Cy2-Cy3-Ak3-Ak4-Ak5-, -Cy1-Cy2-Ak2-Cy3-Ak3-Ak4-Ak5-, -Cy1-Ak1-Cy2-Ak2-Ak3-Ak4-Ak5-, -Cy1-Cy2-Cy3-Cy4-Ak4-Ak5-, -Cy1-Ak1-Ak2-Ak3-Ak4-Ak5-, - Ak1-Cy2-Ak2-Ak3-Ak4-Ak5-, -Ak1-Cy2-Ak2-Ak3-Ak4- or -Ak1-Cy2-Ak2-Ak3-;
the remaining definitions are the same as those of the first, second, third, fourth or fifth embodiment of the present invention.

A seventh embodiment of the present invention is a compound represented by the above general formula (I) or a stereoisomer, a tautomer, a deuterated form, a solvate, a prodrug, a metabolite, a pharmaceutically acceptable salt or a eutectic thereof,
L is selected from a bond or one of the structural fragments shown in Table L-1, wherein the left side of the group is linked to B;
the remaining definitions are the same as those of the first, second, third, fourth, fifth or sixth embodiment of the present invention.

An eighth embodiment of the present invention is a compound represented by the above general formula (I) or a stereoisomer, a tautomer, a deuterated form, a solvate, a prodrug, a metabolite, a pharmaceutically acceptable salt or a eutectic thereof,
L is selected from a bond or one of the structural fragments shown in Table L-2, wherein the left side of the group is linked to B;
K is selected from one of the structural fragments shown in Table K-2;
the remaining definitions are the same as those of the first, second, third, fourth, fifth, sixth or seventh embodiment of the present invention.

A ninth embodiment of the present invention is a compound represented by the above general formula (I) or a stereoisomer, a tautomer, a deuterated form, a solvate, a prodrug, a metabolite, a pharmaceutically acceptable salt or a eutectic thereof, wherein the compound represented by general formula (I) is selected from a compound represented by general formula (II), 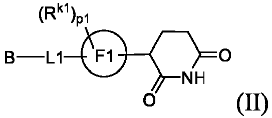
wherein L1 is selected from -Cy1-, -Cy1-Ak1-, -Cy1- Cy2-, -Cy1-Ak1-Cy2- or -Cy1-Ak1-Cy2-Cy3-;
Ak1 is selected from -CH₂-, -O- or -CH₂-CH₂-;
F1 is selected from phenyl, 5- to 6-membered heteroaryl or 13- to 14-membered fused-heterocyclic ring;
the remaining definitions are the same as those of the first, second, third, fourth, fifth, sixth, seventh or eightieth embodiment of the present invention.

A tenth embodiment of the present invention is a compound represented by the above general formula (II) or a stereoisomer, a tautomer, a deuterated form, a solvate, a prodrug, a metabolite, a pharmaceutically acceptable salt or a eutectic thereof,
wherein L1 is selected from -Cy1-, -Cy1-Ak1-, -Cy1- Cy2-, -Cy1-Ak1-Cy2- or -Cy1-Ak1-Cy2-Cy3-;
each Cy1, Cy2 or Cy3 is independently selected from a bond or one of the following substituted or unsubstituted groups: 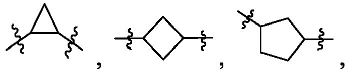 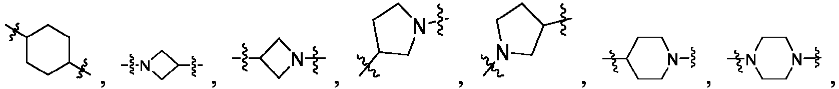 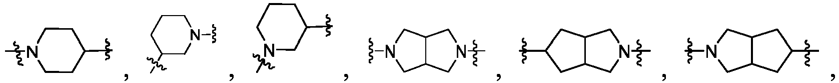 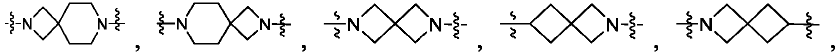 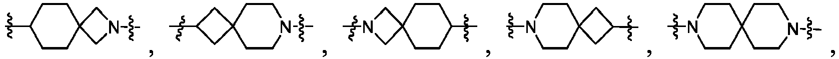 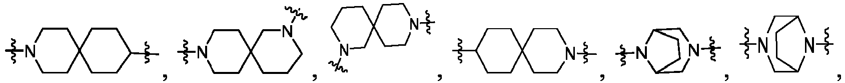 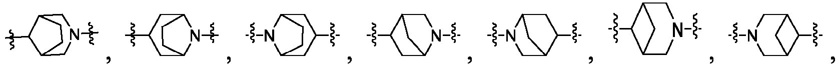 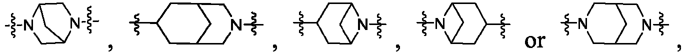 which, when substituted, is substituted with 1 to 4 substituents selected from F, CF₃, OH, methyl, =O, hydroxymethyl, COOH, CN or NH₂;
Ak1 is selected from -CH₂-, -O- or -CH₂-CH₂-;
B is selected from one of the structural fragments shown in Table B-1;
F1 is selected from phenyl, pyrrolyl, pyrazolyl, triazolyl, pyridyl, pyridazinyl, pyrazinyl, thienyl, thiazolyl, triazinyl, 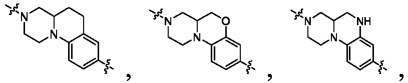 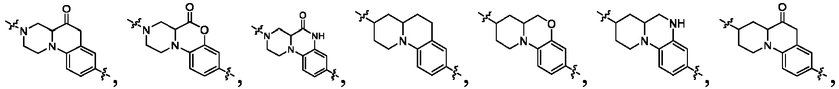 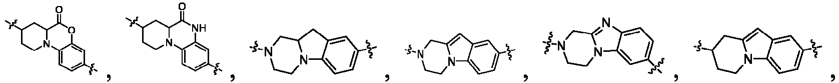 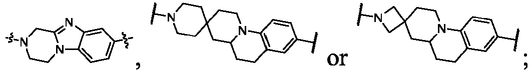
each R^{k1} is independently selected from H, D, F, Cl, Br, I, OH, =O, NH₂, CF₃, CN, COOH, CONH₂, methyl, ethyl, methoxy or ethoxy;
p1 is selected from 0, 1 or 2.

An eleventh embodiment of the present invention is a compound represented by the above general formula (II) or a stereoisomer, a tautomer, a deuterated form, a solvate, a prodrug, a metabolite, a pharmaceutically acceptable salt or a eutectic thereof, wherein
L1 is selected from 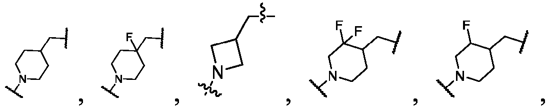 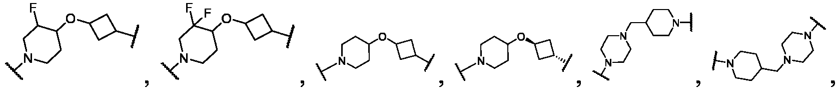 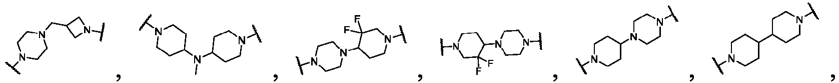 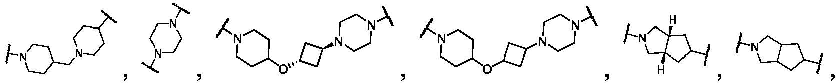 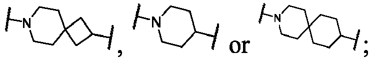
the remaining definitions are the same as the corresponding definitions in the tenth embodiment.

A twelfth embodiment of the present invention is a compound represented by the above general formula (I) or general formula (II) or a stereoisomer, a tautomer, a deuterated form, a solvate, a prodrug, a metabolite, a pharmaceutically acceptable salt or a eutectic thereof, wherein the compound represented by general formula (I) or general formula (II) is selected from a compound represented by general formula (III), 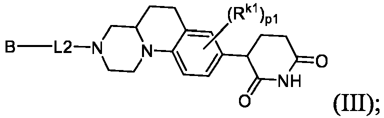
L2 is selected from -Cy1-, -Cy1-Ak1- or -Cy1-Ak1-Cy2-;
each Cy1 or Cy2 is independently selected from one of the following substituted or unsubstituted groups: 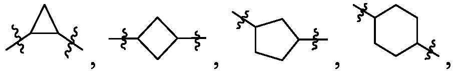 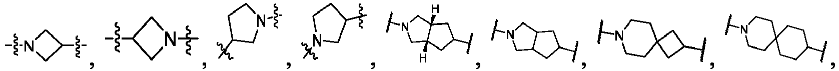 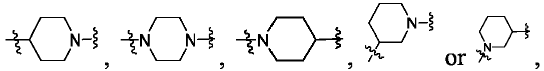 which, when substituted, is substituted with 1 to 4 substituents selected from F, CF₃, OH, methyl, =O, hydroxymethyl, COOH, CN or NH₂;
Ak1 is selected from -CH₂-, -O- or -CH₂-CH₂-;
each R^{k1} is independently selected from H, D, F, Cl, Br, I, OH, =O, NH₂, CF₃, CN, COOH, CONH₂, methyl, ethyl, methoxy or ethoxy;
p1 is selected from 0, 1 or 2.
the definition of B is the same as that in the first, second, third, fourth, fifth, sixth, seventh or eightieth embodiment of the present invention.

A thirteenth embodiment of the present invention is a compound represented by the above general formula (III) or a stereoisomer, a tautomer, a deuterated form, a solvate, a prodrug, a metabolite, a pharmaceutically acceptable salt or a eutectic thereof,
B is selected from 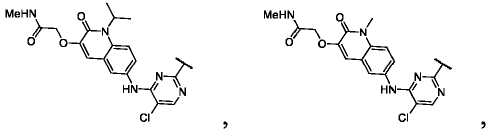 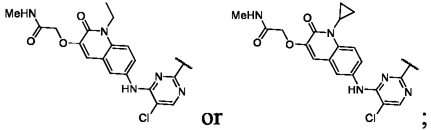
L2 is selected from 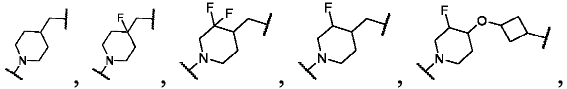 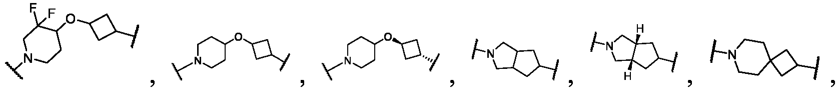 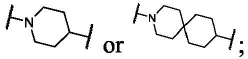
each R^{k1} is independently selected from H, D, F, Cl, Br, I, OH, =O, NH₂, CF₃, CN, COOH, CONH₂, methyl, ethyl, methoxy or ethoxy;
p1 is selected from 0, 1 or 2.

The present invention relates to a compound as described below or a stereoisomer, a tautomer, a deuterated form, a solvate, a prodrug, a metabolite, a pharmaceutically acceptable salt or a eutectic thereof, wherein the compound is selected from one of the structures shown in Table S-1 below:

**Table S-1**

| | | | |
|---|---|---|---|
| 1 | 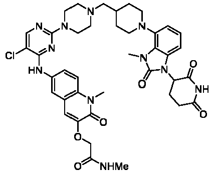 | 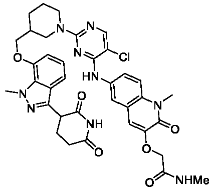 | 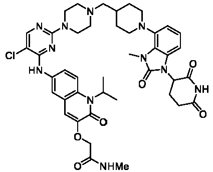 |
| 2 | 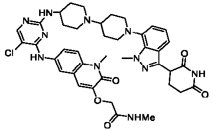 | 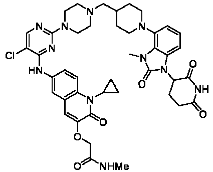 | 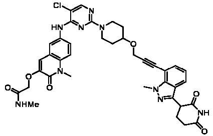 |
| 3 | 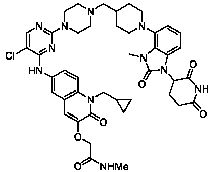 | 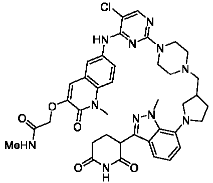 | 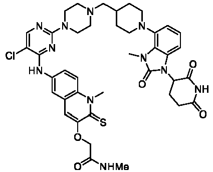 |
| 4 | 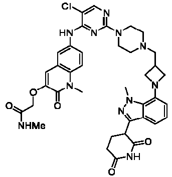 | 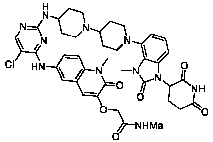 | 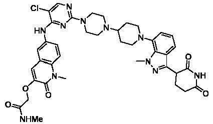 |
| 5 | 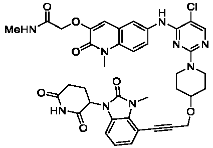 | 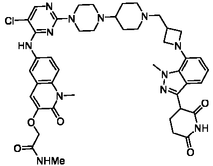 | 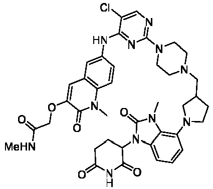 |
| 6 | 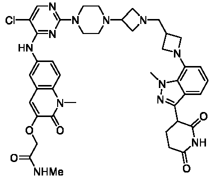 | 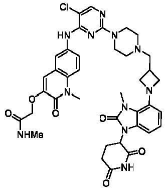 | 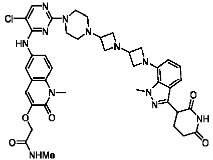 |
| 7 | 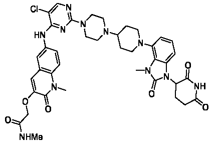 | 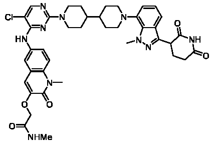 | 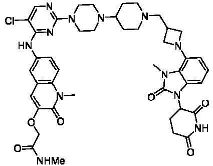 |
| 8 | 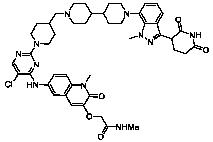 | 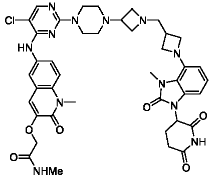 | 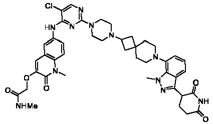 |
| 9 | 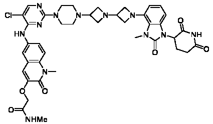 | 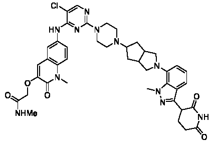 | 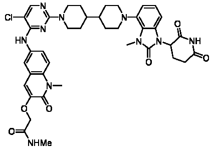 |
| 10 | 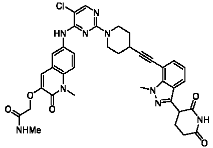 | 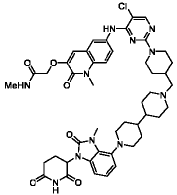 | 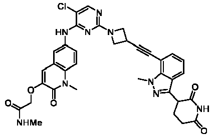 |
| 11 | 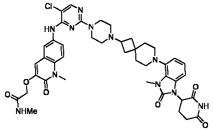 | 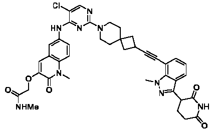 | 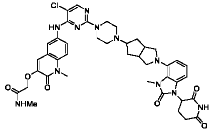 |
| 12 | 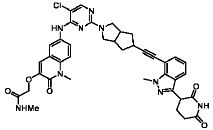 | 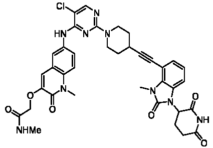 | 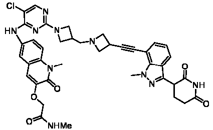 |
| 13 | 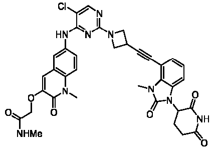 | 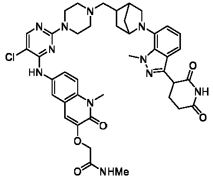 | 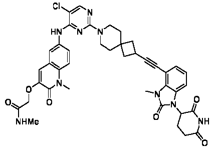 |
| 14 | 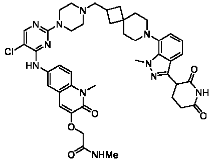 | 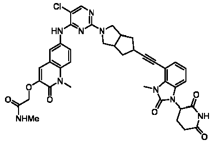 | 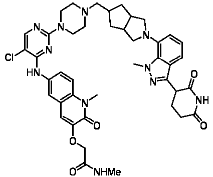 |
| 15 | 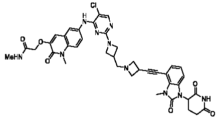 | 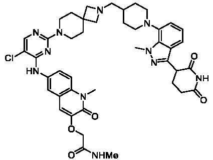 | 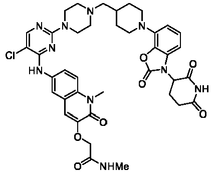 |
| 16 | 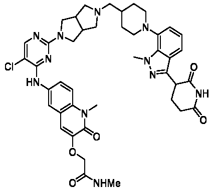 | 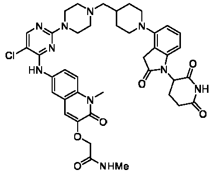 | 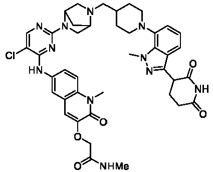 |
| 17 | 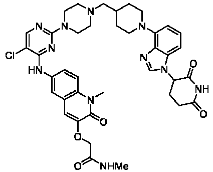 | 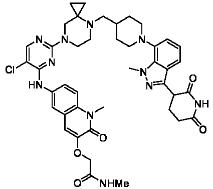 | 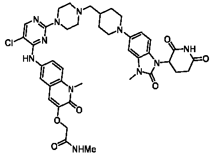 |
| 18 | 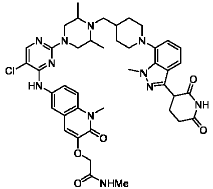 | 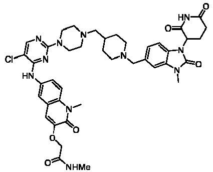 | 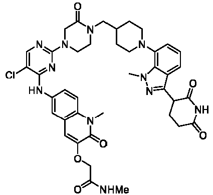 |
| 19 | 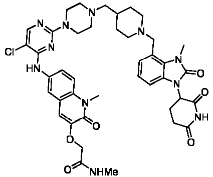 | 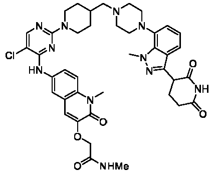 | 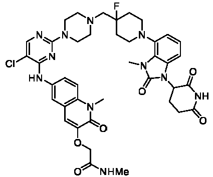 |
| 20 | 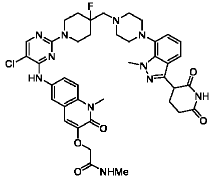 | 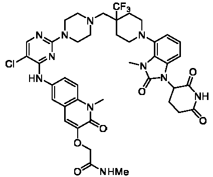 | 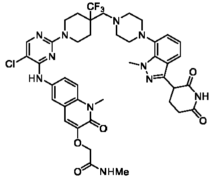 |
| 21 | 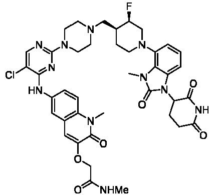 | 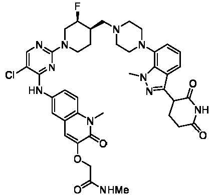 | 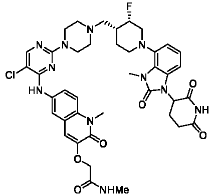 |
| 22 | 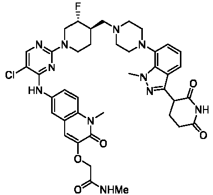 | 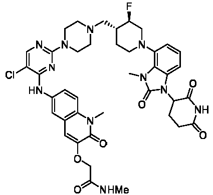 | 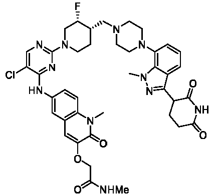 |
| 23 | 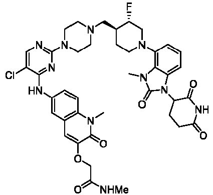 | 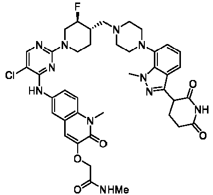 | 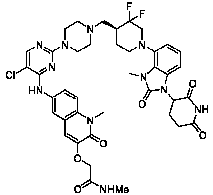 |
| 24 | 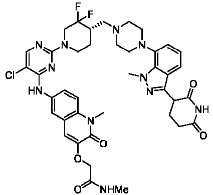 | 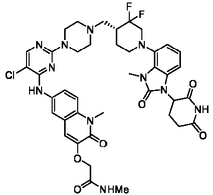 | 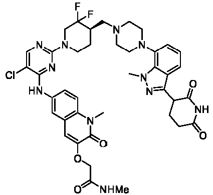 |
| 25 | 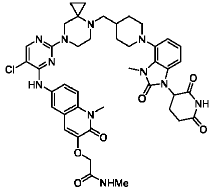 | 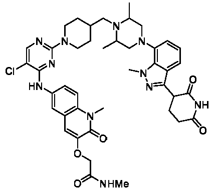 | 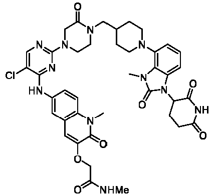 |
| 26 | 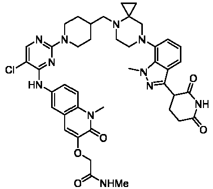 | 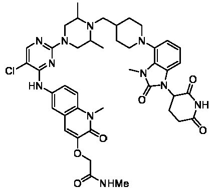 | 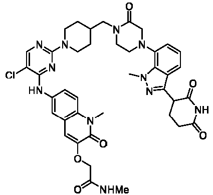 |
| 27 | 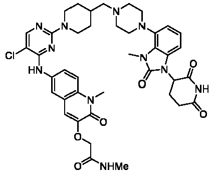 | 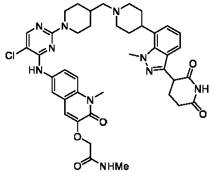 | 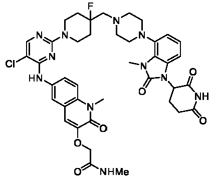 |
| 28 | 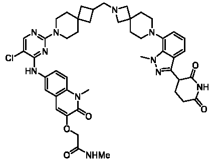 | 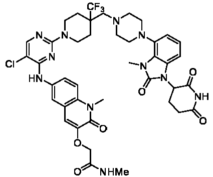 | 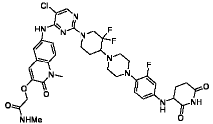 |
| 29 | 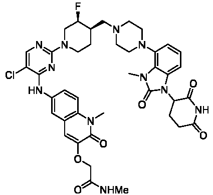 | 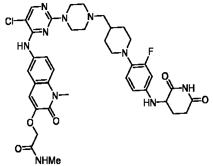 | 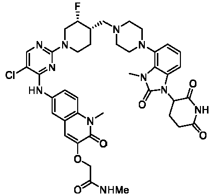 |
| 30 | 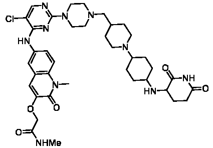 | 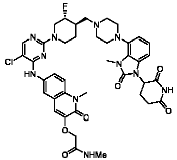 | 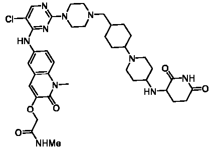 |
| 31 | 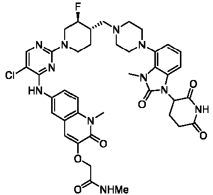 | 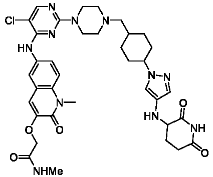 | 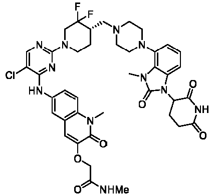 |
| 32 | 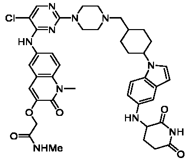 | 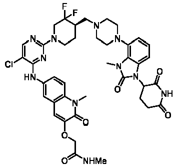 | 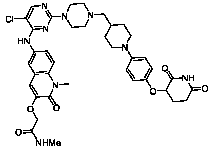 |
| 33 | 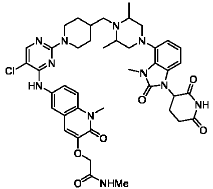 | 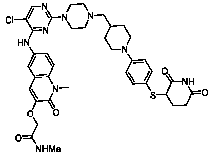 | 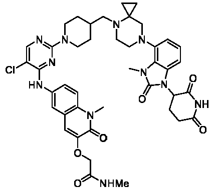 |
| 34 | 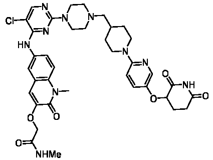 | 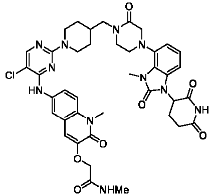 | 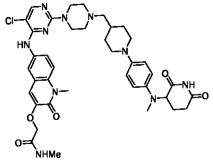 |
| 35 | 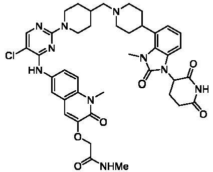 | 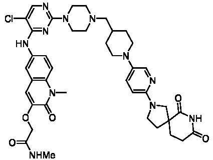 | 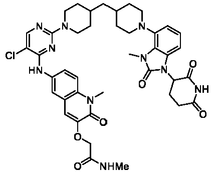 |
| 36 | 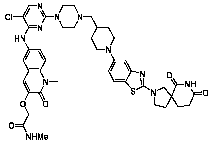 | 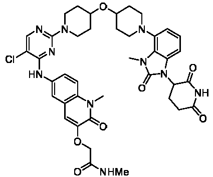 | 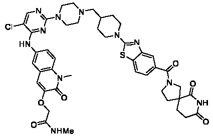 |
| 37 | 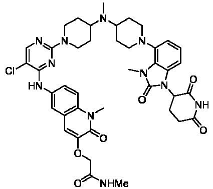 | 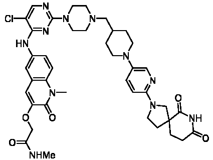 | 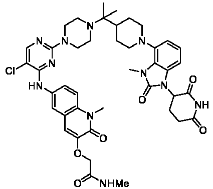 |
| 38 | 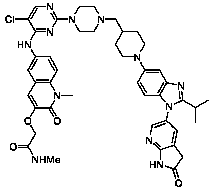 | 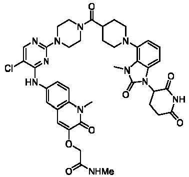 | 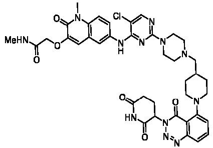 |
| 39 | 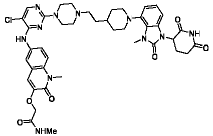 | 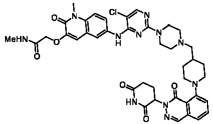 | 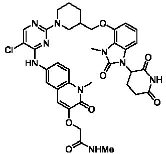 |
| 40 | 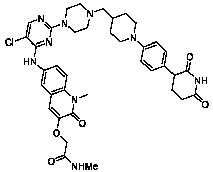 | 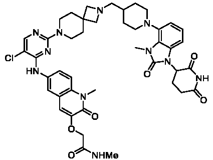 | 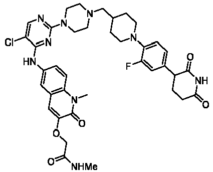 |
| 41 | 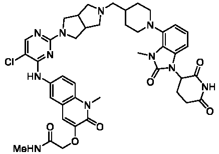 | 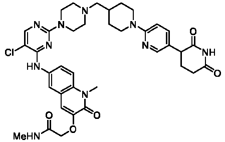 | 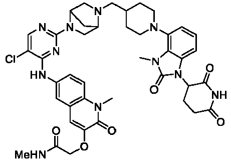 |
| 42 | 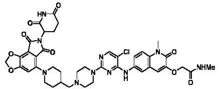 | 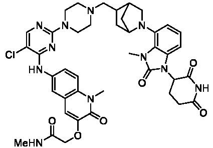 | 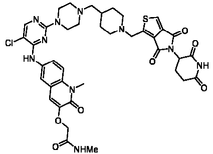 |
| 43 | 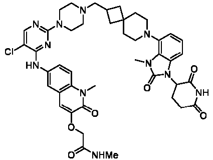 | 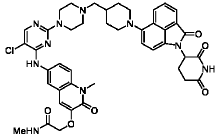 | 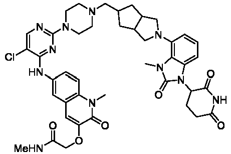 |
| 44 | 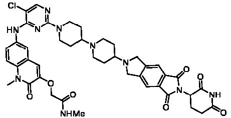 | 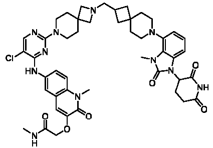 | 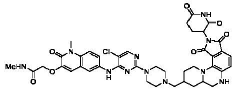 |
| 45 | 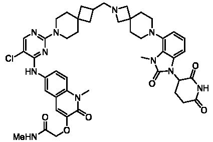 | 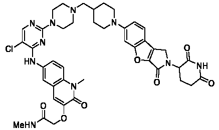 | 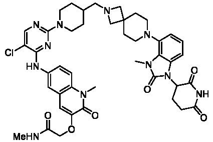 |
| 46 | 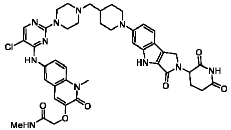 | 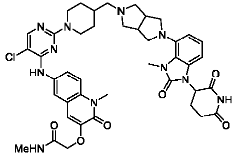 | 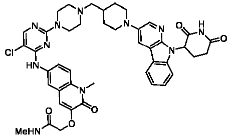 |
| 47 | 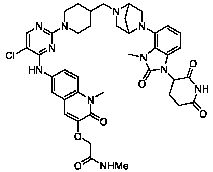 | 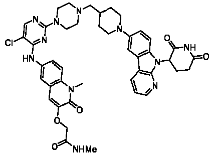 | 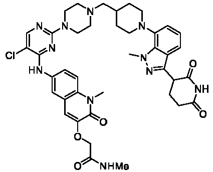 |
| 48 | 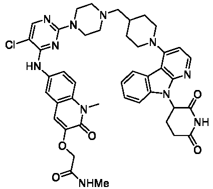 | 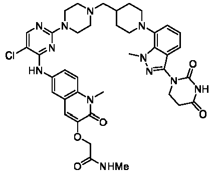 | 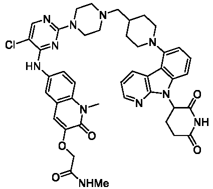 |
| 49 | 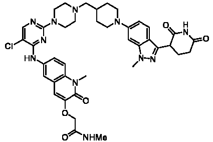 | 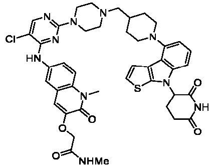 | 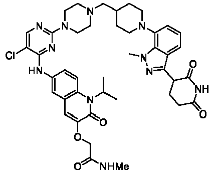 |
| 50 | 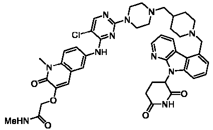 | 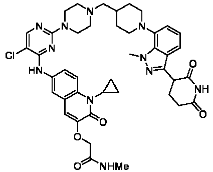 | 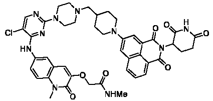 |
| 51 | 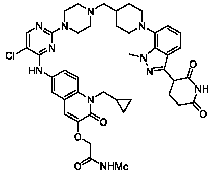 | 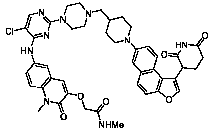 | 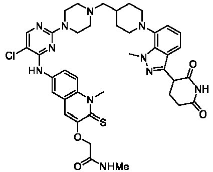 |
| 52 | 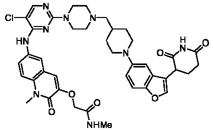 | 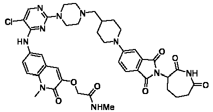 | 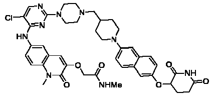 |
| 53 | 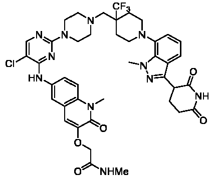 | 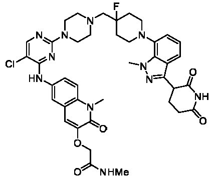 | 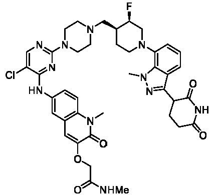 |
| 54 | 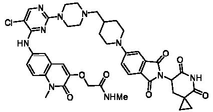 | 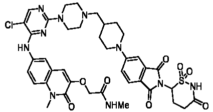 | 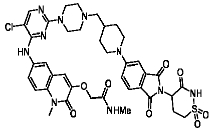 |
| 55 | 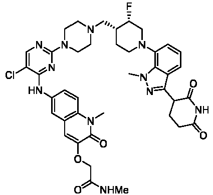 | 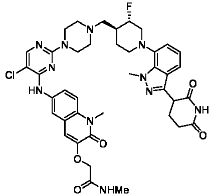 | 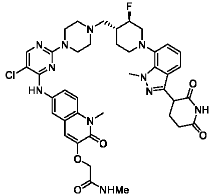 |
| 56 | 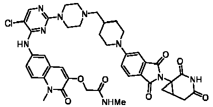 | 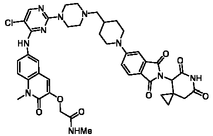 | 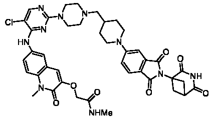 |
| 57 | 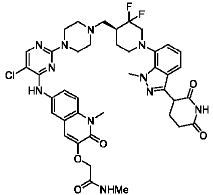 | 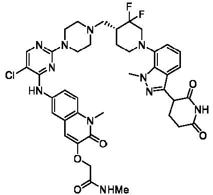 | 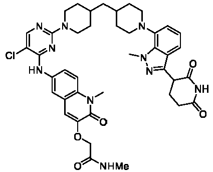 |
| 58 | 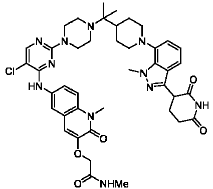 | 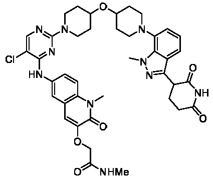 | 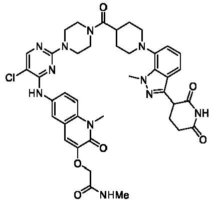 |
| 59 | 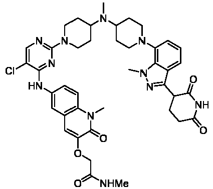 | 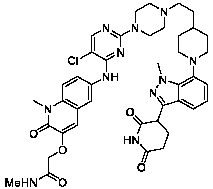 | 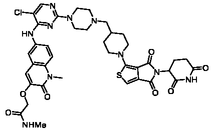 |
| 60 | 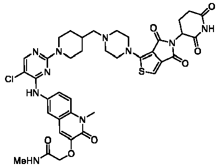 | 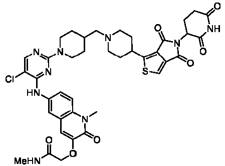 | 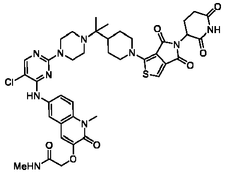 |
| 61 | 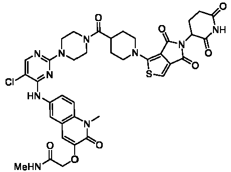 | 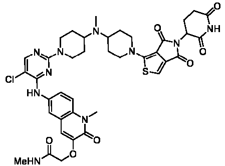 | 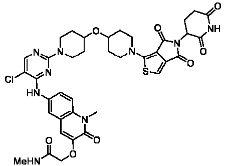 |
| 62 | 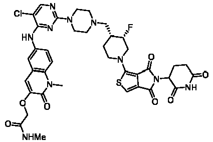 | 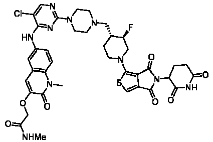 | 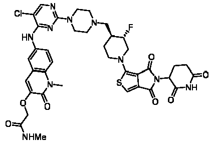 |
| 63 | 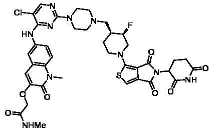 | 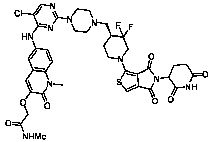 | 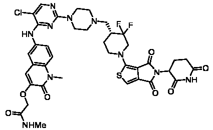 |
| 64 | 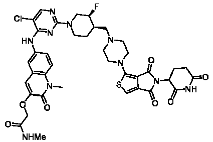 | 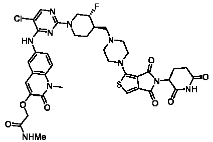 | 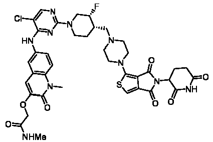 |
| 65 | 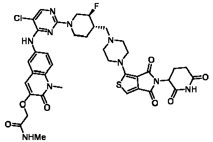 | 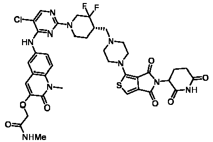 | 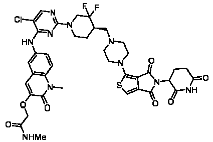 |
| 66 | 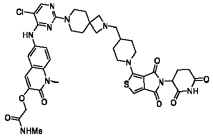 | 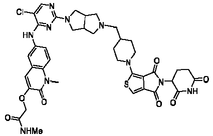 | 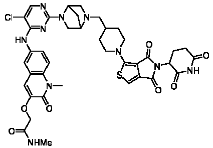 |
| 67 | 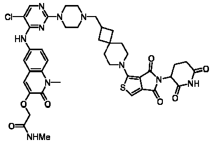 | 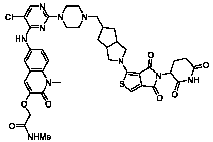 | 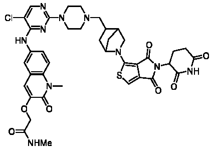 |
| 68 | 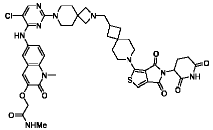 | 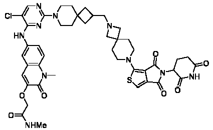 | 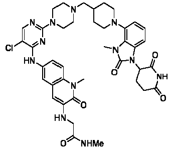 |
| 69 | 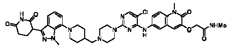 | 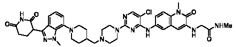 | 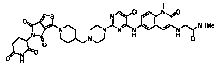 |
| 70 | 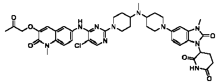 | 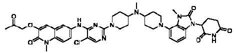 | 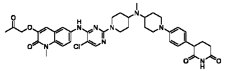 |
| 71 | 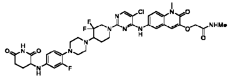 | 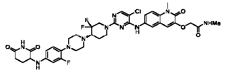 | 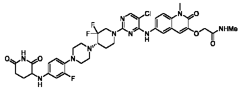 |
| 72 | 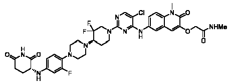 | 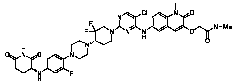 | 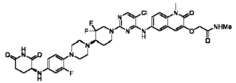 |
| 73 | 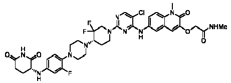 | 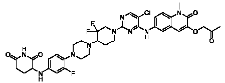 | 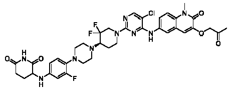 |
| 74 | 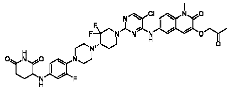 | 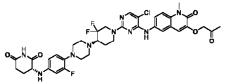 | 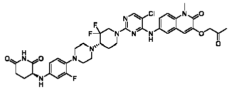 |
| 75 | 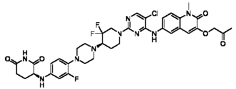 | 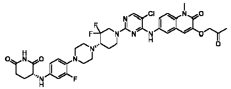 | 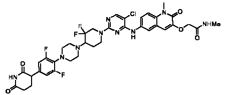 |
| 76 | 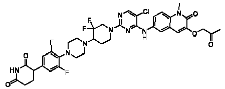 | 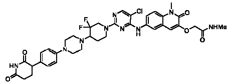 | 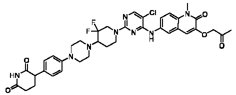 |
| 77 | 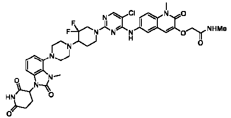 | 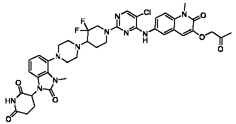 | 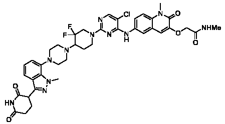 |
| 78 | 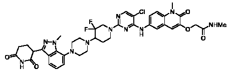 | 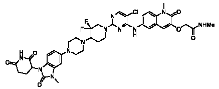 | 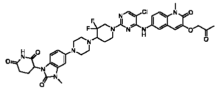 |
| 79 | 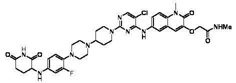 | 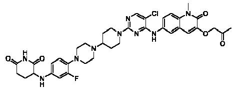 | 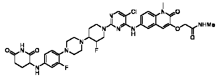 |
| 80 | 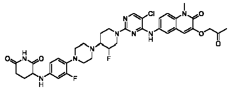 | 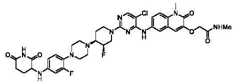 | 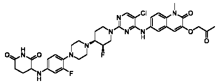 |
| 81 | 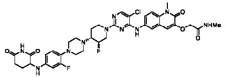 | 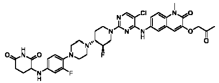 | 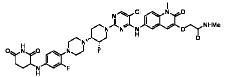 |
| 82 | 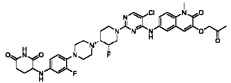 | 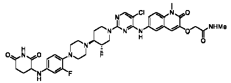 | 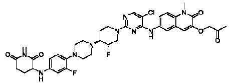 |
| 83 | 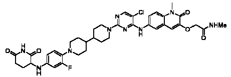 | 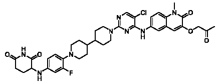 | 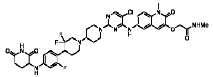 |
| 84 | 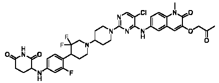 | 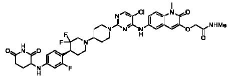 | 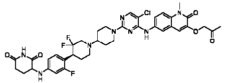 |
| 85 | 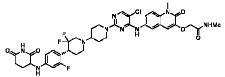 | 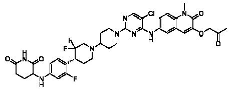 | 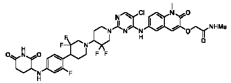 |
| 86 | 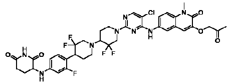 | 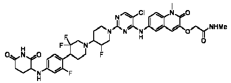 | 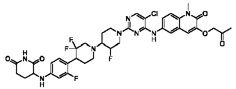 |
| 87 | 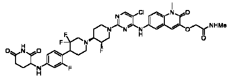 | 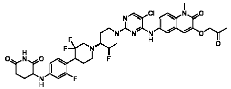 | 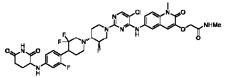 |
| 88 | 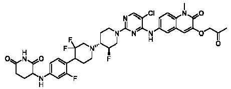 | 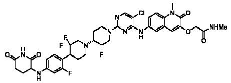 | 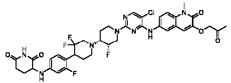 |
| 89 | 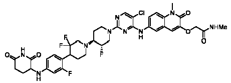 | 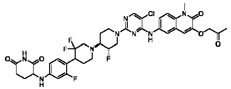 | 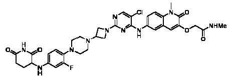 |
| 90 | 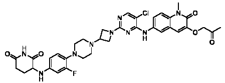 | 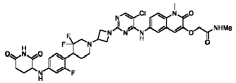 | 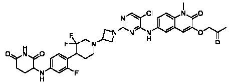 |
| 91 | 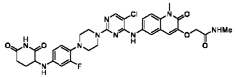 | 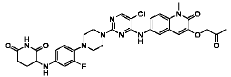 | 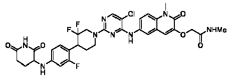 |
| 92 | 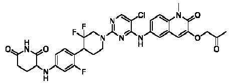 | 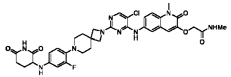 | 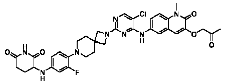 |
| 93 | 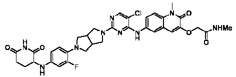 | 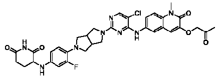 | 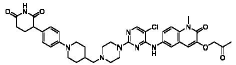 |
| 94 | 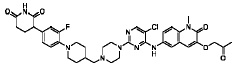 | 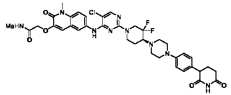 | 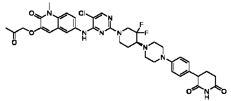 |
| 95 | 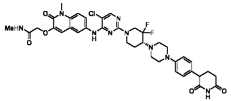 | 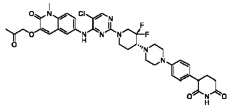 | 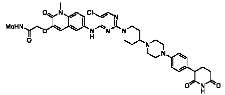 |
| 96 | 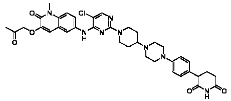 | 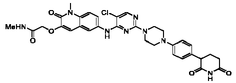 | 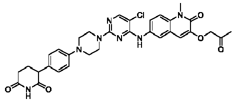 |
| 97 | 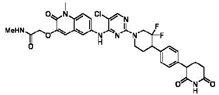 | 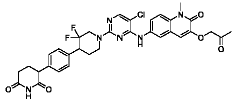 | 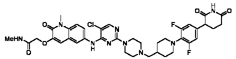 |
| 98 | 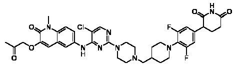 | 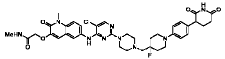 | 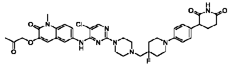 |
| 99 | 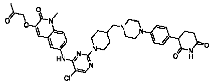 | 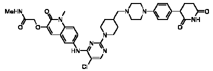 | 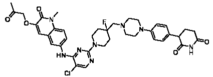 |
| 100 | 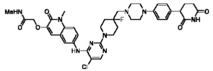 | 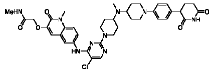 | 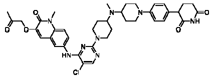 |
| 101 | 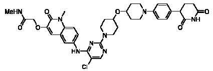 | 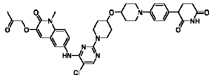 | 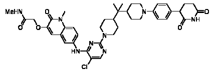 |
| 102 | 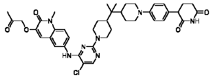 | 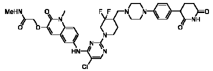 | 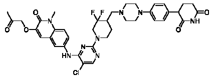 |
| 103 | 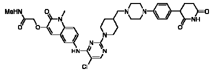 | 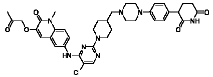 | 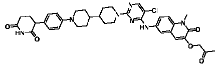 |
| 104 | 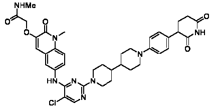 | 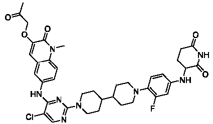 | 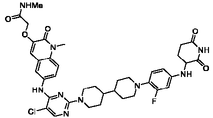 |
| 105 | 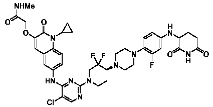 | 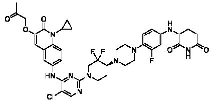 | 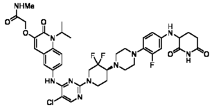 |
| 106 | 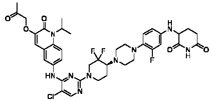 | 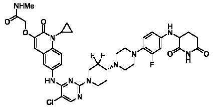 | 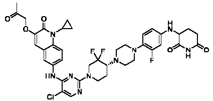 |
| 107 | 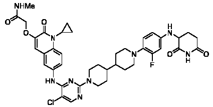 | 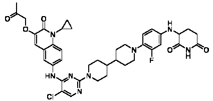 | 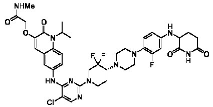 |
| 108 | 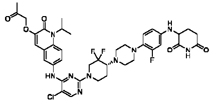 | 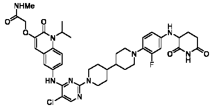 | 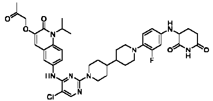 |
| 109 | 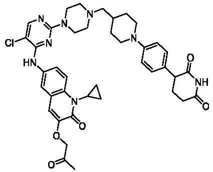 | 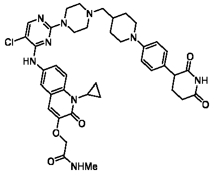 | 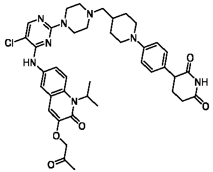 |
| 110 | 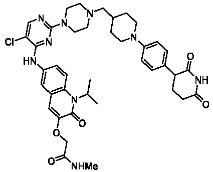 | 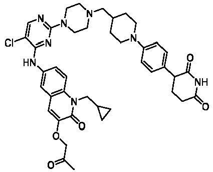 | 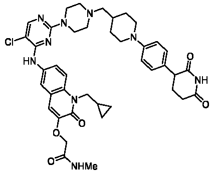 |
| 111 | 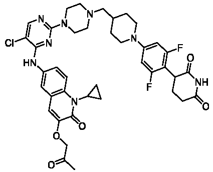 | 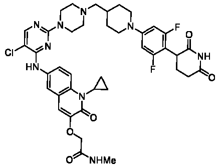 | 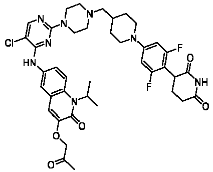 |
| 112 | 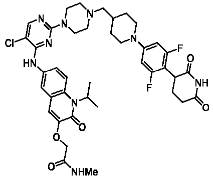 | 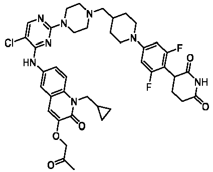 | 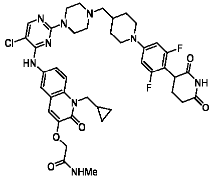 |
| 113 | 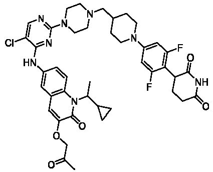 | 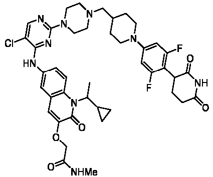 | 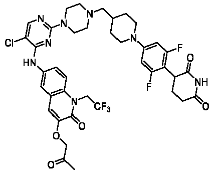 |
| 114 | 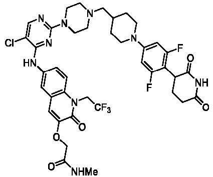 | 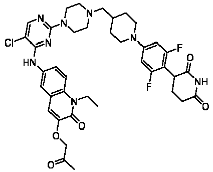 | 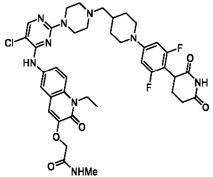 |
| 115 | 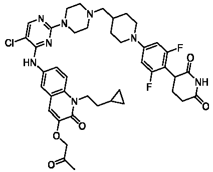 | 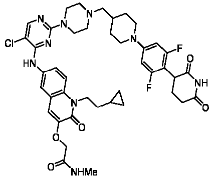 | 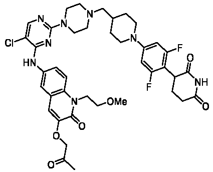 |
| 116 | 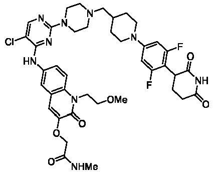 | 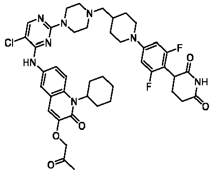 | 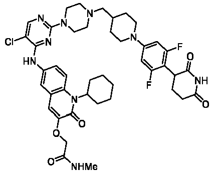 |
| 117 | 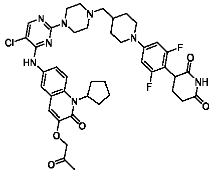 | 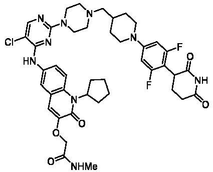 | 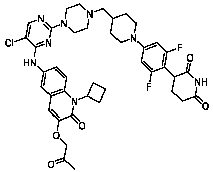 |
| 118 | 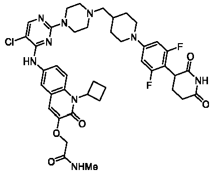 | 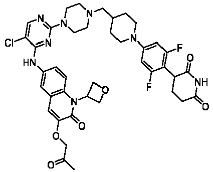 | 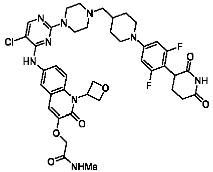 |
| 119 | 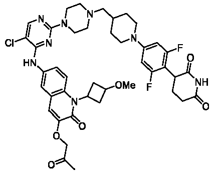 | 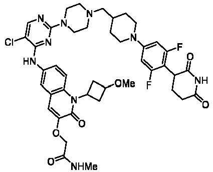 | 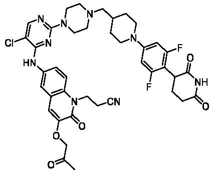 |
| 120 | 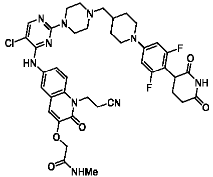 | 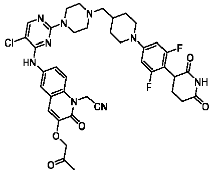 | 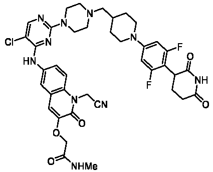 |
| 121 | 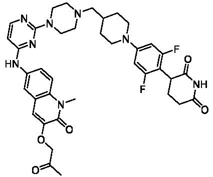 | 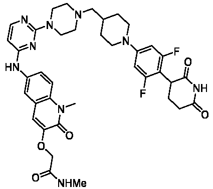 | 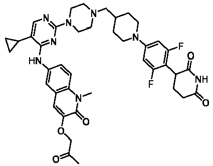 |
| 122 | 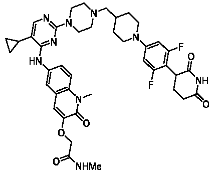 | 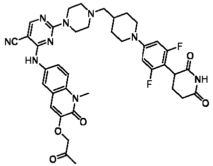 | 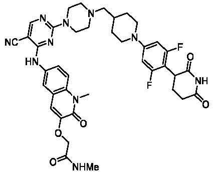 |
| 123 | 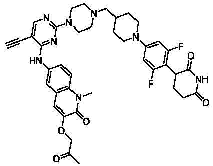 | 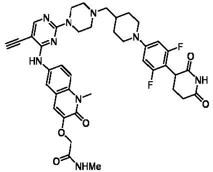 | 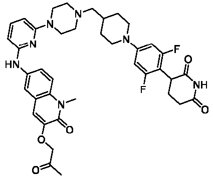 |
| 124 | 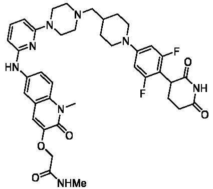 | 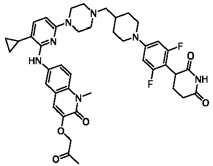 | 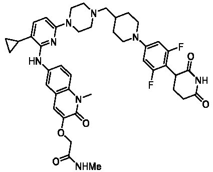 |
| 125 | 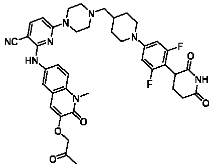 | 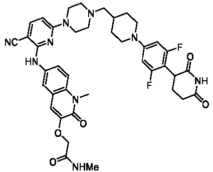 | 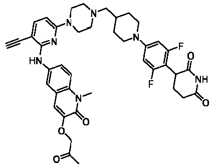 |
| 126 | 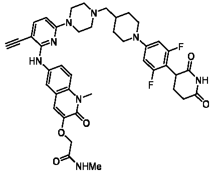 | 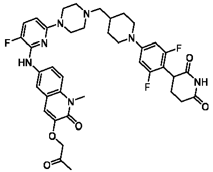 | 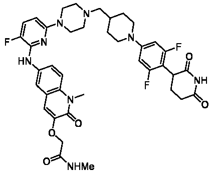 |
| 127 | 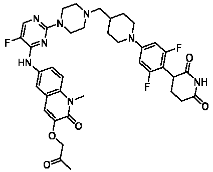 | 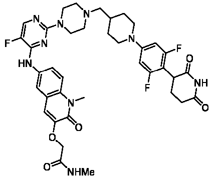 | 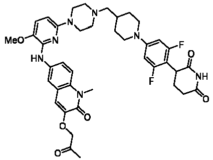 |
| 128 | 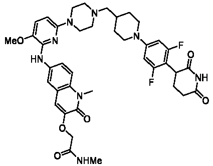 | 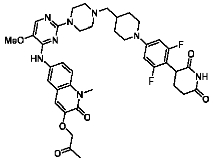 | 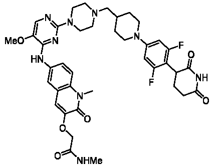 |
| 129 | 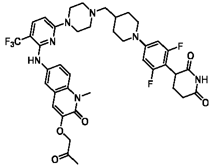 | 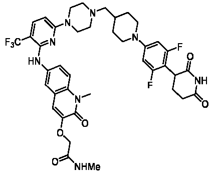 | 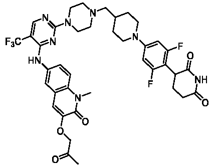 |
| 130 | 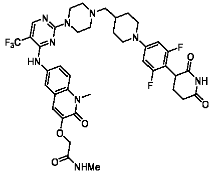 | 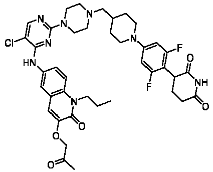 | 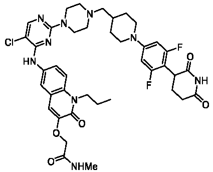 |
| 131 | 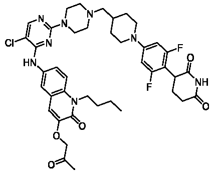 | 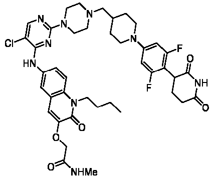 | 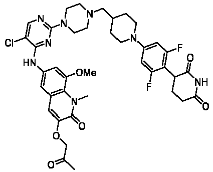 |
| 132 | 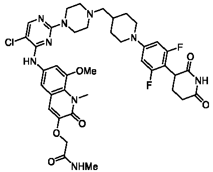 | 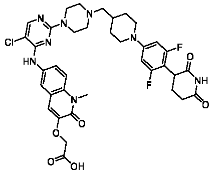 | 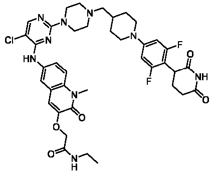 |
| 133 | 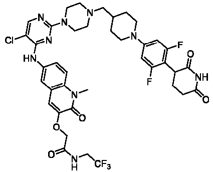 | 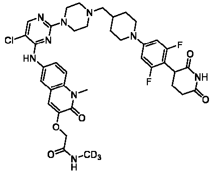 | 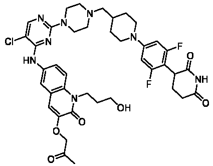 |
| 134 | 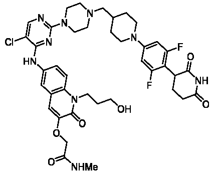 | 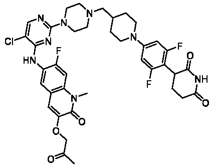 | 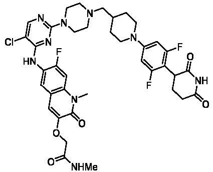 |
| 135 | 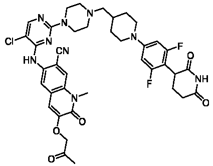 | 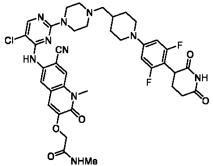 | 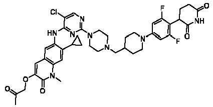 |
| 136 | 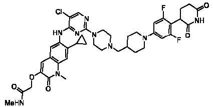 | 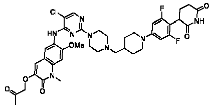 | 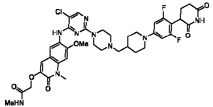 |
| 137 | 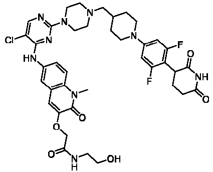 | 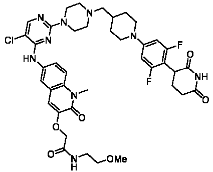 | 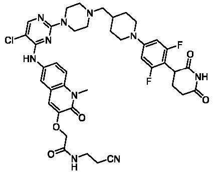 |
| 138 | 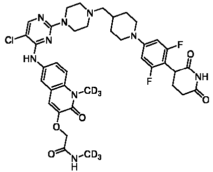 | 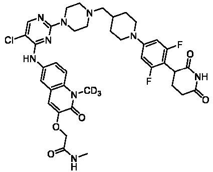 | 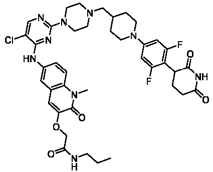 |
| 139 | 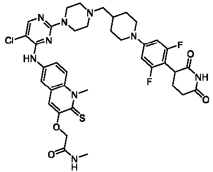 | 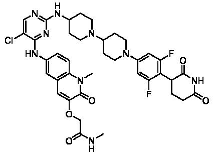 | 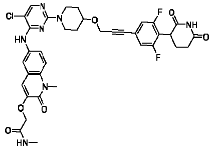 |
| 140 | 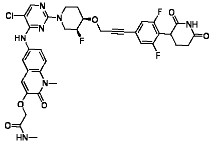 | 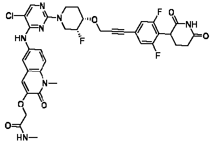 | 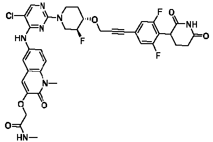 |
| 141 | 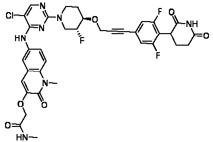 | 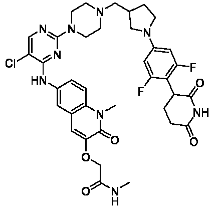 | 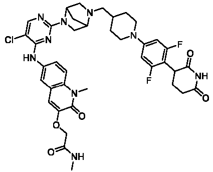 |
| 142 | 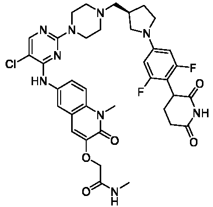 | 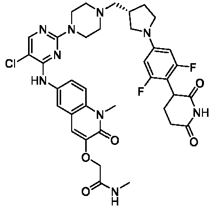 | 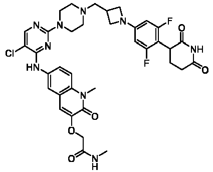 |
| 143 | 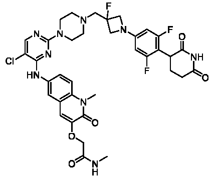 | 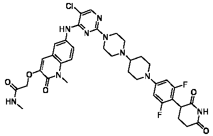 | 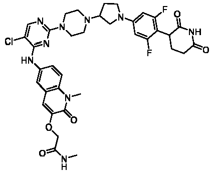 |
| 144 | 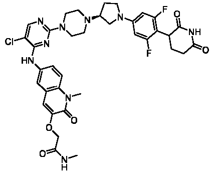 | 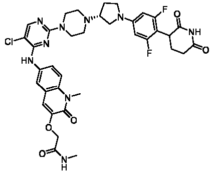 | 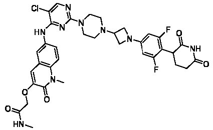 |
| 145 | 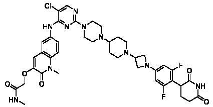 | 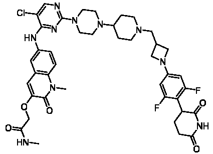 | 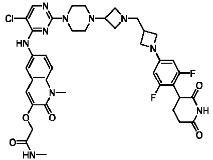 |
| 146 | 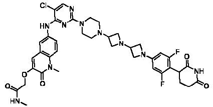 | 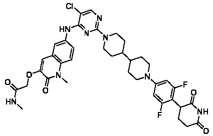 | 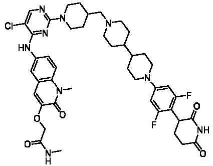 |
| 147 | 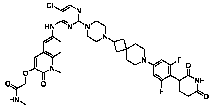 | 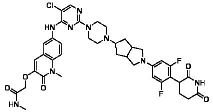 | 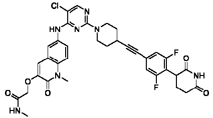 |
| 148 | 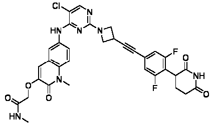 | 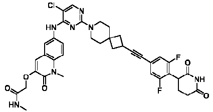 | 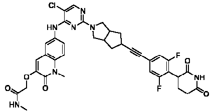 |
| 149 | 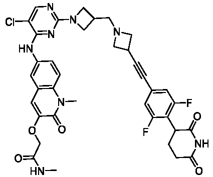 | 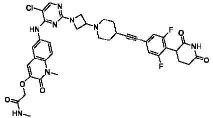 | 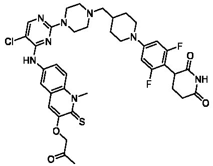 |
| 150 | 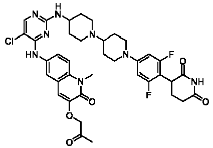 | 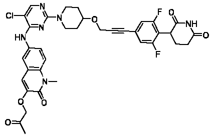 | 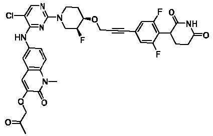 |
| 151 | 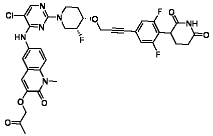 | 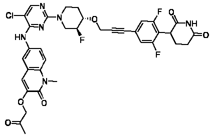 | 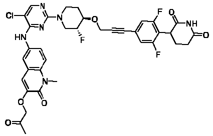 |
| 152 | 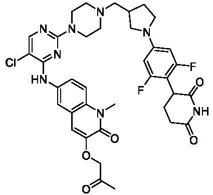 | 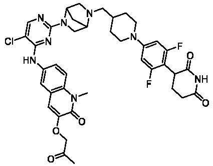 | 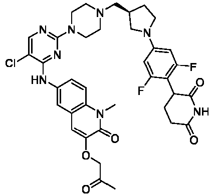 |
| 153 | 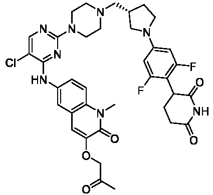 | 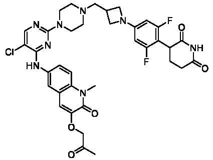 | 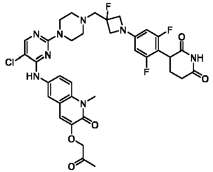 |
| 154 | 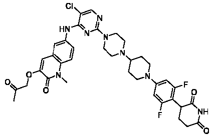 | 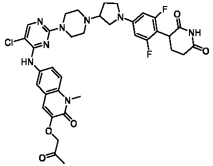 | 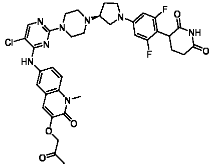 |
| 155 | 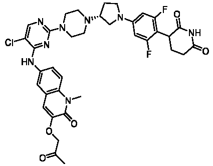 | 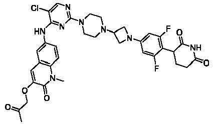 | 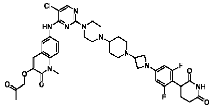 |
| 156 | 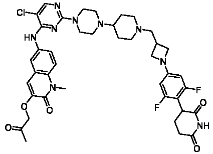 | 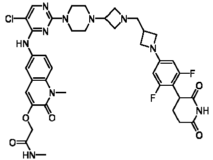 | 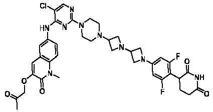 |
| 157 | 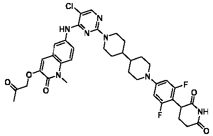 | 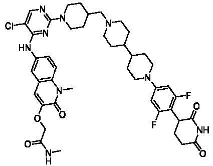 | 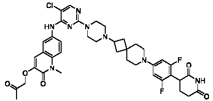 |
| 158 | 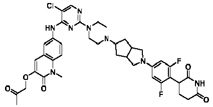 | 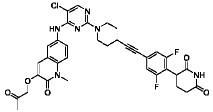 | 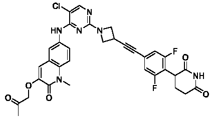 |
| 159 | 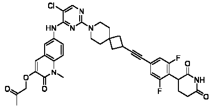 | 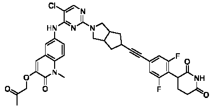 | 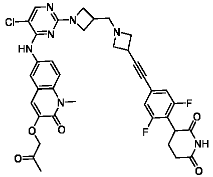 |
| 160 | 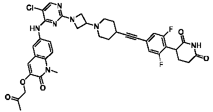 | 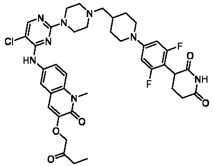 | 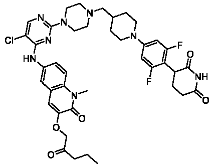 |
| 161 | 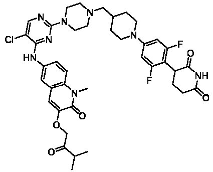 | 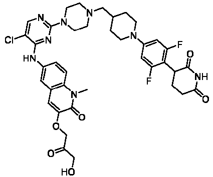 | 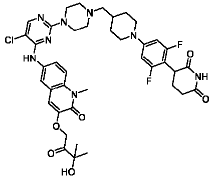 |
| 162 | 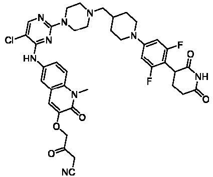 | 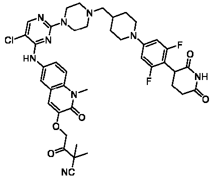 | 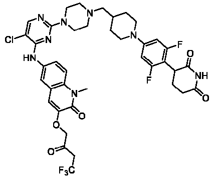 |
| 163 | 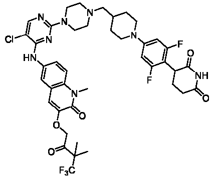 | 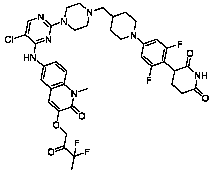 | 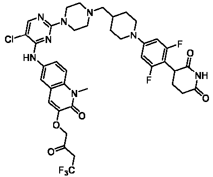 |
| 164 | 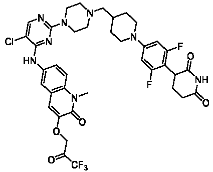 | 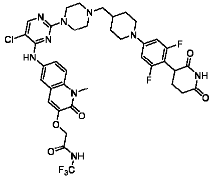 | 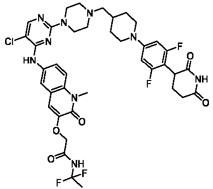 |
| 165 | 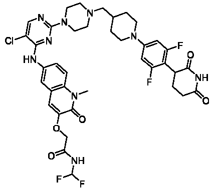 | 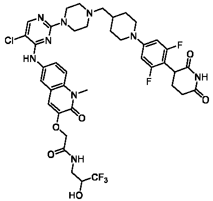 | 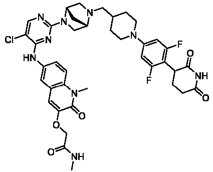 |
| 166 | 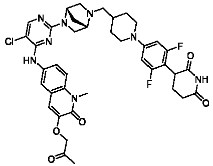 | 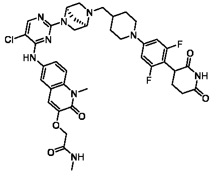 | 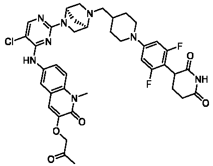 |
| 167 | 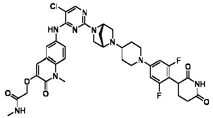 | 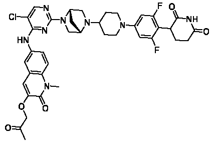 | 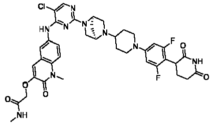 |
| 168 | 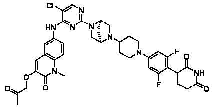 | 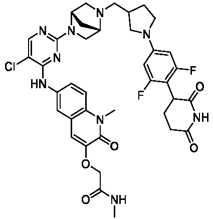 | 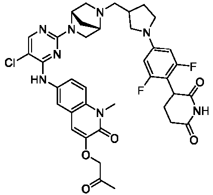 |
| 169 | 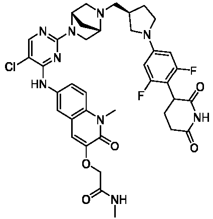 | 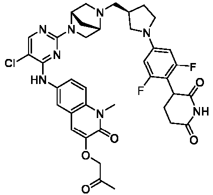 | 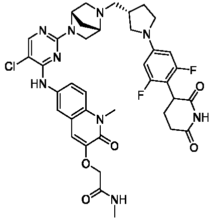 |
| 170 | 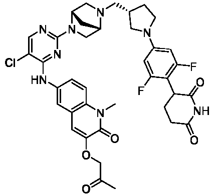 | 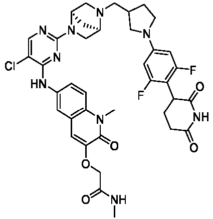 | 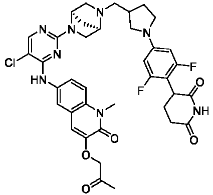 |
| 171 | 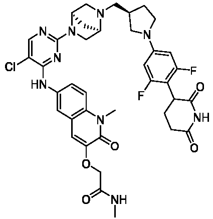 | 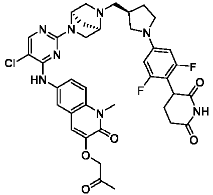 | 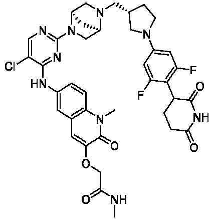 |
| 172 | 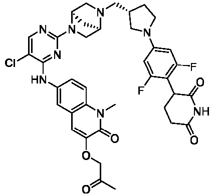 | 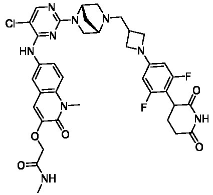 | 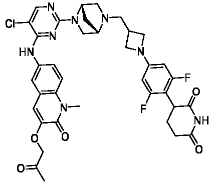 |
| 173 | 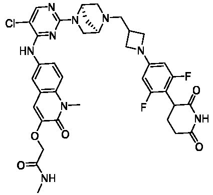 | 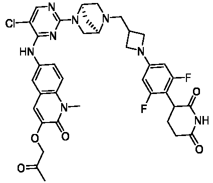 | 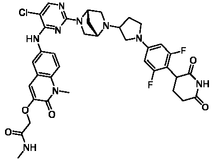 |
| 174 | 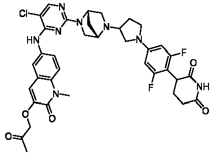 | 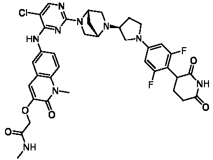 | 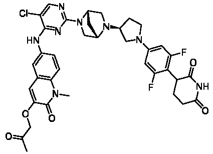 |
| 175 | 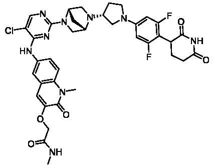 | 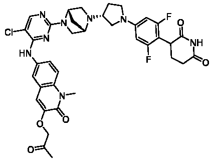 | 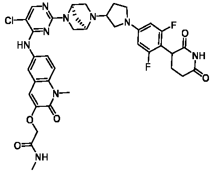 |
| 176 | 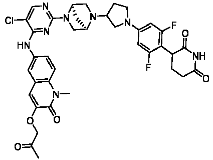 | 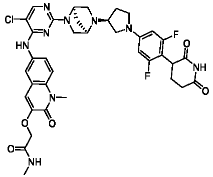 | 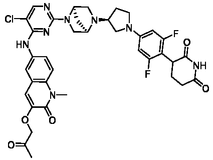 |
| 177 | 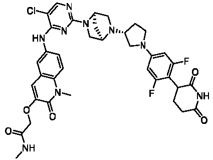 | 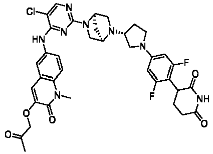 | 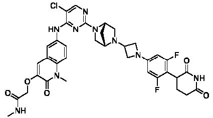 |
| 178 | 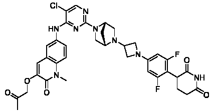 | 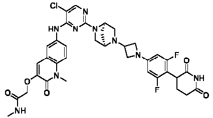 | 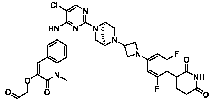 |
| 179 | 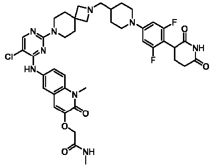 | 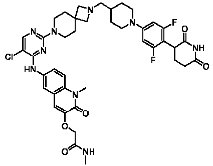 | 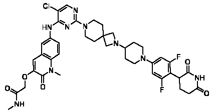 |
| 180 | 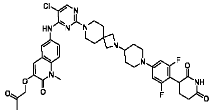 | 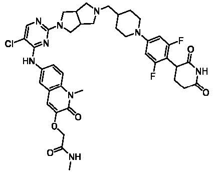 | 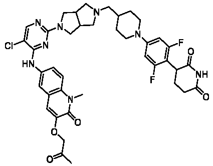 |
| 181 | 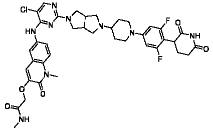 | 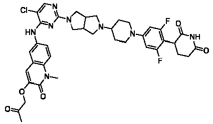 | 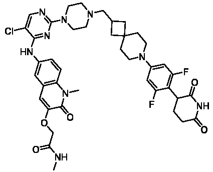 |
| 182 | 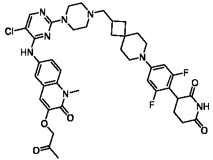 | 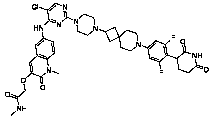 | 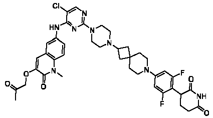 |
| 183 | 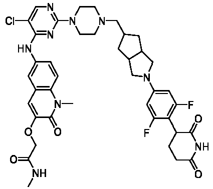 | 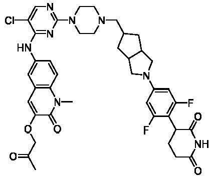 | 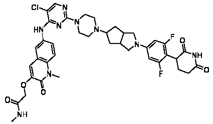 |
| 184 | 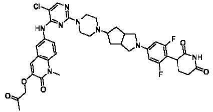 | 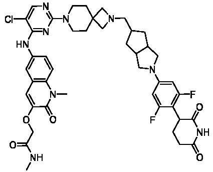 | 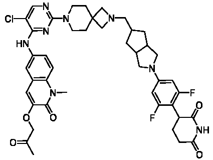 |
| 185 | 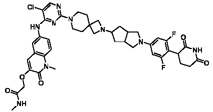 | 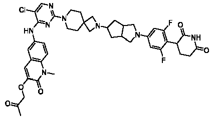 | 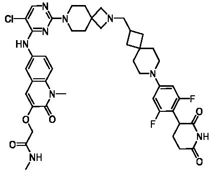 |
| 186 | 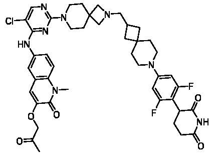 | 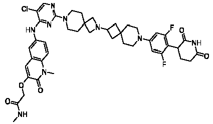 | 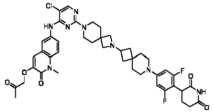 |
| 187 | 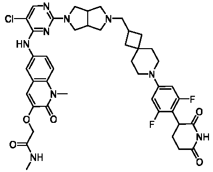 | 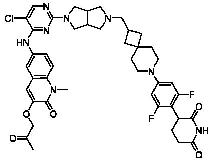 | 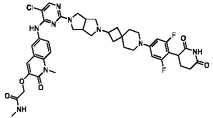 |
| 188 | 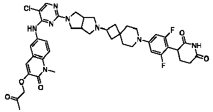 | 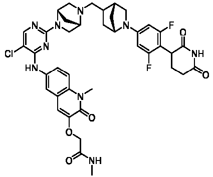 | 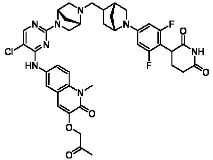 |
| 189 | 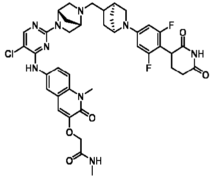 | 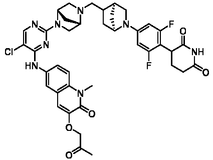 | 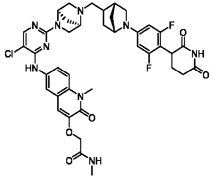 |
| 190 | 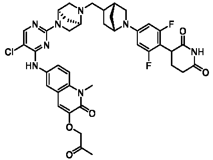 | 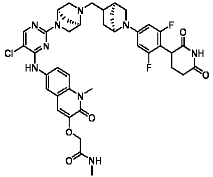 | 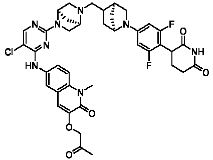 |
| 191 | 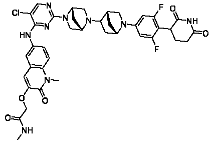 | 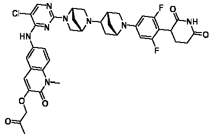 | 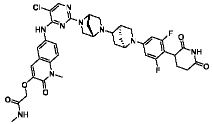 |
| 192 | 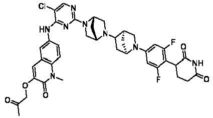 | 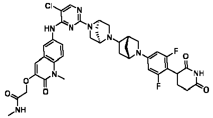 | 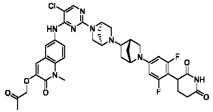 |
| 193 | 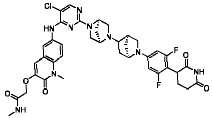 | 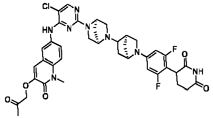 | 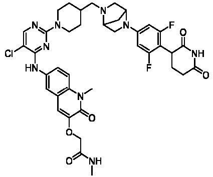 |
| 194 | 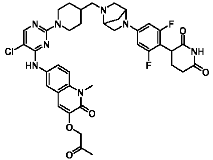 | 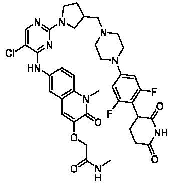 | 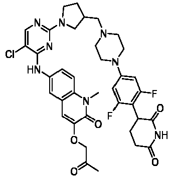 |
| 195 | 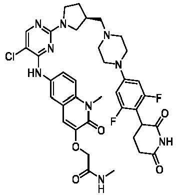 | 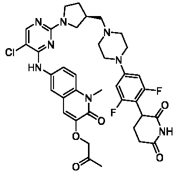 | 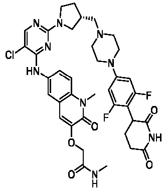 |
| 196 | 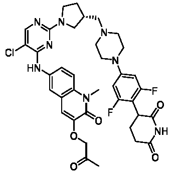 | 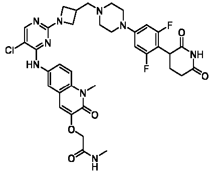 | 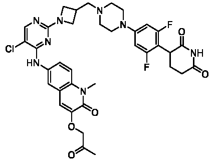 |
| 197 | 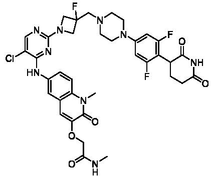 | 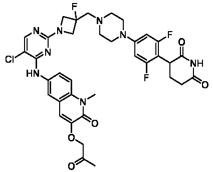 | 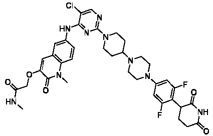 |
| 198 | 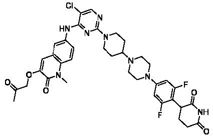 | 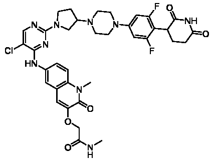 | 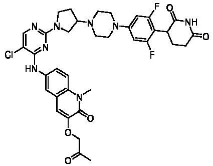 |
| 199 | 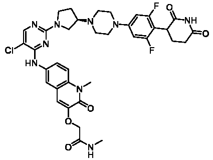 | 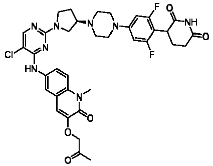 | 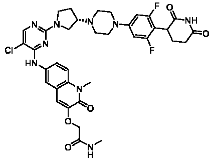 |
| 200 | 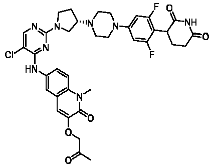 | 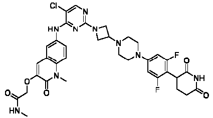 | 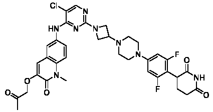 |
| 201 | 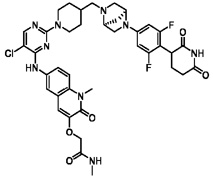 | 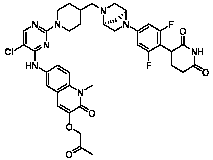 | 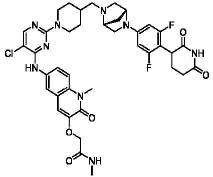 |
| 202 | 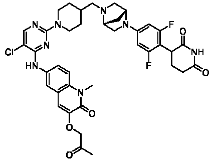 | 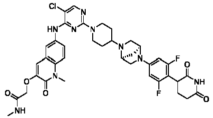 | 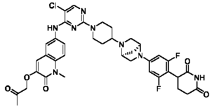 |
| 203 | 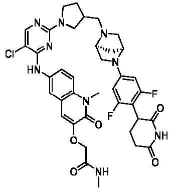 | 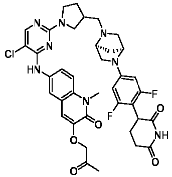 | 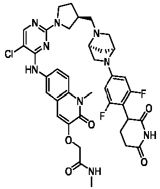 |
| 204 | 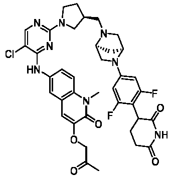 | 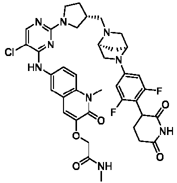 | 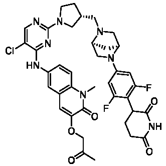 |
| 205 | 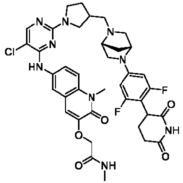 | 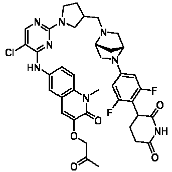 | 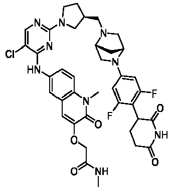 |
| 206 | 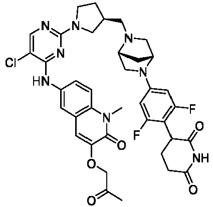 | 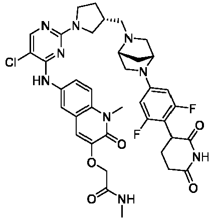 | 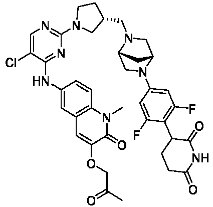 |
| 207 | 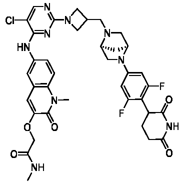 | 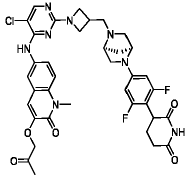 | 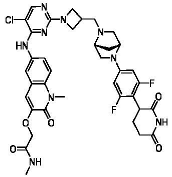 |
| 208 | 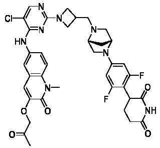 | 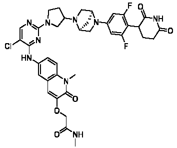 | 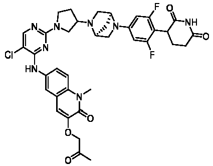 |
| 209 | 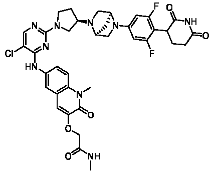 | 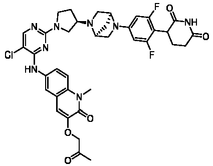 | 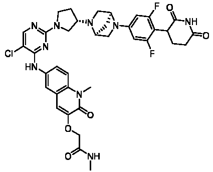 |
| 210 | 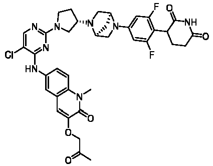 | 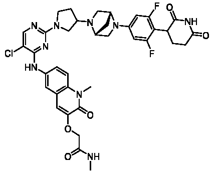 | 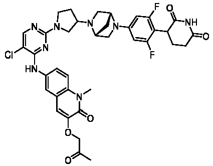 |
| 211 | 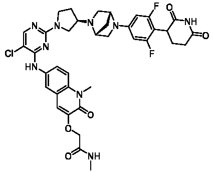 | 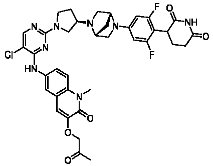 | 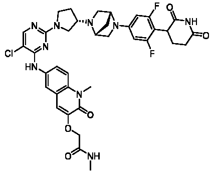 |
| 212 | 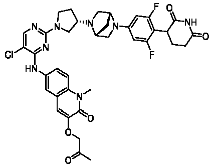 | 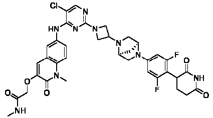 | 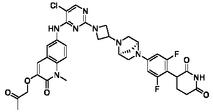 |
| 213 | 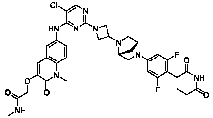 | 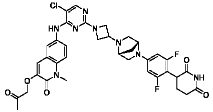 | 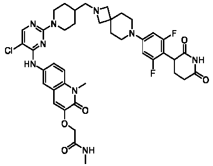 |
| 214 | 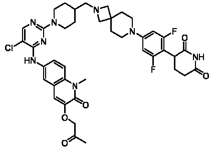 | 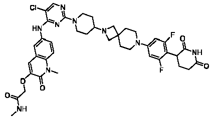 | 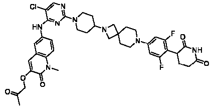 |
| 215 | 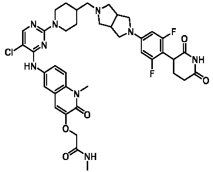 | 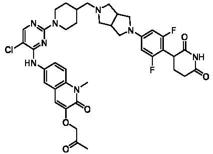 | 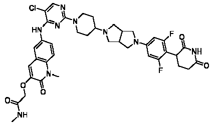 |
| 216 | 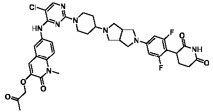 | 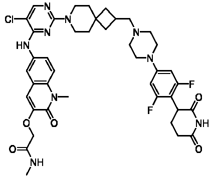 | 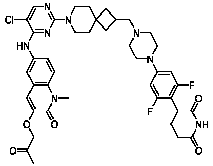 |
| 217 | 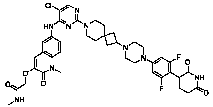 | 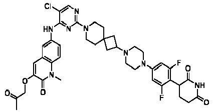 | 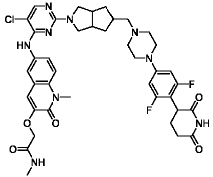 |
| 218 | 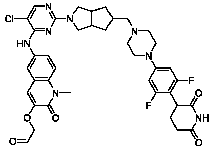 | 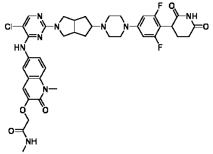 | 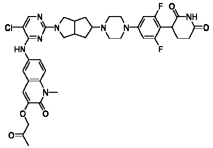 |
| 219 | 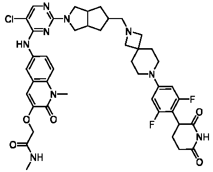 | 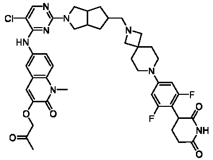 | 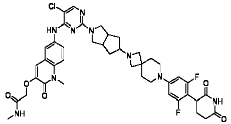 |
| 220 | 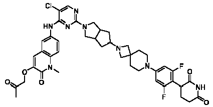 | 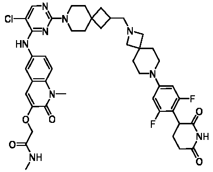 | 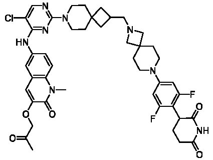 |
| 221 | 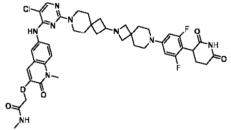 | 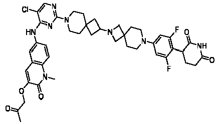 | 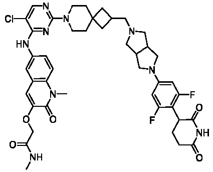 |
| 222 | 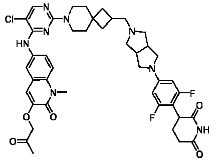 | 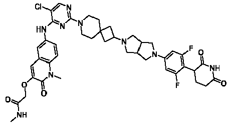 | 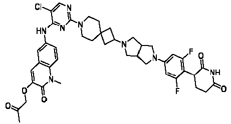 |
| 223 | 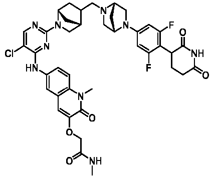 | 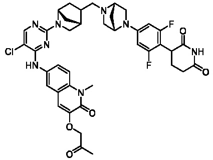 | 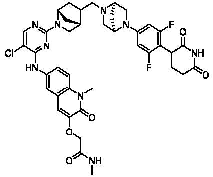 |
| 224 | 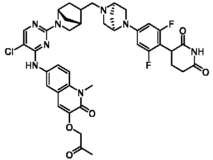 | 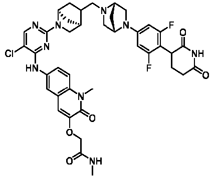 | 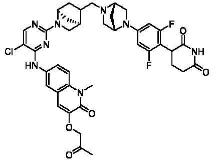 |
| 225 | 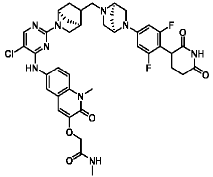 | 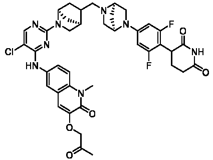 | 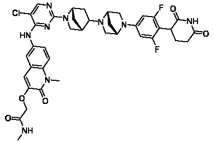 |
| 226 | 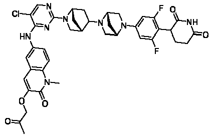 | 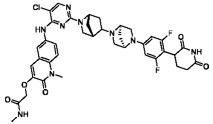 | 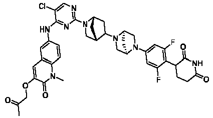 |
| 227 | 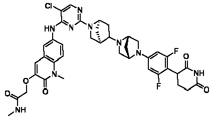 | 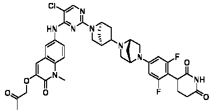 | 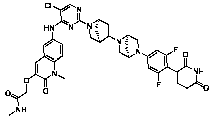 |
| 228 | 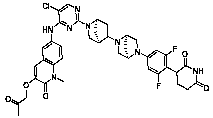 | 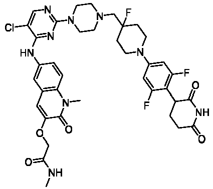 | 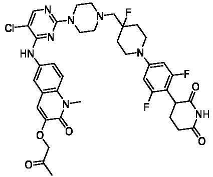 |
| 229 | 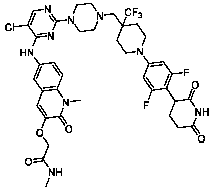 | 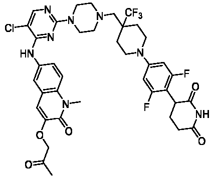 | 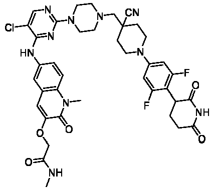 |
| 230 | 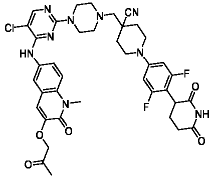 | 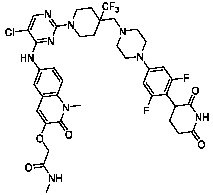 | 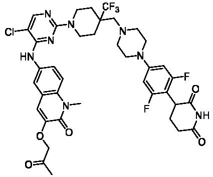 |
| 231 | 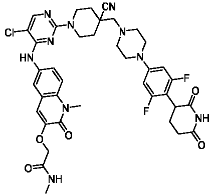 | 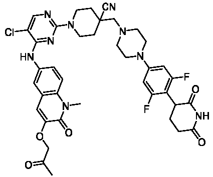 | 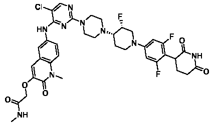 |
| 232 | 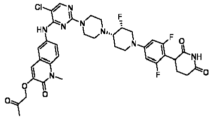 | 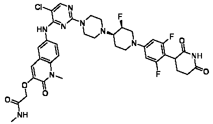 | 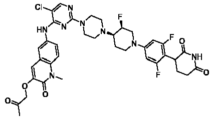 |
| 233 | 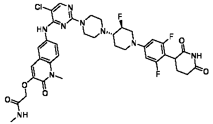 | 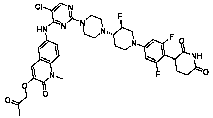 | 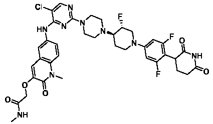 |
| 234 | 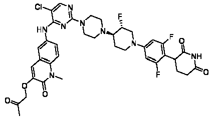 | 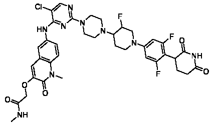 | 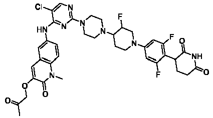 |
| 235 | 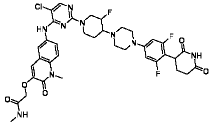 | 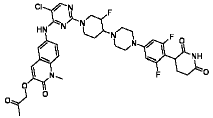 | 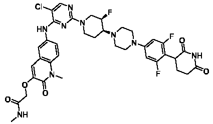 |
| 236 | 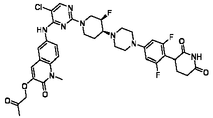 | 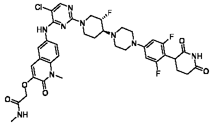 | 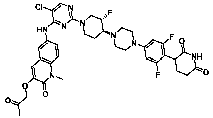 |
| 237 | 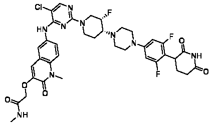 | 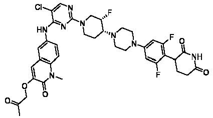 | 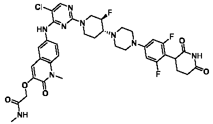 |
| 238 | 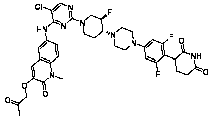 | 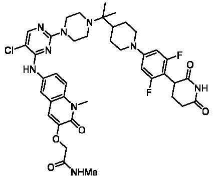 | 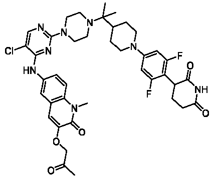 |
| 239 | 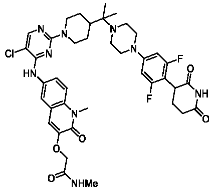 | 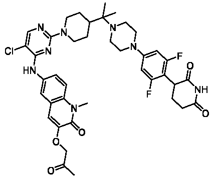 | 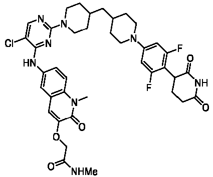 |
| 240 | 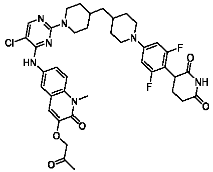 | 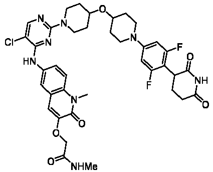 | 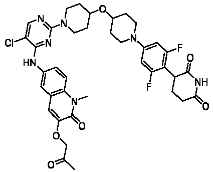 |
| 241 | 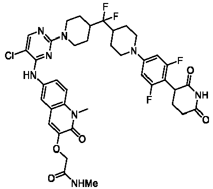 | 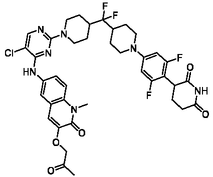 | 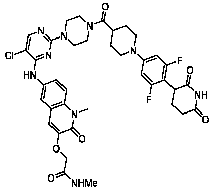 |
| 242 | 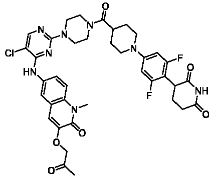 | 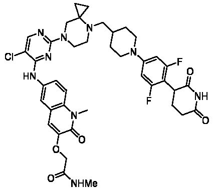 | 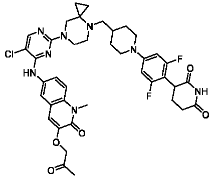 |
| 243 | 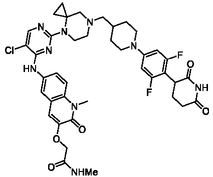 | 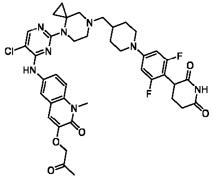 | 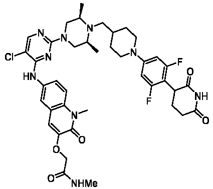 |
| 244 | 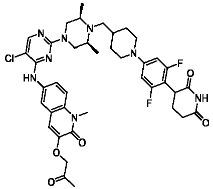 | 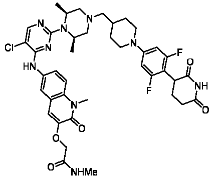 | 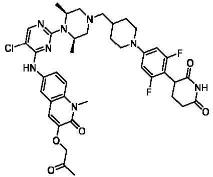 |
| 245 | 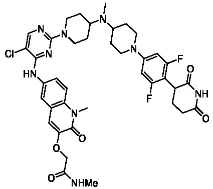 | 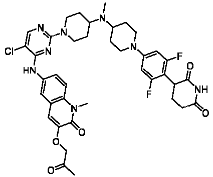 | 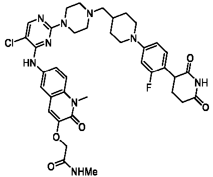 |
| 246 | 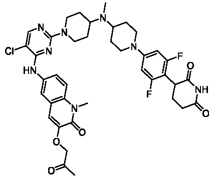 | 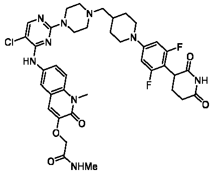 | 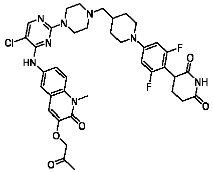 |
| 247 | 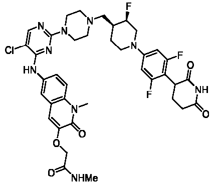 | 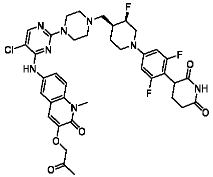 | 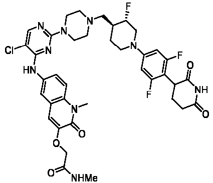 |
| 248 | 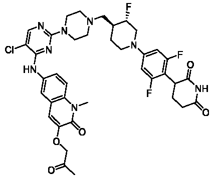 | 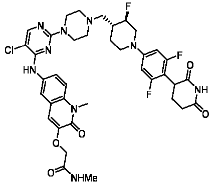 | 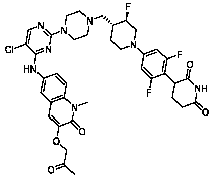 |
| 249 | 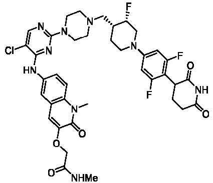 | 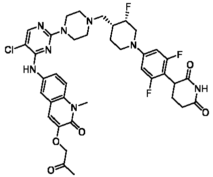 | 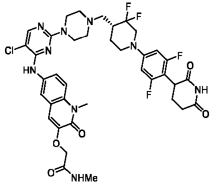 |
| 250 | 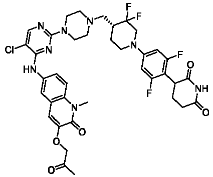 | 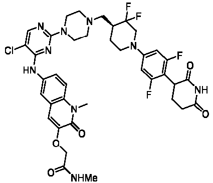 | 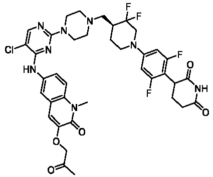 |
| 251 | 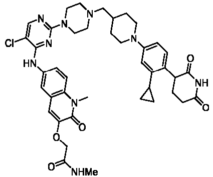 | 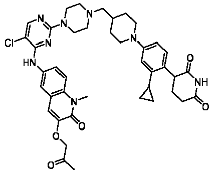 | 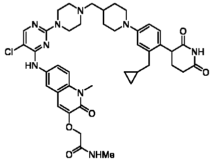 |
| 252 | 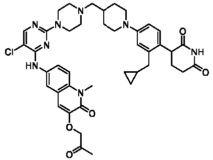 | 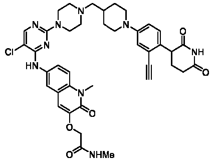 | 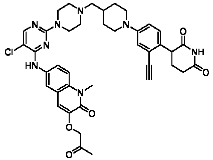 |
| 253 | 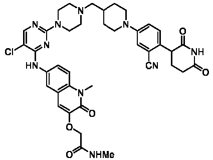 | 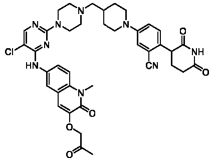 | 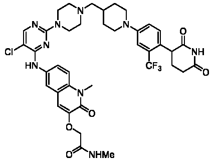 |
| 254 | 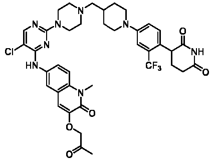 | 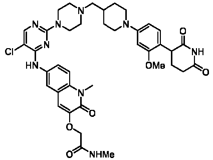 | 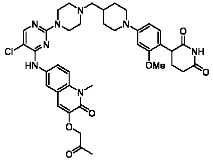 |
| 255 | 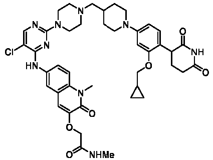 | 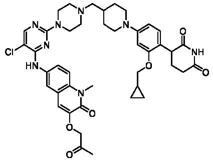 | 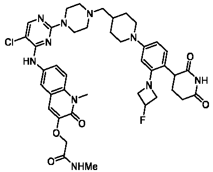 |
| 256 | 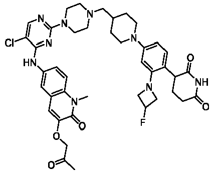 | 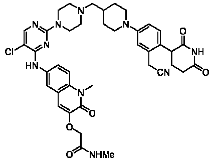 | 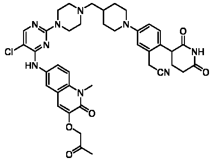 |
| 257 | 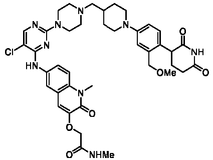 | 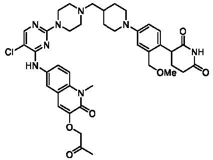 | 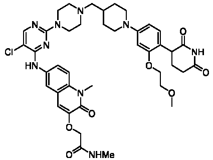 |
| 258 | 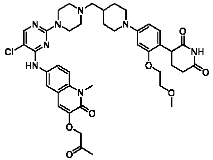 | 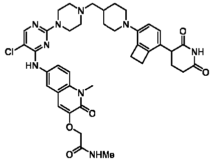 | 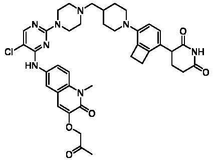 |
| 259 | 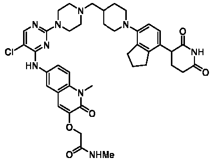 | 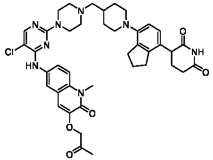 | 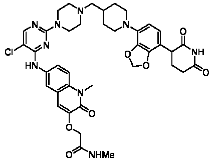 |
| 260 | 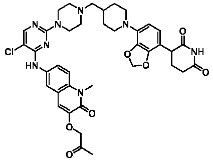 | 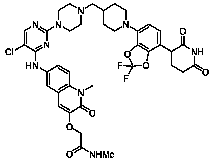 | 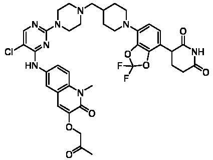 |
| 261 | 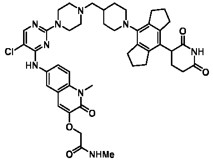 | 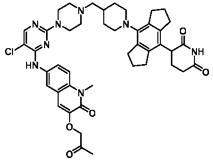 | 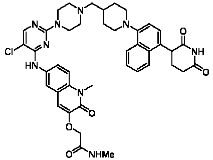 |
| 262 | 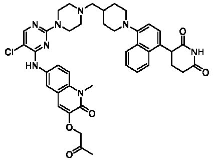 | 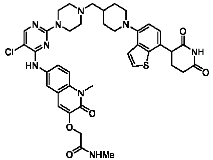 | 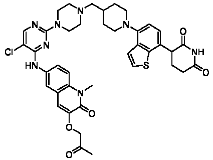 |
| 263 | 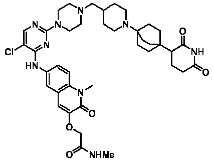 | 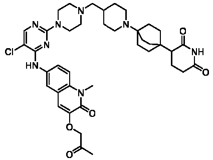 | 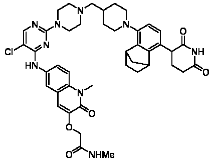 |
| 264 | 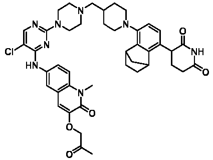 | 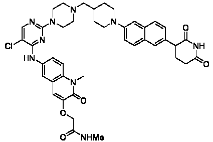 | 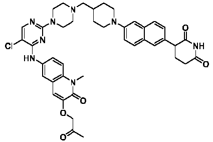 |
| 265 | 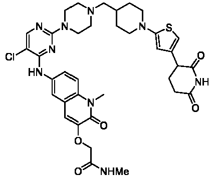 | 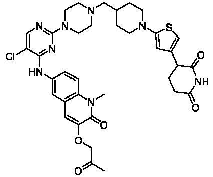 | 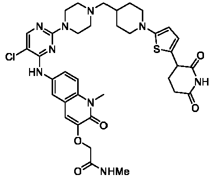 |
| 266 | 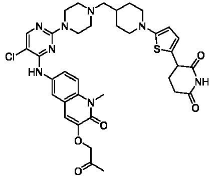 | 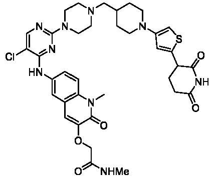 | 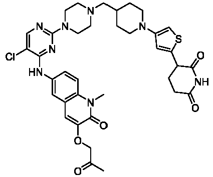 |
| 267 | 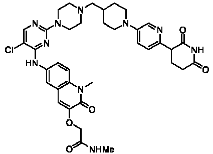 | 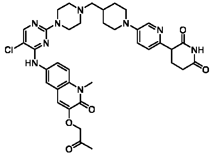 | 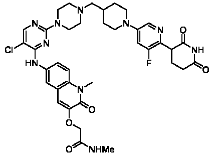 |
| 268 | 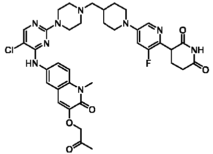 | 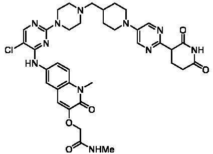 | 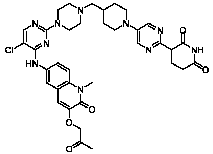 |
| 269 | 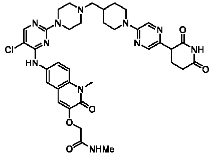 | 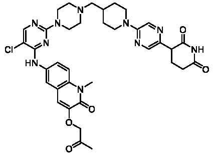 | 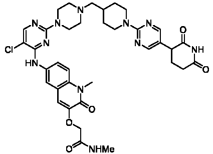 |
| 270 | 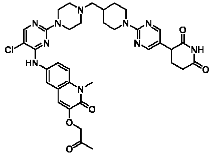 | 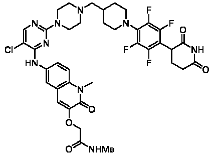 | 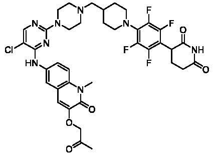 |
| 271 | 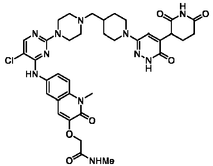 | 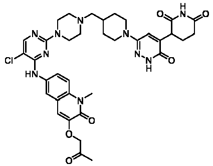 | 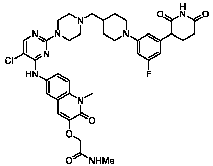 |
| 272 | 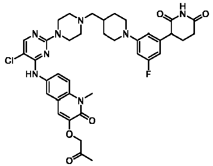 | 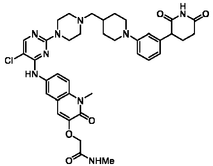 | 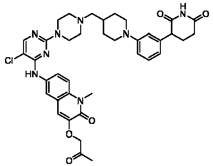 |
| 273 | 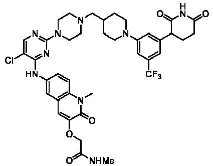 | 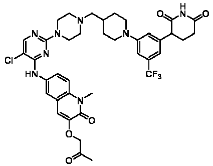 | 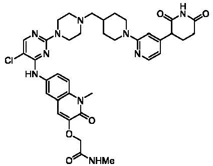 |
| 274 | 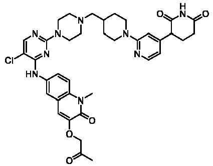 | 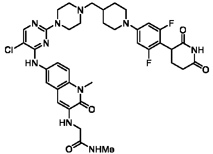 | 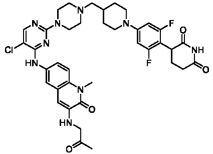 |
| 275 | 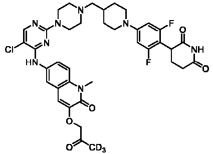 | 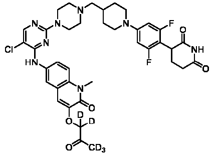 | 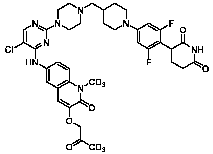 |
| 276 | 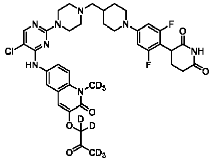 | 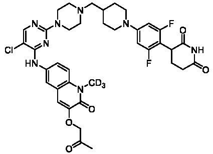 | 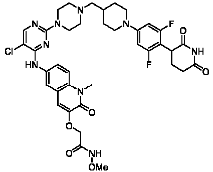 |
| 277 | 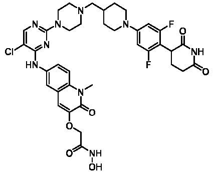 | 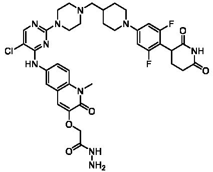 | 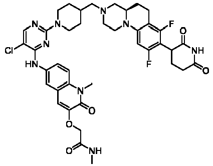 |
| 278 | 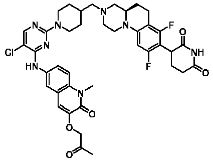 | 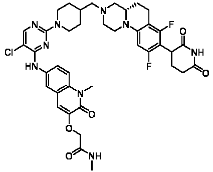 | 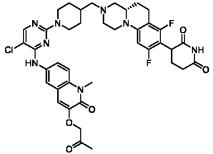 |
| 279 | 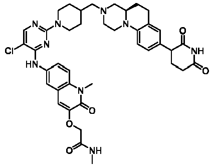 | 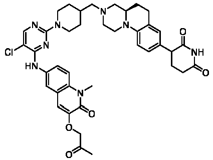 | 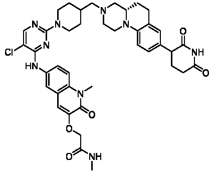 |
| 280 | 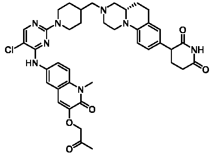 | 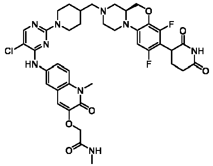 | 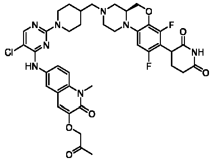 |
| 281 | 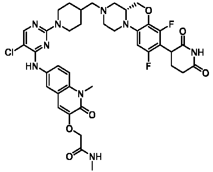 | 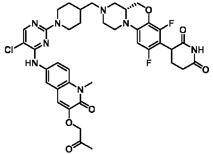 | 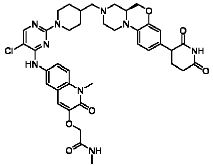 |
| 282 | 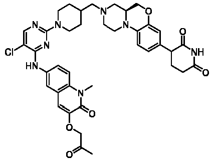 | 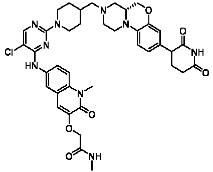 | 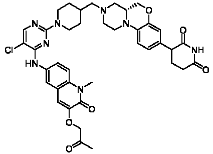 |
| 283 | 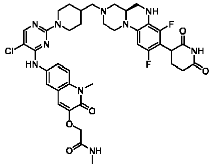 | 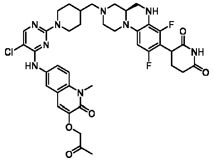 | 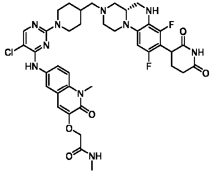 |
| 284 | 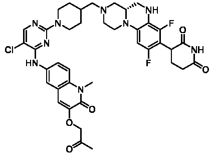 | 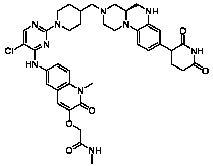 | 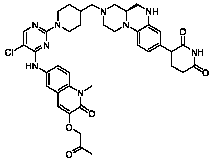 |
| 285 | 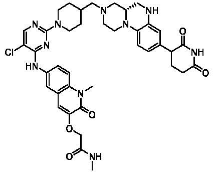 | 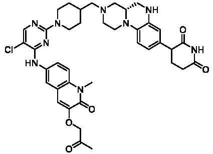 | 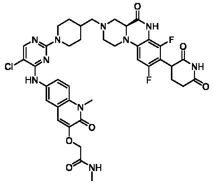 |
| 286 | 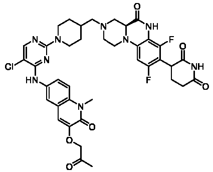 | 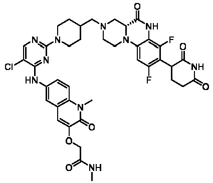 | 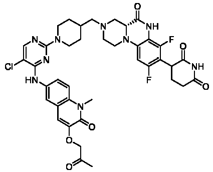 |
| 287 | 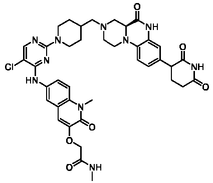 | 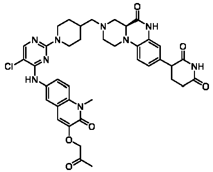 | 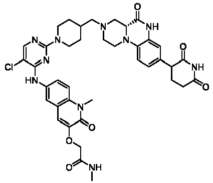 |
| 288 | 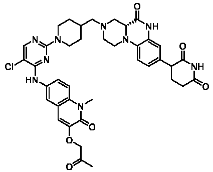 | 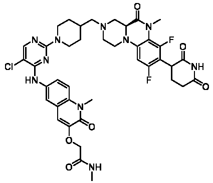 | 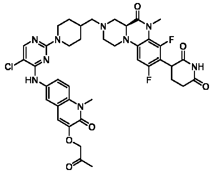 |
| 289 | 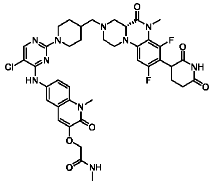 | 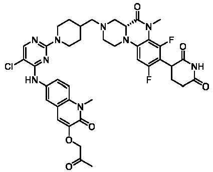 | 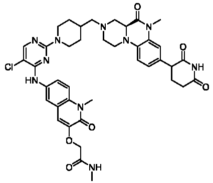 |
| 290 | 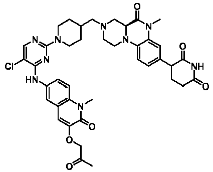 | 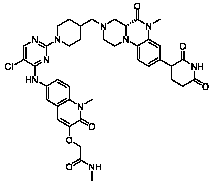 | 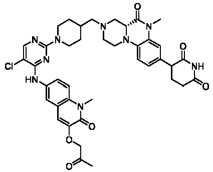 |
| 291 | 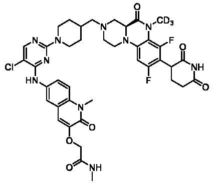 | 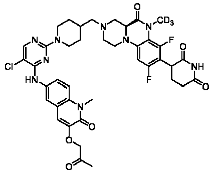 | 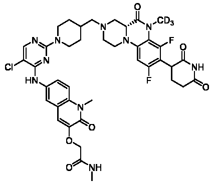 |
| 292 | 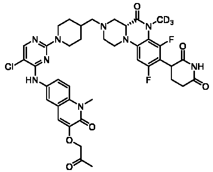 | 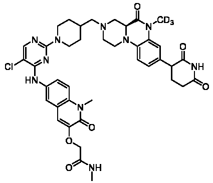 | 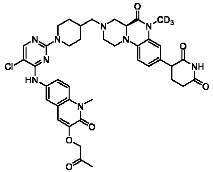 |
| 293 | 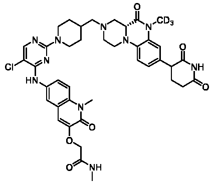 | 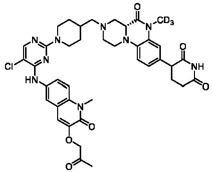 | 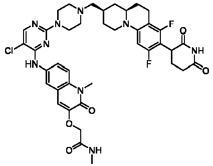 |
| 294 | 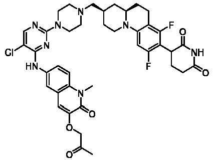 | 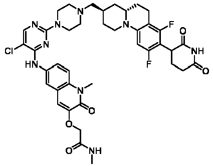 | 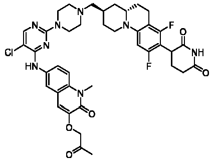 |
| 295 | 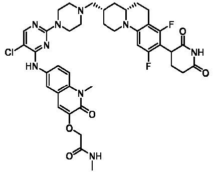 | 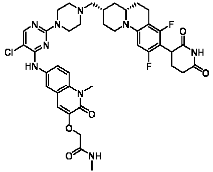 | 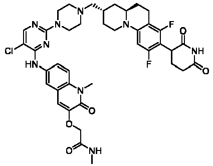 |
| 296 | 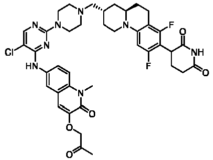 | 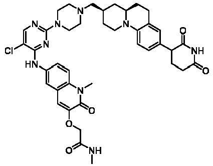 | 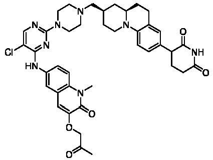 |
| 297 | 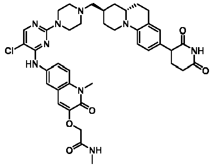 | 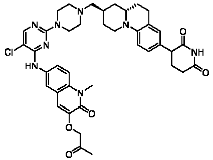 | 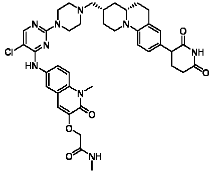 |
| 298 | 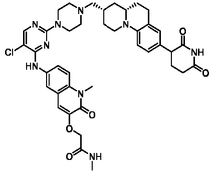 | 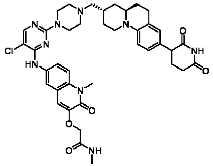 | 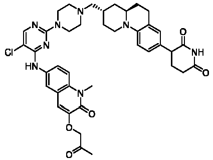 |
| 299 | 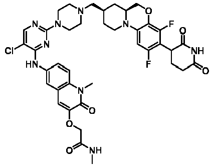 | 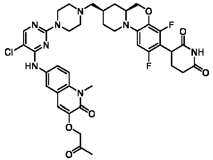 | 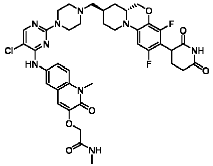 |
| 300 | 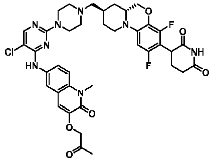 | 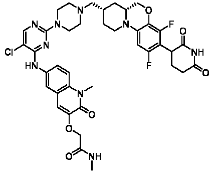 | 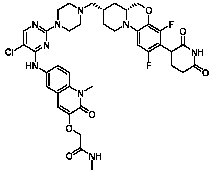 |
| 301 | 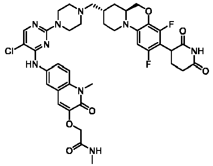 | 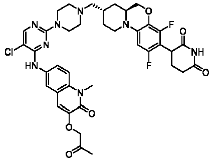 | 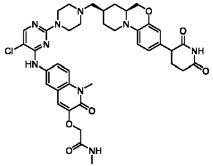 |
| 302 | 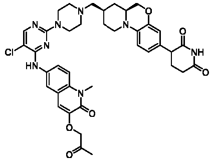 | 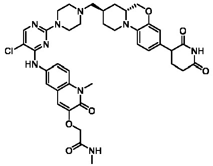 | 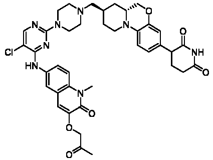 |
| 303 | 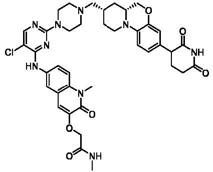 | 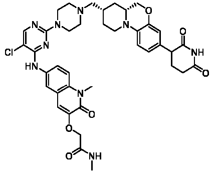 | 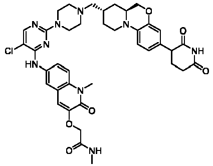 |
| 304 | 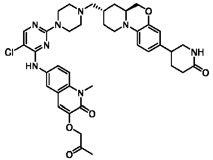 | 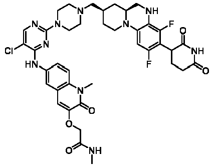 | 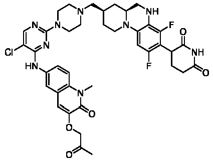 |
| 305 | 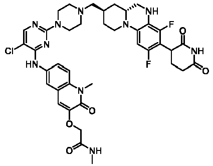 | 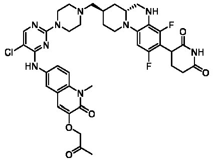 | 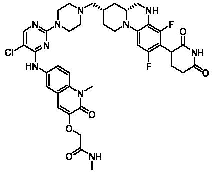 |
| 306 | 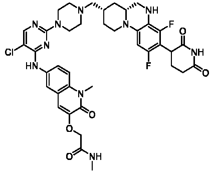 | 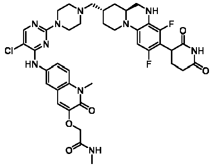 | 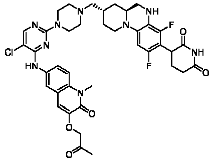 |
| 307 | 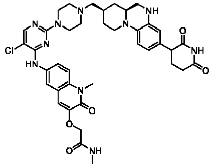 | 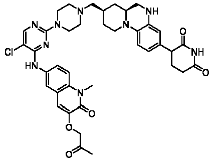 | 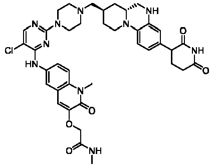 |
| 308 | 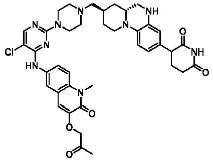 | 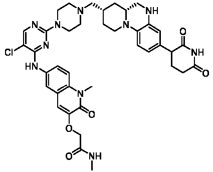 | 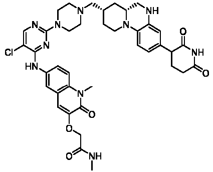 |
| 309 | 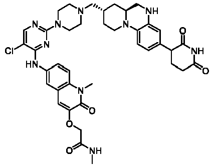 | 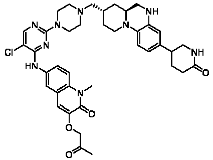 | 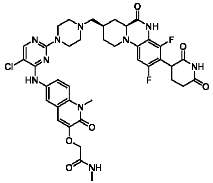 |
| 310 | 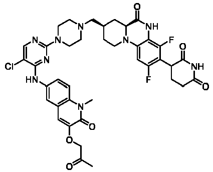 | 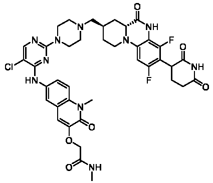 | 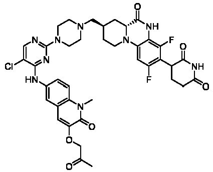 |
| 311 | 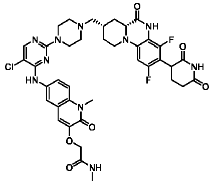 | 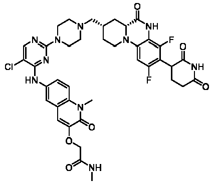 | 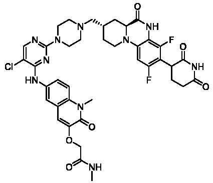 |
| 312 | 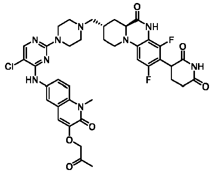 | 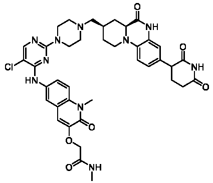 | 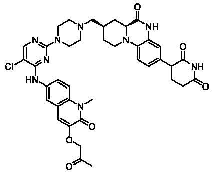 |
| 313 | 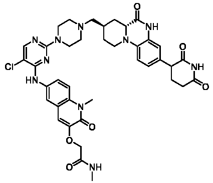 | 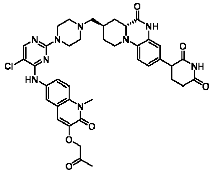 | 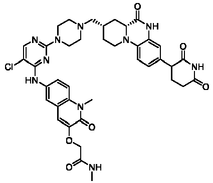 |
| 314 | 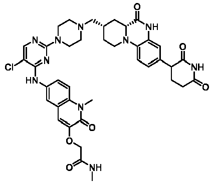 | 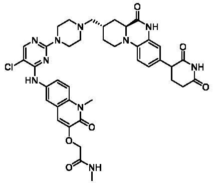 | 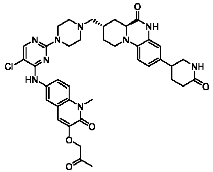 |
| 315 | 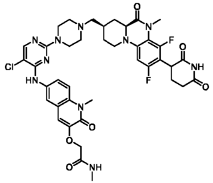 | 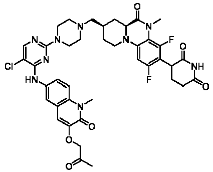 | 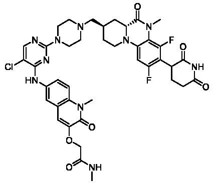 |
| 316 | 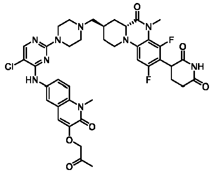 | 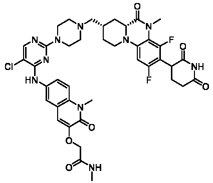 | 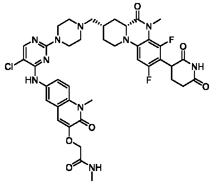 |
| 317 | 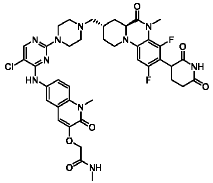 | 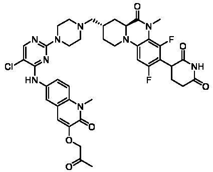 | 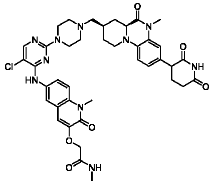 |
| 318 | 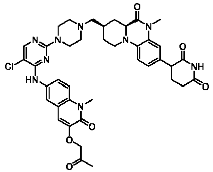 | 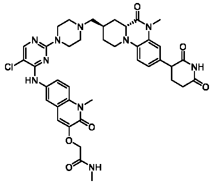 | 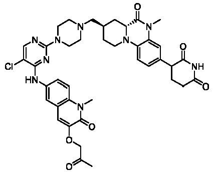 |
| 319 | 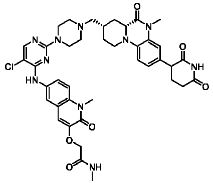 | 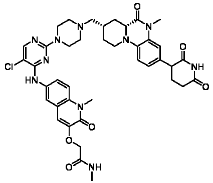 | 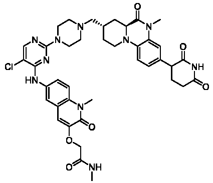 |
| 320 | 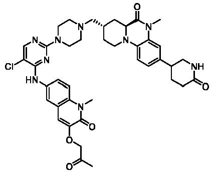 | 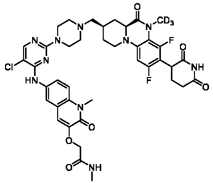 | 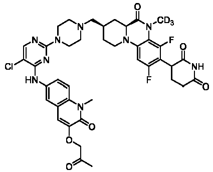 |
| 321 | 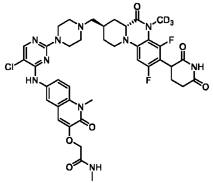 | 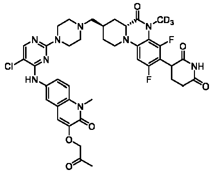 | 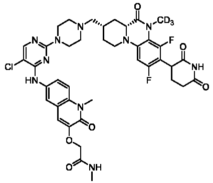 |
| 322 | 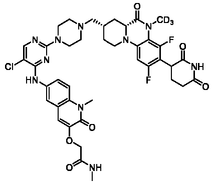 | 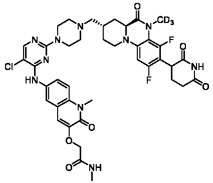 | 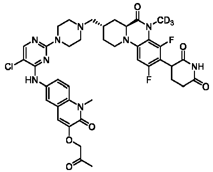 |
| 323 | 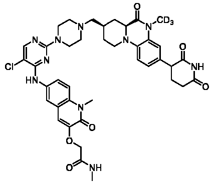 | 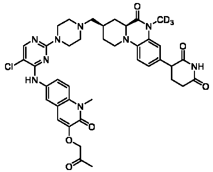 | 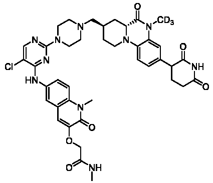 |
| 324 | 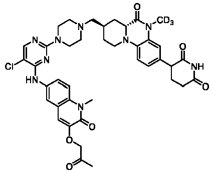 | 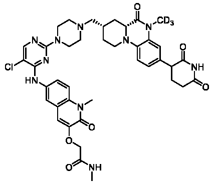 | 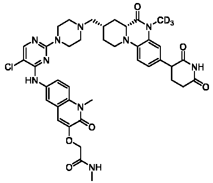 |
| 325 | 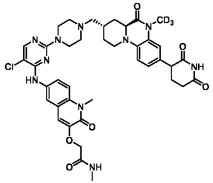 | 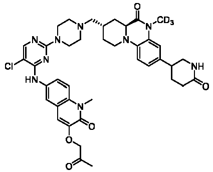 | 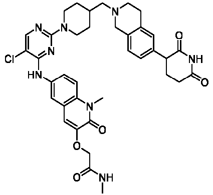 |
| 326 | 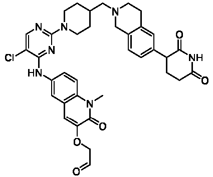 | 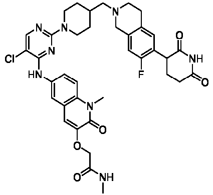 | 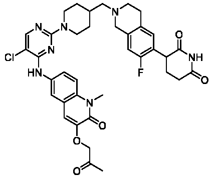 |
| 327 | 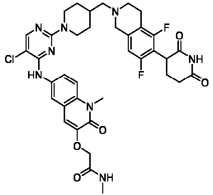 | 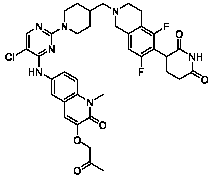 | 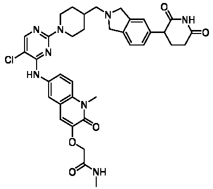 |
| 328 | 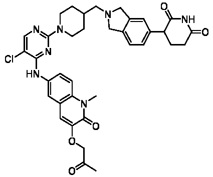 | 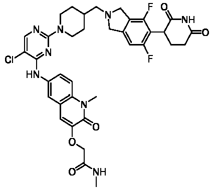 | 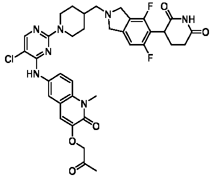 |
| 329 | 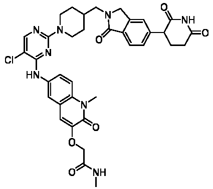 | 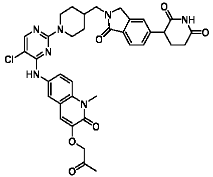 | 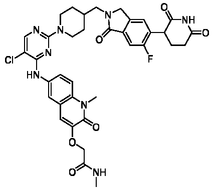 |
| 330 | 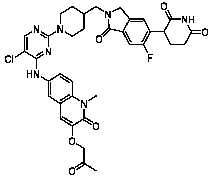 | 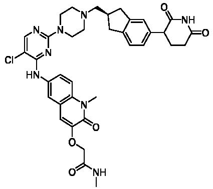 | 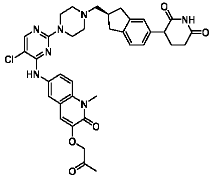 |
| 331 | 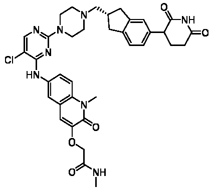 | 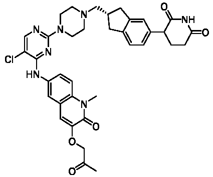 | 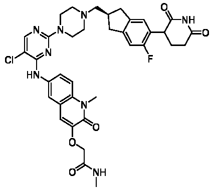 |
| 332 | 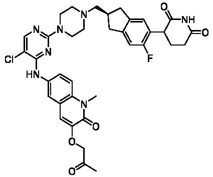 | 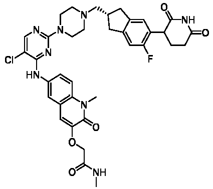 | 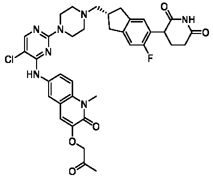 |
| 333 | 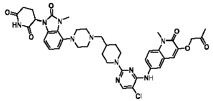 | 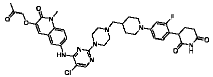 | 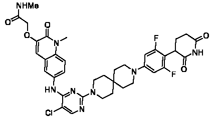 |
| 334 | 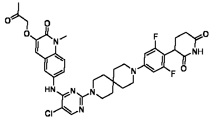 | 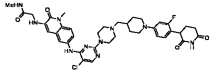 | 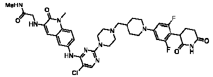 |
| 335 | 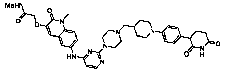 | 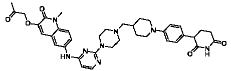 | 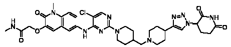 |
| 336 | 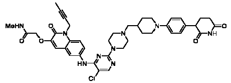 | 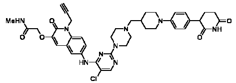 | 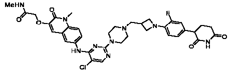 |
| 337 | 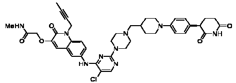 | 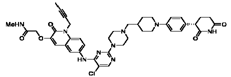 | 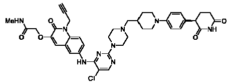 |
| 338 | 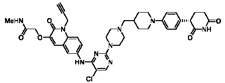 | 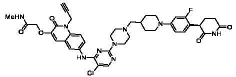 | 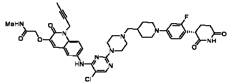 |
| 339 | 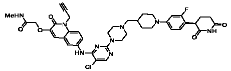 | 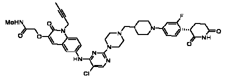 | 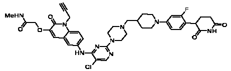 |
| 340 | 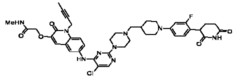 | 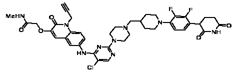 | 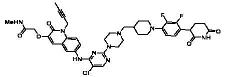 |
| 341 | 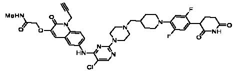 | 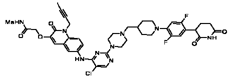 | 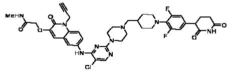 |
| 342 | 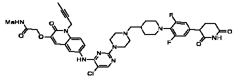 | 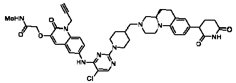 | 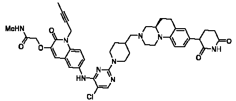 |
| 343 | 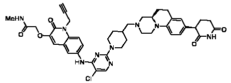 | 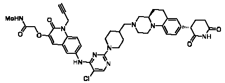 | 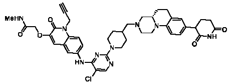 |
| 344 | 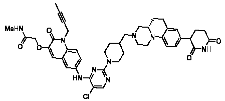 | 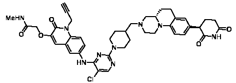 | 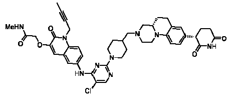 |
| 345 | 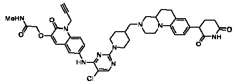 | 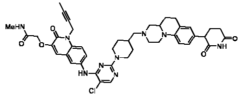 | 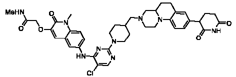 |
| 346 | 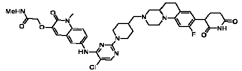 | 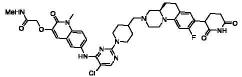 | 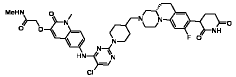 |
| 347 | 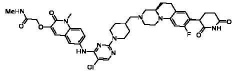 | 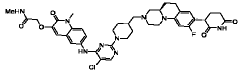 | 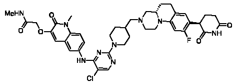 |
| 348 | 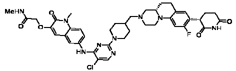 | 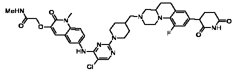 | 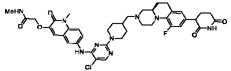 |
| 349 | 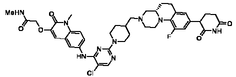 | 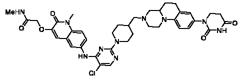 | 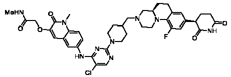 |
| 350 | 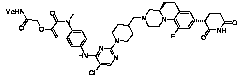 | 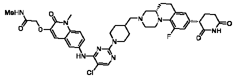 | 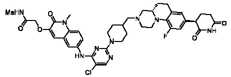 |
| 351 | 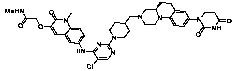 | 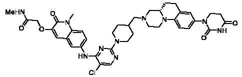 | 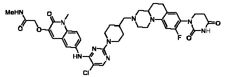 |
| 352 | 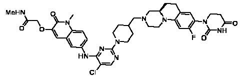 | 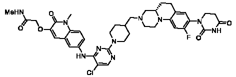 | 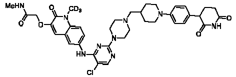 |
| 353 | 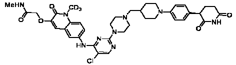 | 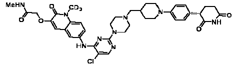 | 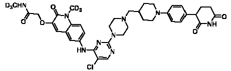 |
| 354 | 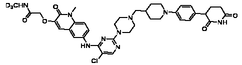 | 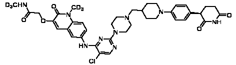 | 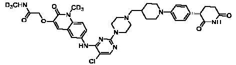 |
| 355 | 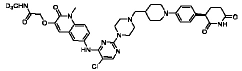 | 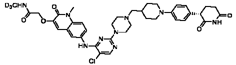 | 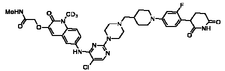 |
| 356 | 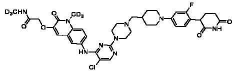 | 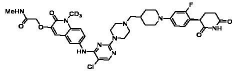 | 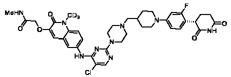 |
| 357 | 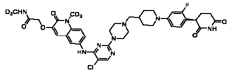 | 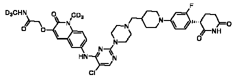 | 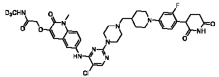 |
| 358 | 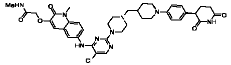 | 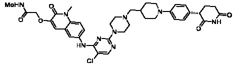 | 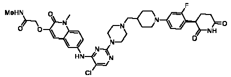 |
| 359 | 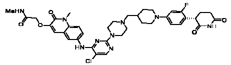 | 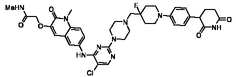 | 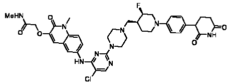 |
| 360 | 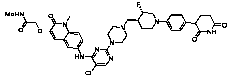 | 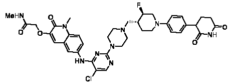 | 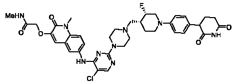 |
| 361 | 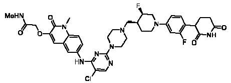 | 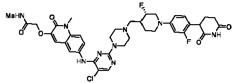 | 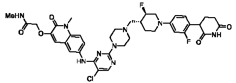 |
| 362 | 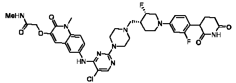 | 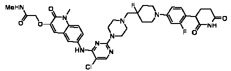 | 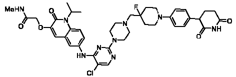 |
| 363 | 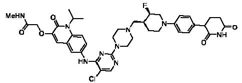 | 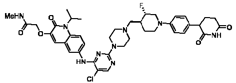 | 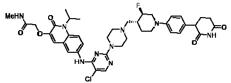 |
| 364 | 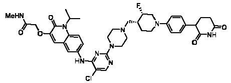 | 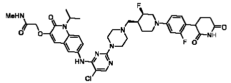 | 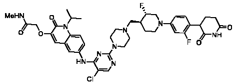 |
| 365 | 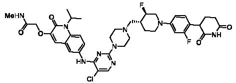 | 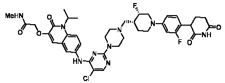 | 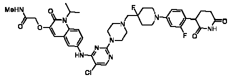 |
| 366 | 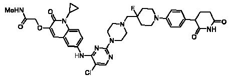 | 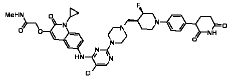 | 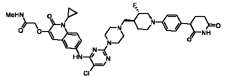 |
| 367 | 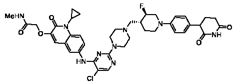 | 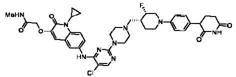 | 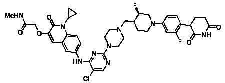 |
| 368 | 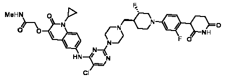 | 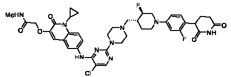 | 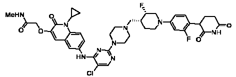 |
| 369 | 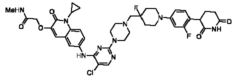 | 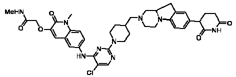 | 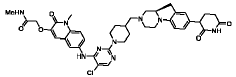 |
| 370 | 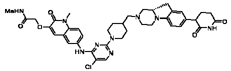 | 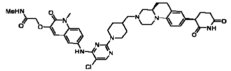 | 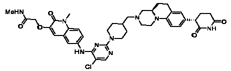 |
| 371 | 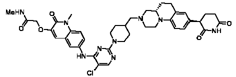 | 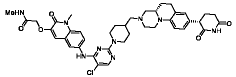 | 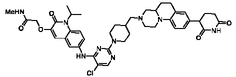 |
| 372 | 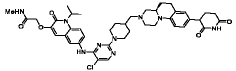 | 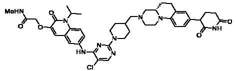 | 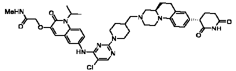 |
| 373 | 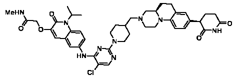 | 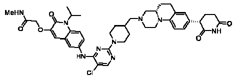 | 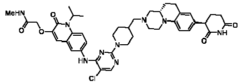 |
| 374 | 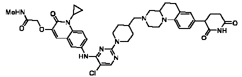 | 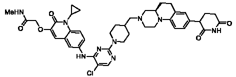 | 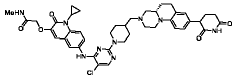 |
| 375 | 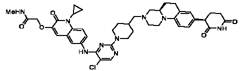 | 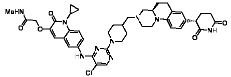 | 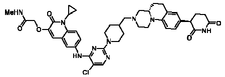 |
| 376 | 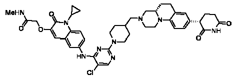 | 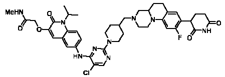 | 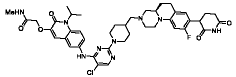 |
| 377 | 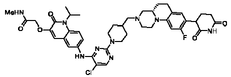 | 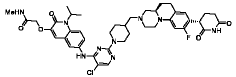 | 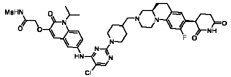 |
| 378 | 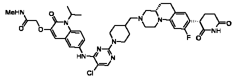 | 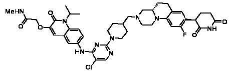 | 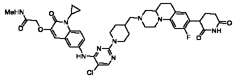 |
| 379 | 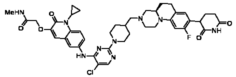 | 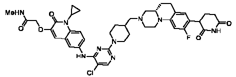 | 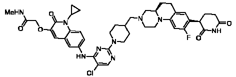 |
| 380 | 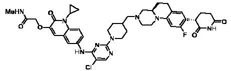 | 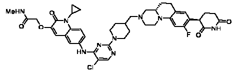 | 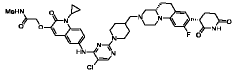 |
| 381 | 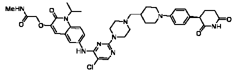 | 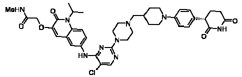 | 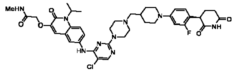 |
| 382 | 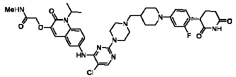 | 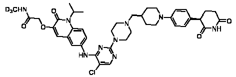 | 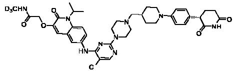 |
| 383 | 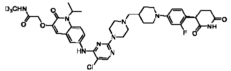 | 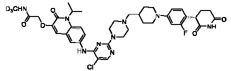 | 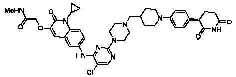 |
| 384 | 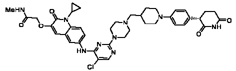 | 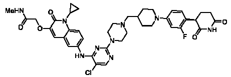 | 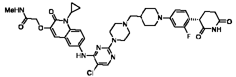 |
| 385 | 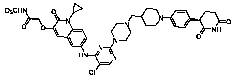 | 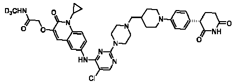 | 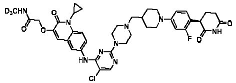 |
| 386 | 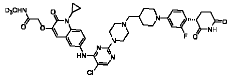 | 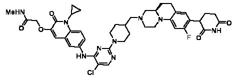 | 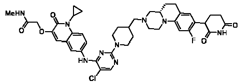 |
| 387 | 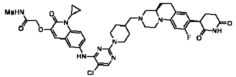 | 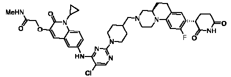 | 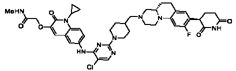 |
| 388 | 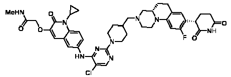 | 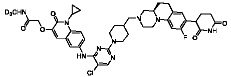 | 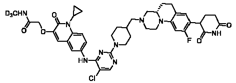 |
| 389 | 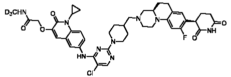 | 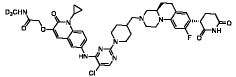 | 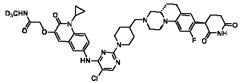 |
| 390 | 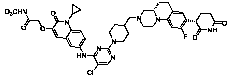 | 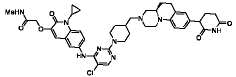 | 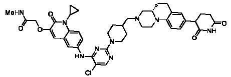 |
| 391 | 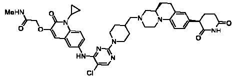 | 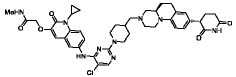 | 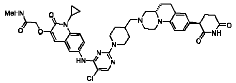 |
| 392 | 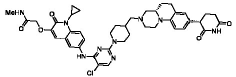 | 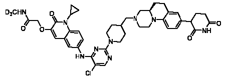 | 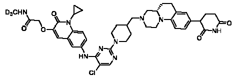 |
| 393 | 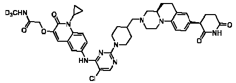 | 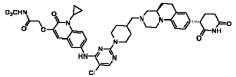 | 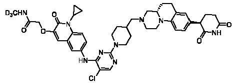 |
| 394 | 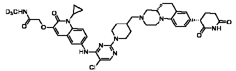 | 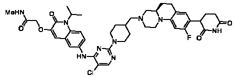 | 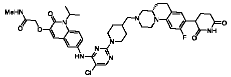 |
| 395 | 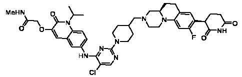 | 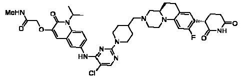 | 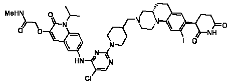 |
| 396 | 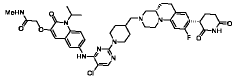 | 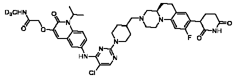 | 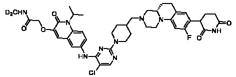 |
| 397 | 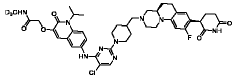 | 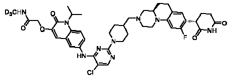 | 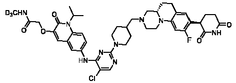 |
| 398 | 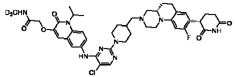 | 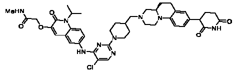 | 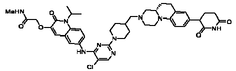 |
| 399 | 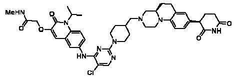 | 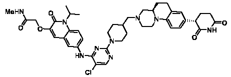 | 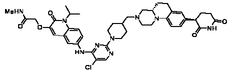 |
| 400 | 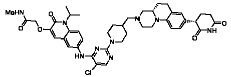 | 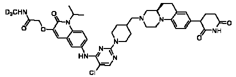 | 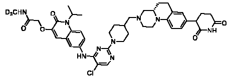 |
| 401 | 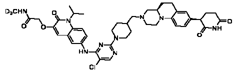 | 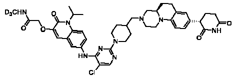 | 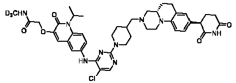 |
| 402 | 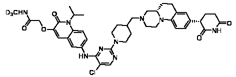 | 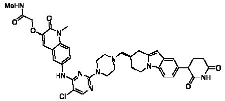 | 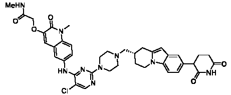 |
| 403 | 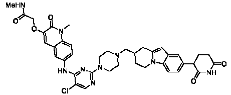 | 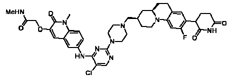 | 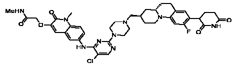 |
| 404 | 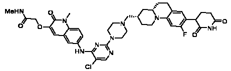 | 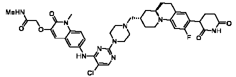 | 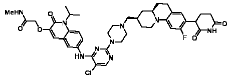 |
| 405 | 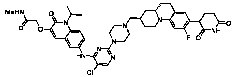 | 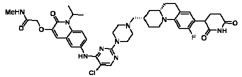 | 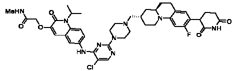 |
| 406 | 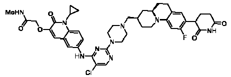 | 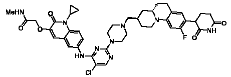 | 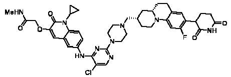 |
| 407 | 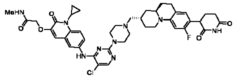 | | 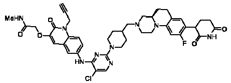 |
| 408 | 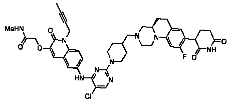 | 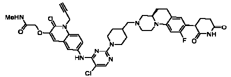 | 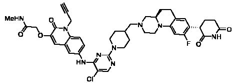 |
| 409 | 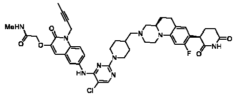 | 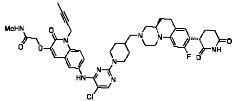 | 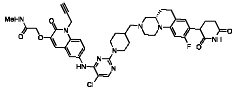 |
| 410 | 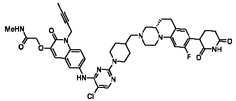 | 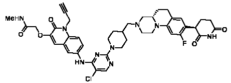 | 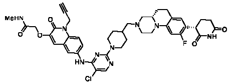 |
| 411 | 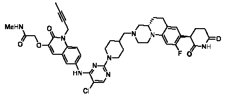 | 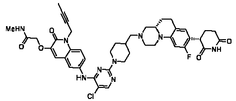 | 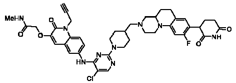 |
| 412 | 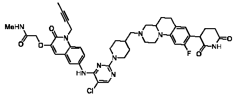 | 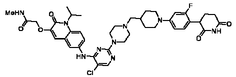 | 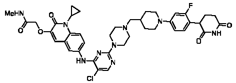 |
| 413 | 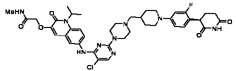 | 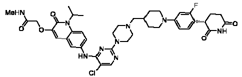 | 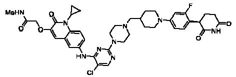 |
| 414 | 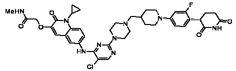 | 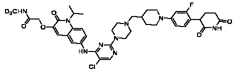 | 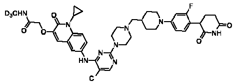 |
| 415 | 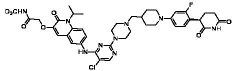 | 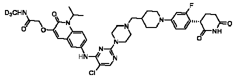 | 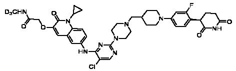 |
| 416 | 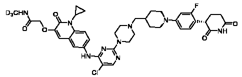 | 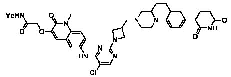 | 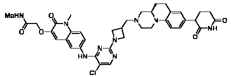 |
| 417 | 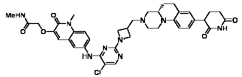 | 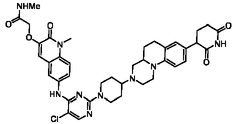 | 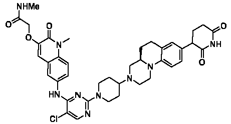 |
| 418 | 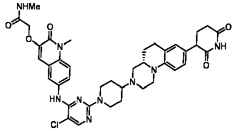 | 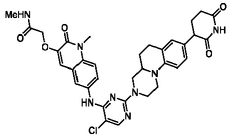 | 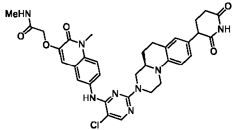 |
| 419 | 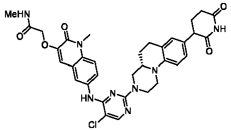 | 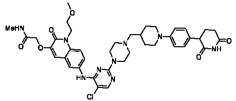 | 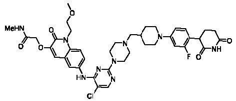 |
| 420 | 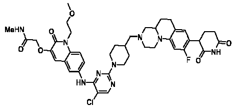 | 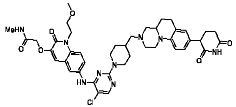 | 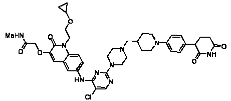 |
| 421 | 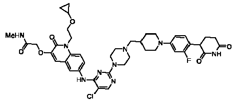 | 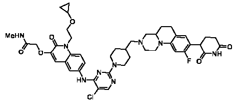 | 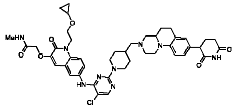 |
| 422 | 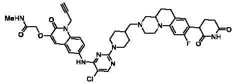 | 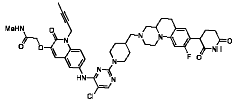 | 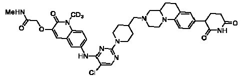 |
| 423 | 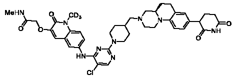 | 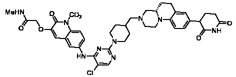 | 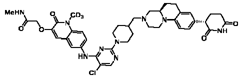 |
| 424 | 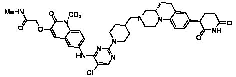 | 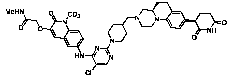 | 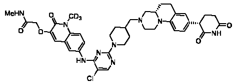 |
| 425 | 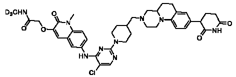 | 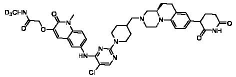 | 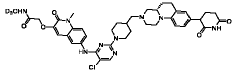 |
| 426 | 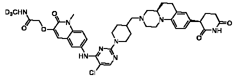 | 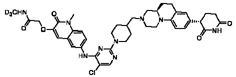 | 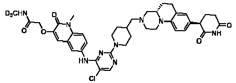 |
| 427 | 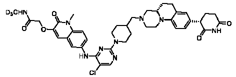 | 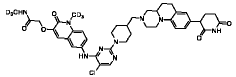 | 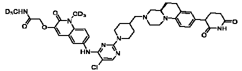 |
| 428 | 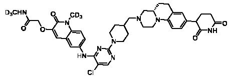 | 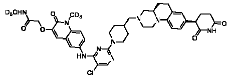 | 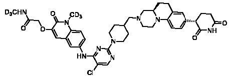 |
| 429 | 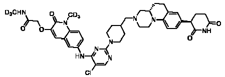 | 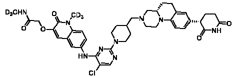 | 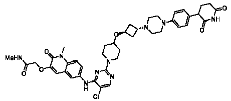 |
| 430 | 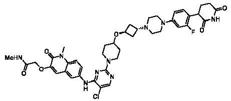 | 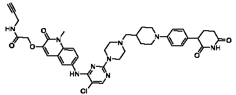 | 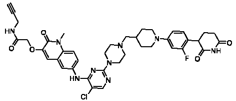 |
| 431 | 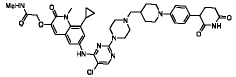 | 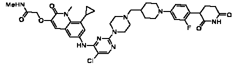 | 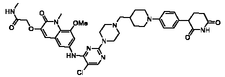 |
| 432 | 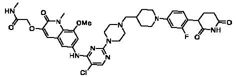 | 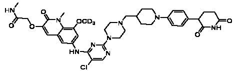 | 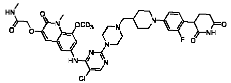 |
| 433 | 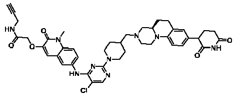 | 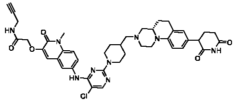 | 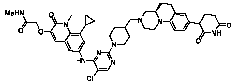 |
| 434 | 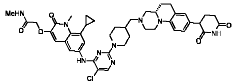 | 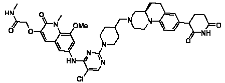 | 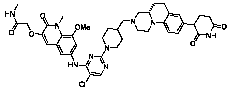 |
| 435 | 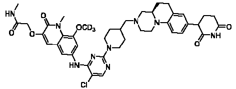 | 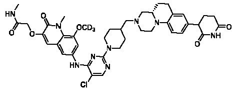 | 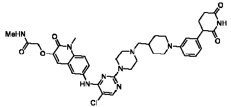 |
| 436 | 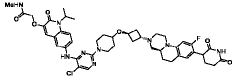 | 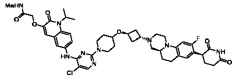 | 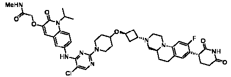 |
| 437 | 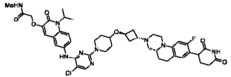 | 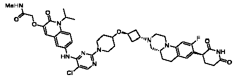 | 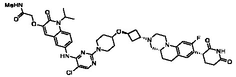 |
| 438 | 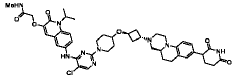 | 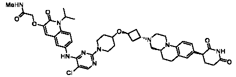 | 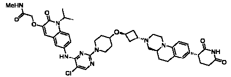 |
| 439 | 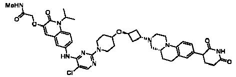 | 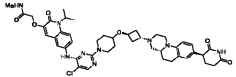 | 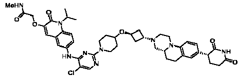 |
| 440 | 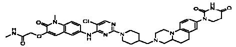 | 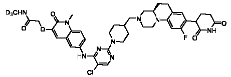 | 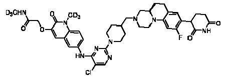 |
| 441 | 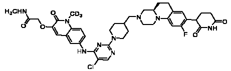 | 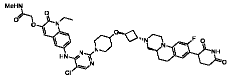 | 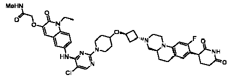 |
| 442 | 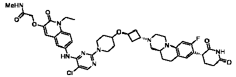 | 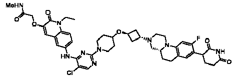 | 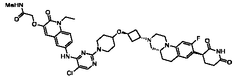 |
| 443 | 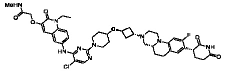 | 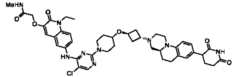 | 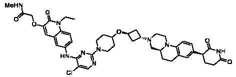 |
| 444 | 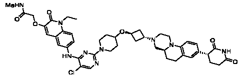 | 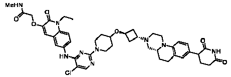 | 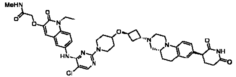 |
| 445 | 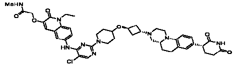 | 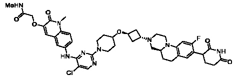 | 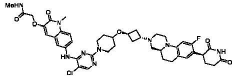 |
| 446 | 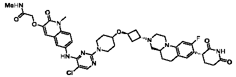 | 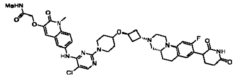 | 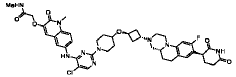 |
| 447 | 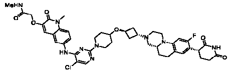 | 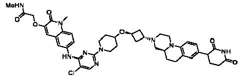 | 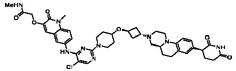 |
| 448 | 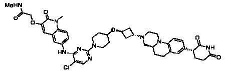 | 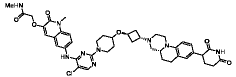 | 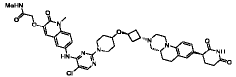 |
| 449 | 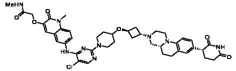 | 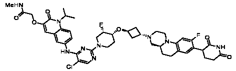 | 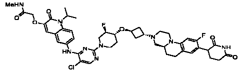 |
| 450 | 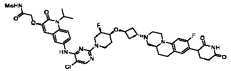 | 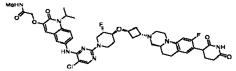 | 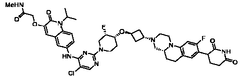 |
| 451 | 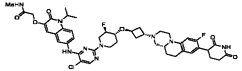 | 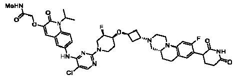 | 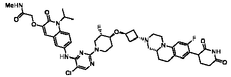 |
| 452 | 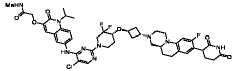 | 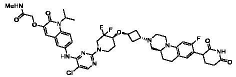 | 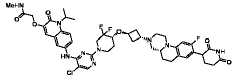 |
| 453 | 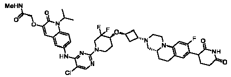 | 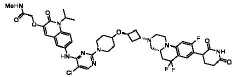 | 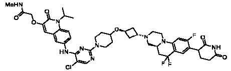 |
| 454 | 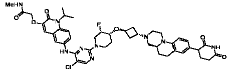 | 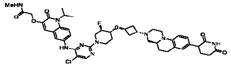 | 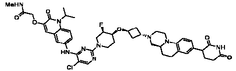 |
| 455 | 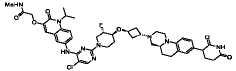 | 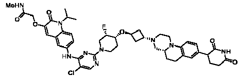 | 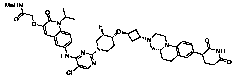 |
| 456 | 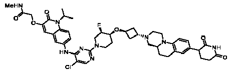 | 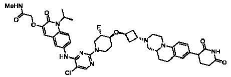 | 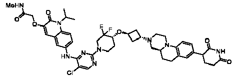 |
| 457 | 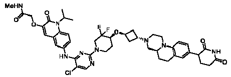 | 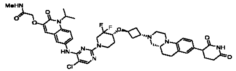 | 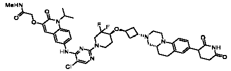 |
| 458 | 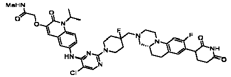 | 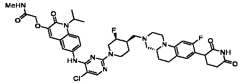 | 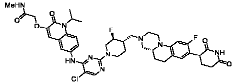 |
| 459 | 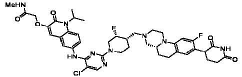 | 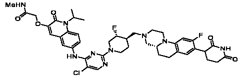 | 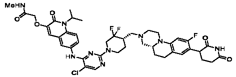 |
| 460 | 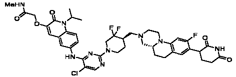 | 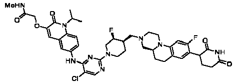 | 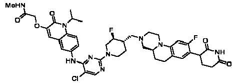 |
| 461 | 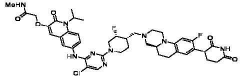 | 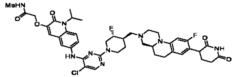 | 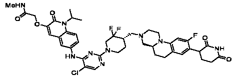 |
| 462 | 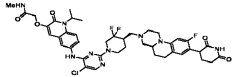 | 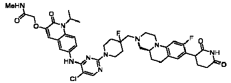 | 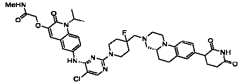 |
| 463 | 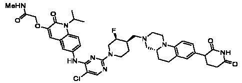 | 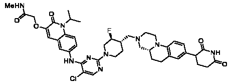 | 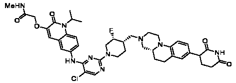 |
| 464 | 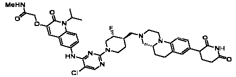 | 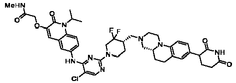 | 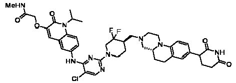 |
| 465 | 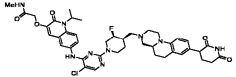 | 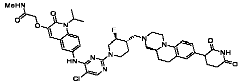 | 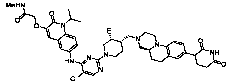 |
| 466 | 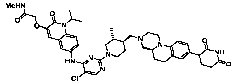 | 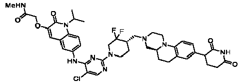 | 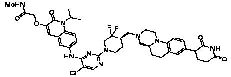 |
| 467 | 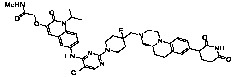 | 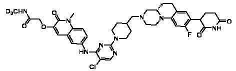 | 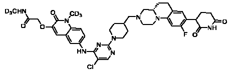 |
| 468 | 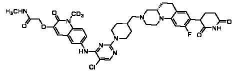 | 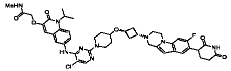 | 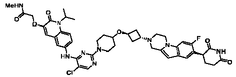 |
| 469 | 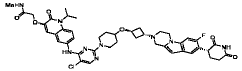 | 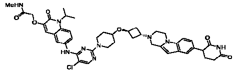 | 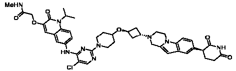 |
| 470 | 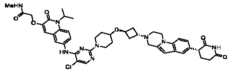 | 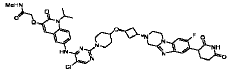 | 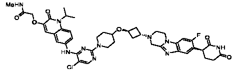 |
| 471 | 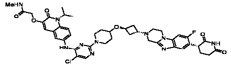 | 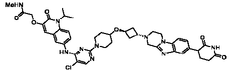 | 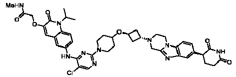 |
| 472 | 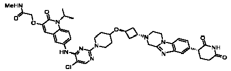 | 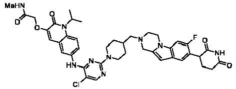 | 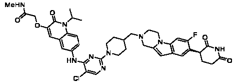 |
| 473 | 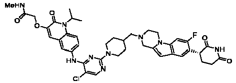 | 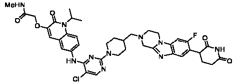 | 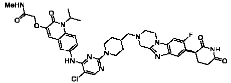 |
| 474 | 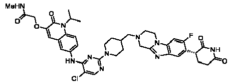 | 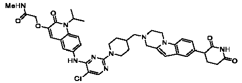 | 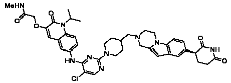 |
| 475 | 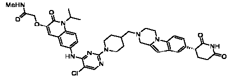 | 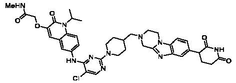 | 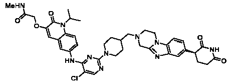 |
| 476 | 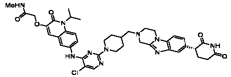 | 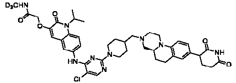 | 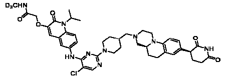 |
| 477 | 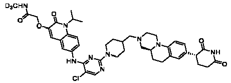 | 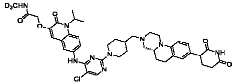 | 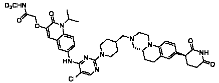 |
| 478 | 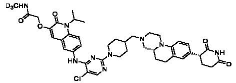 | 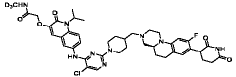 | 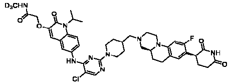 |
| 479 | 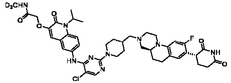 | 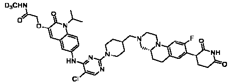 | 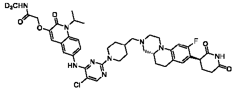 |
| 480 | 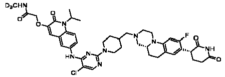 | 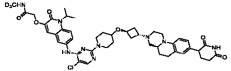 | 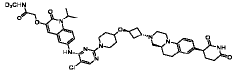 |
| 481 | 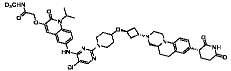 | 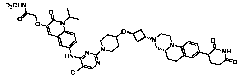 | 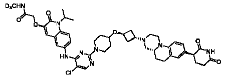 |
| 482 | 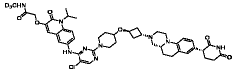 | 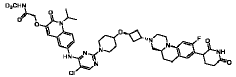 | 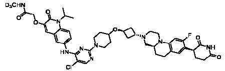 |
| 483 | 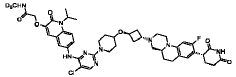 | 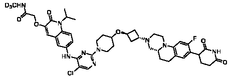 | 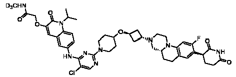 |
| 484 | 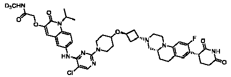 | 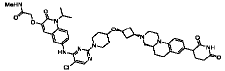 | 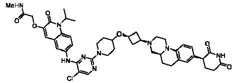 |
| 485 | 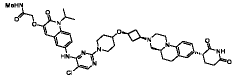 | 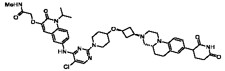 | 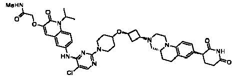 |
| 486 | 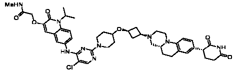 | 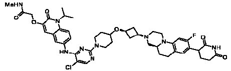 | 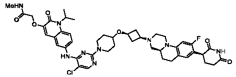 |
| 487 | 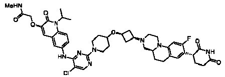 | 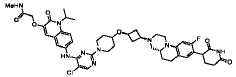 | 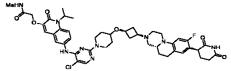 |
| 488 | 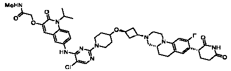 | 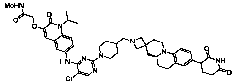 | 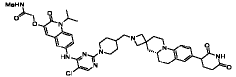 |
| 489 | 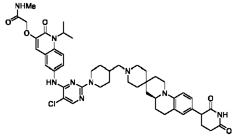 | 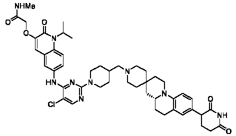 | 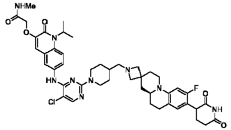 |
| 490 | 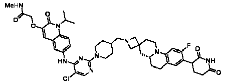 | 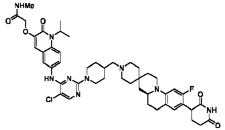 | 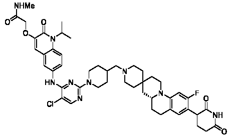 |
| 491 | 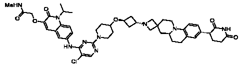 | 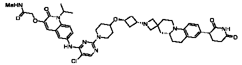 | 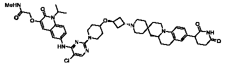 |
| 492 | 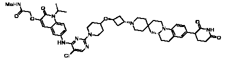 | 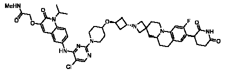 | 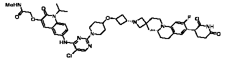 |
| 493 | 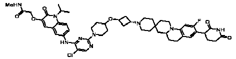 | 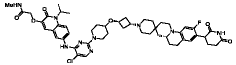 | 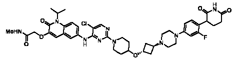 |
| 494 | 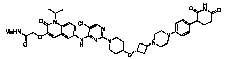 | 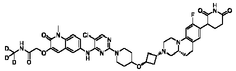 | 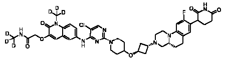 |
| 495 | 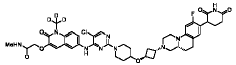 | 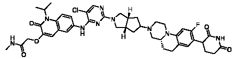 | 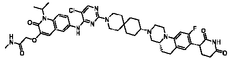 |
| 496 | 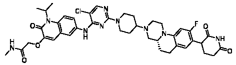 | 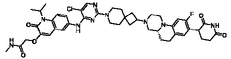 | 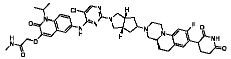 |
| 497 | 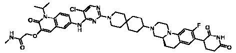 | 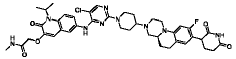 | 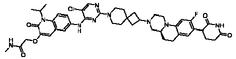 |
| 498 | 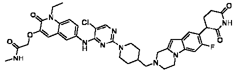 | 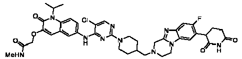 | 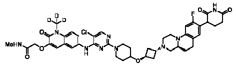 |
| 499 | 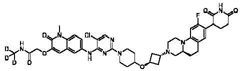 | 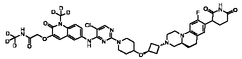 | 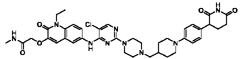 |
| 500 | 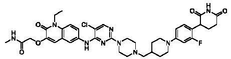 | 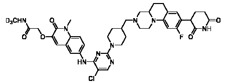 | 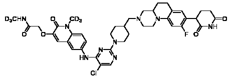 |
| 501 | 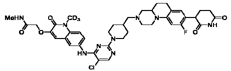 | 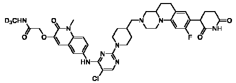 | 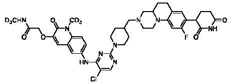 |
| 502 | 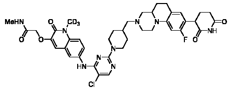 | 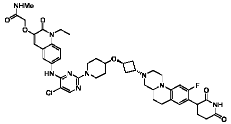 | 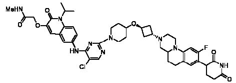 |
| 503 | 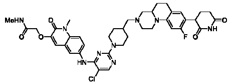 | 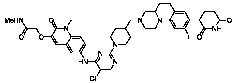 | |

The present invention relates to a pharmaceutical composition, comprising the above-mentioned compound or the stereoisomer, tautomer, deuterated form, solvate, prodrug, metabolite, pharmaceutically acceptable salt or eutectic thereof according to the present invention, and a pharmaceutically acceptable carrier.

The present invention relates to the use of the above-mentioned compound or the stereoisomer, tautomer, deuterated form, solvate, prodrug, metabolite, pharmaceutically acceptable salt or eutectic thereof according to the present invention in the preparation of a drug for treating a disease related to the activity or expression level of Bcl6.

The present invention relates to the use of the above-mentioned compound or the stereoisomer, tautomer, deuterated form, solvate, prodrug, metabolite, pharmaceutically acceptable salt or eutectic thereof according to the present invention in the preparation of a drug for treating a disease related to the inhibition or degradation of Bcl6.

The present invention relates to the use of the above-mentioned compound or the stereoisomer, tautomer, deuterated form, solvate, prodrug, metabolite, pharmaceutically acceptable salt or eutectic thereof according to the present invention, wherein the disease is selected from cancer.

The present invention relates to a pharmaceutical composition or pharmaceutical preparation comprising a therapeutically effective amount of the compound or a stereoisomer, deuterated form, solvate, prodrug, metabolite, pharmaceutically acceptable salt or eutectic thereof according to the present invention, and a pharmaceutical excipient. The pharmaceutical composition can be in a unit preparation form (the amount of the active drug in the unit preparation is also referred to as the "preparation specification").

The present invention further provides a method for treating a disease in a mammal, comprising administering to the mammal a therapeutically effective amount of the compound, or the stereoisomer, deuterated form, solvate, prodrug, metabolite, pharmaceutically acceptable salt or eutectic thereof or the pharmaceutical composition according to the present invention. In some embodiments, the mammal mentioned in the present invention includes human.

The term "effective amount" or "therapeutically effective amount" according to the present application refers to a sufficient amount of the compound disclosed in the present application that is administered to ameliorate, to some extent, one or more symptoms of a disease or condition (such as cancer) being treated. In some embodiments, the outcome is the reduction and/or remission of signs, symptoms or causes of the disease, or any other desired change in the biological system. For example, an "effective amount" in terms of the therapeutic use is an amount comprising the compound disclosed in the present application that is required to provide clinically significant reduction of the symptoms of the disease. Examples of the therapeutically effective amount include, but are not limited to, 1-1500 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, and 150-200 mg;

In some embodiments, the pharmaceutical composition comprises the compound or the stereoisomer, deuterated form, solvate, prodrug, metabolite, pharmaceutically acceptable salt or eutectic thereof according to the present invention in an amount including but not limited to 1-1500 mg, 1-600 mg, 20-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, and 300 mg.

The present invention provides a method for treating a disease in a mammal, comprising administering to a subject a therapeutically effective amount of the compound, or the stereoisomer, deuterated form, solvate, prodrug, metabolite, pharmaceutically acceptable salt, or eutectic thereof according to the present invention, wherein the therapeutically effective amount is preferably 1-1500 mg, and the disease is preferably cancer.

The present invention provides a method for treating a disease in a mammal, comprising administering to a subject a drug, i.e., the compound, or the stereoisomer, deuterated form, solvate, prodrug, metabolite, pharmaceutically acceptable salt, or eutectic thereof according to the present invention in a daily dose of 1-1500 mg/day, wherein the daily dose can be a single dose or divided doses; in some embodiments, the daily dose includes, but is not limited to 10-1500 mg/day, 10-800 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 10 mg/day, 20 mg/day, 25 mg/day, 50 mg/day, 100 mg/day, 125 mg/day, 150 mg/day, 200 mg/day, 400 mg/day, 600 mg/day, 800 mg/day, 1500 mg/day or 2000 mg/day.

The present invention relates to a kit, wherein the kit can comprise a composition in the form of a single dose or multiple doses and comprises the compound, or the stereoisomer, deuterated form, solvate, prodrug, metabolite, pharmaceutically acceptable salt or eutectic thereof according to the present invention, and the amount of the compound, or the stereoisomer, deuterated form, solvate, prodrug, metabolite, pharmaceutically acceptable salt or eutectic thereof according to the present invention is identical to the amount of same in the above-mentioned pharmaceutical composition.

In the present invention, the amount of the compound or a stereoisomer, deuterated form, solvate, prodrug, metabolite, pharmaceutically acceptable salt or eutectic thereof according to the present invention is calculated in the form of free base in each case.

Unless stated to the contrary, the terms used in the description and claims of the present application have the following meanings.

The term "preparation specification" refers to the weight of the active drug contained in each vial, tablet or other unit preparation.

The carbon, hydrogen, oxygen, sulphur, nitrogen or F, Cl, Br, I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise ¹²C, ¹³C and ¹⁴C, the isotopes of hydrogen comprise protium (H), deuterium (D, also known as heavy hydrogen), tritium (T, also known as superheavy hydrogen), the isotopes of oxygen comprise ¹⁶O, ¹⁷O and ¹⁸O, the isotopes of sulphur comprise ³²S, ³³S, ³⁴S and ³⁶S, the isotopes of nitrogen comprise ¹⁴N and ¹⁵N, the isotopes of fluorine comprise ¹⁷F and ¹⁹F, the isotopes of chlorine comprise ³⁵Cl and ³⁷Cl, and the isotopes of bromine comprise ⁷⁹Br and ⁸¹Br.

"Halogen" refers to F, Cl, Br, or I.

"Halogen-substituted" refers to a substitution with F, Cl, Br, or I, including but not limited to a substitution with 1 to 10 substituents selected from F, Cl, Br, or I, or a substitution with 1 to 6 substituents selected from F, Cl, Br, or I, preferably a substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbyl, including but not limited to an alkyl of 1 to 20 carbon atoms, an alkyl of 1 to 8 carbon atoms, an alkyl of 1 to 6 carbon atoms, or an alkyl of 1 to 4 carbon atoms. Non-limiting examples include methyl, ethyl, n-propyl, isopropyl, n-butyl, sec-butyl, neobutyl, tert-butyl, n-pentyl, isoamyl, neopentyl, n-hexyl and various branched isomers thereof. The definition of the "alkyl" herein is consistent with this definition. Alkyl can be monovalent, divalent, trivalent or tetravalent.

"Alkylene" refers to a substituted or unsubstituted linear or branched divalent saturated hydrocarbyl, including -(CH₂)ᵥ- (v is an integer from 1 to 10), and examples of alkylene include, but are not limited to, methylene, ethylene, propylene, butylene, *etc.*

"Cycloalkyl" refers to a substituted or unsubstituted saturated carbocyclic hydrocarbyl, usually having from 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Heterocycloalkyl" refers to a substituted or unsubstituted saturated heteroatom-containing cyclic hydrocarbyl, including but not limited to 3 to 10 atoms, 3 to 8 atoms, or 1 to 3 heteroatoms selected from N, O or S. N and S selectively substituted in the heterocycloalkyl ring can be oxidized to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, azacyclopropyl, oxacyclobutyl, azetidinyl, tetrahydrofuryl, tetrahydro-2H-pyranyl, dioxolanyl, dioxanyl, pyrrolidyl, piperidyl, imidazolidinyl, oxazolidinyl, oxazinanyl, morpholinyl, hexahydropyrimidyl or piperazinyl. Heterocycloalkyl can be monovalent, divalent, trivalent or tetravalent.

"Alkenyl" refers to a substituted or unsubstituted linear or branched unsaturated hydrocarbyl, having at least 1, usually 1, 2 or 3 carbon-carbon double bonds, with a main chain including but not limited to 2 to 10, 2 to 6, or 2 to 4 carbon atoms. Examples of alkenyl include, but are not limited to, ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The definition of the "alkenyl" herein is consistent with this definition. Alkenyl can be monovalent, divalent, trivalent or tetravalent.

"Alkynyl" refers to a substituted or unsubstituted linear or branched unsaturated hydrocarbyl, having at least 1, usually 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, 4-pentynyl, 1-methyl-1-butynyl, 2-methyl-1-butynyl, 2-methyl-3-butynyl, 1-hexynyl, 2-hexynyl, 3-hexynyl, 4-hexynyl, 5-hexynyl, 1-methyl-1-pentynyl, 2-methyl-1-pentynyl, 1-heptynyl, 2-heptynyl, 3-heptynyl, 4-heptynyl, 1-octynyl, 3-octynyl, 1-nonynyl, 3-nonynyl, 1-decynyl, 4-decynyl, *etc.* Alkynyl can be monovalent, divalent, trivalent or tetravalent.

"Alkoxy" refers to a substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring, 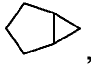 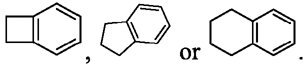 "Carbocyclyl" or "carbocycle" can be monovalent, divalent, trivalent or tetravalent.

"Heterocyclyl" or "heterocycle" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S, and the selectively substituted C, N or S in the heterocyclyl ring can be oxidized to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, azacyclopropyl, oxacyclobutyl, azetidinyl, 1,3-dioxolanyl, 1,4-dioxolanyl, 1,3-dioxanyl, azacycloheptyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl, 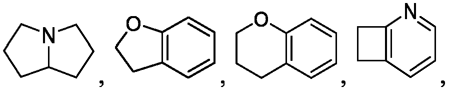 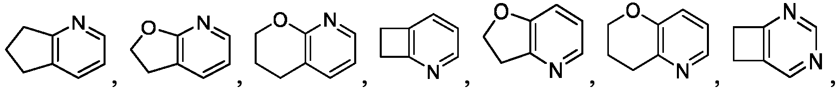 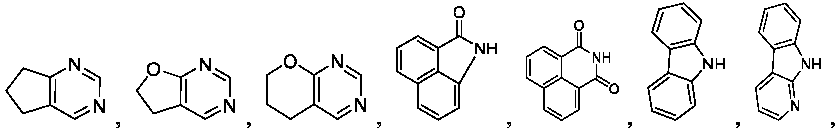 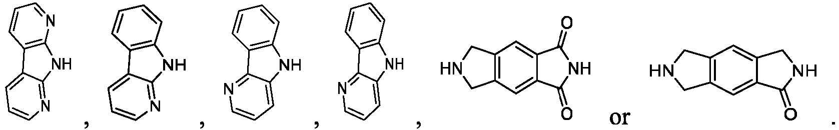 "Heterocyclyl" or "heterocycle" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and can optionally contain 0 to 5 heteroatoms selected from N, O or S(=O)ₙ. Non-limiting examples include: 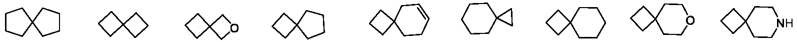 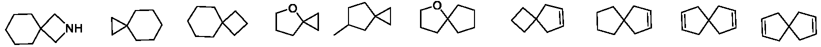 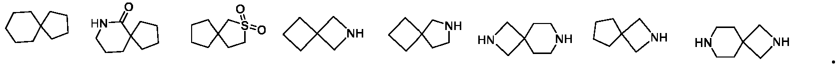 "Spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, S(=O)ₙ or O, wherein n is 0, 1 or 2). The number of ring atoms in the fused ring system includes but is not limited to 5 to 20, 5 to 14, 5 to 12, or 5 to 10. Non-limiting examples include: 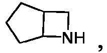 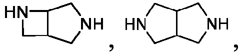 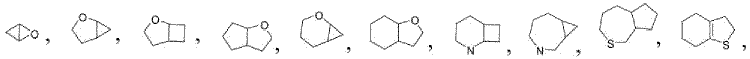 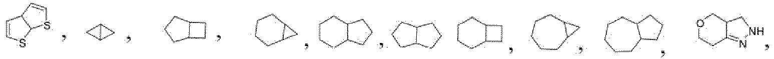 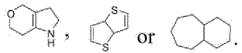 "Fused ring" or "fused ring group" can be monovalent, divalent, trivalent or tetravalent.

"Bridged ring" or "bridged ring group" means a substituted or unsubstituted polycyclic group containing any two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes but is not limited to 5 to 20, 5 to 14, 5 to 12 or 5 to 10. Non-limiting examples include 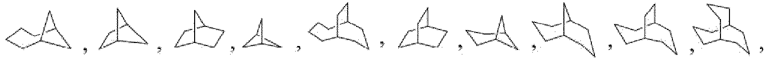 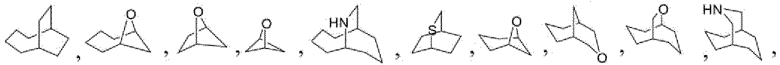 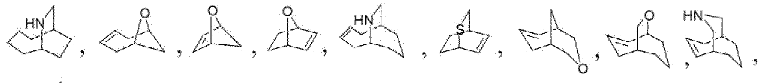 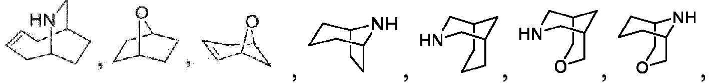 cubane and adamantane. "Bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms. The definition of the "carbospiro ring", "spiro ring carbocyclyl", "spirocarbocyclyl" or "carbospiro ring group" herein is consistent with that of a spiro ring.

"Carbo-fused ring", "fused ring carbocyclyl", "fused carbocyclyl" or "carbo-fused ring group" refers to a "fused ring" with a ring system consisting only of carbon atoms. The definition of the "carbo-fused ring", "fused ring carbocyclyl", "fused carbocyclyl" or "carbo-fused ring group" herein is consistent with that of a fused ring.

"Carbo-bridged ring", "bridged ring carbocyclyl", "bridged carbocyclyl" or "carbo-bridged ring group" refers to a "bridged ring" with a ring system consisting only of carbon atoms. The definition of the "carbo-bridged ring", "bridged ring carbocyclyl", "bridged carbocyclyl" or "carbo-bridged ring group" herein is consistent with that of a bridged ring.

"Mono-heterocyclic ring", "monocyclic heterocyclyl" or "mono-heterocyclic ring group" refers to "heterocyclyl" or "heterocycle" with a monocyclic system. The definition of the "heterocyclyl", "monocyclic heterocyclyl" or "mono-heterocyclic ring group" herein is consistent with that of heterocycle.

"Fused-heterocyclic ring", "fused-heterocyclic ring group", "fused ring heterocyclyl" or "fused-heterocyclic ring group" refers to a "fused ring" containing a heteroatom. The definition of the "fused-heterocyclic ring", "fused-heterocyclic ring group", "fused ring heterocyclyl" or "fused-heterocyclic ring group" herein is consistent with that of a fused ring.

"Spiro-heterocyclic ring", "spiro-heterocyclic ring group", "spiro ring heterocyclyl" or "spiro-heterocyclic ring group" refers to a "spiro ring" containing a heteroatom. The definition of the "spiro-heterocyclic ring", "spiro-heterocyclic ring group", "spiro ring heterocyclyl" or "spiro-heterocyclic ring group" herein is consistent with that of a spiro ring.

"Bridged-heterocyclic ring", "bridged-heterocyclic ring group", "bridged ring heterocyclyl" or "bridged-heterocyclic ring group" refers to a "bridged ring" containing a heteroatom. The definition of the "bridged-heterocyclic ring", "bridged-heterocyclic ring group", "bridged ring heterocyclyl" or "bridged-heterocyclic ring group" herein is consistent with that of a bridged ring.

"Aryl" or "aromatic ring" refers to a substituted or unsubstituted aromatic hydrocarbyl with a monocyclic ring or a fused ring, wherein the number of ring atoms in the aromatic ring includes but is not limited to 6 to 18, 6 to 12 or 6 to 10 carbon atoms. The aryl ring can be fused to a saturated or unsaturated carbocycle or heterocycle, wherein the ring connected to the parent structure is an aryl ring. Non-limiting examples include a benzene ring, a naphthalene ring, or 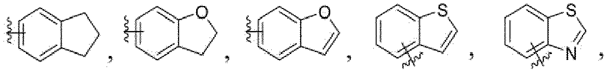 and "aryl" or "aromatic ring" may be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbyl containing 1 to 5 heteroatoms or groups containing heteroatoms (including but not limited to N, O or S(=O)n, wherein n is 0, 1 or 2), wherein the number of ring atoms in the heteroaromatic ring includes but is not limited to 5 to 15, 5 to 10 or 5 to 6. Non-limiting examples of heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazolyl, benzoimidazolyl, benzopyridyl, pyrrolopyridyl, etc. The heteroaryl ring may be fused to a saturated or unsaturated carbocycle or heterocycle, wherein the ring connected to the parent structure is a heteroaryl ring. Non-limiting examples include 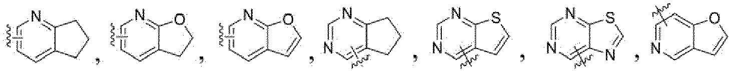 and 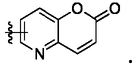 The definition of the "heteroaryl" herein is consistent with this definition. Heteroaryl can be monovalent, divalent, trivalent or tetravalent. When divalent, trivalent or tetravalent, the point of connection is on the heteroaryl ring.

"5-membered ring fused 5-membered heteroaromatic ring" refers to a 5-fused-5-membered fused heteroaromatic ring, wherein at least one of the two fused rings contains at least one heteroatom (including but not limited to O, S or N), and the entire group is aromatic. Non-limiting examples include a pyrrolopyrrole ring, a pyrazolopyrrole ring, a pyrazolopyrazole ring, a pyrrolofuran ring, a pyrazolofuran ring, a pyrrolothiophene ring and a pyrazolothiophene ring.

"5-fused-6-membered heteroaromatic ring" refers to a 5-fused-6-membered fused heteroaromatic ring, wherein at least one of the two fused rings contains at least one heteroatom (including but not limited to O, S or N), and the entire group is aromatic. Non-limiting examples include a benzo 5-membered heteroaryl and 6-membered heteroaromatic ring fused 5-membered heteroaromatic ring.

"Substitution" or "substituted" refers to a substitution with 1 or more (including, but not limited to 2, 3, 4 or 5) substituents including, but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, -(CH₂)ₘ-C(=O)-R^{a}, -O-(CH₂)ₘ-C(=O)-R^{a}, -(CH₂)ₘ₋C(=O)-NR^{b}R^{c}, -(CH₂)ₘS(=O)ₙR^{a}, -(CH₂)ₘ-alkenyl-R^{a}, OR^{d} or -(CH₂)ₘ₋alkynyl-R^{a} (wherein m and n are 0, 1 or 2), arylthio, thiocarbonyl, silyl, -NR^{b}R^{c}, etc., wherein R^{b} and R^{c} are independently selected from H, hydroxyl, amino, carbonyl, alkyl, alkoxy, cycloalkyl, heterocyclyl, aryl, heteroaryl, sulfonyl, or trifluoromethylsulfonyl. Alternatively, R^{d} and R^{c} can form a five- or six-membered cycloalkyl or heterocyclyl. R^{a} and R^{d} are each independently selected from aryl, heteroaryl, alkyl, alkoxy, cycloalkyl, heterocyclyl, carbonyl, ester group, bridged ring group, spiro ring group or fused ring group.

"Containing 1 to 5 heteroatoms selected from O, S or N" means containing 1, 2, 3, 4 or 5 heteroatoms selected from O, S or N.

"Substituted with 1 to X substituents" refers to substituted with 1, 2, 3 ... X substituents, wherein X is selected from any integer between 2 and 10. For example, "substituted with 1 to 4 substituents" refers to substituted with 1, 2, 3 or 4 substituents. For example, "substituted with 1 to 5 substituents" refers to substituted with 1, 2, 3, 4 or 5 substituents. For example, "bridged-heterocyclic ring is optionally substituted with 1 to 4 substituents selected from H or F" means that the bridged-heterocyclic ring is optionally further substituted with 1, 2, 3 or 4 substituents selected from H or F.

An X- to Y-membered ring (X is selected from an integer less than Y and greater than 3, and Y is selected from any integer between 4 and 12) includes X-, X+1-, X+2-, X+3-, X+4-, ..., Y-membered rings. Rings include heterocycle, carbocycle, an aromatic ring, aryl, heteroaryl, cycloalkyl, a mono-heterocyclic ring, a fused-heterocyclic ring, a spiro-heterocyclic ring or a bridged-heterocyclic ring. For example, a "4- to 7-membered mono-heterocyclic ring" refers to a 4-, 5-, 6- or 7-membered mono-heterocyclic ring, and a "5- to 10-membered fused-heterocyclic ring" refers to a 5-, 6-, 7-, 8-, 9- or 10-membered fused-heterocyclic ring.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound according to the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated forms, solvates, prodrugs, metabolites, pharmaceutically acceptable salts or eutectics thereof and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of a compound administered.

"Excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatine, vegetable oils, polyethylene glycols, diluents, granulating agents, lubricants, adhesives and disintegrants.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or *in vivo* to obtain a parent compound. When the prodrug of the present invention is administered to a mammalian individual, the prodrug is split to form a free amino or carboxyl group.

The term "eutectic" refers to a crystal formed by the combination of active pharmaceutical ingredient (API) and eutectic former (CCF) under the action of hydrogen bonds or other non-covalent bonds. The pure state of API and CCF are both solid at room temperature, and there is a fixed stoichiometric ratio between various components. The eutectic is a multi-component crystal, which includes both a binary eutectic formed between two neutral solids and a multi-element eutectic formed between a neutral solid and a salt or solvate.

"Animal" is meant to include mammals, such as humans, companion animals, zoo animals, and domestic animals, preferably humans, horses, or dogs.

The term "stereoisomer" refers to an isomer produced as a result of different spatial arrangement of atoms in molecules, including cis-trans isomers, enantiomers, diastereomers and conformational isomers.

The term "tautomer" refers to a functional group isomer produced by the rapid movement of an atom in two positions in a molecule, such as keto-enol isomerization and amide-imino alcohol isomerization.

"IC₅₀" refers to the concentration of a drug or inhibitor required to inhibit half of a given biological process (or a component of the process such as an enzyme, a receptor and a cell).

### Brief Description of the Drawings

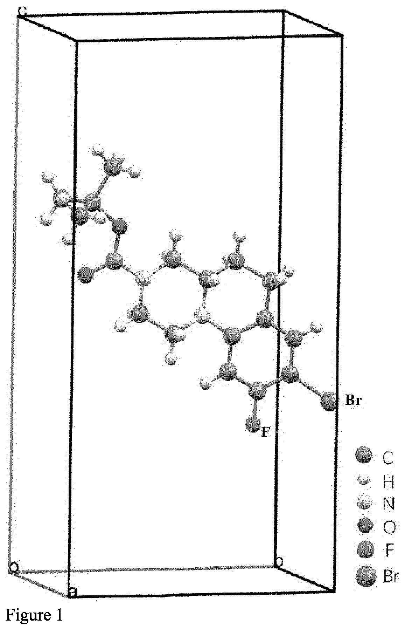

Figure 1 is a diagram of an asymmetrical unit of a unit cell of compound 46b by a MicroED test.

### Detailed Description of Embodiments

The technical solutions of the present invention will be described in detail below in conjunction with examples, but the protection scope of the present invention includes but is not limited thereto.
The structures of the compounds are determined by nuclear magnetic resonance (NMR) or (and) mass spectrometry (MS). The NMR shift (δ) is given in the unit of 10⁻⁶ (ppm). NMR is measured with (Bruker Avance III 400 and Bruker Avance 300) NMR instrument, and the solvent for determination is deuterated dimethyl sulfoxide (DMSO-d₆), deuterated chloroform (CDCl₃), deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 µM);
MicroED is determined with a Thermo Scientific Glacios 200 kv cryo-transmission electron microscopy;
Yantai Huanghai HSGF₂₅₄ or Qingdao GF₂₅₄ silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
for the column chromatography, Yantai Huanghai silica gel of 200-300 mesh silica gel is generally used as a carrier;
Boc: tert-butoxycarbonyl; Ts: p-toluenesulfonyl; Cbz: benzyloxycarbonyl; TMS: trimethylsilyl; DMF: N,N-dimethylformamide; DIPEA: N,N-diisopropylethylamine.

### Preparation of intermediate A-5:

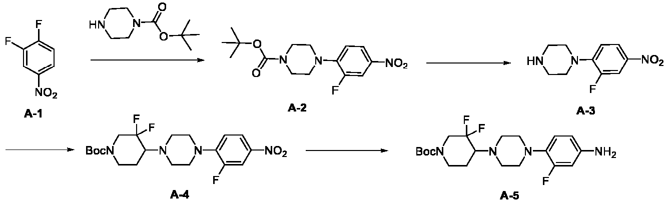

### Step 1: preparation of A-2:

Tert-butyl piperazine-1-carboxylate (82 g, 0.44 mol) was dissolved in DMF (500 mL) and then caesium carbonate (215 g, 0.66 mol) was added. After the mixture was stirred at room temperature for 20 min, compound A-1 (70 g, 0.44 mol) was added. The resulting mixture was reacted at 50°C for 16 h under nitrogen protection. The reaction solution was cooled to room temperature and quenched by adding ice water (1.5 L). A large number of yellow solids were precipitated and filtered. The filter cake was washed with water (500 mL) twice and dried to obtain compound A-2 (135 g, yield: 95%).

LCMS m/z = 326.1 [M+1]⁺.

### Step 2: preparation of A-3:

Under nitrogen protection, compound A-2 (83.0 g, 0.255 mol) was dissolved in acetonitrile (400 mL). The mixture was placed in an ice bath and stirred for 20 min. A solution of hydrogen chloride in 1,4-dioxane (200 mL) was added, and then the resulting mixture was allowed to naturally warm to room temperature and reacted for 1 h. The reaction system was concentrated under reduced pressure to obtain crude hydrochloride of A-3 (72 g).

LCMS m/z = 226.1 [M+1]⁺.

### Step 3: preparation of A-4:

The above-mentioned crude hydrochloride of A-3 (72 g) was dissolved in toluene (800 mL). Under nitrogen protection, sodium acetate (52 g, 0.64 mol) and acetic acid (60 mL) were successively added. The mixture was stirred at room temperature for 20 min. Tert-butyl 3,3-difluoro-4-oxopiperidine-1-carboxylate (66 g, 0.28 mol) was added. The resulting mixture was subjected to water separation at 100°C and reacted for 6 h. The reaction system was cooled to room temperature and filtered over diatomaceous earth. The filtrate was concentrated under reduced pressure to obtain a crude. Under nitrogen protection, the above-mentioned crude was dissolved in a mixed solvent of ultra-dry methanol (200 mL) and ultra-dry 1,2-dichloroethane (200 mL). Sodium cyanoborohydride (41.4 g, 1.275 mol) was added, and the mixture was reacted at room temperature for 16 h. The filtrate was concentrated under reduced pressure, the crude was separated and purified by silica gel column chromatography (petroleum ether/ethyl acetate (v/v) = 1:1) to obtain compound A-4 (65 g, two-step yield: 57%).

LCMS m/z = 445.2 [M+1]⁺.

### Step 4: preparation of A-5:

A-4 (55 g, 0.124 mol) was dissolved in ethyl acetate (1000 mL) and 10% palladium on carbon (5.5 g) was added. The mixture was subjected to hydrogen replacement three times, and reacted at room temperature under the atmosphere of hydrogen balloon for 16 h. The reaction system was filtered over diatomaceous earth, and the filtrate was concentrated under reduced pressure to obtain A-5 (50.5 g, yield: 98%).

LCMS m/z = 415.2 [M+1]⁺.

Compound A-5 was separated and purified by preparative SFC to obtain chiral isomer A-5-P1 (23.15 g, retention time for preparative SFC: 0.946 min; retention time for chiral HPLC: 20.460 min; [α]²⁰_{D} = +31.1°) and isomer A-5-P2 (19.94 g, retention time for preparative SFC: 1.883 min, retention time for chiral HPLC: 17.221 min; [α]²⁰_{D} = -27.1°) (A-5-P1 and A-5-P2 are one of the structures of compounds A-5-a and A-5-b, and A-5-P1 and A-5-P2 are enantiomers of each other).

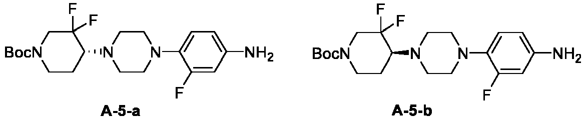

### SFC chiral preparation method:

instrument: Waters 150 SFC; preparative column model: Chiralpak IC-3 Column (250×30 mm, I.D 50 mm, 10 um particle size); mobile phase: A: CO₂, and B: isopropanol and an acetonitrile solution containing 0.1% ammonia water.

Elution condition: 35% B isocratic elution; flow rate: 200 mL/min; column pressure: 100 bar; column temperature: 25°C; detection wavelength: 220 nm; post treatment: after preparative separation, the components with the same retention time were combined and concentrated under reduced pressure to obtain A-5-P1 and A-5-P2, respectively.

### Chiral HPLC analysis conditions:

instrument: Shimadzu LC-20A; chiral column: CHIRALCEL OD-H, 4.6 × 250 mm, 5 µm; mobile phase: n-hexane (containing 0.1% diethylamine)-isopropanol (v/v) = 70:30; flow rate: 1 mL/min; column temperature: 35°C; detection wavelength: 210 nm;
injection volume: 10 µL; run time: 30 min.
Polarimetric method: instrument model: Anton Paar MCP 4100; tube length: 1 dm; detection solvent: methanol;
sample size: A-5-P1: 100.66 mg; A-5-P2: 104.50 mg;
sample concentration: A-5-P1: 3.8024 mg/mL; A-5-P2: 3.8024 mg/mL.

### Preparation of intermediate A-6-P1:

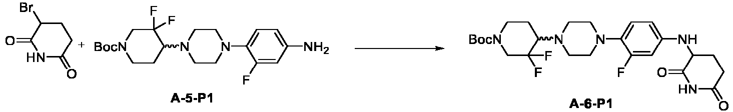

Note: wavy lines in the structures of the present application refer to configuration R or S;

To a reaction flask were respectively added A-5-P1 (0.50 g, 1.21 mmol), 3-bromopiperidine-2,6-dione (0.70 g, 3.65 mmol), sodium bicarbonate (0.61 g, 7.26 mmol) and 4 Ǻ molecular sieve (100 mg). DMF (5 mL) was added, and the mixture was reacted at 80°C for 2 h. The reaction solution was cooled to room temperature, poured into water (20 mL), and extracted with ethyl acetate (20 mL×3). The organic phases were combined, dried over anhydrous sodium sulphate, and concentrated under reduced pressure. The crude was separated and purified by silica gel column chromatography (petroleum ether/ethyl acetate (v/v) = 1:1) to obtain A-6-P1 (0.30 g, yield: 47%).

LCMS m/z = 526.7 [M+1] ⁺.

### Preparation of intermediate A-6-P2:

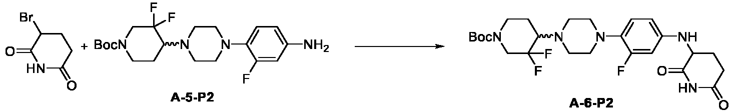

Note: wavy lines in the structures of the present application refer to configuration R or S;

A-5-P2 (2.62 g, 6.33 mmol) and 3-bromopiperidine-2,6-dione (3.65 g, 19 mmol) were placed in a sealed tube, and the mixture was dissolved by adding DMF (20 mL). Sodium bicarbonate (3.19 g, 37.98 mmol) was added, and the resulting mixture was reacted at 90°C for 16 h. The reaction system was cooled to room temperature, and diluted by adding ethyl acetate (100 mL). The organic phase was washed three times with water (50 mL), washed with a saturated aqueous sodium chloride solution (50 mL), dried over anhydrous sodium sulphate, and concentrated under reduced pressure. The residue was separated and purified by silica gel column chromatography (dichloromethane/methanol (v/v) = 75:1) to obtain compound A-6-P2 (2.5 g, yield: 75%).

LCMS m/z = 526.2 [M+1]⁺.

### Example 1: preparation of compound 1

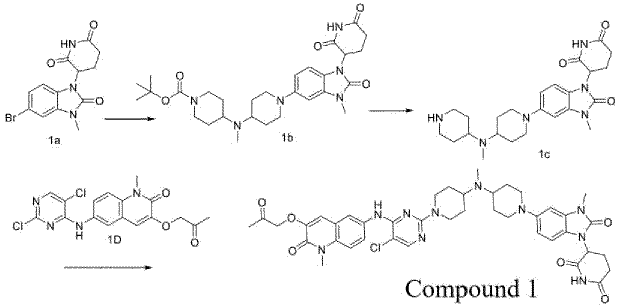

### Step 1: preparation of 1b

Under nitrogen atmosphere, to a reaction flask were respectively added 1a (0.50 g, 1.48 mmol) (see WO 2019060742 for the synthesis method), tert-butyl 4-(methyl(piperidin-4-yl)amino)piperidine-1-carboxylate (0.66 g, 2.22 mmol) (see WO 2021058017 for the synthesis method), RuPhos Pd G3 (CAS: 1445085-77-7) (0.25 g, 0.30 mmol) and RuPhos (CAS: 787618-22-8) (0.14 g, 0.30 mmol). A solution of toluene (5 mL) and LiHMDS (1 mol/L) in THF (8.8 mL, 8.8 mmol) was added, and the mixture was reacted at 80°C for 1.5 h. The reaction solution was cooled to 0°C in an ice bath. The reaction system was quenched by dropwise adding a saturated aqueous ammonium chloride solution (10 mL), and extracted with ethyl acetate (30 mL×3). The organic phases were combined, dried over anhydrous sodium sulphate, and concentrated under reduced pressure. The resulting crude was separated and purified by silica gel column chromatography (petroleum ether/ethyl acetate (v/v) = 1:1-1:2) to obtain 1b (0.19 g, yield: 23%).

LCMS m/z = 555.4 [M+1] ⁺.

### Step 2: preparation of trifluoroacetate of 1c

To a reaction flask were added 1b (0.13 g, 0.23 mmol), trifluoroacetic acid (0.5 mL) and dichloromethane (2 mL), and the mixture was reacted at room temperature for 2 h. The reaction system was concentrated under reduced pressure to obtain crude trifluoroacetate of 1c (0.15 g).

LCMS m/z = 455.3 [M+1] ⁺.

### Step 3: preparation of compound 1

To a reaction flask were respectively added the above-mentioned crude trifluoroacetate of 1c (0.15 g), sodium bicarbonate (0.097 g, 1.15 mmol) and DMSO (5 mL). The mixture was stirred at room temperature for 0.5 h. 1D (0.090 g, 0.23 mmol) (see WO 2021077010 for the synthesis method) and DIPEA (0.19 mL) were added, and the resulting mixture was stirred at 100°C for 7 h. The reaction system was cooled to room temperature, poured into water (50 mL) and filtered. The filter cake was collected, dissolved with a mixed solvent (50 mL) of dichloromethane/methanol (v/v) = 2:1, dried over anhydrous sodium sulphate, and concentrated under reduced pressure. The crude was subjected to Pre-HPLC (instrument and preparative column: using SHIMADZULC-20AP preparative liquid phase chromatographic instrument, preparative column model: PhenomenexC 18). Preparation method: the crude was dissolved in acetonitrile and water, and filtered with a 0.45 µm filter membrane to prepare a sample liquid. Mobile phase system: water (containing 10 mmoL/L ammonium bicarbonate)/acetonitrile. Gradient elution method: gradient elution with acetonitrile from 27% to 57% (elution time: 10 min). Lyophilization was performed to obtain compound 1 (25 mg, yield: 13%).

¹H NMR (400 MHz, DMSO-*d₆*) δ 11.03 (s, 1H), 8.81 (s, 1H), 8.03 (s, 1H), 7.91 (d, 1H), 7.71 (dd, 1H), 7.46 (d, 1H), 7.04 (s, 1H), 6.92 (d, 1H), 6.81 (d, 1H), 6.62 (dd, 1H), 5.34 - 5.22 (m, 1H), 4.90 (s, 2H), 4.59 - 4.45 (m, 2H), 3.67 (s, 3H), 3.65 - 3.54 (m, 2H), 3.40 - 3.20 (m, 3H), 2.97 - 2.75 (m, 4H), 2.75 - 2.55 (m, 5H), 2.23 - 2.14 (m, 6H), 2.05 - 1.92 (m, 1H), 1.85 - 1.55 (m, 6H), 1.50 - 1.32 (m, 2H).

LCMS m/z = 811.3 [M+1] ⁺.

### Example 2: preparation of compound 2

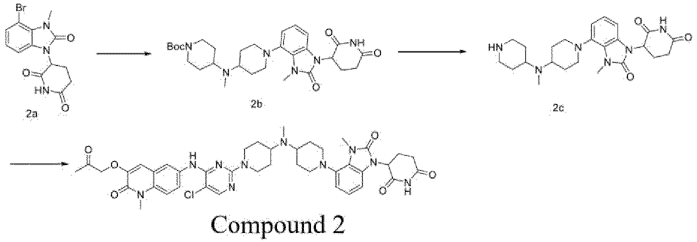

Compound 2 was prepared using 2a (see WO 2021158634 for the synthesis method) as the raw material and referring to the synthesis method of example 1.

¹H NMR (400 MHz, DMSO-*d₆*) δ 11.07 (s, 1H), 8.82 (s, 1H), 8.03 (s, 1H), 7.94 - 7.88 (m, 1H), 7.71 (dd, 1H), 7.52 - 7.41 (m, 1H), 7.04 (s, 1H), 7.01 - 6.82 (m, 3H), 5.34 (dd, 1H), 4.91 (s, 2H), 4.62 - 4.42 (m, 2H), 3.67 (s, 3H), 3.63 (s, 3H), 3.19 - 3.05 (m, 2H), 2.96 - 2.56 (m, 9H), 2.26 - 2.15 (m, 6H), 2.08 - 1.92 (m, 1H), 1.90 - 1.58 (m, 6H), 1.52 - 1.31 (m, 2H).

LCMS m/z = 811.9 [M+1] ⁺.

### Example 3: preparation of compound 3

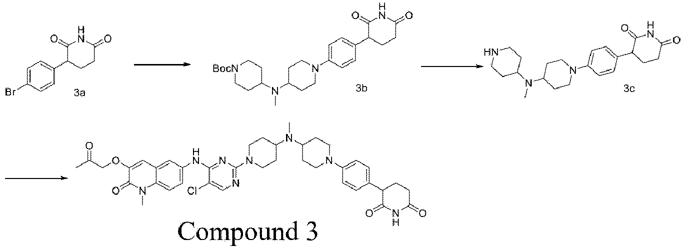

Compound 3 was prepared using 3a (see WO 2017197046 for the synthesis method) as the raw material and referring to the synthesis method of example 1.

¹H NMR (400 MHz, DMSO-*d₆*) δ 10.74 (s, 1H), 8.80 (s, 1H), 8.03 (s, 1H), 7.94 - 7.87 (m, 1H), 7.71 (dd, 1H), 7.50 - 7.41 (m, 1H), 7.08 - 6.97 (m, 3H), 6.94 - 6.82 (m, 2H), 4.90 (s, 2H), 4.59 - 4.44 (m, 2H), 3.77 - 3.60 (m, 6H), 2.92 - 2.74 (m, 3H), 2.72 - 2.56 (m, 4H), 2.49 - 2.37 (m, 1H), 2.24 - 1.94 (m, 8H), 1.84 - 1.30 (m, 8H).

LCMS m/z = 741.3 [M+1] ⁺.

### Example 4: preparation of compound 4

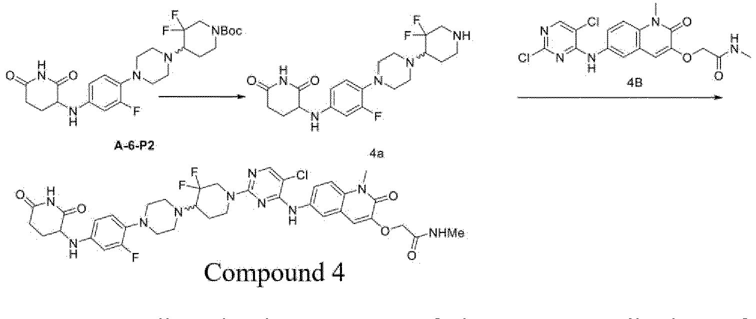

Note: wavy lines in the structures of the present application refer to configuration R or S;

### Step 1: preparation of trifluoroacetate of 4a

To a reaction flask were added A-6-P2 (100 mg, 0.19 mmol), trifluoroacetic acid (1 mL) and dichloromethane (2 mL), and the mixture was reacted at room temperature for 2 h. The reaction solution was concentrated under reduced pressure to obtain crude trifluoroacetate of 4a (100 mg).

### Step 2: preparation of compound 4

To a reaction flask were respectively added the above-mentioned crude trifluoroacetate of 4a (100 mg), solid sodium bicarbonate (80 mg, 0.95 mmol) and DMSO (5 mL). The mixture was reacted at room temperature for 0.5 h. **4B** (78 mg, 0.19 mmol) (see WO 2021077010 for the synthesis method) and DIPEA (0.16 mL) were added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 18 min). Lyophilization was performed to obtain compound 4 (24 mg, yield: 16%).

LCMS m/z = 797.2 [M+1] ⁺.

### Example 5: preparation of trifluoroacetate of compound 5

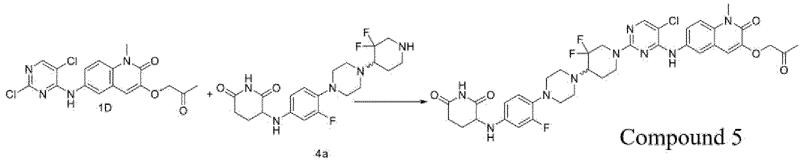

Note: wavy lines in the structures of the present application refer to configuration R or S;

To a reaction flask were respectively added the crude trifluoroacetate of 4a (100 mg), sodium bicarbonate (80 mg, 0.95 mmol) and DMSO (5 mL). The mixture was reacted at room temperature for 0.5 h. 1D (75 mg, 0.19 mmol) and DIPEA (0.16 mL) were added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 12 min). Lyophilization was performed to obtain the trifluoroacetate of compound 5 (4.2 mg).

LCMS m/z = 782.2 [M+1] ⁺.

### Example 6: preparation of compound 6

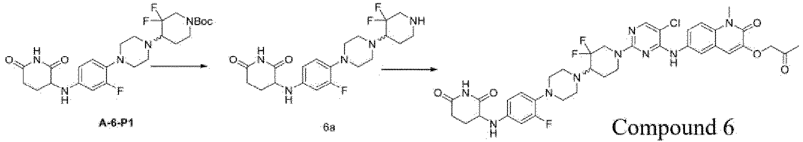

Note: wavy lines in the structures of the present application refer to configuration R or S;

### Step 1: preparation of trifluoroacetate of 6a

To a reaction flask were added A-6-P1 (100 mg, 0.19 mmol), trifluoroacetic acid (1 mL) and dichloromethane (2 mL), and the mixture was reacted at room temperature for 2 h. The reaction solution was concentrated under reduced pressure to obtain crude trifluoroacetate of 6a (100 mg).

### Step 2: preparation of compound 6

To a reaction flask were respectively added the above-mentioned crude trifluoroacetate of 6a (100 mg), sodium bicarbonate (80 mg, 0.95 mmol) and DMSO (5 mL). The mixture was reacted at room temperature for 0.5 h. 1D (75 mg, 0.19 mmol) and DIPEA (0.16 mL) were added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 15 min). Lyophilization was performed to obtain compound 6 (15.0 mg, yield: 10%).

LCMS m/z = 782.2 [M+1] ⁺.

### Example 7: preparation of trifluoroacetate of compound 7

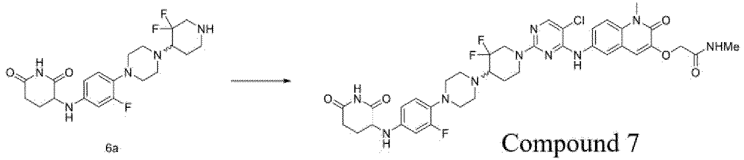

Note: wavy lines in the structures of the present application refer to configuration R or S;

To a reaction flask were added the above-mentioned crude trifluoroacetate of 6a (100 mg), DIPEA (0.31 mL), 4 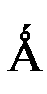 molecular sieve (50 mg) and DMSO (5 mL). The mixture was reacted at room temperature for 0.5 h. **4B** (78 mg, 0.19 mmol) was added, and the resulting mixture was reacted at 90°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 15 min). Lyophilization was performed to obtain the trifluoroacetate of compound 7 (10 mg, yield: 7%).

LCMS m/z = 797.2 [M+1] ⁺.

### Example 8: preparation of compound 8

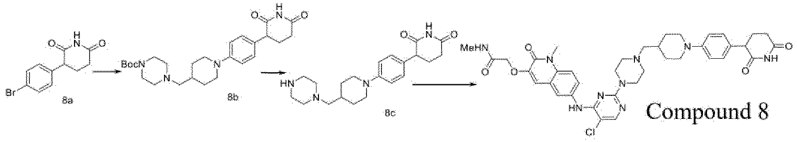

### Step 1: preparation of 8b

Under nitrogen atmosphere, to a reaction flask were respectively added 8a (0.2 g, 0.75 mmol) (see WO 2017197046 for the synthesis method), tert-butyl 4-(piperidin-4-ylmethyl)piperazine-1-carboxylate (0.32 g, 1.13 mmol) (see WO 2020201080 for the synthesis method), RuPhos Pd G3 (0.13 g, 0.16 mmol) and RuPhos (0.07 g, 0.15 mmol). A solution of toluene (5 mL) and LiHMDS in THF (4.5 mL, 1 mol/L) was added, and the mixture was reacted at 80°C for 1.5 h. The reaction solution was cooled to 0°C. The reaction was quenched by dropwise adding a saturated aqueous ammonium chloride solution (10 mL) and extracted with ethyl acetate (30 mL×3). The organic phase was dried over anhydrous sodium sulphate, and concentrated under reduced pressure. The crude was separated and purified by silica gel column chromatography (dichloromethane/methanol (v/v) = 10:1) to obtain 8b (0.09 g, yield: 25%).

LCMS m/z = 471.6 [M+1] ⁺.

### Step 2: preparation of trifluoroacetate of 8c

To a reaction flask were added 8b (0.09 g, 0.19 mmol), trifluoroacetic acid (1.0 mL) and dichloromethane (2 mL), and the mixture was reacted at room temperature for 2 h. The reaction solution was concentrated under reduced pressure to obtain crude trifluoroacetate of 8c (0.09 g).

### Step 3: preparation of compound 8

To a reaction flask were respectively added the crude trifluoroacetate of 8c (90 mg), sodium bicarbonate (80 mg, 0.95 mmol) and DMSO (5 mL). The mixture was stirred at room temperature for 0.5 h. **4B** (78 mg, 0.19 mmol) (see WO 2021077010 for the synthesis method) and DIPEA (0.16 mL) were added, and the resulting mixture was reacted at 100°C for 7 h. The reaction system was cooled to room temperature and subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 15 min). Lyophilization was performed to obtain compound 8 (20 mg, yield: 14%).

¹H NMR (400 MHz, DMSO-*d*₆) δ 10.80 (s, 1H), 9.09 (s, 1H), 8.16 (s, 1H), 8.01 - 7.93 (m, 1H), 7.91 (d, 1H), 7.72 (dd, 1H), 7.52 - 7.46 (m, 1H), 7.17 (s, 1H), 7.14 - 7.06 (m, 2H), 7.06 - 6.94 (m, 2H), 4.60 (s, 2H), 4.56 - 4.42 (m, 2H), 3.80 - 3.65 (m, 6H), 3.64 - 3.52 (m, 2H), 3.40 - 3.23 (m, 2H), 3.15 - 2.97 (m, 4H), 2.89 - 2.71 (m, 2H), 2.71 - 2.57 (m, 4H), 2.50 - 2.42 (m, 1H), 2.24 - 1.94 (m, 3H), 1.92 - 1.79 (m, 2H), 1.46 - 1.28 (m, 2H).

LCMS m/z = 742.3 [M+1] ⁺.

### Example 9: preparation of compound 9

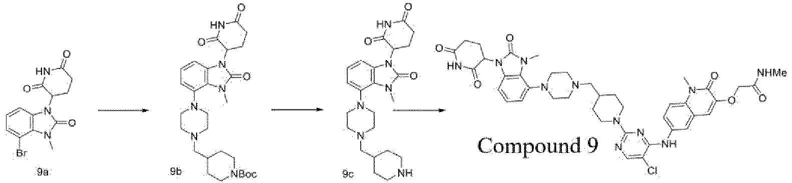

### Step 1: preparation of 9b

Under nitrogen atmosphere, to a reaction flask were respectively added 9a (0.60 g, 1.78 mmol) (see WO 2021158634 for the synthesis method), tert-butyl 4-(piperazin-1-ylmethyl)piperidine-1-carboxylate (0.75 g, 2.65 mmol), RuPhos Pd G3 (0.30 g, 0.36 mmol) and RuPhos (0.17 g, 0.36 mmol). A solution of toluene (10 mL) and LiHMDS (1 mol/L) in THF (10.6 mL, 10.6 mmol) was added, and the mixture was reacted at 80°C for 1.5 h. The reaction solution was cooled to 0°C in an ice bath. The reaction system was quenched by dropwise adding a saturated aqueous ammonium chloride solution (10 mL) and extracted with ethyl acetate (30 mL×3). The organic phases were combined, dried over anhydrous sodium sulphate, and concentrated under reduced pressure. The resulting crude was separated and purified by silica gel column chromatography (dichloromethane/methanol (v/v) = 20:1-10:1) to obtain 9b (0.14 g, yield: 15%).

### Step 2: preparation of trifluoroacetate of 9c

To a reaction flask were added 9b (0.07 g, 0.13 mmol), trifluoroacetic acid (1.0 mL) and dichloromethane (2 mL), and the mixture was reacted at room temperature for 2 h. The reaction solution was concentrated under reduced pressure to obtain crude trifluoroacetate of 9c (0.07 g).

### Step 3: preparation of compound 9

To a reaction flask were respectively added the crude trifluoroacetate of 9c (70 mg), sodium bicarbonate (55 mg, 0.65 mmol) and DMSO (5 mL). The mixture was stirred at room temperature for 0.5 h. **4B** (53 mg, 0.13 mmol) (see WO 2021077010 for the synthesis method) and DIPEA (0.11 mL) were added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 14 min). Lyophilization was performed to obtain compound 9 (22 mg, yield: 21%).

¹H NMR (400 MHz, DMSO-*d*₆) δ 11.06 (s, 1H), 8.80 (s, 1H), 8.04 (s, 1H), 7.99 - 7.89 (m, 2H), 7.82 - 7.71 (m, 1H), 7.54 - 7.44 (m, 1H), 7.12 (s, 1H), 7.03 - 6.84 (m, 3H), 5.41 - 5.29 (m, 1H), 4.58 (s, 2H), 4.54 - 4.41 (m, 2H), 3.68 (s, 3H), 3.62 (s, 3H), 3.10 - 2.55 (m, 14H), 2.40 - 1.68 (m, 8H), 1.14 - 0.96 (m, 2H).

LCMS m/z = 812.3 [M+1] ⁺.

### Example 10: preparation of compound 10

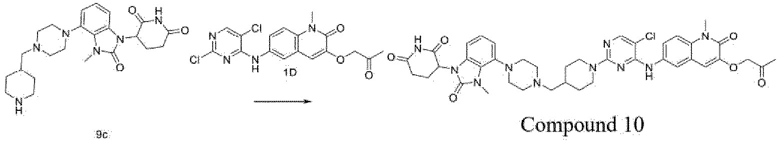

To a reaction flask were respectively added the crude trifluoroacetate of 9c (70 mg), sodium bicarbonate (55 mg, 0.65 mmol) and DMSO (5 mL). The mixture was stirred at room temperature for 0.5 h. 1D (51 mg, 0.13 mmol) (see WO 2021077010 for the synthesis method) and DIPEA (0.11 mL) were added, and the resulting mixture was stirred at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 18 min). Lyophilization was performed to obtain compound 10 (20 mg, yield: 19%).

¹H NMR (400 MHz, DMSO-*d*₆) δ 11.06 (br.s, 1H), 8.80 (s, 1H), 8.03 (s, 1H), 7.94 - 7.85 (m, 1H), 7.78 - 7.67 (m, 1H), 7.52 - 7.42 (m, 1H), 7.06 - 6.82 (m, 4H), 5.40 - 5.29 (m, 1H), 4.91 (s, 2H), 4.56 - 4.42 (m, 2H), 3.67 (s, 3H), 3.62 (s, 3H), 3.11 - 2.55 (m, 11H), 2.35 - 2.10 (m, 7H), 2.06 - 1.93 (m, 1H), 1.88 - 1.70 (m, 3H), 1.15 - 0.97 (m, 2H).

LCMS m/z = 797.3 [M+1] ⁺.

### Example 11: preparation of trifluoroacetate of compound 11

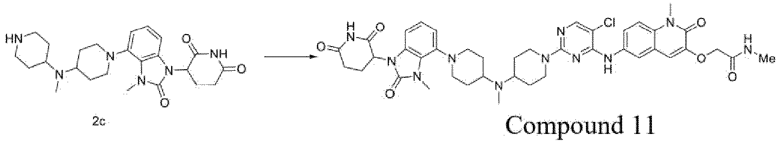

To a reaction flask were respectively added the above-mentioned crude trifluoroacetate of 2c (0.11 g), sodium bicarbonate (0.09 g, 1.07 mmol) and DMSO (5 mL). The mixture was stirred at room temperature for 0.5 h. **4B** (0.09 g, 0.22 mmol) (see WO 2021077010 for the synthesis method) and DIPEA (0.18 mL) were added, and the resulting mixture was reacted at 100°C for 7 h. The reaction system was cooled to room temperature. The reaction solution was poured into water (50 mL) and filtered. The filter cake was collected, dissolved by adding a mixed solvent of dichloromethane/methanol (v/v = 2:1) (50 mL), dried over anhydrous sodium sulphate and concentrated under reduced pressure. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 11 (24 mg).

¹H NMR (400 MHz, DMSO-*d*₆) δ 11.08 (s, 1H), 8.97 (s, 1H), 8.10 (s, 1H), 8.02 - 7.90 (m, 2H), 7.78 - 7.70 (m, 1H), 7.53 - 7.44 (m, 1H), 7.13 (s, 1H), 7.04 - 6.86 (m, 3H), 5.41 - 5.31 (m, 1H), 4.70 - 4.56 (m, 4H), 3.86 - 3.48 (m, 8H), 3.28 - 3.15 (m, 2H), 3.04 - 2.56 (m, 13H), 2.25 - 1.47 (m, 9H).

LCMS m/z = 827.0 [M+1] ⁺.

### Example 12: preparation of compound 12

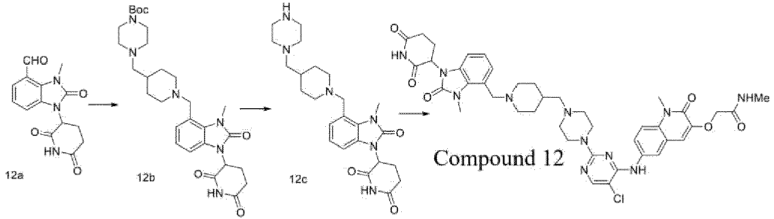

### Step 1: preparation of 12b

To a reaction flask were respectively added tert-butyl 4-(piperidin-4-ylmethyl)piperazine-1-carboxylate (0.18 g, 0.64 mmol) (see WO 2020201080 for the synthesis method), 12a (0.12 g, 0.41 mmol) (see WO 2020113233 for the synthesis method), glacial acetic acid (0.1 mL), 1,2-dichloroethane (3 mL) and DMSO (1 mL). The mixture was stirred at room temperature for 0.5 h, and then sodium triacetoxyborohydride (0.18 g, 0.85 mmol) was added. The resulting mixture was reacted at room temperature for 16 h. To the reaction system was added a saturated aqueous sodium bicarbonate solution (10 mL), and the mixture was extracted with ethyl acetate (20 mL×3). The organic phases were combined, dried over anhydrous sodium sulphate and concentrated under reduced pressure. The crude was separated and purified by silica gel column chromatography (dichloromethane/methanol (v/v) = 10:1) to obtain 12b (95 mg, yield: 42%).

LCMS m/z = 555.8 [M+1] ⁺.

### Step 2: preparation of trifluoroacetate of 12c

To a reaction flask were added 12b (95 mg, 0.17 mmol), trifluoroacetic acid (0.5 mL) and dichloromethane (2 mL), and the mixture was reacted at room temperature for 2 h. The reaction solution was concentrated under reduced pressure to obtain crude trifluoroacetate of 12c (90 mg).

### Step 3: preparation of compound 12

To a reaction flask were respectively added the above-mentioned crude trifluoroacetate of 12c (90 mg), sodium bicarbonate (71 mg, 0.85 mmol) and DMSO (5 mL). The mixture was stirred at room temperature for 0.5 h. **4B** (69 mg, 0.17 mmol) and DIPEA (0.14 mL) were added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 12 (20 mg, yield: 14%).

¹H NMR (400 MHz, DMSO-*d*₆) δ 8.83 (s, 1H), 8.04 (s, 1H), 7.99 - 7.89 (m, 2H), 7.77 - 7.70 (m, 1H), 7.50 - 7.42 (m, 1H), 7.12 (s, 1H), 7.09 - 7.02 (m, 1H), 6.99 - 6.91 (m, 1H), 6.90 - 6.83 (m, 1H), 5.44 - 5.30 (m, 1H), 4.58 (s, 2H), 3.72 - 3.54 (m, 12H), 2.96 - 2.56 (m, 8H), 2.41 - 2.28 (m, 4H), 2.18 - 1.90 (m, 5H), 1.74 - 1.46 (m, 3H), 1.14 - 0.98 (m, 2H).

LCMS m/z = 826.3 [M+1] ⁺.

### Example 13: preparation of trifluoroacetate of compound 13

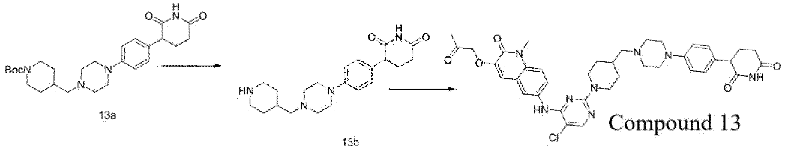

### Step 1: preparation of trifluoroacetate of 13b

To a reaction flask (50 mL) were successively added 13a (0.10 g, 0.21 mmol) (see WO 2022133285 for the synthesis method), dichloromethane (2 mL) and trifluoroacetic acid (0.5 mL), and the mixture was reacted at room temperature for 1 h. The reaction solution was concentrated under reduced pressure to obtain crude trifluoroacetate of 13b (0.10 g).

### Step 2: preparation of trifluoroacetate of compound 13

To a reaction flask (25 mL) were successively added the above-mentioned crude trifluoroacetate of 13b (0.10 g), 1D (0.075 g, 0.19 mmol) (see WO 2021077010 for the synthesis method), DIPEA (0.31 mL) and DMSO (5 mL), and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 12 min). Lyophilization was performed to obtain the trifluoroacetate of compound 13 (15 mg).

¹H NMR (400 MHz, DMSO-*d*₆) δ 10.77 (s, 1H), 8.99 (s, 1H), 8.08 (s, 1H), 7.84 (d, 1H), 7.72 (dd, 1H), 7.45 (d, 1H), 7.15 - 7.06 (m, 3H), 7.01 - 6.94 (m, 2H), 4.93 - 4.87 (m, 2H), 4.53 - 4.39 (m, 2H), 4.00 - 3.50 (m, 8H), 3.25 - 2.83 (m, 8H), 2.73 - 2.57 (m, 1H), 2.50 - 2.42 (m, 1H), 2.24 - 1.94 (m, 6H), 1.84 - 1.69 (m, 2H), 1.27 - 1.07 (m, 2H).

LCMS m/z = 727.3 [M+1] ⁺.

### Example 14: preparation of trifluoroacetate of compound 14

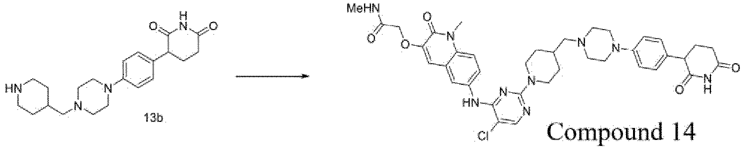

To a reaction flask (25 mL) were successively added the above-mentioned crude trifluoroacetate of 13b (0.10 g), **4B** (0.078 g, 0.19 mmol), DIPEA (0.31 mL) and DMSO (5 mL), and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 13 min). Lyophilization was performed to obtain the trifluoroacetate of compound 14 (20 mg).

¹H NMR (400 MHz, DMSO-*d*₆) δ 10.78 (s, 1H), 9.00 - 8.91 (m, 1H), 8.08 (s, 1H), 8.01 - 7.88 (m, 2H), 7.74 (dd, 1H), 7.47 (d, 1H), 7.16 - 7.06 (m, 3H), 7.02 - 6.93 (m, 2H), 4.59 (s, 2H), 4.55 - 4.40 (m, 2H), 3.87 - 3.72 (m, 3H), 3.68 (s, 3H), 3.65 - 3.54 (m, 2H), 3.26 - 2.84 (m, 8H), 2.71 - 2.58 (m, 4H), 2.49 - 2.41 (m, 1H), 2.23 - 2.08 (m, 2H), 2.07 - 1.95 (m, 1H), 1.85 - 1.71 (m, 2H), 1.27 - 1.11 (m, 2H).

LCMS m/z = 742.3 [M+1] ⁺.

### Example 15: preparation of compound 15

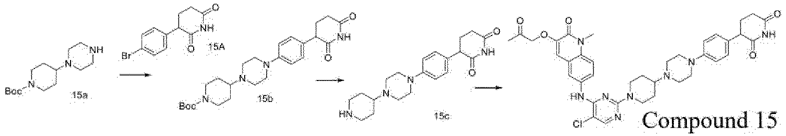

### Step 1: preparation of 15b

To a reaction flask were respectively added 15A (see WO 2017197046 for the synthesis method) (0.30 g, 1.12 mmol), 15a (see WO 2022019597 for the synthesis method) (0.30 g, 1.11 mmol) and toluene (10 mL). Under nitrogen protection, RuPhos (0.10 g, 0.21 mmol), RuPhos Pd G3 (0.19 g, 0.227 mmol) and a solution of LiHMDS (1 mol/L) in THF (6.72 mL, 6.72 mmol) were successively added, and the mixture was reacted at 80°C for 1.5 h. The reaction solution was cooled to room temperature. A saturated aqueous ammonium chloride solution (15 mL) was added, and the mixture was extracted with ethyl acetate (10 mL×3). The organic phases were combined, dried over anhydrous sodium sulphate and concentrated under reduced pressure. The crude was separated and purified by silica gel column chromatography (dichloromethane/methanol (v/v) = 10:1) to obtain 15b (0.23 g, yield: 45%).

LCMS m/z = 457.6 [M+1] ⁺.

### Step 2: preparation of trifluoroacetate of 15c

To a reaction flask were added 15b (0.28 g, 0.61 mmol), trifluoroacetic acid (1.5 mL) and dichloromethane (5 mL), and the mixture was reacted at room temperature for 2 h. The reaction solution was concentrated under reduced pressure to obtain crude trifluoroacetate of 15c (0.30 g).

### Step 3: preparation of compound 15

To a reaction flask were respectively added the above-mentioned crude trifluoroacetate of 15c (0.15 g), DIPEA (0.54 g, 4.2 mmol) and DMSO (5 mL). The mixture was stirred at room temperature for 0.5 h. 1D (0.15 g, 0.38 mmol) (see WO 2021077010 for the synthesis method) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 15 (10 mg, yield: 4%).

¹H NMR (400 MHz, DMSO-*d*₆) δ 10.74 (s, 1H), 8.82 (s, 1H), 8.04 (s, 1H), 7.94 - 7.86 (m, 1H), 7.70 (dd, 1H), 7.46 (d, 1H), 7.08 - 6.99 (m, 3H), 6.93 - 6.82 (m, 2H), 4.90 (s, 2H), 4.60 - 4.40 (m, 2H), 3.76 - 3.64 (m, 4H), 3.14 - 3.04 (m, 4H), 2.92 - 2.77 (m, 2H), 2.71 - 2.56 (m, 5H), 2.49 - 2.39 (m, 2H), 2.24 - 1.94 (m, 5H), 1.89 - 1.75 (m, 2H), 1.52 - 1.20 (m, 2H).

LCMS m/z = 713.8 [M+1] ⁺.

### Example 16: preparation of compound 16

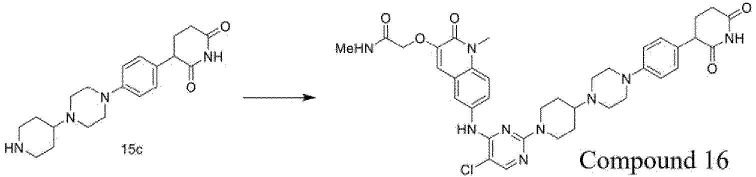

To a reaction flask were respectively added the above-mentioned crude trifluoroacetate of 15c (0.15 g), DIPEA (0.54 g, 4.2 mmol) and DMSO (5 mL). The mixture was stirred at room temperature for 0.5 h. **4B** (0.15 g, 0.37 mmol) (see WO 2021077010 for the synthesis method) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 16 (15 mg, yield: 6%).

¹H NMR (400 MHz, DMSO-*d*₆) δ 10.75 (s, 1H), 8.83 (s, 1H), 8.07 - 7.89 (m, 3H), 7.80 - 7.72 (m, 1H), 7.52 - 7.45 (m, 1H), 7.18 - 6.98 (m, 3H), 6.94 - 6.82 (m, 2H), 4.63 - 4.42 (m, 4H), 3.77 - 3.61 (m, 4H), 3.15 - 3.03 (m, 4H), 2.94 - 2.80 (m, 2H), 2.73 - 2.56 (m, 8H), 2.55 - 2.38 (m, 2H), 2.20 - 1.92 (m, 2H), 1.90 - 1.80 (m, 2H), 1.54 - 1.18 (m, 2H).

LCMS m/z = 728.3 [M+1] ⁺.

### Example 17: preparation of trifluoroacetate of compound 17

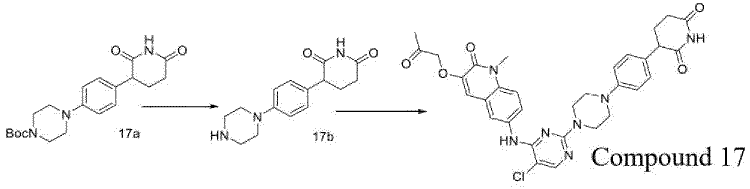

### Step 1: preparation of trifluoroacetate of 17b

To a reaction flask (50 mL) were successively added 17a (0.22 g, 0.59 mmol) (see WO 2022133285 for the synthesis method), dichloromethane (2 mL) and trifluoroacetic acid (1.0 mL), and the mixture was reacted at room temperature for 1 h. The reaction system was concentrated under reduced pressure to obtain crude trifluoroacetate of 17b (0.22 g).

### Step 2: preparation of trifluoroacetate of compound 17

To a reaction flask (25 mL) were successively added the above-mentioned crude trifluoroacetate of 17b (0.11 g), 1D (0.11 g, 0.28 mmol) (see WO 2021077010 for the synthesis method), DIPEA (0.46 mL) and DMSO (5 mL), and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 11 min). Lyophilization was performed to obtain the trifluoroacetate of compound 17 (10 mg).

¹H NMR (400 MHz, DMSO-*d*₆) δ 10.75 (s, 1H), 9.00 (s, 1H), 8.11 (s, 1H), 7.90 (d, 1H), 7.71 (dd, 1H), 7.48 (d, 1H), 7.13 - 7.03 (m, 3H), 7.00 - 6.92 (m, 2H), 4.91 (s, 2H), 3.84 - 3.70 (m, 5H), 3.68 (s, 3H), 3.24 - 3.13 (m, 4H), 2.71 - 2.56 (m, 1H), 2.50 - 2.41 (m, 1H), 2.24 - 1.95 (m, 5H).

LCMS m/z = 630.2 [M+1] ⁺.

### Example 18: preparation of compound 18

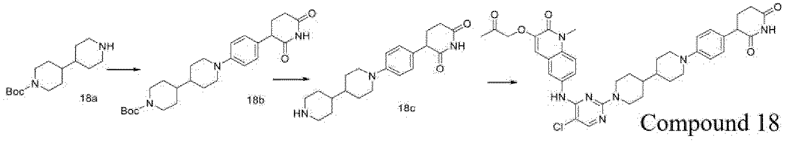

Compound 18 was prepared using compound 18a as the raw material and referring to the synthesis method of example 15.

¹H NMR (400 MHz, DMSO-*d₆*) δ 10.74 (s, 1H), 8.80 (s, 1H), 8.03 (s, 1H), 7.94 - 7.88 (m, 1H), 7.71 (dd, 1H), 7.45 (d, 1H), 7.07 - 6.97 (m, 3H), 6.93 - 6.83 (m, 2H), 4.90 (s, 2H), 4.60 - 4.45 (m, 2H), 3.78 - 3.61 (m, 6H), 2.84 - 2.51 (m, 5H), 2.49 - 2.40 (m, 1H), 2.25 - 1.92 (m, 5H), 1.85 - 1.65 (m, 4H), 1.46 - 1.02 (m, 6H).

LCMS m/z = 356.7 [M/2+1] ⁺.

### Example 19: preparation of compound 19

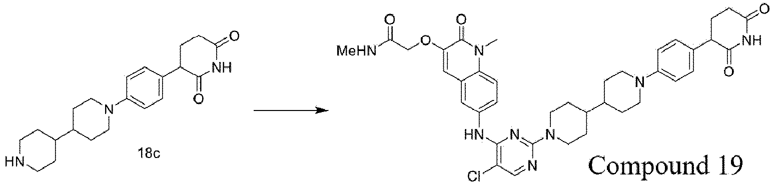

To a reaction flask were respectively added the above-mentioned crude trifluoroacetate of 18c (0.15 g), DIPEA (0.39 g, 3.02 mmol) and DMSO (5 mL). The mixture was stirred at room temperature for 0.5 h. **4B** (0.11 g, 0.27 mmol) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 19 (15 mg, yield: 8%).

¹H NMR (400 MHz, DMSO-*d₆*) δ 10.83 (s, 1H), 9.16 (s, 1H), 8.10 (s, 1H), 8.02 - 7.88 (m, 2H), 7.74 (dd, 1H), 7.54 - 7.46 (m, 1H), 7.34 - 6.95 (m, 5H), 4.58 (s, 2H), 4.55 - 4.42 (m, 2H), 3.90 - 3.77 (m, 1H), 3.73 - 3.57 (m, 5H), 3.25 - 2.95 (m, 2H), 2.94 - 2.76 (m, 2H), 2.74 - 2.57 (m, 4H), 2.50 - 2.42 (m, 1H), 2.27 - 2.10 (m, 1H), 2.07 - 1.95 (m, 1H), 1.95 - 1.82 (m, 2H), 1.82 - 1.70 (m, 2H), 1.60 - 1.27 (m, 4H), 1.25 - 1.05 (m, 2H).

### Example 20: preparation of trifluoroacetate of compound 20

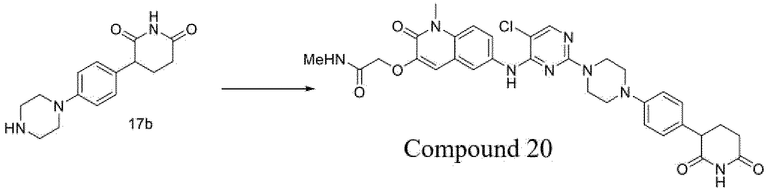

To a reaction flask (25 mL) were successively added the crude trifluoroacetate of 17b (0.11 g), **4B** (0.11 g, 0.27 mmol), DIPEA (0.46 mL) and DMSO (5 mL), and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 13 min). Lyophilization was performed to obtain the trifluoroacetate of compound 20 (18 mg).

¹H NMR (400 MHz, DMSO-*d*₆) δ 10.76 (s, 1H), 9.21 - 9.07 (m, 1H), 8.17 - 8.10 (m, 1H), 8.03 - 7.92 (m, 2H), 7.75 (dd, 1H), 7.51 (d, 1H), 7.18 (s, 1H), 7.13 - 7.04 (m, 2H), 7.03 - 6.94 (m, 2H), 4.60 (s, 2H), 4.00 - 3.68 (m, 8H), 3.28 - 3.15 (m, 4H), 2.72 - 2.57 (m, 4H), 2.50 - 2.39 (m, 1H), 2.22 - 1.94 (m, 2H).

LCMS m/z = 645.2 [M+1] ⁺.

### Example 21: preparation of trifluoroacetate of compound 21

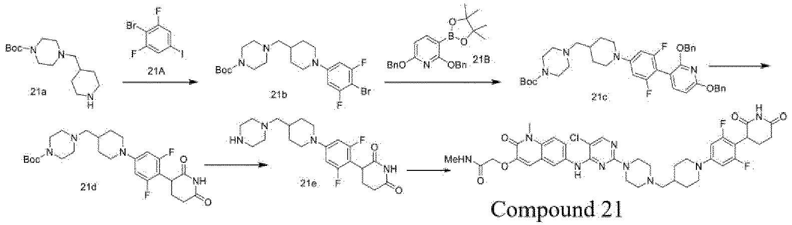

### Step 1: preparation of 21b

21a (2.93 g, 10.34 mmol), potassium carbonate (6.49 g, 46.96 mmol), 21A (3.0 g, 9.41 mmol), L-proline (0.43 g, 3.73 mmol) and CuI (0.36 g, 1.89 mmol) were added to a reaction flask, and DMSO (80 mL) was added. The mixture was subjected to nitrogen replacement three times, and reacted at 90°C for 12 h. The reaction solution was cooled to room temperature, and ethyl acetate (100 mL) and water (100 mL) were added. The aqueous phase was extracted with ethyl acetate (20 mL×2), and the organic phases were combined, dried over anhydrous sodium sulphate, and concentrated under reduced pressure. The crude was separated and purified by silica gel column chromatography (petroleum ether/ethyl acetate (v/v) = 100:1-1:1) to obtain 21b (2.0 g, yield: 45%).

LCMS m/z = 474.6 [M+1]⁺.

### Step 2: preparation of 21c

21b (1.50 g, 3.16 mmol), 21B (see WO 2021262812 for the synthesis method) (1.45 g, 3.47 mmol), Pd(dppf)Cl₂·DCM (CAS: 95464-05-4) (0.26 g, 0.32 mmol) and caesium carbonate (2.06 g, 6.32 mmol) were added to a reaction flask, and 1,4-dioxane (10 mL) and water (2 mL) were added. The mixture was subjected to nitrogen replacement three times, and reacted at 100°C for 20 h. The reaction solution was cooled to room temperature, and water (100 mL) and ethyl acetate (100 mL) were added. Liquid separation was carried out. The aqueous phase was extracted with ethyl acetate (20 mL×2), and the organic phases were combined, washed with a saturated aqueous sodium chloride solution (20 mL), dried over anhydrous sodium sulphate and concentrated under reduced pressure. The crude was separated and purified by silica gel column chromatography (petroleum ether/ethyl acetate (v/v) = 1:0-20:1) to obtain 21c (0.85 g, yield: 39%).

LCMS m/z = 685.4 [M+1]⁺.

### Step 3: preparation of 21d

21c (450 mg, 0.66 mmol), 10% palladium on carbon (330 mg) and methanol (15 mL) were successively added to a reaction flask. The mixture was subjected to hydrogen replacement three times, and reacted at 45°C under the atmosphere of hydrogen balloon for 20 h. The reaction system was cooled to room temperature and filtered by suction. The filtrate was concentrated under reduced pressure to obtain crude 21d (0.30 g).

LCMS m/z = 507.3 [M+1]⁺.

### Step 4: preparation of trifluoroacetate of 21e

The above-mentioned crude 21d (150 mg) was dissolved in dichloromethane (4 mL). Trifluoroacetic acid (2 mL) was added, and the mixture was reacted at room temperature for 3 h. The reaction system was concentrated under reduced pressure to obtain crude trifluoroacetate of 21e (160 mg).

LCMS m/z = 407.5 [M+1]⁺.

### Step 5: preparation of trifluoroacetate of compound 21

To a reaction flask were respectively added the above-mentioned crude trifluoroacetate of 21e (78 mg), DIPEA (200 mg, 1.55 mmol) and DMSO (15 mL). **4B** (61 mg, 0.15 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 21 (15 mg).

¹H NMR (400 MHz, DMSO-*d₆*) δ 10.86 (s, 1H), 9.07 (s, 1H), 8.15 (s, 1H), 8.01 - 7.87 (m, 2H), 7.72 (dd, 1H), 7.48 (d, 1H), 7.17 (s, 1H), 6.71 - 6.59 (m, 2H), 4.59 (s, 2H), 4.55 - 4.40 (m, 2H), 4.10 - 4.00 (m, 1H), 3.87 - 3.75 (m, 2H), 3.68 (s, 3H), 3.63 - 3.51 (m, 2H), 3.41 - 3.23 (m, 2H), 3.14 - 2.97 (m, 4H), 2.86 - 2.71 (m, 3H), 2.66 (d, 3H), 2.56 - 2.51 (m, 1H), 2.20 - 1.70 (m, 5H), 1.37 - 1.15 (m, 2H).

LCMS m/z = 778.8 [M+1] ⁺.

### Example 22: preparation of compound 22

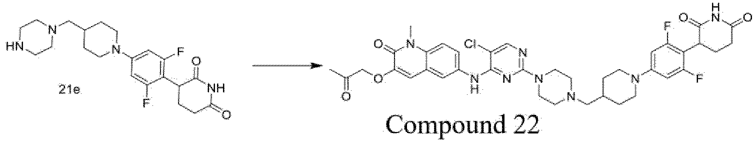

To a reaction flask were respectively added the above-mentioned crude trifluoroacetate of 21e (78 mg), DIPEA (200 mg, 1.55 mmol) and DMSO (15 mL). 1D (60 mg, 0.15 mmol) (see WO 2021077010 for the synthesis method) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 22 (4.5 mg, yield: 4%).

¹H NMR (400 MHz, DMSO-*d₆*) δ 10.85 (s, 1H), 8.85 (s, 1H), 8.04 (s, 1H), 7.93 - 7.86 (m, 1H), 7.70 (dd, 1H), 7.50 - 7.42 (m, 1H), 7.05 (s, 1H), 6.70 - 6.53 (m, 2H), 4.91 (s, 2H), 4.09 - 3.98 (m, 1H), 3.81 - 3.70 (m, 2H), 3.66 (s, 3H), 3.65 - 3.58 (m, 4H), 2.85 - 2.64 (m, 4H), 2.44 - 2.30 (m, 4H), 2.24 - 1.90 (m, 7H), 1.85 - 1.67 (m, 3H), 1.20 - 1.05 (m, 2H).

LCMS m/z = 763.9 [M+1] ⁺.

### Example 23: preparation of compound 23

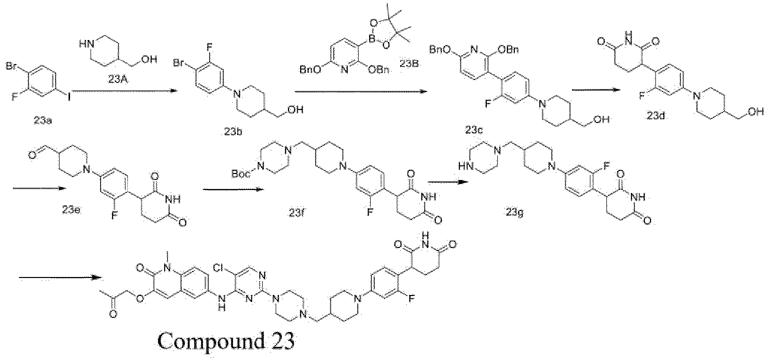

### Step 1: preparation of 23b

To a reaction flask were added 23A (1.5 g, 13.02 mmol), potassium carbonate (3.6 g, 26.05 mmol), 23a (3.92 g, 13.03 mmol), L-proline (0.6 g, 5.21 mmol) and CuI (0.5 g, 2.63 mmol). DMSO (30 mL) was added, and the mixture was subjected to nitrogen replacement three times, and reacted at 50°C for 16 h. The reaction solution was cooled to room temperature, and water (50 mL) was added. The mixture was extracted with ethyl acetate (30 mL×3). The organic phase was dried over anhydrous sodium sulphate, and concentrated under reduced pressure. The crude was separated and purified by silica gel column chromatography (petroleum ether : ethyl acetate (v/v) = 3:2) to obtain 23b (2.5 g, yield: 67%).

LCMS m/z = 288.3 [M+1]⁺.

### Step 2: preparation of 23c

Under nitrogen protection, to a reaction flask were added 23b (2.5 g, 8.70 mmol), 23B (see WO 2021262812 for the synthesis method) (12.68 g, 30.34 mmol), Pd(dppf)Cl₂·DCM (CAS: 95464-05-4) (0.71 g, 0.87 mmol) and caesium carbonate (11.31 g, 34.71 mmol). 1,4-dioxane (40 mL) and water (8 mL) were added, and the mixture was subjected to nitrogen replacement three times, and reacted at 100°C for 6 h. The reaction solution was cooled to room temperature, and water (30 mL) was added. The mixture was extracted with ethyl acetate (50 mL×3). The organic phase was dried over anhydrous sodium sulphate, and concentrated under reduced pressure. The crude was separated and purified by silica gel column chromatography (petroleum ether/ethyl acetate (v/v) = 1:1) to obtain 23c (3.2 g, yield: 74%).

LCMS m/z = 499.3 [M+1]⁺.

### Step 3: preparation of 23d

23c (3.2 g, 6.42 mmol) and 10% palladium on carbon (3 g) were added to THF (100 mL). The mixture was subjected to hydrogen replacement three times, and reacted at room temperature under the atmosphere of hydrogen balloon for 16 h. The reaction system was filtered over diatomaceous earth. The filtrate was concentrated under reduced pressure, and the crude was separated and purified by silica gel column chromatography (dichloromethane/methanol (v/v) = 20:1) to obtain 23d (0.91 g, yield: 44%).

LCMS m/z = 321.4 [M+1]⁺.

### Step 4: preparation of 23e:

23d (0.30 g, 0.94 mmol) was dissolved in DMSO (3 mL). 2-iodoxybenzoic acid (0.42 g, 1.50 mmol) was added, and the mixture was reacted at 50°C for 2 h. The reaction solution was cooled to room temperature, and water (30 mL) was added. The mixture was extracted with ethyl acetate (30 mL×3), and the organic phase was dried over anhydrous sodium sulphate, and concentrated under reduced pressure to obtain crude 23e (0.28 g).

LCMS m/z = 319.3 [M+1]⁺.

### Step 5: preparation of 23f

The above-mentioned crude 23e (0.28 g) was dissolved in 1,2-dichloroethane (30 ml). Tert-butyl piperazine-1-carboxylate (0.25 g, 1.34 mmol) and acetic acid (0.33 mL) were added. The mixture was stirred at room temperature for 1 h, and then sodium triacetoxyborohydride (0.56 g, 2.64 mmol) was added. The resulting mixture was reacted at room temperature for 16 h. To the reaction solution was slowly added a saturated aqueous sodium bicarbonate solution (50 mL), and the mixture was extracted with dichloromethane (30 mL). The organic phase was washed with a saturated aqueous sodium chloride solution (50 mL), dried over anhydrous sodium sulphate, and concentrated under reduced pressure. The crude was separated and purified by silica gel column chromatography (dichloromethane/methanol (v/v) = 10:1) to obtain 23f (0.33 g, yield: 50%).

LCMS m/z = 489.3 [M+1]⁺.

### Step 6: preparation of trifluoroacetate of 23g

23f (330 mg, 0.676 mmol) was dissolved in dichloromethane (4 mL). Trifluoroacetic acid (2 mL) was added, and the mixture was reacted at room temperature for 3 h. The reaction solution was concentrated under reduced pressure to obtain crude trifluoroacetate of 23g (340 mg).

LCMS m/z = 389.3 [M+1]⁺.

### Step 7: preparation of compound 23

To a reaction flask were respectively added the above-mentioned crude trifluoroacetate of 23g (150 mg), DIPEA (301 mg, 2.33 mmol) and DMSO (10 mL). 1D (100 mg, 0.25 mmol) (see WO 2021077010 for the synthesis method) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 23 (24 mg, yield: 13%).

¹H NMR (400 MHz, DMSO-*d₆*) δ 10.78 (s, 1H), 8.85 (s, 1H), 8.05 (s, 1H), 7.89 (d, 1H), 7.70 (dd, 1H), 7.45 (d, 1H), 7.12 - 7.00 (m, 2H), 6.77 - 6.63 (m, 2H), 4.91 (s, 2H), 3.93 - 3.82 (m, 1H), 3.77 - 3.55 (m, 9H), 2.78 - 2.60 (m, 3H), 2.58 - 2.45 (m, 1H), 2.44 - 2.30 (m, 4H), 2.25 - 2.06 (m, 6H), 2.03 - 1.90 (m, 1H), 1.85 - 1.63 (m, 3H), 1.28 - 1.08 (m, 2H).

LCMS m/z = 745.3 [M+1] ⁺.

### Example 24: preparation of trifluoroacetate of compound 24

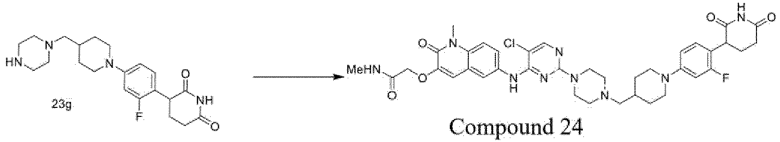

To a reaction flask were respectively added the above-mentioned crude trifluoroacetate of 23g (140 mg), DIPEA (301 mg, 2.33 mmol) and DMSO (10 mL). **4B** (100 mg, 0.24 mmol) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 24 (50 mg).

¹H NMR (400 MHz, DMSO-*d₆*) δ 10.80 (s, 1H), 9.07 (s, 1H), 8.15 (s, 1H), 8.00 - 7.86 (m, 2H), 7.72 (dd, 1H), 7.48 (d, 1H), 7.17 (s, 1H), 7.13 - 7.03 (m, 1H), 6.82 - 6.68 (m, 2H), 4.62 - 4.38 (m, 4H), 3.94 - 3.83 (m, 1H), 3.83 - 3.70 (m, 2H), 3.68 (s, 3H), 3.64 - 3.50 (m, 2H), 3.40 - 3.22 (m, 2H), 3.15 - 2.95 (m, 4H), 2.80 - 2.60 (m, 6H), 2.57 - 2.47 (m, 1H), 2.25 - 1.89 (m, 3H), 1.88 - 1.73 (m, 2H), 1.43 - 1.20 (m, 2H).

LCMS m/z = 760.8 [M+1]⁺.

The trifluoroacetate of compound 24 was subjected to chiral resolution to obtain chiral isomer 1 and chiral isomer 2, respectively. Resolution conditions were as follows:
1. Instrument: Waters 150 Prep-SFC E; chromatographic column: Chiralcel AD column.
2. The sample was dissolved in acetonitrile and filtered with a 0.45 µm filter to prepare a sample liquid.
3. Preparative chromatography conditions: a. the mobile phase consists of systems A and B: mobile phase A: CO₂; mobile phase B: a mixed solvent of methanol/acetonitrile containing 0.1% ammonia water; b. isocratic elution, mobile phase B content: 65%; c. flow rate: 100 mL/min.

Chiral analysis method:
1. Instrument: SHIMAZU LC-30AD; chromatographic column: Chiral AD column.
2. Analytical chromatography conditions: a. the mobile phase consists of systems A and B: mobile phase A: CO₂; mobile phase B: a solution of 0.05% diethylamine in isopropanol and acetonitrile; b. isocratic elution, mobile phase B content: 60%; c. flow rate: 3 mL/min.

Retention time: chiral isomer 1: 1.028 min, chiral isomer 2: 1.431 min.

NMR characterization of chiral isomer 1:
¹H NMR (400 MHz, DMSO-*d₆*) δ 10.77 (s, 1H), 8.84 (s, 1H), 8.20 - 8.00 (m, 1H), 7.98 - 7.87 (m, 2H), 7.74 (dd, 1H), 7.47 (d, 1H), 7.17 - 7.00 (m, 2H), 6.75 - 6.62 (m, 2H), 4.58 (s, 2H), 3.94 - 3.82 (m, 1H), 3.76 - 3.58 (m, 9H), 2.80 - 2.59 (m, 6H), 2.56 - 2.45 (m, 1H), 2.44 - 2.32 (m, 4H), 2.25 - 2.05 (m, 3H), 2.03 - 1.88 (m, 1H), 1.86 - 1.64 (m, 3H), 1.30 - 1.10 (m, 2H).

NMR characterization of chiral isomer 2:
¹H NMR (400 MHz, DMSO-*d₆*) δ 10.77 (s, 1H), 8.84 (s, 1H), 8.20 - 8.00 (m, 1H), 7.98 - 7.87 (m, 2H), 7.74 (dd, 1H), 7.47 (d, 1H), 7.17 - 7.00 (m, 2H), 6.75 - 6.62 (m, 2H), 4.58 (s, 2H), 3.94 - 3.82 (m, 1H), 3.76 - 3.58 (m, 9H), 2.80 - 2.59 (m, 6H), 2.56 - 2.45 (m, 1H), 2.44 - 2.32 (m, 4H), 2.25 - 2.05 (m, 3H), 2.03 - 1.88 (m, 1H), 1.86 - 1.64 (m, 3H), 1.30 - 1.10 (m, 2H).

### Example 25: preparation of trifluoroacetate of compound 25

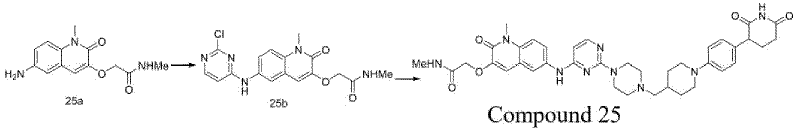

### Step 1: preparation of 25b

To a reaction flask (50 mL) were respectively added 25a (0.3 g, 1.15 mmol) (see WO 2019119138 for the synthesis method), 2,4-dichloropyrimidine (0.21 g, 1.41 mmol), DIPEA (0.45 g, 3.48 mmol) and DMF (5 mL), and the mixture was subjected to nitrogen replacement three times, and reacted at 80°C for 2 h. The reaction system was cooled to room temperature. The solid was precipitated and filtered. The filter cake was washed with a mixed solvent (6 mL) of dichloromethane/methanol (v/v) = 2:1, and dried under reduced pressure to obtain 25b (0.23 g, yield: 54%).

LCMS m/z = 374.1 [M+1] ⁺.

### Step 2: preparation of trifluoroacetate of compound 25

To a reaction flask were respectively added the above-mentioned crude trifluoroacetate of 8c (70 mg), 25b (71 mg, 0.19 mmol), DIPEA (0.12 g, 0.93 mmol) and DMSO (5 mL), and the mixture was subjected to nitrogen replacement three times, and reacted at 100°C for 16 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 50% (elution time: 20 min). Lyophilization was performed to obtain the trifluoroacetate of compound 25 (10.0 mg).

¹H NMR (400 MHz, DMSO-*d*₆) δ 9.36 (s, 1H), 8.01 - 7.88 (m, 3H), 7.67 (dd, 1H), 7.46 (d, 1H), 7.12 (s, 1H), 7.07 - 6.99 (m, 2H), 6.93 - 6.84 (m, 2H), 6.05 (d, 1H), 4.58 (s, 2H), 3.76 - 3.62 (m, 10H), 2.71 - 2.57 (m, 6H), 2.49 - 2.38 (m, 5H), 2.26 - 1.94 (m, 4H), 1.90 - 1.60 (m, 3H), 1.33 - 1.12 (m, 2H).

LCMS m/z = 355.0 [M/2+1] ⁺.

### Example 26: preparation of compound 26

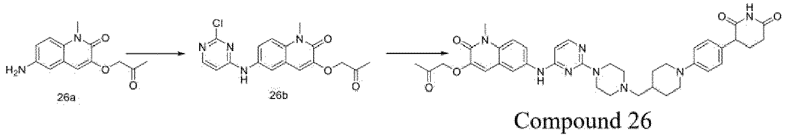

Compound 26 was prepared using 26a (see WO 2021077010 for the synthesis method) as the raw material and referring to the synthesis method of example 25.

¹H NMR (400 MHz, DMSO-*d*₆) δ 10.75 (s, 1H), 9.32 (s, 1H), 7.93 (d, 1H), 7.87 (d, 1H), 7.66 (dd, 1H), 7.44 (d, 1H), 7.08 - 6.98 (m, 3H), 6.93 - 6.84 (m, 2H), 6.04 (d, 1H), 4.91 (s, 2H), 3.80 - 3.58 (m, 10H), 2.72 - 2.57 (m, 3H), 2.50 - 2.35 (m, 5H), 2.26 - 1.94 (m, 7H), 1.90 - 1.61 (m, 3H), 1.33 - 1.14 (m, 2H).

LCMS m/z = 693.4 [M+1] ⁺.

### Example 27: preparation of trifluoroacetate of compound 27

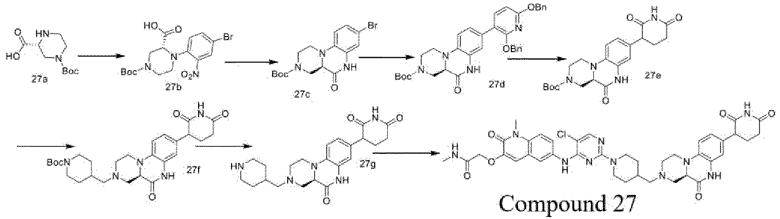

### Step 1: preparation of 27b

To a reaction flask were added 27a (5.00 g, 21.71 mmol), 4-bromo-1-fluoro-2-nitrobenzene (7.16 g, 32.55 mmol), caesium carbonate (21.22 g, 65.13 mmol) and DMSO (30 mL), and the mixture was reacted at 110°C for 2 h. The reaction solution was cooled to room temperature, and ethyl acetate (50 mL) and water (50 mL) were added. The pH was adjusted to 5 with hydrochloric acid (3 mol/L). Liquid separation was carried out. The aqueous phase was extracted with ethyl acetate (20 mL×2). The organic phases were combined, dried over anhydrous sodium sulphate, and concentrated under reduced pressure. The crude was separated and purified by silica gel column chromatography (petroleum ether/ethyl acetate (v/v) = 1:1) to obtain 27b (7.6 g, yield: 81%).

### Step 2: preparation of 27c

To a reaction flask were added 27b (6.60 g, 15.34 mmol), zinc powder (5.02 g, 77.234 mmol), ammonium chloride (8.21 g, 153.48 mmol) and ethanol (80 mL), and the mixture was reacted at reflux for 4 h. The reaction solution was cooled to room temperature, and filtered over diatomaceous earth. The filtrate was concentrated under reduced pressure, and ethyl acetate (50 mL) and water (50 mL) were added. Liquid separation was carried out. The aqueous phase was extracted with ethyl acetate (20 mL×2). The organic phases were combined, dried over anhydrous sodium sulphate, and concentrated under reduced pressure. The crude was separated and purified by silica gel column chromatography (petroleum ether/ethyl acetate (v/v) = 3:1) to obtain 27c (2.30 g, yield: 39%).

### Step 3: preparation of 27d

To a reaction flask were respectively added 27c (1.40 g, 3.66 mmol), 1,4-dioxane (20 mL) and water (5 mL). Under nitrogen protection, 2,6-bis(benzyloxy)-3-(4,4,5,5-tetramethyl-1,3,2-dioxaborolan-2-yl)pyridine (4.58 g, 10.98 mmol) (see WO 2021262812 for the synthesis method), caesium carbonate (3.58 g, 10.99 mmol) and Pd(dppf)Cl₂ (0.27 g, 0.37 mmol) were added, and the mixture was subjected to nitrogen replacement three times, and reacted at 100°C for 16 h. The reaction system was cooled to room temperature, and filtered over diatomaceous earth. To the filtrate were added ethyl acetate (50 mL) and water (50 mL). The aqueous phase was extracted with ethyl acetate (20 mL×3). The organic phases were combined, washed with a saturated aqueous sodium chloride solution (20 mL), dried over anhydrous sodium sulphate, and concentrated under reduced pressure. The crude was separated and purified by silica gel column chromatography (petroleum ether : ethyl acetate (v/v) = 2:1) to obtain 27d (2.0 g, yield: 92%).

### Step 4: preparation of 27e:

To a reaction flask were respectively added 27d (2.0 g, 3.37 mmol), THF (40 mL) and 10% Pd/C (1.00 g). The mixture was subjected to hydrogen replacement three times, and reacted at room temperature under the atmosphere of hydrogen balloon for 16 h. The reaction system was filtered over diatomaceous earth. The filtrate was concentrated under reduced pressure and the crude was separated and purified by silica gel column chromatography (dichloromethane:methanol (v/v) = 10:1) to obtain 27e (0.85 g, yield: 61%).

### Step 5: preparation of 27f

To a reaction flask were added 27e (0.30 g, 0.72 mmol), trifluoroacetic acid (1.5 mL) and dichloromethane (5 mL), and the mixture was reacted at room temperature for 2 h. The reaction solution was concentrated under reduced pressure, and adjusted to pH 7 by adding triethylamine. The mixture was concentrated under reduced pressure. THF (6 mL) was added to the residue. Tert-butyl 4-formylpiperidine-1-carboxylate (0.20 g, 0.94 mmol), acetic acid (0.5 mL) and sodium triacetoxyborohydride (0.31 g, 1.46 mmol) were successively added, and the resulting mixture was reacted at room temperature for 16 h. To the reaction solution were added ethyl acetate (10 mL) and a saturated aqueous sodium bicarbonate solution (10 mL). The aqueous phase was extracted with ethyl acetate (5 mL×3). The organic phases were combined, washed with a saturated aqueous sodium chloride solution (10 mL), dried over anhydrous sodium sulphate, and concentrated under reduced pressure. The crude was separated and purified by silica gel column chromatography (dichloromethane : methanol (v/v) = 20:1) to obtain 27f (0.31 g, yield: 84%).

LCMS m/z = 512.3 [M+1] ⁺.

### Step 6: preparation of trifluoroacetate of 27g

To a reaction flask were added 27f (0.31 g, 0.61 mmol), trifluoroacetic acid (1.5 mL) and dichloromethane (5 mL), and the mixture was reacted at room temperature for 2 h. The reaction solution was concentrated under reduced pressure to obtain crude trifluoroacetate of 27g (0.38 g).

### Step 7: preparation of trifluoroacetate of compound 27

To a reaction flask were respectively added the above-mentioned crude trifluoroacetate of 27g (0.19 g), DIPEA (0.47 g, 3.64 mmol) and DMSO (5 mL). The mixture was stirred at room temperature for 0.5 h. **4B** (0.12 g, 0.29 mmol) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 27 (66 mg).

¹H NMR (400 MHz, DMSO-*d*₆) δ 10.81 (s, 2H), 8.99 (s, 1H), 8.09 (s, 1H), 8.02 - 7.89 (m, 2H), 7.75 (dd, 1H), 7.47 (d, 1H), 7.12 (s, 1H), 6.93 - 6.82 (m, 2H), 6.76 - 6.69 (m, 1H), 4.59 (s, 2H), 4.54 - 4.40 (m, 2H), 4.00 - 3.62 (m, 8H), 3.35 - 2.86 (m, 7H), 2.74 - 2.58 (m, 4H), 2.54 - 2.42 (m, 1H), 2.35 - 1.92 (m, 3H), 1.89 - 1.71 (m, 2H), 1.30 - 1.10 (m, 2H).

LCMS m/z = 392.2 [M/2+1] ⁺.

### Example 28: preparation of compound 28

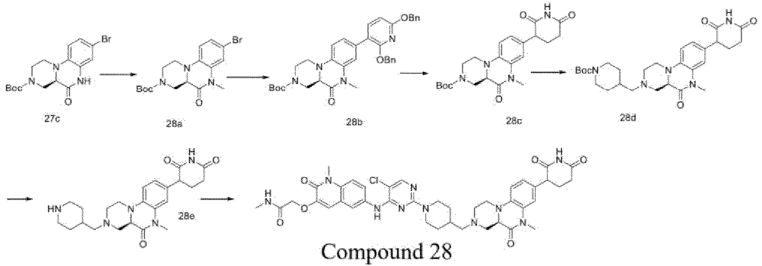

### Step 1: preparation of 28a

To a reaction flask were added 27c (1.3 g, 3.40 mmol), iodomethane (1.45 g, 10.22 mmol), caesium carbonate (3.32 g, 10.19 mmol) and DMF (30 mL), and the mixture was reacted at room temperature for 16 h. The reaction solution was cooled to room temperature, and ethyl acetate (20 mL) and water (20 mL) were added. Liquid separation was carried out. The aqueous phase was extracted with ethyl acetate (10 mL×2). The organic phases were combined, dried over anhydrous sodium sulphate, and concentrated under reduced pressure. The crude was separated and purified by silica gel column chromatography (petroleum ether/ethyl acetate (v/v) = 3:1) to obtain 28a (1.25 g, yield: 93%).

### Step 2: preparation of 28b

To a reaction flask were respectively added 28a (1.30 g, 3.28 mmol), 1,4-dioxane (20 mL) and water (5 mL). Under nitrogen protection, 2,6-bis(benzyloxy)-3-(4,4,5,5-tetramethyl-1,3,2-dioxaborolan-2-yl)pyridine (4.11 g, 9.85 mmol) (see WO 2021262812 for the synthesis method), caesium carbonate (3.21 g, 9.85 mmol) and Pd(dppf)Cl₂ (0.24 g, 0.33 mmol) were added, and the mixture was subjected to nitrogen replacement three times, and reacted at 100°C for 16 h. The reaction system was cooled to room temperature, and filtered over diatomaceous earth. To the filtrate were added ethyl acetate (50 mL) and water (50 mL). The aqueous phase was extracted with ethyl acetate (20 mL×3). The organic phases were combined, washed with a saturated aqueous sodium chloride solution (20 mL), dried over anhydrous sodium sulphate, and concentrated under reduced pressure. The crude was separated and purified by silica gel column chromatography (petroleum ether : ethyl acetate (v/v) = 2:1) to obtain 28b (1.80 g, yield: 90%).

### Step 3: preparation of 28c

To a reaction flask were respectively added 28b (1.8 g, 2.97 mmol), THF (40 mL) and 10% Pd/C (0.9 g). The mixture was subjected to hydrogen replacement three times, and reacted at room temperature under the atmosphere of hydrogen balloon for 16 h. The reaction system was filtered over diatomaceous earth. The filtrate was concentrated under reduced pressure and the crude was separated and purified by silica gel column chromatography (dichloromethane:methanol (v/v) = 10:1) to obtain 28c (0.90 g, yield: 71%).

### Step 4: preparation of 28d:

To a reaction flask were added 28c (0.30 g, 0.70 mmol), trifluoroacetic acid (1.5 mL) and dichloromethane (5 mL), and the mixture was reacted at room temperature for 2 h. The reaction solution was concentrated under reduced pressure, and adjusted to pH 7 by adding triethylamine. The mixture was concentrated under reduced pressure. THF (6 mL) was added. Tert-butyl 4-formylpiperidine-1-carboxylate (0.19 g, 0.891 mmol), acetic acid (0.5 mL) and sodium triacetoxyborohydride (0.30 g, 1.42 mmol) were successively added, and the resulting mixture was reacted at room temperature for 16 h. To the reaction solution were added ethyl acetate (10 mL) and a saturated aqueous sodium bicarbonate solution (10 mL). The aqueous phase was extracted with ethyl acetate (5 mL×3). The organic phases were combined, washed with a saturated aqueous sodium chloride solution (10 mL), dried over anhydrous sodium sulphate, and concentrated under reduced pressure. The crude was separated and purified by silica gel column chromatography (dichloromethane : methanol (v/v) = 20:1) to obtain 28d (0.30 g, yield: 82%).

LCMS m/z = 526.3 [M+1] ⁺.

### Step 5: preparation of trifluoroacetate of 28e

To a reaction flask were added 28d (0.30 g, 0.57 mmol), trifluoroacetic acid (1.5 mL) and dichloromethane (5 mL), and the mixture was reacted at room temperature for 2 h. The reaction solution was concentrated under reduced pressure to obtain crude trifluoroacetate of 28e (0.36 g).

### Step 6: preparation of compound 28

To a reaction flask were respectively added the above-mentioned crude trifluoroacetate of 28e (0.18 g), DIPEA (0.43 g, 3.30 mmol) and DMSO (5 mL). The mixture was stirred at room temperature for 0.5 h. **4B** (0.11 g, 0.27 mmol) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 28 (70 mg, yield: 33%).

¹H NMR (400 MHz, DMSO-*d*₆) δ 10.78 (s, 1H), 8.79 (s, 1H), 8.03 (s, 1H), 7.98 - 7.88 (m, 2H), 7.77 (dd, 1H), 7.47 (d, 1H), 7.10 (s, 1H), 6.98 - 6.92 (m, 1H), 6.91 - 6.77 (m, 2H), 4.63 - 4.41 (m, 4H), 3.85 - 3.74 (m, 1H), 3.73 - 3.63 (m, 3H), 3.58 - 3.47 (m, 1H), 3.47 - 3.37 (m, 1H), 3.25 (s, 3H), 3.00 - 2.78 (m, 3H), 2.76 - 2.58 (m, 5H), 2.56 - 2.43 (m, 2H), 2.34 - 2.16 (m, 3H), 2.14 - 1.95 (m, 3H), 1.90 - 1.70 (m, 3H), 1.15 - 0.97 (m, 2H).

LCMS m/z = 399.5 [M/2+1] ⁺.

### Example 29: preparation of compound 29

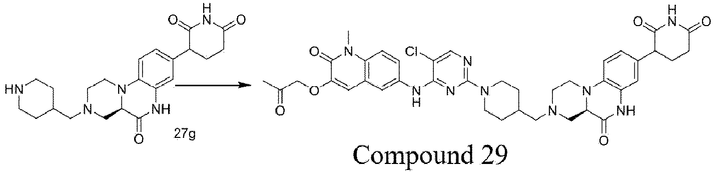

To a reaction flask were respectively added the above-mentioned crude trifluoroacetate of 27g (0.19 g), DIPEA (0.47 g, 3.64 mmol) and DMSO (5 mL). The mixture was stirred at room temperature for 0.5 h. 1D (0.12 g, 0.31 mmol) (see WO 2021077010 for the synthesis method) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 29 (20 mg, yield: 8%).

¹H NMR (400 MHz, DMSO-*d*₆) δ 10.78 (s, 1H), 10.46 (s, 1H), 8.79 (s, 1H), 8.03 (s, 1H), 7.90 (d, 1H), 7.72 (dd, 1H), 7.45 (d, 1H), 7.02 (s, 1H), 6.83 - 6.73 (m, 2H), 6.66 (s, 1H), 4.89 (s, 2H), 4.55 - 4.40 (m, 2H), 3.77 - 3.68 (m, 1H), 3.66 (s, 3H), 3.63 - 3.51 (m, 1H), 3.47 - 3.37 (m, 1H), 3.30 - 3.22 (m, 1H), 2.99 - 2.77 (m, 3H), 2.75 - 2.57 (m, 2H), 2.50 - 2.41 (m, 1H), 2.30 - 2.21 (m, 2H), 2.18 (s, 3H), 2.14 - 1.92 (m, 4H), 1.90 - 1.65 (m, 3H), 1.14 - 0.96 (m, 2H).

LCMS m/z = 384.6 [M/2+1] ⁺.

### Example 30: preparation of compound 30

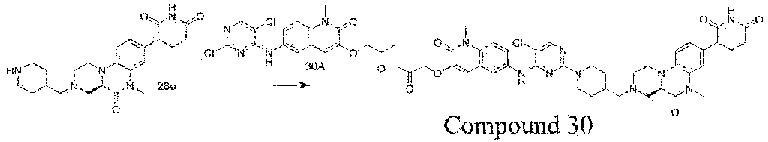

To a reaction flask were respectively added the above-mentioned crude trifluoroacetate of 28e (0.18 g), DIPEA (0.43 g, 3.30 mmol) and DMSO (5 mL). The mixture was stirred at room temperature for 0.5 h. 30A (0.10 g, 0.26 mmol) (see WO 2021077010 for the synthesis method) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 30 (20 mg, yield: 10%).

¹H NMR (400 MHz, DMSO-*d*₆) δ 10.78 (s, 1H), 8.79 (s, 1H), 8.03 (s, 1H), 7.90 (d, 1H), 7.72 (dd, 1H), 7.45 (d, 1H), 7.02 (s, 1H), 6.98 - 6.92 (m, 1H), 6.91 - 6.78 (m, 2H), 4.89 (s, 2H), 4.56 - 4.40 (m, 2H), 3.85 - 3.75 (m, 1H), 3.66 (s, 3H), 3.58 - 3.48 (m, 1H), 3.42 (dd, 1H), 3.37 - 3.28 (m, 1H), 3.25 (s, 3H), 3.02 - 2.77 (m, 3H), 2.77 - 2.57 (m, 2H), 2.55 - 2.42 (m, 1H), 2.35 - 2.14 (m, 6H), 2.14 - 1.95 (m, 3H), 1.92 - 1.65 (m, 3H), 1.14 - 0.95 (m, 2H).

LCMS m/z = 391.7 [M/2+1] ⁺.

### Example 31: preparation of trifluoroacetate of compound 31

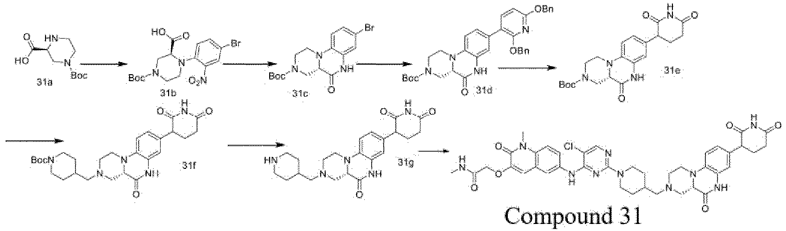

The trifluoroacetate of compound 31 was prepared using compound 31a as the raw material and referring to the synthesis method of example 27.

¹H NMR (400 MHz, DMSO-*d*₆) δ 10.81 (s, 2H), 8.94 (s, 1H), 8.08 (s, 1H), 8.02 - 7.90 (m, 2H), 7.75 (dd, 1H), 7.47 (d, 1H), 7.12 (s, 1H), 6.93 - 6.81 (m, 2H), 6.73 (s, 1H), 4.59 (s, 2H), 4.54 - 4.40 (m, 2H), 4.05 - 3.45 (m, 8H), 3.34 - 2.85 (m, 7H), 2.74 - 2.57 (m, 4H), 2.55 - 2.40 (m, 1H), 2.37 - 1.93 (m, 3H), 1.88 - 1.70 (m, 2H), 1.30 - 1.10 (m, 2H).

LCMS m/z = 392.4 [M/2+1] ⁺.

### Example 32: preparation of trifluoroacetate of compound 32

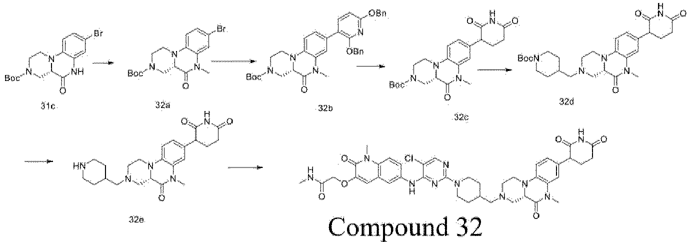

The trifluoroacetate of compound 32 was prepared using compound 31c as the raw material and referring to the synthesis method of example 28.

¹H NMR (400 MHz, DMSO-*d*₆) δ 10.81 (s, 1H), 9.07 (s, 1H), 8.11 (s, 1H), 8.04 - 7.86 (m, 2H), 7.79 - 7.69 (m, 1H), 7.47 (d, 1H), 7.12 (s, 1H), 7.05 (s, 1H), 6.99 - 6.90 (m, 2H), 4.59 (s, 2H), 4.52 - 4.39 (m, 2H), 4.03 - 3.71 (m, 5H), 3.68 (s, 3H), 3.37 - 2.88 (m, 10H), 2.74 - 2.61 (m, 4H), 2.58 - 2.47 (m, 1H), 2.37 - 2.13 (m, 2H), 2.08 - 1.93 (m, 1H), 1.90 - 1.72 (m, 2H), 1.30 - 1.10 (m, 2H).

LCMS m/z = 399.4 [M/2+1] ⁺.

### Example 33: preparation of trifluoroacetate of compound 33

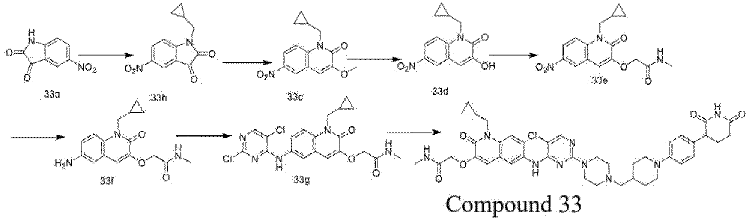

### Step 1: preparation of 33b

To a reaction flask were added 33a (9.90 g, 51.53 mmol), bromomethylcyclopropane (10.43 g, 77.26 mmol), potassium carbonate (17.80 g, 128.8 mmol) and DMF (100 mL), and the mixture was reacted at room temperature for 16 h. To the reaction solution were added ethyl acetate (100 mL) and a saturated aqueous sodium chloride solution (100 mL), and the pH was adjusted to 6 with hydrogen chloride solution (1 mol/L). Liquid separation was carried out. The aqueous phase was extracted with ethyl acetate (100 mL×3). The organic phases were combined, washed with a saturated aqueous sodium chloride solution (100 mL), dried over anhydrous sodium sulphate, and concentrated under reduced pressure to obtain a crude (12 g). To a reaction flask were respectively added the above-mentioned crude (12 g), DIPEA (11.74 g, 90.83 mmol), HATU (18.99 g, 49.94 mmol) and dichloromethane (200 mL), and the mixture was reacted at room temperature for 2 h. To the reaction solution was added water (100 mL). Liquid separation was carried out. The aqueous phase was extracted with dichloromethane (50 mL×2). The organic phases were combined, washed with a saturated aqueous sodium chloride solution (50 mL), dried over anhydrous sodium sulphate, and concentrated under reduced pressure. The crude was separated and purified by silica gel column chromatography (petroleum ether : ethyl acetate (v/v) = 1:1) to obtain 33b (5.2 g, two-step yield: 41%).

LCMS m/z = 247.0 [M+1] ⁺.

### Step 2: preparation of 33c

To a reaction flask were added 33b (5.2 g, 21.14 mmol), triethylamine (4.45 g, 43.98 mmol) and ethanol (100 mL). Under nitrogen protection, a solution of trimethylsilyldiazomethane in n-hexane (22.00 mL, 44 mmol, 2 mol/L) was added dropwise, and the mixture was reacted at room temperature for 3 h. To the reaction solution was added ethyl acetate (300 mL) and a saturated aqueous sodium chloride solution (300 mL). Liquid separation was carried out. The aqueous phase was extracted with ethyl acetate (50 mL×3). The organic phases were combined, washed with a saturated aqueous sodium chloride solution (100 mL), dried over anhydrous sodium sulphate, and concentrated under reduced pressure. The crude was separated and purified by silica gel column chromatography (petroleum ether : ethyl acetate (v/v) = 1:1) to obtain 33c (3.1 g, yield: 53%).

LCMS m/z = 275.1 [M+1] ⁺.

### Step 3: preparation of 33d

To a reaction flask were added 33c (3.1 g, 11.30 mmol) and dichloromethane (50 mL). Under nitrogen protection, the mixture was cooled to 0°C, and a solution of boron tribromide in dichloromethane (33.90 mL, 33.90 mmol, 1 mol/L) was added dropwise. The resulting mixture was reacted at room temperature for 2 h. To the reaction solution was added dichloromethane (30 mL). Liquid separation was carried out. The aqueous phase was extracted with dichloromethane (20 mL×3). The organic phases were combined, washed with a saturated aqueous sodium chloride solution (30 mL), dried over anhydrous sodium sulphate, and concentrated under reduced pressure. The crude was separated and purified by silica gel column chromatography (petroleum ether : ethyl acetate (v/v) = 1:1) to obtain 33d (2.3 g, yield: 78%).

LCMS m/z = 261.2 [M+1] ⁺.

### Step 4: preparation of 33e:

To a reaction flask were added 33d (2.1 g, 8.07 mmol), caesium carbonate (5.26 g, 16.14 mmol), 2-bromo-N-methylacetamide (1.47 g, 9.67 mmol) and DMF (40 mL), and the mixture was reacted at room temperature for 2 h. The reaction solution was poured into ice water (200 mL), and filtered. The filter cake was washed with water (10 mL) to obtain crude 33e (9.0 g).

### Step 5: preparation of 33f

To a reaction flask were added the above-mentioned crude 33e (9.0 g), dichloromethane (70 mL), methanol (70 mL) and 10% Pd/C (1.6 g), and the mixture was subjected to hydrogen replacement three times, and reacted at room temperature under the atmosphere of hydrogen balloon for 16 h. The reaction system was filtered over diatomaceous earth, and the filtrate was concentrated under reduced pressure to obtain crude 33f (2.1 g).

LCMS m/z = 302.3 [M+1] ⁺.

### Step 6: preparation of 33g

To a reaction flask were added the above-mentioned crude 33f (2.1 g), 2,4,5-trichloropyrimidine (1.53 g, 8.34 mmol) and DMF (30 mL), and the mixture was reacted at 70°C for 2 h. The reaction solution was cooled to room temperature, poured into ice water (150 mL), and filtered. The filter cake was washed with water (20 mL), and dried by blowing at 50°C to obtain crude 33g (2.9 g).

### Step 7: preparation of trifluoroacetate of compound 33

To a reaction flask were respectively added the above-mentioned crude trifluoroacetate of 8c (0.25 g), DIPEA (0.67 g, 5.18 mmol) and DMSO (5 mL). The mixture was stirred at room temperature for 0.5 h. The above-mentioned crude 33g (0.19 g) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 33 (200 mg).

¹H NMR (400 MHz, DMSO-*d*₆) δ 10.83 - 10.77 (m, 1H), 9.16 - 9.06 (m, 1H), 8.19 - 8.14 (m, 1H), 8.05 - 7.91 (m, 2H), 7.77 - 7.69 (m, 1H), 7.65 - 7.58 (m, 1H), 7.27 - 7.09 (m, 5H), 4.65 - 4.40 (m, 4H), 4.30 - 4.18 (m, 2H), 3.88 - 3.50 (m, 5H), 3.45 - 3.22 (m, 2H), 3.20 - 2.88 (m, 6H), 2.74 - 2.57 (m, 4H), 2.55 - 2.43 (m, 1H), 2.28 - 1.80 (m, 5H), 1.60 - 1.18 (m, 3H), 0.54 - 0.40 (m, 4H).

LCMS m/z = 391.7 [M/2+1] ⁺.

### Example 34: preparation of trifluoroacetate of compound 34

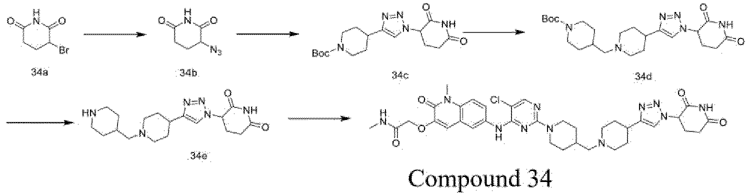

### Step 1: preparation of 34b

To a reaction flask were added 34a (1.50 g, 7.81 mmol), acetone (12 mL) and water (6 mL). Under nitrogen protection, sodium azide (2.55 g, 39.2 mmol) was added, and the mixture was reacted at room temperature for 16 h. The reaction solution was cooled to room temperature, and ethyl acetate (30 mL) and a saturated aqueous sodium chloride solution (50 mL) were added. Liquid separation was carried out. The aqueous phase was extracted with ethyl acetate (15 mL×2). The organic phases were combined, washed with a saturated aqueous sodium chloride solution (50 mL), dried over anhydrous sodium sulphate, and concentrated under reduced pressure to obtain crude 34b (0.92 g).

### Step 2: preparation of 34c

To a reaction flask were respectively added the above-mentioned crude 34b (0.20 g), 1-Boc-4-ethynylpiperidine (0.33 g, 1.58 mmol), anhydrous copper sulphate (0.02 g, 0.125 mmol), sodium ascorbate (0.10 g, 0.505 mmol), THF (3 mL) and water (1 mL), and the mixture was reacted at room temperature for 3 h. The reaction system was filtered. Ethyl acetate (10 mL) and water (10 mL) were added to the filtrate. The aqueous phase was extracted with ethyl acetate (10 mL×3). The organic phases were combined, washed with a saturated aqueous sodium chloride solution (20 mL), dried over anhydrous sodium sulphate, and concentrated under reduced pressure. The crude was separated and purified by silica gel column chromatography (dichloromethane : methanol (v/v) = 10:1) to obtain 34c (0.45 g, yield: 78%).

### Step 3: preparation of 34d

To a reaction flask were added 34c (0.45 g, 1.24 mmol), trifluoroacetic acid (2 mL) and dichloromethane (5 mL), and the mixture was reacted at room temperature for 2 h. The reaction solution was concentrated under reduced pressure, and adjusted to pH 7 with triethylamine. The mixture was concentrated under reduced pressure. THF (10 mL) was added. Tert-butyl 4-formylpiperidine-1-carboxylate (0.29 g, 1.36 mmol), acetic acid (0.5 mL) and sodium triacetoxyborohydride (0.53 g, 2.50 mmol) were successively added, and the resulting mixture was reacted at room temperature for 16 h. To the reaction solution were added ethyl acetate (20 mL) and a saturated aqueous sodium bicarbonate solution (20 mL). The aqueous phase was extracted with ethyl acetate (10 mL×3). The organic phases were combined, washed with a saturated aqueous sodium chloride solution (20 mL), dried over anhydrous sodium sulphate, and concentrated under reduced pressure. The crude was separated and purified by silica gel column chromatography (dichloromethane : methanol (v/v) = 5:1) to obtain 34d (0.38 g, yield: 67%).

### Step 4: preparation of trifluoroacetate of 34e

To a reaction flask were added 34d (0.19 g, 0.41 mmol), trifluoroacetic acid (1 mL) and dichloromethane (3 mL), and the mixture was reacted at room temperature for 2 h. The reaction solution was concentrated under reduced pressure to obtain crude trifluoroacetate of 34e (0.23 g).

### Step 5: preparation of trifluoroacetate of compound 34

To a reaction flask were respectively added the above-mentioned crude trifluoroacetate of 34e (0.38 g), DIPEA (0.62 g, 4.8 mmol) and DMSO (5 mL). The mixture was stirred at room temperature for 0.5 h. **4B** (0.16 g, 0.39 mmol) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 34 (60 mg).

¹H NMR (400 MHz, DMSO-*d*₆) δ 11.23 (s, 1H), 9.09 - 8.97 (m, 1H), 8.17 - 7.89 (m, 4H), 7.88 - 7.68 (m, 1H), 7.47 (d, 1H), 7.12 (s, 1H), 5.84 - 5.72 (m, 1H), 4.60 (s, 2H), 4.53 - 4.40 (m, 2H), 3.74 - 3.55 (m, 5H), 3.26 - 2.77 (m, 8H), 2.75 - 2.55 (m, 5H), 2.37 - 1.69 (m, 8H), 1.30 - 1.05 (m, 2H).

LCMS m/z = 732.9 [M+1] ⁺.

### Example 35: preparation of compound 35

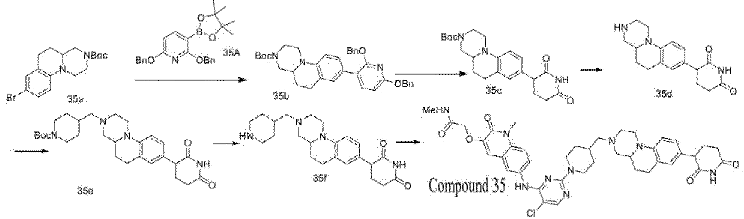

### Step 1: preparation of 35b

35a (see Bioorg. Med. Chem., 2016, 26, 5877 for the synthesis method) (1.50 g, 4.08 mmol), 35A (see WO 2022171123 for the synthesis method) (3.41 g, 8.16 mmol), Pd(dppf)Cl₂·DCM (0.33 g, 0.41 mmol) and caesium carbonate (2.66 g, 8.16 mmol) were successively added to a reaction flask. 1,4-dioxane (30 mL) and water (3 mL) were added, and the mixture was subjected to nitrogen replacement three times, and reacted at 100°C for 20 h. The reaction system was cooled to room temperature, and water (200 mL) and ethyl acetate (200 mL) were added. Liquid separation was carried out. The aqueous phase was extracted with ethyl acetate (50 mL), and the organic phases were combined, washed with a saturated aqueous sodium chloride solution (50 mL), dried over anhydrous sodium sulphate and concentrated under reduced pressure. The crude was separated and purified by silica gel column chromatography (petroleum ether/ethyl acetate (v/v) = 1:0-9:1) to obtain 35b (2.36 g, yield: > 99%).

### Step 2: preparation of 35c

35b (0.95 g, 1.64 mmol) was dissolved in THF (30 mL), and 10% palladium on carbon (0.87 g) was added. The mixture was subjected to hydrogen replacement three times, and reacted at 45°C under the atmosphere of hydrogen balloon for 20 h. The reaction solution was cooled to room temperature, and filtered over diatomaceous earth. The filtrate was concentrated under reduced pressure, and the crude was separated and purified by silica gel column chromatography (methanol :dichloromethane (v/v) = 0:1-5:95) to obtain 35c (0.60 g, yield: 92%).

LCMS m/z = 400.4 [M+1]⁺.

### Step 3: preparation of 35d

35c (600 mg, 1.50 mmol) was dissolved in dichloromethane (4 mL). Trifluoroacetic acid (2 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 35d (450 mg).

LCMS m/z = 300.4 [M+1]⁺.

### Step 4: preparation of 35e:

The above-mentioned crude 35d (0.45 g) was dissolved in dichloroethane (30 mL). Tert-butyl 4-formylpiperidine-1-carboxylate (0.48 g, 2.25 mmol) was added, and acetic acid (3 mL) was added. The mixture was stirred at room temperature for 1 h, and then sodium triacetoxyborohydride (0.64 g, 3.0 mmol) was added. The resulting mixture was reacted at room temperature for 16 h. To the reaction solution was slowly added a saturated aqueous sodium bicarbonate solution (50 mL), and the mixture was extracted twice with dichloromethane (50 mL). The organic phase was washed with a saturated aqueous sodium chloride solution (100 mL), dried over anhydrous sodium sulphate, and concentrated under reduced pressure. The crude was separated and purified by silica gel column chromatography (dichloromethane:methanol (v/v) = 10:1) to obtain 35e (0.33 g, yield: 30%).

LCMS m/z = 497.3 [M+1]⁺.

### Step 5: preparation of trifluoroacetate of 35f

35e (100 mg, 6.65 mmol) was dissolved in dichloromethane (2 mL). Trifluoroacetic acid (1 mL) was added, and the mixture was reacted at room temperature for 3 h. The reaction system was concentrated under reduced pressure to obtain crude trifluoroacetate of 35f (100 mg).

LCMS m/z = 397.3 [M+1]⁺.

### Step 6: preparation of compound 35

To a reaction flask were respectively added the above-mentioned crude trifluoroacetate of 35f (100 mg), DIPEA (200 mg, 1.55 mmol) and DMSO (15 mL). **4B** (80 mg, 0.20 mmol) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 35 (10 mg, yield: 7%).

¹H NMR (400 MHz, DMSO-*d*₆) δ 10.72 (s, 1H), 8.80 (s, 1H), 8.08 - 7.86 (m, 3H), 7.77 (dd, 1H), 7.47 (d, 1H), 7.12 (s, 1H), 6.90 - 6.70 (m, 3H), 4.64 - 4.38 (m, 4H), 3.78 - 3.60 (m, 6H), 2.97 - 2.55 (m, 12H), 2.50 - 2.37 (m, 1H), 2.22 - 1.92 (m, 5H), 1.90 - 1.50 (m, 5H), 1.14 - 0.94 (m, 2H).

LCMS m/z = 768.3 [M+1] ⁺.

### Example 36: preparation of trifluoroacetate of compound 36

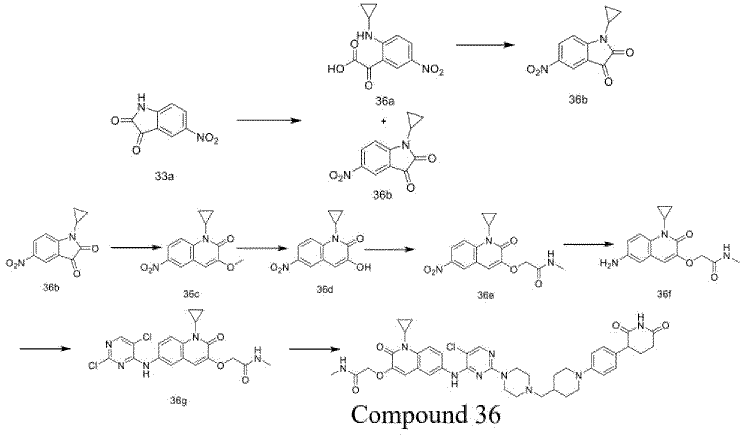

### Step 1: preparation of 36a and 36b

To a reaction flask were added 33a (10 g, 52.05 mmol), cyclopropyl boronic acid (8.94 g, 104.07 mmol), sodium carbonate (11.03 g, 104.07 mmol), 2,2'-bipyridine (8.13 g, 52.06 mmol), anhydrous copper acetate (9.45 g, 52.03 mmol) and 1,2-dichloroethane (200 mL), and the mixture was reacted at 70°C under air atmosphere for 16 h. The reaction solution was cooled to room temperature, and an aqueous ammonium chloride solution (200 mL) was added. The pH was adjusted to 6 with 1 mol/L hydrochloric acid. Liquid separation was carried out. The aqueous phase was extracted with ethyl acetate (100 mL×3). The organic phases were combined, washed with a saturated aqueous sodium chloride solution (100 mL), dried over anhydrous sodium sulphate, and concentrated under reduced pressure. The crude was separated and purified by silica gel column chromatography (first eluted with petroleum ether : ethyl acetate (v/v) = 2:1, and then eluted with dichloromethane : methanol (v/v) = 5:1) to obtain 36b (0.79 g, yield: 7%) and crude 36a (3.5 g), respectively.

### Step 2: preparation of 36b from 36a

To a reaction flask were respectively added the above-mentioned crude 36a (3.5 g), DIPEA (5.42 g, 41.93 mmol), HATU (7.98 g, 20.99 mmol) and dichloromethane (100 mL), and the mixture was reacted at room temperature for 2 h. To the reaction solution was added water (100 mL). Liquid separation was carried out. The aqueous phase was extracted with dichloromethane (50 mL×2). The organic phases were combined, washed with a saturated aqueous sodium chloride solution (50 mL), dried over anhydrous sodium sulphate, and concentrated under reduced pressure. The crude was separated and purified by silica gel column chromatography (petroleum ether : ethyl acetate (v/v) = 2:1) to obtain 36b (1.2 g, two-step yield calculated from compound 33a: 10%).

The trifluoroacetate of compound 36 was prepared by acidic preparative chromatography (water (containing 0.1% TFA)/acetonitrile) and lyophilization using compound 36b as the raw material and referring to the synthesis method of example 33.

¹H NMR (400 MHz, DMSO-*d*₆) δ 10.78 (s, 1H), 9.06 (s, 1H), 8.15 (s, 1H), 8.04 - 7.93 (m, 1H), 7.93 - 7.84 (m, 1H), 7.83 - 7.73 (m, 1H), 7.72 - 7.63 (m, 1H), 7.23 - 7.04 (m, 5H), 4.64 - 4.40 (m, 4H), 3.86 - 3.50 (m, 5H), 3.45 - 3.21 (m, 2H), 3.17 - 2.82 (m, 7H), 2.75 - 2.57 (m, 4H), 2.55 - 2.40 (m, 1H), 2.24 - 1.82 (m, 5H), 1.55 - 1.20 (m, 4H), 0.87 - 0.73 (m, 2H).

LCMS m/z = 385.0 [M/2+1] ⁺.

### Example 37: preparation of trifluoroacetate of compound 37

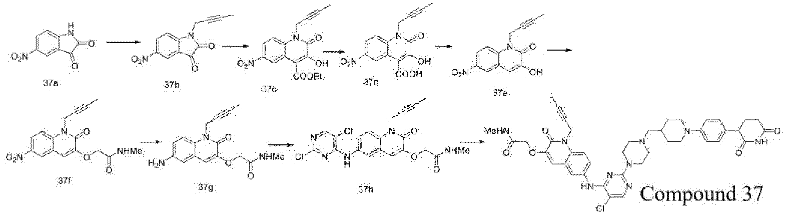

### Step 1: preparation of 37b

To a reaction flask were respectively added 37a (10.0 g, 52.05 mmol) and DMF (100 mL), and the mixture was cooled to 0°C. 60% sodium hydride (3.12 g) was added in batches, and the mixture was reacted at room temperature for 30 min. Then 1-bromo-2-butyne (10.38 g, 78.05 mmol) was added dropwise, and the resulting mixture was reacted at room temperature for 16 h. The reaction solution was poured into ice water (300 mL), and filtered. The filter cake was washed with water (100 mL), collected, and dried under reduced pressure to obtain crude 37b (8.20 g).

### Step 2: preparation of 37c

To a reaction flask were respectively added 37b (8.20 g, 33.58 mmol), 1,8-diazacyclo[5.4.0]undec-7-ene (1.02 g, 6.70 mmol), ethyl diazoacetate (4.84 g, 42.42 mmol) and ethanol (150 mL), and the mixture was reacted at room temperature for 3 h. Then rhodium(II) acetate dimer (74 mg, 0.167 mmol) was added, and the mixture was reacted at room temperature for 16 h. The reaction system was filtered. The filter cake was collected, and washed with ethanol (50 mL). The filter cake was collected, and dried under reduced pressure to obtain crude 37c (4.92 g).

### Step 3: preparation of 37d

To a reaction flask were respectively added the above-mentioned crude 37c (4.00 g), sodium hydroxide (1.94 g, 48.5 mmol) and water (100 mL), and the mixture was reacted at reflux for 16 h. The reaction system was cooled to room temperature, adjusted to pH 1 by adding 1 mol/L hydrochloric acid and filtered. The filter cake was collected, washed with water (50 mL), and dried under reduced pressure to obtain crude 37d (4.03 g).

LCMS m/z = 303.2 [M+1] ⁺.

### Step 4: preparation of 37e

To a reaction flask were respectively added the above-mentioned crude 37d (4.03 g), lithium chloride (1.70 g, 40.1 mmol), water (7 mL) and DMSO (70 mL), and the mixture was reacted at 130°C for 3 h. The reaction system was cooled to room temperature, poured into water (300 mL), and filtered. The filter cake was collected, and washed with water (100 mL). The filter cake was collected, and dried under reduced pressure to obtain crude 37e (3.50 g).

The trifluoroacetate of compound 37 was prepared by acidic preparative chromatography (water (containing 0.1% TFA)/acetonitrile) and lyophilization using compound 37e as the raw material and referring to the synthesis method of example 33.

¹H NMR (400 MHz, DMSO-*d*₆) δ 10.76 (s, 1H), 9.06 (s, 1H), 8.16 (s, 1H), 7.99 - 7.91 (m, 2H), 7.73 (dd, 1H), 7.51 (d, 1H), 7.17 (s, 1H), 7.12 - 7.04 (m, 2H), 7.01 - 6.90 (m, 2H), 5.13 - 5.02 (m, 2H), 4.60 (s, 2H), 4.57 - 4.42 (m, 2H), 3.78 - 3.65 (m, 3H), 3.63 - 3.51 (m, 2H), 3.40 - 3.25 (m, 2H), 3.17 - 2.98 (m, 4H), 2.82 - 2.56 (m, 6H), 2.50 - 2.40 (m, 1H), 2.20 - 1.95 (m, 3H), 1.90 - 1.78 (m, 2H), 1.78 - 1.73 (m, 3H), 1.45 - 1.27 (m, 2H). LCMS m/z = 780.3 [M+1] ⁺.

### Example 38: preparation of trifluoroacetate of compound 38

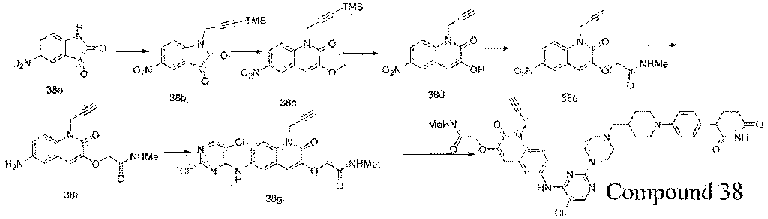

### Step 1: preparation of 38b

To a reaction flask were respectively added 38a (10.0 g, 52.05 mmol) and DMF (100 mL), and the mixture was cooled to 0°C. 60% sodium hydride (3.12 g) was added in batches, and the mixture was reacted at room temperature for 30 min. Then 3-bromo-1-trimethylsilyl-1-propyne (14.92 g, 78.06 mmol) was added dropwise, and the resulting mixture was reacted at room temperature for 16 h. The reaction solution was poured into ice water (300 mL), and filtered. The filter cake was washed with water (100 mL), collected, and dried under reduced pressure to obtain crude 38b (8.40 g).

### Step 2: preparation of 38c

To a reaction flask were respectively added the above-mentioned crude 38b (0.50 g) and ethanol (7 mL). Triethylamine (0.37 g, 3.66 mmol) and a solution of trimethylsilyldiazomethane in n-hexane (1.81 mL, 3.62 mmol, 2 mol/L) were added with stirring, and the mixture was reacted at room temperature for 3 h. To the reaction solution was added water (30 mL). The aqueous phase was extracted with ethyl acetate (30 mL×2). The organic phases were combined, washed with a saturated aqueous sodium chloride solution (50 mL), dried over anhydrous sodium sulphate, and concentrated under reduced pressure. The crude was separated and purified by silica gel column chromatography (petroleum ether : ethyl acetate (v/v) = 4:1) to obtain 38c (0.25 g, two-step yield calculated from compound 38a: 24%).

LCMS m/z = 331.1 [M+1] ⁺.

### Step 3: preparation of 38d

To a reaction flask were respectively added 38c (0.25 g, 0.76 mmol) and dichloromethane (2 mL). The mixture was cooled to 0°C, and a solution of boron tribromide in dichloromethane (2.28 mL, 1 mol/L) was added dropwise. The resulting mixture was reacted at room temperature for 2 h. To the reaction system was added a saturated aqueous sodium bicarbonate solution (10 mL). The aqueous phase was extracted with ethyl acetate (30 mL×2), and the organic phases were combined, washed with a saturated aqueous sodium chloride solution (50 mL), dried over anhydrous sodium sulphate, and concentrated under reduced pressure to obtain crude 38d (0.05 g).

The trifluoroacetate of compound 38 was prepared by acidic preparative chromatography (water (containing 0.1% TFA)/acetonitrile) and lyophilization using compound 38d as the raw material and referring to the synthesis method of example 33.

¹H NMR (400 MHz, DMSO-*d*₆) δ 10.76 (s, 1H), 9.07 (s, 1H), 8.16 (s, 1H), 8.02 - 7.90 (m, 2H), 7.73 (dd, 1H), 7.52 (d, 1H), 7.19 (s, 1H), 7.13 - 7.04 (m, 2H), 7.02 - 6.90 (m, 2H), 5.18 - 5.10 (m, 2H), 4.64 - 4.57 (m, 2H), 4.56 - 4.40 (m, 2H), 3.80 - 3.65 (m, 3H), 3.65 - 3.50 (m, 2H), 3.40 - 3.25 (m, 3H), 3.17 - 2.97 (m, 4H), 2.84 - 2.57 (m, 6H), 2.50 - 2.41 (m, 1H), 2.20 - 1.93 (m, 3H), 1.90 - 1.77 (m, 2H), 1.44 - 1.27 (m, 2H).

LCMS m/z = 383.8 [M/2+1] ⁺.

### Example 39: preparation of compound 39

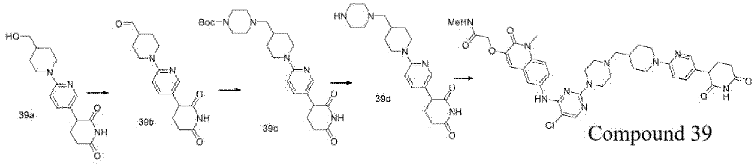

### Step 1: preparation of 39b

39a (0.25 g, 0.82 mmol) (see WO 2021113557 for the synthesis method) was dissolved in DMSO (3 mL). 2-iodoxybenzoic acid (0.37 g, 1.32 mmol) was added, and the mixture was reacted at 55°C for 2 h. The reaction system was cooled to room temperature. Water (100 mL) was added, and the mixture was extracted with ethyl acetate (100 mL). The organic phase was dried over anhydrous sodium sulphate, and concentrated under reduced pressure to obtain crude 39b (0.24 g).

LCMS m/z = 302.3 [M+1]⁺.

### Step 2: preparation of 39c

The above-mentioned crude 39b (0.24 g) was dissolved in 1,2-dichloroethane (30 mL). Tert-butyl piperazine-1-carboxylate (0.22 g, 1.18 mmol) and acetic acid (0.3 mL) were added. The mixture was reacted at room temperature for 1 h, and then sodium triacetoxyborohydride (0.56 g, 2.64 mmol) was added. The resulting mixture was reacted at room temperature for 16 h. To the reaction solution was slowly added a saturated aqueous sodium bicarbonate solution (50 mL), and the mixture was extracted with dichloromethane (30 ml). The organic phase was washed with a saturated aqueous sodium chloride solution (50 mL), dried over anhydrous sodium sulphate, and concentrated under reduced pressure. The crude was separated and purified by silica gel column chromatography (dichloromethane/methanol (v:v) = 10:1) to obtain 39c (0.20 g, yield: 36%).

LCMS m/z = 472.6 [M+1]⁺.

### Step 3: preparation of trifluoroacetate of 39d

39c (200 mg, 0.42 mmol) was dissolved in dichloromethane (4 mL). Trifluoroacetic acid (2 mL) was added, and the mixture was reacted at room temperature for 3 h. The reaction system was concentrated under reduced pressure to obtain crude trifluoroacetate of 39d (200 mg).

LCMS m/z =372.3 [M+1]⁺.

### Step 4: preparation of compound 39

To a reaction flask were respectively added the above-mentioned crude trifluoroacetate of 39d (200 mg), DIPEA (140 mg, 1.08 mmol) and DMSO (10 mL). **4B** (150 mg, 0.375 mmol) (see WO 2021077010 for the synthesis method) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 39 (30 mg, yield: 11%).

¹H NMR (400 MHz, DMSO-*d*₆) δ 10.77 (s, 1H), 8.84 (s, 1H), 8.05 (s, 1H), 7.98 - 7.86 (m, 3H), 7.74 (dd, 1H), 7.47 (d, 1H), 7.35 (dd, 1H), 7.12 (s, 1H), 6.77 (d, 1H), 4.58 (s, 2H), 4.34 - 4.17 (m, 2H), 3.79 - 3.55 (m, 8H), 2.85 - 2.60 (m, 6H), 2.57 - 2.45 (m, 1H), 2.45 - 2.30 (m, 4H), 2.25 - 2.10 (m, 3H), 2.04 - 1.92 (m, 1H), 1.88 - 1.70 (m, 3H), 1.17 - 0.98 (m, 2H).

LCMS m/z = 743.3 [M+1] ⁺.

### Example 40: preparation of trifluoroacetate of compound 40

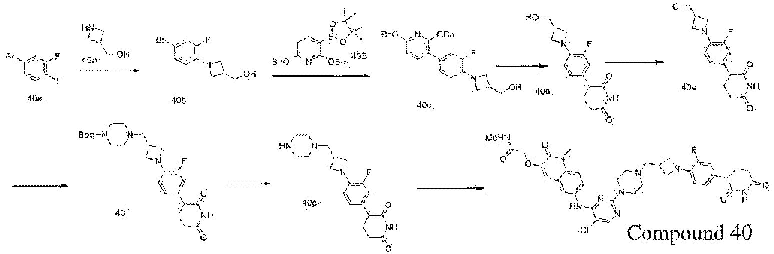

The trifluoroacetate of compound 40 was prepared by acidic preparative chromatography (water (containing 0.1% TFA)/acetonitrile) and lyophilization using compounds 40a and 40A as the raw materials and referring to the synthesis methods of examples 23 and 24.

¹H NMR (400 MHz, DMSO-*d*₆) δ 10.77 (s, 1H), 9.05 (s, 1H), 8.15 (s, 1H), 8.00 - 7.91 (m, 1H), 7.91 - 7.84 (m, 1H), 7.73 (dd, 1H), 7.48 (d, 1H), 7.19 (s, 1H), 7.02 - 6.84 (m, 2H), 6.51 (t, 1H), 4.63 - 4.42 (m, 4H), 4.15 - 4.02 (m, 2H), 3.84 - 3.60 (m, 6H), 3.55 - 3.42 (m, 4H), 3.33 - 2.95 (m, 5H), 2.72 - 2.56 (m, 4H), 2.53 - 2.42 (m, 1H), 2.24 - 2.06 (m, 1H), 2.04 - 1.89 (m, 1H).

LCMS m/z = 732.8 [M+1] ⁺.

### Example 41: preparation of trifluoroacetate of compound 41

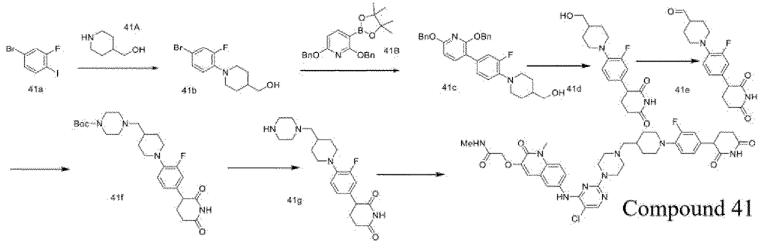

The trifluoroacetate of compound 41 was prepared by acidic preparative chromatography (water (containing 0.1% TFA)/acetonitrile) and lyophilization using compounds 41a and 41A as the raw materials and referring to the synthesis methods of examples 23 and 24.

¹H NMR (400 MHz, DMSO-*d*₆) δ 10.79 (s, 1H), 9.06 (s, 1H), 8.15 (s, 1H), 8.03 - 7.85 (m, 2H), 7.72 (dd, 1H), 7.48 (d, 1H), 7.18 (s, 1H), 7.07 - 6.92 (m, 3H), 4.59 (s, 2H), 4.56 - 4.40 (m, 2H), 3.85 - 3.76 (m, 1H), 3.69 (s, 3H), 3.65 - 3.52 (m, 2H), 3.45 - 3.25 (m, 4H), 3.20 - 2.95 (m, 4H), 2.77 - 2.60 (m, 6H), 2.55 - 2.44 (m, 1H), 2.27 - 2.10 (m, 1H), 2.10 - 1.94 (m, 2H), 1.94 - 1.80 (m, 2H), 1.51 - 1.33 (m, 2H).

LCMS m/z = 760.3 [M+1] ⁺.

### Example 42: preparation of trifluoroacetate of compound 42

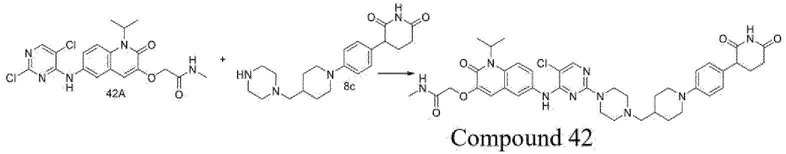

To a reaction flask were respectively added the above-mentioned crude trifluoroacetate of 8c (0.17 g), DIPEA (0.30 g, 2.32 mmol) and DMSO (5 mL). 42A (0.20 g, 0.46 mmol) (see WO 2022221673 for the synthesis method) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 42 (150 mg).

¹H NMR (400 MHz, DMSO-*d*₆) δ 10.78 (s, 1H), 9.05 (s, 1H), 8.15 (s, 1H), 8.00 - 7.86 (m, 2H), 7.75 - 7.60 (m, 2H), 7.20 - 6.93 (m, 5H), 5.60 - 5.00 (m, 1H), 4.68 - 4.38 (m, 4H), 3.82 - 3.51 (m, 5H), 3.43 - 3.25 (m, 2H), 3.20 - 2.97 (m, 4H), 2.93 - 2.73 (m, 2H), 2.72 - 2.58 (m, 4H), 2.51 - 2.42 (m, 1H), 2.24 - 1.95 (m, 3H), 1.94 - 1.80 (m, 2H), 1.58 (d, 6H), 1.48 - 1.31 (m, 2H).

LCMS m/z = 385.8 [M/2+1] ⁺.

### Example 43: preparation of trifluoroacetate of compound 43

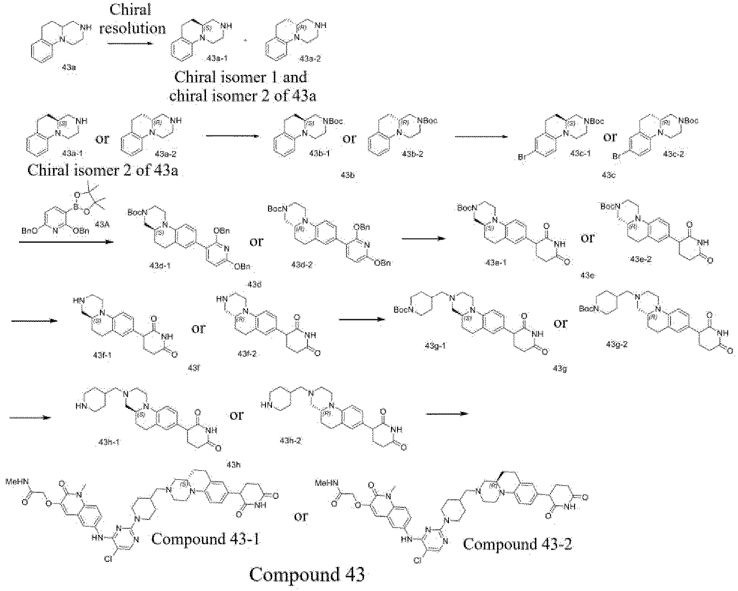

### Step 1: preparation of chiral isomer 1 and chiral isomer 2 of 43a

43a (see WO 2022007866 for the synthesis method) was subjected to chiral resolution. The chiral resolution method was as follows:
1. Instrument: SFC Prep 150 AP; chromatographic column: Daicel AD-H (19 mm × 250 mm).
2. The sample was dissolved in methanol and filtered with a 0.45 µm filter to prepare a sample liquid.
3. Preparative chromatography conditions: a. the mobile phase consists of systems A and B: mobile phase A: CO₂; mobile phase B: methanol; b. isocratic elution, mobile phase B content: 25%; c. flow rate: 40 mL/min.

Retention time: chiral isomer 1: 4.65 min, chiral isomer 2: 5.85 min.
Chiral isomer 1 and chiral isomer 2 of compound 43a are respectively one of the isomers of structure 43a-1 or 43a-2.

### Step 2: preparation of 43b

Chiral isomer 2 of 43a (0.55 g, 2.92 mmol) was dissolved in dichloromethane (30 mL). Boc₂O (1.27 g, 5.82 mmol) and DMAP (0.71 g, 5.81 mmol) were added. The mixture was reacted at room temperature for 16 h. The reaction system was washed with 0.5 mol/L hydrochloric acid (50 mL). The organic phase was dried over anhydrous sodium sulphate, and concentrated under reduced pressure. The crude was separated and purified by silica gel column chromatography (ethyl acetate : petroleum ether (v/v) = 0:1-1:9) to obtain 43b (0.78 g, yield: 93%).

LCMS m/z = 289.3 [M+1]⁺.

Compound 43b is one of the isomers of structure 43b-1 or 43b-2.

### Step 3: preparation of 43c

43b (0.78 g, 2.71 mmol) was dissolved in acetonitrile (20 mL), and NBS (0.53 g, 2.98 mmol) was added. The mixture was reacted at room temperature for 1 h. The reaction system was concentrated under reduced pressure, and the crude was separated and purified by silica gel column chromatography (ethyl acetate : petroleum ether (v/v) = 0:1-1:9) to obtain 43c (0.85 g, yield: 86%).

LCMS m/z = 367.3 [M+1]⁺.

Compound 43c is one of the isomers of structure 43c-1 or 43c-2.

### Step 4: preparation of 43d

43c (1.50 g, 4.10 mmol), 43A (2.89 g, 6.93 mmol) (see WO 2022235945 for the synthesis method), Pd(dppf)Cl₂·DCM (0.19 g, 0.23 mmol) and caesium carbonate (1.51 g, 4.63 mmol) were added to a reaction flask. 1,4-dioxane (30 mL) and water (3 mL) were added, and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate and concentrated under reduced pressure. The crude was separated and purified by silica gel column chromatography (petroleum ether/ethyl acetate (v/v) = 1:0-9:1) to obtain 43d (0.55 g, yield: 23%).

Compound 43d is one of the isomers of structure 43d-1 or 43d-2.

### Step 5: preparation of 43e

43d (0.55 g, 0.95 mmol) was dissolved in THF (20 mL), and 10% palladium on carbon (0.51 g) was added. The mixture was reacted at 45°C under the atmosphere of hydrogen balloon for 20 h. The reaction system was cooled to room temperature, and filtered by suction. The filtrate was concentrated under reduced pressure, and the crude was separated and purified by silica gel column chromatography (MeOH/DCM (v/v) = 0:1-5:95) to obtain 43e (0.22 g, yield: 58%).

LCMS m/z = 400.2 [M+1]⁺.

Compound 43e is one of the isomers of structure 43e-1 or 43e-2.

### Step 6: preparation of 43f

43e (110 mg, 0.276 mmol) was dissolved in dichloromethane (2 mL). Trifluoroacetic acid (1 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 43f (84 mg).

LCMS m/z = 300.3 [M+1]⁺.

Compound 43f is one of the isomers of structure 43f-1 or 43f-2.

### Step 7: preparation of 43g

The above-mentioned crude 43f (0.084 g) was dissolved in 1,2-dichloroethane (30 mL). Tert-butyl 4-formylpiperidine-1-carboxylate (0.090 g, 0.42 mmol) was added, and acetic acid (3 mL) was added. The mixture was stirred at room temperature for 1 h, and then sodium triacetoxyborohydride (0.12 g, 0.57 mmol) was added. The resulting mixture was reacted at room temperature for 16 h. To the reaction solution was slowly added a saturated aqueous sodium bicarbonate solution (50 mL), and the mixture was extracted with dichloromethane (50 mL). The organic phase was washed with a saturated aqueous sodium chloride solution (100 mL), dried over anhydrous sodium sulphate, and concentrated under reduced pressure. The crude was separated and purified by silica gel column chromatography (dichloromethane/methanol (v/v) = 10:1) to obtain 43g (0.13 g, yield: 62%).

LCMS m/z = 497.3 [M+1]⁺.

Compound 43g is one of the isomers of structure 43g-1 or 43g-2.

### Step 8: preparation of trifluoroacetate of 43h

43g (130 mg, 0.26 mmol) was dissolved in dichloromethane (2 mL). Trifluoroacetic acid (1 mL) was added, and the mixture was reacted at room temperature for 3 h. The reaction system was concentrated under reduced pressure to obtain crude trifluoroacetate of 43h (130 mg).

LCMS m/z = 397.3 [M+1]⁺.

Compound 43h is one of the isomers of structure 43h-1 or 43h-2.

### Step 9: preparation of trifluoroacetate of compound 43

To a reaction flask were respectively added the above-mentioned crude trifluoroacetate of 43h (120 mg), DIPEA (300 mg, 2.32 mmol) and DMSO (10 mL). **4B** (100 mg, 0.25 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 70% (elution time: 12 min). Lyophilization was performed to obtain the trifluoroacetate of compound 43 (20 mg).

¹H NMR (400 MHz, DMSO-*d*₆) δ 10.76 (s, 1H), 8.95 (s, 1H), 8.08 (s, 1H), 8.03 - 7.89 (m, 2H), 7.80 - 7.70 (m, 1H), 7.50 - 7.42 (m, 1H), 7.14 - 7.07 (m, 1H), 6.95 - 6.80 (m, 3H), 4.63 - 4.55 (m, 2H), 4.55 - 4.42 (m, 2H), 4.13 - 4.02 (m, 1H), 3.74 - 3.54 (m, 6H), 3.35 - 3.20 (m, 1H), 3.17 - 2.73 (m, 8H), 2.72 - 2.57 (m, 5H), 2.49 - 2.40 (m, 1H), 2.25 - 2.05 (m, 2H), 2.05 - 1.91 (m, 2H), 1.87 - 1.70 (m, 2H), 1.70 - 1.54 (m, 1H), 1.30 - 1.07 (m, 2H).

Compound 43 is one of the isomers of compound 43-1 or 43-2.

### Example 44: preparation of trifluoroacetate of compound 44

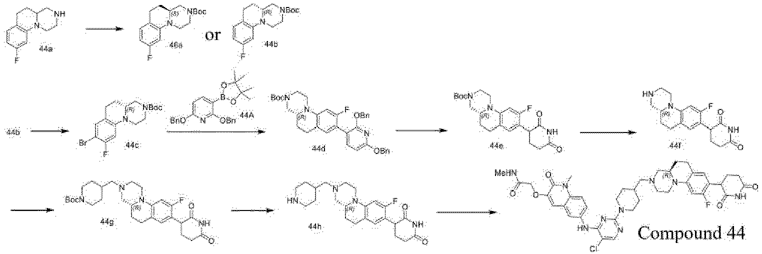

### Step 1: preparation of 44b

44a (2.8 g, 13.58 mmol) (see Bioorganic & Medicinal Chemistry Letters, 2016, 26, 5877-5882 for the synthesis method) was dissolved in dichloromethane (50 mL). Boc₂O (5.93 g, 27.17 mmol) and DMAP (3.32 g, 27.18 mmol) were added. The mixture was reacted at room temperature for 16 h. The reaction system was washed with 0.5 mol/L hydrochloric acid (50 mL), dried over anhydrous sodium sulphate, and concentrated under reduced pressure. The crude was separated and purified by silica gel column chromatography (ethyl acetate : petroleum ether (v/v) = 0:1-1:9) to obtain the racemate of 44b (3.4 g, yield: 82%).

The racemate of 44b was subjected to chiral resolution, and the chiral resolution method was as follows:
1. Instrument: SFC Prep 150 AP; chromatographic column: Daicel IC-H (19 mm × 250 mm).
2. The sample was dissolved in methanol and filtered with a 0.45 µm filter to prepare a sample liquid.
3. Preparative chromatography conditions: a. the mobile phase consists of systems A and B: mobile phase A: CO₂; mobile phase B: methanol/isopropanol (v/v) = 1:1; b. isocratic elution, mobile phase B content: 20%; c. flow rate: 40 mL/min.

Retention time: chiral isomer 1 (compound 44b): 5.7 min, chiral isomer 2 (compound 46a): 6.47 min.

According to the MicroED structure determination of compound 46b, compound 44b is in the R configuration, and compound 46a is in the S configuration.

LCMS m/z = 307.3 [M+1]⁺.

### Step 2: preparation of 44c

44b (1.8 g, 5.88 mmol) was dissolved in acetonitrile (50 mL), and NBS (1.05 g, 5.90 mmol) was added. The mixture was reacted at room temperature for 1 h. The reaction system was concentrated under reduced pressure, and the crude was separated and purified by silica gel column chromatography (ethyl acetate : petroleum ether (v/v) = 0:1-1:9) to obtain 44c (1.6 g, yield: 71%).

LCMS m/z = 385.3 [M+1]⁺.

### Step 3: preparation of 44d

44c (0.77 g, 2.0 mmol), 44A (1.67 g, 4.0 mmol) (see WO 2022235945 for the synthesis method), Pd(dppf)Cl₂·DCM (0.16 g, 0.20 mmol) and caesium carbonate (1.30 g, 4.0 mmol) were added to a reaction flask. 1,4-dioxane (30 mL) and water (3 mL) were added, and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate and concentrated under reduced pressure. The crude was separated and purified by silica gel column chromatography (petroleum ether/ethyl acetate (v/v) = 1:0-9:1) to obtain 44d (0.70 g, yield: 59%).

### Step 4: preparation of 44e

44d (0.70 g, 1.18 mmol) was dissolved in THF (20 mL), and 10% palladium on carbon (0.63 g) was added. The mixture was reacted at 45°C under the atmosphere of hydrogen balloon for 20 h. The reaction system was cooled to room temperature, and filtered by suction. The filtrate was concentrated under reduced pressure, and the crude was separated and purified by silica gel column chromatography (MeOH:DCM (v/v) = 0:1-5:95) to obtain 44e (0.25 g, yield: 51%).

LCMS m/z = 418.1 [M+1]⁺.

### Step 5: preparation of 44f

44e (250 mg, 0.6 mmol) was dissolved in dichloromethane (2 mL). Trifluoroacetic acid (2 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 44f (190 mg).

LCMS m/z = 318.3 [M+1]⁺.

### Step 6: preparation of 44g

The above-mentioned crude 44f (0.19 g) was dissolved in 1,4-dichloroethane (30 mL). Tert-butyl 4-formylpiperidine-1-carboxylate (0.20 g, 0.93 mmol) was added, and acetic acid (3 mL) was added. The mixture was stirred at room temperature for 1 h, and then sodium triacetoxyborohydride (0.40 g, 1.89 mmol) was added. The resulting mixture was reacted at room temperature for 16 h. To the reaction system was slowly added a saturated aqueous sodium bicarbonate solution (10 mL), and the mixture was extracted with dichloromethane (50 mL). The organic phase was washed with a saturated aqueous sodium chloride solution (100 mL), dried over anhydrous sodium sulphate, and concentrated under reduced pressure. The crude was separated and purified by silica gel column chromatography (dichloromethane:methanol (v/v) = 10:1) to obtain 44g (0.30 g, yield: 63%).

LCMS m/z = 515.3 [M+1]⁺.

### Step 7: preparation of 44h

44g (300 mg, 0.58 mmol) was dissolved in dichloromethane (2 mL). Trifluoroacetic acid (2 mL) was added, and the mixture was reacted at room temperature for 3 h. The reaction system was concentrated under reduced pressure, adjusted to pH 9 with a saturated aqueous sodium bicarbonate solution, and extracted with dichloromethane (100 mL×2). The organic phase was dried over anhydrous sodium sulphate, and concentrated under reduced pressure to obtain crude 44h (180 mg).

LCMS m/z = 415.3 [M+1]⁺.

### Step 8: preparation of trifluoroacetate of compound 44

To a reaction flask were respectively added the above-mentioned crude 44h (91 mg), DIPEA (85 mg, 0.66 mmol) and DMSO (10 mL). **4B** (90 mg, 0.23 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 70% (elution time: 12 min). Lyophilization was performed to obtain the trifluoroacetate of compound 44 (80 mg).

¹H NMR (400 MHz, DMSO-*d*₆) δ 10.79 (s, 1H), 8.98 (s, 1H), 8.09 (s, 1H), 8.02 - 7.89 (m, 2H), 7.74 (dd, 1H), 7.50 - 7.43 (m, 1H), 7.12 (s, 1H), 6.87 (d, 1H), 6.79 (d, 1H), 4.59 (s, 2H), 4.54 - 4.40 (m, 2H), 4.14 - 4.00 (m, 1H), 3.93 - 3.80 (m, 1H), 3.73 - 3.55 (m, 5H), 3.40 - 3.27 (m, 1H), 3.20 - 2.80 (m, 7H), 2.80 - 2.58 (m, 6H), 2.58 - 2.50 (m, 1H), 2.27 - 2.05 (m, 2H), 2.05 - 1.87 (m, 2H), 1.87 - 1.70 (m, 2H), 1.70 - 1.53 (m, 1H), 1.28 - 1.06 (m, 2H).

LCMS m/z = 786.3 [M+1] ⁺.

### Example 45: preparation of trifluoroacetate of compound 45

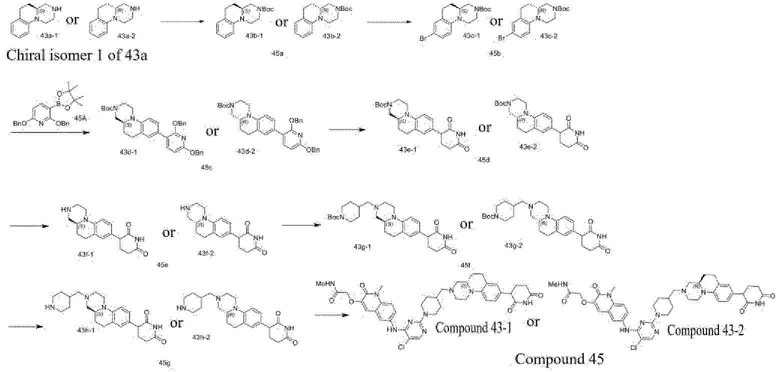

### Step 1: preparation of 45a

Chiral isomer 1 of 43a (0.6 g, 3.19 mmol) was dissolved in dichloromethane (30 mL). Boc₂O (1.39 g, 6.4 mmol) and DMAP (0.39 g, 3.2 mmol) were added. The mixture was reacted at room temperature for 16 h. The reaction system was washed with 0.5 mol/L hydrochloric acid (50 mL). The organic phase was dried over anhydrous sodium sulphate, and concentrated under reduced pressure. The crude was separated and purified by silica gel column chromatography (ethyl acetate:petroleum ether (v/v) = 0:1-1:9) to obtain 45a (0.8 g, yield: 87%).

Compound 45a is one of the isomers of structure 43b-1 or 43b-2.

### Step 2: preparation of 45b

45a (0.78 g, 2.70 mmol) was dissolved in acetonitrile (20 mL), and NBS (0.53 g, 2.98 mmol) was added. The mixture was reacted at room temperature for 1 h. The reaction system was concentrated under reduced pressure, and the crude was separated and purified by silica gel column chromatography (ethyl acetate : petroleum ether (v/v) = 0:1-1:9) to obtain 45b (0.86 g, yield: 87%).

LCMS m/z = 367.5 [M+1]⁺.

Compound 45b is one of the isomers of structure 43c-1 or 43c-2.

### Step 3: preparation of 45c

45b (0.85 g, 2.32 mmol), 45A (2.89 g, 6.93 mmol), Pd(dppf)Cl₂·DCM (0.19 g, 0.23 mmol) and caesium carbonate (1.51 g, 4.63 mmol) were added to a reaction flask. 1,4-dioxane (30 mL) and water (3 mL) were added, and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate and concentrated under reduced pressure. The crude was separated and purified by silica gel column chromatography (petroleum ether/ethyl acetate (v/v) = 1:0-9:1) to obtain 45c (0.9 g, yield: 67%).

Compound 45c is one of the isomers of structure 43d-1 or 43d-2.

### Step 4: preparation of 45d

45c (0.7 g, 1.21 mmol) was dissolved in THF (20 mL), and 10% palladium on carbon (0.51 g) was added. The mixture was reacted at 45°C under the atmosphere of hydrogen balloon for 20 h. The reaction system was cooled to room temperature, and filtered by suction. The filtrate was concentrated under reduced pressure, and the crude was separated and purified by silica gel column chromatography (MeOH:DCM (v/v) = 0:1-5:95) to obtain 45d (0.4 g, yield: 83%).

Compound 45d is one of the isomers of structure 43e-1 or 43e-2.

### Step 5: preparation of 45e

45d (120 mg, 0.3 mmol) was dissolved in dichloromethane (2 mL). Trifluoroacetic acid (1 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 45e (90 mg).

LCMS m/z = 300.3 [M+1]⁺.

Compound 45e is one of the isomers of structure 43f-1 or 43f-2.

### Step 6: preparation of 45f

The above-mentioned crude 45e (0.09 g) was dissolved in 1,2-dichloroethane (20 mL). Tert-butyl 4-formylpiperidine-1-carboxylate (0.090 g, 0.42 mmol) was added, and acetic acid (1.5 mL) was added. The mixture was stirred at room temperature for 1 h, and then sodium triacetoxyborohydride (0.12 g, 0.57 mmol) was added. The resulting mixture was reacted at room temperature for 16 h. To the reaction solution was slowly added a saturated aqueous sodium bicarbonate solution (50 mL), and the mixture was extracted with dichloromethane (50 mL). The organic phase was washed with a saturated aqueous sodium chloride solution (100 mL), dried over anhydrous sodium sulphate, and concentrated under reduced pressure. The crude was separated and purified by silica gel column chromatography (dichloromethane/methanol (v/v) = 10:1) to obtain 45f (0.13 g, yield: 62%).

LCMS m/z = 497.3 [M+1]⁺.

Compound 45f is one of the isomers of structure 43g-1 or 43g-2.

### Step 7: preparation of trifluoroacetate of 45g

45f (130 mg, 0.26 mmol) was dissolved in dichloromethane (2 mL). Trifluoroacetic acid (1 mL) was added, and the mixture was reacted at room temperature for 3 h. The reaction system was concentrated under reduced pressure to obtain crude trifluoroacetate of 45g (100 mg).

LCMS m/z =397.3 [M+1]⁺.

Compound 45g is one of the isomers of structure 43h-1 or 43h-2.

### Step 8: preparation of trifluoroacetate of compound 45

To a reaction flask were respectively added the above-mentioned crude trifluoroacetate of 45g (100 mg), DIPEA (78 mg, 0.60 mmol) and DMSO (10 mL). **4B** (80 mg, 0.20 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 70% (elution time: 12 min). Lyophilization was performed to obtain the trifluoroacetate of compound 45 (20 mg).

¹H NMR (400 MHz, DMSO-*d*₆) δ 10.75 (s, 1H), 9.01 (s, 1H), 8.09 (s, 1H), 8.03 - 7.89 (m, 2H), 7.80 - 7.70 (m, 1H), 7.50 - 7.42 (m, 1H), 7.14 - 7.07 (m, 1H), 6.95 - 6.80 (m, 3H), 4.63 - 4.55 (m, 2H), 4.55 - 4.42 (m, 2H), 4.13 - 4.02 (m, 1H), 3.74 - 3.54 (m, 6H), 3.35 - 3.20 (m, 1H), 3.17 - 2.73 (m, 8H), 2.72 - 2.57 (m, 5H), 2.49 - 2.40 (m, 1H), 2.25 - 2.05 (m, 2H), 2.05 - 1.91 (m, 2H), 1.87 - 1.70 (m, 2H), 1.70 - 1.54 (m, 1H), 1.30 - 1.07 (m, 2H).

Compound 45 is one of the isomers of compound 43-1 or 43-2.

### Example 46: preparation of trifluoroacetate of compound 46

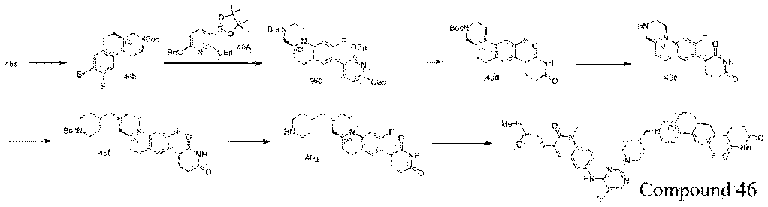

### Step 1: preparation of 46b

46a (1.4 g, 4.57 mmol) was dissolved in acetonitrile (50 mL), and NBS (0.81 g, 4.55 mmol) was added. The mixture was reacted at room temperature for 1 h. The reaction system was concentrated under reduced pressure, and the crude was separated and purified by silica gel column chromatography (ethyl acetate : petroleum ether (v/v) = 0:1-1:9) to obtain 46b (1.6 g, yield: 92%).

LCMS m/z = 385.3 [M+1]⁺.

According to the MicroED structure determination, compound 46b is in the S configuration.

### Step 2: preparation of 46c

46b (1.6 g, 4.16 mmol), 46A (3.46 g, 8.29 mmol), Pd(dppf)Cl₂·DCM (0.34 g, 0.42 mmol) and caesium carbonate (2.70 g, 8.3 mmol) were added to a reaction flask. 1,4-dioxane (50 mL) and water (5 mL) were added, and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate and concentrated under reduced pressure. The crude was separated and purified by silica gel column chromatography (petroleum ether/ethyl acetate (v/v) = 1:0-9:1) to obtain 46c (1.4 g, yield: 56%).

### Step 3: preparation of 46d

46c (1.4 g, 2.35 mmol) was dissolved in THF (50 mL), and 10% palladium on carbon (1.25 g) was added. The mixture was reacted at 45°C under the atmosphere of hydrogen balloon for 20 h. The reaction system was cooled to room temperature, and filtered by suction. The filtrate was concentrated under reduced pressure, and the crude was separated and purified by silica gel column chromatography (MeOH:DCM (v/v) = 0:1-5:95) to obtain 46d (0.85 g, yield: 87%).

LCMS m/z = 418.1 [M+1]⁺.

### Step 4: preparation of 46e

46d (620 mg, 1.49 mmol) was dissolved in dichloromethane (5 mL). Trifluoroacetic acid (3 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 46e (460 mg).

LCMS m/z = 318.3 [M+1]⁺.

### Step 5: preparation of 46f

The above-mentioned crude 46e (0.46 g) was dissolved in 1,2-dichloroethane (30 mL). Tert-butyl 4-formylpiperidine-1-carboxylate (0.46 g, 2.14 mmol) was added, and acetic acid (3 mL) was added. The mixture was stirred at room temperature for 1 h, and then sodium triacetoxyborohydride (0.92 g, 4.34 mmol) was added. The resulting mixture was reacted at room temperature for 16 h. To the reaction solution was slowly added a saturated aqueous sodium bicarbonate solution (20 mL), and the mixture was extracted with dichloromethane (50 mL). The organic phase was washed with a saturated aqueous sodium chloride solution (100 mL), dried over anhydrous sodium sulphate, and concentrated under reduced pressure. The crude was separated and purified by silica gel column chromatography (dichloromethane/methanol (v/v) = 10:1) to obtain 46f (0.70 g, yield: 64%).

LCMS m/z = 515.3 [M+1]⁺.

### Step 6: preparation of 46g

46f (700 mg, 1.36 mmol) was dissolved in dichloromethane (4 mL). Trifluoroacetic acid (2 mL) was added, and the mixture was reacted at room temperature for 3 h. The reaction system was concentrated under reduced pressure, adjusted to pH 9 with a saturated aqueous sodium bicarbonate solution, and extracted with dichloromethane (100 mL×2). The organic phase was dried over anhydrous sodium sulphate, and concentrated under reduced pressure to obtain crude 46g (520 mg).

LCMS m/z = 415.3 [M+1]⁺.

### Step 7: preparation of trifluoroacetate of compound 46

To a reaction flask were respectively added the above-mentioned crude 46g (90 mg), DIPEA (85 mg, 0.66 mmol) and DMSO (10 mL). 4B (90 mg, 0.23 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 46 (70 mg).

¹H NMR (400 MHz, DMSO-*d*₆) δ 10.79 (s, 1H), 8.98 (s, 1H), 8.09 (s, 1H), 8.02 - 7.90 (m, 2H), 7.74 (dd, 1H), 7.51 - 7.43 (m, 1H), 7.12 (s, 1H), 6.87 (d, 1H), 6.79 (d, 1H), 4.59 (s, 2H), 4.54 - 4.41 (m, 2H), 4.13 - 4.00 (m, 1H), 3.92 - 3.80 (m, 1H), 3.73 - 3.54 (m, 5H), 3.40 - 3.27 (m, 1H), 3.20 - 2.80 (m, 7H), 2.80 - 2.58 (m, 6H), 2.58 - 2.50 (m, 1H), 2.27 - 2.05 (m, 2H), 2.05 - 1.87 (m, 2H), 1.87 - 1.70 (m, 2H), 1.70 - 1.53 (m, 1H), 1.30 - 1.06 (m, 2H).

LCMS m/z = 786.3 [M+1] ⁺.

### Example 47: preparation of compound 47

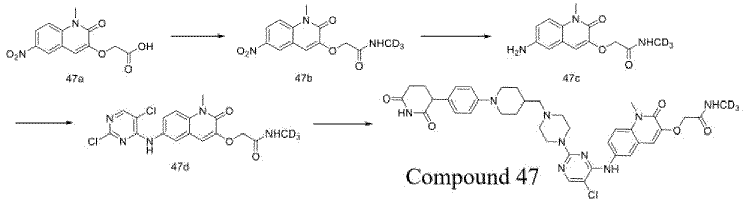

### Step 1: preparation of 47b

To a reaction flask were respectively added 47a (2.00 g, 7.19 mmol) (see CN 112538083 for the synthesis method), HATU (4.10 g, 10.78 mmol) and DMF (30 mL), and the mixture was stirred at room temperature for 2 min. Then deuterated methanamine hydrochloride (1.52 g, 21.55 mmol) and DIPEA (2.79 g, 21.59 mmol) were added, and the resulting mixture was reacted at room temperature for 16 h. The reaction system was poured into water (200 mL) and filtered. The filter cake was dried under reduced pressure to obtain crude 47b (1.03 g).

### Step 2: preparation of 47c

To a reaction flask were respectively added the above-mentioned crude 47b (1.03 g), 10% palladium on carbon (0.6 g), dichloromethane (30 mL) and methanol (30 mL), and the mixture was reacted at room temperature under the atmosphere of hydrogen balloon for 16 h. The reaction system was filtered over diatomaceous earth, and the filtrate was concentrated under reduced pressure to obtain crude 47c (0.62 g).

LCMS m/z = 265.2 [M+1] ⁺.

### Step 3: preparation of 47d

To a reaction flask were respectively added the above-mentioned crude 47c (0.62 g), 2,4,5-trichloropyrimidine (0.47 g, 2.56 mmol), triethylamine (0.48 g, 4.74 mmol) and DMF (5 mL), and the mixture was reacted at 70°C for 2 h. The reaction system was cooled to room temperature, poured into water (50 mL), and filtered. The filter cake was washed with water (20 mL), and dried under reduced pressure to obtain crude 47d (0.70 g).

### Step 4: preparation of compound 47

To a reaction flask were respectively added the crude trifluoroacetate of 8c (90 mg), sodium bicarbonate (80 mg, 0.95 mmol) and DMSO (5 mL). The mixture was stirred at room temperature for 0.5 h. The above-mentioned crude 47d (78 mg) and DIPEA (0.12 g, 0.93 mmol) were added, and the resulting mixture was reacted at 100°C for 7 h. The reaction system was cooled to room temperature, and subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 15 min). Lyophilization was performed to obtain compound 47 (20 mg, four-step yield from compound 47a: 3%).

¹H NMR (400 MHz, DMSO-*d*₆) δ 10.75 (s, 1H), 8.85 (s, 1H), 8.05 (s, 1H), 7.97 - 7.87 (m, 2H), 7.74 (dd, 1H), 7.47 (d, 1H), 7.12 (s, 1H), 7.06 - 6.99 (m, 2H), 6.92 - 6.84 (m, 2H), 4.58 (s, 2H), 3.76 - 3.57 (m, 10H), 2.70 - 2.56 (m, 3H), 2.49 - 2.29 (m, 5H), 2.23 - 1.97 (m, 4H), 1.85 - 1.63 (m, 3H), 1.29 - 1.11 (m, 2H).

LCMS m/z = 745.3 [M+1] ⁺.

### Example 48: preparation of compound 48

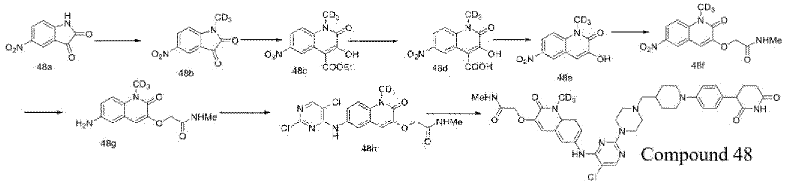

### Step 1: preparation of 48b

To a reaction flask were added 48a (20.0 g, 104.10 mmol) and DMF (200 mL). 60% sodium hydride (4.16 g) was added in batches at 0°C, and the mixture was reacted at room temperature for 30 min. Then deuterated iodomethane (15.09 g, 104.10 mmol) was added dropwise, and the resulting mixture was reacted at room temperature for 16 h. The reaction solution was poured into ice water (1.0 L), and filtered. The filter cake was washed with water (300 mL), and dried under reduced pressure to obtain crude 48b (10.2 g).

### Step 2: preparation of 48c

To a reaction flask were respectively added the above-mentioned crude 48b (12.5 g), DBU (1.82 g, 11.95 mmol), ethyl diazoacetate (8.61 g, 75.46 mmol) and ethanol (200 mL), and the mixture was reacted at room temperature for 3 h. Rhodium(II) acetate dimer (0.13 g, 0.29 mmol) was added, and the mixture was reacted at room temperature for 16 h. The reaction system was filtered. The filter cake was washed with ethanol (50 mL) and dried under reduced pressure to obtain crude 48c (14.5 g).

### Step 3: preparation of 48d

To a reaction flask were respectively added the above-mentioned crude 48c (14.5 g), sodium hydroxide (9.82 g, 245.5 mmol) and water (200 mL), and the mixture was reacted at reflux for 16 h. The reaction system was cooled to room temperature, adjusted to pH 1 with 1 mol/L hydrochloric acid, and filtered. The filter cake was washed with water (50 mL), and dried under reduced pressure to obtain crude 48d (11.5 g).

LCMS m/z = 266.2 [M-1] ⁻.

### Step 4: preparation of 48e

To a reaction flask were respectively added the above-mentioned crude 48d (11.5 g), LiCl (5.47 g, 129.04 mmol), water (10 mL) and DMSO (200 mL), and the mixture was reacted at 130°C for 3 h. The reaction system was cooled to room temperature, poured into water (500 mL), and filtered. The filter cake was washed with water (100 mL), and dried under reduced pressure to obtain crude 48e (7.4 g).

### Step 5: preparation of 48f

To a reaction flask were respectively added the above-mentioned crude 48e (1.0 g), 2-bromo-N-methylacetamide (0.82 g, 5.40 mmol) and caesium carbonate (2.92 g, 8.96 mmol). DMF (15 mL) was added, and the mixture was reacted at room temperature for 2 h. The reaction solution was poured into water (100 mL), and filtered. The filter cake was successively washed with water (50 mL) and ethanol (5 mL), and dried under reduced pressure to obtain crude 48f (1.20 g).

### Step 6: preparation of 48g

To a reaction flask were respectively added the crude 48f (1.20 g), 10% palladium on carbon (0.6 g), dichloromethane (30 mL) and methanol (30 mL). The mixture was reacted at room temperature under the atmosphere of hydrogen balloon for 16 h. The reaction system was filtered over diatomaceous earth, and the filtrate was concentrated under reduced pressure to obtain crude 48g (0.56 g).

LCMS m/z = 265.2 [M+1] ⁺.

### Step 7: preparation of 48h

To a reaction flask were respectively added the above-mentioned crude 48g (0.56 g), 2,4,5-trichloropyrimidine (0.43 g, 2.34 mmol), triethylamine (0.43 g, 4.25 mmol) and DMF (5 mL), and the mixture was reacted at 70°C for 2 h. The reaction solution was cooled to room temperature, poured into water (50 mL), and filtered. The filter cake was washed with water (20 mL), and dried under reduced pressure to obtain crude 48h (0.71 g).

LCMS m/z = 411.1 [M+1]⁺.

### Step 8: preparation of compound 48

To a reaction flask were respectively added the above-mentioned crude trifluoroacetate of 8c (90 mg), sodium bicarbonate (80 mg, 0.95 mmol) and DMSO (5 mL). The mixture was stirred at room temperature for 0.5 h. The above-mentioned crude 48h (78 mg) and DIPEA (0.12 g, 0.93 mmol) were added, and the resulting mixture was reacted at 100°C for 7 h. The reaction system was cooled to room temperature, and subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 15 min). Lyophilization was performed to obtain compound 48 (8 mg, eight-step yield from compound 48a: 0.6%).

¹H NMR (400 MHz, DMSO-*d*₆) δ 10.75 (s, 1H), 8.84 (s, 1H), 8.05 (s, 1H), 7.97 - 7.87 (m, 2H), 7.74 (dd, 1H), 7.47 (d, 1H), 7.12 (s, 1H), 7.06 - 6.99 (m, 2H), 6.92 - 6.84 (m, 2H), 4.58 (s, 2H), 3.76 - 3.57 (m, 7H), 2.70 - 2.56 (m, 6H), 2.49 - 2.29 (m, 5H), 2.23 - 1.96 (m, 4H), 1.85 - 1.63 (m, 3H), 1.29 - 1.11 (m, 2H).

LCMS m/z = 373.2 [M/2+1] ⁺.

### Example 49: preparation of compound 49

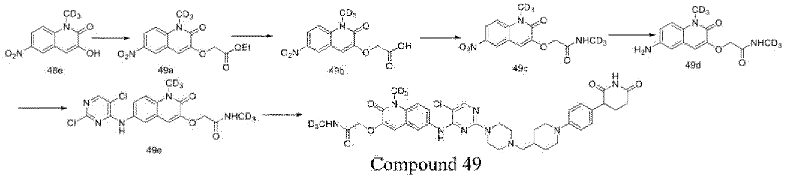

### Step 1: preparation of 49a

To a reaction flask were respectively added the above-mentioned crude 48e (3.0 g), ethyl bromoacetate (2.69 g, 16.11 mmol), caesium carbonate (8.76 g, 26.89 mmol) and DMF (50 mL), and the mixture was reacted at room temperature for 3 h. The reaction system was poured into water (200 mL) and filtered. The filter cake was dried under reduced pressure to obtain crude 49a (4.00 g).

### Step 2: preparation of 49b

To a reaction flask were respectively added the above-mentioned crude 49a (4.00 g), lithium hydroxide (1.56 g, 65.14 mmol), methanol (72 mL) and water (18 mL), and the mixture was reacted at room temperature for 2 h. The reaction solution was poured into water (200 mL), adjusted to pH 1 with 1 mol/L hydrochloric acid, and filtered. The filter cake was dried under reduced pressure to obtain crude 49b (2.35 g).

### Step 3: preparation of 49c

To a reaction flask were respectively added the above-mentioned crude 49b (1.50 g), HATU (3.04 g, 8.00 mmol) and DMF (30 mL), and the mixture was stirred at room temperature for 2 min. Then deuterated methanamine hydrochloride (1.13 g, 16.02 mmol) and DIPEA (2.07 g, 16.02 mmol) were added, and the resulting mixture was reacted at room temperature for 16 h. The reaction system was poured into water (200 mL) and filtered. The filter cake was dried under reduced pressure to obtain crude 49c (1.06 g).

### Step 4: preparation of 49d

To a reaction flask were respectively added the above-mentioned crude 49c (1.20 g), 10% palladium on carbon (0.5 g), dichloromethane (30 mL) and methanol (30 mL), and the mixture was reacted at room temperature under the atmosphere of hydrogen balloon for 16 h. The reaction system was filtered over diatomaceous earth, and the filtrate was concentrated under reduced pressure to obtain crude 49d (0.60 g).

LCMS m/z = 268.2 [M+1] ⁺.

### Step 5: preparation of 49e

To a reaction flask were respectively added the above-mentioned crude 49d (0.60 g), 2,4,5-trichloropyrimidine (0.45 g, 2.45 mmol), triethylamine (0.45 g, 4.45 mmol) and DMF (5 mL), and the mixture was reacted at 70°C for 2 h. The reaction solution was cooled to room temperature, poured into water (50 mL), and filtered. The filter cake was washed with water (20 mL), and dried under reduced pressure to obtain crude 49e (0.70 g).

### Step 6: preparation of compound 49

To a reaction flask were respectively added the above-mentioned crude trifluoroacetate of 8c (90 mg), sodium bicarbonate (80 mg, 0.95 mmol) and DMSO (5 mL). The mixture was stirred at room temperature for 0.5 h. The above-mentioned crude 49e (79 mg) and DIPEA (0.12 g, 0.93 mmol) were added, and the resulting mixture was reacted at 100°C for 7 h. The reaction system was cooled to room temperature, and subjected to Pre-HPLC (instrument and preparative column: using Waters 2767 preparative liquid phase chromatographic instrument, preparative column model: SunFire@Prep C18, 5 µm,inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 15 min). Lyophilization was performed to obtain compound 49 (100 mg, two-step yield from compound 8b: 70%).

¹H NMR (400 MHz, DMSO-*d*₆) δ 10.75 (s, 1H), 8.84 (s, 1H), 8.05 (s, 1H), 7.97 - 7.87 (m, 2H), 7.74 (dd, 1H), 7.47 (d, 1H), 7.12 (s, 1H), 7.06 - 6.99 (m, 2H), 6.92 - 6.84 (m, 2H), 4.58 (s, 2H), 3.76 - 3.57 (m, 7H), 2.70 - 2.56 (m, 3H), 2.49 - 2.29 (m, 5H), 2.23 - 1.96 (m, 4H), 1.85 - 1.63 (m, 3H), 1.29 - 1.11 (m, 2H).

LCMS m/z = 748.6 [M+1] ⁺.

### Example 50: preparation of compound 50

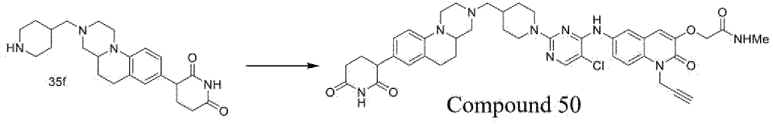

To a reaction flask were respectively added the above-mentioned crude trifluoroacetate of 35f (57 mg), DIPEA (47 mg, 0.36 mmol) and DMSO (5 mL). The above-mentioned crude 38g (52 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 50 (35 mg, two-step yield from compound 38f: 35%).

¹H NMR (400 MHz, DMSO-*d*₆) δ 10.72 (s, 1H), 8.82 (s, 1H), 8.04 (s, 1H), 8.00 - 7.90 (m, 2H), 7.78 (dd, 1H), 7.51 (d, 1H), 7.12 (s, 1H), 6.87 - 6.80 (m, 1H), 6.80 - 6.72 (m, 2H), 5.20 - 5.05 (m, 2H), 4.65 - 4.40 (m, 4H), 3.78 - 3.58 (m, 2H), 3.29 - 3.23 (m, 1H), 2.97 - 2.71 (m, 6H), 2.71 - 2.53 (m, 6H), 2.48 - 2.38 (m, 1H), 2.24 - 2.13 (m, 2H), 2.13 - 1.93 (m, 3H), 1.90 - 1.69 (m, 5H), 1.67 - 1.52 (m, 1H), 1.11 - 0.95 (m, 2H).

LCMS m/z = 792.3 [M+1] ⁺.

### Example 51: preparation of compound 51

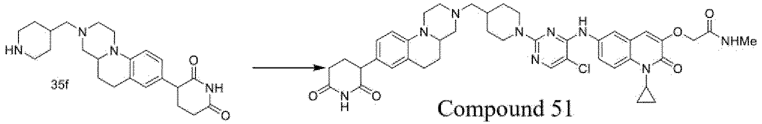

To a reaction flask were respectively added the above-mentioned crude trifluoroacetate of 35f (57 mg), DIPEA (47 mg, 0.36 mmol) and DMSO (5 mL). The above-mentioned crude 36g (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: ammonium acetate in water (5 mmol/L)/acetonitrile. Gradient elution method: gradient elution with acetonitrile from 10% to 70% (elution time: 12 min). Lyophilization was performed to obtain compound 51 (25 mg, four-step yield from compound 36d: 17%).

¹H NMR (400 MHz, DMSO-*d*₆) δ 10.72 (s, 1H), 8.77 (s, 1H), 8.03 (s, 1H), 7.97 - 7.87 (m, 2H), 7.78 - 7.67 (m, 2H), 7.03 (s, 1H), 6.87 - 6.80 (m, 1H), 6.80 - 6.72 (m, 2H), 4.57 - 4.40 (m, 4H), 3.79 - 3.60 (m, 2H), 3.05 - 2.96 (m, 1H), 2.96 - 2.71 (m, 6H), 2.71 - 2.53 (m, 6H), 2.48 - 2.39 (m, 1H), 2.20 - 1.92 (m, 5H), 1.91 - 1.68 (m, 5H), 1.67 - 1.51 (m, 1H), 1.35 - 1.27 (m, 2H), 1.12 - 0.95 (m, 2H), 0.83 - 0.73 (m, 2H).

LCMS m/z = 794.3 [M+1] ⁺.

### Example 52: preparation of trifluoroacetate of compound 52

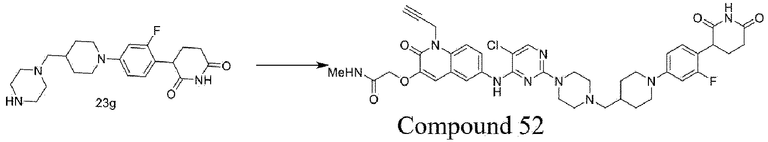

To a reaction flask were respectively added the above-mentioned crude trifluoroacetate of 23g (47 mg), DIPEA (160 mg, 1.24 mmol) and DMSO (5 mL). The above-mentioned crude 38g (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 52 (40 mg).

¹H NMR (400 MHz, DMSO-*d*₆) δ 10.79 (s, 1H), 9.08 (s, 1H), 8.16 (s, 1H), 8.00 - 7.92 (m, 2H), 7.74 (dd, 1H), 7.52 (d, 1H), 7.19 (s, 1H), 7.12 - 7.04 (m, 1H), 6.80 - 6.69 (m, 2H), 5.18 - 5.09 (m, 2H), 4.60 (s, 2H), 4.56 - 4.43 (m, 2H), 3.93 - 3.84 (m, 1H), 3.83 - 3.69 (m, 2H), 3.64 - 3.50 (m, 2H), 3.41 - 3.24 (m, 3H), 3.15 - 2.96 (m, 4H), 2.82 - 2.60 (m, 6H), 2.56 - 2.50 (m, 1H), 2.23 - 1.89 (m, 3H), 1.88 - 1.73 (m, 2H), 1.38 - 1.20 (m, 2H).

LCMS m/z = 784.3 [M+1] ⁺.

### Example 53: preparation of trifluoroacetate of compound 53

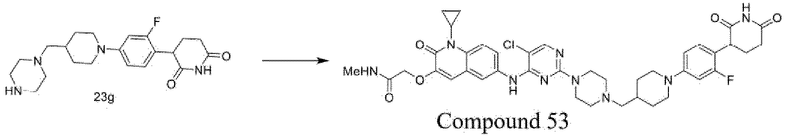

To a reaction flask were respectively added the above-mentioned crude trifluoroacetate of 23g (47 mg), DIPEA (47 mg, 0.36 mmol) and DMSO (5 mL). The above-mentioned crude 36g (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 53 (50 mg).

¹H NMR (400 MHz, DMSO-*d*₆) δ 10.79 (s, 1H), 9.04 (s, 1H), 8.15 (s, 1H), 7.98 - 7.91 (m, 1H), 7.90 - 7.84 (m, 1H), 7.82 - 7.74 (m, 1H), 7.68 (dd, 1H), 7.14 - 7.03 (m, 2H), 6.80 - 6.70 (m, 2H), 4.60 - 4.40 (m, 4H), 3.94 - 3.83 (m, 1H), 3.82 - 3.70 (m, 2H), 3.65 - 3.50 (m, 2H), 3.41 - 3.23 (m, 2H), 3.15 - 2.95 (m, 5H), 2.81 - 2.62 (m, 6H), 2.56 - 2.49 (m, 1H), 2.22 - 1.88 (m, 3H), 1.88 - 1.74 (m, 2H), 1.40 - 1.20 (m, 4H), 0.85 - 0.73 (m, 2H).

LCMS m/z = 786.3 [M+1] ⁺.

### Example 54: preparation of trifluoroacetate of compound 54

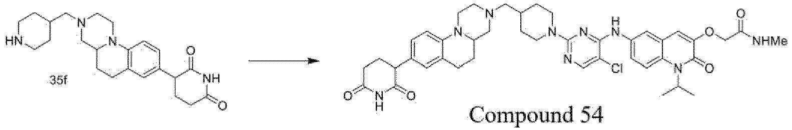

To a reaction flask were respectively added the above-mentioned crude trifluoroacetate of 35f (61 mg), DIPEA (54 mg, 0.42 mmol) and DMSO (5 mL). 42A (61 mg, 0.14 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 54 (25 mg).

¹H NMR (400 MHz, DMSO-*d*₆) δ 10.75 (s, 1H), 8.95 (s, 1H), 8.08 (s, 1H), 8.03 - 7.90 (m, 2H), 7.76 - 7.63 (m, 2H), 7.03 (s, 1H), 6.96 - 6.78 (m, 3H), 5.55 - 5.15 (m, 1H), 4.60 - 4.42 (m, 4H), 4.15 - 4.00 (m, 1H), 3.75 - 3.54 (m, 3H), 3.35 - 3.21 (m, 1H), 3.18 - 2.74 (m, 8H), 2.74 - 2.57 (m, 5H), 2.50 - 2.41 (m, 1H), 2.27 - 2.05 (m, 2H), 2.05 - 1.92 (m, 2H), 1.89 - 1.70 (m, 2H), 1.70 - 1.50 (m, 7H), 1.28 - 1.10 (m, 2H).

LCMS m/z = 796.3 [M+1] ⁺.

### Example 55: preparation of trifluoroacetate of compound 55

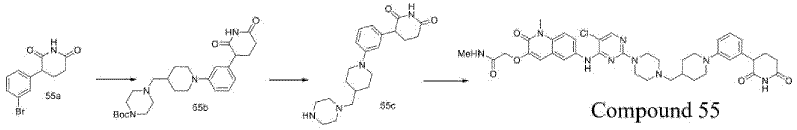

The trifluoroacetate of compound 55 was prepared by acidic preparative chromatography (aqueous trifluoroacetic acid solution (0.1% TFA)/acetonitrile) and lyophilization using compound 55a as the raw material and referring to the synthesis method of example 8.

¹H NMR (400 MHz, DMSO-*d*₆) δ 10.80 (s, 1H), 9.07 (s, 1H), 8.15 (s, 1H), 7.99 - 7.88 (m, 2H), 7.71 (dd, 1H), 7.48 (d, 1H), 7.24 - 7.13 (m, 2H), 6.94 - 6.80 (m, 2H), 6.70 - 6.60 (m, 1H), 4.59 (s, 2H), 4.56 - 4.42 (m, 2H), 3.83 - 3.65 (m, 6H), 3.64 - 3.51 (m, 2H), 3.39 - 3.25 (m, 2H), 3.15 - 2.97 (m, 4H), 2.82 - 2.57 (m, 6H), 2.55 - 2.42 (m, 1H), 2.28 - 2.13 (m, 1H), 2.13 - 1.95 (m, 2H), 1.93 - 1.75 (m, 2H), 1.45 - 1.25 (m, 2H).

LCMS m/z = 742.2 [M+1] ⁺.

### Example 56: preparation of trifluoroacetate of compound 56

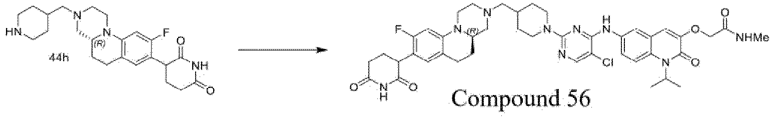

To a reaction flask were respectively added the above-mentioned crude 44h (91 mg), DIPEA (280 mg, 2.17 mmol) and DMSO (10 mL). 42A (96 mg, 0.22 mmol) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 70% (elution time: 12 min). Lyophilization was performed to obtain the trifluoroacetate of compound 56 (60 mg).

¹H NMR (400 MHz, DMSO-*d*₆) δ 10.80 (s, 1H), 9.00 (s, 1H), 8.09 (s, 1H), 8.04 - 7.90 (m, 2H), 7.75 - 7.64 (m, 2H), 7.03 (s, 1H), 6.88 (d, 1H), 6.80 (d, 1H), 5.70 - 4.90 (m, 1H), 4.56 (s, 2H), 4.54 - 4.42 (m, 2H), 4.15 - 4.00 (m, 1H), 3.92 - 3.80 (m, 1H), 3.70 - 3.53 (m, 2H), 3.42 - 3.25 (m, 1H), 3.18 - 3.00 (m, 4H), 3.00 - 2.80 (m, 3H), 2.80 - 2.57 (m, 6H), 2.57 - 2.50 (m, 1H), 2.28 - 2.05 (m, 2H), 2.05 - 1.87 (m, 2H), 1.87 - 1.70 (m, 2H), 1.70 - 1.50 (m, 7H), 1.29 - 1.10 (m, 2H).

LCMS m/z = 814.2 [M+1] ⁺.

### Example 57: preparation of trifluoroacetate of compound 57

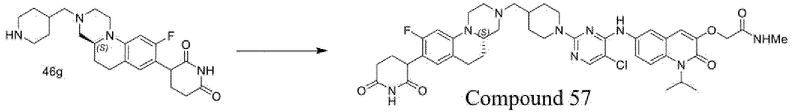

To a reaction flask were respectively added the above-mentioned crude 46g (91 mg), DIPEA (280 mg, 2.17 mmol) and DMSO (10 mL). 42A (96 mg, 0.22 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% trifluoroacetic acid)/acetonitrile. Gradient elution method: gradient elution with acetonitrile from 10% to 70% (elution time: 12 min). Lyophilization was performed to obtain the trifluoroacetate of compound 57 (50 mg).

¹H NMR (400 MHz, CD₃OD) δ 8.06 (s, 1H), 8.04 - 7.99 (m, 1H), 7.84 - 7.75 (m, 1H), 7.72 - 7.64 (m, 1H), 7.22 (s, 1H), 6.98 - 6.88 (m, 1H), 6.73 (d, 1H), 5.75 - 5.05 (m, 1H), 4.62 (s, 2H), 4.56 - 4.44 (m, 2H), 4.18 - 4.04 (m, 1H), 3.95 - 3.85 (m, 1H), 3.81 - 3.62 (m, 2H), 3.44 - 3.34 (m, 1H), 3.30 - 3.05 (m, 6H), 3.05 - 2.92 (m, 1H), 2.92 - 2.80 (m, 4H), 2.80 - 2.62 (m, 3H), 2.40 - 2.17 (m, 2H), 2.17 - 2.02 (m, 2H), 2.01 - 1.88 (m, 2H), 1.86 - 1.75 (m, 1H), 1.75 - 1.67 (m, 6H), 1.48 - 1.32 (m, 2H).

LCMS m/z = 814.2 [M+1] ⁺.

The trifluoroacetate of compound 57 was subjected to chiral resolution to obtain the trifluoroacetate of chiral isomer 1, and chiral isomer 2, respectively. Resolution conditions were as follows:
1. Instrument: Waters 150 Prep-SFC A; chromatographic column: Chiralcel OJ column.
2. The sample was dissolved in acetonitrile and filtered with a 0.45 µm filter to prepare a sample liquid.
3. Preparative chromatography conditions: a. the mobile phase consists of systems A and B: mobile phase A: CO₂; mobile phase B: a mixed solvent of methanol/acetonitrile; b. isocratic elution, mobile phase B content: 40%; c. flow rate: 100 mL/min.

Chiral analysis method:
1. Instrument: SHIMAZU LC-20AD; chromatographic column: Whelk column.
2. Analytical chromatography conditions: a. the mobile phase consists of systems A and B: mobile phase A: n-hexane; mobile phase B: a solution of isopropanol and acetonitrile containing 0.1% isopropylamine; b. isocratic elution, mobile phase B content: 80%; c. flow rate: 1 mL/min.

Retention time: chiral isomer 1: 1.963 min, chiral isomer 2: 3.594 min.

NMR characterization of the trifluoroacetate of chiral isomer 1:
¹H NMR (400 MHz, DMSO-*d*₆) δ 10.77 (s, 1H), 8.85 (s, 1H), 8.06 (s, 1H), 8.02 - 7.88 (m, 2H), 7.76 - 7.60 (m, 2H), 7.03 (s, 1H), 6.94 - 6.70 (m, 2H), 5.55 - 4.95 (m, 1H), 4.63 - 4.37 (m, 4H), 4.18 - 3.97 (m, 1H), 3.92 - 3.77 (m, 1H), 3.70 - 3.52 (m, 2H), 3.45 - 3.22 (m, 1H), 3.17 - 2.98 (m, 4H), 2.98 - 2.80 (m, 3H), 2.80 - 2.59 (m, 6H), 2.57 - 2.50 (m, 1H), 2.25 - 2.05 (m, 2H), 2.05 - 1.87 (m, 2H), 1.87 - 1.70 (m, 2H), 1.68 - 1.50 (m, 7H), 1.30 - 1.10 (m, 2H).

NMR characterization of chiral isomer 2:
¹H NMR (400 MHz, DMSO-*d*₆) δ 10.75 (s, 1H), 8.77 (s, 1H), 8.03 (s, 1H), 7.98 - 7.85 (m, 2H), 7.78 - 7.62 (m, 2H), 7.02 (s, 1H), 6.78 (d, 1H), 6.61 (d, 1H), 5.55 - 5.05 (m, 1H), 4.58 - 4.42 (m, 4H), 3.86 - 3.76 (m, 1H), 3.75 - 3.64 (m, 1H), 3.02 - 2.79 (m, 5H), 2.79 - 2.52 (m, 7H), 2.50 - 2.42 (m, 1H), 2.25 - 1.68 (m, 10H), 1.66 - 1.49 (m, 7H), 1.13 - 0.95 (m, 2H).

### Example 58: preparation of trifluoroacetate of compound 58

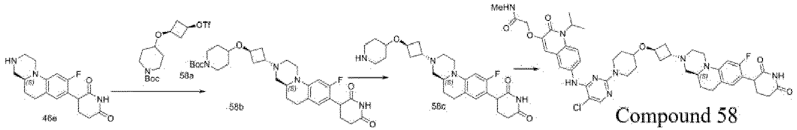

### Step 1: preparation of 58b

The above-mentioned crude 46e (0.24 g) and 58a (0.46 g, 1.14 mmol) (see WO 2022221673 for the synthesis method) were dissolved in acetonitrile (10 mL). Triethylamine (0.38 g, 3.76 mmol) was added, and the mixture was stirred at 25°C for 20 h. The reaction system was concentrated under reduced pressure, and the crude was separated and purified by silica gel column chromatography (methanol/dichloromethane (v/v) = 0:1-1:9) to obtain 58b (0.30 g, yield: 46%).

LCMS m/z = 571.2 [M+1]⁺.

### Step 2: preparation of 58c

58b (300 mg, 0.53 mmol) was dissolved in dichloromethane (2 mL). Trifluoroacetic acid (1 mL) was added, and the mixture was reacted at room temperature for 3 h. The reaction system was concentrated under reduced pressure, adjusted to pH 9 with a saturated aqueous sodium bicarbonate solution, and extracted with dichloromethane (50 mL×2). The organic phase was dried over anhydrous sodium sulphate, and concentrated under reduced pressure to obtain crude 58c (150 mg).

LCMS m/z = 471.3 [M+1]⁺.

### Step 3: preparation of trifluoroacetate of compound 58

To a reaction flask were respectively added the above-mentioned crude 58c (61 mg), DIPEA (170 mg, 1.32 mmol) and DMSO (10 mL). 42A (55 mg, 0.13 mmol) was added, and the mixture was reacted at 100°C for 20 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 58 (40 mg).

¹H NMR (400 MHz, DMSO-*d*₆) δ 10.78 (s, 1H), 8.96 (s, 1H), 8.07 (s, 1H), 8.00 - 7.90 (m, 2H), 7.73 - 7.62 (m, 2H), 7.03 (s, 1H), 6.87 (d, 1H), 6.80 (d, 1H), 5.52 - 5.15 (m, 1H), 4.54 (s, 2H), 4.38 - 4.25 (m, 1H), 4.18 - 4.03 (m, 3H), 3.97 - 3.80 (m, 2H), 3.65 - 3.43 (m, 3H), 3.34 - 3.18 (m, 3H), 3.05 - 2.85 (m, 2H), 2.82 - 2.50 (m, 10H), 2.36 - 1.77 (m, 7H), 1.71 - 1.50 (m, 7H), 1.49 - 1.33 (m, 2H).

LCMS m/z = 870.3 [M+1] ⁺.

### Example 59: preparation of trifluoroacetate of compound 59

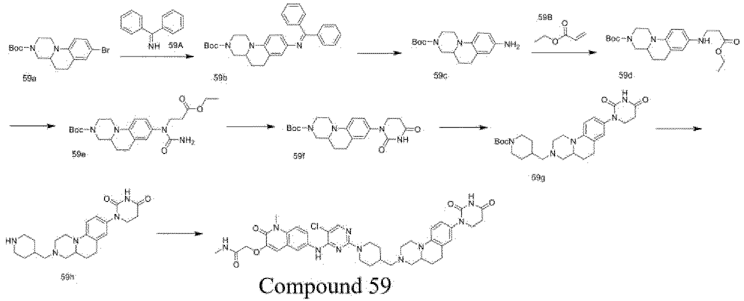

### Step 1: preparation of 59b

59a (1.28 g, 3.48 mmol) (see Bioorg. Med. Chem. Lett. 2016, 26, 5877-5882 for the synthesis method), caesium carbonate (2.27 g, 6.97 mmol), palladium acetate (0.16 g, 0.71 mmol), XantPhos (0.20 g, 0.35 mmol) and 59A (0.89 g, 4.91 mmol) were added to a 1,4-dioxane solution (40 mL), and the mixture was reacted at 105°C under nitrogen atmosphere for 3 h. The reaction system was cooled to room temperature. Water (100 mL) was added, and the mixture was extracted with ethyl acetate (80 mL×3). The organic phase was concentrated under reduced pressure, and the crude was separated and purified by silica gel column chromatography (petroleum ether/ethyl acetate (v/v) = 1:0-7:3) to obtain 59b (1.30 g, yield: 80%).

LCMS m/z = 468.4 [M+1]⁺.

### Step 2: preparation of 59c

59b (1.30 g, 2.78 mmol) was added to methanol (10 mL), and 10% palladium on carbon (1.33 g) and ammonium acetate (1.32 g, 17.13 mmol) were added. The mixture was reacted at room temperature under the atmosphere of hydrogen balloon for 12 h. The reaction system was filtered over diatomaceous earth. The filtrate was concentrated under reduced pressure, and the crude was separated and purified by silica gel column chromatography (petroleum ether/ethyl acetate (v/v) = 1:0-1:1) to obtain 59c (0.76 g, yield: 90%).

LCMS m/z = 304.4 [M+1]⁺.

### Step 3: preparation of 59d

59c (0.76 g, 2.55 mmol), 59B (0.75 g, 7.49 mmol) and DIPEA (0.97 g, 7.50 mmol) were successively added to ethanol (50 mL), and the mixture was reacted at 100°C for 48 h. The reaction system was cooled to room temperature and concentrated under reduced pressure, and the crude was separated and purified by silica gel column chromatography (methanol/dichloromethane (v/v) = 0:1-6:94) to obtain 59d (0.70 g, yield: 68%).

LCMS m/z = 404.2 [M+1]⁺.

### Step 4: preparation of 59e

59d (700 mg, 1.74 mmol) and DIPEA (671 mg, 5.19 mmol) were added to THF (20 mL). Triphosgene (565 mg, 1.90 mmol) was slowly added, and the mixture was reacted at room temperature for 1 h. To the reaction system was added ammonia water (5 mL), and the mixture was reacted at 50°C for 2 h. The reaction system was cooled to room temperature. Water (50 mL) was added, and the mixture was extracted with ethyl acetate (50 mL×2). The organic phase was concentrated under reduced pressure, and the crude was separated and purified by silica gel column chromatography (methanol/dichloromethane (v/v) = 0:1-1:9) to obtain 59e (700 mg, yield: 90%).

### Step 5: preparation of 59f

59f (0.70 g, 1.57 mmol) was added to acetonitrile (30 mL). A solution of 40% benzyltrimethylammonium hydroxide in methanol (2 mL) was added, and the mixture was reacted at 60°C for 2 h. The reaction system was cooled to room temperature and concentrated under reduced pressure, and the crude was separated and purified by silica gel column chromatography (methanol/dichloromethane (v/v) = 0:1-8:92) to obtain 59f (530 mg, yield: 84%).

LCMS m/z = 401.4 [M+1]⁺.

### Step 6: preparation of 59g

To a reaction flask were added 59f (0.18 g, 0.45 mmol), trifluoroacetic acid (1 mL) and dichloromethane (3 mL), and the mixture was reacted at room temperature for 2 h. The reaction solution was concentrated under reduced pressure, and adjusted to pH 7 by adding triethylamine. The mixture was concentrated under reduced pressure. The residue was dissolved with THF (10 mL). Tert-butyl 4-formylpiperidine-1-carboxylate (0.12 g, 0.56 mmol), acetic acid (0.3 mL) and sodium triacetoxyborohydride (0.19 g, 0.90 mmol) were successively added, and the resulting mixture was reacted at room temperature for 16 h. To the reaction solution were added ethyl acetate (12 mL) and a saturated aqueous sodium bicarbonate solution (12 mL). The aqueous phase was extracted with ethyl acetate (5 mL×3). The organic phase was washed with a saturated aqueous sodium chloride solution (5 mL), dried over anhydrous sodium sulphate, and concentrated under reduced pressure. The crude was separated and purified by silica gel column chromatography (dichloromethane : methanol (v/v) = 20:1) to obtain 59g (0.20 g, yield: 89%).

LCMS m/z = 498.2 [M+1] ⁺.

### Step 7: preparation of trifluoroacetate of 59h

To a reaction flask were added 59g (0.20 g, 0.40 mmol), trifluoroacetic acid (1 mL) and dichloromethane (3 mL), and the mixture was reacted at room temperature for 2 h. The reaction solution was concentrated under reduced pressure to obtain crude trifluoroacetate of 59h (0.20 g).

### Step 8: preparation of trifluoroacetate of compound 59

To a reaction flask were respectively added the above-mentioned crude trifluoroacetate of 59h (0.20 g), DIPEA (0.25 g, 1.93 mmol) and DMSO (5 mL). The mixture was stirred at room temperature for 0.5 h. **4B** (0.13 g, 0.33 mmol) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 59 (130 mg).

¹H NMR (400 MHz, DMSO-*d*₆) δ 10.24 (s, 1H), 9.08 (s, 1H), 8.11 (s, 1H), 8.03 - 7.87 (m, 2H), 7.78 - 7.69 (m, 1H), 7.53 - 7.42 (m, 1H), 7.12 (s, 1H), 7.06 - 6.88 (m, 3H), 4.65 - 4.55 (m, 2H), 4.54 - 4.35 (m, 2H), 4.17 - 4.02 (m, 1H), 3.75 - 3.55 (m, 7H), 3.40 - 3.23 (m, 1H), 3.23 - 2.75 (m, 8H), 2.75 - 2.60 (m, 6H), 2.28 - 2.10 (m, 1H), 2.08 - 1.93 (m, 1H), 1.90 - 1.72 (m, 2H), 1.72 - 1.56 (m, 1H), 1.31 - 1.11 (m, 2H).

LCMS m/z = 385.3 [M/2+1] ⁺.

### Example 60: preparation of trifluoroacetate of compound 60

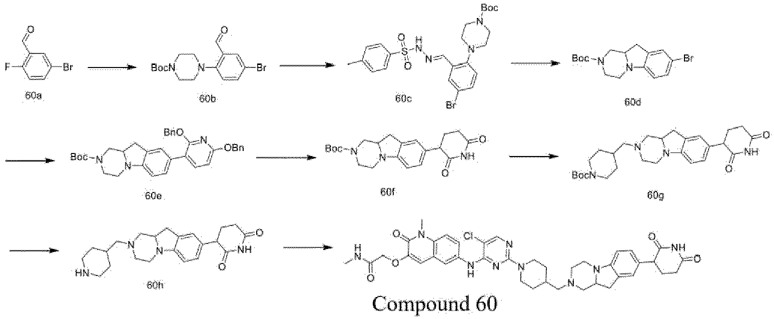

### Step 1: preparation of 60b

To a reaction flask were added 60a (10 g, 49.26 mmol), 1-tertbutoxycarbonyl piperazine (9.18 g, 49.29 mmol), potassium carbonate (8.17 g, 59.18 mmol) and DMSO (100 mL), and the mixture was reacted at 95°C under nitrogen protection for 12 h. The reaction solution was cooled to room temperature, and ethyl acetate (200 mL) and water (200 mL) were added. The organic phase was washed with a saturated aqueous sodium chloride solution (50 mL×3), dried over anhydrous sodium sulphate, and concentrated under reduced pressure. The crude was separated and purified by silica gel column chromatography (petroleum ether : ethyl acetate (v/v) = 10:1) to obtain 60b (6.0 g, yield: 33%).

LCMS m/z = 369.1 [M+1]⁺.

### Step 2: preparation of 60c

To a reaction flask were added 60b (6.0 g, 16.3 mmol), p-toluenesulfonyl hydrazide (3.03 g, 16.27 mmol) and ethanol (60 mL), and the mixture was reacted at room temperature under nitrogen protection for 2 h. The reaction solution was concentrated under reduced pressure to obtain crude 60c (5.0 g).

LCMS m/z = 537.1 [M+1]⁺.

### Step 3: preparation of 60d

To a reaction flask were added the above-mentioned crude 60c (4.0 g) and toluene (20 mL). Under nitrogen protection, 60% sodium hydride (0.36 g) was added in batches, and the mixture was reacted at room temperature under nitrogen atmosphere for 20 min, and then reacted at 135°C for 2 h. The reaction solution was cooled to room temperature, and ethyl acetate (100 mL) and water (100 mL) were added. The organic phase was washed with a saturated aqueous sodium chloride solution (50 mL), and concentrated under reduced pressure. The crude was separated and purified by silica gel column chromatography (petroleum ether : ethyl acetate (v/v) = 10:1) to obtain 60d (1.6 g, two-step yield calculated from 60b: 35%).

LCMS m/z = 353.3 [M+1] ⁺.

### Step 4: preparation of 60e

To a reaction flask were respectively added 60d (1.6 g, 4.54 mmol), 1,4-dioxane (20 mL) and water (5 mL). Under nitrogen protection, 2,6-bis(benzyloxy)-3-(4,4,5,5-tetramethyl-1,3,2-dioxaborolan-2-yl)pyridine (3.78 g, 9.06 mmol) (see WO 2021262812 for the synthesis method), caesium carbonate (4.43 g, 13.60 mmol) and Pd(dppf)Cl₂ (0.33 g, 0.45 mmol) were added, and the mixture was reacted at 100°C under nitrogen atmosphere for 16 h. The reaction system was cooled to room temperature, and filtered over diatomaceous earth. To the filtrate were added ethyl acetate (50 mL) and water (50 mL). The aqueous phase was extracted with ethyl acetate (20 mL×3). The organic phase was washed with a saturated aqueous sodium chloride solution (20 mL), dried over anhydrous sodium sulphate, and concentrated under reduced pressure. The crude was separated and purified by silica gel column chromatography (petroleum ether : ethyl acetate (v/v) = 3:1) to obtain 60e (1.80 g, yield: 70%).

### Step 5: preparation of 60f

To a reaction flask were respectively added 60e (1.8 g, 3.19 mmol), THF (40 mL) and 10% Pd/C (1.02 g). The mixture was reacted at room temperature under the atmosphere of hydrogen balloon for 16 h. The reaction system was filtered over diatomaceous earth. The filtrate was concentrated under reduced pressure and the crude was separated and purified by silica gel column chromatography (dichloromethane:methanol (v/v) = 10:1) to obtain 60f (0.9 g, yield: 73%).

LCMS m/z = 386.1 [M+1] ⁺

### Step 6: preparation of 60g

To a reaction flask were added 60f (0.9 g, 2.34 mmol), trifluoroacetic acid (3 mL) and dichloromethane (9 mL), and the mixture was reacted at room temperature for 2 h. The reaction solution was concentrated under reduced pressure, and adjusted to pH 7 by adding triethylamine. The mixture was concentrated under reduced pressure. The residue was dissolved with THF (30 mL). Tert-butyl 4-formylpiperidine-1-carboxylate (0.54 g, 2.53 mmol), acetic acid (1 mL) and sodium triacetoxyborohydride (0.99 g, 4.67 mmol) were successively added, and the resulting mixture was reacted at room temperature for 16 h. To the reaction solution were added ethyl acetate (30 mL) and a saturated aqueous sodium bicarbonate solution (30 mL). The aqueous phase was extracted with ethyl acetate (15 mL×3). The organic phase was washed with a saturated aqueous sodium chloride solution (10 mL), dried over anhydrous sodium sulphate, and concentrated under reduced pressure. The crude was separated and purified by silica gel column chromatography (dichloromethane : methanol (v/v) = 20:1) to obtain 60g (1.01 g, yield: 89%).

LCMS m/z = 483.6 [M+1] ⁺.

### Step 7: preparation of trifluoroacetate of 60h

To a reaction flask were added 60g (0.15 g, 0.31 mmol), trifluoroacetic acid (1 mL) and dichloromethane (3 mL), and the mixture was reacted at room temperature for 2 h. The reaction solution was concentrated under reduced pressure to obtain crude trifluoroacetate of 60h (0.20 g).

### Step 8: preparation of trifluoroacetate of compound 60

To a reaction flask were respectively added the above-mentioned crude trifluoroacetate of 60h (0.20 g), DIPEA (0.26 g, 2.01 mmol) and DMSO (5 mL). The mixture was stirred at room temperature for 0.5 h. **4B** (0.11 g, 0.28 mmol) was added, and the resulting mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 60 (43 mg).

¹H NMR (400 MHz, DMSO-*d*₆) δ 10.74 (s, 1H), 8.96 (s, 1H), 8.08 (s, 1H), 8.02 - 7.88 (m, 2H), 7.74 (dd, 1H), 7.46 (d, 1H), 7.11 (s, 1H), 7.02 - 6.95 (m, 1H), 6.95 - 6.87 (m, 1H), 6.62 (d, 1H), 4.59 (s, 2H), 4.54 - 4.40 (m, 2H), 3.94 - 3.80 (m, 2H), 3.76 - 3.62 (m, 4H), 3.62 - 3.42 (m, 2H), 3.35 - 3.20 (m, 1H), 3.15 - 2.77 (m, 7H), 2.72 - 2.55 (m, 5H), 2.50 - 2.38 (m, 1H), 2.24 - 1.91 (m, 3H), 1.84 - 1.66 (m, 2H), 1.24 - 1.06 (m, 2H).

LCMS m/z = 377.8 [M/2+1] ⁺

### Example 61: preparation of trifluoroacetate of compound 61

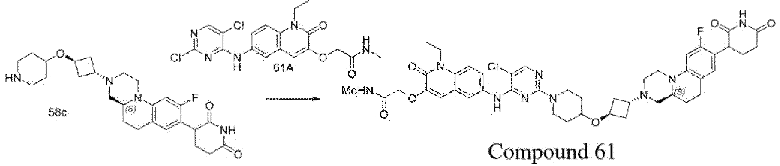

To a reaction flask were respectively added the above-mentioned crude 58c (52 mg), DIPEA (140 mg, 1.08 mmol) and DMSO (3 mL). 61A (45 mg, 0.107 mmol) (see WO 2022221673 for the synthesis method) was added, and the mixture was reacted at 100°C for 20 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 61 (40 mg).

¹H NMR (400 MHz, DMSO-*d*₆) δ 10.78 (s, 1H), 8.94 (s, 1H), 8.07 (s, 1H), 8.00 - 7.92 (m, 2H), 7.71 (dd, 1H), 7.52 (d, 1H), 7.09 (s, 1H), 6.87 (d, 1H), 6.80 (d, 1H), 4.60 - 4.54 (m, 2H), 4.40 - 4.25 (m, 3H), 4.18 - 4.03 (m, 3H), 3.95 - 3.79 (m, 2H), 3.63 - 3.42 (m, 3H), 3.32 - 3.16 (m, 3H), 3.04 - 2.85 (m, 2H), 2.80 - 2.50 (m, 10H), 2.36 - 1.77 (m, 7H), 1.70 - 1.54 (m, 1H), 1.48 - 1.32 (m, 2H), 1.29 - 1.19 (m, 3H).

LCMS m/z = 856.3 [M+1] ⁺.

### Example 62: preparation of trifluoroacetate of compound 62

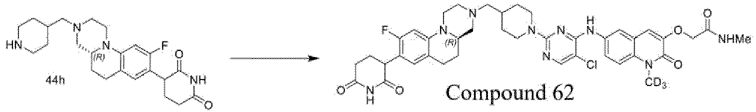

To a reaction flask were respectively added the above-mentioned crude 44h (50 mg), DIPEA (160 mg, 1.24 mmol) and DMSO (10 mL). The above-mentioned crude 48h (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 62 (20 mg).

¹H NMR (400 MHz, DMSO-*d*₆) δ 10.79 (s, 1H), 8.97 (s, 1H), 8.08 (s, 1H), 7.98 - 7.91 (m, 2H), 7.74 (dd, 1H), 7.47 (d, 1H), 7.12 (s, 1H), 6.87 (d, 1H), 6.79 (d, 1H), 4.59 (s, 2H), 4.54 - 4.42 (m, 2H), 4.15 - 4.00 (m, 1H), 3.92 - 3.80 (m, 1H), 3.68 (s, 3H), 3.66 - 3.56 (m, 2H), 3.41 - 3.28 (m, 1H), 3.18 - 2.80 (m, 7H), 2.80 - 2.59 (m, 3H), 2.56 - 2.51 (m, 1H), 2.27 - 2.05 (m, 2H), 2.05 - 1.87 (m, 2H), 1.87 - 1.70 (m, 2H), 1.70 - 1.53 (m, 1H), 1.30 - 1.07 (m, 2H).

LCMS m/z = 789.2 [M+1] ⁺.

### Example 63: preparation of trifluoroacetate of compound 63

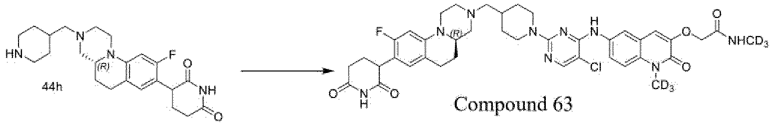

To a reaction flask were respectively added the above-mentioned crude 44h (50 mg), DIPEA (160 mg, 1.24 mmol) and DMSO (10 mL). The above-mentioned crude 49e (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 63 (30 mg).

¹H NMR (400 MHz, DMSO-*d*₆) δ 10.79 (s, 1H), 8.97 (s, 1H), 8.08 (s, 1H), 7.98 - 7.90 (m, 2H), 7.74 (dd, 1H), 7.46 (d, 1H), 7.12 (s, 1H), 6.87 (d, 1H), 6.79 (d, 1H), 4.59 (s, 2H), 4.55 - 4.39 (m, 2H), 4.13 - 4.00 (m, 1H), 3.93 - 3.79 (m, 1H), 3.69 - 3.55 (m, 2H), 3.42 - 3.26 (m, 1H), 3.20 - 2.58 (m, 10H), 2.57 - 2.51 (m, 1H), 2.28 - 2.05 (m, 2H), 2.05 - 1.87 (m, 2H), 1.87 - 1.70 (m, 2H), 1.70 - 1.53 (m, 1H), 1.30 - 1.05 (m, 2H).

LCMS m/z = 792.2 [M+1] ⁺.

### Example 64: preparation of trifluoroacetate of compound 64

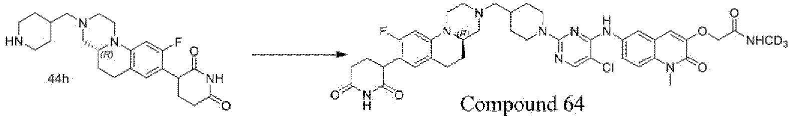

To a reaction flask were respectively added the above-mentioned crude 44h (50 mg), DIPEA (160 mg, 1.24 mmol) and DMSO (10 mL). The above-mentioned crude 47d (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 64 (25 mg).

¹H NMR (400 MHz, DMSO-*d*₆) δ 10.79 (s, 1H), 8.97 (s, 1H), 8.08 (s, 1H), 7.98 - 7.91 (m, 2H), 7.74 (dd, 1H), 7.47 (d, 1H), 7.12 (s, 1H), 6.87 (d, 1H), 6.79 (d, 1H), 4.59 (s, 2H), 4.55 - 4.40 (m, 2H), 4.13 - 4.00 (m, 1H), 3.91 - 3.80 (m, 1H), 3.68 (s, 3H), 3.66 - 3.55 (m, 2H), 3.44 - 3.26 (m, 1H), 3.18 - 2.58 (m, 10H), 2.56 - 2.51 (m, 1H), 2.28 - 2.05 (m, 2H), 2.05 - 1.88 (m, 2H), 1.88 - 1.70 (m, 2H), 1.70 - 1.52 (m, 1H), 1.29 - 1.08 (m, 2H).

LCMS m/z = 789.2 [M+1] ⁺.

### Example 65: preparation of trifluoroacetate of compound 65

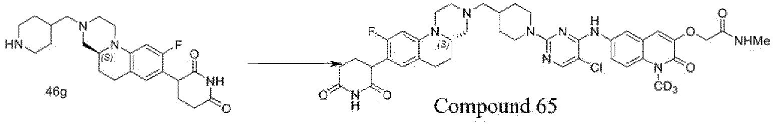

To a reaction flask were respectively added the above-mentioned crude 46g (50 mg), DIPEA (160 mg, 1.24 mmol) and DMSO (10 mL). The above-mentioned crude 48h (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 65 (50 mg).

¹H NMR (400 MHz, DMSO-*d*₆) δ 10.79 (s, 1H), 9.02 - 8.88 (m, 1H), 8.08 (s, 1H), 8.02 - 7.90 (m, 2H), 7.74 (dd, 1H), 7.46 (d, 1H), 7.11 (s, 1H), 6.87 (d, 1H), 6.79 (d, 1H), 4.59 (s, 2H), 4.54 - 4.41 (m, 2H), 4.13 - 4.02 (m, 1H), 3.92 - 3.77 (m, 1H), 3.68 - 3.54 (m, 2H), 3.42 - 3.26 (m, 1H), 3.18 - 2.80 (m, 7H), 2.80 - 2.57 (m, 6H), 2.56 - 2.51 (m, 1H), 2.27 - 2.05 (m, 2H), 2.05 - 1.87 (m, 2H), 1.87 - 1.70 (m, 2H), 1.70 - 1.53 (m, 1H), 1.28 - 1.05 (m, 2H).

LCMS m/z = 789.2 [M+1] ⁺.

### Example 66: preparation of trifluoroacetate of compound 66

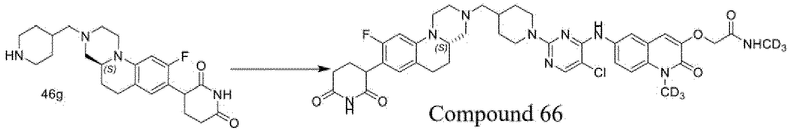

To a reaction flask were respectively added the above-mentioned crude 46g (50 mg), DIPEA (160 mg, 1.24 mmol) and DMSO (10 mL). The above-mentioned crude 49e (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 66 (45 mg).

¹H NMR (400 MHz, DMSO-*d*₆) δ 10.79 (s, 1H), 8.97 (s, 1H), 8.08 (s, 1H), 7.98 - 7.90 (m, 2H), 7.74 (dd, 1H), 7.46 (d, 1H), 7.14 - 7.09 (m, 1H), 6.87 (d, 1H), 6.79 (d, 1H), 4.59 (s, 2H), 4.53 - 4.41 (m, 2H), 4.14 - 4.01 (m, 1H), 3.93 - 3.77 (m, 1H), 3.70 - 3.53 (m, 2H), 3.42 - 3.26 (m, 1H), 3.18 - 2.80 (m, 7H), 2.80 - 2.57 (m, 3H), 2.57 - 2.50 (m, 1H), 2.27 - 2.05 (m, 2H), 2.05 - 1.87 (m, 2H), 1.87 - 1.70 (m, 2H), 1.70 - 1.53 (m, 1H), 1.30 - 1.08 (m, 2H).

LCMS m/z = 792.2 [M+1] ⁺.

### Example 67: preparation of trifluoroacetate of compound 67

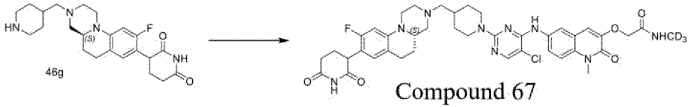

To a reaction flask were respectively added the above-mentioned crude 46g (50 mg), DIPEA (160 mg, 1.24 mmol) and DMSO (10 mL). The above-mentioned crude 47d (50 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 67 (50 mg).

¹H NMR (400 MHz, DMSO-*d*₆) δ 10.82 (s, 1H), 9.06 - 8.94 (m, 1H), 8.09 (s, 1H), 8.02 - 7.89 (m, 2H), 7.74 (dd, 1H), 7.47 (d, 1H), 7.11 (s, 1H), 6.88 (d, 1H), 6.80 (d, 1H), 4.60 (s, 2H), 4.54 - 4.42 (m, 2H), 4.13 - 4.01 (m, 1H), 3.91 - 3.79 (m, 1H), 3.68 (s, 3H), 3.65 - 3.56 (m, 2H), 3.40 - 3.26 (m, 1H), 3.20 - 2.80 (m, 7H), 2.80 - 2.58 (m, 3H), 2.57 - 2.50 (m, 1H), 2.28 - 2.05 (m, 2H), 2.05 - 1.88 (m, 2H), 1.88 - 1.70 (m, 2H), 1.70 - 1.52 (m, 1H), 1.29 - 1.05 (m, 2H).

LCMS m/z = 789.2 [M+1] ⁺.

### Example 68: preparation of trifluoroacetate of compound 68

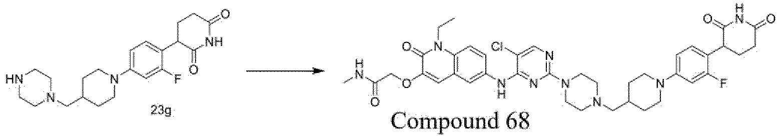

To a reaction flask were added the crude trifluoroacetate of 23g (0.19 g), DIPEA (0.39 g, 3.02 mmol) and DMSO (5 mL). 61A (0.089 g, 0.21 mmol) (see WO 2022221673 for the synthesis method) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 68 (55 mg).

¹H NMR (400 MHz, DMSO-*d*₆) δ 10.79 (s, 1H), 9.06 (s, 1H), 8.15 (s, 1H), 8.03 - 7.90 (m, 2H), 7.72 (dd, 1H), 7.53 (d, 1H), 7.15 (s, 1H), 7.09 (t, 1H), 6.81 - 6.70 (m, 2H), 4.58 (s, 2H), 4.55 - 4.42 (m, 2H), 4.33 (q, 2H), 3.95 - 3.83 (m, 1H), 3.83 - 3.70 (m, 2H), 3.67 - 3.51 (m, 2H), 3.44 - 3.25 (m, 2H), 3.17 - 2.95 (m, 4H), 2.83 - 2.62 (m, 6H), 2.57 - 2.50 (m, 1H), 2.24 - 1.90 (m, 3H), 1.88 - 1.75 (m, 2H), 1.40 - 1.17 (m, 5H).

LCMS m/z = 388.0 [M/2+1] ⁺.

### Example 69: preparation of trifluoroacetate of compound 69

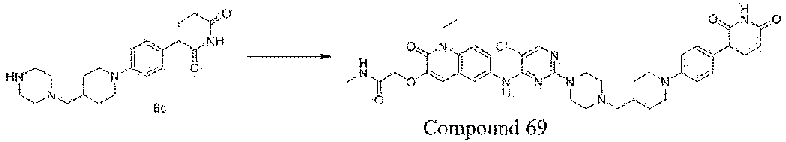

To a reaction flask were respectively added the above-mentioned crude trifluoroacetate of 8c (0.084 g), DIPEA (0.12 g, 0.93 mmol) and DMSO (5 mL). 61A (0.08 g, 0.19 mmol) was added, and the mixture was reacted at 100°C for 7 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 69 (20 mg).

¹H NMR (400 MHz, DMSO-*d*₆) δ 10.80 (s, 1H), 9.08 (s, 1H), 8.16 (s, 1H), 8.04 - 7.96 (m, 1H), 7.96 - 7.91 (m, 1H), 7.72 (dd, 1H), 7.53 (d, 1H), 7.19 - 7.02 (m, 5H), 4.65 - 4.41 (m, 4H), 4.33 (q, 2H), 3.84 - 3.66 (m, 3H), 3.66 - 3.52 (m, 2H), 3.44 - 3.25 (m, 2H), 3.18 - 2.96 (m, 4H), 2.96 - 2.76 (m, 2H), 2.73 - 2.57 (m, 4H), 2.50 - 2.42 (m, 1H), 2.24 - 1.95 (m, 3H), 1.95 - 1.82 (m, 2H), 1.51 - 1.34 (m, 2H), 1.25 (t, 3H).

LCMS m/z = 379.0 [M/2+1] ⁺.

### Example 70: preparation of trifluoroacetate of compound 70

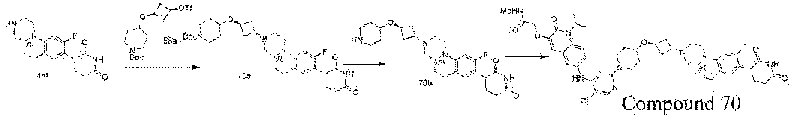

The trifluoroacetate of compound 70 was prepared by acidic preparative chromatography [water (containing 0.1% TFA)/acetonitrile] and lyophilization using 44f and 58a as the raw materials and referring to the synthesis method of example 58.

¹H NMR (400 MHz, DMSO-*d*₆) δ 10.79 (s, 1H), 9.00 (s, 1H), 8.08 (s, 1H), 8.00 - 7.90 (m, 2H), 7.73 - 7.62 (m, 2H), 7.03 (s, 1H), 6.87 (d, 1H), 6.80 (d, 1H), 5.55 - 5.15 (m, 1H), 4.55 (s, 2H), 4.37 - 4.25 (m, 1H), 4.20 - 4.03 (m, 3H), 3.97 - 3.80 (m, 2H), 3.65 - 3.43 (m, 3H), 3.34 - 3.18 (m, 3H), 3.05 - 2.85 (m, 2H), 2.82 - 2.50 (m, 10H), 2.36 - 1.77 (m, 7H), 1.71 - 1.50 (m, 7H), 1.49 - 1.33 (m, 2H). LCMS m/z = 870.3 [M+1] ⁺.

### Example 71: preparation of trifluoroacetate of compound 71

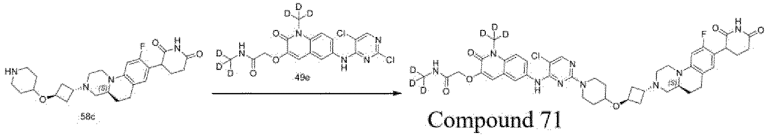

The trifluoroacetate of compound 71 was prepared by acidic preparative chromatography [water (containing 0.1% TFA)/acetonitrile] and lyophilization using compounds 58c and 49e as the raw materials and referring to the synthesis method of example 58.

¹H NMR (400 MHz, DMSO-*d*₆) δ 10.82 (s, 1H), 9.06 (s, 1H), 8.09 (s, 1H), 7.99 - 7.89 (m, 2H), 7.71 (dd, 1H), 7.47 (d, 1H), 7.11 (s, 1H), 6.93 - 6.75 (m, 2H), 4.58 (s, 2H), 4.38 - 4.23 (m, 1H), 4.20 - 4.01 (m, 3H), 3.97 - 3.78 (m, 2H), 3.65 - 3.43 (m, 3H), 3.34 - 3.15 (m, 3H), 3.05 - 2.86 (m, 2H), 2.83 - 2.50 (m, 7H), 2.38 - 2.21 (m, 2H), 2.21 - 2.05 (m, 1H), 2.05 - 1.76 (m, 4H), 1.70 - 1.52 (m, 1H), 1.48 - 1.32 (m, 2H).

LCMS m/z = 425.1 [M/2 +1] ⁺.

### Example 72: preparation of trifluoroacetate of compound 72

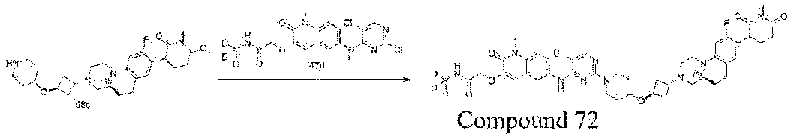

The trifluoroacetate of compound 72 was prepared by acidic preparative chromatography [water (containing 0.1% trifluoroacetic acid)/acetonitrile] and lyophilization using compounds 58c and 47d as the raw materials and referring to the synthesis method of example 58.

¹H NMR (400 MHz, DMSO-*d*₆) δ 10.82 (s, 1H), 8.98 (s, 1H), 8.07 (s, 1H), 7.98 - 7.90 (m, 2H), 7.71 (dd, 1H), 7.48 (d, 1H), 7.11 (s, 1H), 6.92 - 6.77 (m, 2H), 4.59 (s, 2H), 4.36 - 4.25 (m, 1H), 4.20 - 4.04 (m, 3H), 3.93 - 3.80 (m, 2H), 3.68 (s, 3H), 3.63 - 3.44 (m, 3H), 3.32 - 3.15 (m, 3H), 3.05 - 2.85 (m, 2H), 2.83 - 2.50 (m, 7H), 2.37 - 2.21 (m, 2H), 2.21 - 2.05 (m, 1H), 2.05 - 1.76 (m, 4H), 1.70 - 1.53 (m, 1H), 1.48 - 1.32 (m, 2H).

LCMS m/z = 845.3 [M+1] ⁺.

### Example 73: preparation of trifluoroacetate of compound 73

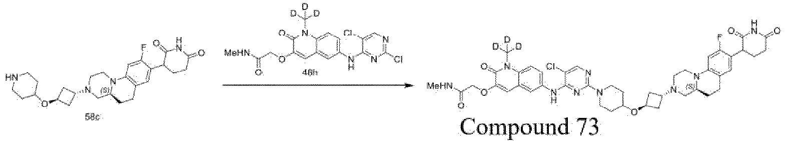

The trifluoroacetate of compound 73 was prepared by acidic preparative chromatography [water (containing 0.1% TFA)/acetonitrile] and lyophilization using compounds 58c and 48h as the raw materials and referring to the synthesis method of example 58.

¹H NMR (400 MHz, DMSO-*d*₆) δ 10.81 (s, 1H), 9.10 - 8.96 (m, 1H), 8.10 - 8.05 (m, 1H), 8.01 - 7.91 (m, 2H), 7.71 (dd, 1H), 7.47 (d, 1H), 7.11 (s, 1H), 6.93 - 6.76 (m, 2H), 4.58 (s, 2H), 4.37 - 4.25 (m, 1H), 4.18 - 4.02 (m, 3H), 3.95 - 3.80 (m, 2H), 3.64 - 3.44 (m, 3H), 3.34 - 3.15 (m, 3H), 3.04 - 2.85 (m, 2H), 2.82 - 2.50 (m, 10H), 2.37 - 2.20 (m, 2H), 2.20 - 2.05 (m, 1H), 2.05 - 1.77 (m, 4H), 1.72 - 1.52 (m, 1H), 1.50 - 1.32 (m, 2H).

LCMS m/z = 423.5 [M/2+1] ⁺.

### Example 74: preparation of trifluoroacetate of compound 74

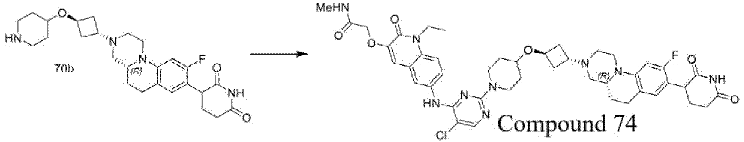

The trifluoroacetate of compound 74 was prepared by acidic preparative chromatography [water (containing 0.1% TFA)/acetonitrile] and lyophilization using compounds 70b and 61A as the raw materials and referring to the synthesis method of example 58.

¹H NMR (400 MHz, DMSO-*d*₆) δ 10.80 (s, 1H), 8.97 (s, 1H), 8.10 - 8.04 (m, 1H), 8.02 - 7.92 (m, 2H), 7.79 - 7.63 (m, 1H), 7.61 - 7.48 (m, 1H), 7.09 (s, 1H), 6.92 - 6.75 (m, 2H), 4.58 (s, 2H), 4.40 - 4.23 (m, 3H), 4.20 - 4.03 (m, 3H), 3.97 - 3.77 (m, 2H), 3.63 - 3.42 (m, 3H), 3.34 - 3.15 (m, 3H), 3.05 - 2.85 (m, 2H), 2.82 - 2.50 (m, 10H), 2.37 - 2.21 (m, 2H), 2.21 - 2.05 (m, 1H), 2.05 - 1.75 (m, 4H), 1.70 - 1.53 (m, 1H), 1.48 - 1.32 (m, 2H), 1.24 (t, 3H).

LCMS m/z = 856.3 [M+1] ⁺.

### Example 75: preparation of trifluoroacetate of compound 75

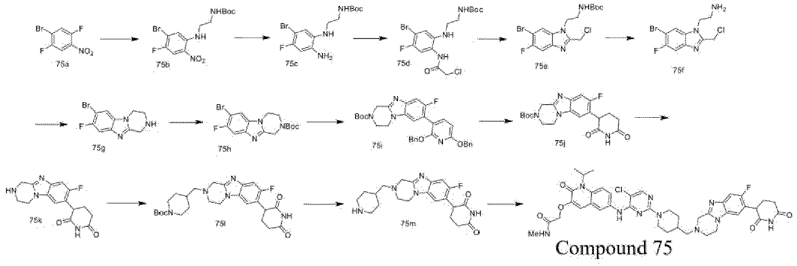

### Step 1: preparation of 75b

75a (2.0 g, 8.40 mmol), N-Boc-ethylenediamine (1.35 g, 8.43 mmol) and DIPEA (3.26 g, 25.22 mmol) were added to a reaction flask. DMSO (20 mL) was added, and the mixture was reacted at 50°C for 3 h. The reaction system was cooled to room temperature, and water (100 mL) and ethyl acetate (50 mL) were added. The organic phase was washed with a saturated aqueous sodium chloride solution (50 mL), dried over anhydrous sodium sulphate, and concentrated under reduced pressure to obtain crude 75b (3.2 g).

### Step 2: preparation of 75c

The above-mentioned crude 75b (3.1 g), iron powder (4.58 g, 81.79 mmol) and ammonium chloride (4.39 g, 82.07 mmol) were added to a reaction flask. Methanol (150 mL) was added, and the mixture was reacted at 70°C for 20 h. The reaction system was cooled to room temperature, and filtered over diatomaceous earth. The filtrate was concentrated under reduced pressure, and the crude was separated and purified by silica gel column chromatography (methanol :dichloromethane (v/v) = 0:1-1:9) to obtain 75c (2.7 g, two-step yield from compound 75a: 96%).

### Step 3: preparation of 75d

75c (2.7 g, 7.75 mmol) and TEA (2.35 g, 23.22 mmol) were dissolved in dichloromethane (25 mL). Chloroacetyl chloride (1.31 g, 11.6 mmol) was added dropwise, and the mixture was reacted at room temperature for 20 h. To the reaction solution was added water (50 mL). The organic phase was dried over anhydrous sodium sulphate, and concentrated under reduced pressure to obtain 75d (3.2 g, yield: 98%).

LCMS m/z = 424.2 [M+1]⁺.

### Step 4: preparation of 75e

75d (3.5 g, 8.27 mmol) was added to acetic acid (50 mL), and the mixture was reacted at 40°C for 7 h. The reaction system was cooled to 0°C, adjusted to pH 8 by adding 25% ammonia water and extracted with ethyl acetate (100 mL). The organic phase was dried over anhydrous sodium sulphate, and concentrated under reduced pressure. The crude was separated and purified by silica gel column chromatography (methanol : dichloromethane (v/v) = 0:1-1:9) to obtain 75e (3.0 g, yield: 89%).

### Step 5: Preparation of hydrochloride of 75f

75e (3.0 g, 7.38 mmol) was dissolved in dichloromethane (50 mL), and a solution of hydrogen chloride in 1,4-dioxane (50 mL, 4 mol/L) was added. The mixture was reacted at room temperature for 20 h. The reaction system was concentrated under reduced pressure to obtain crude hydrochloride of 75f (3.12 g).

### Step 6: preparation of 75g

The above-mentioned crude hydrochloride of 75f (3.0 g), potassium carbonate (3.48 g, 25.18 mmol) and KI (0.3 g, 1.81 mmol) were added to a reaction flask. Acetonitrile (50 mL) was added, and the mixture was reacted at 80°C for 7 h. The reaction system was cooled to room temperature. A mixed solvent of methanol/dichloromethane (v/v) = 1:4 (20 mL) was added, and the mixture was filtered. The filtrate was concentrated under reduced pressure, and the crude was separated and purified by silica gel column chromatography (methanol : dichloromethane (v/v) = 0:1-1:9) to obtain 75g (0.50 g, two-step yield from compound 75e: 26%).

### Step 7: preparation of 75h

75g (0.50 g, 1.85 mmol) was dissolved in dichloromethane (10 mL), Boc anhydride (0.81 g, 3.71 mmol) and DMAP (0.45 g, 3.68 mmol) were added. The mixture was reacted at room temperature for 16 h. The reaction system was washed with 0.5 mol/L hydrochloric acid (20 mL). The organic phase was dried over anhydrous sodium sulphate, and concentrated under reduced pressure. The crude was separated and purified by silica gel column chromatography (ethyl acetate : petroleum ether (v/v) = 0:1-1:9) to obtain 75h (0.2 g, yield: 29%).

### Step 8: preparation of 75i

75h (0.20 g, 0.54 mmol), 2,6-bisbenzyloxypyridine-3-boronic acid pinacol ester (0.34 g, 0.81 mmol), Pd(dppf)Cl₂·DCM (0.044 g, 0.054 mmol) and caesium carbonate (0.35 g, 1.07 mmol) were successively added to a reaction flask. 1,4-dioxane (10 mL) and water (1 mL) were added, and the mixture was reacted at 100°C under nitrogen atmosphere for 20 h. The mixture was cooled to room temperature, and water (50 mL) and ethyl acetate (50 mL) were added. The organic phase was washed with a saturated aqueous sodium chloride solution (50 mL), dried over anhydrous sodium sulphate, and concentrated under reduced pressure. The crude was separated and purified by silica gel column chromatography (petroleum ether/ethyl acetate (v/v) = 1:0-9:1) to obtain 75i (0.23 g, yield: 73%).

LCMS m/z = 581.2 [M+1]⁺.

### Step 9: preparation of 75j

75i (0.23 g, 0.396 mmol) was dissolved in THF (10 mL), and 10% palladium on carbon (0.20 g) was added. The mixture was reacted at 45°C under the atmosphere of hydrogen balloon for 20 h. The reaction system was cooled to room temperature, and filtered. The filtrate was concentrated under reduced pressure to obtain crude 75j (0.16 g).

LCMS m/z = 403.5 [M+1]⁺.

### Step 10: preparation of 75k

The crude 75j (250 mg) was dissolved in dichloromethane (2 mL). Trifluoroacetic acid (1 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude 75k (120 mg).

LCMS m/z = 303.3 [M+1]⁺.

### Step 11: preparation of 75l

The above-mentioned crude 75k (0.12 g) was dissolved in 1,2-dichloroethane (5 mL). Tert-butyl 4-formylpiperidine-1-carboxylate (0.077 g, 0.36 mmol) was added, and acetic acid (1 mL) was added. The mixture was stirred at room temperature for 1 h, and then sodium triacetoxyborohydride (0.13 g, 0.61 mmol) was added. The resulting mixture was reacted at room temperature for 16 h. To the reaction solution was slowly added a saturated aqueous sodium bicarbonate solution (30 mL), and the mixture was extracted with dichloromethane (50 mL). The organic phase was washed with a saturated aqueous sodium chloride solution (100 mL), dried over anhydrous sodium sulphate, and concentrated under reduced pressure. The crude was separated and purified by silica gel column chromatography (dichloromethane/methanol (v/v) = 10:1) to obtain 751 (0.06 g, yield: 33%).

LCMS m/z = 500.2 [M+1]⁺.

### Step 12: preparation of 75m

751 (60 mg, 0.12 mmol) was dissolved in dichloromethane (2 mL). Trifluoroacetic acid (1 mL) was added, and the mixture was reacted at room temperature for 3 h. The reaction system was concentrated under reduced pressure, adjusted to pH 9 with a saturated aqueous sodium bicarbonate solution, and extracted with dichloromethane (20 mL×2). The organic phase was dried over anhydrous sodium sulphate, and concentrated under reduced pressure to obtain crude 75m (48 mg).

LCMS m/z = 400.3 [M+1]⁺.

### Step 13: preparation of trifluoroacetate of compound 75

To a reaction flask were respectively added the above-mentioned crude 75m (48 mg), DIPEA (160 mg, 1.24 mmol) and DMSO (3 mL). 42A (52 mg, 0.12 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 70% (elution time: 12 min). Lyophilization was performed to obtain the trifluoroacetate of compound 75 (25 mg).

¹H NMR (400 MHz, CD₃OD) δ 8.05 (s, 1H), 8.00 - 7.93 (m, 1H), 7.84 - 7.75 (m, 1H), 7.70 - 7.57 (m, 1H), 7.30 - 7.12 (m, 1H), 6.89 (d, 1H), 6.70 (d, 1H), 5.70 - 5.10 (m, 1H), 4.65 - 4.53 (m, 2H), 4.51 - 4.38 (m, 2H), 4.15 - 4.00 (m, 1H), 3.95 - 3.82 (m, 1H), 3.79 - 3.55 (m, 1H), 3.45 - 3.31 (m, 1H), 3.26 - 3.05 (m, 7H), 3.01 - 2.58 (m, 5H), 2.40 - 1.86 (m, 5H), 1.74 - 1.62 (m, 6H), 1.48 - 1.30 (m, 2H).

### Example 76: preparation of trifluoroacetate of compound 76

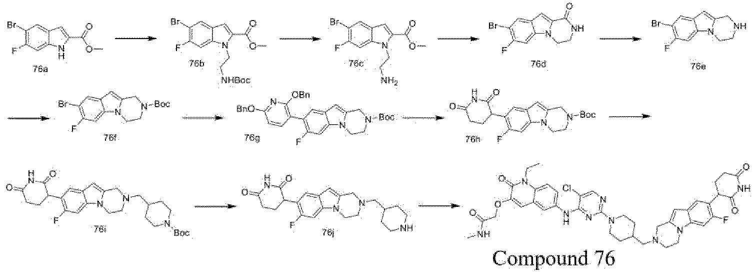

### Step 1: preparation of 76b

To a reaction flask were added 76a (3.0 g, 11.03 mmol) and DMSO (30 mL). Under nitrogen atmosphere, 60% sodium hydride (0.66 g) was added in batches at 0°C, and the mixture was reacted at room temperature for 0.5 h. Then tert-butyl (2-bromoethyl)carbamate (3.21 g, 14.32 mmol) was added, and the resulting mixture was reacted at 70°C for 16 h. The reaction system was cooled to room temperature, poured into ice water (100 mL), and extracted with ethyl acetate (50 mL×3). The organic phase was washed with a saturated aqueous sodium chloride solution (50 mL×3), dried over anhydrous sodium sulphate, and concentrated under reduced pressure. The crude was separated and purified by silica gel column chromatography (petroleum ether : ethyl acetate (v/v) = 10:1) to obtain 76b (2.6 g, yield: 57%).

### Step 2: preparation of trifluoroacetate of 76c

To a reaction flask were added 76b (3.4 g, 8.19 mmol), dichloromethane (15 mL) and trifluoroacetic acid (5 mL), and the mixture was reacted at room temperature for 2 h. The reaction solution was concentrated under reduced pressure to obtain crude trifluoroacetate of 76c (5.0 g).

### Step 3: preparation of 76d

To a reaction flask were added the above-mentioned crude trifluoroacetate of 76c (5.0 g), THF (70 mL) and water (30 mL). Sodium bicarbonate (7.74 g, 92.14 mmol) was added in batches, and the mixture was reacted at 60°C under nitrogen atmosphere for 16 h. The reaction solution was cooled to room temperature, and ethyl acetate (100 mL) and water (100 mL) were added. The organic phase was washed with a saturated aqueous sodium chloride solution (50 mL), and concentrated under reduced pressure. The crude was separated and purified by silica gel column chromatography (dichloromethane : methanol (v/v) = 20:1) to obtain 76d (2.2 g, two-step yield calculated from compound 76b: 95%).

LCMS m/z = 283.0 [M+1] ⁺.

### Step 4: preparation of 76e

To a reaction flask were respectively added 76d (1.6 g, 5.67 mmol), sodium borohydride (0.64 g, 16.92 mmol) and THF (60 mL). A solution of borane in THF (22.6 mL, 22.6 mmol, 1 mol/L) was slowly added dropwise at 50°C, and the mixture was reacted at 70°C for 16 h. The reaction system was cooled to room temperature. Water (16 mL) and hydrochloric acid (20 mL, 2 mol/L) were successively and slowly added dropwise. The pH was adjusted to 10 with an aqueous sodium hydroxide solution (5 mol/L). Ethyl acetate (50 mL) was added, and the mixture was filtered. The filter cake was washed with ethyl acetate (50 mL), and the filtrate was subjected to liquid separation. The aqueous phase was extracted with ethyl acetate (30 mL×2), and the organic phase was washed with a saturated aqueous sodium chloride solution (50 mL), dried over anhydrous sodium sulphate, and concentrated under reduced pressure to obtain crude 76e (1.4 g).

### Step 5: preparation of 76f

To a reaction flask were respectively added the above-mentioned crude 76e (1.4 g), di-tert-butyl dicarbonate (2.27 g, 10.4 mmol), DMAP (1.27 g, 10.4 mmol) and THF (40 mL), and the mixture was reacted at room temperature for 16 h. To the reaction solution was added 1 mol/L aqueous hydrogen chloride solution (20 mL), liquid separation was carried out. The organic phase was washed with 1 mol/L aqueous hydrogen chloride solution (50 mL×2) and a saturated aqueous sodium chloride solution (50 mL), and concentrated under reduced pressure. The crude was separated and purified by silica gel column chromatography (petroleum ether : ethyl acetate (v/v) = 3:1) to obtain 76f (1 g, two-step yield calculated from compound 76d: 48%).

LCMS m/z = 369.3 [M+1] ⁺.

### Step 6: preparation of 76g

To a reaction flask were respectively added 76f (1.0 g, 2.72 mmol), 1,4-dioxane (20 mL) and water (5 mL). Under nitrogen protection, 2,6-bis(benzyloxy)-3-(4,4,5,5-tetramethyl-1,3,2-dioxaborolan-2-yl)pyridine (1.70 g, 4.07 mmol), caesium carbonate (2.65 g, 8.13 mmol) and Pd(dppf)Cl₂ (0.20 g, 0.276 mmol) were added, and the mixture was reacted at 100°C under nitrogen atmosphere for 16 h. The reaction system was cooled to room temperature, and filtered over diatomaceous earth. To the filtrate were added ethyl acetate (50 mL) and water (50 mL). The aqueous phase was extracted with ethyl acetate (20 mL×3). The organic phase was washed with a saturated aqueous sodium chloride solution (20 mL), dried over anhydrous sodium sulphate, and concentrated under reduced pressure. The crude was separated and purified by silica gel column chromatography (petroleum ether : ethyl acetate (v/v) = 3:1) to obtain 76g (1.10 g, yield: 70%).

### Step 7: preparation of 76h

To a reaction flask were respectively added 76g (1.1 g, 1.90 mmol), THF (30 mL) and 10% Pd/C (1 g). The mixture was reacted at 40°C under the atmosphere of hydrogen balloon for 16 h. The reaction system was cooled to room temperature, and filtered over diatomaceous earth. The filtrate was concentrated under reduced pressure, and the crude was separated and purified by silica gel column chromatography (petroleum ether : ethyl acetate (v/v) = 1:1) to obtain 76h (0.52 g, yield: 68%).

LCMS m/z = 402.1 [M+1] ⁺.

### Step 8: preparation of 76i

To a reaction flask were added 76h (0.27 g, 0.67 mmol), trifluoroacetic acid (1 mL) and dichloromethane (3 mL), and the mixture was reacted at room temperature for 2 h. The reaction solution was concentrated under reduced pressure, and adjusted to pH 8 with triethylamine. The mixture was concentrated under reduced pressure. THF (10 mL) was added to the residue. Tert-butyl 4-formylpiperidine-1-carboxylate (0.16 g, 0.75 mmol), acetic acid (0.3 mL) and sodium triacetoxyborohydride (0.28 g, 1.32 mmol) were successively added, and the resulting mixture was reacted at room temperature for 16 h. To the reaction solution were added ethyl acetate (30 mL) and a saturated aqueous sodium bicarbonate solution (30 mL). The aqueous phase was extracted with ethyl acetate (15 mL×3). The organic phase was washed with a saturated aqueous sodium chloride solution (10 mL), dried over anhydrous sodium sulphate, and concentrated under reduced pressure. The crude was separated and purified by silica gel column chromatography (dichloromethane : methanol (v/v) = 20:1) to obtain 76i (0.27 g, yield: 81%).

### Step 9: preparation of 76j

To a reaction flask were added 76i (0.27 g, 0.54 mmol), trifluoroacetic acid (1 mL) and dichloromethane (3 mL), and the mixture was reacted at room temperature for 2 h. The reaction solution was concentrated under reduced pressure, and dichloromethane (15 mL) and a saturated aqueous sodium bicarbonate solution (15 mL) were added. The aqueous phase was extracted with a mixed solvent of dichloromethane/methanol (v/v) = 10:1 (15 mL×5), and the organic phase was washed with a saturated aqueous sodium chloride solution (15 mL), dried over anhydrous sodium sulphate, and concentrated under reduced pressure to obtain crude 76j (0.21 g).

### Step 10: preparation of trifluoroacetate of compound 76

To a reaction flask were respectively added the above-mentioned crude 76j (0.07 g), DIPEA (0.12 g, 0.93 mmol) and DMSO (5 mL). 61A (0.084 g, 0.20 mmol) (see WO 2022221673 for the synthesis method) was added, and the mixture was reacted at 100°C for 16 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 76 (26 mg).

¹H NMR (400 MHz, DMSO-*d*₆) δ 10.86 (s, 1H), 8.97 (s, 1H), 8.08 (s, 1H), 8.04 - 7.90 (m, 2H), 7.74 (dd, 1H), 7.56 - 7.34 (m, 3H), 7.09 (s, 1H), 6.43 (s, 1H), 5.00 - 3.50 (m, 13H), 3.35 - 3.05 (m, 2H), 3.01 - 2.85 (m, 2H), 2.85 - 2.69 (m, 1H), 2.69 - 2.60 (m, 3H), 2.60 - 2.51 (m, 1H), 2.34 - 2.15 (m, 2H), 2.10 - 1.95 (m, 1H), 1.90 - 1.75 (m, 2H), 1.30 - 1.11 (m, 5H).

LCMS m/z = 392.8 [M/2+1] ⁺.

### Example 77: preparation of trifluoroacetate of compound 77

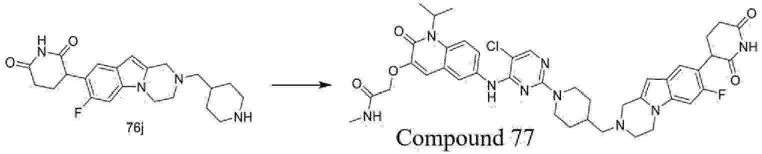

The trifluoroacetate of compound 77 was prepared by acidic preparative chromatography [water (containing 0.1% TFA)/acetonitrile] and lyophilization using compounds 76j and 42A as the raw materials and referring to the synthesis method of example 76.

¹H NMR (400 MHz, CD₃OD) δ 8.04 (s, 1H), 7.97 (d, 1H), 7.81 - 7.73 (m, 1H), 7.66 (dd, 1H), 7.46 (d, 1H), 7.27 - 7.15 (m, 2H), 6.48 (s, 1H), 5.70 - 5.10 (m, 1H), 4.69 (s, 2H), 4.56 (s, 2H), 4.54 - 4.43 (m, 2H), 4.42 - 4.30 (m, 2H), 4.11 - 4.00 (m, 1H), 3.97 - 3.84 (m, 2H), 3.30 - 3.22 (m, 2H), 3.18 - 3.02 (m, 2H), 2.82 (s, 3H), 2.80 - 2.72 (m, 1H), 2.72 - 2.62 (m, 1H), 2.43 - 2.25 (m, 2H), 2.25 - 2.10 (m, 1H), 2.00 - 1.85 (m, 2H), 1.74 - 1.62 (m, 6H), 1.48 - 1.26 (m, 2H).

LCMS m/z = 399.7 [M/2+1] ⁺.

### Example 78: preparation of trifluoroacetate of compound 78

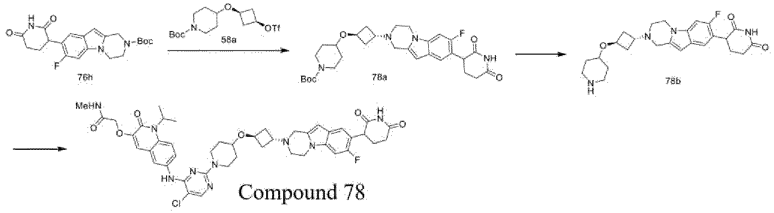

### Step 1: preparation of 78a

To a reaction flask were added 76h (0.27 g, 0.67 mmol), trifluoroacetic acid (1 mL) and dichloromethane (3 mL), and the mixture was reacted at room temperature for 2 h. The reaction solution was concentrated under reduced pressure, adjusted to pH 8 with triethylamine and concentrated under reduced pressure. Dichloromethane (10 mL) was added to the residue. 58a (0.54 g, 1.34 mmol) and TEA (0.34 g, 3.36 mmol) were added, and the resulting mixture was reacted at room temperature for 16 h. To the reaction solution were added dichloromethane (30 mL) and a saturated aqueous sodium bicarbonate solution (30 mL). The aqueous phase was extracted with dichloromethane (15 mL×3). The organic phase was washed with a saturated aqueous sodium chloride solution (10 mL), dried over anhydrous sodium sulphate, and concentrated under reduced pressure. The crude was separated and purified by silica gel column chromatography (dichloromethane : methanol (v/v) = 20:1) to obtain 78a (0.1 g, yield: 27%).

### Step 2: preparation of trifluoroacetate of 78b

To a reaction flask were added 78a (0.1 g, 0.18 mmol), trifluoroacetic acid (0.5 mL) and dichloromethane (1.5 mL), and the mixture was reacted at room temperature for 2 h. The reaction solution was concentrated under reduced pressure to obtain crude trifluoroacetate of 78b (0.14 g).

### Step 3: preparation of trifluoroacetate of compound 78

To a reaction flask were respectively added the above-mentioned crude trifluoroacetate of 78b (0.07 g), DIPEA (0.078 g, 0.60 mmol) and DMSO (3 mL). 42A (0.042 g, 0.097 mmol) was added, and the mixture was reacted at 100°C for 16 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 78 (39 mg).

¹H NMR (400 MHz, CD₃OD) δ 8.05 - 7.97 (m, 2H), 7.83 - 7.74 (m, 1H), 7.64 (dd, 1H), 7.47 (d, 1H), 7.28 - 7.18 (m, 2H), 6.50 (s, 1H), 5.70 - 5.10 (m, 1H), 4.58 (s, 4H), 4.48 - 4.28 (m, 3H), 4.18 - 3.95 (m, 4H), 3.88 - 3.63 (m, 3H), 3.52 - 3.38 (m, 2H), 2.88 - 2.61 (m, 7H), 2.61 - 2.47 (m, 2H), 2.43 - 2.26 (m, 1H), 2.24 - 2.12 (m, 1H), 2.03 - 1.87 (m, 2H), 1.74 - 1.58 (m, 8H).

LCMS m/z = 427.9 [M/2+1] ⁺.

### Example 79: preparation of trifluoroacetate of compound 79

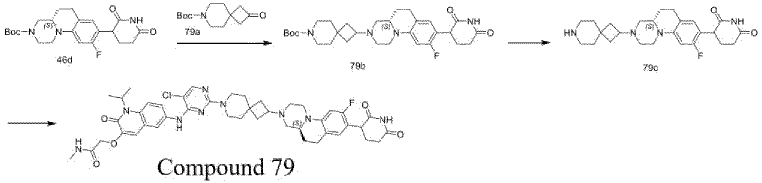

### Step 1: preparation of 79b

To a reaction flask were added 46d (0.15 g, 0.36 mmol), trifluoroacetic acid (1 mL) and dichloromethane (3 mL), and the mixture was reacted at room temperature for 3 h. The reaction solution was concentrated under reduced pressure, adjusted to pH 8 with triethylamine and concentrated under reduced pressure. THF (10 mL) was added to the residue. 79a (0.17 g, 0.71 mmol), acetic acid (0.3 mL) and sodium triacetoxyborohydride (0.15 g, 0.71 mmol) were successively added, and the resulting mixture was reacted at room temperature for 16 h. To the reaction solution were added ethyl acetate (30 mL) and a saturated aqueous sodium bicarbonate solution (30 mL). The aqueous phase was extracted with ethyl acetate (15 mL×3). The organic phase was washed with a saturated aqueous sodium chloride solution (15 mL), dried over anhydrous sodium sulphate, and concentrated under reduced pressure. The crude was separated and purified by silica gel column chromatography (dichloromethane : methanol (v/v) = 20:1) to obtain 79b (0.16 g, yield: 82%).

LCMS m/z = 541.3 [M+1] ⁺.

### Step 2: preparation of 79c

To a reaction flask were added 79b (0.16 g, 0.30 mmol), trifluoroacetic acid (1 mL) and dichloromethane (3 mL), and the mixture was reacted at room temperature for 3 h. The reaction solution was concentrated under reduced pressure, and dichloromethane (15 mL) was added. The pH was adjusted to 9 with a saturated aqueous sodium bicarbonate solution. The aqueous phase was extracted with a mixed solvent of dichloromethane and methanol (v/v) = 10:1 (15 mL×5), and the organic phase was washed with a saturated aqueous sodium chloride solution (15 mL), dried over anhydrous sodium sulphate, and concentrated under reduced pressure to obtain crude 79c (0.12 g).

### Step 3: preparation of trifluoroacetate of compound 79

To a reaction flask were respectively added the above-mentioned crude 79c (0.06 g), DIPEA (0.09 g, 0.70 mmol) and DMSO (5 mL). 42A (0.061 g, 0.14 mmol) was added, and the mixture was reacted at 100°C for 16 h. The reaction system was cooled to room temperature. The reaction solution was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 79 (70 mg).

¹H NMR (400 MHz, DMSO-*d*₆) δ 10.81 (s, 1H), 9.02 (s, 1H), 8.08 (s, 1H), 8.02 - 7.94 (m, 1H), 7.91 (s, 1H), 7.76 - 7.64 (m, 2H), 7.05 (s, 1H), 6.93 - 6.75 (m, 2H), 5.70 - 4.90 (m, 1H), 4.55 (s, 2H), 4.22 - 4.08 (m, 1H), 3.93 - 3.72 (m, 2H), 3.72 - 3.40 (m, 6H), 3.33 - 3.16 (m, 1H), 3.06 - 2.82 (m, 2H), 2.82 - 2.58 (m, 7H), 2.57 - 2.51 (m, 1H), 2.32 - 1.86 (m, 7H), 1.72 - 1.48 (m, 11H).

LCMS m/z = 420.9 [M/2+1] ⁺.

### Example 80: preparation of trifluoroacetate of compound 80

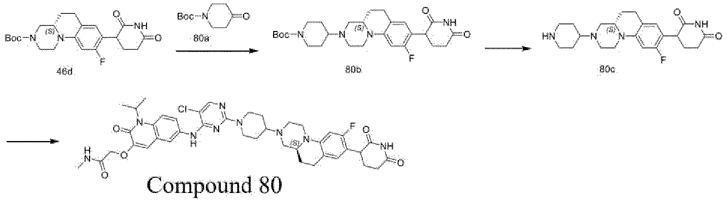

The trifluoroacetate of compound 80 was prepared using compounds 46d and 80a as the raw materials and referring to the synthesis method of example 79.

¹H NMR (400 MHz, DMSO-*d*₆) δ 10.79 (s, 1H), 8.97 (s, 1H), 8.11 (s, 1H), 8.02 - 7.92 (m, 2H), 7.76 - 7.62 (m, 2H), 7.05 (s, 1H), 6.92 - 6.74 (m, 2H), 5.70 - 5.00 (m, 1H), 4.73 - 4.59 (m, 2H), 4.56 (s, 2H), 4.20 - 4.04 (m, 1H), 3.90 - 3.80 (m, 1H), 3.72 - 3.47 (m, 3H), 3.33 - 3.07 (m, 2H), 3.07 - 2.81 (m, 4H), 2.81 - 2.58 (m, 6H), 2.58 - 2.50 (m, 1H), 2.25 - 2.05 (m, 3H), 2.05 - 1.86 (m, 2H), 1.72 - 1.50 (m, 9H).

LCMS m/z = 400.7 [M/2+1] ⁺.

### Example 81: preparation of trifluoroacetate of compound 81

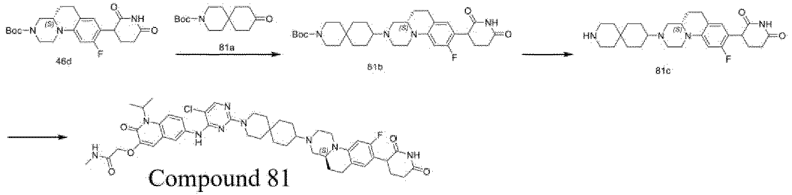

The trifluoroacetate of compound 81 was prepared using compounds 46d and 81a as the raw materials and referring to the synthesis method of example 79.

¹H NMR (400 MHz, DMSO-*d*₆) δ 10.79 (s, 1H), 8.93 (s, 1H), 8.07 (s, 1H), 8.04 - 7.90 (m, 2H), 7.69 (s, 2H), 7.00 (s, 1H), 6.93 - 6.74 (m, 2H), 5.60 - 5.10 (m, 1H), 4.61 - 4.51 (m, 2H), 4.18 - 4.05 (m, 1H), 3.92 - 3.80 (m, 1H), 3.73 - 3.52 (m, 6H), 3.35 - 3.07 (m, 3H), 3.07 - 2.95 (m, 1H), 2.95 - 2.82 (m, 1H), 2.82 - 2.58 (m, 6H), 2.57 - 2.50 (m, 1H), 2.24 - 2.05 (m, 1H), 2.05 - 1.80 (m, 6H), 1.80 - 1.47 (m, 11H), 1.40 - 1.29 (m, 2H), 1.27 - 1.08 (m, 2H).

LCMS m/z = 434.9 [M/2+1] ⁺.

### Example 82: preparation of trifluoroacetate of compound 82

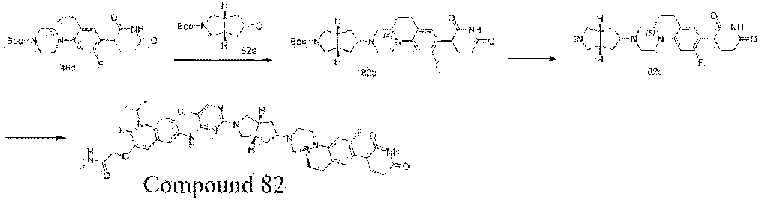

The trifluoroacetate of compound 82 was prepared using compounds 46d and 82a as the raw materials and referring to the synthesis method of example 79.

¹H NMR (400 MHz, DMSO-*d*₆) δ 10.78 (s, 1H), 8.95 (s, 1H), 8.16 - 8.11 (m, 1H), 8.09 (s, 1H), 8.00 - 7.90 (m, 1H), 7.79 (dd, 1H), 7.73 - 7.64 (m, 1H), 7.06 (s, 1H), 6.86 (d, 1H), 6.78 (d, 1H), 5.60 - 5.05 (m, 1H), 4.56 (s, 2H), 4.17 - 4.02 (m, 1H), 3.92 - 3.78 (m, 1H), 3.75 - 3.47 (m, 7H), 3.28 - 3.02 (m, 2H), 3.01 - 2.82 (m, 2H), 2.82 - 2.57 (m, 8H), 2.55 - 2.50 (m, 1H), 2.47 - 2.30 (m, 2H), 2.23 - 1.85 (m, 3H), 1.78 - 1.50 (m, 9H).

LCMS m/z = 413.8 [M/2+1] ⁺.

### Example 83: preparation of trifluoroacetate of compound 83

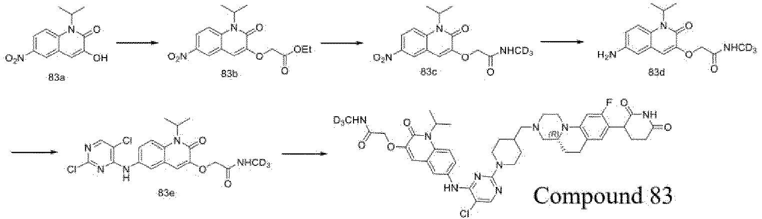

### Step 1: preparation of 83b

To a reaction flask were respectively added 83a (2.00 g, 8.06 mmol) (see WO 2022221673 for the synthesis method), 2-ethyl bromoacetate (1.62 g, 9.70 mmol), caesium carbonate (5.25 g, 16.11 mmol) and DMF (50 mL), and the mixture was reacted at room temperature for 3 h. The reaction system was poured into water (200 mL) and filtered. The filter cake was dried under reduced pressure to obtain crude 83b (2.40 g).

### Step 2: preparation of 83c

To a reaction flask were respectively added the above-mentioned crude 83b (2.40 g), lithium hydroxide (0.86 g, 35.9 mmol), methanol (36 mL) and water (9 mL), and the mixture was reacted at room temperature for 2 h. The reaction solution was poured into water (100 mL), adjusted to pH 1 with 1 mol/L hydrochloric acid, and filtered. The filter cake was dried under reduced pressure to obtain a crude (2.20 g). To a reaction flask were respectively added the above-mentioned crude (2.20 g), HATU (4.10 g, 10.78 mmol) and DMF (15 mL), and the mixture was stirred at room temperature for 2 min. Then deuterated methanamine hydrochloride (1.52 g, 21.55 mmol) and DIPEA (2.78 g, 21.51 mmol) were added, and the resulting mixture was reacted at room temperature for 16 h. The reaction system was poured into water (200 mL) and filtered. The filter cake was dried under reduced pressure to obtain crude 83c (2.30 g).

### Step 3: preparation of 83d

To a reaction flask were respectively added the above-mentioned crude 83c (2.30 g), 10% palladium on carbon (0.4 g), dichloromethane (50 mL) and methanol (50 mL), and the mixture was reacted at room temperature under the atmosphere of hydrogen balloon for 16 h. The reaction system was filtered over diatomaceous earth, and the filtrate was concentrated under reduced pressure to obtain crude 83d (2.00 g).

LCMS m/z = 293.2 [M+1] ⁺.

### Step 4: preparation of 83e

To a reaction flask were respectively added the above-mentioned crude 83d (2.00 g), 2,4,5-trichloropyrimidine (1.38 g, 7.52 mmol), TEA (1.38 g, 13.64 mmol) and DMF (25 mL), and the mixture was reacted at 70°C for 2 h. The reaction system was cooled to room temperature, poured into water (100 mL), and filtered. The filter cake was washed with water (20 mL), and dried under reduced pressure to obtain crude 83e (2.1 g).

LCMS m/z = 439.1 [M+1] ⁺.

### Step 5: preparation of trifluoroacetate of compound 83

To a reaction flask were respectively added the above-mentioned crude 44h (58 mg), DIPEA (180 mg, 1.39 mmol) and DMSO (3 mL). The above-mentioned crude 83e (60 mg) was added, and the resulting mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 83 (40 mg).

¹H NMR (400 MHz, CD₃OD) δ 8.06 (s, 1H), 8.03 (d, 1H), 7.80 (d, 1H), 7.69 (dd, 1H), 7.22 (s, 1H), 6.93 (d, 1H), 6.74 (d, 1H), 5.70 - 5.10 (m, 1H), 4.62 (s, 2H), 4.58 - 4.47 (m, 2H), 4.18 - 4.05 (m, 1H), 3.95 - 3.86 (m, 1H), 3.82 - 3.63 (m, 2H), 3.45 - 3.35 (m, 1H), 3.30 - 3.05 (m, 6H), 3.05 - 2.62 (m, 5H), 2.40 - 2.18 (m, 2H), 2.18 - 2.02 (m, 2H), 2.02 - 1.88 (m, 2H), 1.88 - 1.75 (m, 1H), 1.75 - 1.66 (m, 6H), 1.48 - 1.28 (m, 2H).

### Example 84: preparation of trifluoroacetate of compound 84

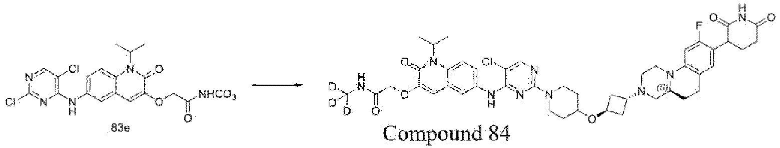

The trifluoroacetate of compound 84 was prepared by acidic preparative chromatography [water (containing 0.1% TFA)/acetonitrile] and lyophilization using 83e and 58c as the raw materials and referring to the synthesis method of example 58.

¹H NMR (400 MHz, DMSO-*d*₆) δ 10.79 (s, 1H), 8.91 (s, 1H), 8.06 (s, 1H), 8.02 - 7.95 (m, 1H), 7.91 (s, 1H), 7.74 - 7.62 (m, 2H), 7.02 (s, 1H), 6.87 (d, 1H), 6.80 (d, 1H), 5.70 - 5.10 (m, 1H), 4.54 (s, 2H), 4.38 - 4.25 (m, 1H), 4.20 - 4.05 (m, 3H), 3.95 - 3.75 (m, 2H), 3.68 - 3.42 (m, 3H), 3.35 - 3.15 (m, 3H), 3.05 - 2.85 (m, 2H), 2.84 - 2.50 (m, 7H), 2.38 - 2.21 (m, 2H), 2.21 - 2.05 (m, 1H), 2.05 - 1.90 (m, 2H), 1.90 - 1.77 (m, 2H), 1.70 - 1.52 (m, 7H), 1.47 - 1.34 (m, 2H).

LCMS m/z = 437.4 [M/2+1] ⁺.

### Example 85: preparation of trifluoroacetate of compound 85

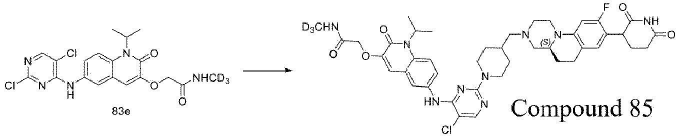

The trifluoroacetate of compound 85 was prepared by acidic preparative chromatography [water (containing 0.1% TFA)/acetonitrile] and lyophilization using 83e and 46g as the raw materials and referring to the synthesis method of example 83.

¹H NMR (400 MHz, CD₃OD) δ 8.05 (s, 1H), 7.97 (d, 1H), 7.79 (d, 1H), 7.66 (dd, 1H), 7.21 (s, 1H), 6.89 (d, 1H), 6.70 (d, 1H), 5.70 - 5.10 (m, 1H), 4.60 (s, 2H), 4.51 - 4.36 (m, 2H), 4.15 - 4.00 (m, 1H), 3.95 - 3.81 (m, 1H), 3.80 - 3.60 (m, 2H), 3.45 - 3.30 (m, 1H), 3.27 - 3.05 (m, 6H), 3.00 - 2.58 (m, 5H), 2.40 - 2.15 (m, 2H), 2.15 - 2.00 (m, 2H), 2.00 - 1.88 (m, 2H), 1.83 - 1.72 (m, 1H), 1.72 - 1.63 (m, 6H), 1.47 - 1.29 (m, 2H).

LCMS m/z = 409.3 [M/2+1] ⁺.

### Example 86: preparation of trifluoroacetate of compound 86

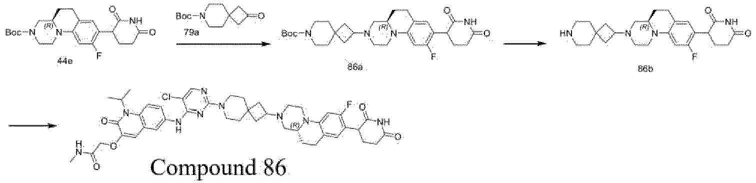

The trifluoroacetate of compound 86 was prepared using compounds 44e and 79a as the raw materials and referring to the synthesis method of example 79.

¹H NMR (400 MHz, DMSO-*d*₆) δ 10.78 (s, 1H), 8.96 (s, 1H), 8.07 (s, 1H), 8.02 - 7.88 (m, 2H), 7.76 - 7.64 (m, 2H), 7.06 (s, 1H), 6.93 - 6.75 (m, 2H), 5.60 - 5.10 (m, 1H), 4.55 (s, 2H), 4.22 - 4.08 (m, 1H), 3.93 - 3.72 (m, 2H), 3.72 - 3.40 (m, 6H), 3.33 - 3.16 (m, 1H), 3.06 - 2.82 (m, 2H), 2.82 - 2.58 (m, 7H), 2.57 - 2.51 (m, 1H), 2.32 - 1.86 (m, 7H), 1.72 - 1.48 (m, 11H).

LCMS m/z = 420.8 [M/2+1] ⁺.

### Example 87: preparation of trifluoroacetate of compound 87

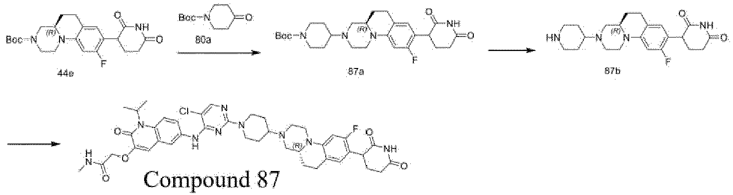

The trifluoroacetate of compound 87 was prepared using compounds 44e and 80a as the raw materials and referring to the synthesis method of example 79.

¹H NMR (400 MHz, DMSO-*d*₆) δ 10.79 (s, 1H), 9.00 (s, 1H), 8.11 (s, 1H), 8.02 - 7.92 (m, 2H), 7.76 - 7.62 (m, 2H), 7.05 (s, 1H), 6.92 - 6.74 (m, 2H), 5.60 - 5.00 (m, 1H), 4.73 - 4.59 (m, 2H), 4.56 (s, 2H), 4.20 - 4.04 (m, 1H), 3.90 - 3.80 (m, 1H), 3.72 - 3.47 (m, 3H), 3.33 - 3.07 (m, 2H), 3.07 - 2.81 (m, 4H), 2.81 - 2.58 (m, 6H), 2.58 - 2.50 (m, 1H), 2.25 - 2.05 (m, 3H), 2.05 - 1.86 (m, 2H), 1.72 - 1.50 (m, 9H).

LCMS m/z = 400.8 [M/2+1] ⁺.

### Example 88: preparation of trifluoroacetate of compound 88

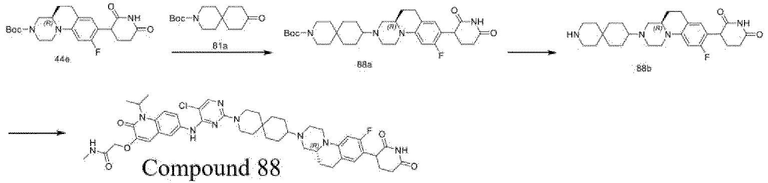

The trifluoroacetate of compound 88 was prepared using compounds 44e and 81a as the raw materials and referring to the synthesis method of example 79.

¹H NMR (400 MHz, DMSO-*d*₆) δ 10.79 (s, 1H), 9.02 (s, 1H), 8.08 (s, 1H), 8.04 - 7.90 (m, 2H), 7.74 - 7.64 (m, 2H), 7.01 (s, 1H), 6.93 - 6.74 (m, 2H), 5.60 - 5.10 (m, 1H), 4.61 - 4.51 (m, 2H), 4.18 - 4.05 (m, 1H), 3.92 - 3.80 (m, 1H), 3.73 - 3.52 (m, 6H), 3.35 - 3.07 (m, 3H), 3.07 - 2.95 (m, 1H), 2.95 - 2.82 (m, 1H), 2.82 - 2.58 (m, 6H), 2.57 - 2.50 (m, 1H), 2.24 - 2.05 (m, 1H), 2.05 - 1.80 (m, 6H), 1.80 - 1.47 (m, 11H), 1.40 - 1.29 (m, 2H), 1.27 - 1.08 (m, 2H).

LCMS m/z = 434.9 [M/2+1] ⁺.

### Example 89: preparation of trifluoroacetate of compound 89

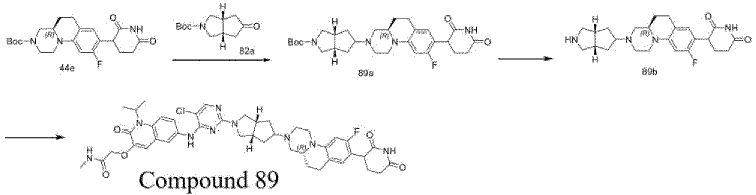

The trifluoroacetate of compound 89 was prepared using compounds 44e and 82a as the raw materials and referring to the synthesis method of example 79.

¹H NMR (400 MHz, DMSO-*d*₆) δ 10.78 (s, 1H), 9.00 (s, 1H), 8.16 - 8.07 (m, 2H), 8.01 - 7.90 (m, 1H), 7.85 - 7.75 (m, 1H), 7.73 - 7.64 (m, 1H), 7.06 (s, 1H), 6.86 (d, 1H), 6.78 (d, 1H), 5.60 - 5.10 (m, 1H), 4.56 (s, 2H), 4.17 - 4.02 (m, 1H), 3.92 - 3.78 (m, 1H), 3.75 - 3.47 (m, 7H), 3.28 - 3.02 (m, 2H), 3.01 - 2.82 (m, 2H), 2.82 - 2.57 (m, 8H), 2.55 - 2.50 (m, 1H), 2.47 - 2.30 (m, 2H), 2.23 - 1.85 (m, 3H), 1.78 - 1.50 (m, 9H).

LCMS m/z = 413.8 [M/2+1] ⁺.

### Example 90: preparation of trifluoroacetate of compound 90

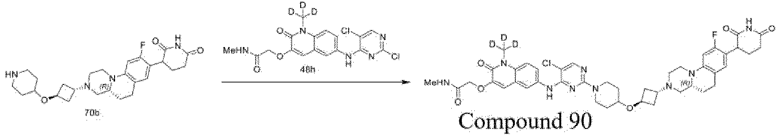

The trifluoroacetate of compound 90 was prepared by acidic preparative chromatography [water (containing 0.1% TFA)/acetonitrile] and lyophilization using compounds 70b and 48h as the raw materials and referring to the synthesis method of example 58.

¹H NMR (400 MHz, DMSO-*d*₆) δ 10.78 (s, 1H), 8.92 (s, 1H), 8.06 (s, 1H), 7.99 - 7.89 (m, 2H), 7.71 (dd, 1H), 7.46 (d, 1H), 7.11 (s, 1H), 6.93 - 6.75 (m, 2H), 4.58 (s, 2H), 4.37 - 4.25 (m, 1H), 4.18 - 4.02 (m, 3H), 3.95 - 3.80 (m, 2H), 3.64 - 3.44 (m, 3H), 3.34 - 3.15 (m, 3H), 3.04 - 2.85 (m, 2H), 2.82 - 2.50 (m, 10H), 2.37 - 2.20 (m, 2H), 2.20 - 2.05 (m, 1H), 2.05 - 1.77 (m, 4H), 1.72 - 1.52 (m, 1H), 1.50 - 1.32 (m, 2H).

LCMS m/z = 423.3 [M/2+1] ⁺.

### Example 91: preparation of trifluoroacetate of compound 91

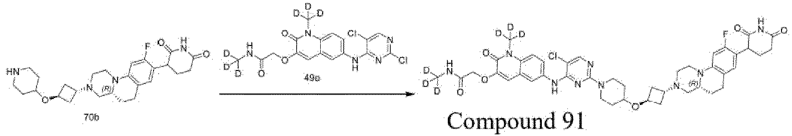

The trifluoroacetate of compound 91 was prepared by acidic preparative chromatography [water (containing 0.1% TFA)/acetonitrile] and lyophilization using compounds 70b and 49e as the raw materials and referring to the synthesis method of example 58.

¹H NMR (400 MHz, DMSO-*d*₆) δ 10.79 (s, 1H), 8.93 (s, 1H), 8.06 (s, 1H), 7.99 - 7.88 (m, 2H), 7.71 (dd, 1H), 7.47 (d, 1H), 7.11 (s, 1H), 6.93 - 6.75 (m, 2H), 4.58 (s, 2H), 4.38 - 4.23 (m, 1H), 4.20 - 4.01 (m, 3H), 3.97 - 3.78 (m, 2H), 3.65 - 3.43 (m, 3H), 3.34 - 3.15 (m, 3H), 3.05 - 2.86 (m, 2H), 2.83 - 2.50 (m, 7H), 2.38 - 2.21 (m, 2H), 2.21 - 2.05 (m, 1H), 2.05 - 1.76 (m, 4H), 1.70 - 1.52 (m, 1H), 1.48 - 1.32 (m, 2H).

LCMS m/z = 424.8 [M/2 +1] ⁺.

### Example 92: preparation of trifluoroacetate of compound 92

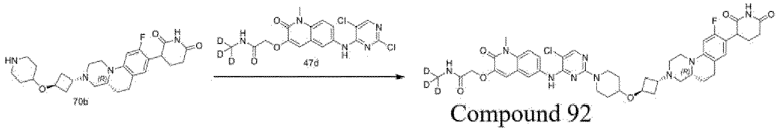

The trifluoroacetate of compound 92 was prepared by acidic preparative chromatography [water (containing 0.1% trifluoroacetic acid)/acetonitrile] and lyophilization using compounds 70b and 47d as the raw materials and referring to the synthesis method of example 58.

¹H NMR (400 MHz, DMSO-*d*₆) δ 10.78 (s, 1H), 8.92 (s, 1H), 8.06 (s, 1H), 7.98 - 7.89 (m, 2H), 7.72 (dd, 1H), 7.47 (d, 1H), 7.11 (s, 1H), 6.92 - 6.75 (m, 2H), 4.58 (s, 2H), 4.36 - 4.25 (m, 1H), 4.20 - 4.04 (m, 3H), 3.93 - 3.80 (m, 2H), 3.68 (s, 3H), 3.63 - 3.44 (m, 3H), 3.32 - 3.15 (m, 3H), 3.05 - 2.85 (m, 2H), 2.83 - 2.50 (m, 7H), 2.37 - 2.21 (m, 2H), 2.21 - 2.05 (m, 1H), 2.05 - 1.76 (m, 4H), 1.70 - 1.53 (m, 1H), 1.48 - 1.32 (m, 2H).

LCMS m/z = 845.3 [M+1] ⁺.

### Example 93: preparation of trifluoroacetate of compound 93

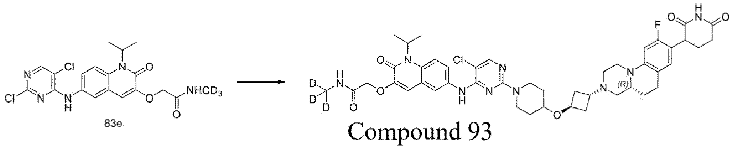

The trifluoroacetate of compound 93 was prepared by acidic preparative chromatography [water (containing 0.1% TFA)/acetonitrile] and lyophilization using compounds 83e and 70b as the raw materials and referring to the synthesis method of example 58.

¹H NMR (400 MHz, DMSO-*d*₆) δ 10.84 - 10.74 (m, 1H), 8.91 (s, 1H), 8.06 (s, 1H), 8.02 - 7.86 (m, 2H), 7.74 - 7.62 (m, 2H), 7.02 (s, 1H), 6.87 (d, 1H), 6.80 (d, 1H), 5.65 - 5.05 (m, 1H), 4.54 (s, 2H), 4.38 - 4.25 (m, 1H), 4.20 - 4.05 (m, 3H), 3.95 - 3.75 (m, 2H), 3.68 - 3.42 (m, 3H), 3.35 - 3.15 (m, 3H), 3.05 - 2.85 (m, 2H), 2.84 - 2.50 (m, 7H), 2.38 - 2.21 (m, 2H), 2.21 - 2.05 (m, 1H), 2.05 - 1.90 (m, 2H), 1.90 - 1.77 (m, 2H), 1.70 - 1.52 (m, 7H), 1.48 - 1.34 (m, 2H).

LCMS m/z = 437.2 [M/2+1] ⁺.

### Example 94: preparation of trifluoroacetate of compound 94

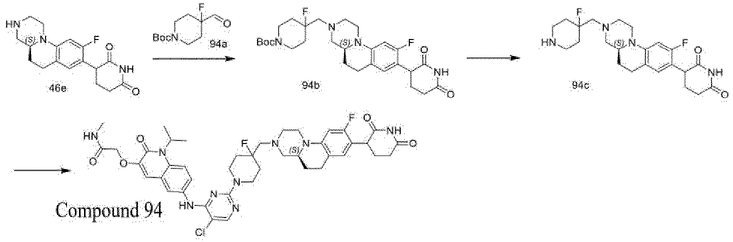

### Step 1: preparation of 94b

The above-mentioned crude 46e (0.15 g) was dissolved in 1,2-dichloroethane (10 mL). 94a (0.16 g, 0.69 mmol) (see CN 114591312 for the synthesis method) and acetic acid (1 mL) were added. The mixture was stirred at room temperature for 0.5 h, and then sodium triacetoxyborohydride (0.30 g, 1.42 mmol) was added. The resulting mixture was reacted at room temperature for 16 h. To the reaction solution was slowly added a saturated aqueous sodium bicarbonate solution (30 mL), and the mixture was extracted with dichloromethane (50 mL×2). The organic phase was washed with a saturated aqueous sodium chloride solution (50 mL), dried over anhydrous sodium sulphate, and concentrated under reduced pressure. The crude was separated and purified by silica gel column chromatography (dichloromethane:methanol (v/v) = 10:1) to obtain 94b (0.22 g, yield: 60%).

LCMS m/z = 533.3 [M+1]⁺.

### Step 2: preparation of 94c

94b (220 mg, 0.41 mmol) was dissolved in dichloromethane (4 mL). Trifluoroacetic acid (2 mL) was added, and the mixture was reacted at room temperature for 3 h. The reaction system was concentrated under reduced pressure, adjusted to pH 9 with a saturated aqueous sodium bicarbonate solution, and extracted with dichloromethane (50 mL×2). The organic phase was dried over anhydrous sodium sulphate, and concentrated under reduced pressure to obtain crude 94c (140 mg).

### Step 3: preparation of trifluoroacetate of compound 94

To a reaction flask were respectively added the above-mentioned crude 94c (61 mg), DIPEA (180 mg, 1.39 mmol) and DMSO (3 mL). 42A (60 mg, 0.14 mmol) was added, and the mixture was reacted at 100°C for 7 h. The reaction system was cooled to room temperature. The crude was subjected to Pre-HPLC (instrument and preparative column: using Waters 2767 preparative liquid phase chromatographic instrument, preparative column model: SunFire@Prep C18, 5 µm, inner diameter × length = 19 mm×250 mm). Preparation method: Preparation method: the crude in DMSO solution was filtered with a 0.45 µm filter membrane to prepare a sample liquid. Mobile phase system: water (containing 0.1% TFA)/acetonitrile. Gradient elution method: gradient elution with acetonitrile from 10% to 50% (elution time: 15 min). Lyophilization was performed to obtain the trifluoroacetate of compound 94 (25 mg).

¹H NMR (400 MHz, CD₃OD) δ 8.05 (s, 1H), 7.97 (d, 1H), 7.77 (d, 1H), 7.67 (dd, 1H), 7.20 (s, 1H), 6.88 (d, 1H), 6.68 (d, 1H), 5.75 - 5.10 (m, 1H), 4.60 (s, 2H), 4.48 - 4.34 (m, 2H), 4.07 - 3.95 (m, 1H), 3.93 - 3.82 (m, 1H), 3.80 - 3.69 (m, 1H), 3.68 - 3.58 (m, 1H), 3.58 - 3.45 (m, 2H), 3.45 - 3.31 (m, 3H), 3.30 - 3.23 (m, 1H), 3.23 - 3.11 (m, 1H), 3.09 - 2.97 (m, 1H), 2.90 - 2.77 (m, 4H), 2.77 - 2.57 (m, 3H), 2.32 - 1.60 (m, 14H).

LCMS m/z = 832.7 [M+1] ⁺.

### Example 95: preparation of compound 95

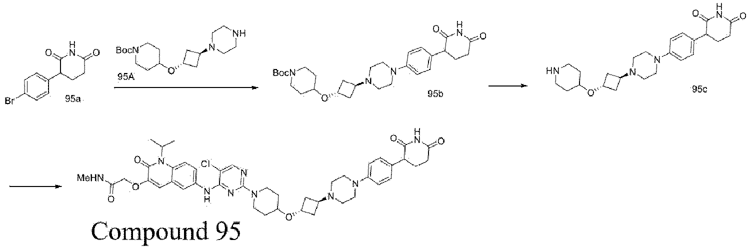

### Step 1: preparation of 95b

Under nitrogen atmosphere, to a reaction flask were respectively added 95a (0.50 g, 1.86 mmol) (see WO 2022235715 for the synthesis method), 95A (0.76 g, 2.24 mmol) (see WO 2022221673 for the synthesis method), RuPhos Pd G3 (0.31 g, 0.37 mmol) and 2-dicyclohexylphosphino-2',6'-diisopropoxy -1,1'-biphenyl (0.17 g, 0.36 mmol). Toluene (15 mL) and a solution of lithium bis(trimethylsilyl)amide in tetrahydrofuran (11.16 mL, 11.16 mmol, 1 mol/L) were added, and the mixture was reacted at 80°C for 1 h. The reaction solution was cooled to 0°C. The reaction was quenched by dropwise adding a saturated aqueous ammonium chloride solution (20 mL) and extracted with ethyl acetate (50 mL×3). The organic phase was dried over anhydrous sodium sulphate, and concentrated under reduced pressure. The crude was separated and purified by silica gel column chromatography (dichloromethane/methanol (v/v) = 10:1) to obtain 95b (0.45 g, yield: 46%).

LCMS m/z = 527.7 [M+1] ⁺.

### Step 2: preparation of trifluoroacetate of 95c

To a reaction flask were added 95b (0.30 g, 0.57 mmol), trifluoroacetic acid (1.5 mL) and dichloromethane (2 mL), and the mixture was reacted at room temperature for 2 h. The reaction system was concentrated under reduced pressure to obtain crude trifluoroacetate of 95c (0.30 g).

### Step 3: preparation of compound 95

To a reaction flask were respectively added the above-mentioned crude trifluoroacetate of 95c (0.30 g), sodium bicarbonate (0.48 g, 5.71 mmol) and dimethyl sulfoxide (5 mL). The mixture was stirred at room temperature for 0.5 h. 42A (0.30 g, 0.69 mmol) and TEA (0.48 g, 4.74 mmol) were added, and the mixture was reacted at 100°C for 16 h. The reaction system was cooled to room temperature. The reaction solution was subjected to Pre-HPLC (instrument and preparative column: using SHIMADZU LC-20AP preparative liquid phase chromatographic instrument, preparative column model: Phenomenex C18, 5 µm, inner diameter × length = 30 mm×150 mm). Preparation method: the crude in acetonitrile solution was filtered with a 0.45 µm filter membrane to prepare a sample liquid. Mobile phase system: ammonium bicarbonate in water (10 mmol/L)/acetonitrile. Gradient elution method: gradient elution with acetonitrile from 35% to 65% (elution time: 10 min). Lyophilization was performed to obtain compound 95 (89 mg, yield: 16%).

¹H NMR (400 MHz, DMSO-*d*₆) δ 10.74 (s, 1H), 8.80 (s, 1H), 8.04 (s, 1H), 7.98 - 7.86 (m, 2H), 7.69 (s, 2H), 7.08 - 6.98 (m, 3H), 6.93 - 6.84 (m, 2H), 5.60 - 5.10 (m, 1H), 4.54 (s, 2H), 4.23 - 4.15 (m, 1H), 4.15 - 4.05 (m, 2H), 3.78 - 3.67 (m, 1H), 3.60 - 3.47 (m, 1H), 3.30 - 3.17 (m, 2H), 3.16 - 3.05 (m, 4H), 2.85 - 2.75 (m, 1H), 2.72 - 2.57 (m, 4H), 2.50 - 2.35 (m, 5H), 2.25 - 2.06 (m, 3H), 2.05 - 1.92 (m, 3H), 1.88 - 1.77 (m, 2H), 1.62 - 1.52 (m, 6H), 1.45 - 1.30 (m, 2H).

LCMS m/z = 826.2 [M+1] ⁺.

### Example 96: preparation of compound 96

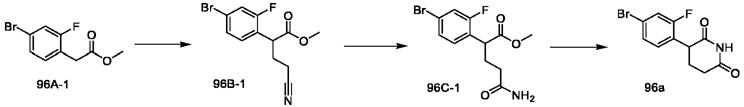

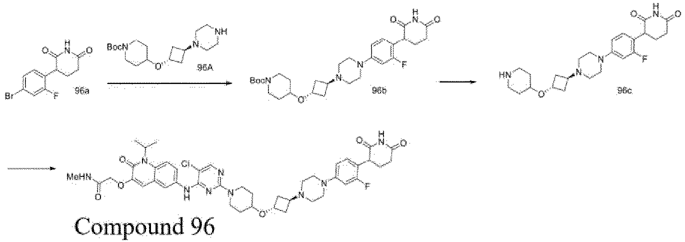

### Step 1: preparation of 96B-1

96A-1 (14.8 g, 59.90 mmol) was dissolved in toluene (100 mL). Acrylonitrile (4.77 g, 89.9 mmol) and a solution of 40% benzyltrimethylammonium hydroxide in methanol (1.18 mL) were added dropwise at room temperature. The mixture was reacted at room temperature for 16 h. To the reaction system were added ethyl acetate (200 mL) and water (200 mL). The aqueous phase was extracted with ethyl acetate (100 mL), and the organic phase was dried over anhydrous sodium sulphate, and concentrated under reduced pressure. The crude was separated and purified by silica gel column chromatography (petroleum ether/ethyl acetate (v/v) = 4:1-2:1) to obtain 96B-1 (14.2 g, yield: 79%).

LCMS m/z = 300.2 [M +1]⁺.

### Step 2: preparation of 96C-1

96B-1 (14.2 g, 47.46 mmol) was dissolved in toluene (100 mL). Acetaldoxime (8.38 g, 141.87 mmol) and indium chloride (5.23 g, 23.65 mmol) were added, and the mixture was reacted at 130°C for 2 h. The reaction solution was cooled to room temperature, and concentrated under reduced pressure. The crude was separated and purified by silica gel column chromatography (dichloromethane/methanol (v/v) = 1:0-10:1) to obtain 96C-1 (14 g, yield: 93%).

LCMS m/z = 318.1 [M+1]⁺.

### Step 3: preparation of 96a

96C-1 (14 g, 44.15 mmol) was dissolved in acetonitrile (120 mL). A solution of 40% benzyltrimethylammonium hydroxide in methanol (34.72 mL) was added at room temperature, and the mixture was reacted at 60°C for 1 h. The reaction solution was cooled to room temperature, and concentrated under reduced pressure. The crude was separated and purified by silica gel column chromatography (dichloromethane/methanol (v/v) = 1:0-10:1) to obtain 96a (4 g, yield: 32%).

LCMS m/z = 286.2 [M +1]⁺.

Compound 96 was prepared using compounds 96a and 96A (see WO 2022221673 for the synthesis method) as the starting materials and referring to the synthesis method of example 95.

¹H NMR (400 MHz, DMSO-*d*₆) δ 10.77 (s, 1H), 8.80 (s, 1H), 8.04 (s, 1H), 7.98 - 7.88 (m, 2H), 7.68 (s, 2H), 7.14 - 7.00 (m, 2H), 6.77 - 6.66 (m, 2H), 5.60 - 5.10 (m, 1H), 4.54 (s, 2H), 4.25 - 4.05 (m, 3H), 3.94 - 3.82 (m, 1H), 3.60 - 3.47 (m, 1H), 3.30 - 3.18 (m, 2H), 3.18 - 3.09 (m, 4H), 2.84 - 2.76 (m, 1H), 2.72 - 2.64 (m, 4H), 2.44 - 2.30 (m, 5H), 2.24 - 2.10 (m, 3H), 2.05 - 1.90 (m, 3H), 1.87 - 1.75 (m, 2H), 1.63 - 1.52 (m, 6H), 1.45 - 1.30 (m, 2H).

LCMS m/z = 844.2 [M+1] ⁺.

### Example 97: preparation of trifluoroacetate of compound 97

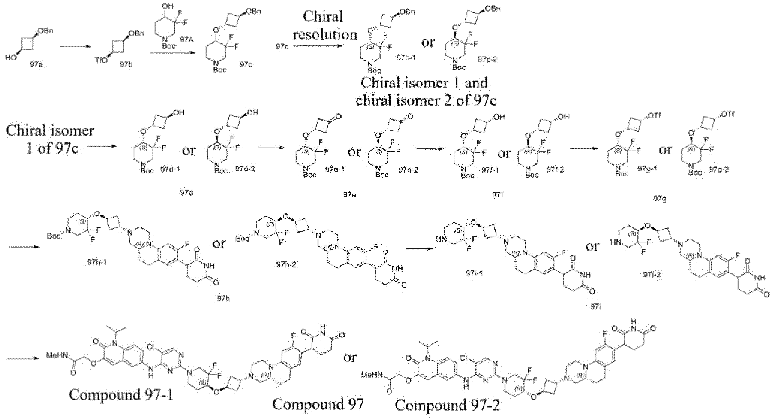

### Step 1: preparation of 97b

Under nitrogen atmosphere, to a reaction flask were respectively added 97a (15 g, 84.16 mmol) (see WO 2022109580 for the synthesis method), TEA (25.55 g, 252.5 mmol) and dichloromethane (200 mL). Trifluoromethanesulfonic anhydride (35.62 g, 126.25 mmol) was slowly added dropwise at 0°C, and the mixture was reacted at room temperature for 1 h. The reaction system was concentrated under reduced pressure, and the crude was separated and purified by silica gel column chromatography (petroleum ether/ethyl acetate (v/v) = 20:1) to obtain 97b (8.60 g, yield: 33%).

### Step 2: preparation of 97c

Under nitrogen atmosphere, to a reaction flask were respectively added 97A (3.00 g, 12.64 mmol) and DMF (20 mL). 60% sodium hydride (1.00 g) was added at 0°C. The mixture was stirred at room temperature for 1 h. 97b (4.71 g, 15.18 mmol) was added at 0°C, and the resulting mixture was reacted at room temperature for 2 h. To the reaction system was added ice water (50 mL), and the mixture was extracted with ethyl acetate (50 mL×3). The organic phase was dried over anhydrous sodium sulphate, and concentrated under reduced pressure. The crude was separated and purified by silica gel column chromatography (petroleum ether/ethyl acetate (v/v) = 3:1) to obtain crude 97c (5.00 g).

### Step 3: preparation of chiral isomer 1 and chiral isomer 2 of 97c

The above-mentioned crude 97c was subjected to chiral resolution, and the preparation conditions were as follows:
1. Instrument: Waters 150 ap; chromatographic column: ChiralPak IC 19×250 5 µm.
2. Preparative chromatography conditions: a. the mobile phase consists of systems A and B: mobile phase A: CO₂; mobile phase B: isopropanol; b. isocratic elution, mobile phase B content: 10%; c. flow rate: 40 mL/min.

Retention time: chiral isomer 1: 5.0 min, chiral isomer 2: 5.95 min.

Chiral isomer 1 and chiral isomer 2 of compound 97c are respectively one of the isomers of structure 97c-1 or 97c-2.

### Step 4: preparation of 97d

To an autoclave were respectively added chiral isomer 1 of 97c (1.70 g, 4.28 mmol), 10% palladium hydroxide on carbon (1.70 g), 10% palladium on carbon (1.70 g) and methanol (10 mL). The mixture was subjected to hydrogen replacement three times and the pressure was maintained at 2 MPa. The resulting mixture was reacted at 70°C for 16 h. The reaction system was cooled to room temperature and filtered over diatomaceous earth. The filtrate was concentrated under reduced pressure, and the crude was separated and purified by silica gel column chromatography (petroleum ether/ethyl acetate (v/v) = 1:0-1:1) to obtain 97d (1.20 g, yield: 91%).

Compound 97d is one of the isomers of structure 97d-1 or 97d-2.

### Step 5: preparation of 97e

To a reaction flask were respectively added 97d (1.20 g, 3.90 mmol), Dess-Martin oxidant (6.00 g, 14.15 mmol) and dichloromethane (20 mL), and the mixture was reacted at room temperature for 0.5 h. The reaction system was concentrated under reduced pressure, and the crude was separated and purified by silica gel column chromatography (petroleum ether/ethyl acetate (v/v) = 1:0-2:1) to obtain 97e (1.10 g, yield: 92%).

Compound 97e is one of the isomers of structure 97e-1 or 97e-2.

### Step 6: preparation of 97f

To a reaction flask were respectively added 97e (1.10 g, 3.60 mmol) and methanol (10 mL). Sodium borohydride (0.14 g, 3.70 mmol) was added at 0°C, and the mixture was reacted at 0°C for 0.5 h. To the reaction system was added dropwise a saturated aqueous ammonium chloride solution (20 mL), and the mixture was extracted with ethyl acetate (30 mL×3). The organic phase was dried over anhydrous sodium sulphate, and concentrated under reduced pressure. The crude was separated and purified by silica gel column chromatography (petroleum ether/ethyl acetate (v/v) = 1:0-1:1) to obtain 97f (0.92 g, yield: 83%).

Compound 97f is one of the isomers of structure 97f-1 or 97f-2.

### Step 7: preparation of 97g

Under nitrogen atmosphere, to a reaction flask were respectively added 97f (0.50 g, 1.63 mmol), TEA (0.82 g, 8.10 mmol) and dichloromethane (10 mL). Trifluoromethanesulfonic anhydride (0.69 g, 2.45 mmol) was slowly added dropwise at 0°C, and the mixture was reacted at room temperature for 1 h. The reaction system was concentrated under reduced pressure to obtain crude 97g (0.62 g).

Compound 97g is one of the isomers of structure 97g-1 or 97g-2.

### Step 8: preparation of 97h

To a reaction flask were respectively added the above-mentioned crude 97g (0.31 g), TEA (0.32 g, 3.16 mmol), the above-mentioned crude 44f (0.25 g) and acetonitrile (10 mL), and the mixture was reacted at room temperature for 16 h. The reaction system was concentrated under reduced pressure, and the crude was separated and purified by silica gel column chromatography (dichloromethane/methanol (v/v) = 98:2) to obtain 97h (0.14 g, two-step yield calculated from compound 97f: 28%).
LCMS m/z = 607.9 [M+1]+

Compound 97h is one of the isomers of structure 97h-1 or 97h-2.

### Step 9: preparation of trifluoroacetate of 97i

To a reaction flask were respectively added compound 97h (0.14 g, 0.23 mmol), trifluoroacetic acid (1.53 g, 13.42 mmol) and dichloromethane (2 mL), and the mixture was reacted at room temperature for 2 h. The reaction system was concentrated under reduced pressure to obtain crude trifluoroacetate of 97i (0.15 g).

Compound 97i is one of the isomers of structure 97i-1 or 97i-2.

### Step 10: preparation of trifluoroacetate of compound 97

Under nitrogen atmosphere, to a reaction flask were respectively added the above-mentioned crude trifluoroacetate of 97i (0.15 g), sodium bicarbonate (0.097 g, 1.15 mmol) and dimethyl sulfoxide (5 mL). The mixture was stirred at room temperature for 0.5 h. 42A (0.10 g, 0.23 mmol) and TEA (0.12 g, 1.19 mmol) were added, and the mixture was reacted at 100°C for 16 h. The reaction system was cooled to room temperature. The reaction solution was subjected to Pre-HPLC (instrument and preparative column: using SHIMADZU LC-20AP preparative liquid phase chromatographic instrument, preparative column model: Phenomenex C18). Preparation method: the crude in acetonitrile solution was filtered with a 0.45 µm filter membrane to prepare a sample liquid. Mobile phase system: water (containing 0.1% trifluoroacetic acid)/acetonitrile. Gradient elution method: gradient elution with acetonitrile from 20% to 40% (elution time: 16 min). Lyophilization was performed to obtain the trifluoroacetate of compound 97 (54 mg).

¹H NMR (400 MHz, CD₃OD) δ 8.17 - 8.11 (m, 1H), 8.02 (s, 1H), 7.75 (d, 1H), 7.58 (dd, 1H), 7.17 (s, 1H), 6.90 (d, 1H), 6.71 (d, 1H), 5.70 - 5.10 (m, 1H), 4.60 - 4.53 (m, 2H), 4.53 - 4.44 (m, 1H), 4.34 - 4.06 (m, 2H), 4.06 - 3.79 (m, 5H), 3.76 - 3.50 (m, 3H), 3.32 - 3.20 (m, 1H), 3.16 - 2.98 (m, 2H), 2.93 - 2.45 (m, 12H), 2.33 - 2.15 (m, 1H), 2.15 - 1.95 (m, 3H), 1.94 - 1.73 (m, 2H), 1.73 - 1.65 (m, 6H).

LCMS m/z = 906.2 [M+1]⁺.

Compound 97 is one of the isomers of compound 97-1 or 97-2.

### Example 98: preparation of trifluoroacetate of compound 98

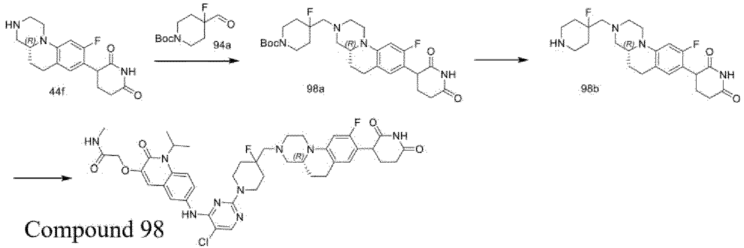

The trifluoroacetate of compound 98 was prepared using compounds 44f and 94a as the starting materials and referring to the synthesis method of example 94.

¹H NMR (400 MHz, CD₃OD) δ 8.07 (s, 1H), 7.97 - 7.91 (m, 1H), 7.78 (d, 1H), 7.67 (dd, 1H), 7.21 (s, 1H), 6.88 (d, 1H), 6.68 (d, 1H), 5.75 - 5.10 (m, 1H), 4.60 (s, 2H), 4.48 - 4.29 (m, 2H), 4.08 - 3.95 (m, 1H), 3.93 - 3.82 (m, 1H), 3.82 - 3.71 (m, 1H), 3.71 - 3.61 (m, 1H), 3.61 - 3.47 (m, 2H), 3.47 - 3.31 (m, 3H), 3.31 - 3.23 (m, 1H), 3.23 - 3.11 (m, 1H), 3.10 - 2.97 (m, 1H), 2.90 - 2.77 (m, 4H), 2.77 - 2.57 (m, 3H), 2.32 - 1.60 (m, 14H).

LCMS m/z = 832.3 [M+1] ⁺.

### Example 99: preparation of trifluoroacetate of compound 99

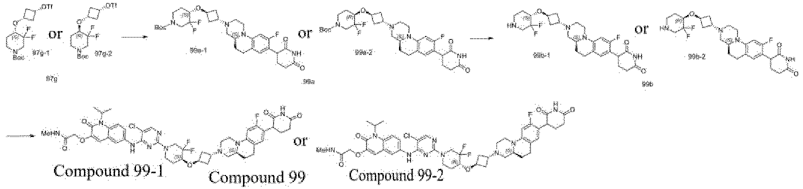

The trifluoroacetate of compound 99 was prepared using compounds 97g and 46e as the starting materials and referring to the synthesis method of example 97. ¹ H NMR (400 MHz, CD₃OD) δ 8.14 (d, 1H), 8.02 (s, 1H), 7.80 - 7.70 (m, 1H), 7.57 (dd, 1H), 7.16 (s, 1H), 6.90 (d, 1H), 6.70 (d, 1H), 5.70 - 5.10 (m, 1H), 4.61 - 4.52 (m, 2H), 4.52 - 4.44 (m, 1H), 4.34 - 4.06 (m, 2H), 4.06 - 3.79 (m, 5H), 3.76 - 3.50 (m, 3H), 3.32 - 3.20 (m, 1H), 3.16 - 2.98 (m, 2H), 2.93 - 2.45 (m, 12H), 2.33 - 2.15 (m, 1H), 2.15 - 1.95 (m, 3H), 1.94 - 1.73 (m, 2H), 1.73 - 1.65 (m, 6H).

LCMS m/z = 906.3 [M+1]⁺.

Compound 99 is one of the isomers of compound 99-1 or 99-2.

### Example 100: preparation of trifluoroacetate of compound 100

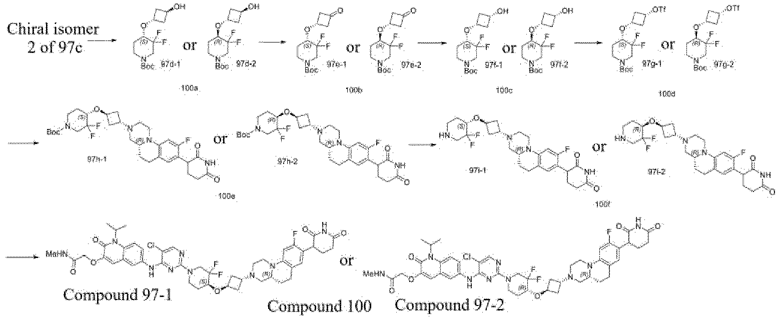

The trifluoroacetate of compound 100 was prepared using chiral isomer 2 of 97c as the starting material and referring to the synthesis method of example 97.

¹H NMR (400 MHz, CD₃OD) δ 8.18 - 8.08 (m, 1H), 8.06 - 8.00 (m, 1H), 7.75 (d, 1H), 7.63 - 7.53 (m, 1H), 7.17 (s, 1H), 6.90 (d, 1H), 6.70 (d, 1H), 5.75 - 5.10 (m, 1H), 4.60 - 4.53 (m, 2H), 4.53 - 4.44 (m, 1H), 4.34 - 4.06 (m, 2H), 4.06 - 3.79 (m, 5H), 3.76 - 3.50 (m, 3H), 3.32 - 3.20 (m, 1H), 3.16 - 2.98 (m, 2H), 2.93 - 2.45 (m, 12H), 2.33 - 2.15 (m, 1H), 2.15 - 1.95 (m, 3H), 1.94 - 1.73 (m, 2H), 1.73 - 1.63 (m, 6H).

LCMS m/z = 453.8 [M/2+1]⁺.

Compound 100 is one of the isomers of compound 97-1 or 97-2.

### Example 101: preparation of trifluoroacetate of compound 101

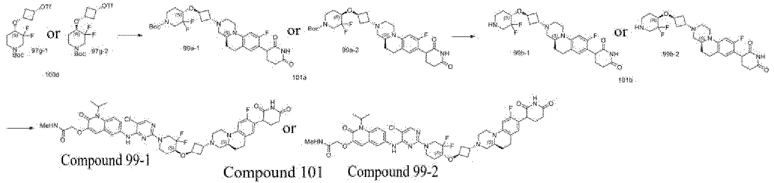

The trifluoroacetate of compound 101 was prepared using compounds 100d and 46e as the starting materials and referring to the synthesis method of example 99.

¹H NMR (400 MHz, CD₃OD) δ 8.13 (d, 1H), 8.03 (s, 1H), 7.75 (d, 1H), 7.58 (dd, 1H), 7.17 (s, 1H), 6.90 (d, 1H), 6.71 (d, 1H), 5.70 - 5.10 (m, 1H), 4.60 - 4.53 (m, 2H), 4.53 - 4.44 (m, 1H), 4.34 - 4.06 (m, 2H), 4.06 - 3.79 (m, 5H), 3.76 - 3.50 (m, 3H), 3.32 - 3.20 (m, 1H), 3.16 - 2.98 (m, 2H), 2.93 - 2.45 (m, 12H), 2.33 - 2.15 (m, 1H), 2.15 - 1.95 (m, 3H), 1.94 - 1.73 (m, 2H), 1.73 - 1.65 (m, 6H).

LCMS m/z = 454.1 [M/2+1] ⁺

Compound 101 is one of the isomers of compound 99-1 or 99-2.

### Example 102: preparation of trifluoroacetate of compound 102

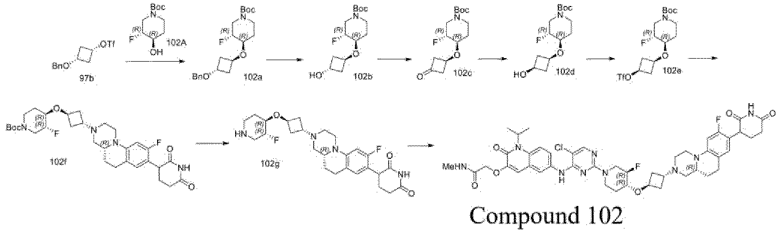

### Step 1: preparation of 102a

Under nitrogen atmosphere, to a reaction flask were respectively added 102A (2.54 g, 11.58 mmol) and DMF (20 mL), and 60% sodium hydride (1.00 g) was added at 0°C. The mixture was stirred at room temperature for 1 h. 97b (3.00 g, 9.67 mmol) was added at 0°C, and the resulting mixture was reacted at room temperature for 2 h. To the reaction system was added dropwise ice water (50 mL), and the mixture was extracted with ethyl acetate (50 mL×3). The organic phase was dried over anhydrous sodium sulphate, and concentrated under reduced pressure. The crude was separated and purified by silica gel column chromatography (petroleum ether/ethyl acetate (v/v) = 3:1) to obtain 102a (2.20 g, yield: 60%).

### Step 2: preparation of 102b

To an autoclave were respectively added 102a (2.20 g, 5.80 mmol), 10% palladium hydroxide on carbon (2.20 g), 10% palladium on carbon (2.20 g) and methanol (10 mL). The mixture was subjected to hydrogen replacement three times and the pressure was maintained at 2 MPa. The resulting mixture was reacted at 70°C for 16 h. The reaction system was cooled to room temperature and filtered over diatomaceous earth. The filtrate was concentrated under reduced pressure, and the crude was separated and purified by silica gel column chromatography (petroleum ether/ethyl acetate (v/v) = 1:0-1:1) to obtain 102b (1.40 g, yield: 83%).

### Step 3: preparation of 102c

To a reaction flask were respectively added 102b (1.40 g, 4.84 mmol), Dess-Martin oxidant (3.70 g, 8.72 mmol) and dichloromethane (20 mL), and the mixture was reacted at room temperature for 0.5 h. The reaction system was concentrated under reduced pressure, and the crude was separated and purified by silica gel column chromatography (petroleum ether/ethyl acetate (v/v) = 1:0-2:1) to obtain 102c (1.30 g, yield: 93%).

### Step 4: preparation of 102d

To a reaction flask were respectively added 102c (1.30 g, 4.52 mmol) and methanol (10 mL). Sodium borohydride (0.17 g, 4.49 mmol) was added at 0°C, and the mixture was reacted at 0°C for 0.5 h. To the reaction system was added dropwise a saturated aqueous ammonium chloride solution (20 mL), and the mixture was extracted with ethyl acetate (30 mL×3). The organic phase was dried over anhydrous sodium sulphate, and concentrated under reduced pressure. The crude was separated and purified by silica gel column chromatography (petroleum ether/ethyl acetate (v/v) = 1:0-1:1) to obtain 102d (0.92 g, yield: 71%).

### Step 5: preparation of 102e

Under nitrogen atmosphere, to a reaction flask were respectively added 102d (0.40 g, 1.38 mmol), TEA (0.42 g, 4.15 mmol) and dichloromethane (10 mL). Trifluoromethanesulfonic anhydride (0.58 g, 2.06 mmol) was slowly added dropwise at 0°C, and the mixture was reacted at room temperature for 1 h. The reaction system was concentrated under reduced pressure to obtain crude 102e (0.60 g).

### Step 6: preparation of 102f

To a reaction flask were respectively added the above-mentioned crude 102e (0.30 g), TEA (0.35 g, 3.46 mmol), the above-mentioned crude 44f (0.24 g) and acetonitrile (10 mL), and the mixture was reacted at room temperature for 16 h. The reaction system was concentrated under reduced pressure, and the crude was separated and purified by silica gel column chromatography (dichloromethane/methanol (v/v) = 98:2) to obtain 102f (0.10 g, two-step yield calculated from compound 102d: 25%).

LCMS m/z = 589.3 [M+1]⁺.

### Step 7: preparation of trifluoroacetate of 102g

To a reaction flask were respectively added 102f (0.10 g, 0.17 mmol), trifluoroacetic acid (1.53 g, 13.42 mmol) and dichloromethane (2 mL), and the mixture was reacted at room temperature for 2 h. The reaction system was concentrated under reduced pressure to obtain crude trifluoroacetate of 102g (0.10 g).

### Step 8: preparation of trifluoroacetate of compound 102

Under nitrogen atmosphere, to a reaction flask were respectively added the above-mentioned crude trifluoroacetate of 102g (0.10 g), sodium bicarbonate (0.071 g, 0.85 mmol) and dimethyl sulfoxide (5 mL). The mixture was stirred at room temperature for 0.5 h. 42A (0.074 g, 0.17 mmol) and TEA (0.086 g, 0.85 mmol) were added, and the mixture was reacted at 100°C for 16 h. The reaction system was cooled to room temperature. The reaction solution was subjected to Pre-HPLC (instrument and preparative column: using SHIMADZU LC-20AP preparative liquid phase chromatographic instrument, preparative column model: Phenomenex C18). Preparation method: the crude in acetonitrile solution was filtered with a 0.45 µm filter membrane to prepare a sample liquid. Mobile phase system: water (containing 0.1% trifluoroacetic acid)/acetonitrile. Gradient elution method: gradient elution with acetonitrile from 16% to 46% (elution time: 15 min). Lyophilization was performed to obtain the trifluoroacetate of compound 102 (90 mg).

¹H NMR (400 MHz, CD₃OD) δ 8.13 (d, 1H), 8.02 (s, 1H), 7.76 (d, 1H), 7.59 (dd, 1H), 7.20 (s, 1H), 6.90 (d, 1H), 6.71 (d, 1H), 5.70 - 5.10 (m, 1H), 4.66 - 4.34 (m, 5H), 4.24 - 4.07 (m, 2H), 4.05 - 3.82 (m, 2H), 3.77 - 3.50 (m, 3H), 3.47 - 3.31 (m, 2H), 3.30 - 3.19 (m, 1H), 3.13 - 3.00 (m, 2H), 2.92 - 2.42 (m, 12H), 2.32 - 2.15 (m, 1H), 2.15 - 2.00 (m, 3H), 1.88 - 1.55 (m, 8H).

LCMS m/z = 444.8 [M/2+1]⁺.

### Example 103: preparation of trifluoroacetate of compound 103

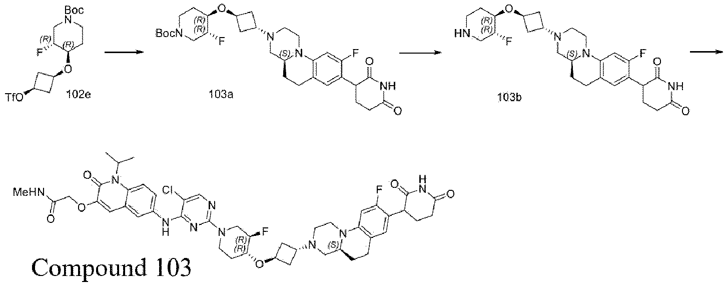

The trifluoroacetate of compound 103 was prepared using compounds 102e and 46e as the starting materials and referring to the synthesis method of example 102.

¹H NMR (400 MHz, CD₃OD) δ 8.11 (d, 1H), 8.03 (s, 1H), 7.77 (d, 1H), 7.60 (dd, 1H), 7.21 (s, 1H), 6.90 (d, 1H), 6.70 (d, 1H), 5.70 - 5.10 (m, 1H), 4.67 - 4.30 (m, 5H), 4.22 - 4.04 (m, 2H), 4.04 - 3.82 (m, 2H), 3.77 - 3.53 (m, 3H), 3.52 - 3.31 (m, 2H), 3.30 - 3.21 (m, 1H), 3.14 - 3.00 (m, 2H), 2.94 - 2.40 (m, 12H), 2.32 - 2.15 (m, 1H), 2.15 - 2.00 (m, 3H), 1.88 - 1.55 (m, 8H).

LCMS m/z = 444.8 [M/2+1]⁺.

### Example 104: preparation of trifluoroacetate of compound 104

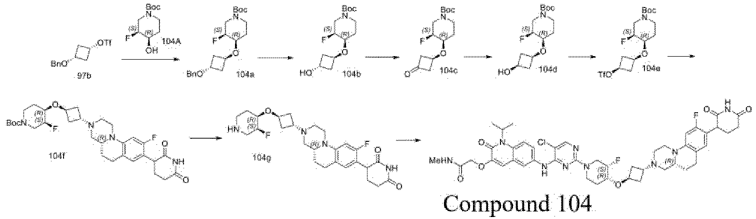

The trifluoroacetate of compound 104 was prepared using compounds 97b and 104A as the raw materials and referring to the synthesis method of example 102.

¹H NMR (400 MHz, CD₃OD) δ 8.13 (d, 1H), 8.07 - 8.03 (m, 1H), 7.79 (d, 1H), 7.63 (dd, 1H), 7.21 (s, 1H), 6.92 (d, 1H), 6.73 (d, 1H), 5.70 - 5.15 (m, 1H), 4.98 - 4.75 (m, 1H), 4.60 (s, 2H), 4.52 - 4.33 (m, 2H), 4.24 - 4.07 (m, 2H), 4.07 - 3.95 (m, 1H), 3.95 - 3.73 (m, 2H), 3.72 - 3.52 (m, 3H), 3.52 - 3.39 (m, 1H), 3.33 - 3.24 (m, 1H), 3.16 - 3.02 (m, 2H), 2.92 - 2.62 (m, 10H), 2.62 - 2.45 (m, 2H), 2.33 - 2.17 (m, 1H), 2.17 - 2.04 (m, 2H), 2.02 - 1.75 (m, 3H), 1.75 - 1.67 (m, 6H).

LCMS m/z = 888.3 [M+1] ⁺.

### Example 105: preparation of trifluoroacetate of compound 105

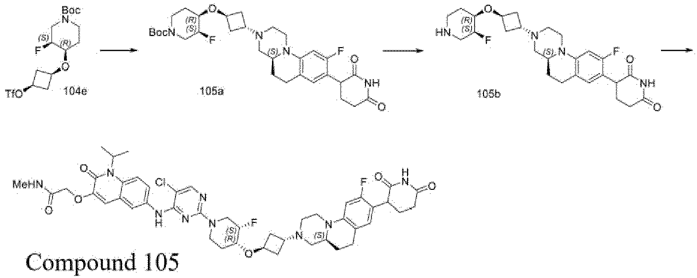

The trifluoroacetate of compound 105 was prepared using compounds 104e and 46e as the starting materials and referring to the synthesis method of example 104.

¹H NMR (400 MHz, CD₃OD) δ 8.13 (d, 1H), 8.07 - 8.03 (m, 1H), 7.79 (d, 1H), 7.63 (dd, 1H), 7.21 (s, 1H), 6.92 (d, 1H), 6.73 (d, 1H), 5.70 - 5.15 (m, 1H), 4.98 - 4.75 (m, 1H), 4.60 (s, 2H), 4.52 - 4.33 (m, 2H), 4.24 - 4.07 (m, 2H), 4.07 - 3.95 (m, 1H), 3.95 - 3.73 (m, 2H), 3.72 - 3.52 (m, 3H), 3.52 - 3.39 (m, 1H), 3.33 - 3.24 (m, 1H), 3.16 - 3.02 (m, 2H), 2.92 - 2.62 (m, 10H), 2.62 - 2.45 (m, 2H), 2.33 - 2.17 (m, 1H), 2.17 - 2.04 (m, 2H), 2.02 - 1.75 (m, 3H), 1.75 - 1.67 (m, 6H).

LCMS m/z = 888.3 [M+1] ⁺.

### Example 106: preparation of trifluoroacetate of compound 106

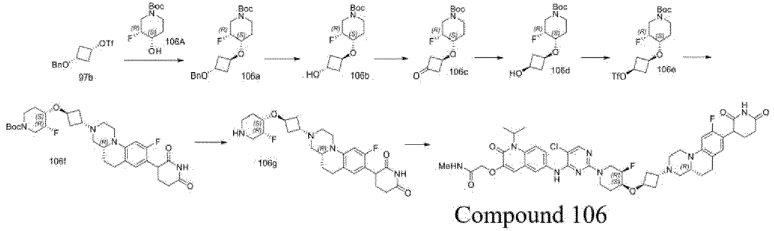

The trifluoroacetate of compound 106 was prepared using compounds 97b and 106A as the raw materials and referring to the synthesis method of example 102.

¹H NMR (400 MHz, CD₃OD) δ 8.11 - 8.02 (m, 2H), 7.80 (d, 1H), 7.64 (dd, 1H), 7.22 (s, 1H), 6.91 (d, 1H), 6.72 (d, 1H), 5.70 - 5.15 (m, 1H), 4.98 - 4.75 (m, 1H), 4.60 (s, 2H), 4.52 - 4.33 (m, 2H), 4.24 - 4.07 (m, 2H), 4.07 - 3.95 (m, 1H), 3.95 - 3.73 (m, 2H), 3.72 - 3.52 (m, 3H), 3.52 - 3.39 (m, 1H), 3.33 - 3.24 (m, 1H), 3.16 - 3.02 (m, 2H), 2.92 - 2.62 (m, 10H), 2.62 - 2.45 (m, 2H), 2.33 - 2.17 (m, 1H), 2.17 - 2.04 (m, 2H), 2.02 - 1.75 (m, 3H), 1.75 - 1.67 (m, 6H).

LCMS m/z = 445.0 [M/2+1] ⁺.

### Example 107: preparation of trifluoroacetate of compound 107

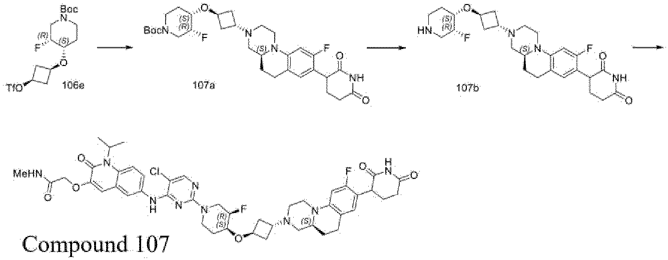

The trifluoroacetate of compound 107 was prepared using compounds 106e and 46e as the starting materials and referring to the synthesis method of example 103.

¹H NMR (400 MHz, CD₃OD) δ 8.12 - 8.02 (m, 2H), 7.80 (d, 1H), 7.64 (dd, 1H), 7.21 (s, 1H), 6.92 (d, 1H), 6.72 (d, 1H), 5.70 - 5.20 (m, 1H), 4.98 - 4.74 (m, 1H), 4.59 (s, 2H), 4.52 - 4.33 (m, 2H), 4.24 - 4.07 (m, 2H), 4.07 - 3.94 (m, 1H), 3.94 - 3.73 (m, 2H), 3.72 - 3.53 (m, 3H), 3.52 - 3.38 (m, 1H), 3.33 - 3.24 (m, 1H), 3.18 - 3.00 (m, 2H), 2.95 - 2.61 (m, 10H), 2.61 - 2.45 (m, 2H), 2.34 - 2.17 (m, 1H), 2.17 - 2.03 (m, 2H), 2.02 - 1.74 (m, 3H), 1.74 - 1.66 (m, 6H).

LCMS m/z = 445.1 [M/2+1] ⁺.

### Example 108: preparation of trifluoroacetate of compound 108

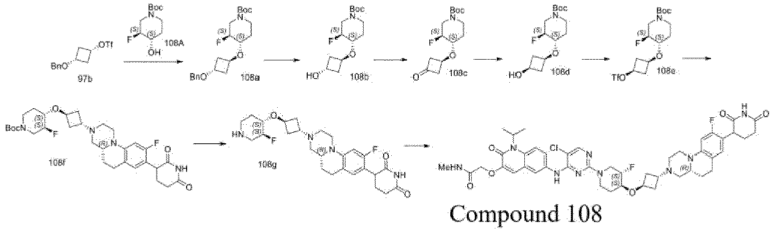

The trifluoroacetate of compound 108 was prepared using compounds 97b and 108A as the raw materials and referring to the synthesis method of example 102.

¹H NMR (400 MHz, CD₃OD) δ 8.12 (d, 1H), 8.03 (s, 1H), 7.77 (d, 1H), 7.59 (dd, 1H), 7.20 (s, 1H), 6.90 (d, 1H), 6.70 (d, 1H), 5.70 - 5.10 (m, 1H), 4.67 - 4.32 (m, 5H), 4.21 - 4.05 (m, 2H), 4.05 - 3.82 (m, 2H), 3.78 - 3.53 (m, 3H), 3.52 - 3.31 (m, 2H), 3.30 - 3.21 (m, 1H), 3.14 - 3.00 (m, 2H), 2.94 - 2.40 (m, 12H), 2.32 - 2.15 (m, 1H), 2.15 - 2.00 (m, 3H), 1.88 - 1.55 (m, 8H).

LCMS m/z = 444.7 [M/2+1]⁺.

### Example 109: preparation of trifluoroacetate of compound 109

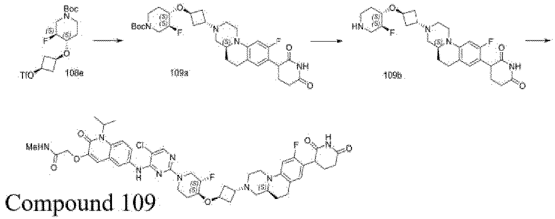

The trifluoroacetate of compound 109 was prepared using compounds 108e and 46e as the starting materials and referring to the synthesis method of example 103.

¹H NMR (400 MHz, CD₃OD) δ 8.09 (d, 1H), 8.03 (s, 1H), 7.77 (d, 1H), 7.60 (dd, 1H), 7.20 (s, 1H), 6.90 (d, 1H), 6.70 (d, 1H), 5.70 - 5.10 (m, 1H), 4.67 - 4.27 (m, 5H), 4.22 - 4.04 (m, 2H), 4.04 - 3.82 (m, 2H), 3.77 - 3.32 (m, 5H), 3.30 - 3.21 (m, 1H), 3.14 - 3.00 (m, 2H), 2.92 - 2.40 (m, 12H), 2.32 - 2.15 (m, 1H), 2.15 - 2.00 (m, 3H), 1.88 - 1.55 (m, 8H).

LCMS m/z = 444.8 [M/2+1]⁺.

### Example 110: preparation of trifluoroacetate of compound 110

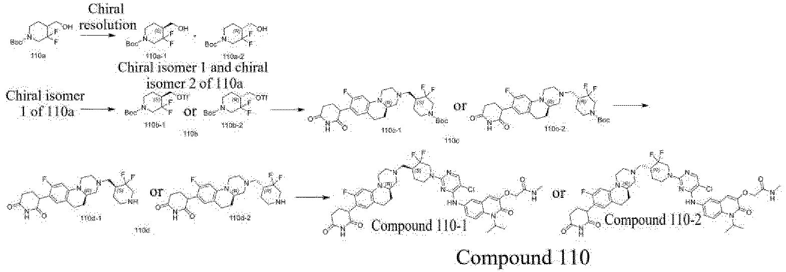

### Step 1: preparation of chiral isomer 1 and chiral isomer 2 of 110a

110a (see WO 2021023105 for the synthesis method) was subjected to chiral resolution, and the preparation conditions were as follows:
1. Instrument: Waters 150 Prep-SFC E; chromatographic column: Chiralcel AD column.
2. The sample was dissolved in acetonitrile and filtered with a 0.45 µm filter to prepare a sample liquid.
3. Preparative chromatography conditions: a. the mobile phase consists of systems A and B: mobile phase A: CO₂; mobile phase B: methanol; b. isocratic elution, mobile phase B content: 10%; c. flow rate: 120 mL/min.

Chiral analysis conditions:
1. Instrument: SHIMADZU LC-30AD; chromatographic column: Chiralcel AD column.
2. Analytical chromatography conditions: a. the mobile phase consists of systems A and B: mobile phase A: CO₂; mobile phase B: methanol (containing 0.05% diethylamine); b. isocratic elution, mobile phase B content: 5%-40%; c. flow rate: 3 mL/min.

Retention time: chiral isomer 1: 0.60 min, chiral isomer 2: 0.78 min.

Chiral isomer 1 and chiral isomer 2 of compound 110a are respectively one of the isomers of structure 110a-1 or 110a-2.

### Step 2: preparation of 110b

Chiral isomer 1 of 110a (0.25 g, 0.99 mmol) and TEA (0.50 g, 4.94 mmol) were dissolved in dichloromethane (10 mL). Trifluoromethanesulfonic anhydride (0.42 g, 1.49 mmol) was slowly added dropwise at -10°C, and the mixture was reacted at -10°C for 30 min. The reaction system was concentrated under reduced pressure (with the temperature controlled < 30°C) to obtain crude 110b (1.04 g).

Compound 110b is one of the isomers of structure 110b-1 or 110b-2.

### Step 3: preparation of 110c

The above-mentioned crude 110b (0.52 g) was dissolved in acetonitrile (8 mL). TEA (0.25 g, 2.47 mmol) and the above-mentioned crude 44f (0.16 g) were added, and the mixture was reacted at room temperature for 16 h. The reaction system was concentrated under reduced pressure, and the crude was separated and purified by silica gel column chromatography (dichloromethane:methanol (v/v) = 30:1) to obtain 110c (0.18 g, two-step yield calculated from chiral isomer 1 of compound 110a: 66%).

LCMS m/z = 551.8 [M+1] ⁺.

Compound 110c is one of the isomers of structure 110c-1 or 110c-2.

### Step 4: preparation of trifluoroacetate of 110d

110c (0.18 g, 0.33 mmol) was dissolved in dichloromethane (5 mL), and trifluoroacetic acid (2 mL) was added. The mixture was reacted at room temperature for 2 h. The reaction system was concentrated under reduced pressure to obtain crude trifluoroacetate of 110d (0.27 g).

LCMS m/z = 451.7 [M+1] ⁺.

Compound 110d is one of the isomers of structure 110d-1 or 110d-2.

### Step 5: preparation of trifluoroacetate of compound 110

The above-mentioned crude trifluoroacetate of 110d (0.27 g) was dissolved in DMSO (5 mL). Sodium bicarbonate (0.11 g, 1.31 mmol) was added, and the mixture was stirred at room temperature for 15 min. TEA (0.17 g, 1.68 mmol) and 42A (0.14 g, 0.32 mmol) were added, and the resulting mixture was reacted at 100°C for 16 h. The reaction system was cooled to room temperature. The reaction solution was subjected to Pre-HPLC (instrument and preparative column: using SHIMADZU LC-20AP preparative liquid phase chromatographic instrument, preparative column model: Phenomenex C18). Preparation method: the crude in acetonitrile solution was filtered with a 0.45 µm filter membrane to prepare a sample liquid. Mobile phase system: water (containing 0.1% trifluoroacetic acid)/acetonitrile. Gradient elution method: gradient elution with acetonitrile from 22% to 42% (elution time: 15 min). Lyophilization was performed to obtain the trifluoroacetate of compound 110 (138 mg).

¹H NMR (400 MHz, CD₃OD) δ 8.12 - 8.07 (m, 1H), 8.04 (s, 1H), 7.75 (d, 1H), 7.61 (dd, 1H), 7.15 (s, 1H), 6.90 (d, 1H), 6.72 (d, 1H), 5.70 - 5.20 (m, 1H), 5.04 - 4.89 (m, 1H), 4.72 - 4.61 (m, 1H), 4.57 (s, 2H), 4.17 - 4.05 (m, 1H), 3.94 - 3.75 (m, 2H), 3.75 - 3.60 (m, 2H), 3.46 - 3.31 (m, 2H), 3.31 - 2.98 (m, 5H), 2.96 - 2.79 (m, 5H), 2.79 - 2.59 (m, 3H), 2.32 - 2.16 (m, 1H), 2.16 - 2.01 (m, 3H), 1.88 - 1.63 (m, 8H).

LCMS m/z = 850.2 [M+1] ⁺.

Compound 110 is one of the isomers of compound 110-1 or compound 110-2.

### Example 111: preparation of trifluoroacetate of compound 111

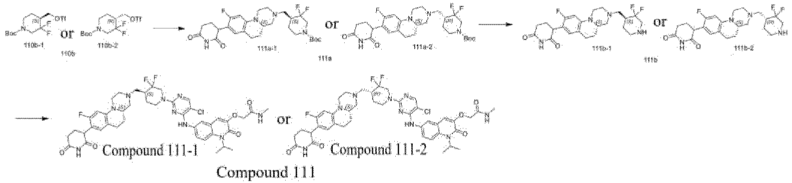

The trifluoroacetate of compound 111 was prepared using compounds 110b and 46e as the starting materials and referring to the synthesis method of example 110.

¹H NMR (400 MHz, CD₃OD) δ 8.10 (d, 1H), 8.03 (s, 1H), 7.74 (d, 1H), 7.60 (dd, 1H), 7.15 (s, 1H), 6.90 (d, 1H), 6.72 (d, 1H), 5.70 - 5.20 (m, 1H), 5.04 - 4.89 (m, 1H), 4.73 - 4.62 (m, 1H), 4.57 (s, 2H), 4.18 - 4.05 (m, 1H), 3.94 - 3.83 (m, 1H), 3.83 - 3.62 (m, 3H), 3.46 - 3.31 (m, 2H), 3.31 - 3.21 (m, 2H), 3.21 - 3.06 (m, 2H), 3.06 - 2.94 (m, 1H), 2.94 - 2.79 (m, 5H), 2.79 - 2.59 (m, 3H), 2.32 - 2.16 (m, 1H), 2.16 - 2.01 (m, 3H), 1.88 - 1.63 (m, 8H).

LCMS m/z = 850.3 [M+1] ⁺.

Compound 111 is one of the isomers of compound 111-1 or compound 111-2.

### Example 112: preparation of trifluoroacetate of compound 112

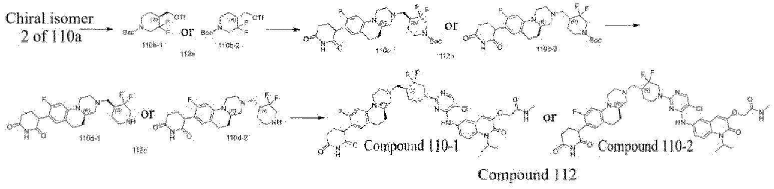

The trifluoroacetate of compound 112 was prepared using chiral isomer 2 of compound 110a as the starting material and referring to the synthesis method of example 110.

¹H NMR (400 MHz, CD₃OD) δ 8.12 (d, 1H), 8.02 (s, 1H), 7.74 (d, 1H), 7.60 (dd, 1H), 7.15 (s, 1H), 6.91 (d, 1H), 6.72 (d, 1H), 5.70 - 5.20 (m, 1H), 5.05 - 4.92 (m, 1H), 4.78 - 4.65 (m, 1H), 4.58 (s, 2H), 4.18 - 4.05 (m, 1H), 3.94 - 3.83 (m, 1H), 3.83 - 3.62 (m, 3H), 3.46 - 2.94 (m, 7H), 2.94 - 2.80 (m, 5H), 2.80 - 2.59 (m, 3H), 2.32 - 2.16 (m, 1H), 2.16 - 2.01 (m, 3H), 1.88 - 1.63 (m, 8H).

LCMS m/z = 425.7 [M/2+1] ⁺

Compound 112 is one of the isomers of compound 110-1 or compound 110-2.

### Example 113: preparation of trifluoroacetate of compound 113

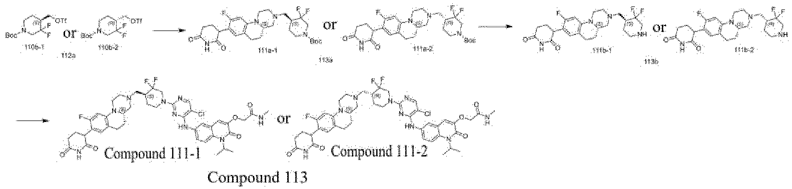

The trifluoroacetate of compound 113 was prepared using compounds 112a and 46e as the starting materials and referring to the synthesis method of example 110.

¹H NMR (400 MHz, CD₃OD) δ 8.15 - 8.07 (m, 1H), 8.07 - 8.00 (m, 1H), 7.75 (d, 1H), 7.65 - 7.56 (m, 1H), 7.15 (s, 1H), 6.90 (d, 1H), 6.72 (d, 1H), 5.70 - 5.20 (m, 1H), 5.05 - 4.90 (m, 1H), 4.75 - 4.62 (m, 1H), 4.57 (s, 2H), 4.18 - 4.05 (m, 1H), 3.94 - 3.75 (m, 2H), 3.75 - 3.62 (m, 2H), 3.44 - 3.31 (m, 2H), 3.31 - 3.00 (m, 5H), 2.96 - 2.79 (m, 5H), 2.79 - 2.59 (m, 3H), 2.32 - 2.16 (m, 1H), 2.16 - 2.01 (m, 3H), 1.88 - 1.63 (m, 8H).

LCMS m/z = 425.6 [M/2+1] ⁺.

Compound 113 is one of the isomers of compound 111-1 or compound 111-2.

### Example 114: preparation of trifluoroacetate of compound 114

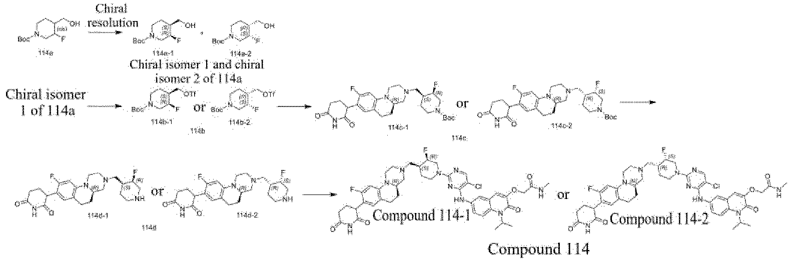

### Step 1: preparation of chiral isomer 1 and chiral isomer 2 of 114a

114a (see WO 2012066488 for the synthesis method) was subjected to chiral resolution, and the preparation conditions were as follows:
1. Instrument: Waters 150 ap; chromatographic column: ChiralPak OZ 19×250 5 µm.
2. Preparative chromatography conditions: a. the mobile phase consists of systems A and B: mobile phase A: CO₂; mobile phase B: methanol; b. isocratic elution, mobile phase B content: 6%; c. flow rate: 400 mL/min.

Retention time: chiral isomer 1: 7.43 min, chiral isomer 2: 8.87 min.

Chiral isomer 1 and chiral isomer 2 of compound 114a are respectively one of the isomers of structure 114a-1 or 114a-2.

### Step 2: preparation of 114b

Chiral isomer 1 of 114a (0.4 g, 1.71 mmol) and TEA (0.87 g, 8.60 mmol) were dissolved in dichloromethane (10 mL). Trifluoromethanesulfonic anhydride (0.72 g, 2.55 mmol) was slowly added dropwise at -20°C, and the mixture was reacted at -20°C for 30 min. The reaction system was concentrated under reduced pressure (with the temperature controlled < 30°C) to obtain crude 114b (1.2 g).

Compound 114b is one of the isomers of structure 114b-1 or 114b-2.

### Step 3: preparation of 114c

The above-mentioned crude 114b (0.6 g) was dissolved in acetonitrile (10 mL). TEA (0.43 g, 4.25 mmol) and the above-mentioned crude 44f (1.5 g) were added, and the mixture was reacted at room temperature for 16 h. The reaction system was concentrated under reduced pressure, and the crude was separated and purified by silica gel column chromatography (dichloromethane:methanol (v/v) = 20:1) to obtain 114c (0.29 g, two-step yield calculated from chiral isomer 1 of compound 114a: 64%).
LCMS m/z = 533.3 [M+1] ⁺

Compound 114c is one of the isomers of structure 114c-1 or 114c-2.

### Step 4: preparation of trifluoroacetate of 114d

114c (0.15 g, 0.28 mmol) was dissolved in dichloromethane (3 mL), and trifluoroacetic acid (1 mL) was added. The mixture was reacted at room temperature for 2 h. The reaction system was concentrated under reduced pressure to obtain crude trifluoroacetate of 114d (0.27 g).

Compound 114d is one of the isomers of structure 114d-1 or 114d-2.

### Step 5: preparation of trifluoroacetate of compound 114

The above-mentioned crude trifluoroacetate of 114d (0.27 g) was dissolved in DMSO (5 mL). DIPEA (0.27 g, 3.21 mmol) was added, and the mixture was stirred at room temperature for 15 min. 42A (0.09 g, 0.21 mmol) were added, and the resulting mixture was reacted at 100°C for 16 h. The reaction system was cooled to room temperature. The reaction solution was subjected to Pre-HPLC (instrument and preparative column: using SHIMADZU LC-20AP preparative liquid phase chromatographic instrument, preparative column model: Phenomenex C18). Preparation method: the crude in acetonitrile solution was filtered with a 0.45 µm filter membrane to prepare a sample liquid. Mobile phase system: water (containing 0.1% trifluoroacetic acid)/acetonitrile. Gradient elution method: gradient elution with acetonitrile from 20% to 40% (elution time: 15 min). Lyophilization was performed to obtain the trifluoroacetate of compound 114 (110 mg).

¹H NMR (400 MHz, CD₃OD) δ 8.06 (s, 1H), 8.04 (d, 1H), 7.79 (d, 1H), 7.67 (dd, 1H), 7.20 (s, 1H), 6.92 (d, 1H), 6.73 (d, 1H), 5.80 - 5.20 (m, 1H), 5.10 - 4.83 (m, 2H), 4.68 - 4.52 (m, 3H), 4.17 - 4.05 (m, 1H), 3.94 - 3.85 (m, 1H), 3.84 - 3.65 (m, 2H), 3.53 - 3.35 (m, 2H), 3.33 - 3.08 (m, 5H), 3.08 - 2.93 (m, 1H), 2.93 - 2.80 (m, 4H), 2.80 - 2.63 (m, 3H), 2.63 - 2.43 (m, 1H), 2.33 - 2.18 (m, 1H), 2.18 - 2.01 (m, 2H), 1.94 - 1.63 (m, 9H).

LCMS m/z = 417.0 [M/2+1] ⁺.

Compound 114 is one of the isomers of compound 114-1 or compound 114-2.

### Example 115: preparation of trifluoroacetate of compound 115

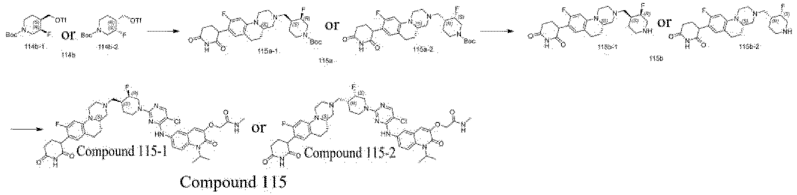

Compound 115 was prepared using compounds 114b and 46e as the starting materials and referring to the synthesis method of example 114.

¹H NMR (400 MHz, CD₃OD) δ 8.07 (s, 1H), 8.05 - 8.01 (m, 1H), 7.80 (d, 1H), 7.67 (dd, 1H), 7.20 (s, 1H), 6.92 (d, 1H), 6.73 (d, 1H), 5.80 - 5.20 (m, 1H), 5.10 - 4.83 (m, 2H), 4.68 - 4.52 (m, 3H), 4.17 - 4.05 (m, 1H), 3.94 - 3.85 (m, 1H), 3.84 - 3.65 (m, 2H), 3.55 - 3.35 (m, 2H), 3.33 - 3.10 (m, 5H), 3.10 - 2.94 (m, 1H), 2.94 - 2.80 (m, 4H), 2.80 - 2.65 (m, 3H), 2.65 - 2.43 (m, 1H), 2.33 - 2.18 (m, 1H), 2.18 - 2.01 (m, 2H), 1.94 - 1.63 (m, 9H).

LCMS m/z = 416.9 [M/2+1] ⁺.

Compound 115 is one of the isomers of compound 115-1 or compound 115-2.

### Example 116: preparation of trifluoroacetate of compound 116

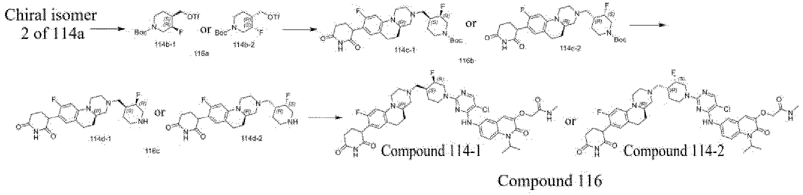

The trifluoroacetate of compound 116 was prepared using chiral isomer 2 of compound 114a as the starting material and referring to the synthesis method of example 114.

¹H NMR (400 MHz, CD₃OD) δ 8.08 - 7.94 (m, 2H), 7.82 - 7.72 (m, 1H), 7.70 - 7.60 (m, 1H), 7.21 - 7.15 (m, 1H), 6.89 (d, 1H), 6.76 - 6.66 (m, 1H), 5.70 - 5.20 (m, 1H), 5.10 - 4.82 (m, 2H), 4.65 - 4.47 (m, 3H), 4.16 - 4.02 (m, 1H), 3.94 - 3.83 (m, 1H), 3.83 - 3.65 (m, 2H), 3.52 - 3.33 (m, 2H), 3.30 - 3.07 (m, 5H), 3.07 - 2.92 (m, 1H), 2.92 - 2.78 (m, 4H), 2.78 - 2.64 (m, 3H), 2.64 - 2.42 (m, 1H), 2.32 - 2.15 (m, 1H), 2.15 - 2.00 (m, 2H), 1.90 - 1.62 (m, 9H).

LCMS m/z = 416.7 [M/2+1] ⁺.

Compound 116 is one of the isomers of compound 114-1 or compound 114-2.

### Example 117: preparation of trifluoroacetate of compound 117

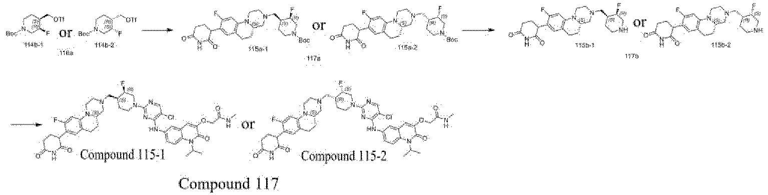

Compound 117 was prepared using compounds 116a and 46e as the starting materials and referring to the synthesis method of example 114.

¹H NMR (400 MHz, CD₃OD) δ 8.10 - 7.97 (m, 2H), 7.82 - 7.73 (m, 1H), 7.69 - 7.61 (m, 1H), 7.21 - 7.14 (m, 1H), 6.90 (d, 1H), 6.71 (d, 1H), 5.70 - 5.15 (m, 1H), 5.10 - 4.83 (m, 2H), 4.68 - 4.50 (m, 3H), 4.17 - 4.03 (m, 1H), 3.92 - 3.83 (m, 1H), 3.83 - 3.64 (m, 2H), 3.52 - 3.32 (m, 2H), 3.32 - 3.07 (m, 5H), 3.07 - 2.91 (m, 1H), 2.91 - 2.79 (m, 4H), 2.79 - 2.64 (m, 3H), 2.64 - 2.40 (m, 1H), 2.32 - 2.16 (m, 1H), 2.16 - 2.00 (m, 2H), 1.92 - 1.63 (m, 9H).

LCMS m/z = 416.9 [M/2+1] ⁺.

Compound 117 is one of the isomers of compound 115-1 or compound 115-2.

### Biological test examples

### 1. SU-DHL-6 cell proliferation experiment

Lymphoma SU-DHL-6 cells purchased from ATCC were placed in an RPMI-1640 complete medium (containing 15% foetal bovine serum, 100 U/mL penicillin and 100 µg/mL streptomycin) and cultured at 37°C and 5% CO₂. The cells in the logarithmic growth phase were collected, and the cell suspension was adjusted to an appropriate density with the culture medium and added to a 96-well culture plate at 3000 cells/well. The test compounds at different concentrations were then added, and the plate was further cultured at 37°C and 5% CO₂. After 5 days, the cells were uniformly mixed with a pipette. 1/10 of the cells were transferred to a new 96-well culture plate, a drug-containing culture medium was added, and the plate was incubated for another 5 days. After the incubation was completed, according to operation instructions for a CellTiter-Glo kit (Promega, G7573), 75 µL of CTG solution, which was equilibrated to room temperature, was added to each well, and the mixtures were uniformly mixed for 2 min using a microplate shaker. The plate was placed at room temperature for 10 min, and then fluorescence signal values were measured using a BMG multimode microplate reader (PHERAstar FSX). The cell proliferation inhibition rate was calculated according to formula (1), where RLU compound was the readout of the drug treated group, RLU control was the average value of the solvent control group, and RLU blank was the average value of the cell-free well. GraphPad Prism software was used to analyse data and calculate IC₅₀ values. Inhibition rate % = [1- (RLUcompound - RLUblank)/(RLUcontrol - RLUblank)] × 100%

Conclusion: The compounds of the present invention, such as the example compounds, have a good inhibitory effect on the proliferation of SU-DHL-6 cells.

### 2. Bcl-6 protein degradation experiment in Farage cells

Farage cells purchased from ATCC were placed in an RPMI-1640 complete medium (containing 10% foetal bovine serum, 100 U/mL penicillin and 100 µg/mL streptomycin) and cultured at 37°C and 5% CO₂. The cells in the logarithmic growth phase were collected, and the cell suspension was adjusted to an appropriate density with the culture medium and added to a 6-well cell culture plate at a volume of 1 mL/well. The compounds to be tested were prepared to 2-fold the final concentration, 1 mL of the compounds at different concentrations were added to administration wells, a medium containing 0.2% DMSO was added to control wells, and the plate was incubated at 37°C for 24 hours. The cells were collected into a 1.5 mL centrifuge tube, 25 µL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 0.8 mg/mL, Bcl6 was detected using a fully automated protein expression quantitative analyser (ProteinSimple), and the internal reference protein was β-actin (both antibodies were from CST). The software (Compass for SW) of the fully automated protein expression quantitative analyser was used to process the raw data and calculate the peak area and the Bcl6 expression rate relative to the control group. DC₅₀ values were calculated using a four-parameter non-linear fitting model in Graphpad 8.3.0 software.

The DC₅₀ results for Bcl-6 degradation in Farage cells by the compounds of the present invention were shown in Table 1.

**Table 1 DC₅₀ for Bcl6 degradation in Farage cells**

| Compound | Bcl6 DC₅₀ (nM) | Compound | Bcl6 DC₅₀ (nM) |
|---|---|---|---|
| Compound 1 | <10 | Trifluoroacetate of | <10 |
| | | compound 63 | |
| Compound 2 | <10 | Trifluoroacetate of compound 64 | <10 |
| Compound 4 | <10 | Trifluoroacetate of compound 65 | <10 |
| Trifluoroacetate of compound 5 | <10 | Trifluoroacetate of compound 66 | <10 |
| Compound 6 | <10 | Trifluoroacetate of compound 67 | <10 |
| Trifluoroacetate of compound 7 | <10 | Trifluoroacetate of compound 68 | <10 |
| Compound 8 | <10 | Trifluoroacetate of compound 69 | <10 |
| Compound 9 | <10 | Trifluoroacetate of compound 70 | <10 |
| Compound 10 | <10 | Trifluoroacetate of compound 71 | <10 |
| Trifluoroacetate of compound 11 | <10 | Trifluoroacetate of compound 72 | <10 |
| Compound 12 | <10 | Trifluoroacetate of compound 73 | <10 |
| Trifluoroacetate of compound 13 | <10 | Trifluoroacetate of compound 74 | <10 |
| Trifluoroacetate of compound 14 | <10 | Trifluoroacetate of compound 75 | <10 |
| Trifluoroacetate of compound 17 | <10 | Trifluoroacetate of compound 76 | <10 |
| Trifluoroacetate of compound 20 | <10 | Trifluoroacetate of compound 77 | <10 |
| Trifluoroacetate of compound 21 | <10 | Trifluoroacetate of compound 78 | <10 |
| Compound 22 | <10 | Trifluoroacetate of compound 79 | <10 |
| Trifluoroacetate of compound 24 | <10 | Trifluoroacetate of compound 80 | <10 |
| Chiral isomer 1 of compound 24 | <10 | Trifluoroacetate of compound 81 | <10 |
| Chiral isomer 2 of | <10 | Trifluoroacetate of | <10 |
| compound 24 | | compound 82 | |
| Trifluoroacetate of compound 31 | <10 | Trifluoroacetate of compound 83 | <10 |
| Trifluoroacetate of compound 32 | <10 | Trifluoroacetate of compound 84 | <10 |
| Trifluoroacetate of compound 33 | <10 | Trifluoroacetate of compound 85 | <10 |
| Compound 35 | <10 | Trifluoroacetate of compound 86 | <10 |
| Trifluoroacetate of compound 36 | <10 | Trifluoroacetate of compound 87 | <10 |
| Trifluoroacetate of compound 37 | <10 | Trifluoroacetate of compound 88 | <10 |
| Trifluoroacetate of compound 38 | <10 | Trifluoroacetate of compound 89 | <10 |
| Compound 39 | <10 | Trifluoroacetate of compound 90 | <10 |
| Trifluoroacetate of compound 40 | <10 | Trifluoroacetate of compound 91 | <10 |
| Trifluoroacetate of compound 41 | <10 | Trifluoroacetate of compound 92 | <10 |
| Trifluoroacetate of compound 42 | <10 | Trifluoroacetate of compound 93 | <10 |
| Trifluoroacetate of compound 43 | <10 | Trifluoroacetate of compound 94 | <10 |
| Trifluoroacetate of compound 44 | <10 | Compound 95 | <10 |
| Trifluoroacetate of compound 45 | <10 | Compound 96 | <10 |
| Trifluoroacetate of compound 46 | <10 | Trifluoroacetate of compound 98 | <10 |
| Compound 47 | <10 | Trifluoroacetate of compound 99 | <10 |
| Compound 49 | <10 | Trifluoroacetate of compound 101 | <10 |
| Compound 50 | <10 | Trifluoroacetate of compound 102 | <10 |
| Compound 51 | <10 | Trifluoroacetate of | <10 |
| | | compound 103 | |
| Trifluoroacetate of compound 52 | <10 | Trifluoroacetate of compound 104 | <10 |
| Trifluoroacetate of compound 53 | <10 | Trifluoroacetate of compound 105 | <10 |
| Trifluoroacetate of compound 54 | <10 | Trifluoroacetate of compound 106 | <10 |
| Trifluoroacetate of compound 55 | <10 | Trifluoroacetate of compound 107 | <10 |
| Trifluoroacetate of compound 56 | <10 | Trifluoroacetate of compound 108 | <10 |
| Trifluoroacetate of compound 57 | <10 | Trifluoroacetate of compound 109 | <10 |
| Trifluoroacetate of chiral isomer 1 of compound 57 | <10 | Trifluoroacetate of compound 112 | <10 |
| Chiral isomer 2 of compound 57 | <10 | Trifluoroacetate of compound 113 | <10 |
| Trifluoroacetate of compound 58 | <10 | Trifluoroacetate of compound 114 | <10 |
| Trifluoroacetate of compound 59 | <10 | Trifluoroacetate of compound 115 | <10 |
| Trifluoroacetate of compound 60 | <10 | Trifluoroacetate of compound 116 | <10 |
| Trifluoroacetate of compound 61 | <10 | Trifluoroacetate of compound 117 | <10 |
| Trifluoroacetate of compound 62 | <10 | | |

Conclusion: The compounds of the present invention, such as the example compounds, have a good degradation effect on Bcl-6 protein in Farage cells.

### 3. Pharmacokinetic test in mice

1.1 Experimental animal: male ICR mice, 25-30 g, 6 mice/compound, purchased from Chengdu Ddossy Experimental Animals Co., Ltd.

1.2 Experimental design: on the day of the experiment, the ICR mice were randomly grouped according to their body weights. The animals were fasted but with water available for 12 to 14 h one day before the administration, and were fed 4 h after the administration.

**Table 2 Administration information of pharmacokinetic test in mice**

| Group | Number | Administration information | | | | | |
|---|---|---|---|---|---|---|---|
| | Male | Test compound | Administ ration dosage (mg/kg) | Administra tion concentrati on (mg/mL) | Administra tion volume (mL/kg) | Collecte d sample | Mode of administration |
| G1 | 3 | Compoun d of the present invention | 2 | 0.4 | 5 | Plasma | Intravenous administration |
| G2 | 3 | | 5 | 0.5 | 10 | Plasma | Intragastric administration |

| | | | | | | | |
|---|---|---|---|---|---|---|---|
| Note: vehicle for intravenous administration: 10% DMA+10% Solutol+80% NS; vehicle for intragastric administration: PO: 5% DMSO+5% Solutol+30% PEG 400+60% (20% SBE-β-CD) | | | | | | | |

Before and after the administration, 0.03 mL of blood was taken from the orbits under isoflurane anaesthesia and placed in an EDTAK2 centrifuge tube. The blood was centrifuged at 5000 rpm at 4°C for 10 min, and the plasma was collected. The blood collection time points for the intravenous administration group and intragastric administration group were 0, 5 min, 15 min, 30 min, 1 h, 2 h, 4 h, 7 h and 24 h. Before analysis and detection, all samples were stored at -80°C. The samples were analysed quantitatively by LC-MS/MS.

**Table 3 Pharmacokinetic parameters of compounds of the present invention in plasma of mice**

| Test compound | Mode of administration | AUC₀₋ₜ (hr*ng/mL) | F (%) |
|---|---|---|---|
| Compound 8 | i.g. (5 mg/kg) | >1000 | >50% |
| Trifluoroacetate of compound 13 | i.g. (5 mg/kg) | >1000 | |
| Trifluoroacetate of compound 14 | i.g. (5 mg/kg) | >1000 | |
| Compound 15 | i.g. (5 mg/kg) | >1000 | |
| Trifluoroacetate of compound 21 | i.g. (5 mg/kg) | >1000 | >50% |
| Compound 23 | i.g. (5 mg/kg) | >1000 | |
| Trifluoroacetate of compound 24 | i.g. (5 mg/kg) | >1000 | >50% |
| Compound 35 | i.g. (5 mg/kg) | >1000 | |
| Trifluoroacetate of compound 44 | i.g. (5 mg/kg) | >1000 | |
| Trifluoroacetate of compound 46 | i.g. (5 mg/kg) | >1000 | |
| Trifluoroacetate of compound 56 | i.g. (5 mg/kg) | >1000 | |
| Trifluoroacetate of compound 57 | i.g. (5 mg/kg) | >1000 | |
| Trifluoroacetate of compound 58 | i.g. (5 mg/kg) | >1000 | >50% |
| Trifluoroacetate of compound 62 | i.g. (5 mg/kg) | >1000 | >50% |
| Trifluoroacetate of compound 63 | i.g. (5 mg/kg) | >1000 | |
| Trifluoroacetate of compound 64 | i.g. (5 mg/kg) | >1000 | |
| Trifluoroacetate of compound 65 | i.g. (5 mg/kg) | >1000 | |
| Trifluoroacetate of compound 66 | i.g. (5 mg/kg) | >1000 | >50% |
| Trifluoroacetate of compound 67 | i.g. (5 mg/kg) | >1000 | >50% |
| Control compound 1 | i.g. (5 mg/kg) | <100 | <5% |

| | | | |
|---|---|---|---|
| Conclusion: The compounds of the present invention, such as the example compounds, have good oral absorption in mice. | | | |

Control compound 1 has a structure: 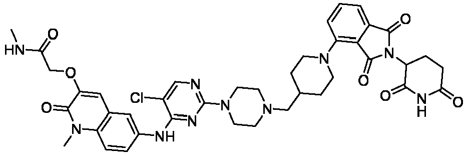 see WO 2021077010 for the synthesis method.

### 4. Bcl-6 protein degradation experiment in OCI-LY1 cells

OCI-LY1 cells purchased from DSMZ were placed in an IMDM complete medium (containing 10% foetal bovine serum, 100 U/mL penicillin, 100 µg/mL streptomycin and 50 µM mercaptoethanol) and cultured at 37°C and 5% CO₂. The cells in the logarithmic growth phase were collected, and the cell suspension was adjusted to an appropriate density with the culture medium and added to a 6-well cell culture plate at a volume of 1 mL/well. The compounds to be tested were prepared to 2-fold the final concentration, 1 mL of the compounds at different concentrations were added to administration wells, a medium containing 0.2% DMSO was added to control wells, and the plate was incubated at 37°C for 24 hours. The cells were collected into a 1.5 mL centrifuge tube, 25 µL of RIPA lysis buffer (containing 1X protease inhibitor cocktail) was added, and the cells were lysed on ice for 15 minutes and centrifuged at 12000 rpm/min at 4°C for 10 minutes. The supernatant was collected and the protein content was determined using the BCA method. The protein sample to be tested was diluted to 0.8 mg/mL, Bcl6 was detected using a fully automated protein expression quantitative analyser (ProteinSimple), and the internal reference protein was β-actin (both antibodies were from CST). The software (Compass for SW) of the fully automated protein expression quantitative analyser was used to process the raw data and calculate the peak area and the Bcl6 expression rate relative to the control group. DC₅₀ values were calculated using a four-parameter non-linear fitting model in Graphpad 8.3.0 software.

The DC₅₀ results for Bcl-6 degradation in OCI-LY1 cells by the compounds of the present invention were shown in Table 4.

**Table 4 DC₅₀ for Bcl6 degradation in OCI-LY1 cells**

| Compound | Bcl6 DC₅₀ (nM) |
|---|---|
| Compound 8 | <100 |
| Compound 15 | <100 |
| Compound 16 | <100 |
| Compound 18 | <100 |
| Compound 23 | <100 |
| Trifluoroacetate of compound 24 | <100 |
| Trifluoroacetate of | <100 |
| compound 27 | |
| Compound 28 | <100 |
| Trifluoroacetate of compound 57 | <100 |
| Trifluoroacetate of compound 58 | <100 |

| | |
|---|---|
| Conclusion: The compounds of the present invention, such as the example compounds, have a good degradation effect on Bcl-6 protein in OCI-LY1 cells. Specifically, for example compound 8, the trifluoroacetate of compound 24, the trifluoroacetate of compound 57 and the trifluoroacetate of compound 58 have a DC₅₀ of less than 5 nM for Bcl-6 protein in OCI-LY1 cells. | |

### 5. Pharmacokinetic test in rats

**Experimental objective:** in this experiment, a single dose of each test compound was administered to SD rats intravenously and intragastrically, and the concentrations of the test compounds in plasma of rats were measured to evaluate the pharmacokinetic characteristics of the test compounds in rats.

**Experimental animal:** male SD rats, 200-220 g, 6-8 weeks old, 6 rats/compound, purchased from Chengdu Ddossy Experimental Animals Co., Ltd.

**Experimental method:** on the day of the experiment, the SD rats were randomly grouped according to their body weights. The animals were fasted but with water available for 12 to 14 h one day before the administration, and were fed 4 h after the administration.

**Table 5 Administration information of pharmacokinetic test in rats**

| Group | Number | Administration information | | | | | | |
|---|---|---|---|---|---|---|---|---|
| | Male | Test compound | Administr ation dosage* (mg/kg) | Administration concentration (mg/mL) | Administ ration volume (mL/kg) | Collecte d sample | Mode of administra tion | Vehicle |
| G1 | 3 | Compound | 5 | 0.5 | 10 | Plasma | Oral (Intragastr ic administra tion) | 5% DMSO+ 5% Solutol+ 30% PEG-400+60% (20% SBE-β-CD) |
| | | Compound | | | | | | |
| G2 | 3 | Compound of the present invention | 2 | 0.4 | 5 | Plasma | Intraveno us injection | 5% DMA+5% HS-15+90% NS |

| | | | | | | | | |
|---|---|---|---|---|---|---|---|---|
| *Dosage is calculated based on free base. | | | | | | | | |

**Sampling:** Before and after the administration, 0.1 mL of blood was taken from the orbits under isoflurane anaesthesia and placed in an EDTAK2 centrifuge tube. Centrifugation was carried out at 5000 rpm at 4°C for 10 min, and the plasma was collected.

The plasma collection time points for IV&PO groups were 0, 5 min, 15 min, 30 min, 1 h, 2 h, 4 h, 7 h, and 24 h.

Before analysis and detection, all samples were stored at -60°C. The samples were analysed quantitatively by LC-MS/MS.

**Table 6 Pharmacokinetic parameters of compounds of the present invention in plasma of rats**

| Test compound | Mode of administration | AUC₀₋ₜ (hr*ng/mL) | F (%) |
|---|---|---|---|
| Compound 8 | i.g. (5 mg/kg) | >1000 | >50% |
| Compound 35 | i.g. (5 mg/kg) | >1000 | >50% |
| Trifluoroacetate of compound 71 | i.g. (5 mg/kg) | >1000 | - |
| Trifluoroacetate of compound 72 | i.g. (5 mg/kg) | >1000 | - |
| Trifluoroacetate of compound 73 | i.g. (5 mg/kg) | >1000 | - |
| Trifluoroacetate of compound 74 | i.g. (5 mg/kg) | >1000 | - |
| Trifluoroacetate of compound 78 | i.g. (5 mg/kg) | >1000 | - |
| Trifluoroacetate of compound 79 | i.g. (5 mg/kg) | >1000 | - |
| Trifluoroacetate of compound 83 | i.g. (5 mg/kg) | >1000 | - |

| | | | |
|---|---|---|---|
| Conclusion: The compounds of the present invention, such as the example compounds, have good oral absorption in rats. Specifically, for example compound 35 has a bioavailability (F) of greater than 90%. | | | |

### 6. Pharmacokinetic test in beagle dogs

**Experimental animal:** male beagle dogs, about 8-10 kg, 6 beagle dogs/compound, purchased from Beijing Marshall Biotechnology Co., Ltd.

**Experimental method:** on the day of the experiment, the beagle dogs were randomly grouped according to their body weights. The animals were fasted but with water available for 14 to 18 h one day before the administration, and were fed 4 h after the administration.

**Table 7 Administration information of pharmacokinetic test in beagle dogs**

| Group | Number | Administration information | | | | | |
|---|---|---|---|---|---|---|---|
| | Male | Test compound | Administra tion dosage (mg/kg) | Administra tion concentrati on (mg/mL) | Administ ration volume (mL/kg) | Collected sample | Mode of administration |
| G1 | 3 | Compound of the present invention | 0.5 | 0.5 | 1 | Plasma | Intravenous administration |
| G2 | 3 | Compound of the present invention | 2 | 0.4 | 5 | Plasma | Oral (intragastric administration) |

| | | | | | | | |
|---|---|---|---|---|---|---|---|
| Note: Vehicle for intravenous administration: 5% DMA+5% Solutol+90% Saline; vehicle for oral administration (intragastric administration): 5% DMSO+5% Solutol+30% PEG400+60% (20% SBE-CD); *Dosage is calculated based on free base. | | | | | | | |

Before and after the administration, 1 ml of blood was taken from the jugular veins or limb veins, and placed in an EDTAK2 centrifuge tube. Centrifugation was carried out at 5000 rpm at 4°C for 10 min, and the plasma was collected. The blood collection time points for the intravenous administration group and intragastric administration group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were analysed quantitatively by LC-MS/MS.

Conclusion: The compounds of the present invention, such as the example compounds, have good oral absorption in beagle dogs.

### 7. Pharmacokinetic test in monkeys

**Experimental animal:** male cynomolgus monkeys, 3-5 kg, 3-6 years old, 4 monkeys/compound, purchased from Suzhou Xishan Biotechnology Inc.

**Experimental method:** on the day of the experiment, the monkeys were randomly grouped according to their body weights. The animals were fasted but with water available for 14 to 18 h one day before the administration, and were fed 4 h after the administration.

**Table 8 Administration information of pharmacokinetic test in monkeys**

| Group | Number | Administration information | | | | | |
|---|---|---|---|---|---|---|---|
| | Male | Test compound | Administra tion dosage (mg/kg) | Administr ation concentrati on (mg/mL) | Administr ation volume (mL/kg) | Collected sample | Mode of administration |
| G1 | 2 | Compound of the present invention | 0.5 | 0.5 | 1 | Plasma | Intravenous administration |
| G2 | 2 | Compound of the present invention | 2 | 0.4 | 5 | Plasma | Oral (intragastric administration ) |

| | | | | | | | |
|---|---|---|---|---|---|---|---|
| Note: vehicle for intravenous administration: 5% DMA+5% Solutol+90% Saline; vehicle for oral administration (intragastric administration): 5% DMSO+5% Solutol+30% PEG400+60% (20% SBE-CD); *Dosage is calculated based on free base. | | | | | | | |

Before and after the administration, 1.0 mL of blood was taken from the jugular veins and placed in an EDTAK2 centrifuge tube. Centrifugation was carried out at 5000 rpm at 4°C for 10 min, and the plasma was collected. The blood collection time points for the intravenous administration group and intragastric administration group were 0, 5 min, 15 min, 30 min, 1 h, 2 h, 4 h, 6 h, 8 h, 10 h, 12 h, 24 h and 48 h. Before analysis and detection, all samples were stored at -60°C. The samples were analysed quantitatively by LC-MS/MS.

Conclusion: The compounds of the present invention, such as the example compounds, have good oral absorption in monkeys.

### 8. Test for hERG potassium ion channel

**Experimental platform:** electrophysiological manual patch-clamp system

**Cell line:** Chinese hamster ovary (CHO) cell lines stably expressing hERG potassium ion channel

**Experimental method:** in CHO (Chinese Hamster Ovary) cells stably expressing hERG potassium channel, the whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (*I* _{hERG}) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

**Data processing:** data analysis processing was carried out by using pClamp 10, GraphPad Prism 5 and Excel software. The inhibition degree of hERG potassium current (peak value of hERG tail current induced at -50 mV) at different compound concentrations was calculated by the following formula:$Inhibition % = \left[1-\left(I/Io\right)\right] \times 100 %$ where Inhibition% represents the percentage of inhibition of hERG potassium current by the compound, and *I* and *I*o represent the amplitude of hERG potassium current after and before the administration, respectively.

Compound IC₅₀ was calculated using GraphPad Prism 5 software by fitting according to the following equation:$Y = Bottom + \left(Tio-Bottom\right) / \left(1+10\left(\left({LogIC}_{50}-X\right)*HillSlope\right)\right)$

Among the equation, X represents the Log value of the tested concentration of the test sample, Y represents the inhibition percentage at the corresponding concentration, and Bottom and Top represent the minimum and maximum inhibition percentage, respectively.

The IC₅₀ values of inhibitory effects of compounds of the present invention on hERG potassium channel current were shown in Table 9.

**Table 9 IC₅₀ values of inhibitory effects on hERG potassium channel current**

| Compound | IC₅₀ (µM) |
|---|---|
| Compound 8 | >40 |
| Compound 35 | >30 |
| Trifluoroacetate of compound 46 | >30 |
| Trifluoroacetate of compound 67 | >40 |
| Trifluoroacetate of compound 71 | >40 |
| Trifluoroacetate of compound 72 | >40 |
| Trifluoroacetate of compound 73 | >40 |
| Trifluoroacetate of compound 75 | >40 |
| Trifluoroacetate of compound 76 | >40 |
| Trifluoroacetate of compound 77 | >40 |
| Trifluoroacetate of compound 78 | >40 |

| | |
|---|---|
| Conclusion: The compounds of the present invention, such as the example compounds, do not exhibit obvious inhibitory effects on hERG potassium channel current. | |

### 9. Test of stability in liver microsomes

In this experiment, liver microsomes of five species, including human, dog, rat, and mouse, were used as *in vitro* models to evaluate the metabolic stability of test compounds. At 37°C, 1 µM of the test compound was co-incubated with microsomal protein and coenzyme NADPH. At given time points (5 min, 10 min, 20 min, 30 min and 60 min), the reaction was terminated by adding ice-cold acetonitrile containing an internal standard. The LC-MS/MS method was used to measure the concentration of the test compound in the sample. T_{1/2} was calculated using the natural logarithm (ln) of the residual rate of the drug in the incubation system and the incubation time. In addition, the intrinsic clearance in liver microsomes (CL_{int(mic)}) and the intrinsic clearance in liver (CL_{int(Liver)}) were further calculated.

Conclusion: The compounds of the present invention, such as the example compounds, have good stability in liver microsomes.

### 10. CYP450 enzyme inhibition test

The purpose of this study was to evaluate the effect of test compounds on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were co-incubated with human liver microsomes and test compounds at different concentrations, and the reaction was initiated by adding reduced nicotinamide adenine dinucleotide phosphate (NADPH). After the completion of the reaction, the samples were treated and subjected to the liquid chromatography-tandem mass spectrometry (LC-MS/MS) to quantitatively detect metabolites produced by specific substrates. The changes in CYP enzyme activity were determined, and IC₅₀ values were calculated to evaluate the inhibitory potential of the test compounds on each CYP enzyme subtype.

Conclusion: The compounds of the present invention, such as the example compounds, do not exhibit obvious inhibitory effects on the five isozymes of human liver microsomal cytochrome P450.

### 11. Caco2 permeability test

The experiment used a monolayer of Caco-2 cells incubated in triplicate in a 96-well Transwell plate. A transport buffer solution (HBSS, 10 mM HEPES, pH 7.4 ± 0.05) containing the compounds of the present invention (2 µM) or the control compounds of digoxin (10 µM), nadolol (2 µM) and metoprolol (2 µM) was added to the administration end hole on the apical side or basal side. DMSO-containing transport buffer solution was added to the corresponding receiving end hole. After incubation for 2 hours at 37°C ± 1°C, the cell plate was removed and an appropriate amount of samples were taken from the apical side and basal side and transferred to a new 96-well plate. Acetonitrile containing an internal standard was then added to precipitate the protein. Samples were analysed using LC MS/MS and the concentrations of the compounds of the present invention and the control compounds were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was assessed by leakage of Lucifer Yellow.

Conclusion: The compounds of the present invention, such as the example compounds, have good Caco2 permeability.

## Claims

1. A compound or a stereoisomer, a tautomer, a deuterated form, a solvate, a prodrug, a metabolite, a pharmaceutically acceptable salt or a eutectic thereof, **characterized in that** the compound is selected from a compound represented by general formula (I),
B-L-K (I);
L is selected from a bond or -C₁₋₅₀ hydrocarbyl-, wherein the hydrocarbyl has 1 to 20 methylene units optionally replaced by -Ak- or -Cy-;
each -Ak- is independently selected from -(CH₂)_{q}-, -(CH₂)_{q}-O-, -O-(CH₂)_{q}-, - (CH₂)_{q}-NR^{L}-, -NR^{L}-(CH₂)_{q}-, -(CH₂)_{q}-NR^{L}C(=O)-, -NR^{L}(CH₂)_{q}C(=O)-, -(CH₂)_{q}-C(=O)NR^{L}-, -C(=O)-, -C(=O)-(CH₂)_{q}-NR^{L}-, -(C=C)_{q}-, -CH=CH-, -Si(R^{L})₂-, - Si(OH)(R^{L})-, -Si(OH)₂-, -P(=O)(OR^{L})-, -P(=O)(R^{L})-, -S-, -S(=O)-, -S(=O)₂- or a bond, wherein the -CH₂- is optionally substituted with 1 to 2 substituents selected from halogen, OH, CN, NH₂, C₁₋₆ alkyl, C₁₋₆ alkoxy, halogen-substituted C₁₋₆ alkyl, hydroxyl-substituted C₁₋₆ alkyl or cyano-substituted C₁₋₆ alkyl;
each q is independently selected from 0, 1, 2, 3, 4, 5 or 6;
each R^{L} is independently selected from H, C₁₋₆ alkyl, 3- to 7-membered heterocyclyl, 3- to 7-membered cycloalkyl, phenyl or 5- to 6-membered heteroaryl, wherein the heterocyclyl or heteroaryl contains 1 to 4 heteroatoms selected from O, S or N;
each -Cy- is independently selected from a bond, a 4- to 8-membered mono-heterocyclic ring, a 4- to 10-membered fused-heterocyclic ring, a 5- to 12-membered spiro-heterocyclic ring, a 7- to 10-membered bridged-heterocyclic ring, C₃₋₇ monocycloalkyl, C₄₋₁₀ fused cycloalkyl, C₅₋₁₂ spirocycloalkyl, C₇₋₁₀ bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally substituted with 1 to 2 substituents selected from halogen, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally substituted with 1 or 2 =O when the heteroatom is selected from S;
B is selected from 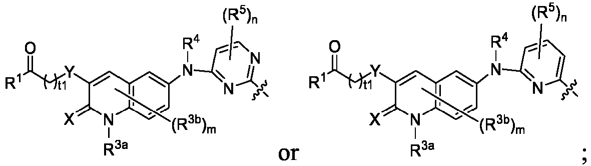
X is selected from O, S or CH₂;
Y is selected from NR^{Y} or O;
R^{Y} is selected from H, C₁₋₆ alkyl, C₃₋₁₀ carbocycle or 3- to 10-membered heterocycle, wherein the alkyl, carbocycle or heterocycle is optionally substituted with 1 to 3 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₃₋₆ carbocycle or 3- to 8-membered heterocycle, and the heterocycle contains 1 to 3 heteroatoms selected from O, S or N;
R¹ is selected from H, OH, -(CH₂)ₜ₂NR^{1a}R^{1b}, C₁₋₆ alkyl or C₁₋₆ alkoxy, wherein the alkyl or alkoxy is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl or C₁₋₆ alkoxy;
R^{1a} and R¹⁶ are each independently selected from H, OH, NH₂, C₁₋₄ alkyl, C₂₋₆ alkynyl or C₁₋₄ alkoxy, wherein the alkyl, alkynyl or alkoxy is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy or C₂₋₆ alkynyl;
or R^{1a} and R^{1b} together with the atom to which they are attached form a 3- to 10-membered ring;
each R^{3a} or R⁴ is independently selected from H, OH, NH₂, C₁₋₆ alkyl, C₂₋₆ alkynyl, C₁₋₆ alkoxy, C₃₋₁₀ carbocycle or 3- to 10-membered heterocycle, wherein the alkyl, alkoxy, alkynyl, carbocycle or heterocycle is optionally substituted with 1 to 4 substituents selected from halogen, OH, cyano, NH(CH₃), N(CH₃)₂, NH(CH₂CH₃), N(CH₂CH₃)₂, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₂₋₆ alkynyl, C₃₋₆ cycloalkyloxy, C₃₋₆ carbocycle or 3- to 8-membered heterocycle, and the heterocycle contains 1 to 3 heteroatoms selected from O, S or N;
each R^{3b} or R⁵ is independently selected from H, halogen, cyano, NH₂, NO₂, OH, C₁₋₆ alkyl, C₂₋₄ alkenyl, C₂₋₄ alkynyl, C₁₋₆ alkoxy, C₃₋₁₀ carbocycle or 3- to 10-membered heterocycle, wherein the alkyl, alkoxy, carbocycle or heterocycle is optionally substituted with 1 to 4 substituents selected from halogen, OH, cyano, NH₂, C₁₋₆ alkyl, halogen-substituted C₁₋₆ alkyl, hydroxyl-substituted C₁₋₆ alkyl, cyano-substituted C₁₋₆ alkyl, C₁₋₆ alkoxy, C₃₋₆ carbocycle or 3- to 8-membered heterocycle, and the heterocycle contains 1 to 3 heteroatoms selected from O, S or N;
t1 is selected from 1, 2, 3, 4 or 5;
t2 is selected from 0, 1, 2, 3, 4 or 5;
m is selected from 0, 1, 2, 3 or 4;
n is selected from 0, 1 or 2;
K is selected from 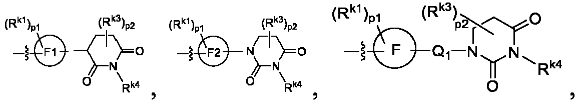 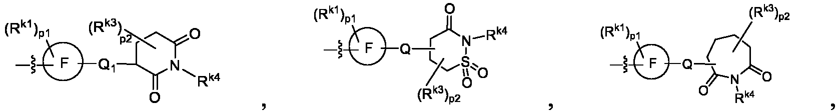 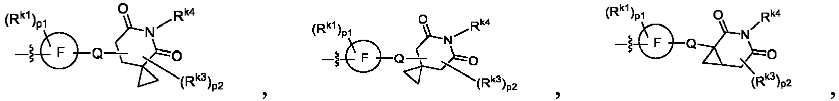 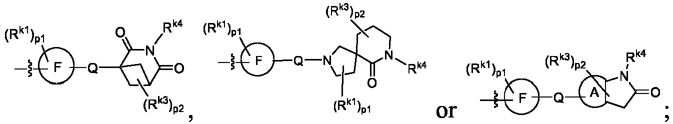
each F1 is independently selected from C₃₋₇ monocyclic carbocycle, C₄₋₁₄ fused ring carbocycle, C₅₋₁₂ spiro ring carbocycle, C₅₋₁₀ bridged ring carbocycle, C₆₋₁₄ aryl, a 6- to 7-membered non-aromatic mono-heterocyclic ring, a 5- to 14-membered bridged-heterocyclic ring, 5- to 6-membered heteroaryl, a 11- to 20-membered fused-heterocyclic ring, phthalazin-1(2*H*)-onyl, benzo[d][1,2,3]triazin-4(3H)-onyl, benzothienyl, 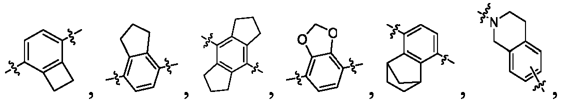 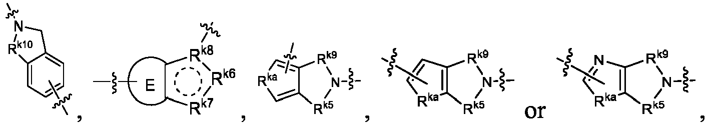 wherein the mono-heterocyclic ring, fused-heterocyclic ring, spiro-heterocyclic ring, bridged-heterocyclic ring or heteroaryl contains 1 to 4 heteroatoms selected from O, S or N, provided that when F1 is selected from 6-membered heteroaryl, the right connection site on the ring is a carbon atom;
each E is independently selected from C₅₋₁₂ carbocycle, 5- to 12-membered heterocycle or a 5- to 12-membered heteroaromatic ring, wherein the heterocycle or heteroaromatic ring contains 1 to 4 heteroatoms selected from O, S or N;
each F2 is independently selected from C₃₋₇ monocyclic non-aromatic carbocycle, C₄₋₁₀ fused ring non-aromatic carbocycle, C₅₋₁₂ spiro ring carbocycle, C₅₋₁₀ bridged ring carbocycle, C₁₂₋₁₄ aryl, a 4- to 7-membered non-aromatic mono-heterocyclic ring, a 5- to 14-membered spiro-heterocyclic ring, a 5- to 14-membered bridged-heterocyclic ring, a 11- to 20-membered fused-heterocyclic ring or 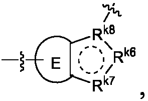 wherein the mono-heterocyclic ring, fused-heterocyclic ring, spiro-heterocyclic ring, bridged-heterocyclic ring or heteroaromatic ring contains 1 to 4 heteroatoms selected from O, S or N;
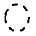 represents a ring selected from an aromatic ring or a non-aromatic ring;
each F is independently selected from C₃₋₇ monocyclic carbocycle, C₄₋₂₀ fused ring carbocycle, C₅₋₂₀ spiro ring carbocycle, C₅₋₂₀ bridged ring carbocycle, a 4- to 7-membered mono-heterocyclic ring, a 4- to 20-membered fused-heterocyclic ring, a 5- to 20-membered spiro-heterocyclic ring or a 5- to 20-membered bridged-heterocyclic ring, wherein the mono-heterocyclic ring, fused-heterocyclic ring, spiro-heterocyclic ring, bridged-heterocyclic ring or heteroaromatic ring contains 1 to 4 heteroatoms selected from O, S or N;
A is selected from C₃₋₈ carbocycle, a benzene ring, 4- to 7-membered heterocycle or a 5- to 6-membered heteroaromatic ring, wherein the heterocycle or heteroaromatic ring contains 1 to 4 heteroatoms selected from O, S or N;
each Q1 is independently selected from -O-, -S-, -CH₂-, -NR^{q}- or -CO-;
each Q is independently selected from a bond, -O-, -S-, -CH₂-, -NR^{q}-, -CO-, - NR^{q}CO-, -CONR^{q}- or 4- to 7-membered heterocycle, wherein the heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
R^{q} is selected from H or C₁₋₄ alkyl;
each R^{k1} is independently selected from H, D, F, Cl, Br, I, OH, =O, NH₂, CN, COOH, CONH₂, C₁₋₆ alkyl, C₁₋₆ alkoxy, C₃₋₆ cycloalkyl or R^{k7a}, wherein the alkyl, alkoxy or cycloalkyl is optionally substituted with 1 to 4 substituents selected from D, F, Cl, Br, I, OH, =O, NH₂, CN, COOH, CONH₂, C₁₋₄ alkyl, C₁₋₄ alkoxy or C₃₋₆ cycloalkyl;
each R^{k3} is independently selected from H, D, F, Cl, Br, I, OH, =O, NH₂, CN, COOH, CONH₂, C₁₋₆ alkyl, C₁₋₆ alkoxy, C₃₋₈ cycloalkyl or 3- to 8-membered heterocyclyl, wherein the alkyl, alkoxy, cycloalkyl or heterocyclyl is optionally substituted with 1 to 4 substituents selected from D, F, Cl, Br, I, OH, =O, NH₂, CN, COOH, CONH₂, C₁₋₄ alkyl or C₁₋₄ alkoxy, and the heterocyclyl contains 1 to 4 heteroatoms selected from O, S or N;
or two R^{k3} together with the carbon atom or ring backbone to which they are directly attached form C₃₋₈ carbocycle or 3- to 8-membered heterocycle, or two R^{k1} together with the carbon atom or ring backbone to which they are directly attached form C₃₋₈ carbocycle or 3- to 8-membered heterocycle, wherein the carbocycle or heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
each R^{k4} is independently selected from H or C₁₋₄ alkyl;
each R^{k5} is independently selected from 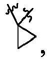 C(CH₃)₂, CO, CH₂, SO₂, 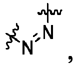 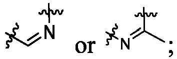
each R^{k6} is independently selected from CO, CH, SO, SO₂, CH₂, N or NR^{k7a};
each R^{k7} is independently selected from 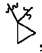 C(CH₃)₂, CO, CH, N, CH₂, O, S or NR^{k7a};
R^{k7a} is selected from H, C₁₋₄ alkyl, C₂₋₄ alkenyl, C₂₋₄ alkynyl, C₃₋₆ cycloalkyl or 3- to 6-membered heterocycloalkyl, wherein the alkyl, cycloalkyl or heterocycloalkyl is optionally substituted with 1 to 4 substituents selected from F, Cl, Br, I, OH, NH₂, CN, CF₃, C₁₋₄ alkyl, C₁₋₄ alkoxy, C₂₋₄ alkenyl, C₂₋₄ alkynyl or C₃₋₆ cycloalkyl;
each R^{k8} is independently selected from C, N or CH;
each R^{k9} is independently selected from a bond, 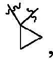 C(CH₃)₂, CO, CH₂, CH₂CH₂ or SO₂;
R^{ka} is selected from O, S or NH;
R^{k10} is selected from CH₂ or CO;
each p1 or p2 is independently selected from 0, 1, 2, 3, 4 or 5;
the compound is optionally substituted with 1-30 D.

2. The compound or the stereoisomer, tautomer, deuterated form, solvate, prodrug, metabolite, pharmaceutically acceptable salt or eutectic thereof according to claim 1, **characterized in that**
L is selected from -Cy1-Ak1-Cy2-Ak2-Cy3-Ak3-Cy4-Ak4-Cy5-Ak5-, -Cyl-Cy2-Cy3-Cy4-Ak1-Ak2-Ak3-Ak4-Ak5-, -Cy1-Ak1-Cy2-Ak2-Cy3-Ak3-Cy4-Ak4-Ak5-, -Ak1-Cy1-Ak2-Cy2-Ak3-Cy3-Ak4-Cy4-Ak5-, -Cy1-Ak1-Cy2-Ak2-Cy3-Cy4-Ak3-Ak4-Ak5-, -Cy1-Ak1-Cy2-Ak2-Ak3-Cy3-Cy4-Ak4-Ak5-, -Cy1-Ak1-Ak2-Ak3-Ak4-Ak5-Cy2-Cy3-Cy4-, -Cy1-Cy2-Ak1-Ak2-Ak3-Ak4-Ak5-Cy3-Cy4-, -Cy1-Cy2-Cy3-Ak1-Ak2-Ak3-Ak4-Ak5-Cy4-, -Cy1-Cy2-Cy3-Cy4-Ak1-Ak2-Ak3-Ak4-Ak5-, -Cy1-Ak1-Cy2-Cy3-Cy4-Ak2-Ak3-Ak4-Ak5-, -Cy1-Cy2-Ak1-Cy3-Cy4-Ak2-Ak3-Ak4-Ak5-, -Cy1-Cy2-Cy3-Ak1-Cy4-Ak2-Ak3-Ak4-Ak5-, - Cy1-Ak1-Ak2-Cy2-Cy3-Cy4-Ak3-Ak4-Ak5-, -Cy1-Cy2-Ak1-Ak2-Cy3-Cy4-Ak3-Ak4-Ak5-, -Cy1-Cy2-Cy3-Ak1-Ak2-Cy4-Ak3-Ak4-Ak5-, -Cy1-Ak1-Ak2-Ak3-Cy2-Cy3-Cy4-Ak4-Ak5-, -Cy1-Cy2-Ak1-Ak2-Ak3-Cy3-Cy4-Ak4-Ak5-, -Cyl-Cy2-Cy3-Ak1-Ak2-Ak3-Cy4-Ak4-Ak5-, -Cy1-Ak1-Ak2-Ak3-Ak4-Cy2-Cy3-Cy4-Ak5-, -Cy1-Cy2-Ak1-Ak2-Ak3-Ak4-Cy3-Cy4-Ak5-, -Cy1-Cy2-Cy3-Ak1-Ak2-Ak3-Ak4-Cy4-Ak5-, -Ak1-Ak2-Ak3-Ak4-Ak5-Cy1-Cy2-Cy3-Cy4-, -Ak1-Cy1-Cy2-Cy3-Cy4-Ak2-Ak3-Ak4-Ak5-, -Ak1-Ak2-Cy1-Cy2-Cy3-Cy4-Ak3-Ak4-Ak5-, -Ak1-Ak2-Ak3-Cy1-Cy2-Cy3-Cy4-Ak4-Ak5-, -Ak1-Ak2-Ak3-Ak4-Cy1-Cy2-Cy3-Cy4-Ak5-, -Ak1-Cy1-Ak2-Ak3-Ak4-Ak5-Cy2-Cy3-Cy4-, -Ak1-Cy1-Cy2-Ak2-Ak3-Ak4-Ak5-Cy3-Cy4-, -Ak1-Cy1-Cy2-Cy3-Ak2-Ak3-Ak4-Ak5-Cy4-, - Ak1-Ak2-Cy1-Ak3-Ak4-Ak5-Cy2-Cy3-Cy4-, -Ak1-Ak2-Cy1-Cy2-Ak3-Ak4-Ak5-Cy3-Cy4-, -Ak1-Ak2-Cy1-Cy2-Cy3-Ak3-Ak4-Ak5-Cy4-, -Ak1-Ak2-Ak3-Cy1-Ak4-Ak5-Cy2-Cy3-Cy4-, -Ak1-Ak2-Ak3-Cy1-Cy2-Ak4-Ak5-Cy3-Cy4-, -Ak1-Ak2-Ak3-Cy1-Cy2-Cy3-Ak4-Ak5-Cy4-, -Ak1-Ak2-Ak3-Ak4-Cy1-Ak5-Cy2-Cy3-Cy4-, -Ak1-Ak2-Ak3-Ak4-Cy1-Cy2-Ak5-Cy3-Cy4-, -Ak1-Ak2-Ak3-Ak4-Cy1-Cy2-Cy3-Ak5-Cy4-, -Ak1-, -Ak1-Ak2-, -Ak1-Ak2-Ak3-, -Ak1-Ak2-Ak3-Ak4-, - Ak1-Ak2-Ak3-Ak4-Ak5-, -Ak1-Ak2-Ak3-Ak4-Ak5-Ak6-, -Ak1-Ak2-Ak3-Ak4-Ak5-Ak6-Ak7-, -Ak1-Ak2-Ak3-Ak4-Ak5-Ak6-Ak7-Ak8-, or -Ak1-Ak2-Ak3-Ak4-Ak5-Ak6-Ak7-Ak8-Ak9;
Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from halogen, OH, CN, NH₂, C₁₋₄ alkyl, C₁₋₄ alkoxy, halogen-substituted C₁₋₄ alkyl, hydroxyl-substituted C₁₋₄ alkyl or cyano-substituted C₁₋₄ alkyl;
each Cy1, Cy2, Cy3, Cy4 or Cy5 is independently selected from a bond, a 4-to 7-membered mono-heterocyclic ring, a 4- to 10-membered fused-heterocyclic ring, a 5- to 12-membered spiro-heterocyclic ring, a 7- to 10-membered bridged-heterocyclic ring, C₃₋₇ monocycloalkyl, C₄₋₁₀ fused cycloalkyl, C₅₋₁₂-membered spirocycloalkyl, C₇₋₁₀-membered bridged cycloalkyl, 5- to 10-membered heteroaryl or 6- to 10-membered aryl, wherein the aryl, heteroaryl, cycloalkyl, mono-heterocyclic ring, fused-heterocyclic ring, spiro-heterocyclic ring or bridged-heterocyclic ring is optionally substituted with 1 to 2 substituents selected from F, Cl, Br, I, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the heteroaryl, mono-heterocyclic ring, fused-heterocyclic ring, spiro-heterocyclic ring or bridged-heterocyclic ring contains 1 to 4 heteroatoms selected from O, S or N, and is optionally substituted with 1 or 2 =O when the heteroatom is selected from S;
each q is independently selected from 0, 1, 2, 3 or 4;
each R^{L} is independently selected from H or C₁₋₆ alkyl.

3. The compound or the stereoisomer, tautomer, deuterated form, solvate, prodrug, metabolite, pharmaceutically acceptable salt or eutectic thereof according to claim 2, **characterized in that**
Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C-C)_{q}- or a bond, wherein the -CH₂- is optionally substituted with 1 to 2 substituents selected from F, Cl, Br, I, OH, CN, NH₂, CF₃, hydroxymethyl, C₁₋₄ alkyl or C₁₋₄ alkoxy;
each R^{L} is independently selected from H or C₁₋₄ alkyl;
each Cy1, Cy2, Cy3, Cy4 or Cy5 is independently selected from a bond, a 4-to 7-membered nitrogen-containing mono-heterocyclic ring, a 4- to 10-membered nitrogen-containing fused-heterocyclic ring, a 5- to 12-membered nitrogen-containing spiro-heterocyclic ring, a 7- to 10-membered nitrogen-containing bridged-heterocyclic ring, C₃₋₇ monocycloalkyl, C₄₋₁₀ fused cycloalkyl, C₅₋₁₂ spirocycloalkyl, C₇₋₁₀ bridged cycloalkyl, 5- to 10-membered heteroaryl or 6- to 10-membered aryl, wherein the mono-heterocyclic ring, fused-heterocyclic ring, bridged-heterocyclic ring, spiro-heterocyclic ring, cycloalkyl, aryl or heteroaryl is optionally substituted with 1 to 2 substituents selected from F, Cl, Br, I, OH, COOH, CN, NH₂, =O, C₁₋₄ alkyl, halogen-substituted C₁₋₄ alkyl, hydroxyl-substituted C₁₋₄ alkyl or C₁₋₄ alkoxy, and the mono-heterocyclic ring, fused-heterocyclic ring, bridged-heterocyclic ring, spiro-heterocyclic ring or heteroaryl contains 1 to 4 heteroatoms selected from O, S or N, and is optionally substituted with 1 or 2 =O when the heteroatom is selected from S;
R^{Y} is selected from H, methyl, ethyl, propyl, isopropyl or cyclopropyl, wherein the methyl, ethyl, propyl, isopropyl, cyclopropyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
R¹ is selected from H, OH, NR^{1a}R^{1b}, methyl, ethyl, propyl, isopropyl or methoxy, wherein the methyl, ethyl, propyl, isopropyl or methoxy is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl or C₁₋₄ alkoxy;
R^{1a} and R^{1b} are each independently selected from H, OH, NH₂, methyl, ethyl, propyl, isopropyl, methoxy, propargyl or propynyl, wherein the methyl, ethyl, propyl, isopropyl, methoxy, propargyl or propynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy or C₂₋₄ alkynyl;
each R^{3a} or R⁴ is independently selected from H, OH, NH₂, methyl, ethyl, propyl, propargyl, propynyl, isopropyl, cyclopropyl, cyclobutyl, cyclopentyl, cyclohexyl, phenyl, azetidinyl, pyrrolidyl or piperidyl, wherein the methyl, ethyl, propyl, isopropyl, cyclopropyl, cyclobutyl, cyclopentyl, cyclohexyl, phenyl, azetidinyl, pyrrolidyl, piperidyl, propargyl or propynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ cycloalkyloxy, C₂₋₄ alkynyl, C₃₋₆ carbocycle or 3- to 6-membered heterocycle, and the heterocycle contains 1 to 3 heteroatoms selected from O, S or N;
each R^{3b} or R⁵ is independently selected from H, F, Cl, Br, I, cyano, NH₂, OH, methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl, piperidyl or ethynyl, wherein the methyl, ethyl, propyl, isopropyl, methoxy, ethoxy, propoxy, isopropoxy, cyclopropyl, cyclobutyl, cyclopentyl, cyclohexyl, phenyl, azetidinyl, pyrrolidyl, piperidyl or ethynyl is optionally substituted with 1 to 3 substituents selected from halogen, OH, cyano, NH₂, C₁₋₄ alkyl, halogen-substituted C₁₋₄ alkyl, hydroxyl-substituted C₁₋₄ alkyl, cyano-substituted C₁₋₄ alkyl, C₁₋₄ alkoxy, C₃₋₆ carbocycle or 3- to 6-membered heterocycle, and the heterocycle contains 1 to 3 heteroatoms selected from O, S or N;
each F1 is independently selected from cyclobutyl, cyclopentyl, cyclohexyl, piperidyl, piperazinyl, phenyl, naphthyl, pyridyl, pyrazinyl, pyridazinyl, pyridazinonyl, pyrimidyl, phthalazin-1(2*H*)-onyl, benzo[d][1,2,3]triazin-4(3H)-onyl, thienyl, benzothienyl, triazolyl, 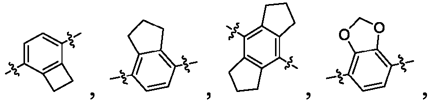 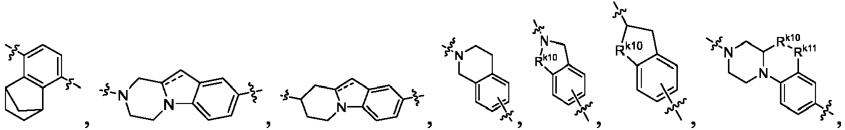 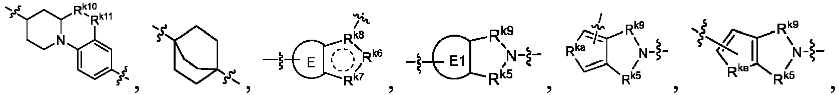 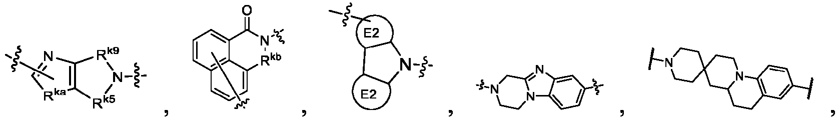 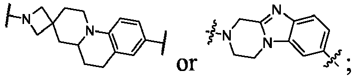
-̅ -̅ -̅ -̅ -̅ is selected from a single bond or a double bond;
each F2 is independently selected from 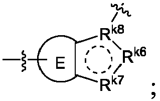
each F is independently selected from cyclobutyl, cyclopentyl, cyclohexyl, azetidinyl, pyrrolidyl, piperidyl, piperazinyl, pyridyl, pyrimidyl, pyridazinyl, pyrazinyl, triazinyl, pyrrolyl, imidazolyl, triazolyl, oxazolyl, furyl, thienyl, thiazolyl, 2-pyridonyl, pyrazolyl, phenyl, naphthyl, anthryl, phenanthryl, benzothiazolyl, indolyl, 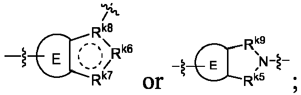
each E or A is independently selected from a benzene ring, a naphthalene ring or a pyridine ring;
each E1 is independently selected from 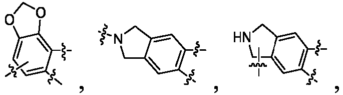 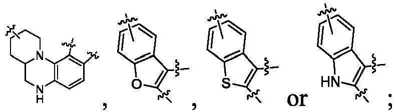
each E2 is independently selected from a benzene ring, a pyridine ring, a pyrimidine ring, a thiazole ring, a thiophene ring or a furan ring;
R^{q} is selected from H, methyl or ethyl;
R^{k1} and R^{k3} are each independently selected from H, D, F, Cl, Br, I, OH, =O, NH₂, CF₃, CN, COOH, CONH₂, C₁₋₄ alkyl, C₁₋₄ alkoxy or R^{k7a}, wherein the alkyl or alkoxy is optionally substituted with 1 to 4 substituents selected from D, F, Cl, Br, I, OH or NH₂;
or two R^{k3} together with the carbon atom or ring backbone to which they are directly attached form C₃₋₇ carbocycle or 3- to 7-membered heterocycle, or two R^{k1} together with the carbon atom or ring backbone to which they are directly attached form C₃₋₇ carbocycle or 3- to 7-membered heterocycle, wherein the carbocycle or heterocycle is optionally substituted with 1 to 4 substituents selected from F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
each R^{k4} is independently selected from H, methyl, ethyl or propyl;
each R^{k5} is independently selected from CO, CH₂, SO₂ or 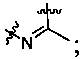
R^{k7a} is selected from H, C₁₋₄ alkyl, C₂₋₄ alkenyl, C₂₋₄ alkynyl, C₃₋₆ cycloalkyl or 3- to 6-membered heterocyclyl, wherein the alkyl, alkenyl, alkynyl, heterocyclyl or cycloalkyl is optionally substituted with 1 to 4 substituents selected from F, Cl, Br, I, OH, CN, CF₃, C₁₋₄ alkyl, C₁₋₄ alkoxy, C₂₋₄ alkenyl, C₂₋₄ alkynyl or C₃₋₆ cycloalkyl;
each R^{k9} is independently selected from CO, SO₂ or CH₂;
R^{kb} is selected from a bond or CO;
R^{k10} is selected from CH₂ or CO;
R^{k11} is selected from NH, O or CH₂.

4. The compound or the stereoisomer, tautomer, deuterated form, solvate, prodrug, metabolite, pharmaceutically acceptable salt or eutectic thereof according to claim 3, **characterized in that**
Ak1, Ak2, Ak3, Ak4, Ak5, Ak6, Ak7, Ak8 and Ak9 are each independently selected from -(CH₂)_{q}-, -(CH₂)_{q}-O-, -O-(CH₂)_{q}-, -(CH₂)_{q}-NR^{L}-, -NR^{L}-(CH₂)_{q}-, - (CH₂)_{q}-NR^{L}C(=O)-, -(CH₂)_{q}-C(=O)NR^{L}-, -C(=O)-, -C(=O)-(CH₂)_{q}-NR^{L}-, -(C≡C)_{q}- or a bond, wherein the -CH₂- is optionally substituted with 1 to 2 substituents selected from F, Cl, Br, I, OH, CN, NH₂, CF₃, hydroxymethyl, methyl, ethyl, methoxy or ethoxy;
R^{L} is selected from H, methyl or ethyl;
each q is independently selected from 0, 1, 2 or 3;
each Cy1, Cy2, Cy3, Cy4 or Cy5 is independently selected from a bond or one of the following substituted or unsubstituted groups: cyclopropyl, cyclobutyl, cyclopentyl, cyclohexyl, azetidinyl, pyrrolidyl, azacyclohexenyl, piperidyl, morpholinyl, piperazinyl, 1,4-diazepanyl, phenyl, pyridyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-pyrrolidyl, cyclopropyl-fused-piperidyl, cyclobutyl-fused-azetidinyl, cyclobutyl-fused-pyrrolidyl, cyclobutyl-fused-piperidyl, cyclopentyl-fused-azetidinyl, cyclopentyl-fused-pyrrolidyl, cyclopentyl-fused-piperidyl, cyclohexyl-fused-azetidinyl, cyclohexyl-fused-pyrrolidyl, cyclohexyl-fused-piperidyl, azetidinyl-fused-azetidinyl, azetidinyl-fused-pyrrolidyl, azetidinyl-fused-piperidyl, pyrrolidyl-fused-azetidinyl, pyrrolidyl-fused-pyrrolidyl, pyrrolidyl-fused-piperidyl, piperidyl-fused-azetidinyl, piperidyl-fused-pyrrolidyl, piperidyl-fused-piperidyl, cyclopropyl-spiro-azetidinyl, cyclopropyl-spiro-pyrrolidyl, cyclopropyl-spiro-piperidyl, cyclopropyl-spiro-piperazinyl, cyclobutyl-spiro-azetidinyl, cyclobutyl-spiro-pyrrolidyl, cyclobutyl-spiro-piperidyl, cyclopentyl-spiro-azetidinyl, cyclopentyl-spiro-pyrrolidyl, cyclopentyl-spiro-piperidyl, cyclohexyl-spiro-azetidinyl, cyclohexyl-spiro-pyrrolidyl, cyclohexyl-spiro-piperidyl, azetidinyl-spiro-azetidinyl, azetidinyl-spiro-pyrrolidyl, azetidinyl-spiro-piperidyl, pyrrolidyl-spiro-azetidinyl, pyrrolidyl-spiro-pyrrolidyl, pyrrolidyl-spiro-piperidyl, piperidyl-spiro-azetidinyl, piperidyl-spiro-pyrrolidyl, piperidyl-spiro-piperidyl, cyclopropyl-spiro-piperazinyl, 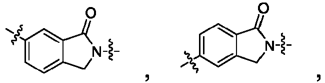 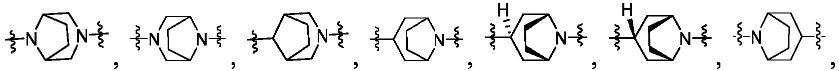 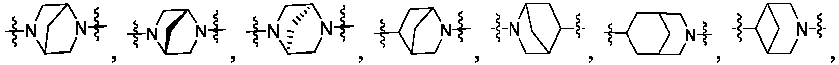 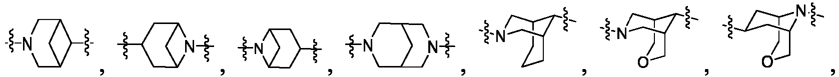 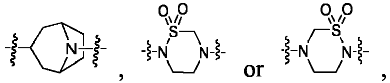 which, when substituted, is optionally substituted with 1 to 4 substituents selected from F, Cl, Br, I, OH, NH₂, COOH, CN, =O, C₁₋₄ alkyl, halogen-substituted C₁₋₄ alkyl, hydroxyl-substituted C₁₋₄ alkyl or C₁₋₄ alkoxy;
X is selected from O or S;
Y is selected from NH or O;
R¹ is selected from H, OH, NR^{1a}R^{1b}, methyl, ethyl, propyl, isopropyl or methoxy, wherein the methyl, ethyl, propyl, isopropyl or methoxy is optionally substituted with 1 to 3 substituents selected from F, Cl, Br, I, OH, cyano, NH₂, methyl, ethyl or CF₃;
R^{1a} and R^{1b} are each independently selected from H, OH, NH₂, methyl, ethyl, propyl, isopropyl, methoxy, propargyl or propynyl, wherein the methyl, ethyl, propyl, isopropyl, methoxy, propargyl or propynyl is optionally substituted with 1 to 3 substituents selected from F, Cl, Br, I, OH, cyano, NH₂, methyl, ethyl, methoxy, ethynyl or CF₃;
R^{3a} is selected from H, OH, NH₂, methyl, ethyl, propyl, isopropyl, cyclopropyl, cyclobutyl, cyclopentyl, cyclohexyl, phenyl, azetidinyl, pyrrolidyl, piperidyl, propargyl or propynyl, wherein the methyl, ethyl, propyl, isopropyl, cyclopropyl, cyclobutyl, cyclopentyl, cyclohexyl, phenyl, azetidinyl, pyrrolidyl, piperidyl, propargyl or propynyl is optionally substituted with 1 to 3 substituents selected from F, Cl, Br, I, OH, cyano, NH₂, methyl, ethyl, cyclopropyl, cyclobutyl, cyclopentyl, cyclohexyl, ethynyl, methoxy or cyclopropyloxy;
each R⁴ is independently selected from H, methyl, ethyl or cyclopropyl;
each R^{3b} is independently selected from H, F, Cl, Br, I, OH, CN, methoxy, methyl, ethyl or cyclopropyl;
each R⁵ is independently selected from H, F, Cl, Br, I, cyano, NH₂, OH, methyl, ethyl, propyl, isopropyl, methoxy, ethoxy, propoxy, isopropoxy, cyclopropyl or ethynyl, wherein the methyl, ethyl, propyl, isopropyl, methoxy, ethoxy, propoxy, isopropoxy, cyclopropyl or ethynyl is optionally substituted with 1 to 3 substituents selected from F, Cl, Br, I, OH, cyano, NH₂, methyl, ethyl or cyclopropyl;
each F1 is independently selected from cyclohexyl, piperidyl, piperazinyl, phenyl, naphthyl, pyridyl, pyrimidyl, pyrazinyl, pyridazinyl, pyridazinonyl, phthalazin-1(2*H*)-onyl, benzo[d][1,2,3]triazin-4(3H)-onyl, thienyl, triazolyl, 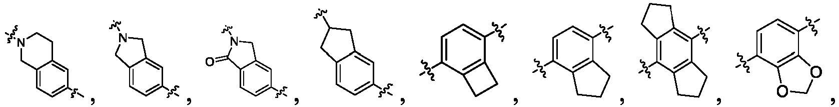 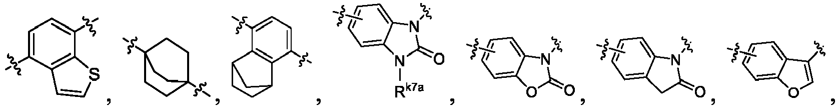 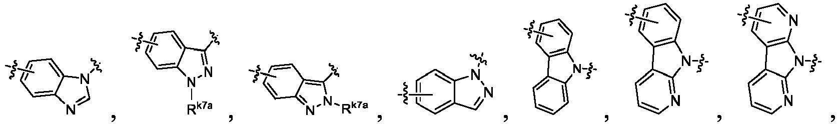 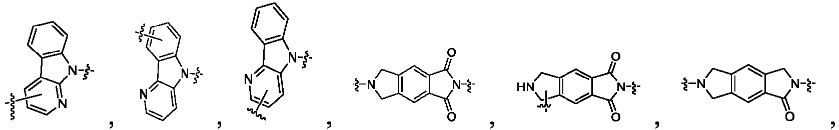 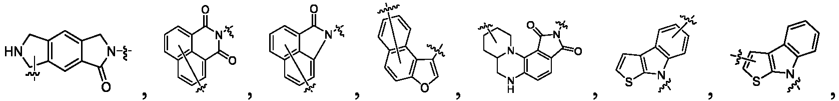 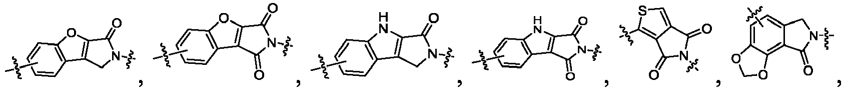 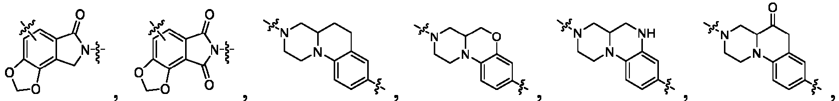 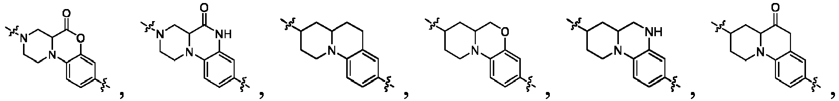 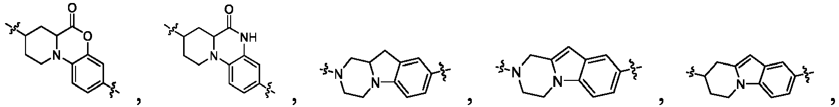 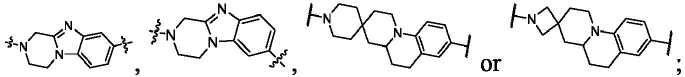
each F2 is independently selected from 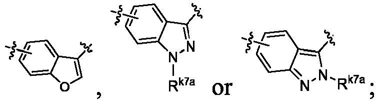
each F is independently selected from cyclobutyl, cyclopentyl, cyclohexyl, azetidinyl, pyrrolidyl, piperidyl, piperazinyl, pyridyl, pyrimidyl, pyridazinyl, pyrazinyl, triazinyl, pyrrolyl, imidazolyl, triazolyl, oxazolyl, furyl, thienyl, thiazolyl, 2-pyridonyl, pyrazolyl, phenyl, naphthyl, anthryl, phenanthryl, benzothiazolyl, indolyl, 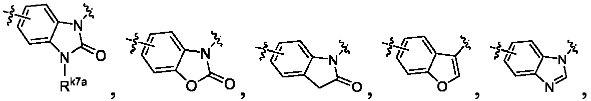 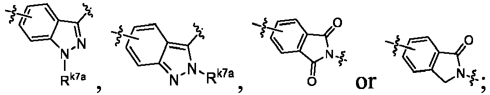
Q1 is selected from -O-, -S-, -CH₂-, NH, N(CH₃) or -CO-;
Q is selected from a bond, C(=O), CH₂, NH, N(CH₃), O, S, NHC(=O), C(=O)NH, N(CH₃)C(=O), C(=O)N(CH₃), 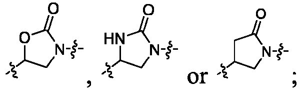
R^{k7a} is selected from H, methyl, ethyl, propyl, isopropyl, ethenyl, propenyl, allyl, ethynyl, propynyl, propargyl, cyclopropyl, cyclobutyl, cyclopentyl, cyclohexyl, azetidinyl, pyrrolidyl, piperidyl, oxacyclobutyl, tetrahydrofuryl or tetrahydropyranyl, wherein the methyl, ethyl, propyl, isopropyl, cyclopropyl, cyclobutyl, cyclopentyl, cyclohexyl, azetidinyl, pyrrolidyl, piperidyl, oxacyclobutyl, tetrahydrofuryl or tetrahydropyranyl is optionally substituted with 1 to 4 substituents selected from F, Cl, Br, I, OH, CN, CF₃, C₁₋₄ alkyl, C₁₋₄ alkoxy, ethenyl, propenyl, allyl, ethynyl, propynyl, propargyl or C₃₋₆ cycloalkyl;
R^{k1} and R^{k3} are each independently selected from H, D, F, Cl, Br, I, OH, =O, NH₂, CF₃, CN, COOH, CONH₂, methyl, ethyl, isopropyl, methoxy, ethoxy, isopropoxy, ethynyl, cyclopropyl or azetidinyl, wherein the methyl, ethyl, isopropyl, methoxy, ethoxy, isopropoxy, ethynyl, cyclopropyl or azetidinyl is optionally substituted with 1 to 4 substituents selected from D, F, Cl, Br, I, OH, NH₂, CN, cyclopropyl or methoxy;
or two R^{k3} together with the carbon atom or ring backbone to which they are directly attached form C₃₋₆ carbocycle or 3- to 7-membered heterocycle, or two R^{k1} together with the carbon atom or ring backbone to which they are directly attached form C₃₋₆ carbocycle or 3- to 7-membered heterocycle, wherein the carbocycle or heterocycle is optionally substituted with 1 to 4 substituents selected from D, F, Cl, Br, I, OH, =O, NH₂, CN, COOH, CONH₂, C₁₋₄ alkyl or C₁₋₄ alkoxy, and the heterocycle contains 1 to 4 heteroatoms selected from O, S or N;
each p1 or p2 is independently selected from 0, 1, 2 or 3.

5. The compound or the stereoisomer, tautomer, deuterated form, solvate, prodrug, metabolite, pharmaceutically acceptable salt or eutectic thereof according to claim 4, **characterized in that**
Ak1, Ak2, Ak3, Ak4, Ak5, Ak6, Ak7, Ak8 and Ak9 are each independently selected from a bond, -O-, -OCH₂-, -CH₂O-, -OCH₂CH₂-, -CH₂CH₂O-, -C=C-, - C(CH₃)₂-, -CF₂-, -CH₂-, -CH₂CH₂-, -CH₂CH₂CH₂-, -N(CH₃)-, -NH-, -CH₂N(CH₃)-, -CH₂NH-, -NHCH₂-, -CH₂CH₂N(CH₃)-, -CH₂CH₂NH-, -NHCH₂CH₂-, -C(=O)-, - C(=O)CH₂NH-, -CH₂C(=O)NH-, -C(=O)NH- or -NHC(=O)-;
each Cy1, Cy2, Cy3, Cy4 or Cy5 is independently selected from a bond or one of the following substituted or unsubstituted groups: 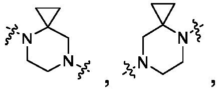 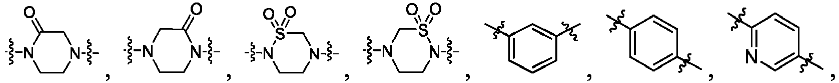 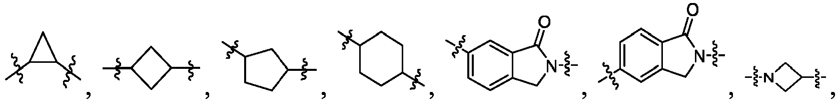 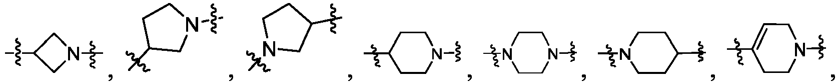 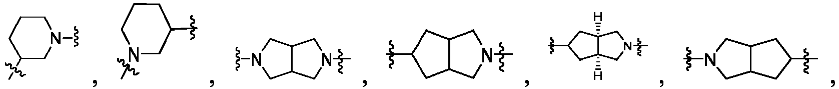 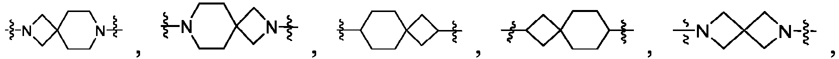 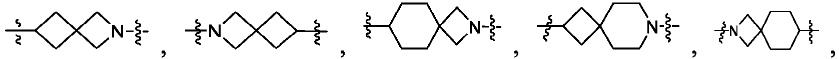 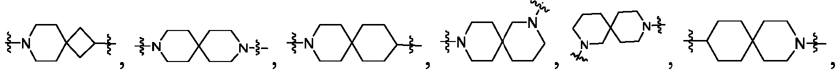 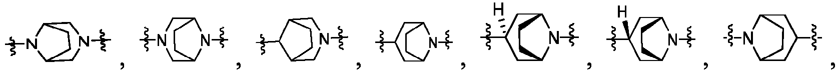 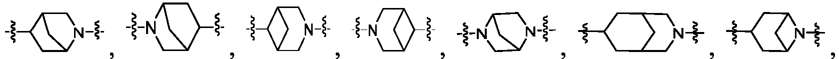 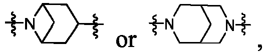 which, when substituted, is substituted with 1 to 4 substituents selected from F, CF₃, OH, methyl, =O, hydroxymethyl, COOH, CN or NH₂;
B is selected from one of the structural fragments shown in Table B-1;
K is selected from one of the structural fragments shown in Table K-1 or Table K-2.

6. The compound or the stereoisomer, tautomer, deuterated form, solvate, prodrug, metabolite, pharmaceutically acceptable salt or eutectic thereof according to claim 5, **characterized in that**
L is selected from a bond, -Ak1-, -Ak1-Ak2-, -Ak1-Ak2-Ak3-, -Ak1-Ak2- Ak3-Ak4-, -Ak1-Ak2-Ak3-Ak4-Ak5-, -Ak1-Ak2-Ak3-Ak4-Ak5-Ak6-, -Cy1-, - Cy1-Ak1-, -Cy1-Ak1-Ak2-, -Cy1-Ak1-Ak2-Ak3-, -Cy1-Ak1-Ak2-Ak3-Ak4-, - Cy1-Cy2-, -Cy1-Ak1-Cy2-, -Cy1-Cy2-Ak2-, -Cy1-Ak1-Cy2-Ak2-, -Cy1-Ak1- Cy2-Ak2-Ak3-, -Cy1-Ak1-Cy2-Ak2-Ak3-Ak4-, -Cy1-Cy2-Ak2-Ak3-, -Cy1-Cy2- Ak2-Ak3-Ak4-, -Cy1-Ak1-Cy2-Ak2-Ak3-Ak4-, -Cy1-Ak1-Ak2-Cy3-, -Cy1-Ak1- Ak2-Cy3-Ak3-, -Cy1-Cy2-Cy3-, -Cy1-Ak1-Cy2-Cy3-, -Cy1-Cy2-Ak2-Cy3-, - Cy1-Cy2-Cy3-Ak3-, -Cy1-Ak1-Cy2-Cy3-Ak3-, -Cy1-Cy2-Ak2-Cy3-Ak3-, -Cyl- Ak1-Cy2-Ak2-Cy3-, -Cy1-Ak1-Cy2-Ak2-Cy3-Ak3-, -Cy1-Cy2-Cy3-Ak3-Ak4-, - Cy1-Cy2-Cy3-Ak3-Cy4-, -Cy1-Cy2-Cy3-Cy4-, -Cy1-Ak1-Cy2-Cy3-Cy4-, -Cyl- Cy2-Ak2-Cy3-Cy4-, -Cy1-Cy2-Cy3-Ak3-Cy4-, -Cy1-Cy2-Cy3-Cy4-Ak4-, -Cyl- Ak1-Cy2-Ak2-Cy3-Ak3-Cy4-, -Cy1-Ak1-Cy2-Ak2-Cy3-Cy4-, -Ak1-Cy2-, -Ak1- Cy2-Cy3-, -Ak1-Ak2-Cy3-, -Ak1-Ak2-Cy3-Cy4-, -Ak1-Cy2-Ak2-Cy3-, -Ak1- Cy2-Cy3-Ak3-Cy4-, -Ak1-Cy2-Cy3-Cy4-Ak4-Cy5-, -Ak1-Cy2-Ak2-, -Cy1-Cy2- Cy3-Ak3-Ak4-Ak5-, -Cy1-Cy2-Ak2-Cy3-Ak3-Ak4-Ak5-, -Cy1-Ak1-Cy2-Ak2- Ak3-Ak4-Ak5-, -Cy1-Cy2-Cy3-Cy4-Ak4-Ak5-, -Cy1-Ak1-Ak2-Ak3-Ak4-Ak5-, - Ak1-Cy2-Ak2-Ak3-Ak4-Ak5-, -Ak1-Cy2-Ak2-Ak3-Ak4- or -Ak1-Cy2-Ak2-Ak3-.

7. The compound or the stereoisomer, tautomer, deuterated form, solvate, prodrug, metabolite, pharmaceutically acceptable salt or eutectic thereof according to claim 6, **characterized in that**
L is selected from a bond or one of the structural fragments shown in Table L-1, wherein the left side of the group is linked to B.

8. The compound or the stereoisomer, tautomer, deuterated form, solvate, prodrug, metabolite, pharmaceutically acceptable salt or eutectic thereof according to claim 6, **characterized in that**
L is selected from a bond or one of the structural fragments shown in Table L-2, wherein the left side of the group is linked to B;
K is selected from one of the structural fragments shown in Table K-2.

9. The compound or the stereoisomer, tautomer, deuterated form, solvate, prodrug, metabolite, pharmaceutically acceptable salt or eutectic thereof according to claim 1, **characterized in that** the compound represented by general formula (I) is selected from a compound represented by general formula (II), 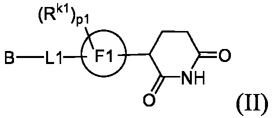
L1 is selected from -Cy1-, -Cy1-Ak1-, -Cy1-Cy2-, -Cy1-Ak1-Cy2- or -Cyl-Ak1-Cy2-Cy3-;
Ak1 is selected from -CH₂-, -O- or -CH₂-CH₂-;
the definitions of Cy1, Cy2 and Cy3 are the same as those in claim 5;
F1 is selected from phenyl, 5- to 6-membered heteroaryl or a 13- to 14-membered fused heterotricyclic ring;
the definition of B is the same as that in claim 1;
the definitions of R^{k1} and p1 are the same as those in claim 4.

10. The compound or the stereoisomer, tautomer, deuterated form, solvate, prodrug, metabolite, pharmaceutically acceptable salt or eutectic thereof according to claim 1, **characterized in that** the compound represented by general formula (I) is selected from a compound represented by general formula (III), 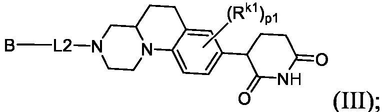
the definition of B is the same as that in claim 1;
L2 is selected from -Cy1-, -Cy1-Ak1- or -Cy1-Ak1-Cy2-;
each Cy1 or Cy2 is independently selected from one of the following substituted or unsubstituted groups: 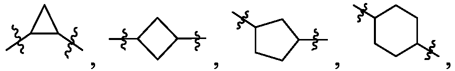 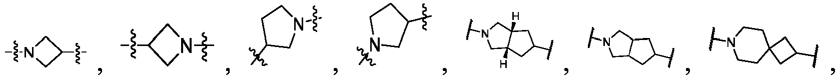 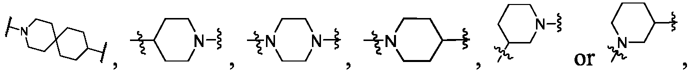 which, when substituted, is substituted with 1 to 4 substituents selected from F, CF₃, OH, methyl, =O, hydroxymethyl, COOH, CN or NH₂;
Ak1 is selected from -CH₂-, -O- or -CH₂-CH₂-;
each R^{k1} is independently selected from H, D, F, Cl, Br, I, OH, =O, NH₂, CF₃, CN, COOH, CONH₂, methyl, ethyl, methoxy or ethoxy;
p1 is selected from 0, 1 or 2.

11. The compound or the stereoisomer, tautomer, deuterated form, solvate, prodrug, metabolite, pharmaceutically acceptable salt or eutectic thereof according to claim 1, **characterized in that** the compound is selected from one of the structures shown in Table S-1.

12. A pharmaceutical composition, **characterized in that** the pharmaceutical composition comprises the compound or the stereoisomer, tautomer, deuterated form, solvate, prodrug, metabolite, pharmaceutically acceptable salt or eutectic thereof according to any one of claims 1-11, and a pharmaceutically acceptable carrier.

13. A pharmaceutical composition or pharmaceutical preparation, **characterized in that** the pharmaceutical composition or pharmaceutical preparation comprises 1-1500 mg of the compound or the stereoisomer, tautomer, deuterated form, solvate, prodrug, metabolite, pharmaceutically acceptable salt or eutectic thereof according to any one of claims 1-11, and a pharmaceutical excipient.

14. Use of the compound or the stereoisomer, tautomer, deuterated form, solvate, prodrug, metabolite, pharmaceutically acceptable salt or eutectic thereof according to any one of claims 1-11, or the pharmaceutical composition according to claim 12 or 13 in the preparation of a drug for treating a disease related to the activity or expression level of Bcl6.

15. Use of the compound or the stereoisomer, tautomer, deuterated form, solvate, prodrug, metabolite, pharmaceutically acceptable salt or eutectic thereof according to any one of claims 1-11, or the pharmaceutical composition according to claim 12 or 13 in the preparation of a drug for treating a disease related to the inhibition or degradation of Bcl6.

16. The use according to claim 14 or 15, **characterized in that** the disease is selected from cancer.

17. A method for treating a disease in a mammal, **characterized in** the method comprises administering to a subject a therapeutically effective amount of the compound or the stereoisomer, tautomer, deuterated form, solvate, prodrug, metabolite, pharmaceutically acceptable salt or eutectic thereof according to any one of claims 1-11, or the pharmaceutical composition according to claim 12 or 13, and the therapeutically effective amount is preferably 1-1500 mg, and the disease is preferably cancer.
